(19) Europäisches Patentamt
European Patent Office
Office européen des brevets

(11) EP 1 756 084 B1

(12) **EUROPEAN PATENT SPECIFICATION**

(45) Date of publication and mention of the grant of the patent:
**26.11.2008 Bulletin 2008/48**

(21) Application number: **05755596.3**

(22) Date of filing: **02.06.2005**

(51) Int Cl.:
***C07D 401/04*** (2006.01) ***C07D 401/12*** (2006.01)
***C07D 401/14*** (2006.01) ***C07D 403/04*** (2006.01)
***C07D 403/14*** (2006.01) ***C07D 405/14*** (2006.01)
***C07D 409/14*** (2006.01) ***C07D 413/14*** (2006.01)
***C07D 417/14*** (2006.01) ***A61K 31/506*** (2006.01)
***A61P 3/10*** (2006.01)

(86) International application number:
**PCT/US2005/019318**

(87) International publication number:
**WO 2005/121121 (22.12.2005 Gazette 2005/51)**

(54) **SUBSTITUTED ARYL AND HETEROARYL DERIVATIVES AS MODULATORS OF METABOLISM AND THE PROPHYLAXIS AND TREATMENT OF DISORDERS RELATED THERETO**

SUBSTITUIERTE ARYL- UND HETEROARYLDERIVATE ALS MODULATOREN DES STOFFWECHSELS UND FÜR DIE PROPHYLAXE UND BEHANDLUNG VON DAMIT IN ZUSAMMENHANG STEHENDEN ERKRANKUNGEN

DERIVES D'ARYLE ET D'HETEROARYLE SUBSTITUES TENANT LIEU DE MODULATEURS DU METABOLISME ET PREVENTION ET TRAITEMENT DE TROUBLES ASSOCIES

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **04.06.2004 US 577354 P**

(43) Date of publication of application:
**28.02.2007 Bulletin 2007/09**

(73) Proprietor: **Arena Pharmaceuticals, Inc.**
**San Diego, CA 92121 (US)**

(72) Inventors:
- **JONES, Robert, M.**
  **San Diego, CA 92130 (US)**
- **SEMPLE, Graeme**
  **San Diego, CA 92127 (US)**
- **XIONG, Yifeng**
  **San Diego, CA 92130 (US)**
- **SHIN, Young-Jun**
  **San Diego, CA 92126 (US)**
- **REN, Albert, S.**
  **San Diego, CA 92130 (US)**
- **LEHMANN, Juerg**
  **San Diego, CA 92129 (US)**
- **FIORAVANTI, Beatriz**
  **Tucson, AR 85704 (US)**
- **BRUCE, Marc, A.**
  **Escondido, California 92029 (US)**
- **CHOI, Jin, Sun, Karoline**
  **San Diego, CA 92131 (US)**

(74) Representative: **Wytenburg, Wilhelmus Johannes et al**
**Mewburn Ellis LLP**
**York House**
**23 Kingsway**
**GB-London WC2B 6HP (GB)**

(56) References cited:
**EP-A- 1 054 005** **WO-A-01/17995**
**WO-A-02/45652** **WO-A-03/000663**
**WO-A-03/000687** **WO-A-03/002544**
**WO-A-20/04041164** **WO-A-20/04078114**
**US-A1- 2005 070 562**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 756 084 B1

SEQUENCE LISTING

<110> Arena Pharmaceuticals, Inc.
Jones, Robert M.
Semple, Graeme
xiong, Yifeng
Shin, Young Jun
Ren, Albert
Lehmann, Juerg
Fioravanti, Beatriz
Bruce, Marc
Choi, Jin Sun Karoline

<120> SUBSTITUTED ARYL AND HETEROARYL DERIVATIVES AS MODULATORS OF METABOLISM AND THE PROPHYLAXIS AND TREATMENT OF DISORDERS RELATED THERETO

<130> 70.WO1

<160> 7

<170> PatentIn version 3.2

<210> 1
<211> 1191
<212> DNA
<213> Homo sapien

<400> 1

```
atgtacccat acgacgtccc agactacgct ggaagcttgg aatcatcttt ctcatttgga      60
gtgatccttg ctgtcctggc ctccctcatc attgctacta acacactagt ggctgtggct     120
gtgctgctgt tgatccacaa gaatgatggt gtcagtctct gcttcacctt gaatctggct     180
gtggctgaca ccttgattgg tgtggccatc tctggcctac tcacagacca gctctccagc     240
ccttctcggc ccacacagaa gaccctgtgc agcctgcgga tggcatttgt cacttcctcc     300
gcagctgcct ctgtcctcac ggtcatgctg atcacctttg acaggtacct tgccatcaag     360
cagcccttcc gctacttgaa gatcatgagt gggttcgtgg ccggggcctg cattgccggg     420
ctgtggttag tgtcttacct cattggcttc ctcccactcg gaatccccat gttccagcag     480
actgcctaca aagggcagtg cagcttcttt gctgtatttc accctcactt cgtgctgacc     540
ctctcctgcg ttggcttctt cccagccatg ctcctctttg tcttcttcta ctgcgacatg     600
ctcaagattg cctccatgca cagccagcag attcgaaaga tggaacatgc aggagccatg     660
gctggaggtt atcgatcccc acggactccc agcgacttca aagctctccg tactgtgtct     720
gttctcattg ggagctttgc tctatcctgg acccccttcc ttatcactgg cattgtgcag     780
gtggcctgcc aggagtgtca cctctaccta gtgctggaac ggtacctgtg gctgctcggc     840
gtgggcaact ccctgctcaa cccactcatc tatgcctatt ggcagaagga ggtgcgactg     900
cagctctacc acatggccct aggagtgaag aaggtgctca cctcattcct cctctttctc     960
tcggccagga attgtggccc agagaggccc agggaaagtt cctgtcacat cgtcactatc    1020

tccagctcag agtttgatgg cgaattcgga tccaagggca attctgcaga tatccagcac    1080
agtggcggcc gctcgagtct agagggcccg cggttcgaag gtaagcctat ccctaaccct    1140
ctcctcggtc tcgattctac gcgtaccggt catcatcacc atcaccattg a             1191
```

<210> 2
<211> 396
<212> PRT
<213> Homo sapien

<400> 2

Met Tyr Pro Tyr Asp Val Pro Asp Tyr Ala Gly Ser Leu Glu Ser Ser
1 5 10 15

Phe Ser Phe Gly Val Ile Leu Ala Val Leu Ala Ser Leu Ile Ile Ala
20 25 30

Thr Asn Thr Leu Val Ala Val Ala Val Leu Leu Leu Ile His Lys Asn
35 40 45

Asp Gly Val Ser Leu Cys Phe Thr Leu Asn Leu Ala Val Ala Asp Thr
50 55 60

Leu Ile Gly Val Ala Ile Ser Gly Leu Leu Thr Asp Gln Leu Ser Ser
65 70 75 80

Pro Ser Arg Pro Thr Gln Lys Thr Leu Cys Ser Leu Arg Met Ala Phe
85 90 95

Val Thr Ser Ser Ala Ala Ala Ser Val Leu Thr Val Met Leu Ile Thr
100 105 110

Phe Asp Arg Tyr Leu Ala Ile Lys Gln Pro Phe Arg Tyr Leu Lys Ile
115 120 125

Met Ser Gly Phe Val Ala Gly Ala Cys Ile Ala Gly Leu Trp Leu Val
130 135 140

Ser Tyr Leu Ile Gly Phe Leu Pro Leu Gly Ile Pro Met Phe Gln Gln
145 150 155 160

Thr Ala Tyr Lys Gly Gln Cys Ser Phe Phe Ala Val Phe His Pro His
165 170 175

Phe Val Leu Thr Leu Ser Cys Val Gly Phe Phe Pro Ala Met Leu Leu
180 185 190

Phe Val Phe Phe Tyr Cys Asp Met Leu Lys Ile Ala Ser Met His Ser

```
        195                 200                 205

Gln Gln Ile Arg Lys Met Glu His Ala Gly Ala Met Ala Gly Gly Tyr
    210                 215                 220

Arg Ser Pro Arg Thr Pro Ser Asp Phe Lys Ala Leu Arg Thr Val Ser
225                 230                 235                 240

Val Leu Ile Gly Ser Phe Ala Leu Ser Trp Thr Pro Phe Leu Ile Thr
                245                 250                 255

Gly Ile Val Gln Val Ala Cys Gln Glu Cys His Leu Tyr Leu Val Leu
            260                 265                 270

Glu Arg Tyr Leu Trp Leu Leu Gly Val Gly Asn Ser Leu Leu Asn Pro
        275                 280                 285

Leu Ile Tyr Ala Tyr Trp Gln Lys Glu Val Arg Leu Gln Leu Tyr His
    290                 295                 300

Met Ala Leu Gly Val Lys Lys Val Leu Thr Ser Phe Leu Leu Phe Leu
305                 310                 315                 320

Ser Ala Arg Asn Cys Gly Pro Glu Arg Pro Arg Glu Ser Ser Cys His
                325                 330                 335

Ile Val Thr Ile Ser Ser Ser Glu Phe Asp Gly Glu Phe Gly Ser Lys
            340                 345                 350

Gly Asn Ser Ala Asp Ile Gln His Ser Gly Gly Arg Ser Ser Leu Glu
        355                 360                 365

Gly Pro Arg Phe Glu Gly Lys Pro Ile Pro Asn Pro Leu Leu Gly Leu
    370                 375                 380

Asp Ser Thr Arg Thr Gly His His His His His His
385                 390                 395
```

<210> 3
<211> 24
<212> DNA
<213> Artificial

<220>
<223> Homo sapien Primer

<400> 3
cattgccggg ctgtggttag tgtc 24

<210> 4
<211> 24

<212> DNA
<213> Artificial

<220>
<223> Homo sapien Primer

<400> 4
ggcatagatg agtgggttga gcag 24

<210> 5
<211> 22
<212> DNA
<213> Artificial

<220>
<223> Rat Primer

<400> 5
catgggccct gcaccttctt tg 22

<210> 6
<211> 24
<212> DNA
<213> Artificial

<220>
<223> Rat Primer

<400> 6
gctccggatg gctgatgata gtga 24

<210> 7
<211> 23
<212> PRT
<213> Artificial

<220>
<223> Novel Sequence

<400> 7

```
Arg Gly Pro Glu Arg Thr Arg Glu Ser Ala Tyr His Ile Val Thr Ile
1               5                   10                  15

Ser His Pro Glu Leu Asp Gly
            20
```

## Description

### FIELD OF THE INVENTION

The present invention relates to certain substituted aryl and heteroaryl derivatives that are modulators of glucose metabolism. Accordingly, compounds of the present invention are useful in the treatment of metabolic-related disorders and complications thereof, such as, diabetes and obesity.

### BACKGROUND OF THE INVENTION

Diabetes mellitus is a serious disease afflicting over 100 million people worldwide. In the United States, there are more than 12 million diabetics, with 600,000 new cases diagnosed each year.

Diabetes mellitus is a diagnostic term for a group of disorders characterized by abnormal glucose homeostasis resulting in elevated blood sugar. There are many types of diabetes, but the two most common are Type I (also referred to as insulin-dependent diabetes mellitus or IDDM) and Type II (also referred to as non-insulin-dependent diabetes mellitus or NIDDM).

The etiology of the different types of diabetes is not the same; however, everyone with diabetes has two things in common: overproduction of glucose by the liver and little or no ability to move glucose out of the blood into the cells where it becomes the body's primary fuel.

People who do not have diabetes rely on insulin, a hormone made in the pancreas, to move glucose from the blood into the cells of the body. However, people who have diabetes either don't produce insulin or can't efficiently use the insulin they produce; therefore, they can't move glucose into their cells. Glucose accumulates in the blood creating a condition called hyperglycemia, and over time, can cause serious health problems.

Diabetes is a syndrome with interrelated metabolic, vascular, and neuropathic components. The metabolic syndrome, generally characterized by hyperglycemia, comprises alterations in carbohydrate, fat and protein metabolism caused by absent or markedly reduced insulin secretion and/or ineffective insulin action. The vascular syndrome consists of abnormalities in the blood vessels leading to cardiovascular, retinal and renal complications. Abnormalities in the peripheral and autonomic nervous systems are also part of the diabetic syndrome.

People with IDDM, which accounts for about 5% to 10% of those who have diabetes, don't produce insulin and therefore must inject insulin to keep their blood glucose levels normal. IDDM is characterized by low or undetectable levels of endogenous insulin production caused by destruction of the insulin-producing β cells of the pancreas, the characteristic that most readily distinguishes IDDM from NIDDM. IDDM, once termed juvenile-onset diabetes, strikes young and older adults alike.

Approximately 90 to 95% of people with diabetes have Type II (or NIDDM). NIDDM subjects produce insulin, but the cells in their bodies are insulin resistant: the cells don't respond properly to the hormone, so glucose accumulates in their blood. NIDDM is characterized by a relative disparity between endogenous insulin production and insulin requirements, leading to elevated blood glucose levels. In contrast to IDDM, there is always some endogenous insulin production in NIDDM; many NIDDM patients have normal or even elevated blood insulin levels, while other NIDDM patients have inadequate insulin production (Rotwein, R. et al. N. Engl. J. Med. 308, 65-71 (1983)). Most people diagnosed with NIDDM are age 30 or older, and half of all new cases are age 55 and older. Compared with whites and Asians, NIDDM is more common among Native Americans, African-Americans, Latinos, and Hispanics. In addition, the onset can be insidious or even clinically inapparent, making diagnosis difficult.

The primary pathogenic lesion on NIDDM has remained elusive. Many have suggested that primary insulin resistance of the peripheral tissues is the initial event. Genetic epidemiological studies have supported this view. Similarly, insulin secretion abnormalities have been argued as the primary defect in NIDDM. It is likely that both phenomena are important contributors to the disease process (Rimoin, D. L., et. al. Emery and Rimoin's Principles and Practice of Medical Genetics 3rd Ed. 1:1401-1402 (1996)).

Many people with NIDDM have sedentery lifestyles and are obese; they weigh approximately 20% more than the recommended weight for their height and build. Furthermore, obesity is characterized by hyperinsulinemia and insulin resistance, a feature shared with NIDDM, hypertension and atherosclerosis.

Obesity and diabetes are among the most common human health problems in industrialized societies. In industrialized countries a third of the population is at least 20% overweight. In the United States, the percentage of obese people has increased from 25% at the end of the 1970s, to 33% at the beginning the 1990s. Obesity is one of the most important risk factors for NIDDM. Definitions of obesity differ, but in general, a subject weighing at least 20% more than the recommended weight for his/her height and build is considered obese. The risk of developing NIDDM is tripled in subjects 30% overweight, and three-quarters with NIDDM are overweight.

Obesity, which is the result of an imbalance between caloric intake and energy expenditure, is highly correlated with insulin resistance and diabetes in experimental animals and human. However, the molecular mechanisms that are

involved in obesity-diabetes syndromes are not clear. During early development of obesity, increase insulin secretion balances insulin resistance and protects patients from hyperglycemia (Le Stunff, et al. Diabetes 43, 696-702 (1989)). However, after several decades, β cell function deteriorates and non-insulin-dependent diabetes develops in about 20% of the obese population (Pederson, P. Diab. Metab. Rev. 5, 505-509 (1989)) and (Brancati, F. L., et al., Arch. Intern. Med. 159, 957-963 (1999)). Given its high prevalence in modem societies, obesity has thus become the leading risk factor for NIDDM (Hill, J. O., et al., Science 280,1371-1374 (1998)). However, the factors which predispose a fraction of patients to alteration of insulin secretion in response to fat accumulation remain unknown.

Whether someone is classified as overweight or obese is generally determined on the basis of their body mass index (BMI) which is calculated by dividing body weight (kg) by height squared ($m^2$). Thus, the units of BMI are $kg/m^2$ and it is possible to calculate the BMI range associated with minimum mortality in each decade of life. Overweight is defined as a BMI in the range 25-30 $kg/m^2$, and obesity as a BMI greater than 30 $kg/m^2$ (see TABLE below). There are problems with this definition in that it does not take into account the proportion of body mass that is muscle in relation to fat (adipose tissue). To account for this, obesity can also be defined on the basis of body fat content: greater than 25% and 30% in males and females, respectively.

**CLASSIFICATION OF WEIGHT BY BODY MASS INDEX (BMI)**

| BMI | CLASSIFICATION |
|---|---|
| < 18.5 | Underweight |
| 18.5-24.9 | Normal |
| 25.0-29.9 | Overweight |
| 30.0-34.9 | Obesity (Class I) |
| 35.0-39.9 | Obesity (Class II) |
| >40 | Extreme Obesity (Class III) |

As the BMI increases there is an increased risk of death from a variety of causes that is independent of other risk factors. The most common diseases with obesity are cardiovascular disease (particularly hypertension), diabetes (obesity aggravates the development of diabetes), gall bladder disease (particularly cancer) and diseases of reproduction. Research has shown that even a modest reduction in body weight can correspond to a significant reduction in the risk of developing coronary heart disease.

Compounds marketed as anti-obesity agents include Orlistat (XENICAL™) and Sibutramine. Orlistat (a lipase inhibitor) inhibits fat absorption directly and tends to produce a high incidence of unpleasant (though relatively harmless) side-effects such as diarrhea. Sibutramine (a mixed 5-HT/noradrenaline reuptake inhibitor) can increase blood pressure and heart rate in some patients. The serotonin releaser/reuptake inhibitors fenfluramine (Pondimin™) and dexfenfluramine (Redux™) have been reported to decrease food intake and body weight over a prolonged period (greater than 6 months). However, both products were withdrawn after reports of preliminary evidence of heart valve abnormalities associated with their use. Accordingly, there is a need for the development of a safer anti-obesity agent.

Obesity considerably increases the risk of developing cardiovascular diseases as well. Coronary insufficiency, atheromatous disease, and cardiac insufficiency are at the forefront of the cardiovascular complication induced by obesity. It is estimated that if the entire population had an ideal weight, the risk of coronary insufficiency would decrease by 25% and the risk of cardiac insufficiency and of cerebral vascular accidents by 35%. The incidence of coronary diseases is doubled in subjects less than 50 years of age who are 30% overweight. The diabetes patient faces a 30% reduced lifespan. After age 45, people with diabetes are about three times more likely than people without diabetes to have significant heart disease and up to five times more likely to have a stroke. These findings emphasize the inter-relations between risks factors for NIDDM and coronary heart disease and the potential value of an integrated approach to the prevention of these conditions based on the prevention of these conditions based on the prevention of obesity (Perry, I. J., et al., BMJ 310, 560-564 (1995)).

Diabetes has also been implicated in the development of kidney disease, eye diseases and nervous-system problems. Kidney disease, also called nephropathy, occurs when the kidney's "filter mechanism" is damaged and protein leaks into urine in excessive amounts and eventually the kidney fails. Diabetes is also a leading cause of damage to the retina at the back of the eye and increases risk of cataracts and glaucoma. Finally, diabetes is associated with nerve damage, especially in the legs and feet, which interferes with the ability to sense pain and contributes to serious infections.

Taken together, diabetes complications are one of the nation's leading causes of death.

## SUMMARY OF THE INVENTION

The present invention is drawn to compounds which bind to and modulate the activity of a GPCR, referred to herein as **RUP3**, and uses thereof. The term **RUP3** as used herein includes the human sequences found in GeneBank accession number AY288416, naturally-occurring allelic variants, mammalian orthologs, and recombinant mutants thereof. A preferred human **RUP3** for use in screening and testing of the compounds of the invention is provided in the nucleotide sequence of SEQ ID NO:1 and the corresponding amino acid sequence in SEQ ID NO: 2.

One aspect of the present invention encompasses certain substituted aryl and heteroaryl derivatives as shown in Formula (**I**):

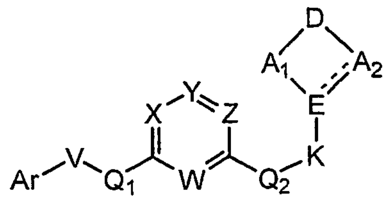

**(I)**

or pharmaceutically acceptable salt, solvate, hydrate or N-oxide thereof;
wherein:

$A_1$ and $A_2$ are both $-CH_2CH_2-$, and each $A_1$ and $A_2$ is optionally substituted with 1, 2, 3 or 4 methyl groups;
D is $CR_1R_2$ or $NR_2$, wherein $R_1$ is selected from the group consisting of H, $C_{1-6}$ alkyl, $C_{1-6}$ alkoxy, halogen and hydroxyl;
E is N, or $CR_3$, wherein $R_3$ is H or $C_{1-6}$ alkyl;
--- is a single bond;
K is absent, $C_{3-6}$ cycloalkylene, or $C_{1-3}$ alkylene group each optionally substituted with one or more substituents selected independently from the group consisting of $C_{1-6}$ alkyl, $C_{1-6}$ alkoxy, carboxy, cyano, and halogen;
$Q_1$ is $NR_4$, O, S, S(O), or $S(O)_2$, wherein $R_4$ is H, $C_{1-6}$ acyl, $C_{1-6}$ alkyl, $C_{2-6}$ alkenyl, $C_{2-6}$ alkynyl, $C_{3-7}$ cycloalkyl, or $C_{3-7}$-cycloalkyl-$C_{1-3}$-alkylene, wherein said $C_{1-6}$ alkyl is optionally substituted with one or more substituents selected independently from the group consisting of $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino and nitro;
$Q_2$ is absent, $NR_5$ or O, wherein $R_5$ is H, $C_{1-6}$ acyl, $C_{1-6}$ alkyl, $C_{2-6}$ alkenyl, $C_{2-6}$ alkynyl, $C_{3-7}$ cycloalkyl, or $C_{3-7}$-cycloalkyl-$C_{1-3}$-alkylene, wherein said $C_{1-6}$ alkyl is optionally substituted with one or more substituents selected independently from the group consisting of $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$
alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino and nitro;
W is CH;
X is N or $CR_6$;
Y is N or $CR_7$;
Z is N;
V is absent, $C_{1-3}$ heteroalkylene, or $C_{1-3}$ alkylene wherein each are optionally substituted with one or more substituents selected independently from the group consisting of $C_{1-3}$ alkyl, $C_{1-6}$ alkoxy, carboxy, cyano, $C_{1-3}$ haloalkyl, and halogen;
$R_6$, $R_7$, and $R_8$ are each independently selected from the group consisting of H, $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-

$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino and nitro, wherein said $C_{2-6}$ alkenyl, $C_{1-6}$ alkyl, $C_{2-6}$ alkynyl and $C_{3-6}$ cycloalkyl are each optionally substituted with one or more substituents independently selected from the group consisting of $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ akylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino and nitro;

Ar is phenyl, naphthyl, pyridyl, benzofuranyl, pyrazinyl, pyridazinyl, pyrimidinyl, triazinyl, quinoline, benzoxazole, benzothiazole, 1*H*-benzimidazole, isoquinoline, quinazoline, quinoxaline, pyrrole, or indole,optionally substituted with $R_9$, $R_{10}$, $R_{11}$, $R_{12}$, and $R_{13}$;

$R_9$ is selected from the group consisting of $C_{1-6}$ acyl, $C_{1-6}$ acylsulfonamide, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, aryl, arylcarbonyl, arylsulfonyl, di-$C_{1-6}$-alkylamino, carbamimidoyl, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, guanidine, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, heterocyclic, heterocyclicsulfonyl, heteroaryl, hydroxyl, hydroxylamino, nitro, $C_{3-6}$ oxo-cycloalkyl, phenoxy, sulfonamide, sulfonic acid and thiol; and wherein each $R_9$ is optionally substituted with one or more substituents selected independently from the group consisting of $C_{1-6}$ acyl, $C_{1-6}$ acylsulfonamide, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkyltbiocarboxamide, $C_{1-6}$ alkylthioureyl, $C_{1-6}$ alkylureyl, amino, aryl, arylcarbonyl, arylsulfonyl, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, heteroaryl, heteroarylcarbonyl, heteroarylsulfonyl, heterocyclic, hydroxyl, hydroxylamino, and nitro;

$R_{10}$, $R_{11}$, $R_{12}$, and $R_{13}$ are independently selected from the group consisting of $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino, nitro, and thiol; or two adjacent $R_{10}$, $R_{11}$, $R_{12}$, and $R_{13}$ groups together with the atoms to which they are bonded form a 5, 6 or 7 member cycloalkyl, 5, 6 or 7 member cycloalkenyl, or 5, 6 or 7 member heterocyclic group wherein the 5, 6 or 7 member group is optionally substituted with halogen or oxo; and

either:

$R_2$ is aryl, arylcarbonyl, $C_{1-6}$ alkoxycarbonyl, $C_{3-7}$-cycloalkoxycarbonyl, heteroaryl, or heteroarylcarbonyl; each optionally substituted with one or more substituents selected independently from $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, aryl, di-$C_{1-6}$-alkylamino, carbo-$C_{1-6}$-alkoxy, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, heterocyclic, heteroaryl, hydroxyl, hydroxylamino, nitro, and thiol;

or:

$R_2$ is $C_{1-6}$ alkoxycarbonyl, $C_{1-6}$ alkoxycarbonyl substituted by $C_{3-6}$ cycloalkyl, $C_{3-7}$-cycloalkoxycarbonyl, or heteroaryl-$C_{1-3}$-alkylene optionally substituted with 1,2, or 3 $C_{1-6}$ alkyl;

provided that the compound is not 4-[6-(2-fluoro-4-methanesulfonyl-phenoxy)-pyrimidin-4-yloxy]-piperidine-1-carboxylic acid isopropyl ester.

One aspect of the present invention relates to compounds of Formula (I) and pharmaceutically acceptable salts, solvates, hydrates and N-oxides thereof:

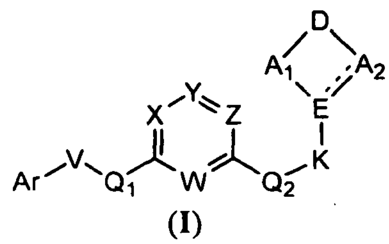

(I)

wherein:

$A_1$ and $A_2$ are independently $C_{1-3}$ alkylene optionally substituted with one or more substituents selected independently from $C_{1-6}$ alkyl, $C_{1-6}$ alkoxy, and carboxy;
D is $CR_1R_2$, wherein $R_1$ is selected from H, $C_{1-6}$ alkyl, $C_{1-6}$ alkoxy, halogen and hydroxyl;
E is N, C or $CR_3$, wherein $R_3$ is H or $C_{1-6}$ alkyl;
--- is a single bond when E is N or $CR_3$, or a double bond when E is C;
K is absent, $C_{3-6}$ cycloalkylene, or $C_{1-3}$ alkylene group each optionally substituted with one or more substituents selected independently from $C_{1-6}$ alkyl, $C_{1-6}$ alkoxy, carboxy, cyano, and halogen;
$Q_1$ is NH;
$Q_2$ is absent;
W is CH;
X is N or $CR_6$;
Y is N or $CR_7$;
Z is N;
V is absent, $C_{1-3}$ heteroalkylene, or $C_{1-3}$ alkylene wherein each are optionally substituted with one or more substituents selected independently from $C_{1-3}$ alkyl, $C_{1-6}$ alkoxy, carboxy, cyano, $C_{1-3}$ haloalkyl, and halogen;
$R_6$, $R_7$, and $R_8$ are each independently selected from H, $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino and nitro, wherein said $C_{2-6}$ alkenyl, $C_{1-6}$ alkyl, $C_{2-6}$ alkynyl and $C_{3-6}$ cycloalkyl are each optionally substituted with one or more substituents independently selected from $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino and nitro;
Ar is phenyl, naphthyl, pyridyl, benzofuranyl, pyrazinyl, pyridazinyl, pyrimidinyl, triazinyl, quinoline, benzoxazole, benzothiazole, 1*H*-benzimidazole, isoquinoline, quinazoline, quinoxaline, pyrrole, or indole, optionally substituted with $R_9$, $R_{10}$, $R_{11}$, $R_{12}$, and $R_{13}$;
$R_9$ is selected from $C_{1-6}$ acyl, $C_{1-6}$ acylsulfonamide, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, aryl, arylcarbonyl, arylsulfonyl, di-$C_{1-6}$-alkylamino, carbamimidoyl, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, guanidine, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, heterocyclic, heterocyclicsulfonyl, heteroaryl, hydroxyl, hydroxylamino, nitro, $C_{3-6}$ oxo-cycloalkyl, phenoxy, sulfonamide, sulfonic acid and thiol; and wherein each $R_9$ is optionally substituted with one or more substituents selected independently from $C_{1-6}$ acyl, $C_{1-6}$ acylsulfonamide, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, aryl, arylcarbonyl, arylsulfonyl, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, heteroaryl, heteroarylcarbonyl, heteroarylsulfonyl, heterocyclic, hydroxyl, hydroxylamino, and nitro;
$R_{10}$, $R_{11}$, $R_{12}$, and $R_{13}$ are independently selected from $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkox-

ycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino, nitro, and thiol; or two adjacent $R_{10}$, $R_{11}$, $R_{12}$, and $R_{13}$ groups together with the atoms to which they are bonded form a 5, 6 or 7 member cycloalkyl, 5, 6 or 7 member cycloalkenyl, or 5, 6 or 7 member heterocyclic group wherein the 5, 6 or 7 member group is optionally substituted with halogen or oxo; and $R_2$ is selected from $C_{1-6}$ alkyl, aryl, aryloxy, heteroaryl, and heteroaryloxy; wherein $R_2$ is optionally substituted with 1, 2, 3, 4, or 5 substituents selected independently from $C_{1-6}$ alkoxy, $C_{1-6}$ alkyl, $C_{1-6}$ alkylamino, amino, di-$C_{1-6}$-alkylamino, $C_{1-6}$ alkoxycarbonyl, carboxy, halogen, and heteroaryl, and wherein $C_{1-6}$ alkyl is further optionally substituted with 1, 2, or 3 substituents selected independently from $C_{1-6}$ alkylamino, di-$C_{1-6}$-alkylamino, $C_{3-6}$ cycloalkyl, and halogen.

One aspect of the present invention pertains to pharmaceutical compositions comprising at least one compound of the present invention and a pharmaceutically acceptable carrier.

Described herein are methods for the treatment of a metabolic-related disorder in an individual comprising administering to the individual in need of such treatment a therapeutically effective amount of a compound of the present invention or a pharmaceutical composition thereof.

Described herein are methods of decreasing food intake of an individual comprising administering to the individual in need thereof a therapeutically effective amount of a compound of the present invention or pharmaceutical composition thereof.

Described herein are methods of inducing satiety in an individual comprising administering to the individual in need thereof a therapeutically effective amount of a compound of the present invention or pharmaceutical composition thereof.

Described herein are methods of controlling or decreasing weight gain of an individual comprising administering to the individual in need thereof a therapeutically effective amount of a compound of the present invention or pharmaceutical composition thereof.

Described herein are methods of modulating a **RUP3** receptor in an individual comprising contacting the receptor with a compound of the present invention. In some embodiments, the compound is an agonist for the **RUP3** receptor. In some embodiments, the modulation of the **RUP3** receptor is the treatment of a metabolic-related disorder.

Described herein is a method of modulating a **RUP3** receptor in an individual comprising contacting the receptor with a compound of the present invention wherein the modulation of the **RUP3** receptor reduces food intake of the individual.

Described herein is a method of modulating a **RUP3** receptor in an individual comprising contacting the receptor with a compound of the present invention wherein the modulation of the **RUP3** receptor induces satiety in the individual.

Described herein is a method of modulating a **RUP3** receptor in an individual comprising contacting the receptor with a compound of the present invention wherein the modulation of the **RUP3** receptor controls or reduces weight gain of the individual.

One aspect of the present invention pertains to use of a compound of the present invention for production of a medicament for use in the treatment of a metabolic-related disorder.

One aspect of the present invention pertains to use of a compound of the present invention for production of a medicament for use in decreasing food intake in an individual.

One aspect of the present invention pertains to use of a compound of the present invention for production of a medicament for use of inducing satiety in an individual.

One aspect of the present invention pertains to use of a compound of the present invention for production of a medicament for use in controlling or decreasing weight gain in an individual.

One aspect of the present invention pertains to a compound of the present invention for use in a method of treatment of the human or animal body by therapy.

One aspect of the present invention pertains to a compound of the present invention for use in a method of treatment of a metabolic-related disorder of the human or animal body by therapy.

Described herein are methods wherein the human has a body mass index of about 18.5 to about 45. In some embodiments, the human has a body mass index of about 25 to about 45. In some embodiments, the human has a body mass index of about 30 to about 45. In some embodiments, the human has a body mass index of about 35 to about 45.

In some embodiments the individual is a mammal. In some embodiments the mammal is a human.

In some embodiments, the metabolic-related disorder is hyperlipidemia, type 1 diabetes, type 2 diabetes mellitus, idiopathic type 1 diabetes (Type 1b), latent autoimmune diabetes in adults (LADA), early-onset type 2 diabetes (EOD), youth-onset atypical diabetes (YOAD), maturity onset diabetes of the young (MODY), malnutrition-related diabetes, gestational diabetes, coronary heart disease, ischemic stroke, restenosis after angioplasty, peripheral vascular disease, intermittent claudication, myocardial infarction (e.g. necrosis and apoptosis), dyslipidemia, post-prandial lipemia, conditions of impaired glucose tolerance (IGT), conditions of impaired fasting plasma glucose, metabolic acidosis,

ketosis, arthritis, obesity, osteoporosis, hypertension, congestive heart failure, left ventricular hypertrophy, peripheral arterial disease, diabetic retinopathy, macular degeneration, cataract, diabetic nephropathy, glomerulosclerosis, chronic renal failure, diabetic neuropathy, metabolic syndrome, syndrome X, premenstrual syndrome, coronary heart disease, angina pectoris, thrombosis, atherosclerosis, myocardial infarction, transient ischemic attacks, stroke, vascular restenosis, hyperglycemia, hyperinsulinemia, hyperlipidemia, hypertrygliceridemia, insulin resistance, impaired glucose metabolism, conditions of impaired glucose tolerance, conditions of impaired fasting plasma glucose, obesity, erectile dysfunction, skin and connective tissue disorders, foot ulcerations and ulcerative colitis, endothelial dysfunction and impaired vascular compliance.

In some embodiments, the metabolic-related disorder is type I diabetes, type II diabetes, inadequate glucose tolerance, insulin resistance, hyperglycemia, hyperlipidemia, hypertriglyceridemia, hypercholesterolemia, dyslipidemia or syndrome X. In some embodiments, the metabolic-related disorder is type II diabetes. In some embodiments, the metabolic-related disorder is hyperglycemia. In some embodiments, the metabolic-related disorder is hyperlipidemia. In some embodiments, the metabolic-related disorder is hypertriglyceridemia. In some embodiments, the metabolic-related disorder is type I diabetes. In some embodiments, the metabolic-related disorder is dyslipidemia. In some embodiments, the metabolic-related disorder is syndrome X.

One aspect of the present invention pertains to a method of producing a pharmaceutical composition comprising admixing at least one compound, as described herein, and a pharmaceutically acceptable carrier.

The inventions described in this application were made by Arena Pharmaceuticals, Inc as a result of activities undertaken within the scope of a December 20, 2004 joint research agreement between Ortho-McNeil Pharmaceutical, Inc. and Arena Pharmaceuticals, Inc.

Applicant reserves the right to exclude any one or more of the compounds from any of the embodiments of the invention. Applicant additionally reserves the right to exclude any disease, condition or disorder from any of the embodiments of the invention.

## BRIEF DESCRIPTION OF THE DRAWINGS

**Figure 1A** shows RT-PCR analysis of **RUP3** expression in human tissues. A total of twenty-two (22) human tissues were analyzed.
**Figure 1B** shows the cDNA Dot-Blot analysis of RUP 3 expression in human tissues.
**Figure 1C** shows analysis of **RUP3** by RT-PCR with isolated human pancreatic islets of Langerhans.
**Figure 1D** shows analysis of **RUP3** expression with cDNAs of rat origin by RT-PCR.
**Figure 2A** shows a polyclonal anti-**RUP3** antibody prepared in Rabbits.
**Figure 2B** shows the expression of **RUP3** in insulin-producing βcells of pancreatic islets.
**Figure 3** shows *in vitro* functional activities of **RUP3**.
**Figure 4** shows a **RUP3** RNA blot.

## DETAILED DESCRIPTION OF THE INVENTION

## DEFINITIONS

The scientific literature that has evolved around receptors has adopted a number of terms to refer to ligands having various effects on receptors. For clarity and consistency, the following definitions will be used throughout this patent document.

**AGONISTS** shall mean moieties that interact and activate the receptor, such as the **RUP3** receptor and initiates a physiological or pharmacological response characteristic of that receptor. For example, when moieties activate the intracellular response upon binding to the receptor, or enhance GTP binding to membranes.

**AMINO ACID ABBREVIATIONS** used herein are set out in TABLE 1:

| TABLE 1 | | |
|---|---|---|
| ALANINE | ALA | A |
| ARGININE | ARG | R |
| ASPARAGINE | ASN | N |
| ASPARTIC ACID | ASP | D |

(continued)

| TABLE 1 | | |
|---|---|---|
| CYSTEINE | CYS | C |
| GLUTAMIC ACID | GLU | E |
| GLUTAMINE | GLN | Q |
| GLYCINE | GLY | G |
| HISTIDINE | HIS | H |
| ISOLEUCINE | ILE | I |
| LEUCINE | LEU | L |
| LYSINE | LYS | K |
| METHIONINE | MET | M |
| PHENYLALANINE | PHE | F |
| PROLINE | PRO | P |
| SERINE | SER | S |
| THREONINE | THR | T |
| TRYPTOPHAN | TRP | W |
| TYROSINE | TYR | Y |
| VALINE | VAL | V |

The term **ANTAGONISTS** is intended to mean moieties that competitively bind to the receptor at the same site as agonists (for example, the endogenous ligand), but which do not activate the intracellular response initiated by the active form of the receptor, and can thereby inhibit the intracellular responses by agonists or partial agonists. Antagonists do not diminish the baseline intracellular response in the absence of an agonist or partial agonist.

**CHEMICAL GROUP, MOIETY OR RADICAL:**

The term "**$C_{1-6}$ acy**l" refers to a $C_{1-6}$ alkyl radical attached directly to the carbon of a carbonyl group wherein the definition for alkyl is as described herein; some examples include, but not limited to, acetyl, propionyl, *n*-butanoyl, *iso*-butanoyl, *sec*-butanoyl, *t*-butanoyl (also referred to as pivaloyl), pentanoyl and the like.

The term "**$C_{1-6}$ acyloxcy**" refers to an acyl radical attached directly to an oxygen atom [-OC(=O)-$C_{1-6}$ alkyl] wherein acyl has the same definition has described herein; some examples include but not limited to acetyloxy [-OC (=O)$CH_3$], propionyloxy, butanoyloxy, *iso*-butanoyloxy, sec-butanoyloxy, t-butanoyloxy and the like.

The term "**$C_{1-6}$ acylsulfonamide**" refers to a $C_{1-6}$ acyl attached directly to the nitrogen of the sulfonamide, wherein the definitions for $C_{1-6}$ acyl and sulfonamide have the same meaning as described herein, and a $C_{1-6}$ acylsulfonamide can be represented by the following formula:

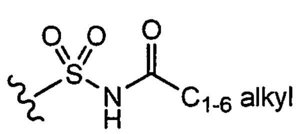

Some embodiments of the present invention are when acylsulfonamide is a $C_{1-5}$ acylsulfonamide, some embodiments are $C_{1-4}$ acylsulfonamide, some embodiments are $C_{1-3}$ acylsulfonamide, and some embodiments are $C_{1-2}$ acylsulfonamide. Examples of an acylsulfonamide include, but not limited to, acetylsulfamoyl [-S(=O)$_2$NHC(=O)Me], propionylsulfamoyl [-S(=O)$_2$NHC(=O)Et], isobutyrylsulfamoyl, butyrylsulfamoyl, 2-methyl-butyrylsulfamoyl, 3-methyl-butyrylsulfamoyl, 2,2-dimethyl-propionylsulfamoyl, pentanoylsulfamoyl, 2-methyl-pentanoylsulfamoyl, 3-methyl-pentanoylsulfamoyl, 4-methyl-pentanoylsulfamoyl, and the like.

The term "**$C_{2-6}$ alkenyl**" refers to a radical containing 2 to 6 carbons wherein at least one carbon-carbon double

bond is present, some embodiments are 2 to 4 carbons, some embodiments are 2 to 3 carbons, and some embodiments have 2 carbons. Both *E* and *Z* isomers are embraced by the term **"alkenyl."** Furthermore, the term "**alkenyl**" includes di-and tri-alkenyls. Accordingly, if more than one double bond is present then the bonds may be all *E* or *Z* or a mixtures of *E* and *Z*. Examples of an alkenyl include vinyl, allyl, 2-butenyl, 3-butenyl, 2-pentenyl, 3-pentenyl, 4-pentenyl, 2-hexenyl, 3-hexenyl, 4-hexenyl, 5-hexanyl, 2,4-hexadienyl and the like.

The term "**$C_{1-6}$ alkoxy**" refers to an alkyl radical, as defined herein, attached directly to an oxygen atom (i.e., -O-$C_{1-6}$ alkyl). Examples include methoxy, ethoxy, n-propoxy, isopropoxy, n-butoxy, t-butoxy, iso-butoxy, sec-butoxy and the like.

The term "**$C_{1-6}$ alkyl**" refers to a straight or branched carbon radical containing 1 to 6 carbons, some embodiments are 1 to 5 carbons, some embodiments are 1 to 4 carbons, some embodiments are 1 to 3 carbons, and some embodiments are 1 or 2 carbons. Examples of an alkyl include, but not limited to, methyl, ethyl, n-propyl, iso-propyl, n-butyl, iso-butyl, t-butyl, sec-butyl, n-pentyl, iso-pentyl, sec-pentyl, neo-pentyl, pent-3-yl, 2-methyl-but-1-yl, 1,2-dimethyl-prop-1-yl, n-hexyl, iso-hexyl, sec-hexyl, neo-hexyl, 1-ethyl-2-methyl-prop-1-yl, 1,2,2-trimethyl-prop-1-yl, 1,1,2-trimethyl-prop-1-yl, 1-ethyl-1-methyl-prop-1-yl, 1,1-dimethyl-but-1-yl, 1,2-dimethyl-but-1-yl, 2,3-dimethyl-but-1-yl, 2,2-dimethyl-but-1-yl, 1,3-dimethyl-but-1-yl, hex-3-yl, 2-methyl-pent-1-yl, 3-methyl-pent-1-yl, and the like.

The term "**$C_{1-6}$ alkylamino**" refers to one alkyl radical attached directly to an amino radical (-HN-$C_{1-6}$ alkyl) wherein the alkyl radical has the same meaning as described herein. Some examples include, but not limited to, methylamino (i.e., -$HNCH_3$), ethylamino, n-propylamino, iso-propylamino, n-butylamino, sec-butylamino, iso-butylamino, t-butylamino, and the like.

The term "**$C_{1-6}$ alkylcarboxamide**" or "**$C_{1-6}$ alkylcarboxamido**" refers to a single $C_{1-6}$ alkyl group attached to the nitrogen of an amide group, wherein alkyl has the same definition as described herein. The $C_{1-6}$ alkylcarboxamido may be represented by the following:

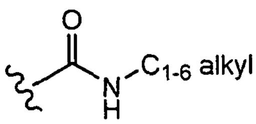

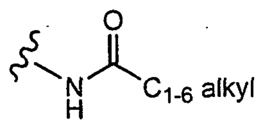

Examples include, but not limited to, *N*-methylcarboxamide, *N*-ethylcarboxamide, *N*-*n*-propylcarboxamide, *N*-*iso*-propylcarboxamide, *N*-*n*-butylcarboxamide, *N*-*sec*-butylcarboxamide, *N*-*iso*-butylcarboxamide, *N*-*t*-butylcarboxamide and the like.

The term "**$C_{1-3}$ alkylene**" refers to a $C_{1-3}$ divalent straight carbon group. Examples of a $C_{1-3}$ alkylene group include, -$CH_2$-, -$CH_2CH_2$-, and -$CH_2CH_2CH_2$-. Other examples include, =CH-, =$CHCH_2$-, =$CHCH_2CH_2$- wherein these examples relate generally to "$A_2$" when E is C (i.e., a carbon atom).

The term "**$C_{1-6}$ alkylsulfinyl**" refers to an alkyl radical attached directly to a sulfoxide radical of the formula: -S(=O)- wherein the alkyl radical has the same definition as described herein. Examples include, but not limited to, methylsulfinyl, ethylsulfinyl, n-propylsulfinyl, iso-propylsulfinyl, n-butylsulfinyl, sec-butylsulfinyl, iso-butylsulfinyl, t-butyl, and the like.

The term "**$C_{1-6}$ alkylsulfonamide**" refers to the groups

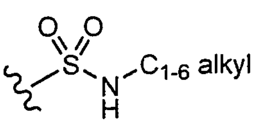

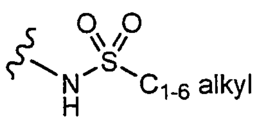

wherein $C_{1-6}$ alkyl has the same definition as described herein.

The term "**$C_{1-6}$ alkylsulfonyl"** refers to a alkyl radical attached to a sulfone radical of the formula: -$S(O)_2$- wherein the alkyl radical has the same definition as described herein. Examples include, but not limited to, methylsulfonyl, ethylsulfonyl, n-propylsulfonyl, isopropylsulfonyl, n-butylsulfonyl, sec-butylsulfonyl, iso-butylsulfonyl, t-butyl, and the like.

The term "**$C_{1-6}$ alkylthio**" refers to a alkyl radical attached to a sulfide of the formula: -S- wherein the alkyl radical has the same definition as described herein. Examples include, but not limited to, methylsulfanyl (i.e., $CH_3S$-), ethylsulfanyl, n-propylsulfanyl, isopropylsulfanyl, n-butylsulfanyl, sec-butylsulfanyl, iso-butylsulfanyl, t-butyl, and the like.

The term "$C_{1-6}$ **alkylthiocarboxamide**" refers to an alkyl attached directly to a thiocarboxamide group at either the nitrogen or at the carbon of the thiocarbonyl and has the following respective formulae:

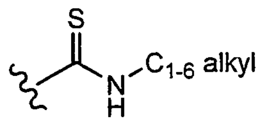

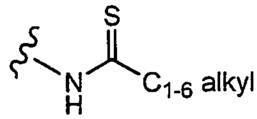

wherein $C_{1-4}$ alkyl has the same definition as described herein.

The term "**$C_{1-6}$ alkylthioureyl**" refers to the group of the formula: -NC(=S)NH- wherein one are both of the nitrogens are independently substituted with the same or different $C_{1-6}$ alkyl groups and alkyl has the same definition as described herein. Examples of an alkylthioureyl include, but not limited to, $CH_3NHC(S)NH-$, $NH_2C(S)NCH_3-$, $(CH_3)_2N(S)NH-$, $(CH_3)_2N(S)NH-$, $(CH_3)_2N(S)NCH_3-$, $CH_3CH_2NHC(S)NH-$, $CH_3CH_2NHC(S)NCH_3-$, and the like.

The term "**$C_{1-6}$ alkylureyl**" refers to the group of the formula: -NC(=O)NH- wherein one are both of the nitrogens are independently substituted with the same or different $C_{1-6}$ alkyl group wherein alkyl has the same definition as described herein. Examples of an alkylureyl include, but not limited to, $CH_3NHC(O)NH-$, $NH_2C(O)NCH_3-$, $(CH_3)_2N(O)NH-$, $(CH_3)_2N(O)NH-$, $(CH_3)_2N(O)NCH_3-$, $CH_3CH_2NHC(O)NH-$, $CH_3CH_2NHC(O)NCH_3-$, and the like.

The term "**$C_{2-6}$ alkynyl**" refers to a radical containing 2 to 6 carbons and at least one carbon-carbon triple bond (-C≡C-), some embodiments are 2 to 4 carbons, some embodiments are 2 to 3 carbons, and some embodiments have 2 carbons (-C≡CH). Examples of a $C_{2-6}$alkynyl include, but not limited to, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like. The term $C_{2-6}$ alkynyl includes di- and tri-ynes.

The term "**amino**" refers to the group $-NH_2$.

The term "**aryl**" refers to an aromatic ring radical containing 6 to 10 ring carbons. Examples include phenyl, naphthyl, and the like.

The term "**arylcarbonyl**" refers to an aryl group attached directly to the carbon of a carbonyl group, wherein aryl has the same definition as described herein. Examples include phenylcarbonyl (or also referred to as benzoyl), naphthalene-1-carbonyl, naphthalene-2-carbonyl, and the like.

The term "**aryloxy**" refers to an aryl group attached directly to an oxygen atom [i.e., aryl-O-] wherein aryl has the same definition as described herein. Examples include, but not limited to, phenoxy, naphthalen-1-yloxyl, naphthalen-2-yloxy, and the like.

The term "**arylsulfonyl**" refers to an aryl group attached directly to the sulfur of a sulfonyl group [i.e., $-S(=O)_2-$] wherein aryl has the same defmition as described herein. Examples include benzenesulfonyl, naphthalene-1-sulfonyl, naphthalene-2-sulfonyl, and the like.

The term "**$C_{1-6}$-alkoxycarbonyl**" refers to an alkoxy group attached directly to the carbon of a carbonyl and can be represented by the formula $-C(=O)O-C_{1-6}$-alkyl, wherein the $C_{1-6}$ alkyl group is as defined herein. In some embodiments, the $C_{1-6}$-alkoxycarbonyl group is further bonded to a nitrogen atom and together form a carbamate group (e.g., $NC(=O)O-C_{1-6}$-alkyl). Examples include, but not limited to, methoxycarbonyl, ethoxycarbonyl, propoxycarbonyl, *iso*-propoxycarbonyl, butoxycarbonyl, *sec*-butoxycarbonyl, *iso*butoxycarbonyl, *t*-butoxycarbonyl, *n*-pentoxycarbonyl, *iso*-pentoxycarbonyl, *t*-pentoxycarbonyl, *neo*-pentoxycarbonyl, *n*-hexyloxycarbonyl, and the like.

The term "**carbamimidoyl**" refers to a group of the following chemical formula:

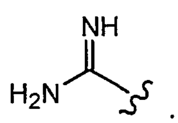

The term "**carboxamide**" refers to the group $-C(=O)NH_2$.

The term "**carboxy**" or "**carboxyl**" refers to the group $-CO_2H$; also referred to as a carboxylic acid group.

The term "**cyano**" refers to the group -CN.

The term "**$C_{3-7}$ cycloalkyl**" refers to a saturated ring radical containing 3 to 7 carbons; some embodiments contain 3 to 6 carbons ("**$C_{3-6}$ cycloalkyl**"); some embodiments contain 3 to 5 carbons ("**$C_{3-5}$ cycloalkyl**"); some embodiments contain 3 to 4 carbons ("$C_{3-4}$ **cycloalkyl**"). Examples include cyclopropyl, cyclobutyl, cyclopentyl, cyclopenyl, cyclohexyl, cycloheptyl and the like.

The term "**$C_{3-7}$-cycloalkyl-$C_{1-3}$-alkylene"** refers to a $C_{1-3}$ divalent straight chain carbon group bonded to a $C_{3-7}$-cycloalkyl group. Examples include, cyclopropylmethyl, cyclobutylmethyl, cyclopentylmethyl, cyclohexylmethyl, 2-cyclopropyl-ethyl, 2-cyclobutyl-ethyl, 2-cyclopentyl-ethyl, and the like.

The term "**$C_{3-7}$-cycloalkoxy**" refers to a cycloalkyl, as defined herein, attached directly to an oxygen atom (i.e., $-O-C_{3-7}$ cycloalkyl). Example include, but not limited to, cyclopropoxy, cyclobutoxy, cyclopentoxy, and the like.

The term "**$C_{3-7}$-cycloalkoxycarbonyl**" refers to a $C_{3-7}$ cycloalkoxy group attached directly to the carbon of a carbonyl group, and can be represented by the formula: $-C(=O)O-C_{3-7}$-cycloalkyl, wherein the cycloalkyl group has as described herein. In some embodiments, the $C_{3-7}$-cycloalkoxycarbonyl group is bonded to a nitrogen atom and together form a carbamate group (e.g., $NC(=O)O-C_{3-7}$-cycloalklyl). Examples include, but not limited to, cyclopropoxycarbonyl, cyclobutoxycarbonyl, cyclopentoxycarbonyl, and the like.

The term "**$C_{3-6}$ cycloalkylene**" refers to a divalent cycloalkyl radical, where cycloalkyl is as defmed herein, containing 3 to 6 carbons; some embodiments contain 3 to 5 carbons; some embodiments contain 3 to 4 carbons. In some embodiments, the two bonding groups are on the same carbon, for example:

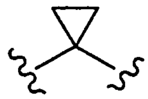 , 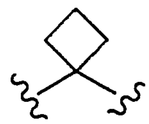 , 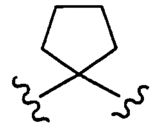 and 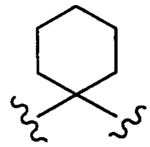

In some embodiments, the two bonding groups are on different carbons.

The term "**di-$C_{1-6}$-dialkylamino**" refers to an amino substituted with two of the same or different $C_{1-6}$ alkyl radicals wherein alkyl radical has the same definition as described herein. Some examples include, but not limited to, dimethylamino, methylethylamino, diethylamino, methylpropylamino, methylisopropylamino, ethylpropylamino, ethylisopropylamino, dipropylamino, propylisopropylamino and the like.

The term "**di**-$C_{1-6}$-**alkylcarboxamide**" or "**di-$C_{1-6}$-alkylcarboxamido**" refers to two $C_{1-6}$ alkyl radicals, that are the same or different, attached to an amide group, wherein alkyl has the same definition as described herein. A di-$C_{1-6}$-alkylcarboxamido can be represented by the following groups:

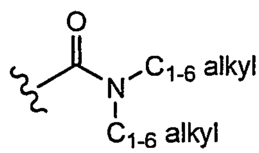 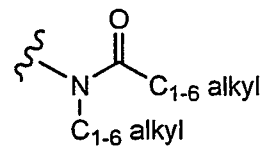

wherein $C_{1-6}$ has the same definition as described herein. Examples of a dialkylcarboxamide include, but not limited to, *N,N*- dimethylcarboxamide, *N*-methyl-*N*-ethylcarboxamide, *N,N*-diethylcarboxamide, *N*-methyl-*N*-isopropylcarboxamide, and the like.

The term "**di-$C_{1-6}$-alkylsulfonamide**" refers to two $C_{1-6}$ alkyl radicals, that are the same or different, attached to a sulfonamide group, wherein alkyl has the same definition as described herein. A di-$C_{1-6}$-alkylsulfonamide can be represented by the following groups:

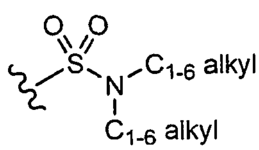 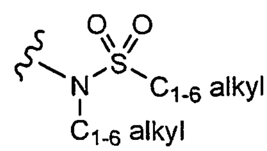

Examples include, but not limited to, dimethylsulfamoyl [$-S(=O)_2N(CH_3)_2$], ethylmethylsulfamoyl, methanesulfonyl-methyl-amino [$-N(CH_3)S(=O)_2CH_3$], ethyl-methanesulfonyl-amino [$-N(CH_2CH_3)S(=O)_2CH_3$], and the like.

The term "**di-$C_{1-6}$-alkylthiocarboxamido**" or "**di-$C_{1-6}$-alkylthiocarboxamide**" refers to two $C_{1-6}$ alkyl radicals, that are the same or different, attached to a thioamide group, wherein alkyl has the same definition as described herein. A $C_{1-6}$ dialkylthiocarboxamido can be represented by the following groups:

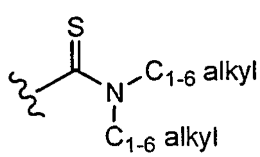 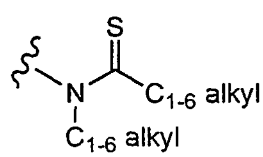 .

Examples of a dialkylthiocarboxamide include, but not limited to, *N,N*-dimethylthiocarboxamide, *N*-methyl-*N*-ethylthiocarboxamide and the like.

The term "**guanidine**" refers to a group of the following chemical formula:

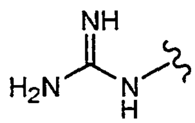 .

The term "$C_{1-6}$ **haloalkoxy**" refers to a haloalkyl, as defined herein, which is directly attached to an oxygen atom. Examples include, but not limited to, difluoromethoxy, trifluoromethoxy ($OCF_3$), 2,2,2-trifluoroethoxy, pentafluoroethoxy and the like.

The term "**$C_{1-6}$ haloalkyl**" refers to an alkyl group, defined herein, wherein the alkyl is substituted with one halogen up to fully substituted, a fully substituted haloalkyl can be represented by the formula $C_nL_{2n+1}$ wherein L is a halogen and "n" is 1, 2, 3, 4, 5, or 6 and when more than one halogen is present then they may be the same or different and selected from the group consisting of F, Cl, Br and I, in some embodiments, halogen is F. Examples of haloalkyl groups include, but not limited to, fluoromethyl, difluoromethyl, trifluoromethyl, chlorodifluoromethyl, 2,2,2-trifluoroethyl, pentafluoroethyl, and the like.

The term "**$C_{1-6}$ haloalkylsulfinyl**" refers to a $C_{1-6}$ haloalkyl radical directly attached to the sulfur of a sulfoxide group of the formula: -S(=O)-, wherein the $C_{1-6}$ haloalkyl radical has the same definition as described herein. Examples include, but not limited to, trifluoromethylsulfinyl, 2,2,2-trifluoroethylsulfinyl, 2,2-difluoroethylsulfinyl and the like.

The term "**$C_{1-6}$ haloalkylsulfonyl**" refers to a haloalkyl radical directly attached to the sulfur of a sulfone group of the formula: $-S(=O)_2-$ wherein haloalkyl has the same definition as described herein. Examples include, but not limited to, trifluoromethylsulfonyl, 2,2,2-trifluoroethylsulfonyl, 2,2-difluoroethylsulfonyl and the like.

The term "**$C_{1-6}$ haloalkylthio**" refers to a haloalkyl radical directly attached to a sulfur wherein the haloalkyl has the same meaning as described herein. Examples include, but not limited to, trifluoromethylthio (i.e., $CF_3S$-), 1,1-difluoroethylthio, 2,2,2-trifluoroethylthio and the like.

The term "**halogen**" or "**halo**" refers to to a fluoro, chloro, bromo or iodo group.

The term "**$C_{1-3}$ heteroalkylene**" refers to an alkylene bonded to a heteroatom selected from -O-, -S-, -S(=O)-, $-S(=O)_2-$, and -NH-. Some represented examples include, but not limited to, the groups of the following formulae:

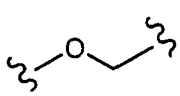 , 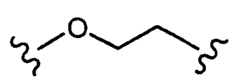 , 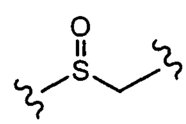 , 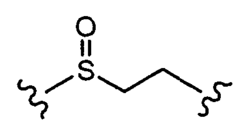 ,

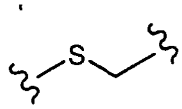 , 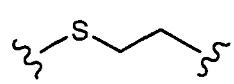 , 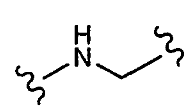 , 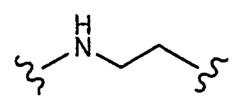 ,

and the like.

The term "**heteroaryl**" refers to an aromatic ring system that may be a single ring, two fused rings or three fused rings wherein at least one ring carbon is replaced with a heteroatom selected from, but not limited to, the group consisting of O, S, N, and NH. Examples of heteroaryl groups include, but not limited to, pyridyl, benzofuranyl, pyrazinyl, pyridazinyl, pyrimidinyl, triazinyl, quinoline, benzoxazole, benzothiazole, 1*H*-benzimidazole, isoquinoline, quinazoline, quinoxaline, pyrrole, indole, and the like. Other examples include, but not limited to, heteroaryl groups in TABLE 3, TABLE 4, and the like.

The term "**heteroaryl**-$C_{1-3}$-**alkylene**" refers to a heteroaryl that is directly attached to an alkylene group, wherein both heteroaryl and alkylene are the same as described herein. Examples of a heteroaryl-$C_{1-3}$-alkylene include, but not limited to, isoxazol-3-ylmethyl, isoxazol-4-ylmethyl, isoxazol-5-ylmethyl, 2-isoxazol-3-yl-ethyl, 2-isoxazol-4-yl-ethyl, 2-isoxazol-5,-yl-ethyl, [1,2,4]oxadiazol-3-ylinethyl, [1,2,4]oxadiazol-5-ylmethyl, 2-[1,2,4]oxadiazol-3-yl-ethyl, 2-[1,2,4]oxadiazol-5-yl-ethyl, and the like.

The term "**heteroarylcarbonyl**" refers to a heteroaryl group attached directly to the carbon of a carbonyl group, wherein heteroaryl has the same definition as described herein. Examples include [1,2,4]oxadiazole-3-carbonyl, [1,2,4] oxadiazole-5-carbonyl, isoxazole-3-carbonyl, isoxazole-4-carbonyl, isoxazole-5-carbonyl, furan-2-carbonyl, furan-3-carbonyl, furan-4-carbonyl, furan-5-carbonyl, thiophene-2-carbonyl, thiophene-3-carbonyl, pyridine-2-carbonyl, pyridine-3-carbonyl, pyridine-4-carbonyl, and the like.

The term "**heteroaryloxy**" refers to a heteroaryl group attached directly to an oxygen atom [i.e., heteroaryl-O-] wherein heteroaryl has the same definition as described herein. Examples include, but not limited to, [1,2,4]oxadiazole-3-yloxy, [1,2,4]oxadiazole-5-yloxy, isoxazole-3-yloxy, isoxazole-4-yloxy, isoxazole-5-yloxy, furan-2-yloxy, furan-3-yloxy, thiophen-2-yloxy, thiophen-3-yloxy, pyridin-2-yloxy, pyridin-3-yloxy, pyridin-4-yloxy, and the like.

The term "**heteroarylsulfonyl**" refers to an heteroaryl group attached directly to the sulfur of a sulfonyl group [i.e., $-S(=O)_2-$] wherein heteroaryl has the same definition as described herein. Examples include, but not limited to, [1,2,4]oxadiazole-3-sulfonyl, [1,2,4]oxadiazole-5-sulfonyl, isoxazole-3-sulfonyl, isoxazole-4-sulfonyl, isoxazole-5-sulfonyl, furan-2-sulfonyl, furan-3-sulfonyl, thiophene-2-sulfonyl, thiophene-3-sulfonyl, pyridine-2-sulfonyl, pyridine-3-sulfonyl, pyridine-4-sulfonyl, and the like.

The term "**heterocyclic**" refers to a non-aromatic carbon ring (i.e., cycloalkyl or cycloalkenyl as defined herein) wherein one, two or three ring carbons are replaced by a heteroatom selected from, but not limited to, the group consisting of -O-, -S-, -S(=O)-, $-S(=O)_2-$, and -NH-, and the ring carbon atoms are optionally substituted with oxo or thiooxo thus forming a carbonyl or thiocarbonyl group respectively. The heterocyclic group can be a 3, 4, 5, 6 or 7-member containing ring. Examples of a heterocyclic group include but not limited to aziridin-1-yl, aziridin-2-yl, azetidin-1-yl, azetidin-2-yl, azetidin-3-yl, piperidin-1-yl, piperidin-4-yl, morpholin-4-yl, piperzin-1-yl, piperzin-4-yl, pyrrolidin-1-yl, pyrrolidin-3-yl, [1,3]-dioxolan-2-yl and the like. Additional examples of heterocyclic groups are shown below in TABLE 2:

**TABLE 2**

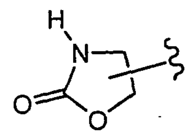, 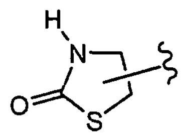, 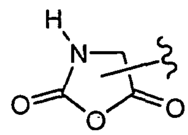, 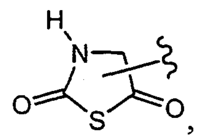,

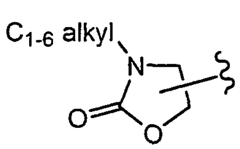, 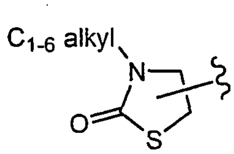, 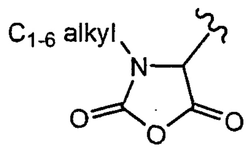, 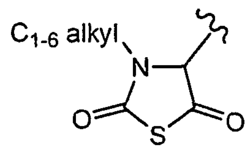,

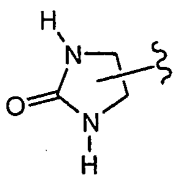, 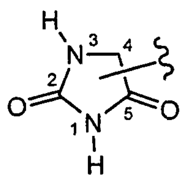, 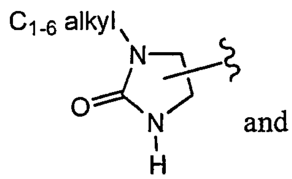 and 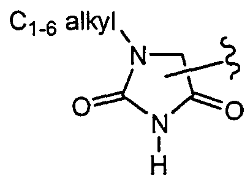.

It is understood that any one of the heterocyclic groups shown herein can be bonded at any ring carbon or ring nitrogen as allowed by the respective formula unless otherwise specified. For example, a 2,5-dioxo-imidazolidinyl group may be bonded at the ring carbon or at either of the two ring nitrogens to give the following formulae respectively:

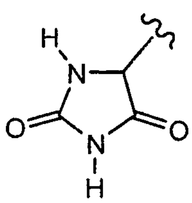, 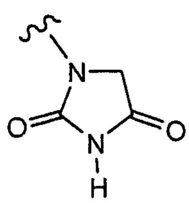, 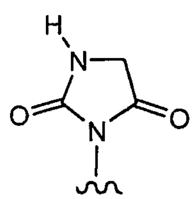.

The term "**heterocycliccarboxamide**" or "**heterocycliccarboxamido**" refers to a heterocyclic group, as defined herein, which has at least one nitrogen ring atom and the ring nitrogen is bonded directly to the carbon of a carbonyl group forming an amide. Examples include, but not limited to,

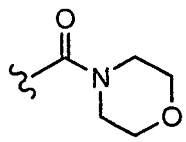, 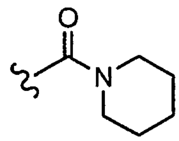, 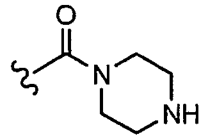, 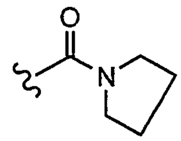

and the like.

The term "**heterocyclicsulfonyl**" refers to a heterocyclic group, as defined herein, which has at least one ring nitrogen and the ring nitrogen is bonded directly to the sulfur of a - $S(=O)_2$- group forming an sulfonamide group. Examples include, but not limited to,

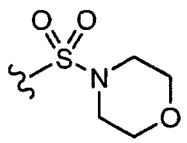, 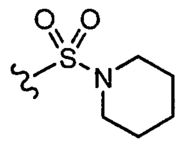, 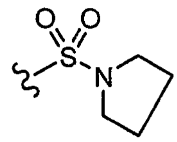

and the like.

The term "**hydroxyl**" refers to the group -OH.

The term "**hydroxylamino**" refers to the group -NHOH.

The term "**nitro**" refers to the group $-NO_2$.

The term "**oxo**" refers generally to a double bonded oxygen; typically "oxo" is a substitution on a carbon and together form a carbonyl group.

The term "**$C_{3-6}$ oxo-cycloalkyl**" refers to a $C_{3-6}$ cycloalkyl, as defined herein, wherein 1 or 2 of the ring carbons is substituted with an oxo group thus forming a carbonyl group. Examples of oxo-cycloalkyl groups include, but are not limited to, 2-oxo-cyclobutyl, 3-oxo-cyclobutyl, 3-oxo-cyclopentyl, 3-oxo-cyclohexyl, 2-oxo-cyclohexyl, 4-oxo-cyclohexyl, and the like and represented by the following structures respectively:

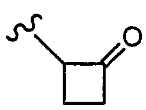, 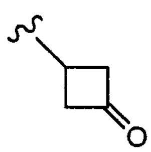, 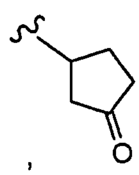, 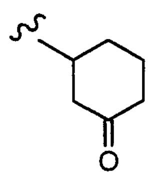, 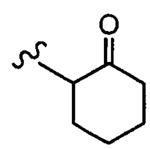 and 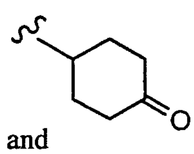.

The term "**phenoxy**" refers to the group $C_6H_5O$-.

The term "**phenyl**" refers to the group $C_6H_5$-.

The term "**sulfonamide**" refers to the group $-S(=O)_2NH_2$.

The term" **sulfonic acid**" refers to the group $-SO_3H$.

The term "**thiol**" refers to the group -SH.

**COMPOSITION** shall mean a material comprising at least two compounds or two components; for example, and without limitation, a Pharmaceutical Composition is a Composition comprising a compound of the present invention and a pharmaceutically acceptable carrier.

**COMPOUND EFFICACY** shall mean a measurement of the ability of a compound to inhibit or stimulate receptor functionality, as opposed to receptor binding affinity.

**CONTACT** or **CONTACTING** shall mean bringing the indicated moieties together, whether in an in vitro system or an in vivo system. Thus, "contacting" a **RUP3** receptor with a compound of the invention includes the administration of a compound of the present invention to an individual, for example a human, having a **RUP3** receptor, as well as, for example, introducing a compound of the invention into a sample containing a cellular or more purified preparation containing a **RUP3** receptor.

**IN NEED OF TREATMENT** as used herein refers to a judgment made by a caregiver (e.g. physician, nurse, nurse practitioner, etc. in the case of humans; veterinarian in the case of animals, including non-human mammals) that an individual or animal requires or will benefit from treatment. This judgment is made based on a variety of factors that are in the realm of a caregiver's expertise, but that includes the knowledge that the individual is ill, or will be ill, as the result of a disease, condition or disorder that is treatable by the compounds of the invention. The term "treatment" also

refers in the alternative to "prophylaxis." Therefore, in general, "in need of treatment" refers to the judgment of the caregiver that the individual is already ill, accordingly, the compounds of the present invention are used to alleviate, inhibit or ameliorate the disease, condition or disorder. Furthermore, the phrase also refers, in the alternative, to the judgment made by the caregiver that the individual will become ill. In this context, the compounds of the invention are used in a protective or preventive manner.

**INDIVIDUAL** as used herein refers to any animal, including mammals, preferably mice, rats, other rodents, rabbits, dogs, cats, swine, cattle, sheep, horses, or primates, and most preferably humans.

**INHIBIT** or **INHIBITING,** in relationship to the term "response" shall mean that a response is decreased or prevented in the presence of a compound as opposed to in the absence of the compound.

**INVERSE AGONISTS** shall mean moieties that bind the endogenous form of the receptor or to the constitutively activated form of the receptor, and which inhibit the baseline intracellular response initiated by the active form of the receptor below the normal base level of activity which is observed in the absence of agonists or partial agonists, or decrease GTP binding to membranes. Preferably, the baseline intracellular response is inhibited in the presence of the inverse agonist by at least 30%, more preferably by at least 50%, and most preferably by at least 75%, as compared with the baseline response in the absence of the inverse agonist.

**LIGAND** shall mean an endogenous, naturally occurring molecule specific for an endogenous, naturally occurring receptor.

As used herein, the terms **MODULATE** or **MODULATING** shall mean to refer to an increase or decrease in the amount, quality, response or effect of a particular activity, function or molecule.

**PHARMACEUTICAL COMPOSITION** shall mean a composition comprising at least one active ingredient, whereby the composition is amenable to investigation for a specified, efficacious outcome in a mammal (for example, without limitation, a human). Those of ordinary skill in the art will understand and appreciate the techniques appropriate for determining whether an active ingredient has a desired efficacious outcome based upon the needs of the artisan.

**THERAPEUTICALLY EFFECTIVE AMOUNT** as used herein refers to the amount of active compound or pharmaceutical agent that elicits the biological or medicinal response in a tissue, system, animal, individual or human that is being sought by a researcher, veterinarian, medical doctor or other clinician, which includes one or more of the following:

(1) Preventing the disease; for example, preventing a disease, condition or disorder in an individual that may be predisposed to the disease, condition or disorder but does not yet experience or display the pathology or symptomatology of the disease,

(2) Inhibiting the disease; for example, inhibiting a disease, condition or disorder in an individual that is experiencing or displaying the pathology or symptomatology of the disease, condition or disorder (i.e., arresting further development of the pathology and/or symptomatology), and

(3) Ameliorating the disease; for example, ameliorating a disease, condition or disorder in an individual that is experiencing or displaying the pathology or symptomatology of the disease, condition or disorder (i.e.,reversing the pathology and/or symptomatology).

**Compounds of the Present Invention:**

One aspect of the present invention encompasses certain substituted aryl and heteroaryl derivatives as shown in Formula (I):

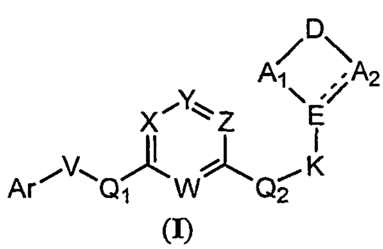

(I)

or pharmaceutically acceptable salt, solvate, hydrate or N-oxide thereof; wherein Ar, V, $Q_1$, $Q_2$, K, E, - - -, $A_1$, $A_2$, D', W, X, Y, and Z have the same definitions as described herein, *supra* and *infra*.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable

subcombination.

As used herein, "substituted" indicates that at least one hydrogen atom of the chemical group is replaced by a non-hydrogen substituent or group, the non-hydrogen substituent or group can be monovalent or divalent. When the substituent or group is divalent, then it is understood that this group is further substituted with another substituent or group. When a chemical group herein is "substituted" it may have up to the full valance of substitution; for example, a methyl group can be substituted by 1, 2, or 3 substituents, a methylene group can be substituted by 1 or 2 substituents, a phenyl group can be substituted by 1, 2, 3, 4, or 5 substituents, a naphthyl group can be substituted by 1, 2, 3, 4, 5, 6, or 7 substituents and the like. Likewise, "substituted with one or more substituents" refers to the substitution of a group with one substituent up to the total number of substituents physically allowed by the group. Further, when a group is substituted with more than one group they can be identical or they can be different.

It is understood herein that where a moiety is said to be substituted, that moiety is meant to be the one recited in the immediately preceding list of variables. For example, where a $C_{1-6}$ alkyl group is substituted by a substituent selected from another $C_{1-6}$ alkyl and other substitutents, subsequent language of "wherein $C_{1-6}$ alkyl is further optionally substituted" refers to the immediately preceding $C_{1-6}$ alkyl substitutent, as opposed to the original $C_{1-6}$ alkyl group; such that the original $C_{1-6}$ alkyl can be substituted by another $C_{1-6}$ alkyl which is itself further optionally substituted.

It is understood and appreciated that compounds of the invention may have one or more chiral centers, and therefore can exist as enantiomers and/or diastereomers. The invention is understood to extend to and embrace all such enantiomers, diastereomers and mixtures thereof, including, but not limited to, racemates. Accordingly, some embodiments of the present invention pertain to compounds of Formula (**I**) and formulae used throughout this disclosure that are *R* enantiomers. Further, some embodiments of the present invention pertain to compounds of Formula (**I**) and formulae used throughout this disclosure that are *S* enantiomers. When more than one chiral center is present, for example two chiral centers then, some embodiments of the present invention include compounds that are *RS* or *SR* enantiomers. In further embodiments, compounds of the present invention are *RR* or *SS* enantiomers. It is understood that compounds of Formula (**I**) and formulae used throughout this disclosure are intended to represent all individual enantiomers and mixtures thereof, unless stated or shown otherwise.

Compounds of the invention can also include tautomeric forms, such as keto-enol tautomers, and the like. Tautomeric forms can be in equilibrium or sterically locked into one form by appropriate substitution. It is understood that the various tautomeric forms are within the scope of the compounds of the present invention.

Compounds of the invention can also include all isotopes of atoms occurring in the intermediates and/or final compounds. Isotopes include those atoms having the same atomic number but different mass numbers. For example, isotopes of hydrogen include deuterium and tritium.

In some embodiments, heteroaryl refers to 5-member heteroaryl groups. In some embodiments, heteroaryl refers to heteroatom containing aromatic rings selected from the group consisting of the following formulae:

**TABLE 3**

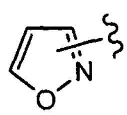 , 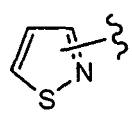 , 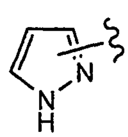 , 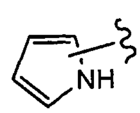 , 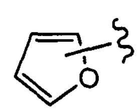 ,

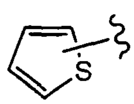 , 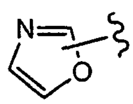 , 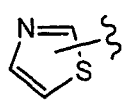 , 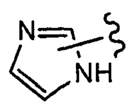 , 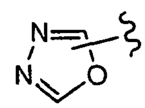 ,

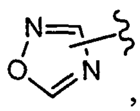 , 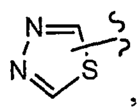 , 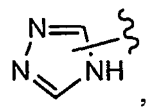 , 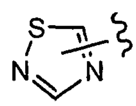 , 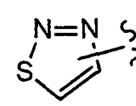 and 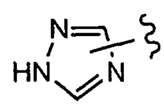

wherein the 5-member heteroaryl is bonded at any available position of the ring, for example, a imidazolyl ring can be bonded at one of the ring nitrogens (i.e., imidazol-1-yl group) or at one of the ring carbons (i.e., imidazol-2-yl, imidazol-4-yl or imiadazol-5-yl group).

In some embodiments, heteroaryl refers to 6-member heteroatom containing aromatic rings selected from the group consisting of the following formulae:

## TABLE 4

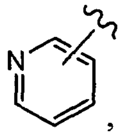, 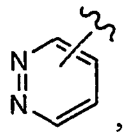, 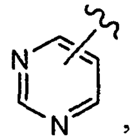, 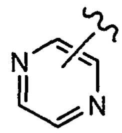, 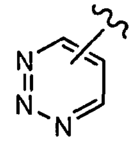,

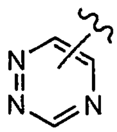, 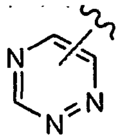, 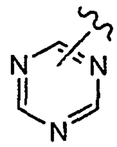 and 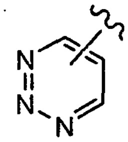

wherein the heteroaryl group is bonded at any ring carbon.

In some embodiments, $A_1$ and $A_2$ are both $-CH_2-$ and each optionally substituted with one or more substituents (i.e., 1 or 2 substituents for $A_1$ and 1 or 2 substituents for $A_2$) selected independently from the group consisting of $C_{1-6}$ alkyl, $C_{1-6}$ alkoxy, and carboxy.

Some embodiments of the present invention pertain to compounds wherein $A_1$ and $A_2$ are both $-CH_2-$, forming a four-member ring, and each $A_1$ and $A_2$ is optionally substituted with 1 or 2 methyl groups.

In some embodiments, $A_1$ and $A_2$ are both $-CH_2-$ and can be represented by Formula (**Ib**) as illustrated below:

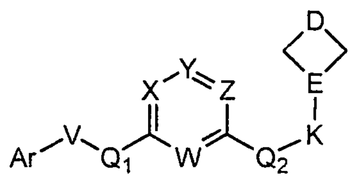

**(Ib)**

wherein each variable in Formula (**Ib**) has the same meaning as described herein, *supra* and *infra.*

In some embodiments, $A_1$ is $-CH_2-$ and $A_2$ is $-CH_2CH_2-$, and each optionally substituted with one or more substituents (i.e., 1 or 2 substituents for $A_1$ and 1, 2, 3, or 4 substituents for $A_2$) selected independently from the group consisting of $C_{1-6}$ alkyl, $C_{1-6}$ alkoxy, and carboxy.

Some embodiments of the present invention pertain to compounds wherein $A_1$ is $-CH_2-$ and $A_2$ is $-CH_2CH_2-$, forming a five-member ring, and $A_1$ is optionally substituted with 1 or 2 methyl groups and $A_2$ is optionally substituted with 1, 2, 3 or 4 methyl groups.

In some embodiments, $A_1$ is $-CH_2-$ and $A_2$ is $-CH_2CH_2-$ and can be represented by Formula (**Id**) as illustrated below:

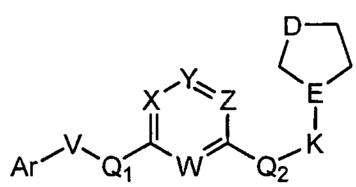

**(Id)**

wherein each variable in Formula (**Id**) has the same meaning as described herein, *supra* and *infra.*

In some embodiments, $A_1$ and $A_2$ are both $-CH_2CH_2-$, and each optionally substituted with one or more substituents (i.e., 1, 2, 3, or 4 substituents for $A_1$ and 1, 2, 3, or 4 substituents for $A_2$) selected independently from the group consisting of $C_{1-6}$ alkyl, $C_{1-6}$ alkoxy, and carboxy.

Some embodiments of the present invention pertain to compounds wherein $A_1$ and $A_2$ are both $-CH_2CH_2-$, forming a six-member ring, and each $A_1$ and $A_2$ is optionally substituted with 1, 2, 3 or 4 methyl groups.

In some embodiments, $A_1$ and $A_2$ are both $-CH_2CH_2-$ and can be represented by Formula (**If**) as illustrated below:

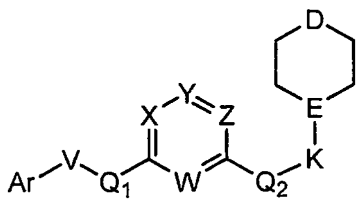

**(If)**

wherein each variable in Formula (**If**) has the same meaning as described herein, *supra* and *infra.*

In some embodiments, $A_1$ is $-CH_2CH_2-$ and $A_2$ is $-CH_2CH_2CH_2-$, and each optionally substituted with one or more substituents (i.e., 1, 2, 3, or 4 substituents for $A_1$ and 1, 2, 3, 4, 5, or 6 substituents for $A_2$) selected independently from the group consisting of $C_{1-6}$ alkyl, $C_{1-6}$alkoxy, and carboxy.

Some embodiments of the present invention pertain to compounds wherein $A_1$ is $-CH_2CH_2-$ and $A_2$ is $-CH_2CH_2CH_2-$, forming a seven-member ring, and each $A_1$ and $A_2$ is optionally substituted with 1, 2, 3 or 4 methyl groups.

In some embodiments, $A_1$ is $-CH_2CH_2-$ and $A_2$ is $-CH_2CH_2CH_2-$ and can be represented by Formula (**Ih**) as illustrated below:

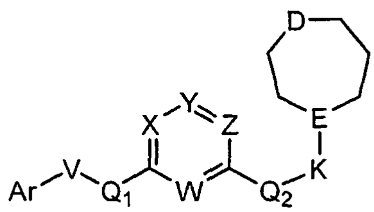

**(Ih)**

wherein each variable in Formula (**Ih**) has the same meaning as described herein, *supra* and *infra*.

In some embodiments, $A_1$ and $A_2$ are both $-CH_2CH_2CH_2-$, and each optionally substituted with one or more substituents (i.e., 1, 2, 3, 4, 5, or 6 substituents for $A_1$ and 1, 2, 3, 4, 5, or 6 substituents for $A_2$) selected independently from the group consisting of $C_{1-6}$ alkyl, $C_{1-6}$ alkoxy, and carboxy.

Some embodiments of the present invention pertain to compounds wherein $A_1$ and $A_2$ are both $-CH_2CH_2CH_2-$, forming an eight-member ring, and each $A_1$ and $A_2$ is optionally substituted with 1, 2, 3 or 4 methyl groups.

In some embodiments, $A_1$ and $A_2$ are both $-CH_2CH_2CH_2-$ and can be represented by Formula (**Ij**) as illustrated below:

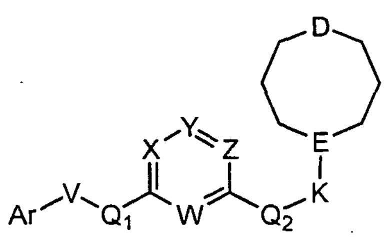

**(Ij)**

wherein each variable in Formula (**Ij**) has the same meaning as described herein, *supra* and *infra*.

Some embodiments of the present invention pertain to compounds wherein $A_1$ is $-CH_2-$ and $A_2$ is $=CHCH_2-$; or $A_1$ is $-CH_2CH_2-$ and $A_2$ is $=CH-$; and each $A_1$ and $A_2$ is optionally substituted with 1, 2, 3 or 4 methyl groups as permitted.

In some embodiments, $A_1$ is $-CH_2-$ and $A_2$ is $=CHCH_2-$; or $A_1$ is $-CH_2CH_2-$ and $A_2$ is $=CH-$, and can be represented by Formulae (**Ik**) and (**Im**), respectively, as illustrated below:

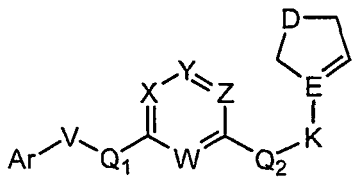
(Ik)

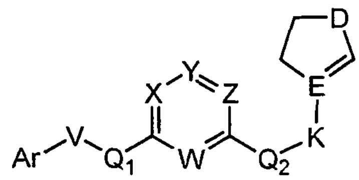
(Im)

wherein each variable in Formulae (**Ik**) and (**Im**) has the same meaning as described herein, *supra* and *infra.*

Some embodiments of the present invention pertain to compounds wherein $A_1$ is $-CH_2CH_2-$ and $A_2$ is $=CHCH_2-$, and $A_1$ is optionally substituted with 1, 2, 3 or 4 methyl groups and $A_2$ is optionally substituted with 1, 2 or 3 methyl groups.

In some embodiments, $A_1$ is $-CH_2CH_2-$ and $A_2$ is $=CHCH_2-$ and can be represented by Formula (**Io**) as illustrated below:

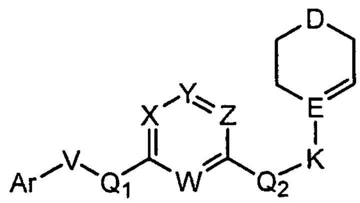
(Io)

wherein each variable in Formula (**Io**) has the same meaning as described herein, *supra* and *infra.*

Some embodiments of the present invention pertain to compounds wherein $A_1$ is $-CH_2CH_2-$ and $A_2$ is $=CHCH_2CH_2-$; or $A_1$ is $-CH_2CH_2CH_2-$ and $A_2$ is $=CHCH_2-$; and each $A_1$ and $A_2$ is optionally substituted with 1, 2, 3 or 4 methyl groups.

In some embodiments, $A_1$ is $-CH_2CH_2-$ and $A_2$ is $=CHCH_2CH_2-$; or $A_1$ is $-CH_2CH_2CH_2-$ and $A_2$ is $=CHCH_2-$; and can be represented by Formulae (**Iq**) and (**Is**), respectively, as illustrated below:

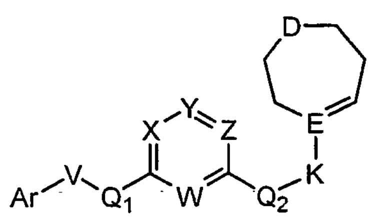
(Iq)

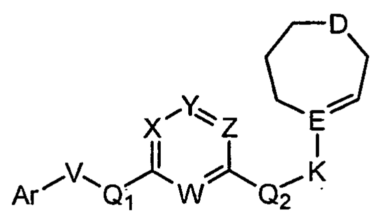
(Is)

wherein each variable in Formulae (**Iq**) and (**Is**) has the same meaning as described herein, *supra* and *infra.*

Some embodiments of the present invention pertain to compounds wherein $A_1$ is $-CH_2CH_2CH_2-$ and $A_2$ is $=CHCH_2CH_2-$, and each $A_1$ and $A_2$ is optionally substituted with 1, 2, 3 or 4 methyl groups.

In some embodiments, $A_1$ is $-CH_2CH_2CH_2-$ and $A_2$ is $=CHCH_2CH_2-$ and can be represented by Formula (**Iu**) as illustrated below:

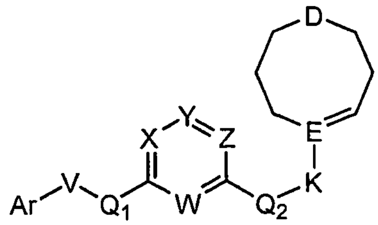

(Iu)

wherein each variable in Formula (**Iu**) has the same meaning as described herein, *supra* and *infra.*

Some embodiments of the present invention pertain to compounds wherein - - - is a single bond.

In some embodiments, compounds of the present invention can be represented by Formula (**Iw**) as illustrated below:

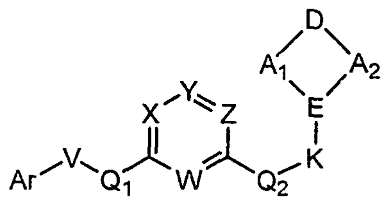

(Iw)

wherein each variable in Formula (**Iw**) has the same meaning as described herein, *supra* and *infra.*

Some embodiments of the present invention pertain to compounds wherein - - - is a double bond. It is understood that when - - - is a double bond then E is C (i.e., carbon atom) and E is not N (i.e., a nitrogen atom).

Some embodiments of the present invention pertain to compounds wherein K is absent.

In some embodiments, compounds of the present invention can be represented by Formula (**Iy**) as illustrated below:

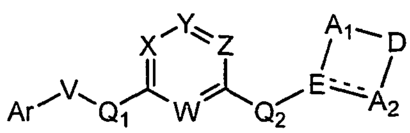

(Iy)

wherein each variable in Formula (**Iy**) has the same meaning as described herein, *supra* and *infra.*

In some embodiments, K is $C_{3-6}$ cycloalkylene optionally substituted with one or more substituents selected independently from the group consisting of $C_{1-6}$ alkyl, $C_{1-6}$ alkoxy, carboxy, cyano, and halogen.

Some embodiments of the present invention pertain to compounds wherein K is $C_{3-6}$ cycloalkylene.

In some embodiments, K is selected from the group consisting of cyclopropylene, cyclobutylene, cyclopentylene, and cyclohexylene.

In some embodiments, K is cyclobutylene.

In some embodiments, K is cyclopropylene.

In some embodiments, compounds of the present invention can be represented by Formula (**IIa**) as illustrated below:

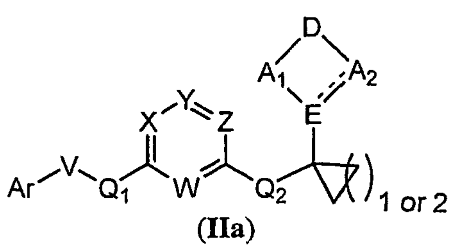

(IIa)

wherein each variable in Formula (**IIa**) has the same meaning as described herein, *supra* and *infra.*

Some embodiments of the present invention pertain to compounds wherein K is $C_{1-3}$ alkylene group each optionally substituted with one or more substituents selected independently from the group consisting of $C_{1-6}$ alkyl, $C_{1-6}$ alkoxy, carboxy, cyano, and halogen.

Some embodiments of the present invention pertain to compounds wherein K is $C_{1-3}$ alkylene optionally substituted with 1, 2, 3, or 4 substituents selected independently from the group consisting of $C_{1-6}$ alkyl, $C_{1-6}$ alkoxy, carboxy, cyano, and halogen.

Some embodiments of the present invention pertain to compounds wherein K is $-CH_2CH_2-$ optionally substituted with 1, 2, 3, or 4 substituents selected independently from the group consisting of $C_{1-6}$ alkyl, $C_{1-6}$ alkoxy, carboxy, cyano, and halogen.

Some embodiments of the present invention pertain to compounds wherein K is $-CH_2$-optionally substituted with 1 or 2 substituents selected independently from the group consisting of $C_{1-6}$ alkyl, $C_{1-6}$alkoxy, carboxy, cyano, and halogen.

In some embodiments, K is $-CH_2-$.

In some embodiments, compounds of the present invention can be represented by Formula (**IIc**) as illustrated below:

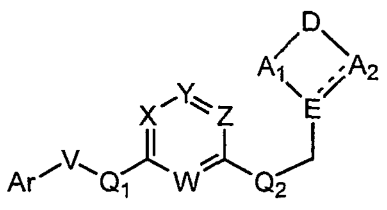

(IIc)

wherein each variable in Formula (**IIc**) has the same meaning as described herein, *supra* and *infra.*

Some embodiments of the present invention pertain to compounds wherein V is absent.

In some embodiments, compounds of the present invention can be represented by Formula (IIe) as illustrated below:

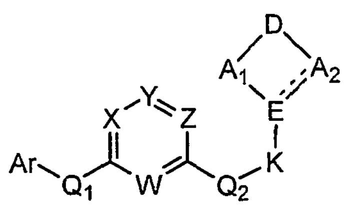

(IIe)

wherein each variable in Formula (**IIe**) has the same meaning as described herein, *supra* and *infra.*

In some embodiments, V is $C_{1-3}$ heteroalkylene optionally substituted with one or more substituents selected independently from the group consisting of $C_{1-3}$ alkyl, $C_{1-6}$ alkoxy, carboxy, cyano, $C_{1-3}$ haloalkyl, and halogen.

Some embodiments of the present invention pertain to compounds wherein V is $C_{1-3}$ heteroalkylene optionally substituted with 1, 2, 3, or 4 substituents selected independently from the group consisting of $C_{1-3}$ alkyl, $C_{1-6}$ alkoxy, carboxy, cyano, $C_{1-3}$ haloalkyl, and halogen.

Some embodiments of the present invention pertain to compounds wherein V is- $OCH_2CH_2$- optionally substituted with 1 or 2 substituents selected independently from the group consisting of $C_{1-3}$ alkyl, $C_{1-6}$ alkoxy, carboxy, cyano, $C_{1-3}$ haloalkyl, and halogen.

In some embodiments, V is $-OCH_2CH_2-$ and can be represented by Formula (**IIg**) as illustrated below:

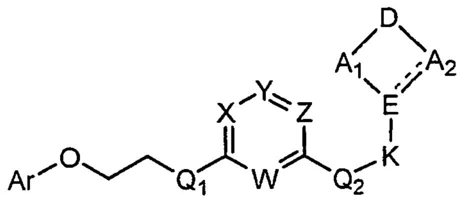

**(IIg)**

wherein each variable in Formula (**IIg**) has the same meaning as described herein, *supra* and *infra.*

In some embodiments, V is $C_{1-3}$ alkylene optionally substituted with one or more substituents selected independently from the group consisting of $C_{1-3}$ alkyl, $C_{1-6}$ alkoxy, carboxy, cyano, $C_{1-3}$ haloalkyl, and halogen.

Some embodiments of the present invention pertain to compounds wherein V is $C_{1-3}$ alkylene optionally substituted with 1, 2, 3, or 4 substituents selected independently from the group consisting of $C_{1-3}$ alkyl, $C_{1-6}$ alkoxy, carboxy, cyano, $C_{1-3}$ haloalkyl, and halogen.

Some embodiments of the present invention pertain to compounds wherein V is $-CH_2-$optionally substituted with 1 or 2 substituents selected independently from the group consisting of $C_{1-3}$ alkyl, $C_{1-6}$ alkoxy, carboxy, cyano, $C_{1-3}$ haloalkyl, and halogen.

In some embodiments, V is $-CH_2-$ and can be represented by Formula **(IIi)** as illustrated below:

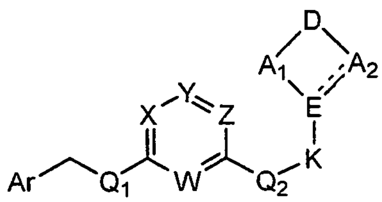

**(IIi)**

wherein each variable in Formula **(IIi)** has the same meaning as described herein, *supra* and *infra.*

Some embodiments of the present invention pertain to compounds wherein $Q_1$ is $NR_4$.

In some embodiments, $Q_1$ is $NR_4$ and can be represented by Formula (**IIk**) as illustrated below:

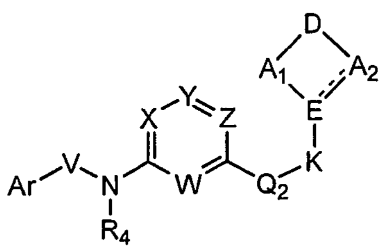

**(IIk)**

wherein each variable in Formula (**IIk**) has the same meaning as described herein, *supra* and *infra.*

Some embodiments of the present invention pertain to compounds wherein $R_4$ is H, or $C_{1-6}$ alkyl.

Some embodiments of the present invention pertain to compounds wherein $R_4$ is H.

Some embodiments of the present invention pertain to compounds wherein $R_4$ is $C_{3-7}$ cycloalkyl. In some embodiments, $R_4$ is cyclopropyl.

Some embodiments of the present invention pertain to compounds wherein $R_4$ is $C_{1-3}$-alkylene-$C_{3-7}$-cycloalkyl. In some embodiments, $R_4$ is cyclopropylmethyl (i.e., $cC_3H_5CH_2-$).

Some embodiments of the present invention pertain to compounds wherein $Q_1$ is O.

In some embodiments, $Q_1$ is O and can be represented by Formula (**IIm**) as illustrated below:

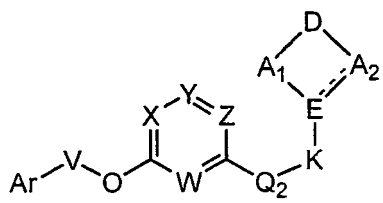

**(IIm)**

wherein each variable in Formula (**IIm**) has the same meaning as described herein, *supra* and *infra.*

In some embodiments, $Q_1$ is S.

In some embodiments, $Q_1$ is S(O), also represented as -S(=O)-.

In some embodiments, Q, is $S(O)_2$, also represented as $-S(=O)_2-$.

Some embodiments of the present invention pertain to compounds wherein $Q_2$ is absent.

In some embodiments, $Q_2$ is absent and can be represented by Formula (**IIo**) as illustrated below:

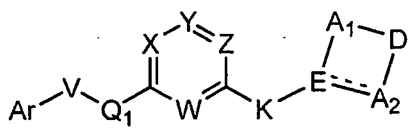

**(IIo)**

wherein each variable in Formula (**IIo**) has the same meaning as described herein, *supra* and *infra.*

Some embodiments of the present invention pertain to compounds wherein $Q_2$ is $NR_5$.

In some embodiments, $Q_2$ is $NR_5$ and can be represented by Formula (**IIq**) as illustrated below:

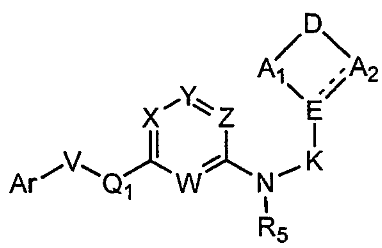

**(IIq)**

wherein each variable in Formula (**IIq**) has the same meaning as described herein, *supra* and *infra.*

Some embodiments of the present invention pertain to compounds wherein $R_5$ is H, $C_{1-6}$ alkyl, $C_{3-7}$ cycloalkyl, or $C_{3-7}$-cycloalkyl-$C_{1-3}$-alkylene.

Some embodiments of the present invention pertain to compounds wherein $R_5$ is H.

Some embodiments of the present invention pertain to compounds wherein $Q_2$ is O.

In some embodiments, $Q_2$ is O and can be represented by Formula (**IIs**) as illustrated below:

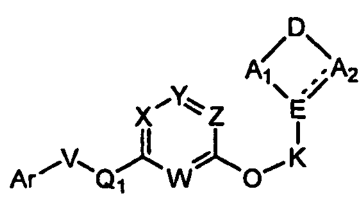

**(IIs)**

wherein each variable in Formula (**IIs**) has the same meaning as described herein, *supra* and *infra*.

In all embodiments, W is CH.

In some embodiments, X is N.

In some embodiments, X is $CR_6$.

In some embodiments, Y is N.

In some embodiments, Y is $CR_7$.

In all embodiments, Z is N.

Some embodiments of the present invention pertain to compounds wherein W is CH; X is N or N-oxide; Y is $CR_7$; and Z is N or N-oxide.

In some embodiments, W is CH; X is N; Y is $CR_7$; and Z is N; and can be represented by Formula (**IIu**) as illustrated below:

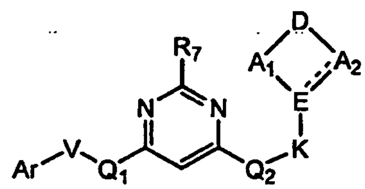

**(IIu)**

wherein each variable in Formula (**IIu**) has the same meaning as described herein, *supra* and *infra.*

Some embodiments of the present invention pertain to compounds wherein W is CH; X is $CR_6$; Y is $CR_7$; and Z is N or N-oxide.

In some embodiments, W is CH; X is $CR_6$; Y is $CR_7$; and Z is N; and can be represented by Formula (**IIy**) as illustrated below:

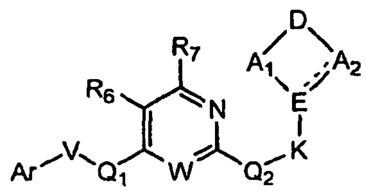

**(IIy)**

wherein each variable in Formula (**IIy**) has the same meaning as described herein, *supra* and *infra.*

Some embodiments of the present invention pertain to compounds wherein $R_6$ is selected from the group consisting of H, $C_{1-6}$ alkyl, and halogen.

Some embodiments of the present invention pertain to compounds wherein $R_6$ is H.

Some embodiments of the present invention pertain to compounds wherein $R_7$ is selected from the group consisting of H, $C_{1-6}$ alkyl, and halogen.

Some embodiments of the present invention pertain to compounds wherein $R_7$ is H.

Some embodiments of the present invention pertain to compounds wherein $R_8$ is selected from the group consisting of H, $C_{1-6}$ alkyl, and halogen.

Some embodiments of the present invention pertain to compounds wherein $\mathbf{R_8}$ is H.

In some embodiments, X is CH.

In some embodiments, Y is CH.

Some embodiments of the present invention pertain to compounds wherein W and Y are both CH, and X and Z are both N.

In some embodiments, W and Y are both CH, and X and Z are both N; and can be represented by Formula (**IIIe**) as illustrated below:

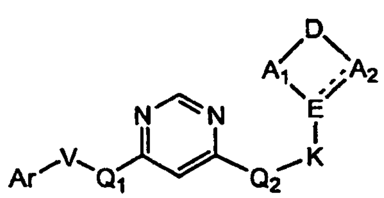

**(IIIe)**

wherein each variable in Formula (**IIIe**) has the same meaning as described herein, *supra* and *infra*.

Some embodiments of the present invention pertain to compounds wherein E is N.

Some embodiments of the present invention pertain to compounds wherein E is C (i.e., a carbon atom).

Some embodiments of the present invention pertain to compounds wherein E is $CR_3$.

Some embodiments of the present invention pertain to compounds wherein E is CH.

Some embodiments of the present invention pertain to compounds wherein D is $CR_1R_2$.

Some embodiments of the present invention pertain to compounds wherein D is $NR_2$.

Some embodiments of the present invention pertain to compounds wherein E is N and D is $CR_1R_2$. In some embodiments, $R_1$ is H.

Some embodiments of the present invention can be represented by Formula (IIIg) as illustrated below:

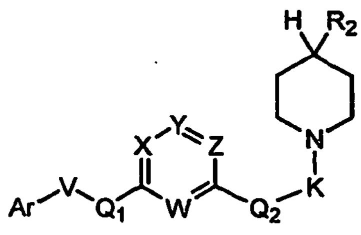

**(IIIg)**

wherein each variable in Formula (**IIIg**) has the same meaning as described herein, *supra* and *infra.*

Some embodiments of the present invention pertain to compounds wherein E is $CR_3$ and D is $NR_2$.

Some embodiments of the present invention can be represented by Formula (**IIIi**) as illustrated below:

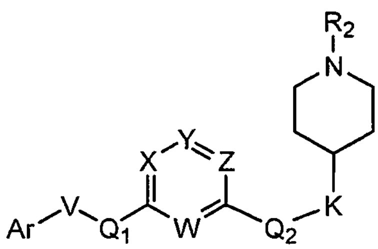

**(IIIi)**

wherein each variable in Formula (**IIIi**) has the same meaning as described herein, *supra* and *infra.*

Some embodiments of the present invention pertain to compounds wherein $R_2$ is selected from the group consisting of $C_{1-6}$ alkyl, aryl, aryloxy, heteroaryl, and heteroaryloxy; wherein $R_2$ is optionally substituted with 1, 2, 3, 4, or 5 substituents selected independently from the group consisting $_O$f $C_{1-6}$ alkoxy, $C_{1-6}$ alkyl, $C_{1-6}$ alkylamino, amino, di-$C_{1-6}$-alkylamino, $C_{1-6}$ alkoxycarbonyl, carboxy, halogen, and heteroaryl, and wherein $C_{1-6}$ alkyl is further optionally substituted with 1, 2, or 3 substituents selected independently from the group consisting of $C_{1-6}$ alkylamino, di-$C_{1-6}$-alkylamino, $C_{3-6}$ cycloalkyl, and halogen.

Some embodiments of the present invention pertain to compounds wherein $R_2$ is $C_{1-6}$ alkyl optionally substituted $C_{1-6}$ alkoxycarbonyl, or carboxy.

Some embodiments of the present invention pertain to compounds wherein $R_2$ is ethoxycarbonylmethyl (i.e., $-CH_2CO_2Et$), or carboxymethyl (i.e., $-CH_2CO_2H$).

Some embodiments of the present invention pertain to compounds wherein $R_2$ is aryl optionally substituted with 1, 2, or 3 substituents selected from the group consisting of $C_{1-6}$ alkoxy, and $C_{1-6}$ alkyl.

Some embodiments of the present invention pertain to compounds wherein $R_2$ is 4-isopropylphenyl, 4-isobutylphenyl, or 4-isopropoxy-phenyl.

Some embodiments of the present invention pertain to compounds wherein $R_2$ is aryloxy optionally substituted with 1, 2, 3, 4, or 5 halogens.

Some embodiments of the present invention pertain to compounds wherein $R_2$ is 3-fluoro-phenoxy

In some embodiments $R_2$ is a 5-member heteroaryl, for example but not limited to those shown in TABLE **3**, optionally substituted with 1 to 4 substituents selected from the group consisting of $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$alkylsulfonamide, $C_{1-6}$ alkylsulfinyl,

$C_{1-6}$ alkylsulfonyl, $C_{1-6}$ alkylthio, $C_{1-6}$ alkylthiocarboxamide, $C_{1-6}$ alkylthioureyl, $C_{1-6}$alkylureyl, amino, aryl, di-$C_{1-6}$-alkylamino, carbo-$C_{1-6}$-alkoxy, carboxamide, carboxy, cyano, $C_{3-6}$ cycloalkyl, di-$C_{1-6}$-alkylearboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, heterocyclic, heteroaryl, hydroxyl, hydroxylamino, nitro, and thiol.

In some embodiments, E is N, D is $CHR_2$, and $R_2$ is a 5-member heteroaryl, for example but not limited to those shown in TABLE 3, optionally substituted with 1 to 4 substituents selected from the group consisting of $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, aryl, di-$C_{1-6}$-alkylamino, carbo-$C_{1-6}$-alkoxy, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloallcylsulfinyl, $C_{1-6}$ haloalkylsulfonyl, $C_{1-6}$ haloalkylthio, heterocyclic, heteroaryl, hydroxyl, hydroxylamino, nitro, and thiol.

Some embodiments of the present invention pertain to compounds wherein $R_2$ is heteroaryl optionally substituted with 1, 2, or 3 substituents selected independently from the group consisting of $C_{1-6}$ alkyl, and heteroaryl, and wherein $C_{1-6}$ alkyl is further optionally substituted with 1, or 2 substituents selected independently from the group consisting of $C_{1-6}$ alkylamino, di-$C_{1-6}$-alkylamino, and $C_{3-6}$ cycloalkyl.

Some embodiments of the present invention pertain to compounds wherein $R_2$ is 3-isopropyl-[1,2,4]oxadiazol-5-yl, 3-isobutyl-[1,2,4]oxadiazol-5-yl, 3-dimethylaminomethyl-[1,2,4]oxadiazol-5-yl, 3-cyclopropylmethyl-[1,2,4]oxadiazol-5-yl, or 3-pyridin-2-yl-[1,2,4]oxadiazol-5-yl.

Some embodiments of the present invention pertain to compounds wherein $R_2$ is heteroaryloxy optionally substituted with 1, 2, or 3 substituents selected independently from $C_{1-6}$ alkoxy.

Some embodiments of the present invention pertain to compounds wherein $R_2$ is 5-isopropoxy-pyridin-2-yloxy.

Some embodiments of the present invention pertain to compounds wherein $R_2$ is aryl, arylcarbonyl, $C_{1-6}$ alkoxycarbonyl, $C_{3-7}$-cycloalkoxycarbonyl, heteroaryl, and heteroarylcarbonyl; wherein each $R_2$ is optionally substituted with one or more substituents selected independently from the group consisting of $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1}$-$_{6}$ alkylureyl, amino, aryl, di-$C_{1}$-$_{6}$-alkylamino, carbo-$C_{1}$-$_{6}$-alkoxy, carboxamide, carboxy, cyano, $C_{3-6}$ cycloalkyl, di-$C_{1}$-$_{6}$-alkylcarboxamide, di-$C_{1}$-$_{6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, heterocyclic, heteroaryl, hydroxyl, hydroxylamino, nitro, and thiol.

Some embodiments of the present invention pertain to compounds wherein $R_2$ is aryl, arylcarbonyl, $C_{1-6}$ alkoxycarbonyl, $C_{3-7}$-cycloalkoxycarbonyl, heteroaryl, and heteroarylcarbonyl; wherein each $R_2$ is optionally substituted with one or more substituents selected independently from the group consisting of $C_{1-6}$acyl, $C_{1-6}$alkoxy, $C_{1-6}$alkyl, $C_{1-6}$alkylamino, $C_{1-6}$alkylcarboxamide, $C_{1-6}$ alkylsulfonyl, di-$C_{1-6}$-akylamino, carboxamide, carboxy, cyano, $C_{1-6}$haloalkoxy, $C_{1-6}$haloalkyl, halogen, $C_{1-6}$haloalkylsulfonyl, heterocyclic, and hydroxyl.

Some embodiments of the present invention pertain to compounds wherein $R_2$ is $C_{1-6}$ alkoxycarbonyl, $C_{1-6}$alkoxycarbonyl substituted by $C_{3-6}$cycloalkyl or $C_{3-7}$-cycloalkoxycarbonyl.

Some embodiments of the present invention pertain to compounds wherein $R_2$ is *tert*-butoxycarbonyl, isobutoxycarbonyl, isopropoxycarbonyl, butoxycarbonyl, cyclopropylmethoxycarbonyl, 3-methyl-butoxycarbonyl, cyclobutoxycarbonyl, or 1-ethyl-propoxycarbonyl.

Some embodiments of the present invention pertain to compounds wherein $R_2$ is heteroarylcarbonyl optionally substituted with 1, 2, 3, or 4 substituents selected independently from the group consisting of $C_{1-6}$alkoxy, $C_{1-6}$alkyl, and halogen.

Some embodiments of the present invention pertain to compounds wherein $R_2$ is selected from the group consisting of 5-butyl-pyridine-2-carbonyl, 6-chloro-pyridine-2-carbonyl, 6-bromopyridine-2-carbonyl, 6-methyl-pyridine-2-carbonyl, 6-fluoro-pyridine-2-carbonyl, pyridine-2-carbonyl, 5-bromo-pyridine-3-carbonyl, 5-methyl-pyridine-3-carbonyl, and 5,6-dichloro-pyridine-3-carbonyl.

Some embodiments of the present invention pertain to compounds wherein $R_2$ is heteroaryl optionally substituted with 1, 2, 3, or 4 substituents selected independently from the group consisting of $C_{1-6}$alkoxy, $C_{1-6}$alkyl, and halogen.

Some embodiments of the present invention pertain to compounds wherein $R_2$ is 5-fluoro-pyridin-2-yl, 5-isopropoxy-pyridin-2-yl, or 3-isopropyl-[1,2,4]oxadiazol-5-yl.

Some embodiments of the present invention pertain to compounds wherein $R_2$ is heteroaryl-$C_{1-3}$-alkylene optionally substituted with 1,2, or 3 substituents selected independently from $C_{1-6}$alkyl.

Some embodiments of the present invention pertain to compounds wherein $R_2$ is 3-isopropyl-[1,2,4]oxadiazol-5-ylmethyl, or 2-(3-isopmpyl-[1,2,4]oxadiazol-5-yl)-ethyl.

Some embodiments of the present invention pertain to compounds wherein $R_2$ is $C_{1-6}$alkyl optionally substituted with a $C_{1-6}$alkoxycarbonyl or carboxy group.

Some embodiments of the present invention pertain to compounds wherein $R_2$ is ethoxycarbonylmethyl (-$CH_2CO_2Et$), carboxymethyl (-$CH_2CO_2H$), 2-ethoxycarbonyl-ethyl (-$CH_2CH_2CO_2Et$), or 2-carboxy-ethyl (-

$CH_2CH_2CO_2H$).

Some embodiments of the present invention pertain to compounds wherein $R_2$ is aryl optionally substituted with 1, 2, or 3, $C_{1-6}$alkoxy groups.

Some embodiments of the present invention pertain to compounds wherein $R_2$ is 4-isopropoxy-phenyl.

Some embodiments of the present invention pertain to compounds wherein Ar is phenyl optionally substituted with $R_9$, $R_{10}$, $R_{11}$, $R_{12}$, and $R_{13}$.

Some embodiments of the present invention can be represented by Formula (**IIIk**) as illustrated below:

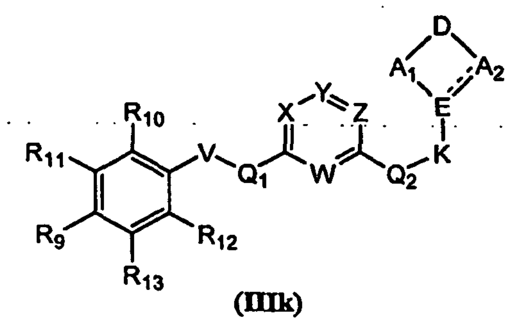

**(IIIk)**

wherein each variable in Formula (**IIIk**) has the same meaning as described herein, *supra* and *infra*.

Some embodiments of the present invention can be represented by Formula (**IIIm**) as illustrated below:

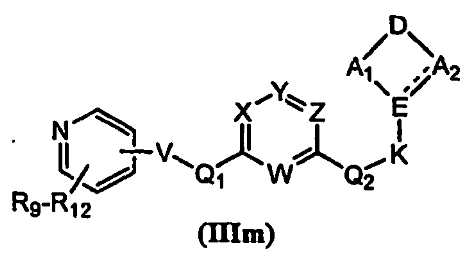

**(IIIm)**

wherein each variable in Formula (**IIIm**) has the same meaning as described herein, *supra* and *infra*.

Some embodiments of the present invention can be represented by Formula (**IIIo**) as illustrated below:

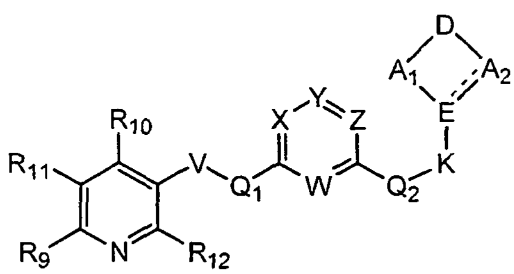

**(IIIo)**

wherein each variable in Formula (**IIIo**) has the same meaning as described herein, *supra* and *infra.*

Some embodiments of the present invention pertain to compounds wherein $R_9$ is $C_{1-6}$ acyl, $C_{1-6}$acylsulfonamide, $C_{1-6}$alkoxy, $C_{1-6}$alkyl, $C_{1-6}$alkylamino, $C_{1-6}$alkylcarboxamide, $C_{2-6}$alkynyl, $C_{1-6}$ alkylsulfonamide, $C_{1-6}$alkylsulfinyl, $C_{1-6}$alkylsulfonyle, $C_{1-6}$alkylthio, amino, aryl, arylsulfonyl, di-$C_{1-6}$-alkylamino, carbamimidoyl, $C_{1-6}$ alkoxycarbonyl, carboxamide, carboxy, cyano, $C_{3-6}$ cycloalkoxy, $C_{3-6}$cycloalkyl, di-$C_{1-6}$-alkylsulfonamide, guanidine, $C_{1-6}$haloalkoxy, $C_{1-6}$haloalkyl, halogen, $C_{1-6}$ haloalkylsulfonyl, heteroaryl, heteroarylcarbonyl, heteroarylsulfonyl, heterocyclic, heterocyclicsulfonyl, hydroxyl, sulfonamide, and thiol; wherein $C_{1-6}$alkoxy, $C_{1-6}$alkyl, $C_{1-6}$ alkylamino, amino, aryl, carbamimidoyl, heterocyclic, are optionally substituted with 1, 2, 3 or 4 substituents selected independently from the group consisting of $C_{1-6}$alkoxy, $C_{1-6}$alkyl, $C_{2-6}$alkynyl, $C_{1-6}$alkylsulfonamide, $C_{1-6}$alkylsulfonyl, amino, aryl, $C_{3-6}$cycloalkyl, di-$C_{1-6}$-alkylamino, halogen, heteroaryl, heterocyclic, and hydroxyl.

Some embodiments of the present invention pertain to compounds wherein $R_9$ is $C_{1-6}$ alkyl optionally substituted with $C_{1-6}$acylsulfonamide. In some embodiments, $R_9$ is:

acetylsulfamoyl-methyl [i.e. $-CH_2S(=O)_2NHC(=O)CH_3$],
propionylsulfamoyl-methyl [i.e. $-CH_2S(=O)_2NHC(=O)CH_2CH_3$],
2-acetylsulfamoyl-ethyl [i.e. $-CH_2CH_2S(=O)_2NHC(=O)CH_3$], or
2-propionylsulfamoyl-ethyl [i.e. $-CH_2CH_2S(=O)_2NHC(-O)CH_2CH_3$].

Some embodiments of the present invention pertain to compounds wherein $R_{10}$, $R_{11}$, $R_{12}$, and $R_{13}$ are independently selected from the group consisting of $C_{1-6}$alkoxy, $C_{1-6}$alkyl, $C_{1-6}$alkylamino, carboxy, cyano, halogen, $C_{1-6}$haloalkoxy, $C_{1-6}$haloalkylsulfonyl, and hydroxyl; or two adjacent $R_{10}$, $R_{11}$, $R_{12}$, and $R_{13}$ groups together with the atoms to which they are bonded form a 5 or 6 member cycloalkyl or 5 or 6 member heterocyclic group wherein said 5 or 6 member group is optionally substituted with halogen or oxo.

Some embodiments of the present invention pertain to compounds wherein $R_9$ is $C_{1-6}$acyl, $C_{1-6}$acylsulfonamide, $C_{1-6}$alkoxy, $C_{1-6}$alkyl, $C_{1-6}$alkylamino, $C_{1-6}$alkylcarboxamide, $C_{1-6}$ alkylsulfonamide, $C_{1-6}$alkylsulfinyl, $C_{1-6}$alkylsulfonyl, $C_{1-6}$alkylthio, amino, di-$C_{1-6}$-alkylamino, carbamimidoyl, $C_{1-6}$alkoxycarbonyl, carboxy, cyano, $C_{3-6}$cycloalkoxy, di-$C_{1-6}$-alkylsulfonamide, guanidine, halogen, $C_{1-6}$haloalkylsulfonyl, heteroaryl, heteroarylcarbonyl, heterocyclicsulfonyl, hydroxyl, sulfonamide, and thiol; wherein $C_{1-6}$alkoxy, $C_{1-6}$alkyl, $C_{1-6}$alkylamino, amino, and carbamimidoyl, are optionally substituted with 1, 2, 3 or 4 substituents selected independently from the group consisting of $C_{1-6}$alkoxy, $C_{1-6}$ alkyl, $C_{2-6}$alkynyl, $C_{1-6}$alkylsulfonyl, amino, aryl, $C_{3-6}$ cycloalkyl, heteroaryl, heterocyclic, and hydroxyl.

Some embodiments of the present invention pertain to compounds wherein $R_9$ is $C_{1-6}$ acyl, $C_{1-6}$acylsulfonamide, $C_{1-6}$alkoxy, $C_{1-6}$alkyl, $C_{1-6}$alkylamino, $C_{1-6}$alkylcarboxamide, $C_{1-6}$ alkylsulfonamide, $C_{1-6}$alkylsulfonyl, $C_{1-6}$alkylthio, amino, carbamimidoyl, $C_{1-6}$alkoxycarbonyl, carboxy, cyano, di-$C_{1-6}$-alkylsulfonamide, halogen, $C_{1-6}$ haloalkylsulfonyl, heteroaryl, heteroarylcarbonyl, heterocyclicsulfonyl, hydroxyl, and sulfonamide; wherein $C_{1-6}$alkyl, $C_{1-6}$ alkylamino, and carbamimidoyl, are optionally substituted with 1, 2, 3 or 4 substituents selected independently from the group consisting of $C_{1-6}$alkyl, $C_{1-6}$alkylsulfonyl, amino, heteroaryl, heterocyclic, and hydroxyl.

Some embodiments of the present invention pertain to compounds wherein $R_9$ is selected from the group consisting of methanesulfonyl ($GH_3SO_2$-), 2-methanesulfonyl-ethyl ($CH_3SO_2CH_2CH_2$-), acetylsulfamoyl [$MeC(=O)NHS(=O)_2$-], propionylsulfamoyl [$EtC(=O)NHS(=O)_2$-], ethylsulfanyl ($CH_3CH_2S$-), isopropylsulfanyl [$(CH_3)_2CHS$-], ethylsulfamoyl ($CH_3CH_2NHSO_2$-), methylsulfamoyl ($CH_3NHSO_2$-), dimethylsulfamoyl [$(CH_3)_2NSO_2$-], methylsulfamoylmethyl [$CH_3NHSO_2CH_2$-], sulfamoyl ($H_2NSO_2$-), [1,2,4]triazol-1-yl, [1,2,4]triazol-1-ylmethyl, 2-[1,2,4]triazol-1-yl-ethyl, methoxy ($CH_3O$-), 2-oxo-oxazolidin-4-ylmethyl, 1,1-dioxo-1$\lambda^6$-thiomorpholin-4-ylmethyl, pyrazol-1-yl, trifluoromethanesulfonyl ($CF_3SO_2$-), morpholine-4-sulfonyl, pyridine-2-carbonyl, F, Cl, cyano, Br, carboxy, butyryl [$CH_3CH_2CH_2C(=O)$-], propoxycarbonyl [$CH_3CH_2CH_2OC(=O)$-], hydroxy, propylcarbamoyl [$CH_3CH_2NHC(=O)$-], *N*-hydroxycarbamimidoyl [$NH_2C(=NOH)$-], carbamimidoyl [$NH_2C(=NH)$-],-*N*-ethylcarbamimidoyl [$CH_3CH_2NHC(=NH)$-], and 2-amino-ethylamino [$NH_2CH_2CH_2NH$-].

Some embodiments of the present invention pertain to compounds wherein $R_{10}$, $R_{11}$, $R_{12}$, and $R_{13}$ are independently selected from the group consisting of $C_{1-6}$alkoxy, $C_{1-6}$alkyl, carboxy, and halogen; or two adjacent $R_{10}$, $R_{11}$, $R_{12}$, and $R_{13}$ groups together with the atoms to which they are bonded form a 5 member heterocyclic group and is optionally substituted with halogen.

Some embodiments of the present invention pertain to compounds wherein $R_{10}$, $R_{11}$, $R_{12}$, and $R_{13}$ are independently selected from the group consisting of F, methoxy ($CH_3O$-), methyl, ethyl, and carboxy.

Some embodiments of the present invention pertain to compounds wherein $R_9$ is selected from the group consisting of $C_{1-6}$acylsulfonamide, $C_{1-6}$alkoxy, $C_{1-6}$alkyl, $C_{1-6}$alkylamino, $C_{1-6}$ alkylcarboxamide, $C_{2-6}$alkynyl, $C_{1-6}$alkylsulfinyl, $C_{1-6}$alkylsulfonyl, $C_{1-6}$alkylthio, amino, di-$C_{1-6}$-alkylamino, carbamimidoyl, cyano, $C_{3-6}$cycloalkoxy, guanidine, $C_{1-6}$haloalkoxy, and halogen; wherein $C_{1-6}$alkoxy, $C_{1-6}$alkyl, $C_{1-6}$alkylamino, and amino, are optionally substituted with 1, 2, 3 or 4 substituents selected independently from the group consisting of $C_{1-6}$alkoxy, $C_{1-6}$alkyl, $C_{2-6}$alkynyl, $C_{1-6}$alkylsulfonyl, aryl, $C_{3-6}$cycloalkyl, di-$C_{1-6}$-alkylamino, heteroaryl, and heterocyclic.

Some embodiments of the present invention pertain to compounds wherein $R_9$ is selected from the group consisting of $C_{1-6}$ acylsulfonamide, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$alkylsulfonyl, $C_{1-6}$alkylthio, di-$C_{1-6}$-alkylamino, cyano, $C_{1-6}$ haloalkoxy, and halogen; wherein $C_{1-6}$alkoxy, $C_{1-6}$ alkylamino, and amino, are optionally substituted with 1, 2, 3 or 4 substituents selected independently from the group consisting of $C_{1-6}$ alkoxy, $C_{1-6}$ alkyl, di-$C_{1-6}$-alkylamino, and heterocyclic.

Some embodiments of the present invention pertain to compounds wherein $R_9$ is selected from the group consisting of methanesulfonyl ($CH_3SO_2$-), cyano, F, Cl, Br, I, methyl, methoxy ($CH_3O$-), ethylamino ($CH_3CH_2NH$-), ethylsulfanyl ($CH_3CH_2S$-), isopropylsulfanyl [$(CH_3)_2CHS$-] , hydroxy, isopropoxy [$(CH_3)_2CHO$-], propoxy ($CH_3CH_2CH_2O$-), dimethylamino [$(CH_3)_2N$-], propylamino ($CH_3CH_2CH_2NH$-), isopropylamino [$(CH_3)_2CHNH$-], acetylamino [$CH_3C(=O)NH$-], piperidin-1-yl, trifluoromethoxy ($CF_3O$-) oxazol-5-yl, ethynyl ($HC\equiv C$-), 3-methyl-butylamino

[$(CH_3)_2CHCH_2CH_2NH$-], 2-morpholin-4-yl-ethylamino, acetylsulfamoyl [MeC(=O)NHS$(=O)_2$-], propionylsulfamoyl [EtC(=O)NHS$(=O)_2$-], tetrahydro-furan-2-ylmethoxy, morpholin-4-yl, 4-methyl-piperazin-1-yl, butylamino, 2-pyrrolidin-1-yl-ethoxy, 2-dimethylamino-ethoxy, 2-morpholin-4-yl-ethoxy, morpholin-4-ylamino, 2-methoxy-ethylamino, and tetrahydro-furan-2-ylmethyl-amino.

Some embodiments of the present invention pertain to compounds wherein $R_{10}$, $R_{11}$, $R_{12}$, and $R_{13}$ are independently selected from the group consisting of $C_{1-6}$ alkoxy, $C_{1-6}$ alkyl, cyano, halogen, $C_{1-6}$ haloalkoxy, and hydroxyl; or two adjacent $R_{10}$, $R_{11}$, $R_{12}$, and $R_{13}$ groups together with the atoms to which they are bonded form a 5 or 6 member cycloalkyl or 5 or 6 member heterocyclic group wherein said 5 or 6 member group is optionally substituted with oxo.

Some embodiments of the present invention pertain to compounds wherein $R_{10}$, $R_{11}$, $R_{12}$, and $R_{13}$ are independently selected from the group consisting of F, Cl, Br, I, hydroxyl, methoxy ($CH_3O$-), cyano, methyl, and trifluoromethoxy.

Some embodiments of the present invention pertain to compounds wherein $R_9$ is selected from the group consisting of $C_{1-6}$acyl, $C_{1-6}$ acylsulfonamide, $C_{1-6}$lkoxy, $C_{1-6}$alkyl, $C_{1-6}$alkylamino, $C_{1-6}$alkylcarboxamide, $C_{1-6}$alkylsulfinyl, $C_{1-6}$alkylsulfonyl, $C_{1-6}$alkylthio, amino, di-$C_{1-6}$-alkylamino, carbamimidoyl, carboxy, cyano, $C_{3-6}$cycloalkoxy, guanidine, $C_{1-6}$haloalkyl, and halogen; wherein $C_{1-6}$alkoxy, $C_{1-6}$alkyl, $C_{1-6}$alkylamino, and amino are optionally substituted with 1, 2, 3 or 4 substituents selected independently from the group consisting of $C_{1-6}$alkoxy, $C_{1-6}$alkyl, $C_{2-6}$alkynyl, $C_{1-6}$ alkylsulfonyl, aryl, $C_{3-6}$cycloalkyl, heteroaryl, and heterocyclic.

Some embodiments of the present invention pertain to compounds wherein $R_9$ is selected from the group consisting of $C_{1-6}$acyl, $C_{1-6}$acylsulfonamide, $C_{1-6}$alkoxy, $C_{1-6}$alkyl, $C_{1-6}$ alkylcarboxamide, $C_{1-6}$alkylsulfonyl, carboxy, $C_{1-6}$haloalkyl, and halogen.

Some embodiments of the present invention pertain to compounds wherein $R_9$ is selected from the group consisting of methanesulfonyl ($CH_3SO_2$-), methoxy ($CH_3O$-), carboxy, acetylsulfamoyl [MeC(=O)NHS$(=O)_2$-], propionylsulfamoyl [EtC(=O)NHS$(=O)_2$-], acetylamino [$CH_3C(=O)NH$-], F, Cl, Br, methyl, and trifluoromethyl.

Some embodiments of the present invention pertain to compounds wherein $R_{10}$, $R_{11}$, $R_{12}$, and $R_{13}$ are independently selected from the group consisting of $C_{1-6}$alkoxy, $C_{1-6}$alkyl, and halogen.

Some embodiments of the present invention pertain to compounds wherein $R_{10}$, $R_{11}$, $R_{12}$, and $R_{13}$ are independently selected from the group consisting of methoxy ($CH_3O$-), methyl, F, Cl, and Br.

Some embodiments of the present invention pertain to compounds wherein $R_9$ is selected from the group consisting of $C_{1-6}$acylsulfonamide, $C_{1-6}$alkoxy, $C_{1-6}$alkyl, $C_{1-6}$alkylamino, $C_{1-6}$ alkylcarboxamide, $C_{1-6}$alkylsulfinyl, $C_{1-6}$ alkylsulfonyl, $C_{1-6}$alkylthio, amino, aryl, arylsulfonyl, di-$C_{1-6}$-alkylamino, carbamimidoyl, carboxamide, cyano, $C_{3-6}$ cycloalkoxy, guanidine, $C_{1-6}$ haloalkyl, halogen, heteroaryl, and heterocyclic; wherein $C_{1-6}$alkoxy, $C_{1-6}$alkyl, $C_{1-6}$alkylamino, amino, and aryl, are optionally substituted with 1, 2, 3 or 4 substituents selected independently from the group consisting of $C_{1-6}$alkoxy, $C_{1-6}$alkyl, $C_{2-6}$alkynyl, $C_{1-6}$alkylsulfonyl, aryl, $C_{3-6}$cycloalkyl, halogen, heteroaryl, and heterocyclic.

Some embodiments of the present invention pertain to compounds wherein $R_9$ is selected from the group consisting of $C_{1-6}$acylsulfonamide, $C_{1-6}$alkoxy, $C_{1-6}$alkyl, $C_{1-6}$alkylamino, $C_{1-6}$ alkylcarboxamide, $C_{1-6}$alkylthio, aryl, arylsulfonyl, carboxamide, cyano, $C_{3-6}$cycloalkoxy, $C_{1-6}$ haloalkyl, halogen, heteroaryl, and heterocyclic; wherein aryl, is optionally substituted with 1, 2, 3 or 4 substituents selected independently from the group consisting of $C_{1-6}$ alkyl, and halogen.

Some embodiments of the present invention pertain to compounds wherein $R_9$ is selected from the group consisting of cyano, F, Cl, Br, acetylamino [$CH_3C(=O)NH$-], methoxy ($CH_3O$-), methyl, propoxy ($CH_3CH_2CH_2O$-), propylamino ($CH_3CH_2CH_2NH$-), isopropylamino [$(CH_3)_2CHNH$-], phenyl, *t*-butyl, 4-methylphenyl, ethyl, methylsulfanyl ($CH_3S$-), morpholin-4-yl, benzenesulfonyl, trifluoromethyl ($CF_3$-), cyclopropyl, carbamoyl [$H_2NC(O)$-], 3,4-difluorophenyl, 4-chlorophenyl, 1-methyl-pyrrolidin-2-yl, acetylsulfamoyl [MeC(=O)NHS$(=O)_2$-] , propionylsulfamoyl [EtC(=O)NHS$(=O)_2$-], and pyridine-2-yl.

Some embodiments of the present invention pertain to compounds wherein $R_{10}$, $R_{11}$, $R_{12}$, and $R_{13}$are independently selected from the group consisting of $C_{1-6}$alkoxy, $C_{1-6}$alkyl, and halogen.

Some embodiments of the present invention pertain to compounds wherein $R_{10}$, $R_{11}$, $R_{12}$, and $R_{13}$ are independently methyl, F or Cl.

Some embodiments of the present invention pertain to compounds wherein Ar is phenyl and two adjacent $R_{10}$, $R_{11}$, $R_{12}$, and $R_{13}$ groups together with the carbons they are bonded form a 5, 6 or 7 member cycloalkyl, 5, 6 or 7 member cycloalkenyl, or 5, 6 or 7 member heterocyclic group wherein the 5, 6 or 7 member group is optionally substituted with halogen or oxo.

In some embodiments, Ar is phenyl and together with two adjacent $R_{10}$ and $R_{11}$ groups form a 5, 6 or 7 member cycloalkyl as represented in TABLE 5:

TABLES

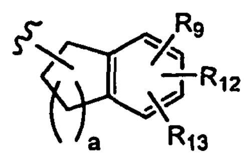 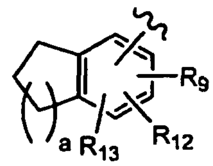

wherein "a" is 1, 2 or 3 to give a 5, 6 or 7 member cycloalkyl that is fused together with the phenyl group where two of the ring carbons are shared between the cycloalkyl and phenyl group.

In some embodiments, the cycloalkyl carbons (i.e., the non aromatic ring carbons) in TABLE 5 are replaced by 1, 2 or 3 heteroatoms selected from, but not limited to, O, S, and N, wherein N is substituted with H or $C_{1-6}$alkyl, thus forming a 5, 6 or 7 member heterocyclic group.

In some embodiments, the two adjacent groups form a 5 member heterocyclic group with the phenyl group.

In some embodiments, the 5 member heterocyclic group with the phenyl group together is a 2,3-dihydro-benzofuran-5-yl or benzo[1,3]dioxol-5-yl group.

In some embodiments, the two adjacent groups form a 6 member heterocyclic group with the phenyl group. In some embodiments, the 6 member heterocyclic group with the phenyl group together is a 2,3-dihydro-benzo[1,4]dioxin-6-yl or 2,3-dihydro-benzo[1,4]dioxin-2-yl group.

In some embodiments, the two adjacent groups form a 7 member heterocyclic group with the phenyl group. In some embodiments, the 7 member heterocyclic group with the phenyl group together is a 3,4-dihydro-2H-benzo[b][1,4]dioxepin-7-yl group.

Some embodiments of the present invention are illustrated in TABLES A and B shown below, wherein "(REF)" denotes a reference compound.

**TABLE A**

| Example No. | Chemical Structure | Chemical Name |
|---|---|---|
| A1 | 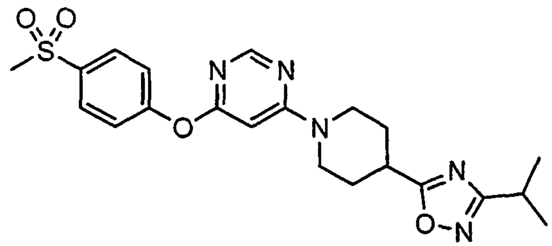 | 4-[4-(3-Isopropyl-[1,2,4]oxadiazol-5-yl)-piperidin-1-yl]-6-(4-methanesulfonyl-phenoxy)-pyrimidine |
| A2 | 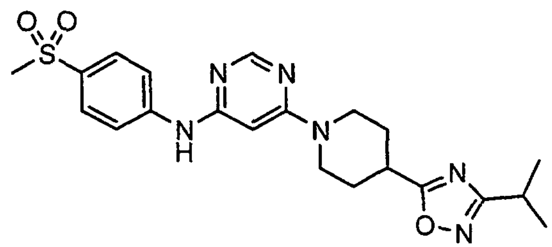 | {6-[4-(3-Isopropyl-[1,2,4]oxadiazol-5-yl)-piperidin-1-yl]-pyrimidin-4-yl} -(4-methanesulfonyl-phenyl)-amine |
| A3 | 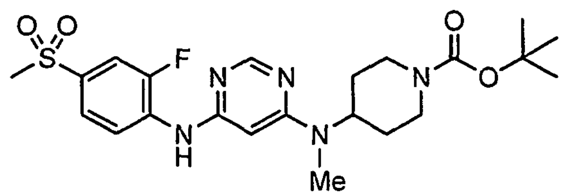 | 4-{[6-(2-Fluoro-4-methanesulfonyl-phenylamino)-pyrimidin-4-yl]-methyl-amino}-piperidine-1-carboxylic acid tert-butyl ester |
| A4 | 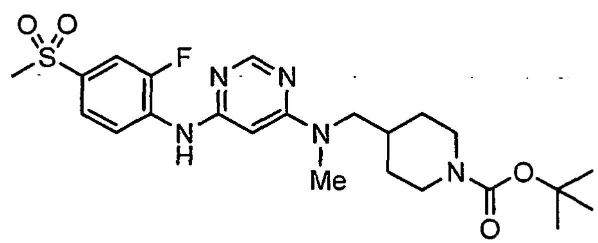 | 4-({[6-(2-Fluoro-4-methanesulfonyl-phenylamino)-pyrimidin-4-yl]-methyl-amino}-methyl)-piperidine-1-carboxylic acid tert-butyl ester |

(continued)

| Example No. | Chemical Structure | Chemical Name |
|---|---|---|
| A5 | 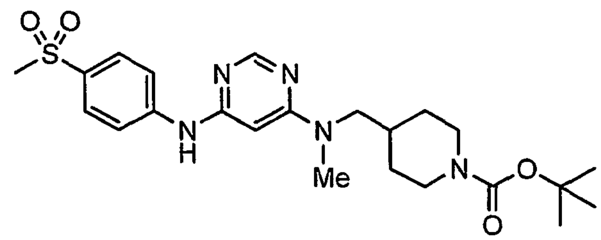 | 4-({[6-(4-Methanesulfonyl-phenylamino)-pyrimidin-4-yl]-methyl-amino}-methyl)-piperidine-1-carboxylic acid tert-butyl ester |
| A6 | 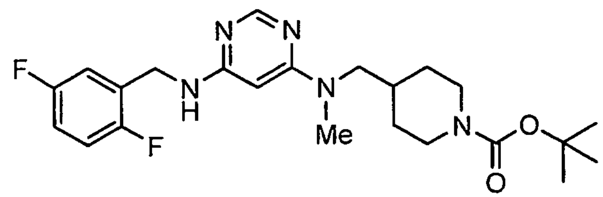 | 4-({[6-{2,5-Difluoro-benzylamino)-pyrimidin-4-yl]-methyl-amino}-methyl)-piperidine-1-carboxylic acid tert-butyl ester |
| A7 | 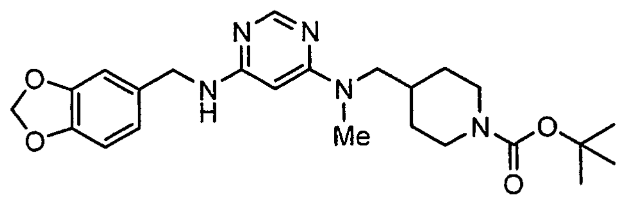 | 4-[({6-[(Benzo[1,3]dioxol-5-ylmethyl)-amino]-pyrimidin-4-yl}-methyl-amino)-methyl]-piperidine-1-carboxylic acid tert-butyl ester |
| A8 | 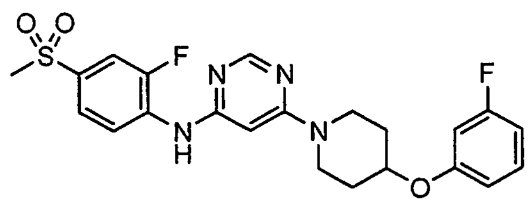 | (2-Fluoro-4-methanesulfonyl-phenyl)-{6-[4-(3-fluoro-phenoxy)-piperidin-1-yl]-pyrimidin-4-yl}-amine |

(continued)

| Example No. | Chemical Structure | Chemical Name |
|---|---|---|
| A9 | 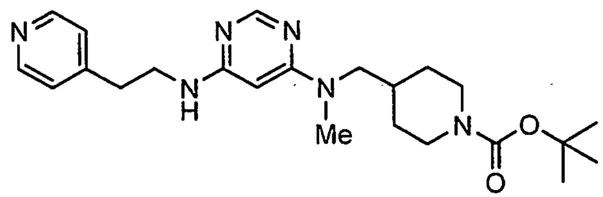 | 4-({Methyl-[6-(2-pyridin-4-yl-ethylamino)-pyrimidin-4-yl]-amino}-methyl)-piperidine-1-carboxylic acid tert-butyl ester |
| A10 | 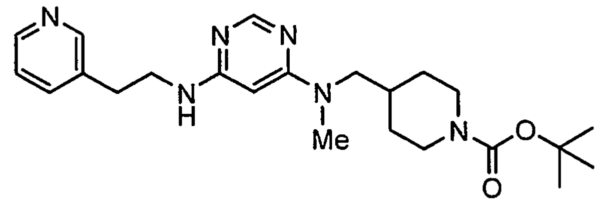 | 4-({Methyl-[6-(2-pyridin-3-yl-ethylamino)-pyrimidin-4-yl]-amino}-methyl)-piperidine-1-carboxylic acid tert-butyl ester |
| A11 | 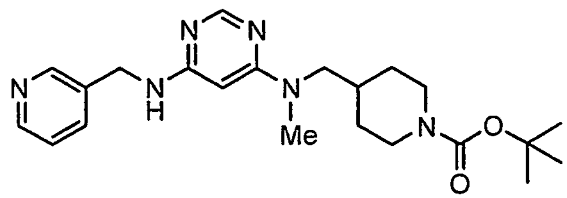 | 4-[(Methyl-{6-[(pyridin-3-ylmethyl)-amino]-pyrimidin-4-yl}-amino)-methyl]-piperidine-1-carboxylic acid tert-butyl ester |
| A12 | 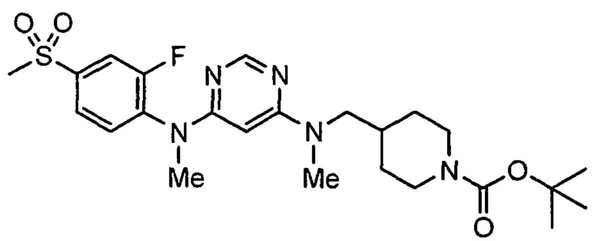 | 4-[({6-[(2-Fluoro-4-methanesulfonyl-phenyl)-methyl-amino]-pyrimidin-4-yl}-methyl-amino)-methyl]-piperidine-1-carboxylic acid tert-butyl ester |

(continued)

| Example No. | Chemical Structure | Chemical Name |
|---|---|---|
| A13 | 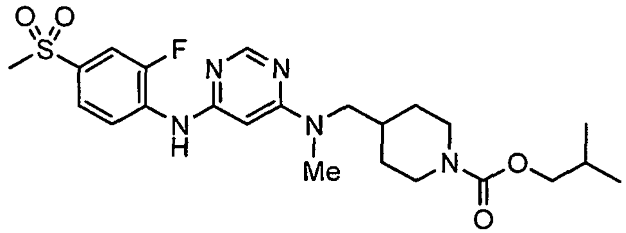 | 4-({[6-(2-Fluoro-4-methanesulfonyl-phenylamino)-pyrimidin-4-yl]-methyl-amino}-methyl)-piperidine-1-carboxylic acid isobutyl ester |
| A14 | 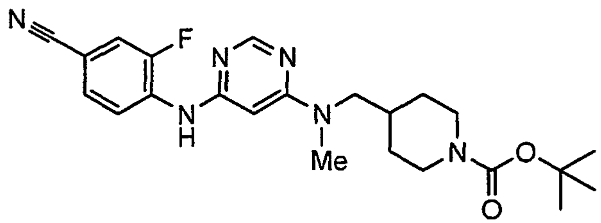 | 4-({[6-(4-Cyano-2-fluoro-phenylamino)-pyrimidin-4-yl]-methyl-amino}-methyl)-piperidine-1-carboxylic acid tert-butyl ester |
| A15 | 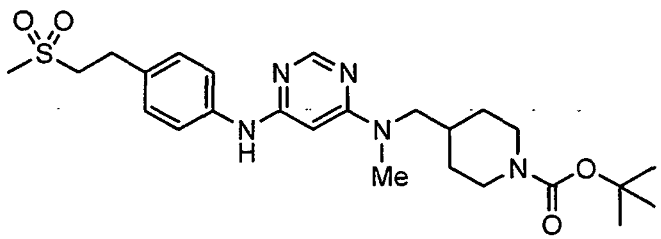 | 4-[({6-[4-(2-Methanesulfonyl-ethyl)-phenylamino]-pyrimidin-4-yl}-methyl-amino)-methyl]-piperidine-1-carboxylic acid tert-butyl ester |
| A16 | 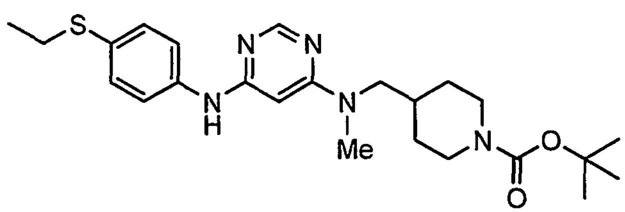 | 4-({[6-(4-Ethylsulfanyl-phenylamino)-pyrimidin-4-yl]-methyl-amino}-methyl)-piperidine-1-carboxylic acid tert-butyl ester |

(continued)

| Example No. | Chemical Structure | Chemical Name |
|---|---|---|
| A17 | 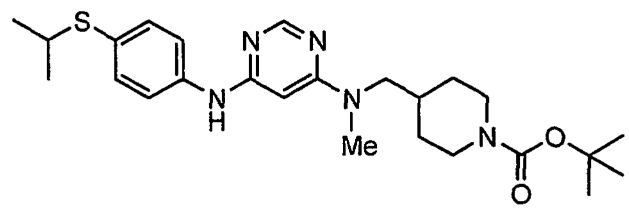 | 4-({[6-(4-Isopropylsulfanyl-phenylamino)-pyrimidin-4-yl]-methyl-amino}-methyl), piperidine-1-carboxylic acid tert-butyl ester |
| A18 | 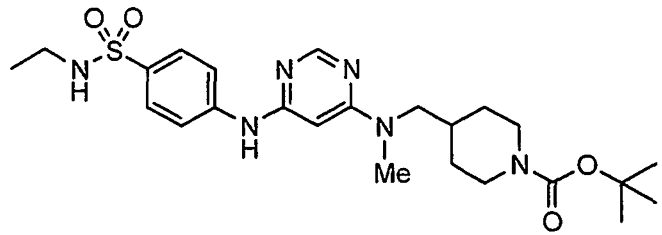 | 4-({[6-(4-Ethylsulfamoyl-phenylamino)-pyrimidin-4-yl]-methyl-amino}-methyl)-piperidine-1-carboxylic acid tert-butyl ester |
| A19 | 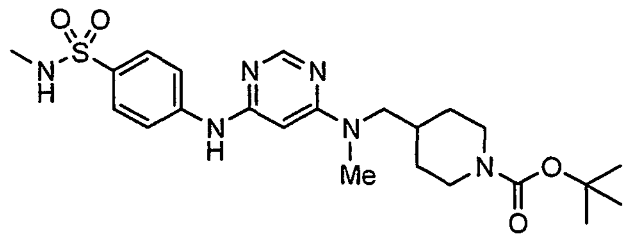 | 4-({Methyl-[6-(4-methylsulfamoyl-phenylamino)-pyrimidin-4-yl]-amino}-methyl)-piperidine-1-carboxylic acid tert-butyl ester |
| A20 | 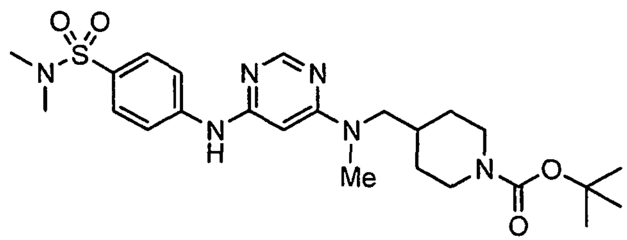 | 4-({[6-(4-Dimethylsulfamoyl-phenylamino)-pyrimidin-4-yl]-methyl-amino}-methyl)-piperidine-1-carboxylic acid tert-butyl ester |

(continued)

| Example No. | Chemical Structure | Chemical Name |
| --- | --- | --- |
| A21 | 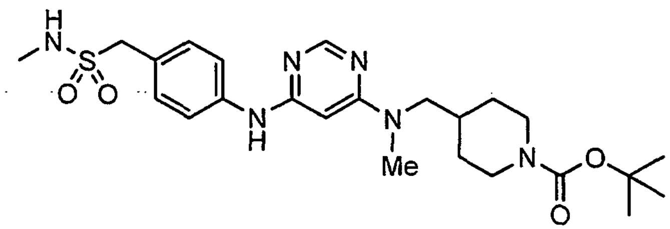 | 4-({Methyl-[6-(4-methylsulfamoylmethyl-phenylamino)-pyrimidin-4-yl]-amino}-methyl)-piperidine-1 -carboxylic acid tert-butyl ester |
| A22 | 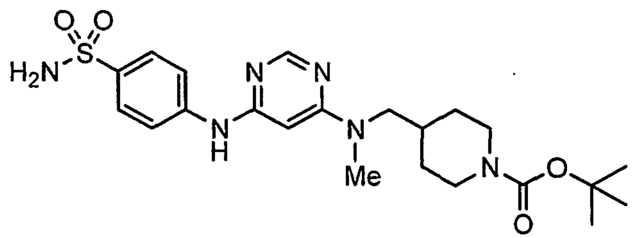 | 4-({Methyl-[6-(4-sulfamoyl-phenylamino)-pyrimidin-4-yl]-amino}-methyl)-piperidine-1-carboxylic acid tert-butyl ester |
| A23 | 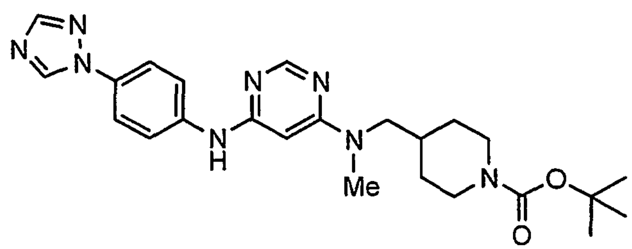 | 4-({Methyl-[6-(4-[1,2,4]triazol-1-yl-phenylamino)-pyrimidin-4-yl]-amino}-methyl)-piperidine-1-carboxylic acid tert-butyl ester |
| A24 | 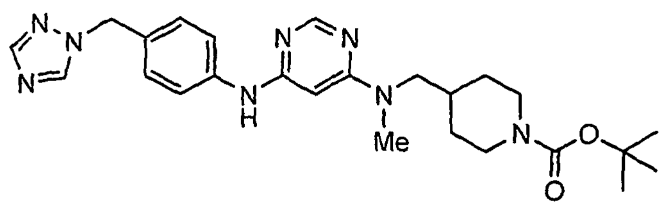 | 4-({Methyl-[6-(4-[1,2,4]triazol-1-ylmethyl-phenylamino)-pyrimidin-4-yl]-amino}-methyl)-piperidine-1-carboxylic acid tert-butyl ester |

(continued)

| Example No. | Chemical Structure | Chemical Name |
|---|---|---|
| A25 | 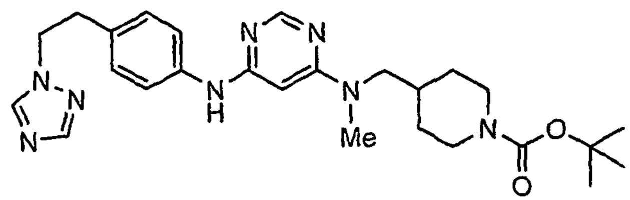 | 4-[(Methyl-{6-[4-(2-[1,2,4]triazol-1-yl-ethyl)-phenylamino]-pyrimidin-4-yl}-amino)-methyl]-piperidine-1-carboxylic acid tert-butyl ester |
| A26 | 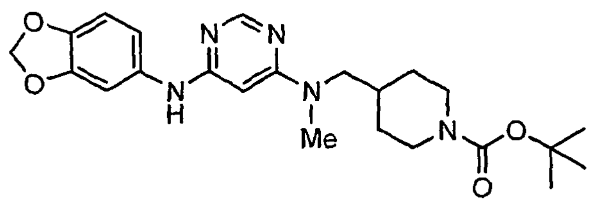 | 4-({[6-(Benzo[1,3]dioxol-5-ylamino)-pyrimidin-4-yl]- methyl-amino}-methyl)-piperidine-1-carboxylic acid tert-butyl ester |
| A27 | 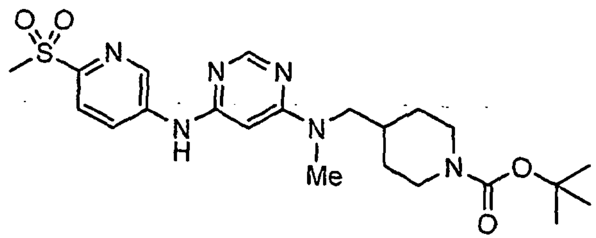 | 4-({ [6-(6-Methanesulfonyl-pyridin-3-ylamino)-pyrimidin-4-yl]-methyl-amino}-methyl)-piperidine-1-carboxylic acid tert-butyl ester |
| A28 | 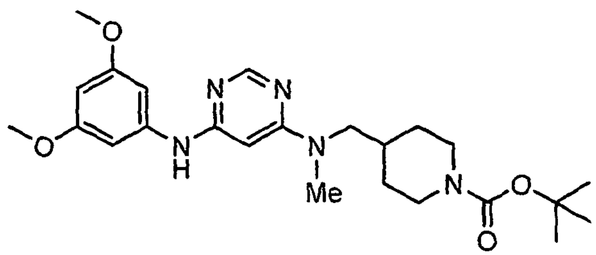 | 4-({[6-(3,5-Dimethoxy-phenylamino)-pyrimidin-4-yl]-methyl-amino}-methyl)-piperidine-1-carboxylic acid tert-butyl ester |

(continued)

| Example No. | Chemical Structure | Chemical Name |
|---|---|---|
| A29 | 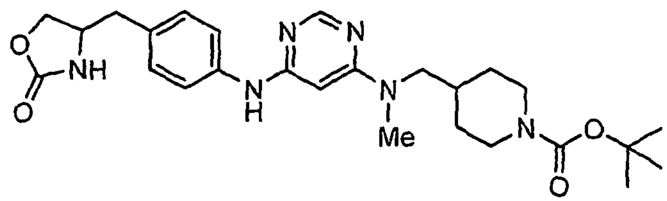 | 4-[(Methyl-{6-[4-(2-oxo-oxazolidin-4-ylmethyl)-phenylamino]-pyrimidin-4-yl}-amino)-methyl]-piperidine-1-carboxylic acid tert-butyl ester |
| A30 | 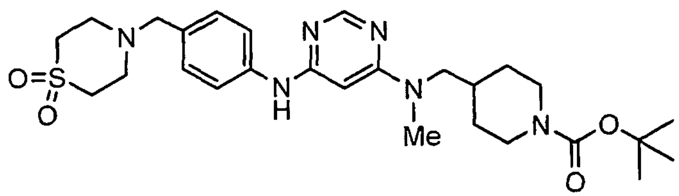 | 4-[({6-[4-(1,1-Dioxo-1λ6-thiomorpholin-4-ylmethyl)-phenylamino]-pyrimidin-4-yl}-methyl-amino)-methyl]-piperidine-1-carboxylic acid tert-butyl ester |
| A31 | 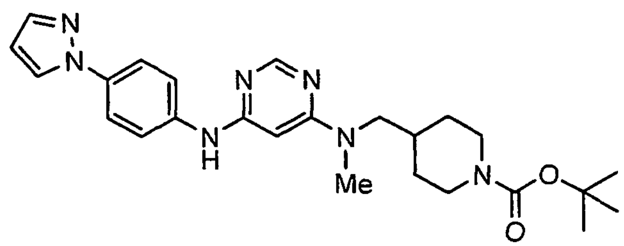 | 4-({Methyl-[6-(4-pyrazol-1-yl-phenylamino)-pyrimidin-4-yl]-amino}-methyl)-piperidine-1-carboxylic acid tert-butyl ester |
| A32 | 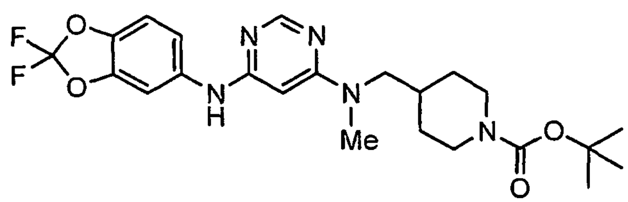 | 4-({[6-(2,2-Difluoro-benzo[1,3]dioxol-5-ylamino)-pyrimidin-4-yl]-methyl-amino}-methyl)-piperidine-1-carboxylic acid tert-butyl ester |

(continued)

| Example No. | Chemical Structure | Chemical Name |
|---|---|---|
| A33 | 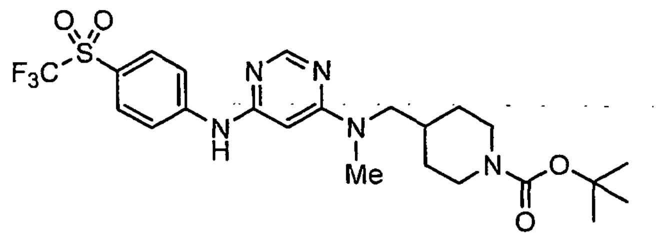 | 4-({Methyl-[6-(4-trifluoromethanesulfonyl-phenylamino)-pyrimidin-4-yl]-amino}-methyl)-piperidine-1-carboxylic acid tert-butyl ester |
| A34 | 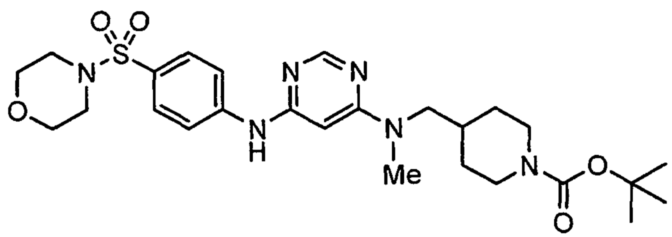 | 4-[(Methyl-{6-[4-(morpholine-4-sulfonyl)-phenylamino]-pyrimidin-4-yl}-amino)-methyl]-piperidine-1-carboxylic acid tert-butyl ester |
| A35 | 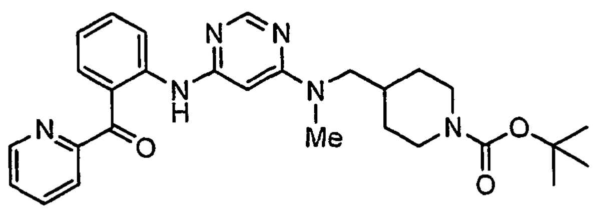 | 4-[(Methyl-{6-[2-(pyridine-2-carbonyl)-phenylamino]-pyrimidin-4-yl}-amino)-methyl]-piperidine-1-carboxylic acid tert-butyl ester |
| A36 | 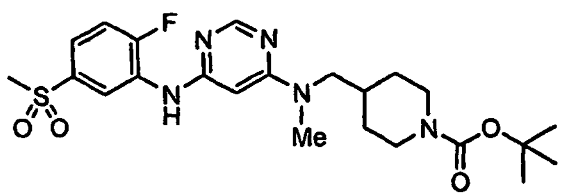 | 4-({[6-(2-Fluoro-5-methanesulfonyl-phenylamino)-pyrimidin-4-yl]-methyl-amino}-methyl)-piperidine-1-carboxylic acid tert-butyl ester |

(continued)

| Example No. | Chemical Structure | Chemical Name |
|---|---|---|
| A37 (REF) | 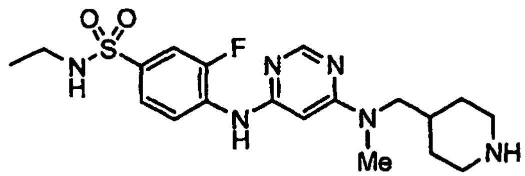 | N-Ethyl-3-fluoro-4-[6-(methyl-piperidin-4-ylmethyl-amino)-pyrimidin-4-ylamino]-benzenesulfonamide |
| A38 (REF) | 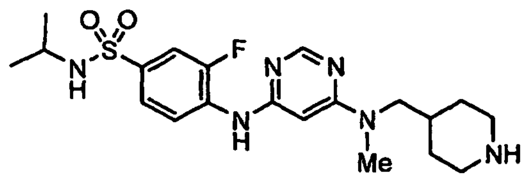 | 3-Fluoro-N-isopropyl-4-[6-(methyl-piperidin-4-ylmethyl-amino)-pyrimidin-4-ylamino]-benzenesulfonamide |
| A39 | 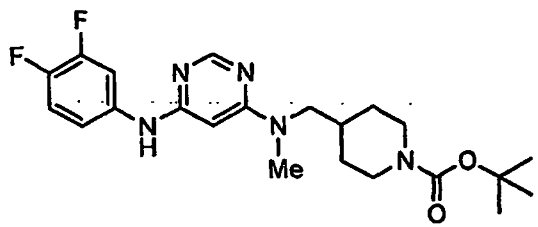 | 4-({[6-(3,4-Difluoro-phenylamino)-pyrimidin-4-yl]-methyl-amino}-methyl)-piperidine-1-carboxylic acid tert-butyl ester |
| A40 | 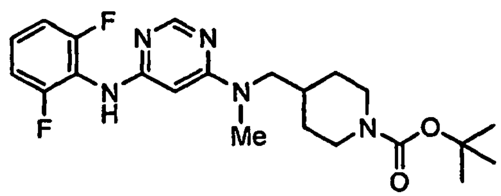 | 4-({[6-(2,6-Difluoro-phenylamino)-pyrimidin-4-yl]-methyl-amino}-methyl)-piperidine-1-carboxylic acid tert-butyl ester |

(continued)

| Example No. | Chemical Structure | Chemical Name |
|---|---|---|
| A41 | 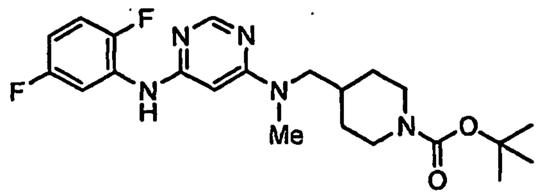 | 4-({[6-(2,5-Difluoro-phenylamino)-pyrimidin-4-yl]-methyl-amino}-methyl)-piperidine-1-carboxylic acid tert-butyl ester |
| A42 | 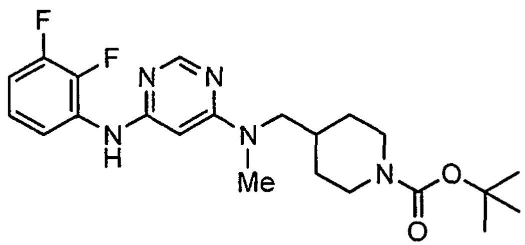 | 4-({[6-(2,3-Difluoro-phenylamino)-pyrimidin-4-yl]-methyl-amino}-methyl)-piperidine-1-carboxylic acid tert-butyl ester |
| A43 | 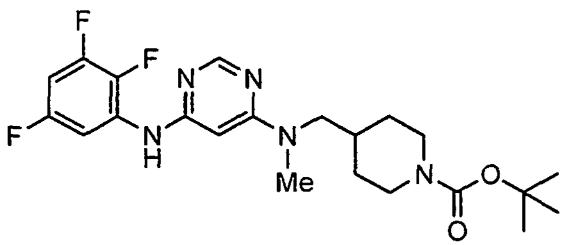 | 4-({Methyl-[6-(2,3,5-trifluoro-phenylamino)-pyrimidin-4-yl]-amino}-methyl)-piperidine-1-carboxylic acid tert-butyl ester |
| A44 | 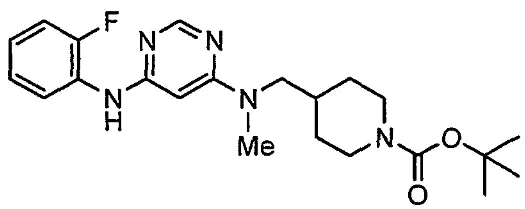 | 4-({[6-(2-Fluoro-phenylamino)-pyrimidin-4-yl]-methyl-amino}-methyl)-piperidine-1-carboxylic acid tert-butyl ester |

(continued)

| Example No. | Chemical Structure | Chemical Name |
|---|---|---|
| A45 | 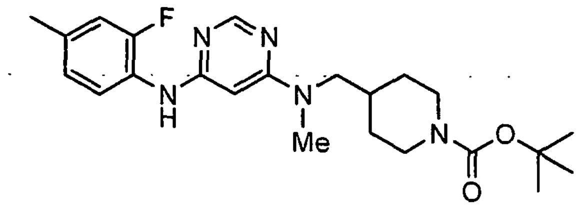 | 4-({[6-(2-Fluoro-4-methyl-phenylamino)-pyrimidin-4-yl]-methyl-amino}-methyl)-piperidine-1-carboxylic acid tert-butyl ester |
| A46 | 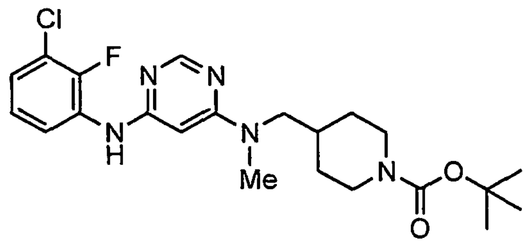 | 4-({[6-(3-Chloro-2-fluoro-phenylamino)-pyrimidin-4-yl]-methyl-amino}-methyl)-piperidine-1-carboxylic acid tert-butyl ester |
| A47 | 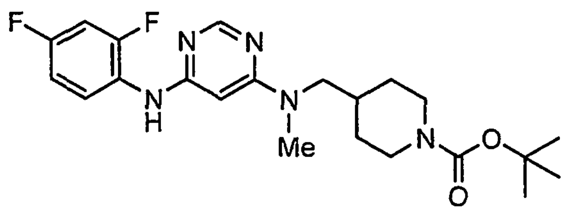 | 4-({[6-(2,4-Difluoro-phenylamino)-pyrimidin-4-yl]-methyl-amino}-methyl)-piperidine-1-carboxylic acid tert-butyl ester |
| A48 | 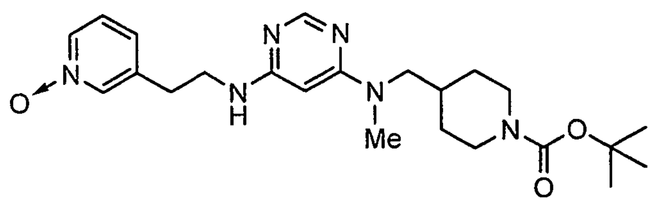 | 4-[(Methyl-{6-[2-(1-oxy-pyridin-3-yl)-ethylamino]-pyrimidin-4-yl}-amino)-methyl]-piperidine-1-carboxylic acid tert-butyl ester |

(continued)

| Example No. | Chemical Structure | Chemical Name |
|---|---|---|
| A49 | 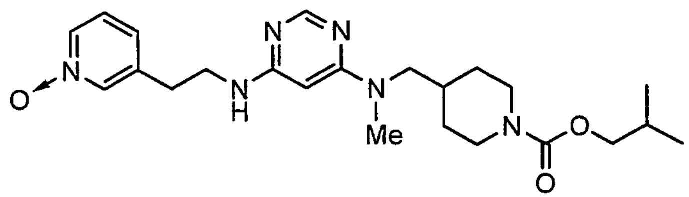 | 4-[(Methyl-{6-[2-(1-oxy-pyridin-3-yl)-ethylamino]-pyrimidin-4-yl}-amino)-methyl]-piperidine-1-carboxylic acid isobutyl ester |
| A50 | 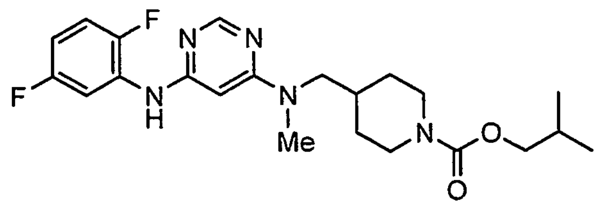 | 4-({[6-(2,5-Difluoro-phenylamino)-pyrimidin-4-yl]-methyl-amino}-methyl)-piperidine-1-carboxylic acid isobutyl ester |
| A51 | 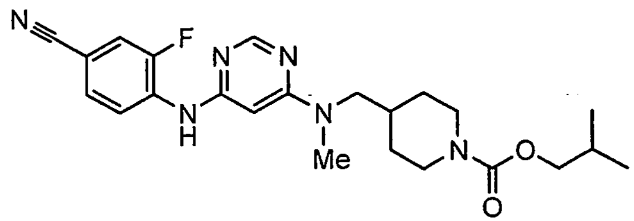 | 4-({[6-(4-Cyano-2-fluoro-phenylamino)-pyrimidin-4-yl]-methyl-amino}-methyl)-piperidine-1-carboxylic acid isobutyl ester |
| A52 | 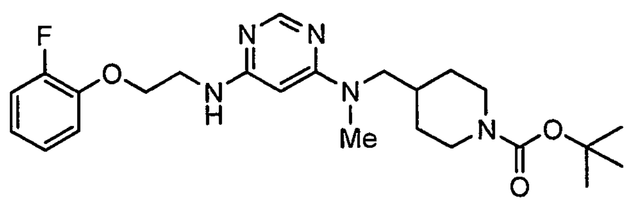 | 4-[({6-[2-(2-Fluoro-phenoxy)-ethylamino]-pyrimidin-4-yl}-methyl-amino)-methyl]-piperidine-1-carboxylic acid tert-butyl ester |

(continued)

| Example No. | Chemical Structure | Chemical Name |
|---|---|---|
| A53 | 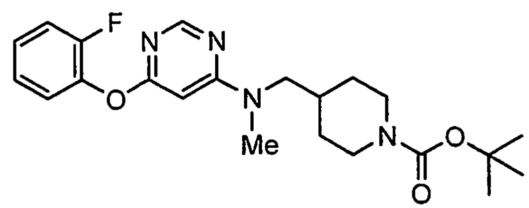 | 4-({[6-(2-Fluoro-phenoxy)-pyrimidin-4-yl]-methyl-amino}-methyl)-piperidine-1-carboxylic acid tert-butyl ester |
| A54 | 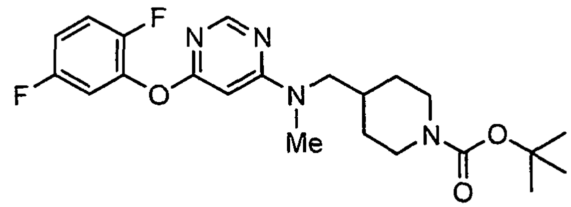 | 4-({[6-(2,5-Difluoro-phenoxy)-pyrimidin-4-yl]-methyl-amino}-methyl)-piperidine-1-carboxylic acid tert-butyl ester |
| A55 | 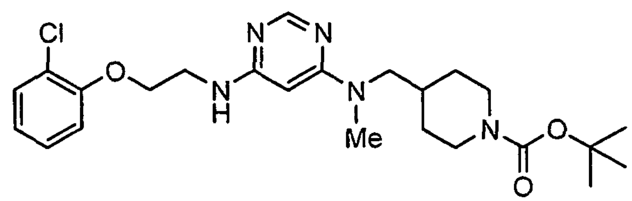 | 4-[({6-[2-(2-Chloro-phenoxy)-ethylamino]-pyrimidin-4-yl}-methyl-amino)-methyl]-piperidine-1-carboxylic acid tert-butyl ester |
| A56 | 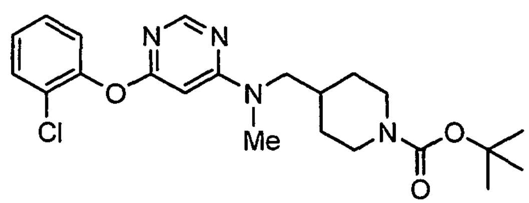 | 4-({[6-(2-Chloro-phenoxy)-pyrimidin-4-yl]-methyl-amino}-methyl)-piperidine-1-carboxylic acid tert-butyl ester |

(continued)

| Example No. | Chemical Structure | Chemical Name |
| --- | --- | --- |
| A57 | 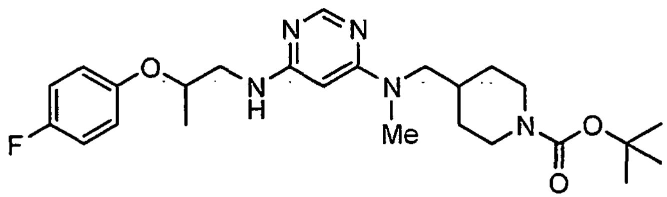 | 4-[({6-[2-(4-Fluoro-phenoxy)-propylamino]-pyrimidin-4-yl}-methyl-amino)-methyl]-piperidine-1-carboxylic acid tert-butyl ester |
| A58 | 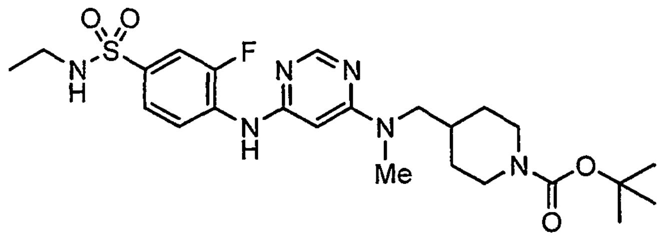 | 4-({[6-(4-Ethylsulfamoyl-2-fluoro-phenylamino)-pyrimidin-4-yl]-methyl-amino}-methyl)-piperidine-1-carboxylic acid tert-butyl ester |
| A59 | 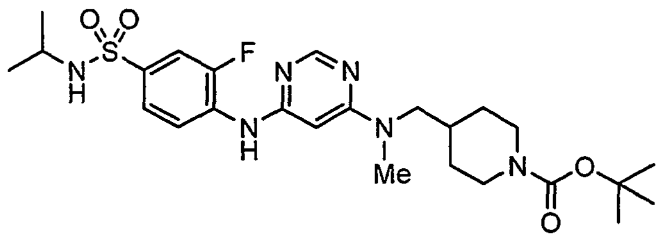 | 4-({[6-(2-Fluoro-4-isopropylsulfamoyl-phenylamino)-pyrimidin-4-yl]-methyl-amino}-methyl)-piperidine-1-carboxylic acid tert-butyl ester |
| A60 | 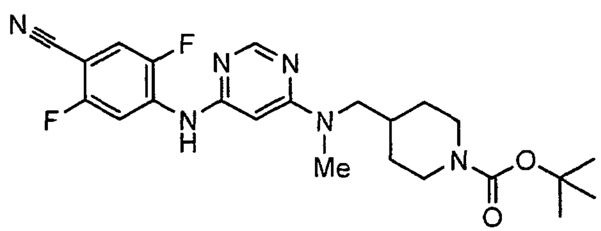 | 4-({[6-(4-Cyano-2,5-difluoro-phenylamino)-pyrimidin-4-yl]-methyl-amino}-methyl)-piperidine-1-carboxylic acid tert-butyl ester |

(continued)

| Example No. | Chemical Structure | Chemical Name |
|---|---|---|
| A61 | 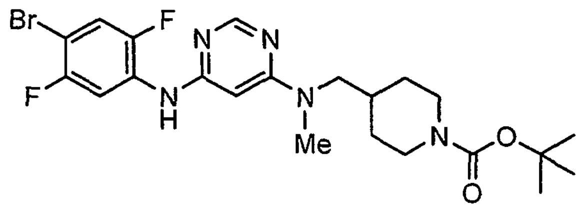 | 4-({[6-(4-Bromo-2,5-difluoro-phenylamino)-pyrimidin-4-yl]-methyl-amino}-methyl)-piperidine-1-carboxylic acid tert-butyl ester |
| A62 | 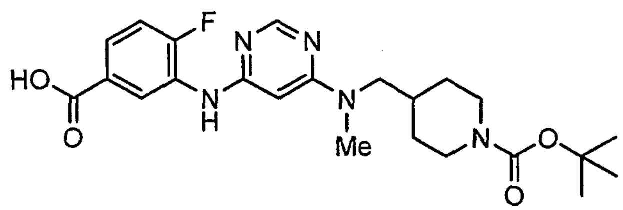 | 4-({[6-(5-Carboxy-2-fluoro-phenylamino)-pyrimidin-4-yl]-methyl-amino}-methyl)-piperidine-1-carboxylic acid tert-butyl ester |
| A63 | 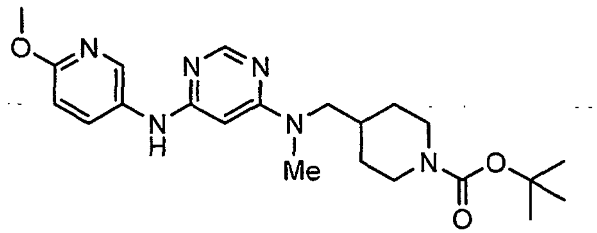 | 4-({[6-(6-Methoxy-pyridin-3-ylamino)-pyrimidin-4-yl]-methyl-amino}-methyl)-piperidine-1-carboxylic acid tert-butyl ester |
| A64 | 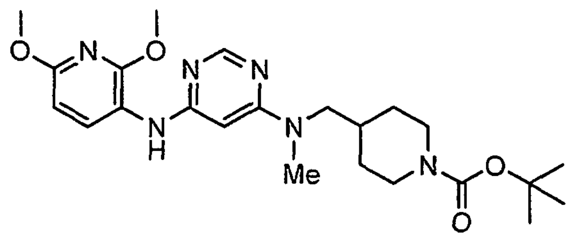 | 4-({[6-(2,6-Dimethoxy-pyridin-3-ylamino)-pyrimidin-4-yl]-methyl-amino}-methyl)-piperidine-1-carboxylic acid tert-butyl ester |

(continued)

| Example No. | Chemical Structure | Chemical Name |
|---|---|---|
| A65 | 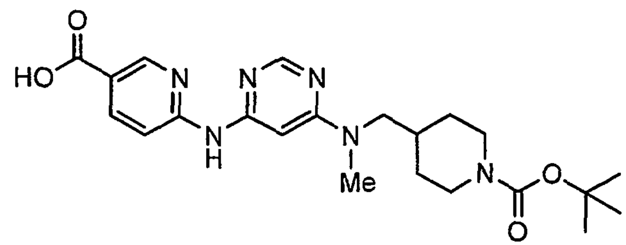 | 6-{6-[(1-tert-Butoxycarbonyl-piperidin-4-ylmethyl)-methyl-amino] -pyrimidin-4-ylamino}-nicotinic acid |
| A66 | 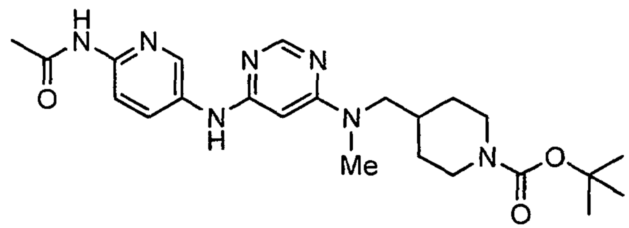 | 4-({[6-(6-Acetylamino-pyridin-3-ylamino)-pyrimidin-4-yl]-methyl-amino}-methyl)-piperidine-1-carboxylic acid tert-butyl ester |
| A67 | 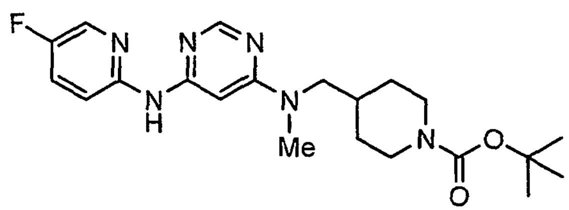 | 4-({[6-(5-Fluoro-pyridin-2-ylamino)-pyrimidin-4-yl]-methyl-amino}-methyl)-piperidine-1-carboxylic acid tert-butyl ester |
| A68 | 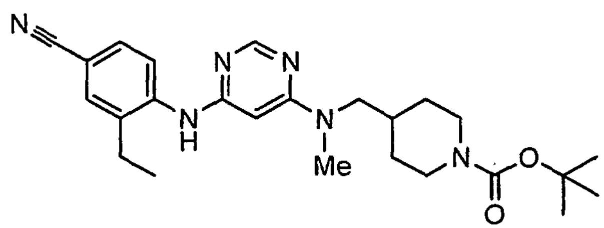 | 4-({[6-(4-Cyano-2-ethyl-phenylamino)-pyrimidin-4-yl]-methyl-amino}-methyl)-piperidine-1-carboxylic acid tert-butyl ester |

(continued)

| Example No. | Chemical Structure | Chemical Name |
|---|---|---|
| A69 | 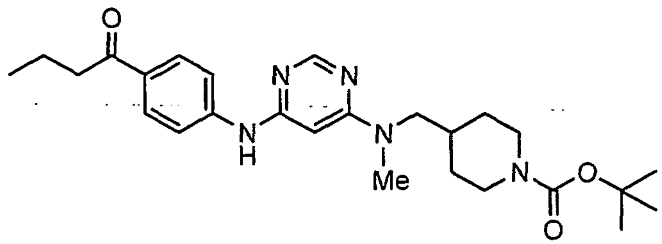 | 4-({[6-(4-Butyryl-phenylamino)-pyrimidim-4-yl]-methyl-amino}-methyl)-piperidine-1-carboxylic acid tert-butyl ester |
| A70 | 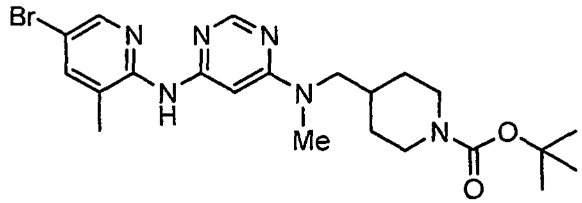 | 4-({[6-(5-Bromo-3-methyl-pyridin-2-ylamino)-pyrimidin-4-yl]-methyl-amino}-methyl)-piperidine-1-carboxylic acid tert-butyl ester |
| A71 | 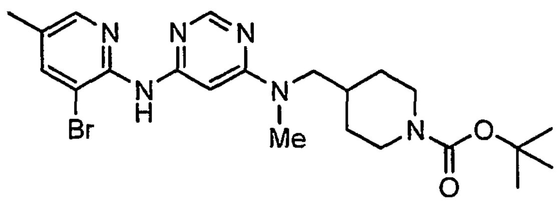 | 4-({[6-(3-Bromo-5-methyl-pyridin-2-ylamino)-pyrimidin-4-yl]-methyl-amino}-methyl)-piperidine-1-carboxylic acid tert-butyl ester |
| A72 | 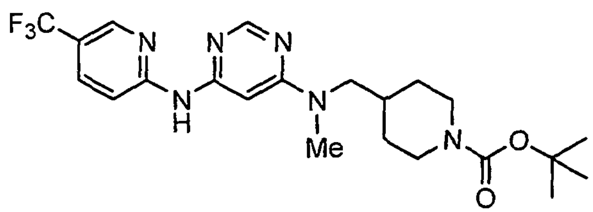 | 4-({Methyl-[6-(5-trifluoromethyl-pyridin-2-ylamino)-pyrimidin-4-yl]-amino}-methyl)-piperidine-1-carboxylic acid tert-butyl ester |

(continued)

| Example No. | Chemical Structure | Chemical Name |
|---|---|---|
| A73 | 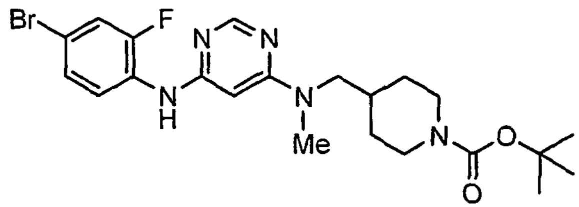 | 4-({[6-(4-Bromo-2-fluoro-phenylamino)-pyrimidin-4-yl]-methyl-amino}-methyl)-piperidine-1-carboxylic acid tert-butyl ester |
| A74 | 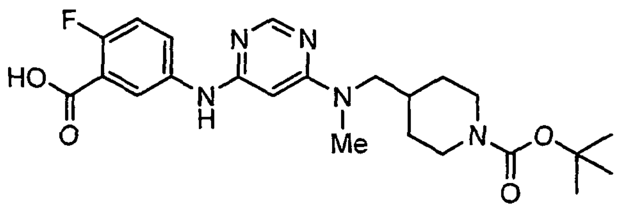 | 4-({[6-(3-Carboxy-4-fluoro-phenylamino)-pyrimidin-4-yl]-methyl-amino}-methyl)-piperidine-1-carboxylic acid tert-butyl ester |
| A75 | 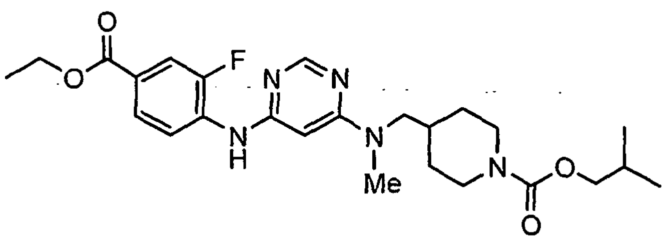 | 4-({[6-(4-Ethoxycarbonyl-2-fluoro-phenylamino)-pyrimidin-4-yl]-methyl-amino}-methyl)-piperidine-1-carboxylic acid isobutyl ester |
| A76 | 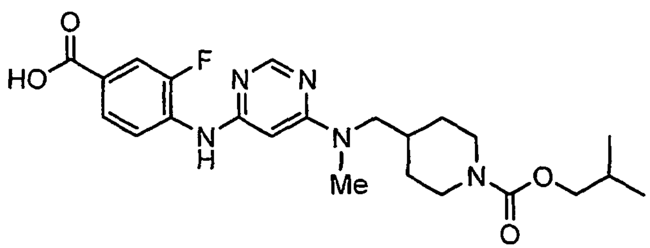 | 4-({[6-(4-Carboxy-2-fluoro-phenylamino)-pyrimidin-4-yl]-methyl-amino}-methyl)-piperidine-1-carboxylic acid isobutyl ester |

(continued)

| Example No. | Chemical Structure | Chemical Name |
|---|---|---|
| A77 | 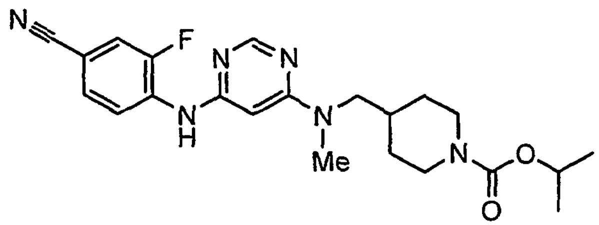 | 4-({[6-(4-Cyano-2-fluoro-phenylamino)-pyrimidin-4-yl]-methyl-amino}-methyl)-piperidine-1-carboxylic acid isopropyl ester |
| A78 | 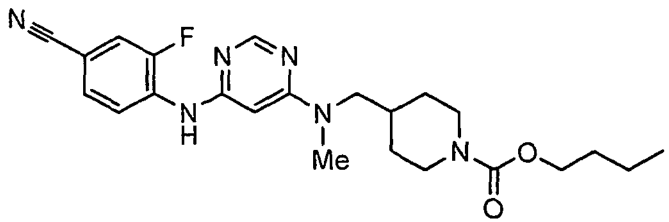 | 4-({[6-(4-Cyano-2-fluoro-phenylamino)-pyrimidin-4-yl]-methyl-amino}-methyl)-piperidine-1-carboxylic acid butyl ester |
| A79 | 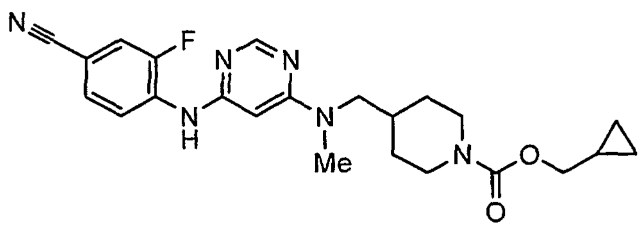 | 4-({[6-(4-Cyano-2-fluoro-phenylamino)-pyrimidin-4-yl]-methyl-amino}-methyl)-piperidine-1-carboxylic acid cyclopropylmethyl ester |
| A80 | 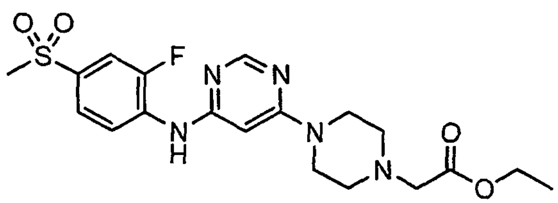 | {4-[6-(2-Fluoro-4-methanesulfonyl-phenylamino)-pyrimidin-4-yl]-piperazin-1-yl}-acetic acid ethyl ester |

(continued)

| Example No. | Chemical Structure | Chemical Name |
|---|---|---|
| A81 | 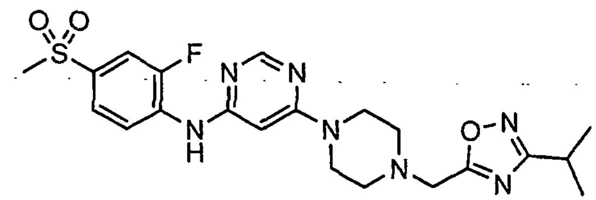 | (2-Fluoro-4-methanesulfonyl-phenyl)-{6-[4-(3-isopropyl-[1,2,4]oxadiazol-5-ylmethyl)-piperazin-1-yl]-pyrimidin-4-yl}-amine |
| A82 | 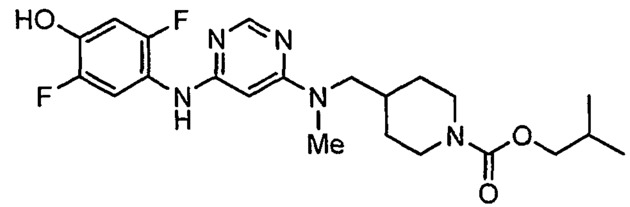 | 4-({[6-(2,5-Difluoro-4-hydroxy-phenylamino)-pyrimidin-4-yl]-methyl-amino}-methyl)-piperidine-1-carboxylic acid isobutyl ester |
| A83 | 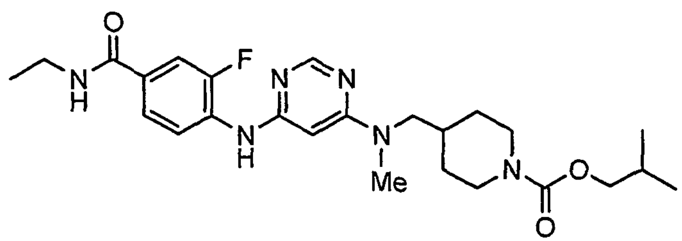 | 4-({[6-(4-Ethylcarbamoyl-2-fluoro-phenylamino)-pyrimidin-4-yl]-methyl-amino}-methyl)-piperidine-1-carboxylic acid isobutyl ester |
| A84 | 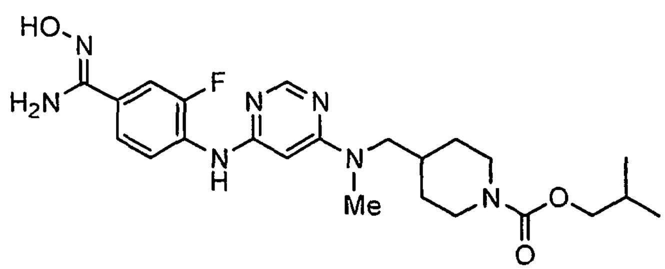 | 4-[({6-[2-Fluoro-4-(N-hydroxycarbamimidoyl)-phenylamino]-pyrimidin-4-yl}-methyl-amino)-methyl]-piperidine-1-carboxylic acid isobutyl ester |

(continued)

| Example No. | Chemical Structure | Chemical Name |
|---|---|---|
| A85 | 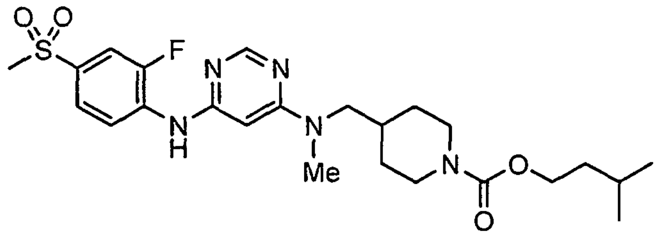 | 4-({[6-(2-Fluoro-4-methanesulfonyl-phenylamino)-pyrimidin-4-yl]-methyl-amino}-methyl)-piperidine-1-carboxylic acid 3-methyl-butyl ester |
| A86 | 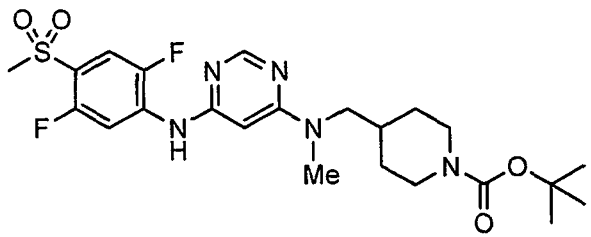 | 4-({[6-(2,5-Difluoro-4-methanesulfonyl-phenylamino)-pyrimidin-4-yl]-methyl-amino}-methyl)-piperidine-1-carboxylic acid tert-butyl ester |
| A87 | 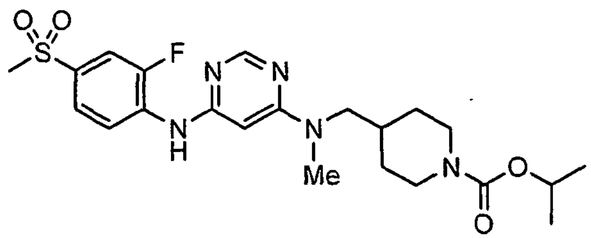 | 4-({[6-(2-Fluoro-4-methanesulfonyl-phenylamino)-pyrimidin-4-yl]-methyl-amino}-methyl)-piperidine-1-carboxylic acid isopropyl ester |
| A88 | 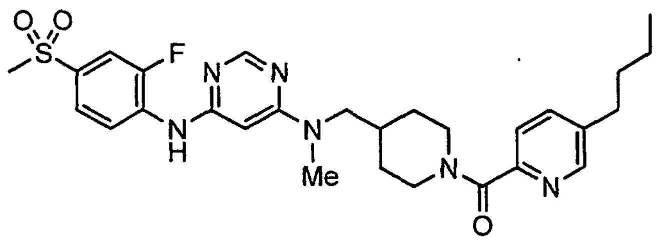 | (5-Butyl-pyridin-2-yl)-[4-({[6-(2-fluoro-4-methanesulfonyl-phenylamino)-pyrimidin-4-yl]-methyl-amino}-methyl)-piperidin-1-yl]-methanone |

(continued)

| Example No. | Chemical Structure | Chemical Name |
|---|---|---|
| A89 | 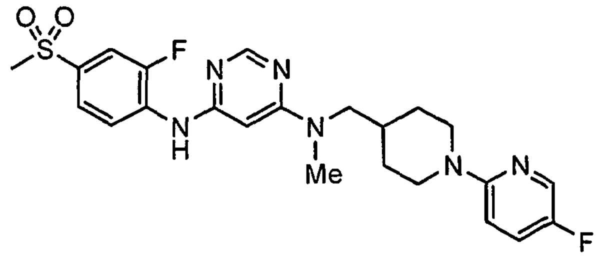 | N-(2-Fluoro-4-methanesulfonyl-phenyl)-N'-(5'-fluoro-3,4,5,6-tetrahydro-2H-[1,2'] bipyridinyl-4-ylmethyl)-N'-methyl-pyrimidine-4,6-diamine |
| A90 | 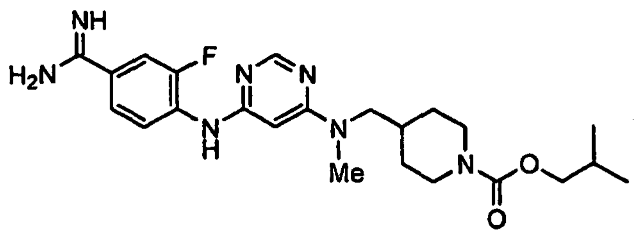 | 4-({[6-(4-Carbamimidoyl-2-fluoro-phenylamino)-pyrimidin-4-yl]-methyl-amino}-methyl)-piperidine-1-carboxylic acid isobutyl ester |
| A91 | 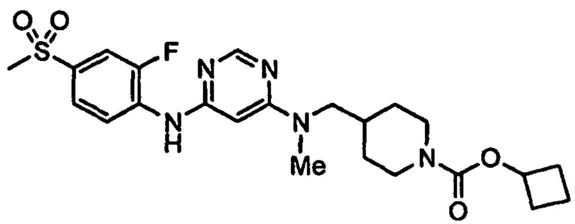 | 4-({[6-(2-Fluoro-4-methanesulfonyl-phenylamino)-pyrimidin-4-yl]-methyl-amino}-methyl)-piperidine-1-carboacylic acid cyclobutyl ester |
| A92 | 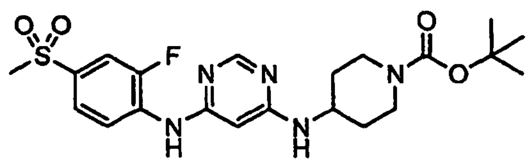 | 4-[6-(2-Fluoro-4-methanesulfonyl-phenylamino)-pyrimidin-4-ylammo]-piperidine-1-carboxylic acid tert-butyl ester |

(continued)

| Example No. | Chemical Structure | Chemical Name |
|---|---|---|
| A93 (REF) | 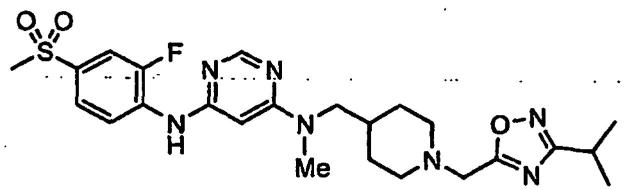 | N-(2-Fluoro-4-methanesulfonyl-phenyl)-N'-[1-(3-isopropyl-[1,2,4]oxadiazol-5-ylmethyl)-piperidin-4-ylmethyl]-N'-methyl-pyrimidine-4,6-diamine |
| A94 | 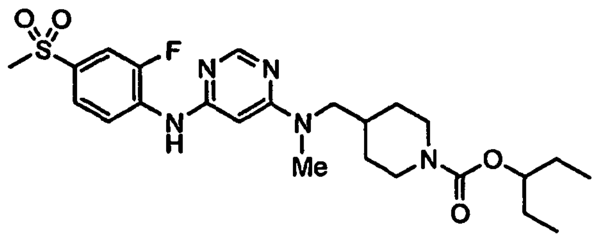 | 4-({[6-2-Fluoro-4-methanesulfonyl-phenylamino)-pyrimidin-4-yl]-methyl-amino}-methyl)-piperidine-1-carboxylic acid 1-ethyl-propyl ester |
| A95 | 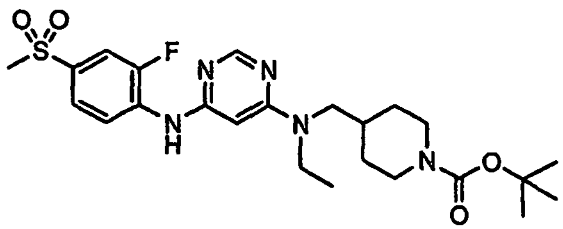 | 4-({Ethyl-[6-(2-fluoro-4-methanesulfonyl-phenylamino)-pyrimidin-4-yl]-amino}-methyl)-piperidine-1-carboxylic acid tert-butyl ester |
| A96 | 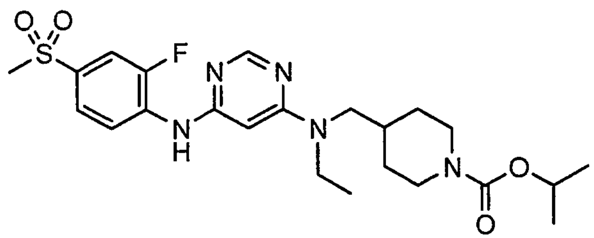 | 4-({Ethyl-[6-(2-fluoro-4-methanesulfonyl-phenylamino)-pyrimidin-4-yl]-amino }-methyl)-piperidine-1-carboxylic acid isopropyl ester |

(continued)

| Example No. | Chemical Structure | Chemical Name |
|---|---|---|
| A97 | 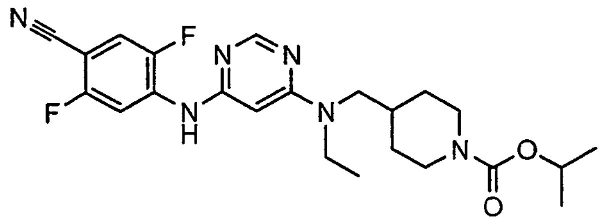 | 4-({[6-(4-Cyano-2,5-difluoro-phenylamino)-pyrimidin-4-yl]-ethyl-amino -methyl)-piperidine-1-carboxylic acid isopropyl ester |
| A98 | 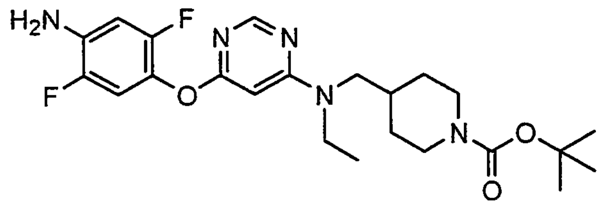 | 4-({[6-(4-Amino-2,5-difluoro-phenoxy)-pyrimidin-4-yl]-ethyl-amino}-methyl)-piperidine-1-carboxylic acid tert-butyl ester |
| A99 | 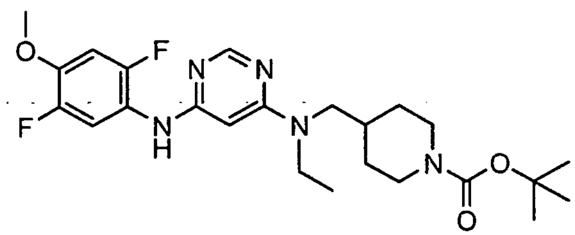 | 4-({[6-(2,5-Difluoro-4-methoxy-phenylamino)-pyrimidin-4-yl]-ethyl-amino}-methyl)-piperidine-1-carboxylic acid tert-butyl ester |
| A100 | 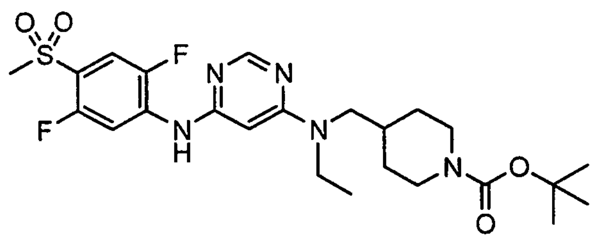 | 4-({[6-(2,5-Difluoro-4-methanesulfonyl-phenylamino)-pyrimidin-4-yl]-ethyl-amino}-methyl)-piperidine-1-carboxylic acid tert-butyl ester |

(continued)

| Example No. | Chemical Structure | Chemical Name |
| --- | --- | --- |
| A101 | 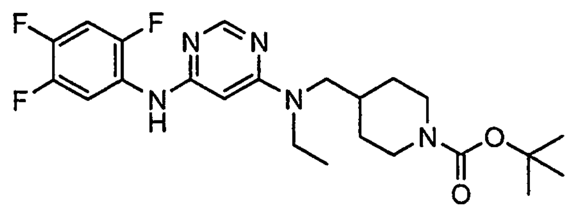 | 4-({Ethyl-[6-(2,4,5-trifluoro-phenylamino)-pyrimidin-4-yl]-amino}-methyl)-piperidine-1-carboxylic acid tert-butyl ester |
| A102 | 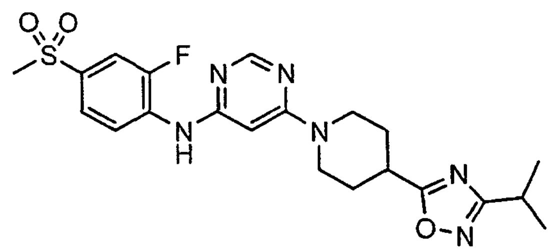 | (2-Fluoro-4-methanesulfonyl-phenyl)-{6-[4-(3-isopropyl-[1,2,4]oxadiazol-5-yl)-piperidin-1-yl]-pyrimidin-4-yl-amine |
| A103 | 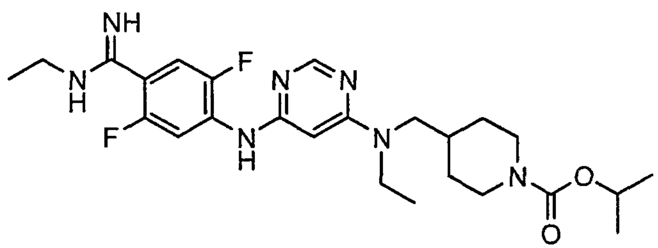 | 4-[(Ethyl-{6-[4-(N-ethylcarbamimidoyl)-2,5-difluoro-phenylamino]-pyrimidin-4-yl}-amino)-methyl]-piperidine-1-carboxylic acid isopropyl ester |
| A104 | 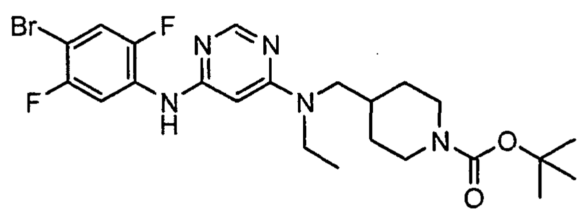 | 4-({[6-(4-Bromo-2,5-difluoro-phenylamino)-pyrimidin-4-yl]-ethyl-amino}-methyl)-piperidine-1-carboxylicacid tert-butyl ester |

(continued)

| Example No. | Chemical Structure | Chemical Name |
|---|---|---|
| A105 | 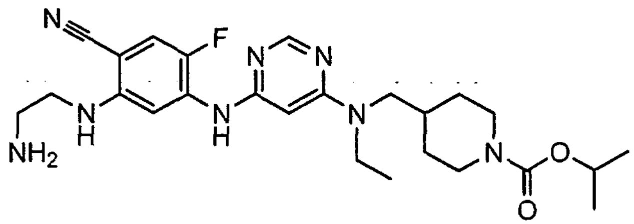 | 4-[({6-[5-(2-Amino-ethylamino)-4-cyano-2-fluoro-phenylamino]-pyrimidin-4-yl}-ethyl-amino)-methyl]-piperidine-1-carboxylic acid isopropyl ester |
| A106 | 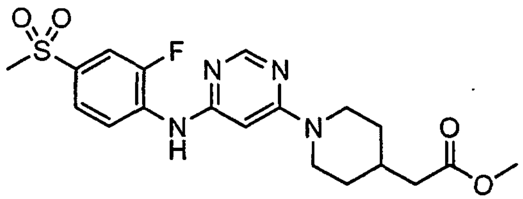 | {1-[6-(2-Fluoro-4-methanesulfonyl-phenylamino)-pyrimidin-4-yl]-piperidin-4-yl}-acetic acid methyl ester |
| A107 | 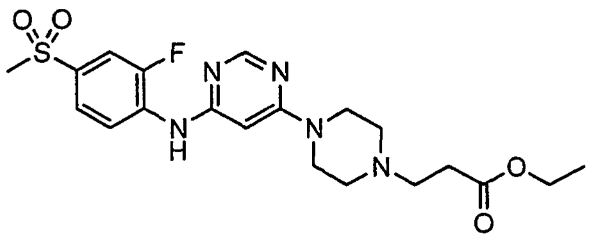 | 3-{4-[6-(2-Fluoro-4-methanesulfonyl-phenylamino)-pyrimidin-4-yl]-piperazin-1-yl}-propionic acid ethyl ester |
| A108 | 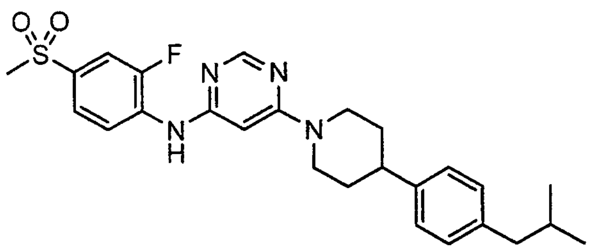 | (2-Fluoro-4-methanesulfonyl-phenyl)-{6-[4-(4-isobutyl-phenyl)-piperidin-1-yl]-pyrimidin-4-yl}-amine |

(continued)

| Example No. | Chemical Structure | Chemical Name |
|---|---|---|
| A109 | 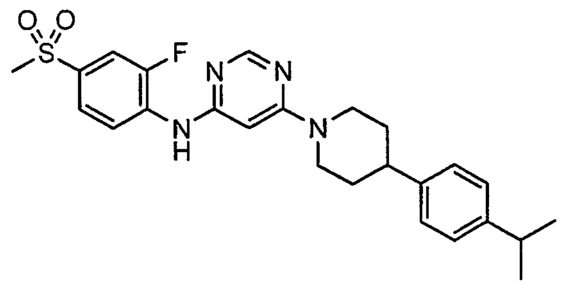 | (2-Fluoro-4-methanesulfonyl-phenyl)-{6-[4-(4-isopropyl-phenyl)-piperidin-1-yl]-pyrimidin-4-yl}-amine |
| A110 | 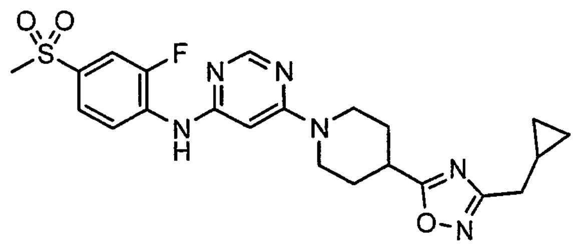 | {6-[4-(3-Cyclopropylmethyl-[1,2,4]oxadiazol-5-yl)-piperidin-1-yl]-pyrimidin-4-yl}-(2-fluoro-4-methanesulfonyl-phenyl)-amine |
| A111 | 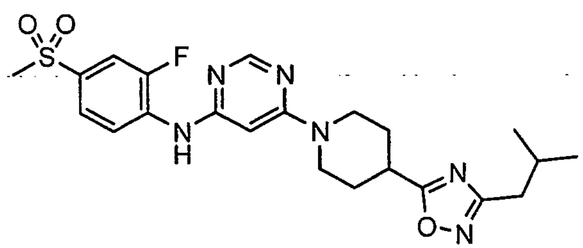 | (2-Fluoro-4-methanesulfonyl-phenyl)-{6-(4-(3-isobutyl-[1,2,4]oxadiazol-5-yl)-piperidin-1-yl]-pyrimidin-4-yl}-amine |

(continued)

| Example No. | Chemical Structure | Chemical Name |
|---|---|---|
| A112 | 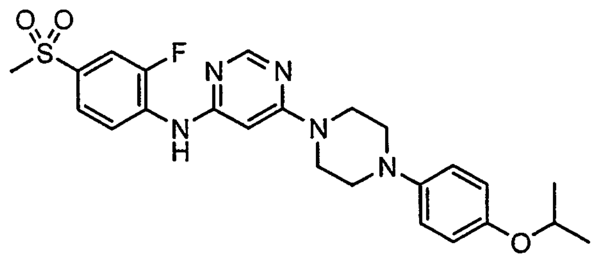 | (2-Fluoro-4-methanesulfonyl-phenyl)-{6-[4-(4-isopropoxy-phenyl)-piperazin-1-yl]-pyrimidin-4-yl}-amine |
| A113 | 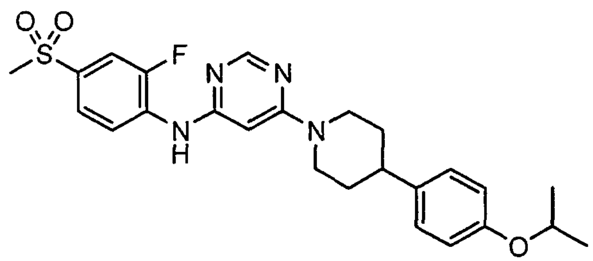 | (2-Fluoro-4-methanesulfonyl-phenyl)-{6-[4-(4-isopropoxy-phenyl)-piperidin-1-yl]-pyrimidin-4-yl}-amine |
| A114 | 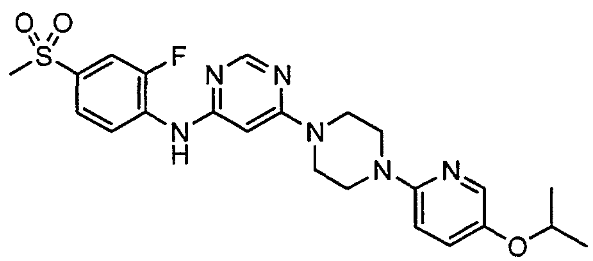 | (2-Fluoro-4-methanesulfonyl-phenyl)-{6-[4-(5-isopropoxy-pyridin-2-yl)-piperazin-1-yl]-pyrimidin-4-yl}-amine |

(continued)

| Example No. | Chemical Structure | Chemical Name |
|---|---|---|
| A115 | 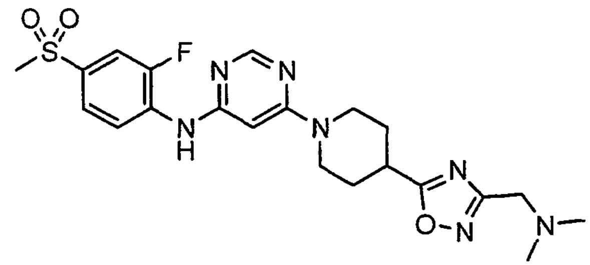 | {6-[4-(3-Dimethylaminomethyl-[1,2,4]oxadiazol-5-yl)-piperidin-1-yl]-pyrimidin-4-yl}-(2-fluoro-4-methanesulfonyl-phenyl)-amine |
| A116 | 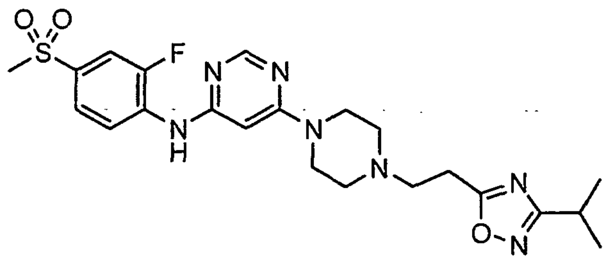 | (2-Fluoro-4-methanesulfonyl-phenyl)-(6-{4-[2-(3-isopropyl-[1,2,4]oxadiazol-5-yl)-ethyl]-piperazin-1-yl}-pyrimidin-4-yl)-amine |
| A117 | 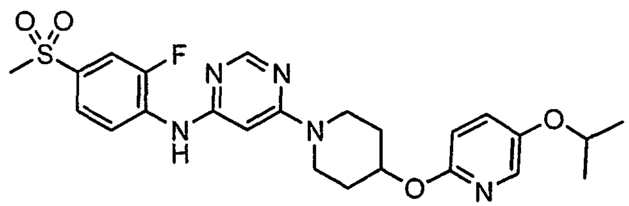 | (2-Fluoro-4-methanesulfonyl-phenyl)-{6-[4-(5-isopropoxy-pyridin-2-yloxy)-piperidin-1-yl]-pyrimidin-4-yl}-amine |
| A118 | 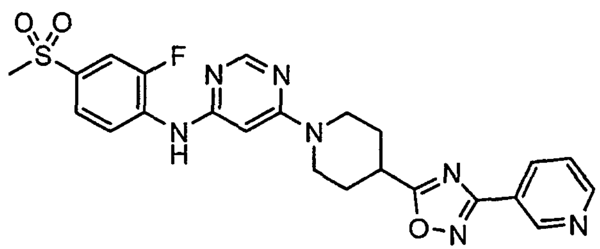 | (2-Fluoro-4-methanesulfonyl-phenyl)-{6-[4-(3-pyridin-3-yl-[1,2,4]oxadiazol-5-yl)-piperidin-1-yl]-pyrimidin-4-yl}-amine |

(continued)

| Example No. | Chemical Structure | Chemical Name |
|---|---|---|
| A119 | 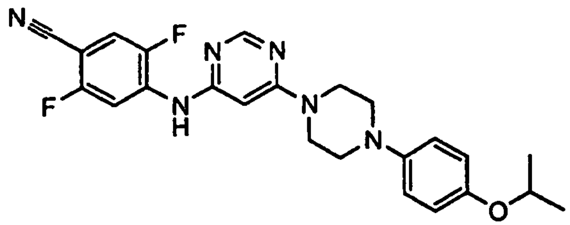 | 2,5-Difluoro-4-{6-[4-(4-isopropoxy-phenyl)-piperazin-1-yl]-pyrimidin-4-ylamino}-benzonitrile |
| A120 | 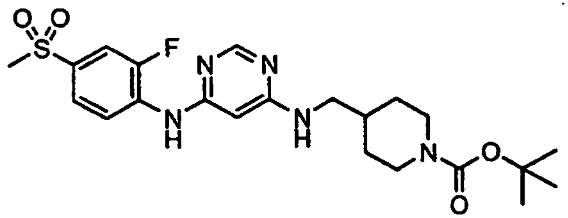 | 4-{[6-(2-Fluoro-4-methanesulfonyl-phenylamino)-pyrimidin-4-ylamino]-methyl}-piperidine-1-carboxylic acid tert-butyl ester |
| A121 | 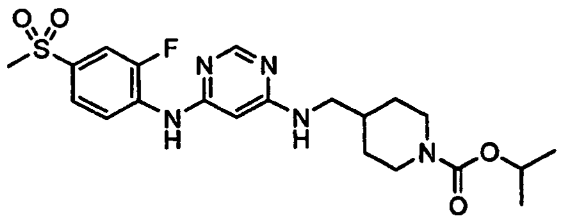 | 4-{[6-(2-Fluoro-4-methanesulfonyl-phenylamino)-pyrimidin-4-ylamino]-methyl}-piperidine-1-carboxylic acid isopropyl ester |
| A122 | 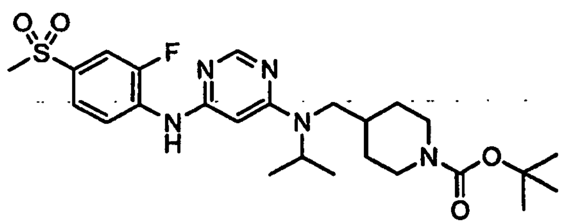 | 4-({[6-(2-Fluoro-4-methanesulfonyl-phenylamino)-pyrimidin-4-yl]-isopropyl-amino}-methyl)-piperidine-1-carboxylic acid tert-butyl ester |

(continued)

| Example No. | Chemical Structure | Chemical Name |
|---|---|---|
| A123 | 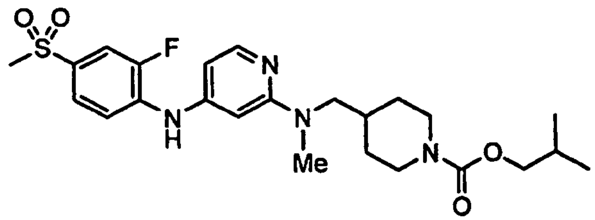 | 4-({[4-(2-Fluoro-4-methanesulfonyl-phenylamino)-pyridin-2-yl]-methyl-amino}-methyl)-piperidine-1-carboxytic acid isobutyl ester |
| A124 (REF) | 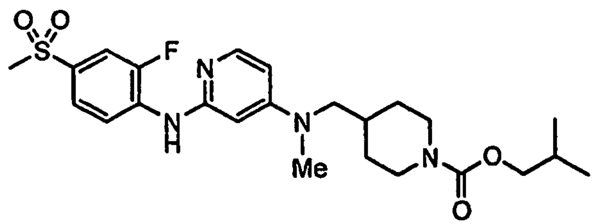 | 4-({[2-(2-Fluoro-4-methanesulfonyl-phenylamino)-pyridin-4-yl]-methyl-amino}-methyl)-piperidine-1-carboxylic acid isobutyl ester |

**TABLE B**

| Example No. | Chemical Structure | Chemical Name |
|---|---|---|
| B1 | 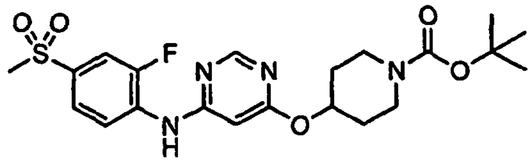 | 4-[6-(2-Fluoro-4-methanesulfonyl-phenylamino)-pyrimidin-4-yloxy]-piperidine-1-carboxylic acid tert-butyl ester |
| B2 (REF) | 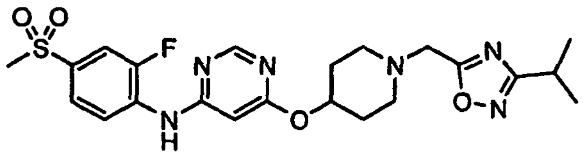 | (2-Fluoro-4-methanesulfonyl-phenyl)-{6-[1-(3-isopropyl-[1,2,4]oxadiazol-5-ylmethyl)-piperidin-4-yloxy]-pyrimidin-4-yl}-amine |
| B3 | 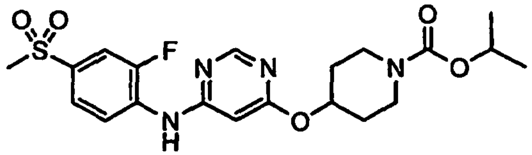 | 4-[6-(2-Fluoro-4-methanesulfonyl-phenylamino)-pyrimidin-4-yloxy]-piperidine-1-carboxylic acid isopropyl ester |
| B4 | 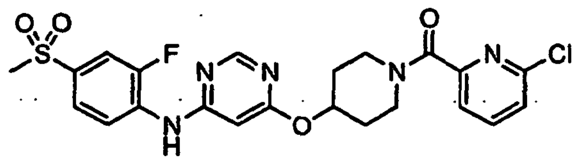 | (6-Chloro-pyridin-2-yl)-{4-[6-(2-fluoro-4-methanesulfonyl-phenylamino)-pyrimidin-4-yloxy]-piperidin-1-yl}-methanone |
| B5 | 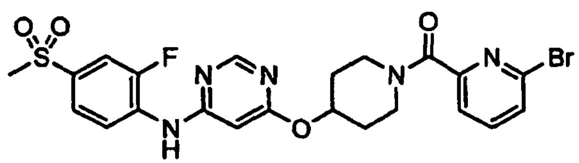 | (6-Bromo-pyridin-2-yl)-{4-[6-(2-fluoro-4-methanesulfonyl-phenylamino)-pyrimidin-4-yloxy]-piperidin-1-yl}-methanone |

(continued)

| Example No. | Chemical Structure | Chemical Name |
|---|---|---|
| B6 | 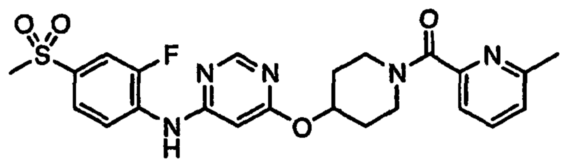 | {4-[6-(2-Fluoro-4-methanesulfonyl-phenylamino)-pyrimidin-4-yloxy]-piperidin-1-yl}-(6-methyl-pyridin-2-yl)-methanone |
| B7 | 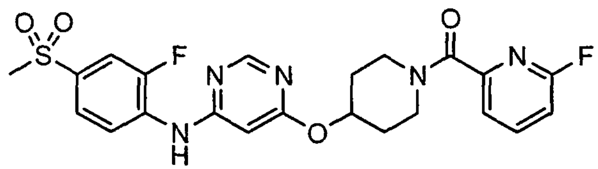 | {4-[6-(2-Fluoro-4-methanesulfonyl-phenylamino)-pyrimidin-4-yloxy]-piperidin-1-yl}-(6-fluoro-pyridin-2-yl)-methanone |
| B8 | 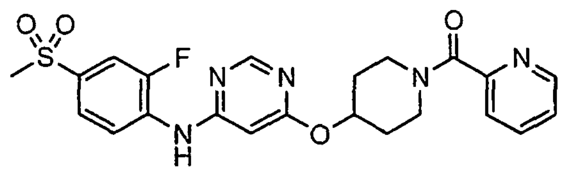 | {4-[6-(2-Fluoro-4-methanesulfonyl-phenylamino)-pyrimidin-4-yloxy]-piperidin-1-yl}-pyridin-2-yl-methanone |
| B9 | 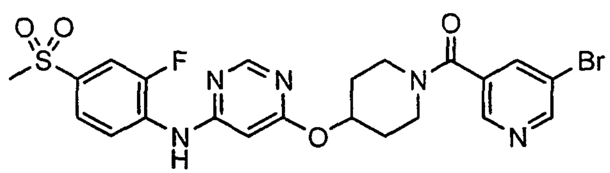 | (5-Bromo-pyridin-3-yl)-{4-[6-(2-fluoro-4-methanesulfonyl-phenylamino)-pyrimidin-4-yloxy]-piperidin-1-yl}-methanone |
| B10 | 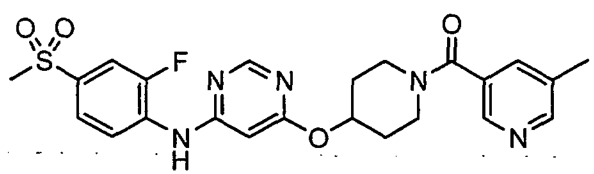 | {4-[6-(2-Fluoro-4-methanesulfonyl-phenylamino)-pyrimidin-4-yloxy]-piperidin-1-yl}-(5-methyl-pyridin-3-yl)-methanone |

(continued)

| Example No. | Chemical Structure | Chemical Name |
|---|---|---|
| B11 | 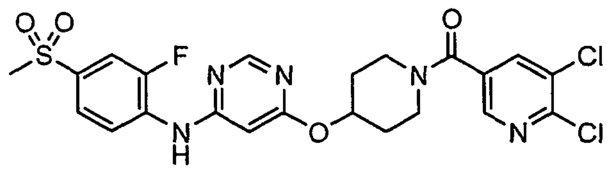 | (5,6-Dichloro-pyridin-3-yl)-{4-[6-(2-fluoro-4-methanesulfonyl-phenylamino)-pyrimidin-4-yloxy]-piperidin-1-yl}-methanone |
| B12 | 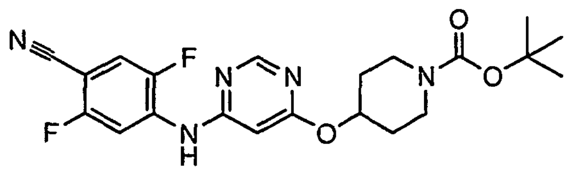 | 4-[6-(4-Cyano-2,5-difluoro-phenylamino)-pyrimidin-4-yloxy]-piperidine-1-carboxylic acid tert-butyl ester |
| B13 | 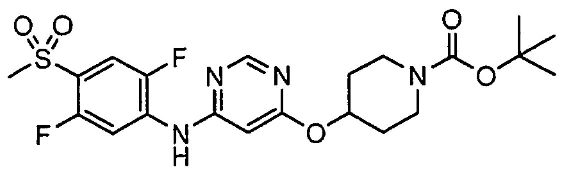 | 4-[6-(2,5-Difluoro-4-methanesulfonyl-phenylamino)-pyrimidin-4-yloxy]-piperidine-1-carboxylic acid tert-butyl ester |
| B14 | 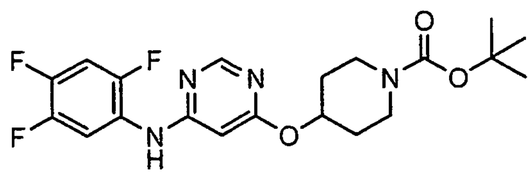 | 4-[6-(2,4,5-Trifluoro-phenylamino)-pyrimidin-4-yloxy]-piperidine-1-carboxylic acid tert-butyl ester |
| B15 | 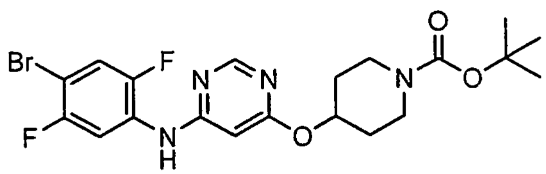 | 4-[6-(4-Bromo-2,5-difluoro-phenylamino)-pyrimidin-4-yloxy]-piperidine-1-carboxylic acid tert-butyl ester |

(continued)

| Example No. | Chemical Structure | Chemical Name |
|---|---|---|
| B16 | 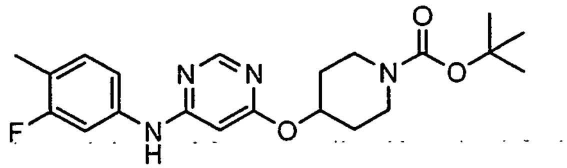 | 4-[6-(3-Fluoro-4-methyl-phenylamino)-pyrimidin-4-yloxy]-piperidine-1-carboxylic acid tert-butyl ester |
| B17 | 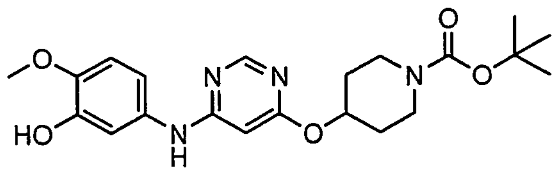 | 4-[6-(3-Hydroxy-4-methoxy-phenylamino)-pyrimidin-4-yloxy]-piperidine-1-carboxylic acid tert-butyl ester |
| B18 | 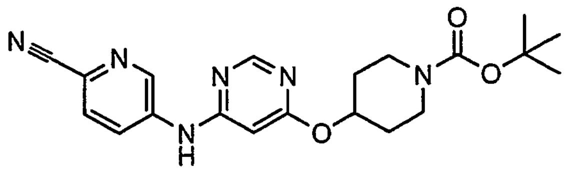 | 4-[6-(6-Cyano-pyridin-3-ylamino)-pyrimidin-4-yloxy]-piperidine-1-carboxylic acid tert-butyl ester |
| B19 | 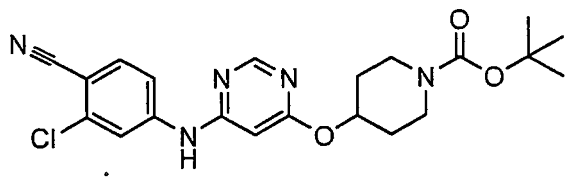 | 4-[6-(3-Chloro-4-cyano-phenylamino)-pyrimidin-4-yloxy]-piperidine-1-carboxylic acid tert-butyl ester |
| B20 | 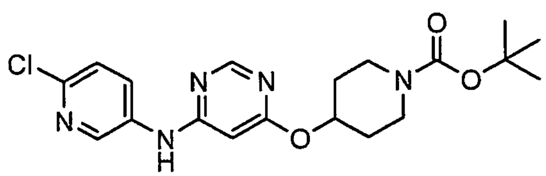 | 4-[6-(6-Chloro-pyridin-3-ylamino)-pyrimidin-4-yloxy]-piperidine-1-carboxylic acid tert-butyl ester |

(continued)

| Example No. | Chemical Structure | Chemical Name |
|---|---|---|
| B21 | 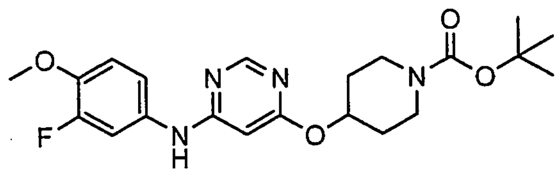 | 4-[6-(3-Fluoro-4-methoxy-phenylamino)-pyrimidin-4-yloxy]-piperidine-1-carboxylic acid tert-butyl ester |
| B22 | 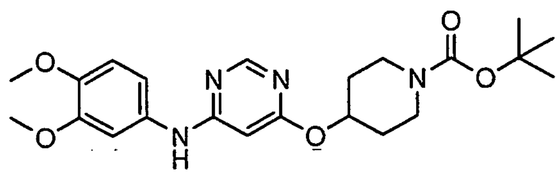 | 4-[6-(3,4-Dimethoxy-phenylamino)-pyrimidin-4-yloxy]-piperidine-1-carboxylic acid tert-butyl ester |
| B23 | 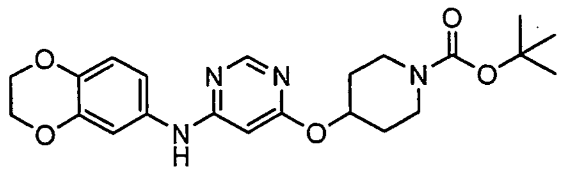 | 4-[6-(2,3-Dihydro-benzo[1,4]dioxin-6-ylamino)-pyrimidin-4-yloxy]-piperidine-1-carboxylic acid tert-butyl ester |
| B24 | 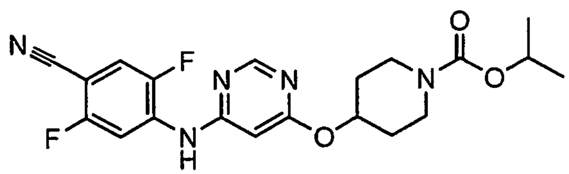 | 4-[6-(4-Cyano-2,5-difluoro-phenylamino)-pyrimidin-4-yloxy]-piperidine-1-carboxylic acid isopropyl ester |
| B25 | 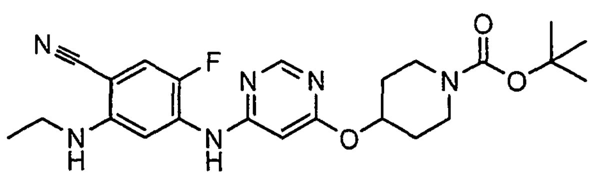 | 4-[6-(4-Cyano-5-ethylamino-2-fluoro-phenylamino)-pyrimidin-4-yloxy]-piperidine-1-carboxylic acid tert-butyl ester |

(continued)

| Example No. | Chemical Structure | Chemical Name |
|---|---|---|
| B26 | 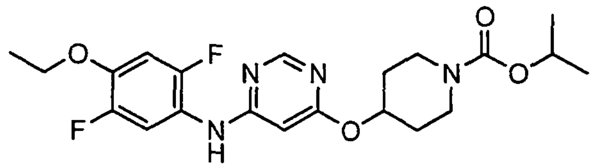 | 4-[6-(4-Ethoxy-2,5-difluoro-phenylamino)-pyrimidin-4-yloxy]-piperidine-1-carboxylic acid isopropyl ester |
| B27 | 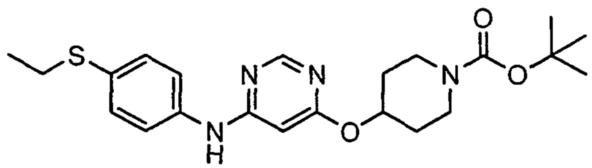 | 4-[6-(4-Ethylsulfanyl-phenylamino)-pyrimidin-4-yloxy]-piperidine-1-carboaylic acid tert-butyl ester |
| B28 | 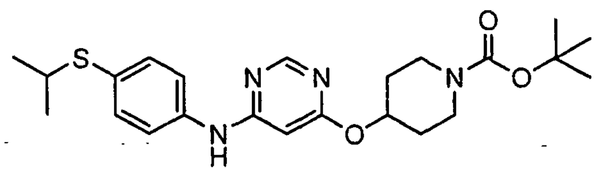 | 4-[6-(4-Isopropylsulfanyl-phenylamino)-pyrimidin-4-yloxy]-piperidine-1-carboxylic acid tert-butyl ester |
| B29 | 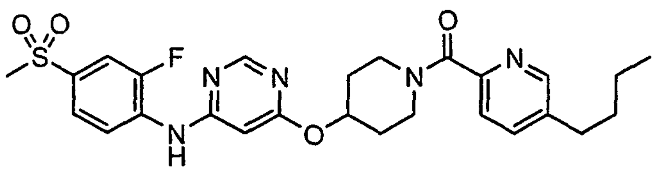 | (5-Butyl-pyridin-2-yl)-{4-[6-(2-fluoro-4-methanesulfonyl-phenylamino)-pyrimidin-4-yloxy]-piperidin-1-yl}-methanone |
| B30 | 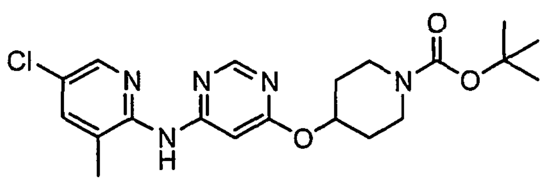 | 4-[6-(5-Chloro-3-methyl-pyridin-2-ylamino)-pyrimidin-4-yloxy]-piperidine-1-carboxylic acid tert-butyl ester |

(continued)

| Example No. | Chemical Structure | Chemical Name |
|---|---|---|
| B31 | 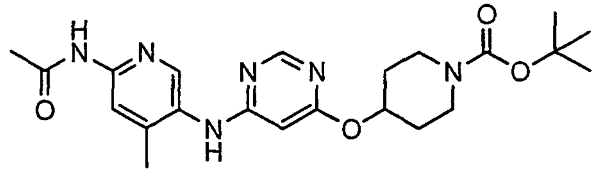 | 4-[6-(6-Acetylamino-4-methyl-pyridin-3-ylamino)-pyrimidin-4-yloxy]-piperidine-1-carboxylic acid tert-butyl ester |
| B32 | 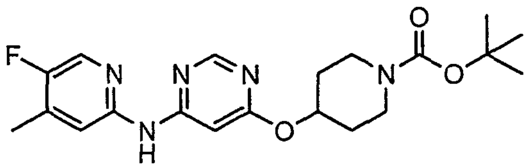 | 4-[6-(5-Fluoro-4-methyl-pyridin-2-ylamino)-pyrimidin-4-yloxy]-piperidine-1-carboxylic acid tert-butyl ester |
| B33 | 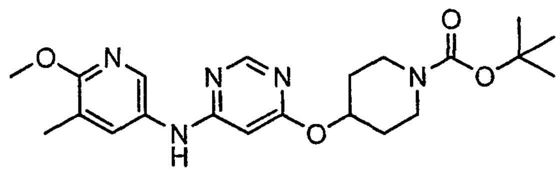 | 4-[6-(6-Methoxy-5-methyl-pyridin-3-ylamino)-pyrimidin-4-yloxy]-piperidine-1-carboxylic acid tert-butyl ester |
| B34 | 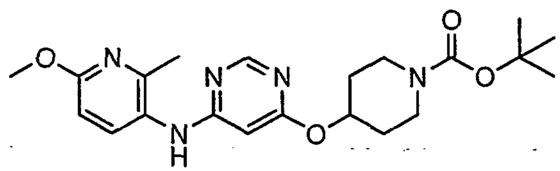 | 4-[6-(6-Methoxy-2-methyl-pyridin-3-ylamino)-pyrimidin-4-yloxy]-piperidine-1-carboxylic acid tert-butyl ester |

(continued)

| Example No. | Chemical Structure | Chemical Name |
| --- | --- | --- |
| B35 | 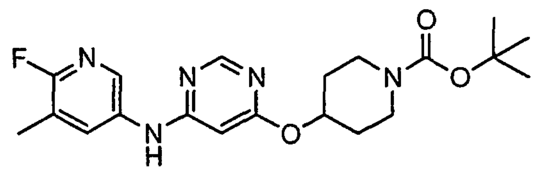 | 4-[6-(6-Fluoro-5-methyl-pyridin-3-ylamino)-pyrimidin-4-yloxy]-piperidine-1-carboxylic acid tert-butyl ester |
| B36 | 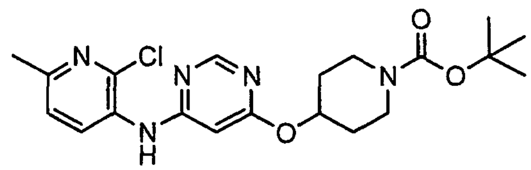 | 4-[6-(2-Chloro-6-methyl-pyridin-3-ylamino)-pyrimidin-4-yloxy]-piperidine-1-carboxylic acid tert-butyl ester |
| B37 | 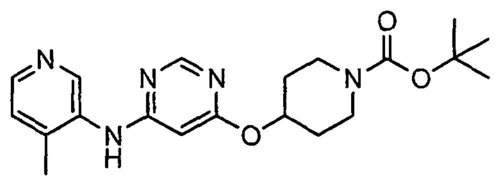 | 4-[6-(4-Methyl-pyridin-3-ylamino)-pyrimidin-4-yloxy]-piperidine-1-carboxylic acid tert-butyl ester |
| B38 | 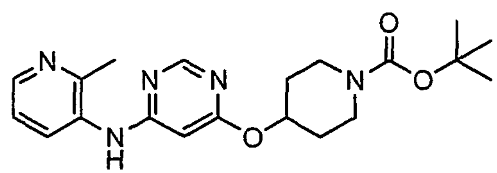 | 4-[6-(2-Methyl-pyridin-3-ylamino)-pyrimidin-4-yloxy]-piperidine-1-carboxylic acid tert-butyl ester |
| B39 | 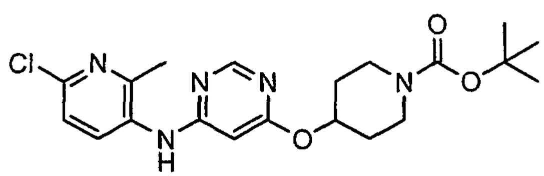 | 4-[6-(6-Chloro-2-methyl-pyridin-3-ylamino)-pyrimidin-4-yloxy]-piperidine-1-carboxylic acid tert-butyl ester |

(continued)

| Example No. | Chemical Structure | Chemical Name |
|---|---|---|
| B40 | 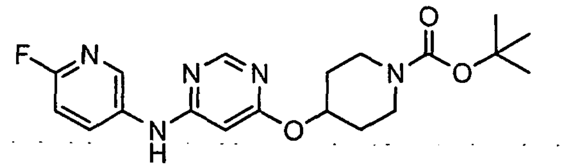 | 4-[6-(6-Fluoro-pyridin-3-ylamino)-pyrimidin-4-yloxy]-piperidine-1-carboxylic acid tert-butyl ester |
| B41 | 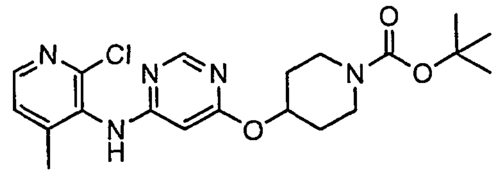 | 4-[6-(2-Chloro-4-methyl-pyridin-3-ylamino)-pyrimidin-4-yloxy]-piperidine-1-carboxylic acid tert-butyl ester |
| B42 | 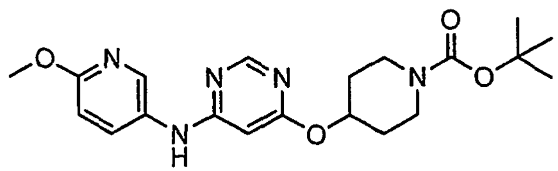 | 4-[6-(6-Methoxy-pyridin-3-ylamino)-pyrimidin-4-yloxy]-piperidine-1-carboxylic acid tert-butyl ester |
| B43 | 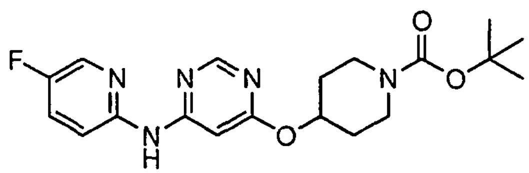 | 4-[6-(5-Fluoro-pyridin-2-ylamino)-pyrimidin-4-yloxy]-piperidine-1-carboxylic acid tert-butyl ester |
| B44 | 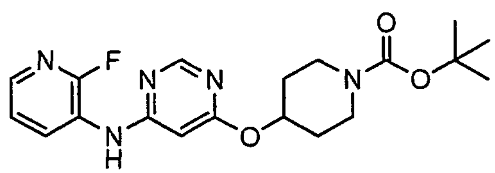 | 4-[6-(2-Fluoro-pyridin-3-ylamino)-pyrimidin-4-yloxy]-piperidine-1-carboxylic acid tert-butyl ester |

(continued)

| Example No. | Chemical Structure | Chemical Name |
|---|---|---|
| B45 | 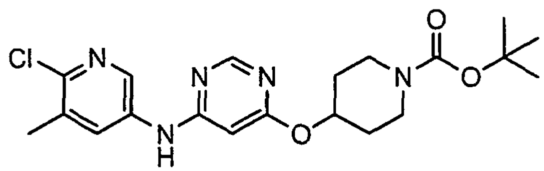 | 4-[6-(6-Chloro-5-methyl-pyridin-3-ylamino)-pyrimidin-4-yloxy]-piperidine-1-carboxylic acid tert-butyl ester |
| B46 | 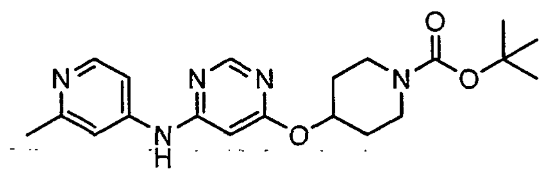 | 4-[6-(2-Methyl-pyridin-4-ylamino)-pyrimidin-4-yloxy]-piperidine-1-carboxylic acid tert-butyl ester |
| B47 | 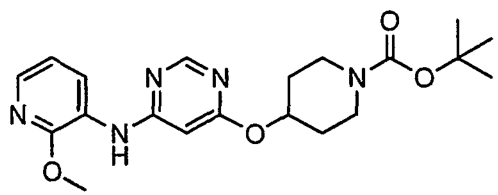 | 4-[6-(2-Methoxy-pyridin-3-ylamino)-pyrimidin-4-yloxy]-piperidine-1-carboxylic acid tert-butyl ester |
| B48 | 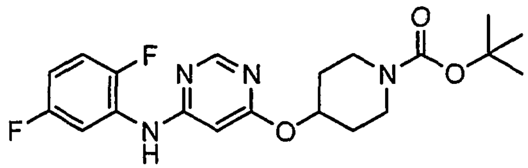 | 4-[6-(2,5-Difluoro-phenylamino)-pyrimidin-4-yloxy]-piperidine-1-carboxylic acid tert-butyl ester |

(continued)

| Example No. | Chemical Structure | Chemical Name |
|---|---|---|
| B49 | 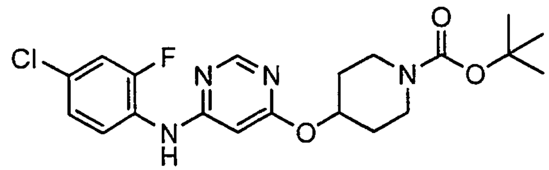 | 4-[6-(4-Chloro-2-fluoro-phenylamino)-pyrimidin-4-yloxy]-piperidine-1-carboxylic acid tert-butyl ester |
| B50 | 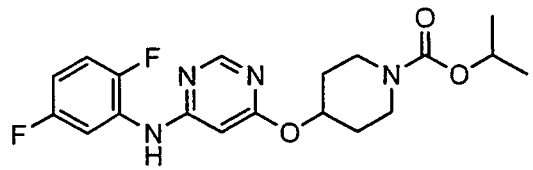 | 4-[6-(2,5-Difluoro-phenylamino)-pyrimidin-4-yloxy]-piperidine-1-carboxylic acid isopropyl ester |
| B51 | 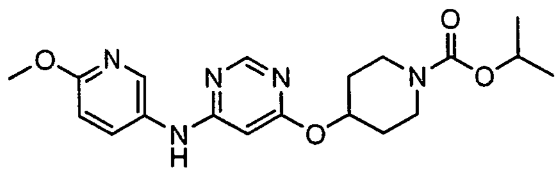 | 4-[6-(6-Methoxy-pyridin-3-ylamino)-pyrimidin-4-yloxy]-piperidine-1-carboxylic acid isopropyl ester |
| B52 | 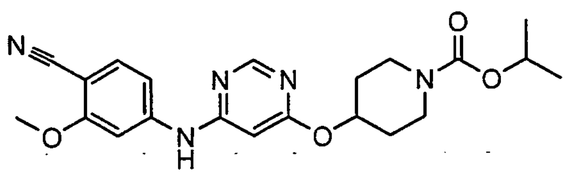 | 4-[6-(4-Cyano-3-methoxy-phenylamino)-pyrimidin-4-yloxy]-piperidine-1-carboxylic acid isopropyl ester |
| B53 | 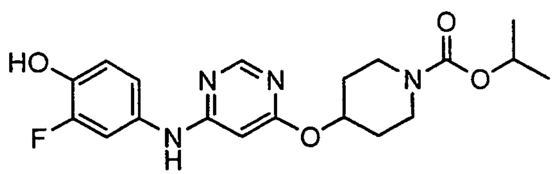 | 4-[6-(3-Fluoro-4-hydroxy-phenylamino)-pyrimidin-4-yloxy]-piperidine-1-carboxylic acid isopropyl ester |

(continued)

| Example No. | Chemical Structure | Chemical Name |
|---|---|---|
| B54 | 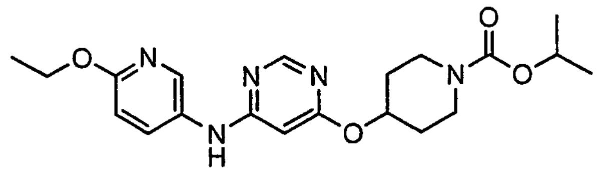 | 4-[6-(6-Ethoxy-pyridin-3-ylamino)-pyrimidin-4-yloxy]-piperidine-1-carboxylic acid isopropyl ester |
| B55 | 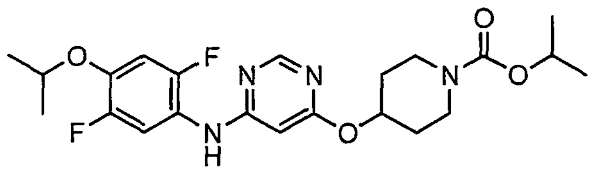 | 4-[6-(2,5-Difluoro-4-isopropoxy-phenylamino)-pyrimidin-4-yloxy]-piperidine-1-carboxylic acid isopropyl ester |
| B56 | 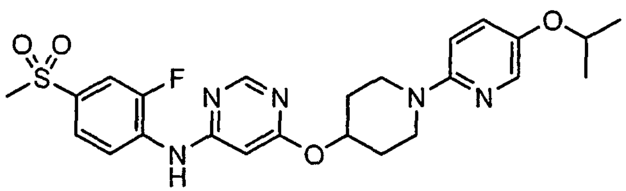 | (2-Fluoro-4-methanesulfonyl-phenyl)-[6-(5'-isopropoxy-3,4,5,6-tetrahydro-2H-[1,2']bipyridinyl-4-yloxy)-pyrimidin-4-yl]-amine |
| B57 | 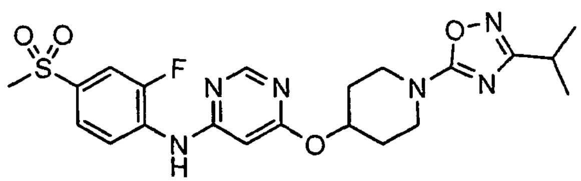 | (2-Fluoro-4-methanesulfonyl-phenyl)-{6-[1-(3-isopropyl-[1,2,4]oxadiazol-5-yl)-piperidin-4-yloxy]-pyrimidin-4-yl}-amine |

(continued)

| Example No. | Chemical Structure | Chemical Name |
|---|---|---|
| B58 | 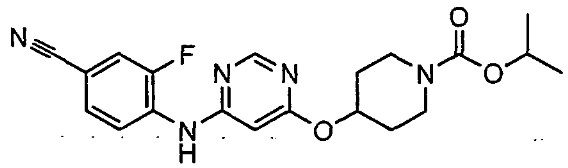 | 4-[6-(4-Cyano-2-fluoro-phenylamino)-pyrimidin-4-yloxy]-piperidine-1-carboxylic acid isopropyl ester |
| B59 | 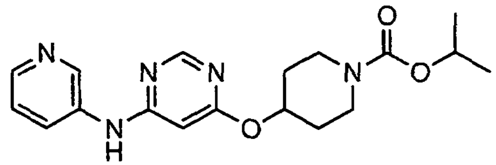 | 4-[6-(Pyridin-3-ylamino)-pyrimidin-4-yloxy]-piperidine-1-carboxylic acid isopropyl ester |
| B60 | 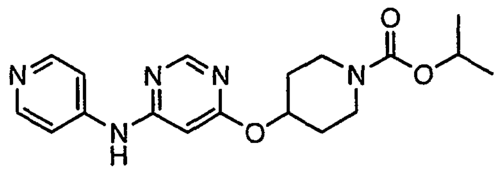 | 4-[6-(Pyridin-4-ylamino)-pyrimidin-4-yloxy]-piperidine-1-carboxylic acid isopropyl ester |
| B61 | 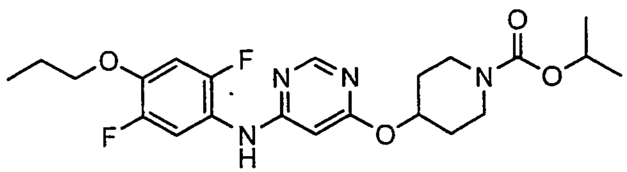 | 4-[6-(2,5-Difluoro-4-propoxy-phenylamino)-pyrimidin-4-yloxy]-piperidine-1-carboxylic acid isopropyl ester |
| B62 | 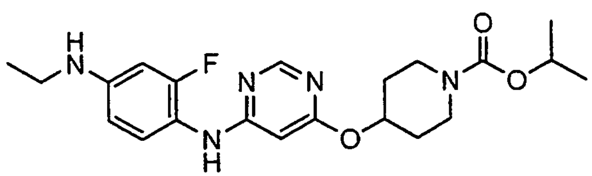 | 4-[6-(4-Ethylamino-2-fluoro-phenylamino)-pyrimidin-4-yloxy]-piperidine-1-carboxylic acid isopropyl ester |

(continued)

| Example No. | Chemical Structure | Chemical Name |
|---|---|---|
| B63 | 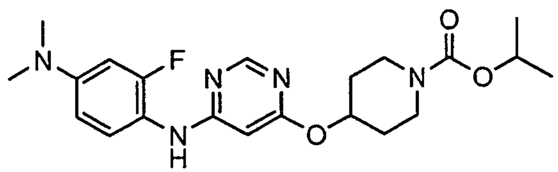 | 4-[6-(4-Dimethylamino-2-fluoro-phenylamino)-pyrimidin-4-yloxy]-piperidine-1-carboxylic acid isopropyl ester |
| B64 | 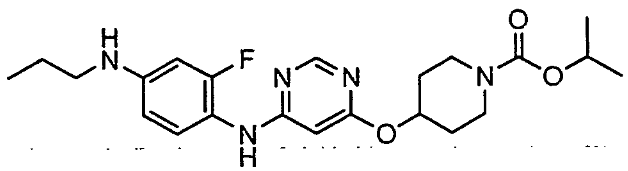 | 4-[6-(2-Fluoro-4-propylamino-phenylamino)-pyrimidin-4-yloxy]-piperidine-1-carboxylic acid isopropyl ester |
| B65 | 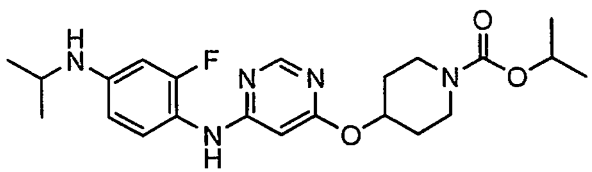 | 4-[6-(2-Fluoro-4-isopropylamino-phenylamino)-pyrimidin-4-yloxy]-piperidine-1-carboxylic acid isopropyl ester |
| B66 | 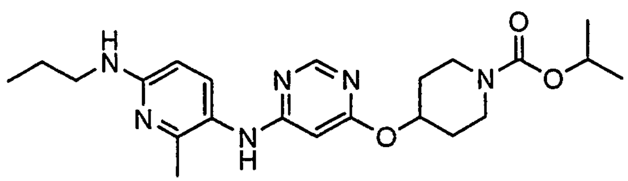 | 4-[6-(2-Methyl-6-propylamino-pyridin-3-ylamino)-pyrimidin-4-yloxy]-piperidine-1-carboaylic acid isopropyl ester |
| B67 | 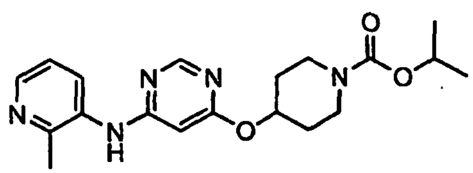 | 4-[6-(2-Methyl-pyridin-3-ylamino)-pyrimidin-4-yloxy]-piperidine-1-carboxylic acid isopropyl ester |

(continued)

| Example No. | Chemical Structure | Chemical Name |
|---|---|---|
| B68 | 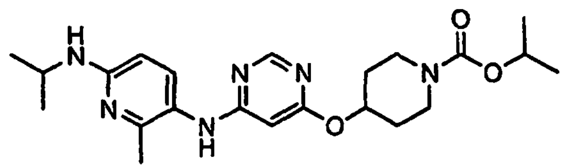 | 4-[6-(6-Isopropylamino-2-methyl-pyridin-3-ylamino)-pyrimidin-4-yloxy]-piperidine-1-carboxylic acid isopropyl ester |
| B69 | 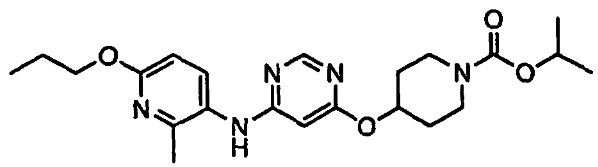 | 4-[6-(2-Methyl-6-propoxy-pyridin-3-ylamino)-pyrimidin-4-yloxy]-piperidine-1-carboxylic acid isopropyl ester |
| B70 | 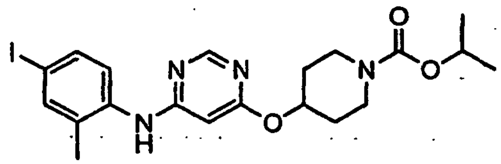 | 4-[6-(4-Iodo-2-methyl-phenylamino)-pyrimidin-4-yloxy]-piperidine-1-carboxylic acid isopropyl ester |
| B71 | 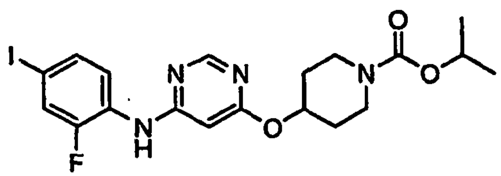 | 4-[6-(2-Fluoro-4-iodo-phenylamino)-pyrimidin-4-yloxy]-piperidine-1-carboxylic acid isopropyl ester |
| B72 (REF) | 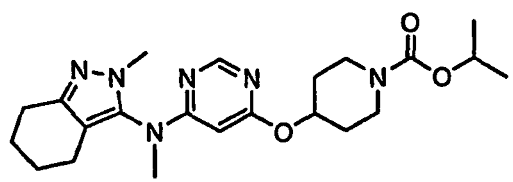 | 4-{6-[Methyl-(2-methyl-4,5,6,7-tetrahydro-2H-indazol-3-yl)-amino]-pyrimidin-4-yloxy}-piperidine-1-carboxylic acid isopropyl ester |

(continued)

| Example No. | Chemical Structure | Chemical Name |
|---|---|---|
| B73 (REF) | 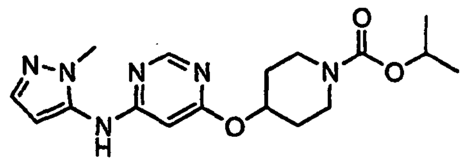 | 4-[6-(2-Methyl-2H-pyrazol-3-ylamino)-pyrimidin-4-yloxyl-piperidine-1-carboxylic acid isopropyl ester |
| B74 (REF) | 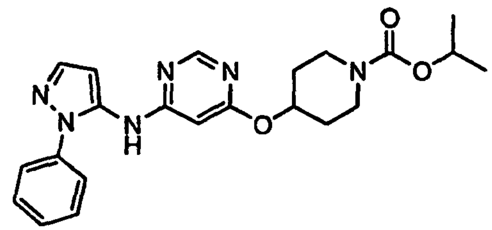 | 4-[6-(2-Phenyl-2H-pyrazol-3-ylamino)-pyrimidin-4-yloxy]-piperidine-1-carboxylic acid isopropyl ester |
| B75 (REF) | 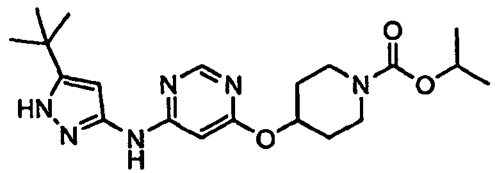 | 4-[6-(5-tert-Butyl-1H-pyrazol-3-ylamino)-pyrimidin-4-yloxy]-piperidine-1-carboxylic acid isopropyl ester |
| B76 (REF) | 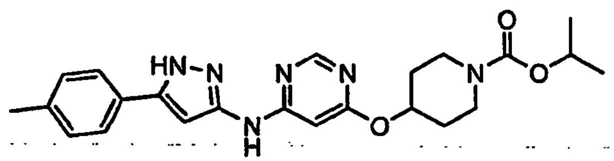 | 4-[6-(5-p-Tolyl-1H-pyrazol-3-ylamino)-pyrimidin-4-yloxy]-piperidine-1-carboxylic acid isopropyl ester |
| B77 | 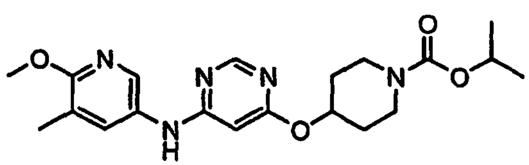 | 4-[6-(6-Methoxy-5-methyl-pyridin-3-ylamino)-pyrimidin-4-yloxy]-piperidine-1-carboxylic acid isopropyl ester |

(continued)

| Example No. | Chemical Structure | Chemical Name |
|---|---|---|
| B78 | 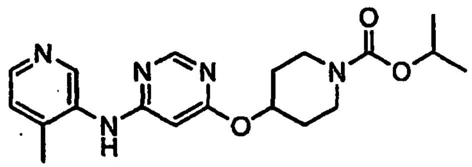 | 4-[6-(4-Methyl-pyridin-3-ylamino)-pyrimidin-4-yloxy]-piperidine-1-carboxylic acid isopropyl ester |
| B79 | 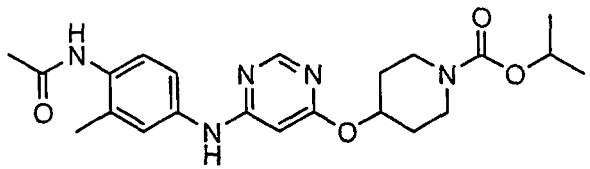 | 4-[6-(4-Acetylamino-3-methyl-phenylamino)-pyrimidin-4-yloxy]-piperidine-1-carboxylic acid isopropyl ester |
| B80 | 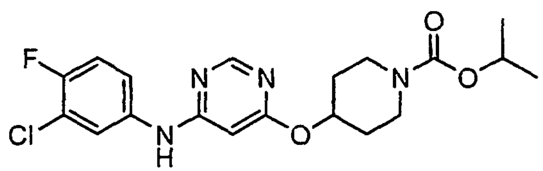 | 4-[6-(3-Chloro-4-fluoro-phenylamino)-pyrimidin-4-yloxy]-piperidine-1-carboxylic acid isopropyl ester |
| B81 | 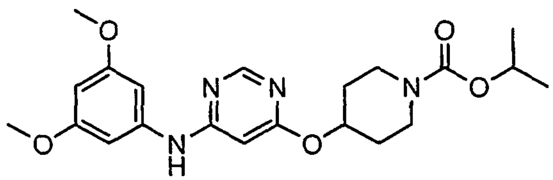 | 4-[6-(3,5-Dimethoxy-phenylamino)-pyrimidin-4-yloxy]-piperidine-1-carboxylic acid isopropyl ester |
| B82 | 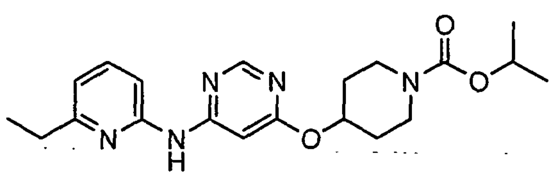 | 4-[6-(6-Ethyl-pyridin-2-ylamino)-pyrimidin-4-yloxy]-piperidine-1-carboxylic acid isopropyl ester |

(continued)

| Example No. | Chemical Structure | Chemical Name |
|---|---|---|
| B83 | 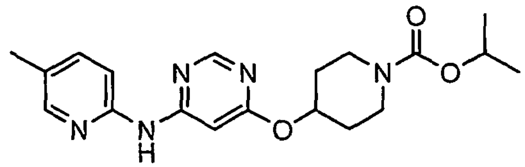 | 4-[6-(5-Methyl-pyridin-2-ylamino)-pyrimidin-4-yloxy]-piperidine-1-carboxylic acid isopropyl ester |
| B84 | 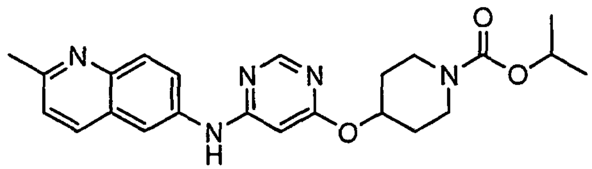 | 4-[6-(2-Methyl-quinolin-6-ylamino)-pyrimidin-4-yloxy]-piperidine-1-carboxylic acid isopropyl ester |
| B85 | 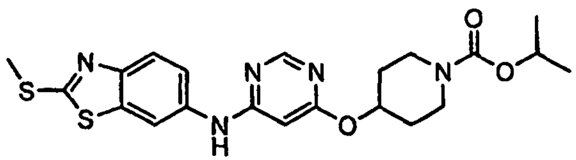 | 4-[6-(2-Methylsulfanyl-benzothiazol-6-ylamino)-pyrimidin-4-yloxy]-piperidine-1-carboxylic acid isopropyl ester |
| B86 | 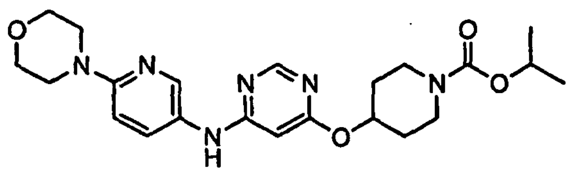 | 4-[6-(6-Morpholin-4-yl-pyridin-3-ylamino)-pyrimidin-4-yloxy]-piperidine-1-carboxylic acid isopropyl ester |

(continued)

| Example No. | Chemical Structure | Chemical Name |
|---|---|---|
| B87 (REF) | 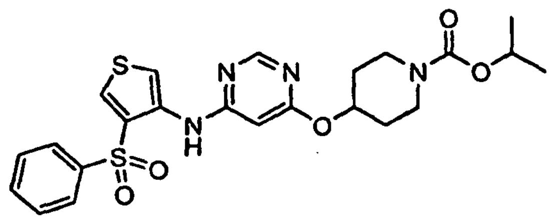 | 4-[6-(4-Benzenesulfonyl-thiophen-3-ylamino)-pyrimidin-4-yloxy]-piperidine-1-carboxylic acid isopropyl ester |
| B88 | 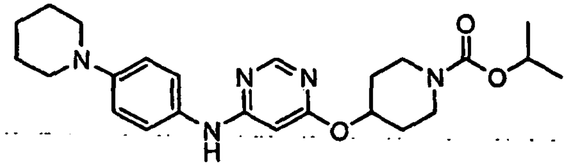 | 4-[6-(4-Piperidin-1-yl-phenylamino)-pyrimidin-4-yloxy]-piperidine-1-carboxylic acid isopropyl ester |
| B89 | 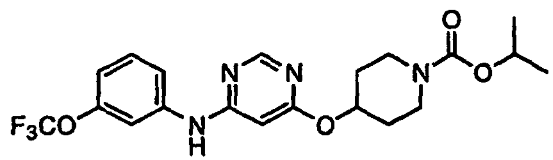 | 4-[6-(3-Trifluoromethoxy-phenylamino)-pyrimidin-4-yloxy]-piperidine-1-carboxylic acid isopropyl ester |
| B90 | 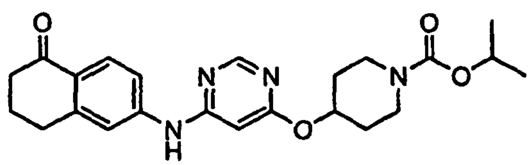 | 4-[6-(5-Oxo-5,6,7,8-tetrahydronaphthalen-2-ylamino)-pyrimidin-4-yloxy]-piperidine-1-carboxylic acid isopropyl ester |
| B91 (REF) | 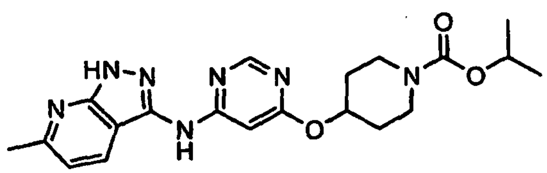 | 4-[6-(6-Methyl-1H-pyrazolo[3,4-b]pyridin-3-ylamino)-pyrimidin-4-yloxy]-piperidine-1-carboxylic acid isopropyl ester |

(continued)

| Example No. | Chemical Structure | Chemical Name |
|---|---|---|
| B92 | 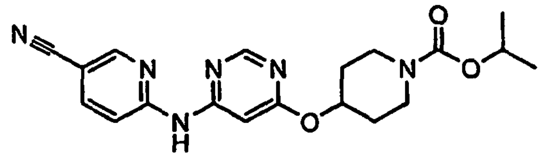 | 4-[6-(5-Cyano-pyridin-2-ylamino)-pyrimidin-4-yloxy]-piperidine-1-carboxylic acid isopropyl ester |
| B93 | 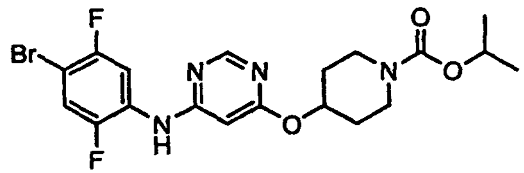 | 4-[6-(4-Bromo-2,5-difluoro-phenylamino)-pyrimidin-4-yloxy]-piperidine-1-carboxylic acid isopropyl ester |
| B94 | 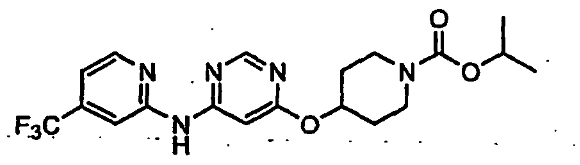 | 4-[6-(4-Trifluoromethyl-pyridin-2-ylamino)-pyrimidin-4-yloxy]-piperidine-1-carboxylic acid isopropyl ester |
| B95 (REF) | 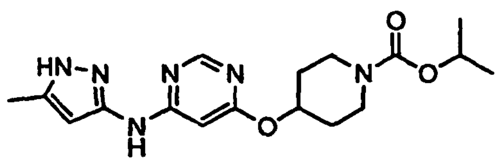 | 4-[6-(5-Methyl-1H-pyrazol-3-ylamino)-pyrimidin-4-yloxy]-piperidine-1-carboxylic acid isopropyl ester |
| B96 (REF) | 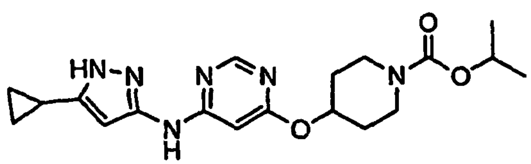 | 4-[6-(5-Cyclopropyl-1H-pyrazol-3-ylamino)-pyrimidin-4-yloxy]-piperidine-1-carboxylic acid isopropyl ester |

(continued)

| Example No. | Chemical Structure | Chemical Name |
|---|---|---|
| B97 | 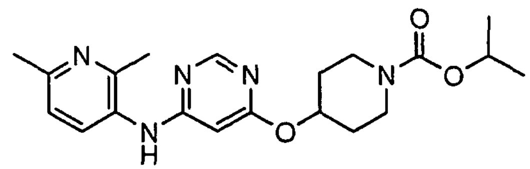 | 4-[6-(2,6-Dimethyl-pyridin-3-ylamino)-pyrimidin-4-yloxy]-piperidine-1-carboxylic acid isopropyl ester |
| B98 | 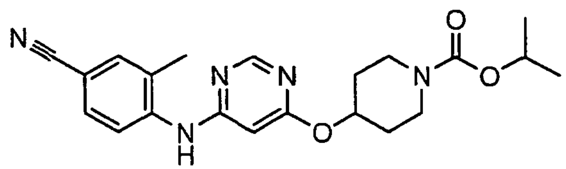 | 4-[6-(4-Cyano-2-methyl-phenylamino)-pyrimidin-4-yloxy]-piperidine-1-carboxylic acid isopropyl ester |
| B99 | 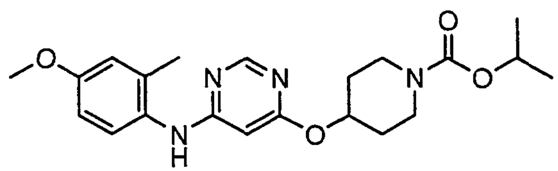 | 4-[6-(4-Methoxy-2-methyl-phenylamino)-pyrimidin-4-yloxy]-piperidine-1-carboxylic acid isopropyl ester |
| B100 | 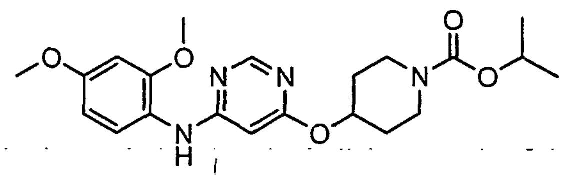 | 4-[6-(2,4-Dimethoxy-phenylamino)-pyrimidin-4-yloxy]-piperidine-1-carboxylic acid isopropyl ester |

(continued)

| Example No. | Chemical Structure | Chemical Name |
|---|---|---|
| B101 | 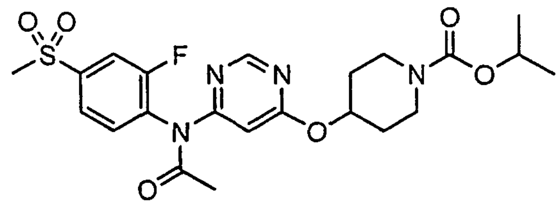 | 4-{6-[Acetyl-(2-fluoro-4-methanesulfonyl-phenyl)-amino]-pyrimidin-4-yloxy}-piperidine-1-carboxylic acid isopropyl ester |
| B102 | 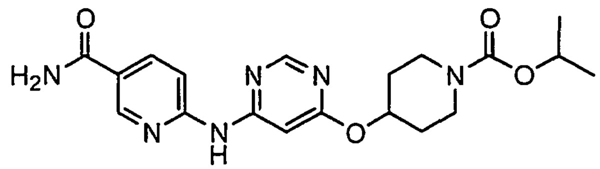 | 4-[6-(5-Carbamoyl-pyridin-2-ylamino)-pyrimidin-4-yloxy]-piperidine-1-carboxylic acid isopropyl ester |
| B103 (REF) | 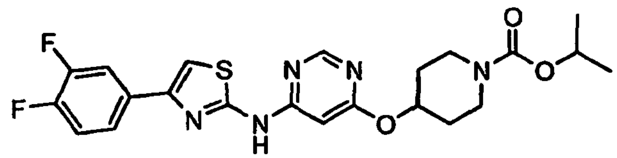 | 4-{6-[4-(3,4-Difluoro-phenyl)-thiazol-2-ylamino]-pyrimidin-4-yloxy}-piperidine-1-carboxylic acid isopropyl ester |
| B104 (REF) | 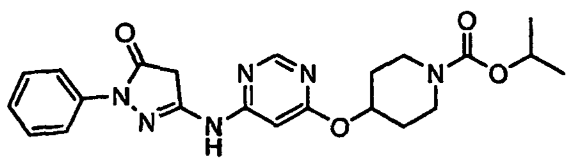 | 4-[6-(5-Oxo-1-phenyl-4,5-dihydro-1H-pyrazol-3-ylamino)-pyrimidin-4-yloxy]-piperidine-1-carboxylic acid isopropyl ester |
| B105 | 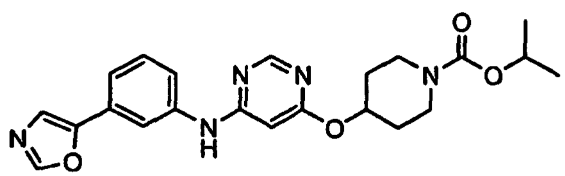 | 4-[6-(3-Oxazol-5-yl-phenylamino)-pyrimidin-4-yloxy]-piperidine-1-carboxylic acid isopropyl ester |

(continued)

| Example No. | Chemical Structure | Chemical Name |
|---|---|---|
| B106 | 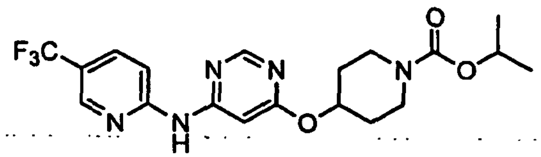 | 4-[6-(5-Trifluoromethyl-pyridin-2-ylamino)-pyrimidin-4-yloxy]-piperidine-1-carboxylic acid isopropyl ester |
| B107 | 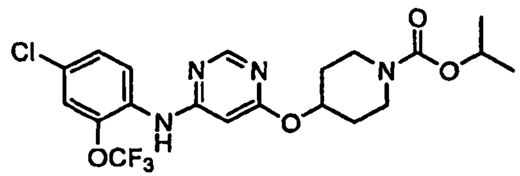 | 4-[6-(4-Chloro-2-trifluoromethoxy-phenylamino)-pyrimidin-4-yloxy]-piperidine-1-carboxylic acid isopropyl ester |
| B108 (REF) | 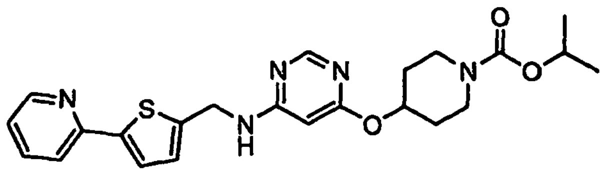 | 4-{6-[(5-Pyridin-2-yl-thiophen-2-ylmethyl)-amino]-pyrimidin-4-yloxy}-piperidine-1-carboxylic acid isopropyl ester |
| B109 (REF) | 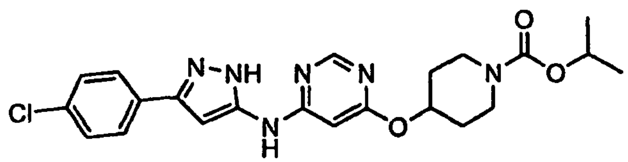 | 4-{6-[5-(4-Chloro-phenyl)-2H-pyrazol-3-ylamino]-pyrimidin-4-yloxy}-piperidine-1-carboxylic acid isopropyl ester |
| B110 | 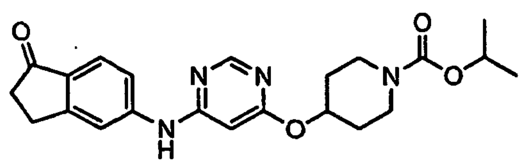 | 4-[6-(1-Oxo-indan-5-ylamino)-pyrimidin-4-yloxy]-piperidine-1-carboxylic acid isopropyl ester |

(continued)

| Example No. | Chemical Structure | Chemical Name |
|---|---|---|
| B111 | 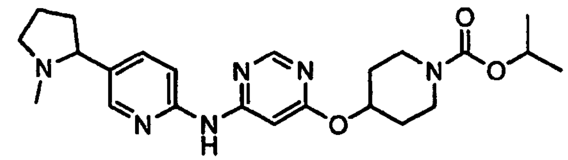 | 4-{6-[5-(1-Methyl-pyrrolidin-2-yl)-pyridin-2-ylamino]-pyrimidin-4-yloxy}-piperidine-carboxylic acid isopropyl ester |
| B112 | 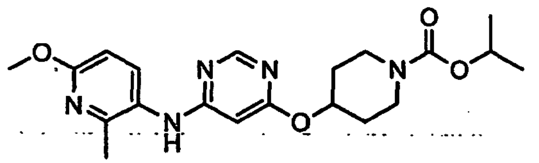 | 4-[6-(6-Methoxy-2-methyl-pyridin-3-ylamino)-pyrimidin-4-yloxy]-piperidine-1-carboxylic acid isopropyl ester |
| B113 | 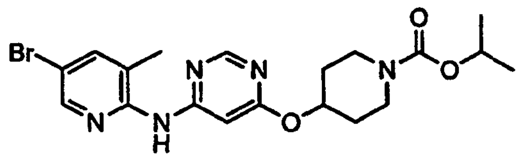 | 4-[6-(5-Bromo-3-methyl-pyridin-2-ylamino)-pyrimidin-4-yloxy]-piperidine-1-carboxylic acid isopropyl ester |
| B114 | 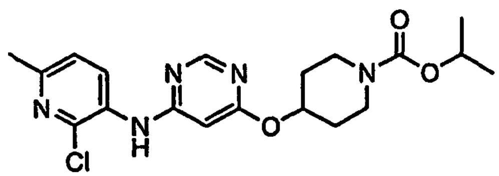 | 4-[6-(2-Chloro-6-methyl-pyridin-3-ylamino)-pyrimidin-4-yloxy]-piperidine-1-carboxylic acid isopropyl ester |
| B115 | 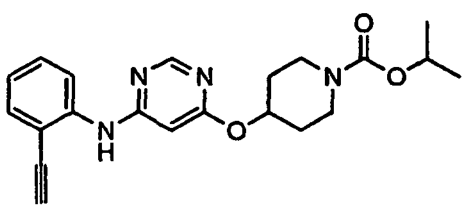 | 4-[6-(2-Ethynyl-phenylamino)-pyrimidin-4-yloxy]-piperidine-1-carboxylic acid isopropyl ester |

(continued)

| Example No. | Chemical Structure | Chemical Name |
|---|---|---|
| B116 | 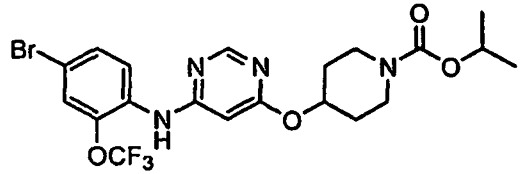 | 4-[6-(4-Bromo-2-trifluoromethoxy-phenylamino)-pyrimidin-4-yloxy]-piperidine-1-carboxylic acid isopropyl ester |
| B117 | 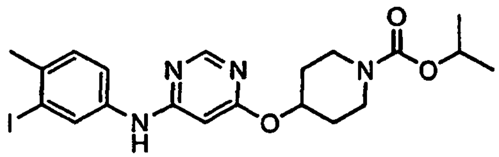 | 4-[6-(3-Iodo-4-methyl-phenylamino)-pyrimidin-4-yloxy]-piperidine-1-carboxylic acid isopropyl ester |
| B118 | 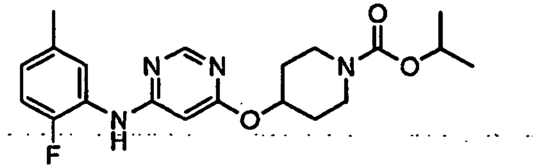 | 4-[6-(2-Fluoro-5-methyl-phenylamino)-pyrimidin-4-yloxy]-piperidine-1-carboxylic acid isopropyl ester |
| B119 (REF) | 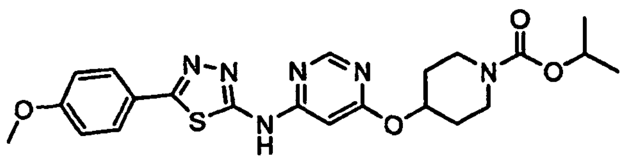 | 4-{6-[5-(4-Methoxy-phenyl)-[1,3,4]thiadiazol-2-ylamino]-pyrimidin-4-yloxy}-piperidine 1-carboxylic acid isopropyl ester |
| B120 (REF) | 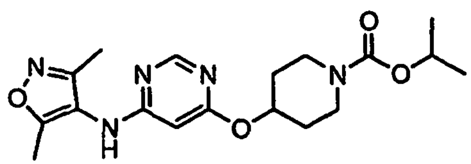 | 4-[6-(3,5-Dimethyl-isoxazol-4-ylamino)-pyrimidin-4-yloxy]-piperidine-1-carboxylic acid isopropyl ester |

(continued)

| Example No. | Chemical Structure | Chemical Name |
|---|---|---|
| B121 (REF) | 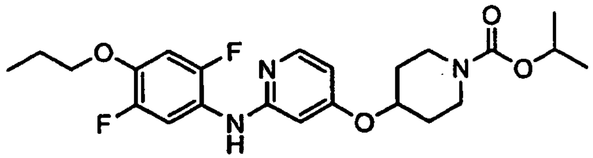 | 4-[2-(2,5-Difluoro-4-propoxy-phenylamino)-pyridin-4-yloxy]-piperidine-1-carboxylic acid isopropyl ester |
| B122 | 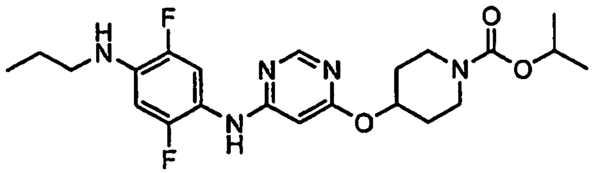 | 4-[6-(2,5-Difluoro-4-propylamino-phenytamino)-pyrimidin-4-yloxy]-piperidine-1-carboxylic acid isopropyl ester |
| B123 | 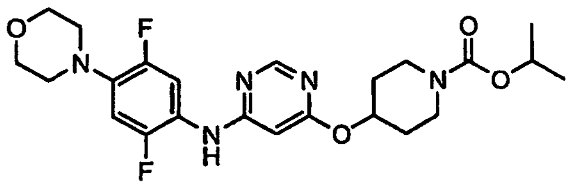 | 4-[6-(2,5-Difluoro-4-morpholin-4-yl-phenylamino)-pyrimidin-4-yloxy]-piperidine-1-carboxylic acid isopropyl ester |
| B124 | 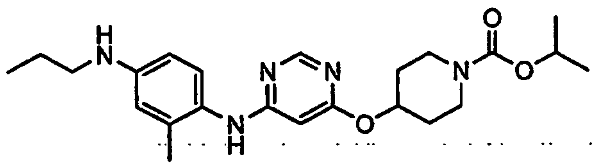 | 4-[6-(2-Methyl-4-propylamino-phenylamino)-pyrimidin-4-yloxy]-piperidine-1-caboxylic acid isopropyl ester |
| B125 | 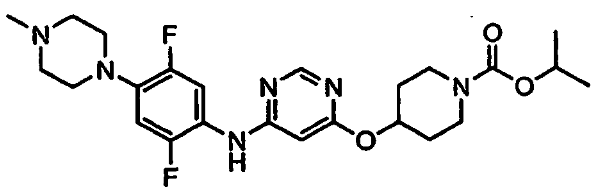 | 4-{6-[2,5-Difluoro-4-(4-methyl-piperazin-1-yl)-phenylamino]-pyrimidin-4-yloxy}-piperidine-1-carboxylic acid isopropyl ester |

(continued)

| Example No. | Chemical Structure | Chemical Name |
|---|---|---|
| B126 | 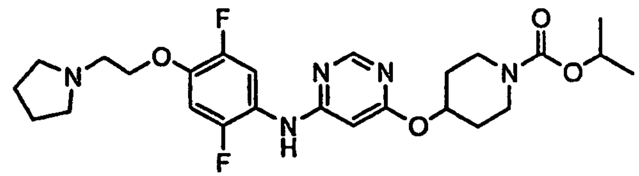 | 4-{6-[2,5-Difluoro-4-(2-pyrrolidin-1-yl-ethoxy)-phenylamino]-pyrimidin-4-yloxy}-piperidine-1-carboxylic acid isopropyl ester |
| B127 | 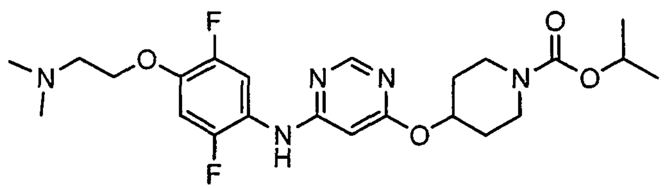 | 4-{6-[4-(2-Dimethylamino-ethoxy)-2,5-difluoro-phenylamino]-pyrimidin-4-yloxy}-piperidine-1-carboxylic acid isopropyl ester |
| B128 | 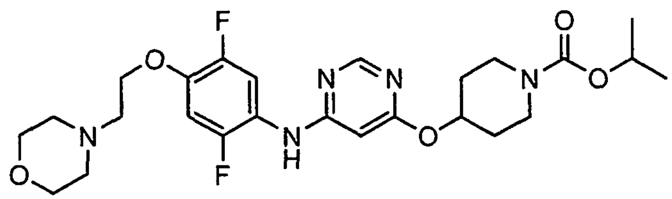 | 4-{6-[2,5-Difluoro-4-(2-morpholin-4-yl-ethoxy)-phenylamino]-pyrimidin-4-yloxy}-piperidine-1-carboxylic acid isopropyl ester |
| B129 | 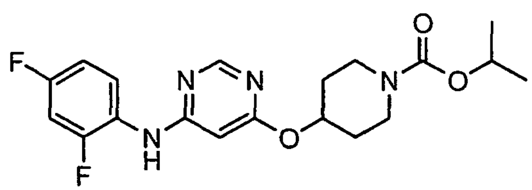 | 4-[6-(2,4-Difluoro-phenylamino)-pyrimidin-4-yloxy]-piperidine-1-carboxylic acid isopropyl ester |

(continued)

| Example No. | Chemical Structure | Chemical Name |
|---|---|---|
| B130 | 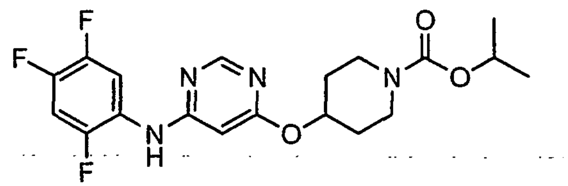 | 4-[6-(2,4,5-Trifluoro-phenylamino)-pyrimidin-4-yloxy]-piperidine-1-carboxylic acid isopropyl ester |
| B131 | 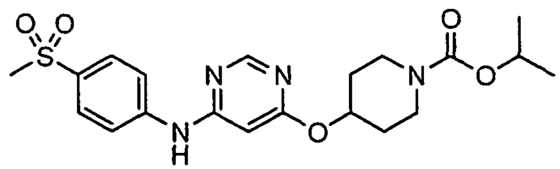 | 4-[6-(4-Methanesulfonyl-phenylamino)-pyrimidin-4-yloxy]-piperidine-1-carboxylic acid isopropyl ester |
| B132 | 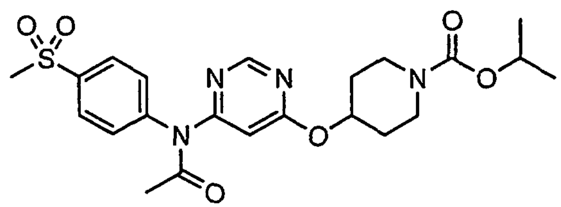 | 4-{6-[Acetyl-(4-methanesulfonyl-phenyl)-amino]-pyrimidin-4-yloxy}-piperidine-1-carboxylic acid isopropyl ester |
| B133 | 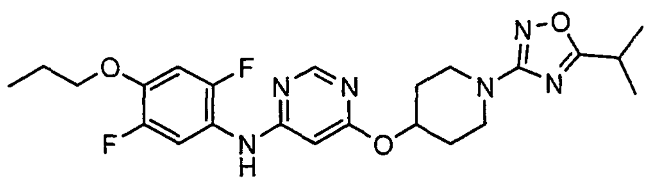 | (2,5-Difluoro-4-propoxy-phenyl)-{6-[1-(5-isopropyl-[1,2,4]oxadiazol-3-yl)-piperidin-4-yloxy]-pyrimidin-4-yl} -amine |

(continued)

| Example No. | Chemical Structure | Chemical Name |
|---|---|---|
| B134 | 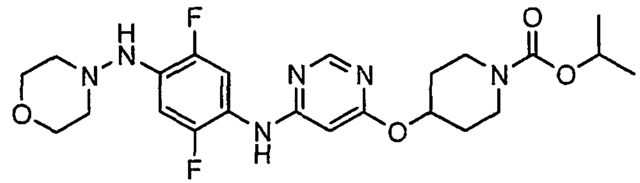 | 4-{6-[2,5-Difluoro-4-(morpholin-4-ylamino)-phenylamino]-pyrimidin-4-yloxy}-piperidine-1-carboxylic acid isopropyl ester |
| B135 | 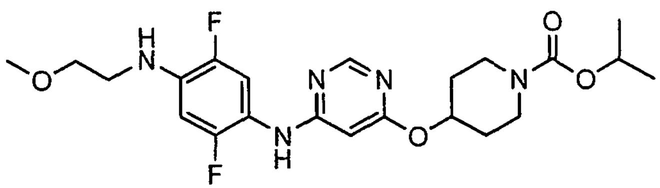 | 4-{6-[2,5-Difluoro-4-(2-methoxy-ethylamino)-phenylamino]-pyrimidin-4-yloxy}-piperidine-1-carboxylic acid isopropyl ester |
| B136 | 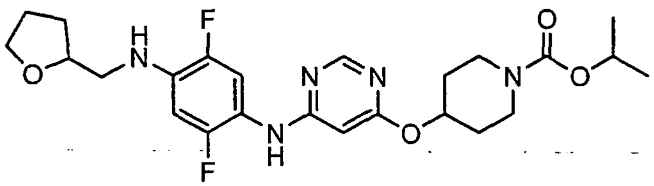 | 4-(6-{2,5-Difluoro-4-[(tetrahydro-furan-2-ylmethyl)-amino]-phenylamino}-pyrimidin-4-yloxy)-piperidine-1-carboxylic acid isopropyl ester |
| B137 | 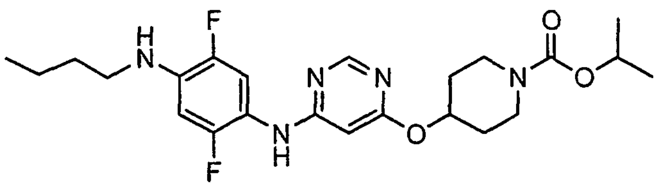 | 4-[6-(4-Butylamino-2,5-difluoro-phenylamino)-pyrimidin-4-yloxy]-piperidine-1-carboxylic acid isopropyl ester |

(continued)

| Example No. | Chemical Structure | Chemical Name |
|---|---|---|
| B138 | 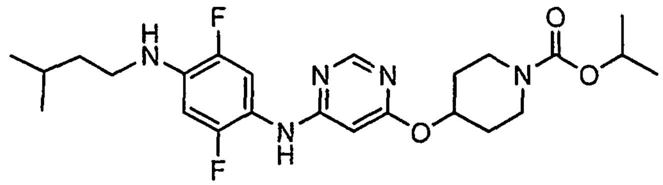 | 4-{6-[2,5-Difluoro-4-(3-methyl-butylamino)-phenylamino]-pyrimidin-4-yloxy}-piperidine-1-carboxylic acid isopropyl ester |
| B139 | 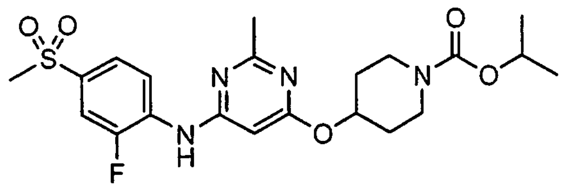 | 4-[6-(2-Fluoro-4-methanesulfonyl-phenylamino)-2-methyl-pyrimidin-4-yloxy]-piperidine-1-carboxylic acid isopropyl ester |
| B140 | 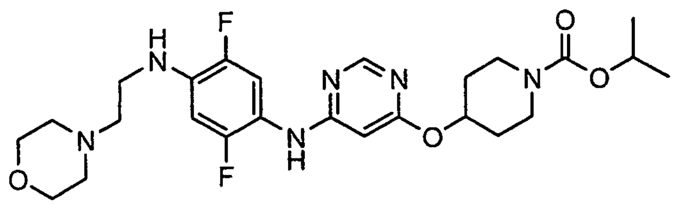 | 4-{6-[2,5-Difluoro-4-(2-morpholin-4-yl-ethylamino)-phenylamino]-pyrimidin-4-yloxy}-piperidine-1-carboxylic acid isopropyl ester |
| B141 | 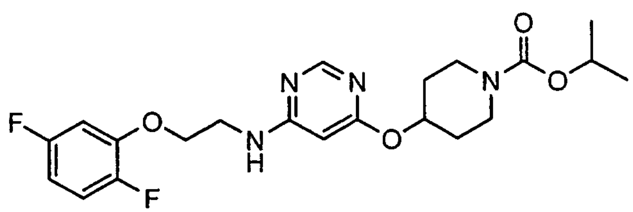 | 4-{6-[2-(2,5-Difluoro-phenoxy)-ethylamino]-pyrimidin-4-yloxy}-piperidine-1-carboxylic acid isopropyl ester |

(continued)

| Example No. | Chemical Structure | Chemical Name |
|---|---|---|
| B142 | 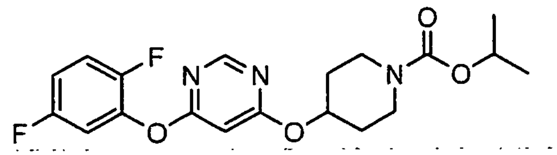 | 4-[6-(2,5-Difluoro-phenoxy)-pyrimidin-4-yloxy]-piperidine-1-carboxylic acid isopropyl ester |
| B143 | 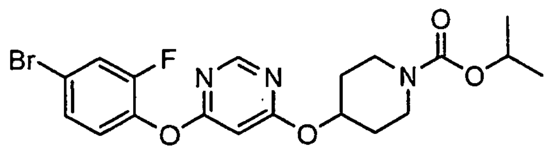 | 4-[6-(4-Bromo-2-fluoro-phenoxy)-pyrimidin-4-yloxy]-piperidine-1-carboxylic acid isopropyl ester |
| B144 | 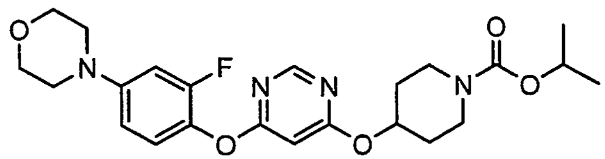 | 4-[6-(2-Fluoro-4-morpholin-4-yl-phenoxy)-pyrimidin-4-yloxy]-piperidine-1-carboxylic acid isopropyl ester |
| B145 | 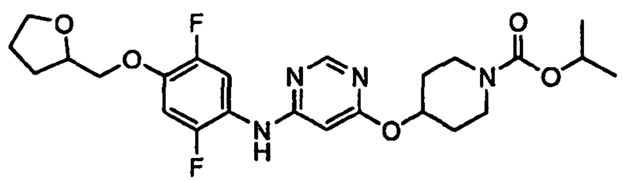 | 4-{6-[2,5-Difluoro-4-(tetrahydro-furan-2-ylmethoxy)-phenylamino]-pyrimidin-4-yloxy}-piperidine-1-carboxylic acid isopropyl ester |
| B146 (REF) | 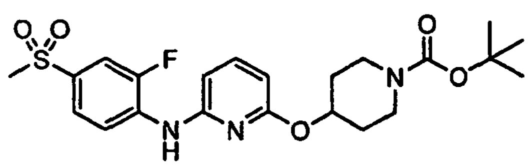 | 4-[6-(2-Fluoro-4-methanesulfonyl-phenylamino)-pyridin-2-yloxy]-piperidine-1-carboxylic acid tert-butyl ester |

(continued)

| Example No. | Chemical Structure | Chemical Name |
|---|---|---|
| B147 (REF) | 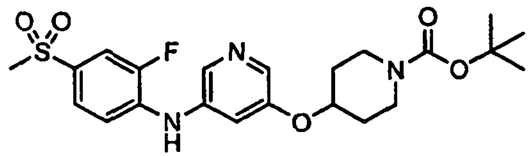 | 4-[5-(2-Fluoro-4-methanesulfonyl-phenylamino)-pyridin-3-yloxy]-piperidine-1-carboxylic acid tert-butyl ester |
| B148 (REF) | 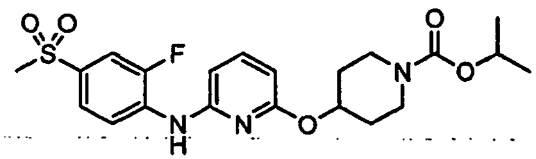 | 4-[6-(2-Fluoro-4-methanesulfonyl-phenylamino)-pyridin-2-yloxy]-piperidine-1-carboxylic acid isopropyl ester |
| B149 | 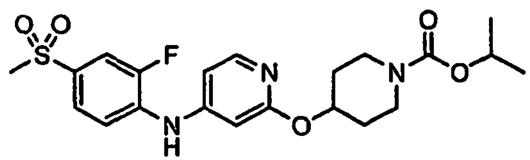 | 4-[4-(2-Fluoro-4-methanesulfonyl-phenylamino)-pyridin-2-yloxy]-piperidine-1-carboxylic acid isopropyl ester |
| B150 | 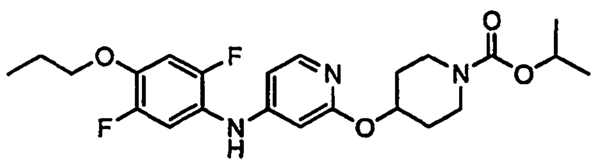 | 4-[4-(2,5-Difluoro-4-propoxy-phenylamino)-pyridin-2-yloxy]-piperidine-1-carboxylic acid isopropyl ester |
| B151 (REF) | 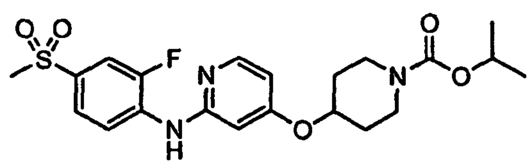 | 4-[2-(2-Fluoro-4-methanesulfonyl-phenylamino)-pyridin-4-yloxy]-piperidine-1-carboxylic acid isopropyl ester |

(continued)

| Example No. | Chemical Structure | Chemical Name |
|---|---|---|
| B152 (REF) | 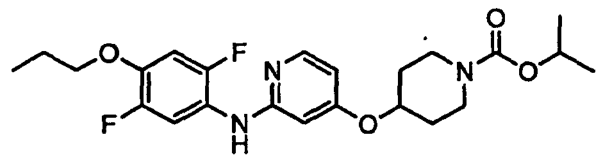 | 4-[2-(2,5-Difluoro-4-propoxyphenylamino)-pyridin-4-yloxy]-piperidine-1-carboxylic acid isopropyl ester |
| B153 | 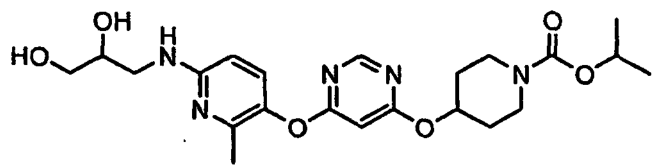 | 4-{6-[6-(2,3-Dihydroxy-propylamino)-2-methyl-pyridin-3-yloxy]-pyrimidin-4-yloxy}-piperidine-1-carboxylic acid isopropyl ester |
| B154 (REF) | 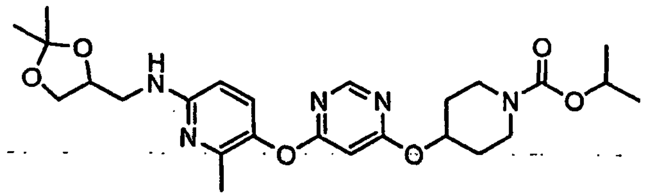 | 4-(6-{6-[(2,2-Dimethyl-[1,3]dioxolan-4-ylmethyl)-amino]-2-methyl-pyridin-3-yloxy}-pyrimidin-4-yloxy)-piperidine-1-carboxylic acid isopropyl ester |
| B155 | 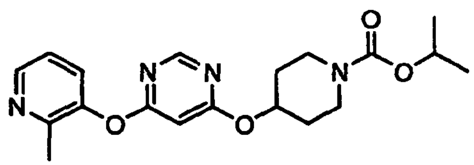 | 4-[6-(2-Methyl-pyridin-3-yloxy)-pyrimidin-4-yloxy]-piperidine-1-carboxylic acid isopropyl ester |
| B156 | 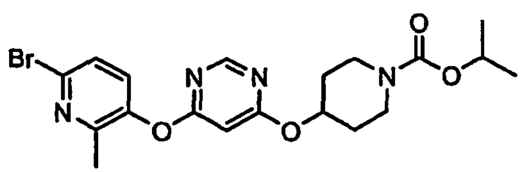 | 4-[6-(6-Bromo-2-methyl-pyridin-3-yloxy)-pyrimidin-4-yloxy]-piperidine-1-carboxylic acid isopropyl ester |

(continued)

| Example No. | Chemical Structure | Chemical Name |
|---|---|---|
| B157 | 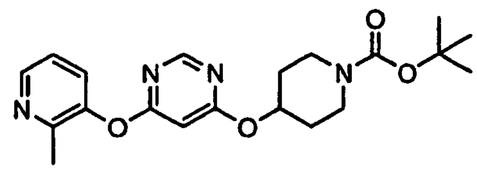 | 4-[6-(2-Methyl-pyridin-3-yloxy)-pyrimidin-4-yloxy]-piperidine-1-carboxylic acid tert-butyl ester |
| B158 (REF) | 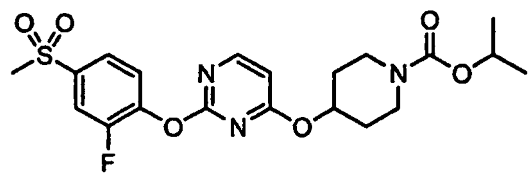 | 4-[2-(2-Fluoro-4-methanesulfonyl-phenoxy)-pyrimidin-4-yloxy]-piperidine-1-carboxylic acid isopropyl ester |
| B159 | 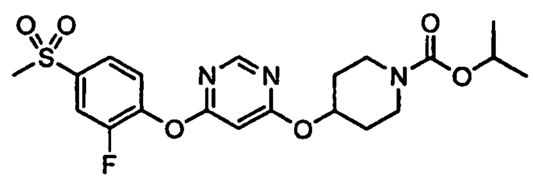 | 4-[6-(2-Fluoro-4-methanesulfonyl-phenoxy)-pyrimidin-4-yloxy]-piperidine-1-carboxylic acid isopropyl ester |

Additionally, compounds of Formula (I), such as those illustrated in Tables **A** and **B,** encompass all pharmaceutically acceptable salts, solvates, particularly hydrates, thereof.

In some embodiments, a compound of the present invention is 4-[6-(2-fluoro-4-methanesulfonyl-phenoxy)-pyrimidin-4-yloxy]-piperidine-1-carboxylic acid isopropyl ester.

In some embodiments, a compound of the present invention is not 4-[6-(2-fluoro-4-methanesulfonyl-phenoxy)-pyrimidin-4-yloxyl-piperidine-1-carboxylic acid isopropyl ester.

**General Synthetic Methods**

The *de novo* biosynthesis of pyrimidine nucleotides provides essential precursors for multiple growth-related events in higher eukaryotes. Assembled from ATP, bicarbonate and glutamine, the uracil and cytosine nucleotides are fuel for the synthesis of RNA, DNA, phospholipids, UDP sugars and glycogen. Over the past 2 decades considerable progress has been made in elucidating the mechanisms by which cellular pyrimidines are modulated to meet the needs of the cell. These studies point to increasing evidence for cooperation between key cell signaling pathways and basic elements of cellular metabolism, and suggest that these events have the potential to determine distinct cellular fates, including growth, differentiation and death.

As a result of their profound biological significance in higher eukaryotes and utilization of the pyrimidine core in a number of marketed drugs (**Scheme 1**) and other medicinally relevant compounds, pyrimidines and pyridines play pivotal roles as chemotypes in drug discovery campaigns. As a direct consequence of this there is a wealth of scientific literature describing synthetic construction, as well as chemical modification and elaboration of these classes of heterocyles.

**Scheme 1**

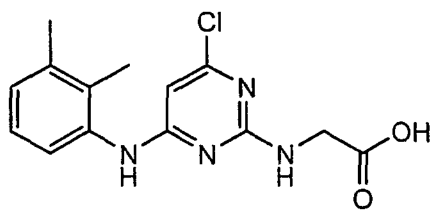

aronixil [1]

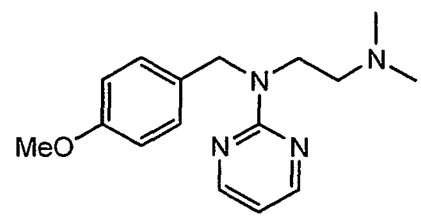

thonzylamine [2]

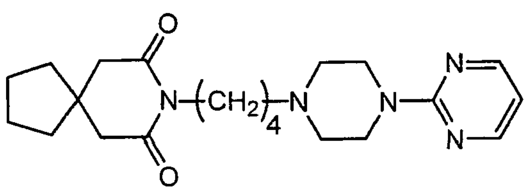

buspirone [3]

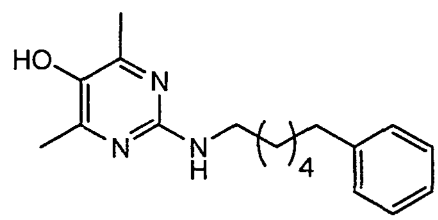

enazadrem [4]

The novel substituted pyrimidines and pyridines of the current invention can prepared according to a variety of synthetic manipulations, all of which would be familiar to one skilled in the art of synthetic organic chemistry. Certain methods for the preparation of compounds of the present invention include, but are not limited to, those described in Schemes 2-10 set forth in this section of the specification.

Common dichloro-substituted intermediate **8,** used as a starting point for the synthesis of compounds of the present invention can be prepared as depicted in **Scheme 2.** This is accomplished in two steps from a di-$C_{1-6}$-alkylmalonate, one particularly useful di-$C_{1-6}$-alkylmalonate is diethyl malonate 5. Cyclization to the 4,6-dihydroxypyrimidine 7 is achieved by reacting 5 with formamidine in the presence of an alkali metal alkoxide, by mixing the malonate and all or part of the formamidine with the alkoxide or with the alkoxide and the rest of the formamide. Alternative reagents such as dimethylmalonate, sodium methoxide, formamide, in low molecular weight alcoholic solvents, including methanol, ethanol, 2-propanol and the like, may be utilized in the synthesis by heating at a temperature range between about 80 to about 100°C for about 30 mins to about 90 mins followed by a mineral acid work up. Preparation of dihydroxypyrimidines can also be achieved using microorganisms such as *Rhodococcus* (see for reference W097008152 A1).

Chlorination of the **4** and **6** ring positions to produce intermediate **8** maybe carried out by reacting **7** with a

chlorinating reagent, such as, phosgene, $POCl_3$ (for reference see A. Gomtsyan et al., J. Med. Chem. 2002, 45, 3639-3648), thionyl chloride, oxalyl chloride and by mixtures of the above reagents including $PCl_3$ / $POCl_3$ at elevated reaction temperatures.

**Scheme 2**

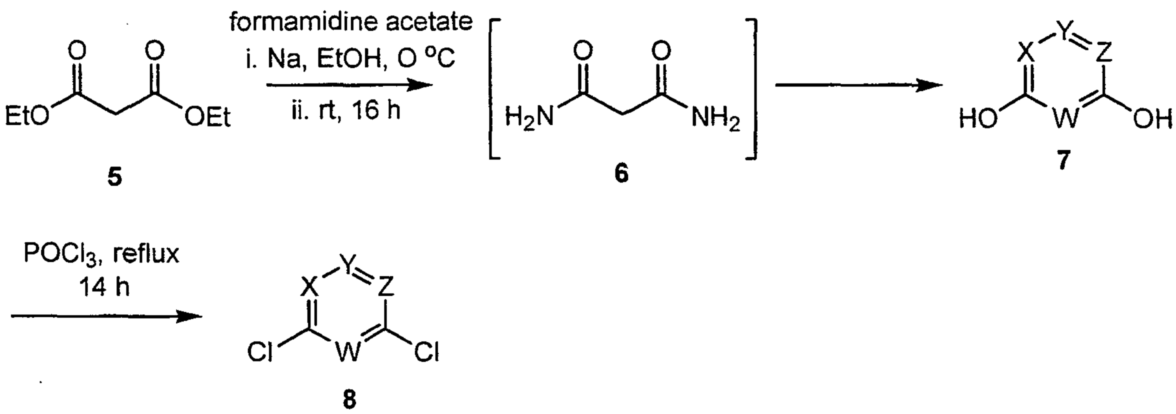


5 6 7 8

Conventional thermal aromatic substitution reactions of amines and alcohols with halogenated pyrimidines have been well documented (see for example A. G. Arvanitis et al., J. Medicinal Chemistry, 1999, 42, 805-818 and references therein). Nucleophilic aromatic ($SN_{Ar}$) substitution reactions of electron deficient halogenated pyrimidines are usually rapid and high yielding. However, in certain cases, such as electron rich or neutral halogenated heterocycles, successful substitution is afforded by prolonged heating. To facilitate rapid entry into many of the compounds of the invention microwave synthesis was utilized (**Schemes 3 and 4**). The Smith synthesizer from Personal Chemistry is a commercially available focussed field heating instrument that provides safer and more uniform conditions for performing the base catalysed substitution reactions depicted in Schemes **3a, 3b** and **3c.** Bases employed for such conversions (whereby $Q_2$ = N) include tertiary amines such as triethylamine, Hunig's base (i.e. diisopropyl-ethylamine), N-methylmorpholine and the like. Alternatively, one skilled in the art can employ alkali metal hydrides, alkali metal carbonates (such as, $Li_2CO_3$, $Na_2CO_3$, $K_2CO_3$ and the like), an alkali metal hydrogencarbonate (such as, $LiHCO_3$, $NaHCO_3$, $KHCO_3$ and the like). Wherein $Q_2$ = N, inert lower alkyl alcoholic solvent can be emplyed (such as, MeOH, EtOH, i-PrOH, n-BuOH and the like) or wherein $Q_2$ = O, an ethereal solvent such as tetrahydrofuran, 1,4-dioxane, and the like can be used. Reaction times to access typical intermediates such as, **9, 10,** and **11,** can range from about 300 s to about 3000 s and when conventional thermal methods are employed (wherein $Q_2$ = O) about 20 mins to about 120 mins.

**Scheme 3**

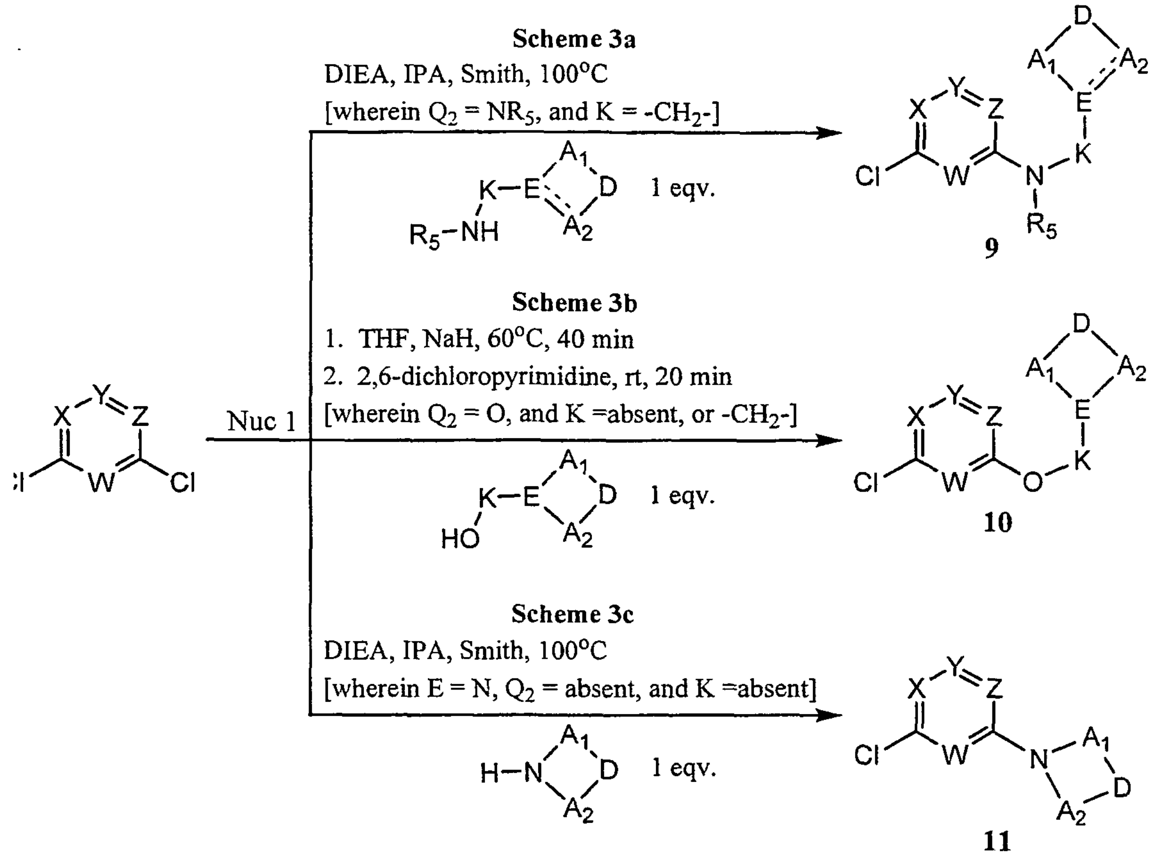


9

10

11

Methods for conversion of intermediate monosubstituted pyrimidines and pyridines **9, 10** and **11** are illustrated in **Scheme 4.** Examples wherein $Q_1$ = N (Schemes **4a, 4b, 4c**) were obtained using palladium catalysed aminations. This synthetic strategy has emerged as a powerful tool for synthesis of substituted aryl and heteroaryl anilines in recent times (for reference see S.L. Buchwald., Top. Curr. Chem., 2002, 219, 131 and references therein). Reaction of a suitably substituted amine (such as, Intermediate **16**) in the presence of a palladium or alternative transition metal catalyst selected from but not limited to $Pd_2(dba)_3$, $Pd(OAC)_2$, CuI, $Cu(OTf)_2$, $Ni(COD)_2$, $Ni(acac)_2$ in a suitable anhydrous solvent (such as, THF, 1,4-dioxane, and the like) with as strong alkali metal alkoxide base (such as, $NaO^tBu$, $KO^tBu$ and the like). A suitable ligand employed in this step can be selected from BINAP, $P(o\text{-tolyl})_3$, $tBu_3P$, DPPF, $P[N(^iBu)CH_2CH_3]_3N$ and the like when the catalyst is a palladium derived complex.

Alternatively, for "Ullman-type" aryl aminations catalysed by copper derived complexes the base employed maybe selected from an alkali metal carbonate in an aprotic polar solvent (such as *N,N*-dimethylacetamide, DMF, DMSO, and the like) with L-proline, N-methylglycine or diethylsalicyclamide as the ligand (for reference see D. Ma, Organic Lett., 2003, 5, 14, 2453 - 2455).

**Scheme 4**

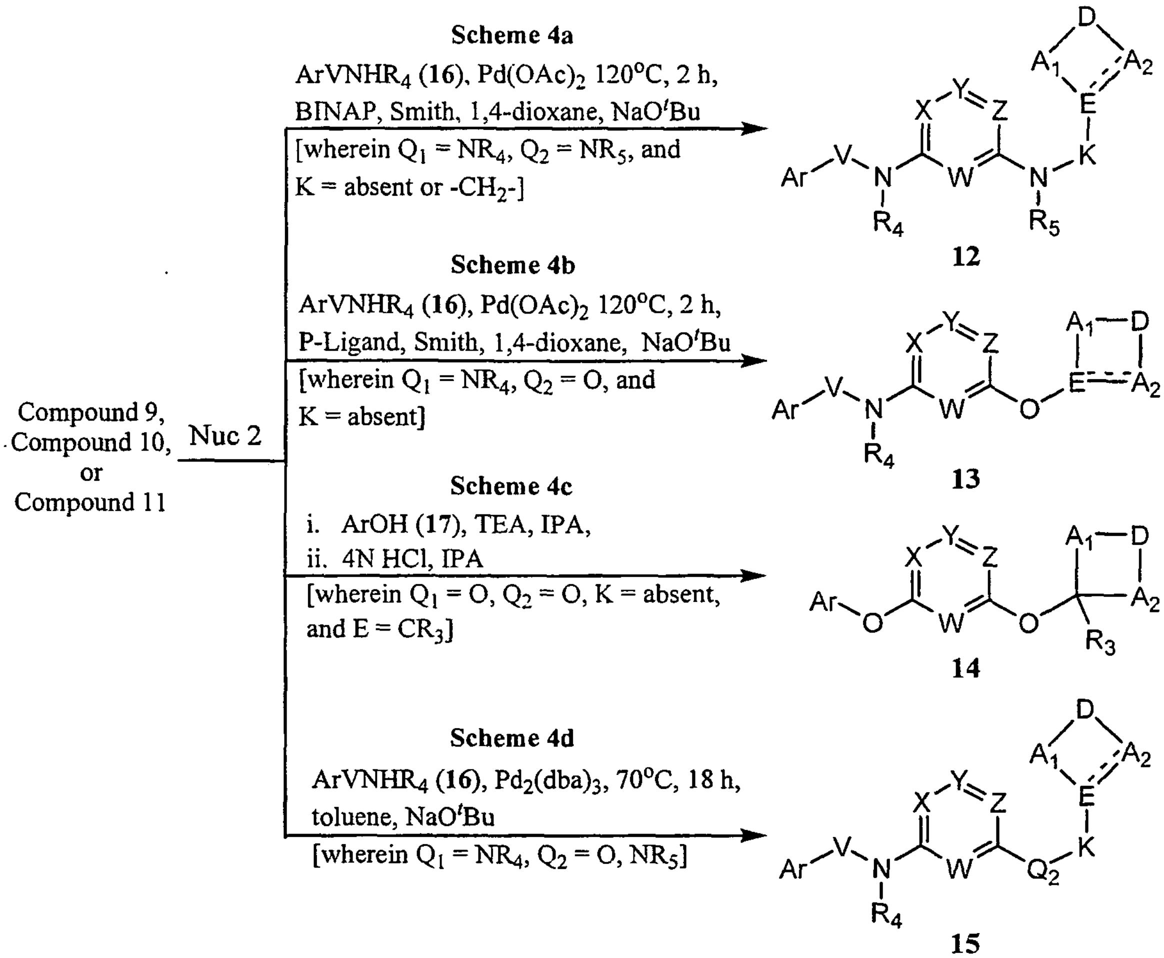


12

13

14

15

Compounds of general formula **12** to **15** may also be obtained by reversing the order of the reaction steps (i.e. introduction of $Q_1$ followed by $Q_2$), wherein the initial step comprises of introduction of either Intermediate **16** or **17** by using base in $^i$PrOH followed by addition of 4N HCl in dioxane.

As illustrated in **Scheme 5,** a similar transition metal catalysed couplings were utilized to obtain molecules of general formula **21a** and **21b** (**Scheme 5a**) wherein the "Ar" substituent (Hal = Br, I) of intermediate **20** is modified to give analogs with alkyl amino substituents (i.e., $NR_aR_b$, wherein $R_a$ and $F_b$ are each independently H, $C_{1-6}$ alkyl or a substituted $C_{1-6}$ alkyl, or $R_a$ and $R_b$ together with the nitrogen form a heterocyclic ring, as described herein). Alternatively, the linker atom can be oxygen by utilizing the CuI catalysed method for aromatic C-O formation described by Buchwald (see for reference S. L. Buchwald; Organic Lett., 2002, 4, 6, 973-976) by utilizing, for example, 10 mol% CuI, 20 mol% 1,10-phenanthroline, 2 equivalents of $Cs_2CO_3$, at 110°C for 18 h (**Scheme 5b**), with an "Ar" iodo substitution in the substrate. One particular embodiment is when the Hal group on "Ar" is substituted at the para position of a phenyl ring.

## Scheme 5

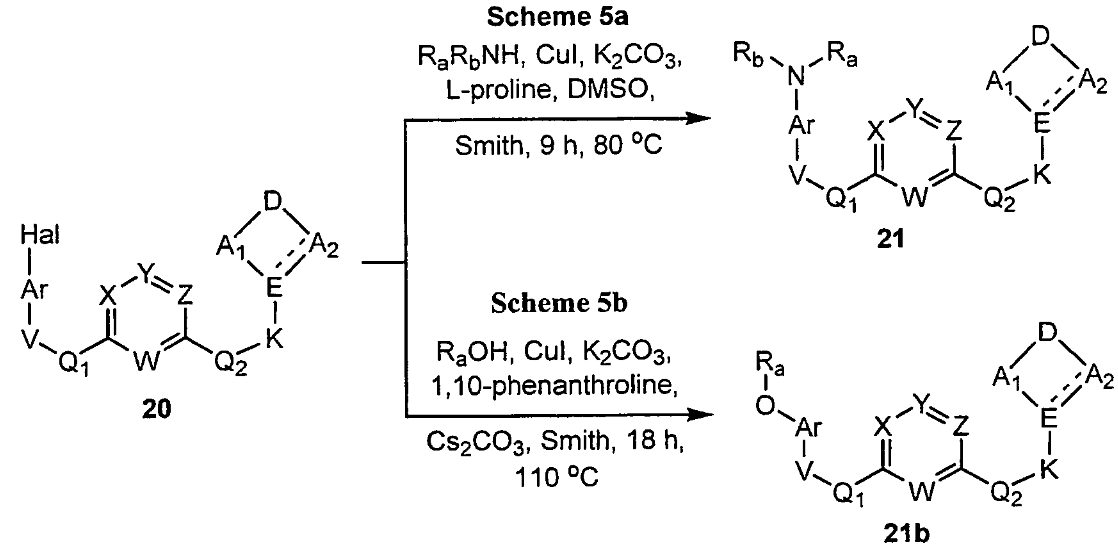


20 21 21b

A particular substitution for compounds **12, 13, 14,** and **15** is wherein D = $NCOOR_c$ wherein $R_c$ is $C_{1-6}$ alkyl, or $C_{3-7}$ cycloalkyl and each can be further substituted. Urethanes of this type can be prepared directly from intermediates depicted in **Schemes** 3 and **4** when D = NH. In certain reactions, use of a suitable nitrogen protecting group (such as, $^t$Boc, Cbz, Moz, Alloc, Fmoc and the like) may be necessary during further chemical modification of the core. Deprotection maybe achieved using standard reagents familiar to one skilled in the art (these might include TFA, mineral acid, Palladium /hydrogen gas and the like in an alcoholic or ethereal solvent system chosen from methanol, ethanol, *tert*-butanol, THF, 1,4-dioxane, and the like). On occasion wherein the target molecule contains 2 protecting groups, an orthogonal protection strategy may be adopted. The deprotected secondary amine (D = NH) can subsequently be modified accordingly.

**Schemes 6** and **7** illustrate such chemistries wherein generation of a carbamate, urea or amide can be executed using an appropriate reaction in the presence of a base, for example, a tertiary amine base such as TEA, DIEA and the like, in an inert solvent system.

As illustrated in **Scheme 6,** urethane **19** can be obtained by a urethane reaction using $R_c$OCO-halide (wherein $R_a$ is as described *supra,* and halide is chloro, bromo, or iodo, particularly useful is chloro) in an inert solvent with or without a base. Suitable bases include an alkali metal carbonate (such as, sodium carbonate, potassium carbonate, and the like), an alkali metal hydrogencarbonate (such as, sodium hydrogencarbonate, potassium hydrogencarbonate, and the like), an alkali hydroxide (such as, sodium hydroxide, potassium hydroxide, and the like), a tertiary amine (such as, *N,N*-diisopropylethylamine, triethylamine, N-methylmorpholine, and the like), or an aromatic amine (such as, pyridine, imidazole, poly-(4-vinylpyridine), and the like). The inert solvent includes lower halocarbon solvents (such as, dichloromethane, dichloroethane, chloroform, and the like), ethereal solvents (such as, tetrahydrofuran, dioxane, and the like), aromatic solvents (such as, benzene, toluene, and the like), or polar solvents (such as, *N,N*-dimethylformamide, dimethyl sulfoxide, and the like). Reaction temperature ranges from about -20°C to 120°C, preferably about 0°C to 100°C.

## Scheme 6

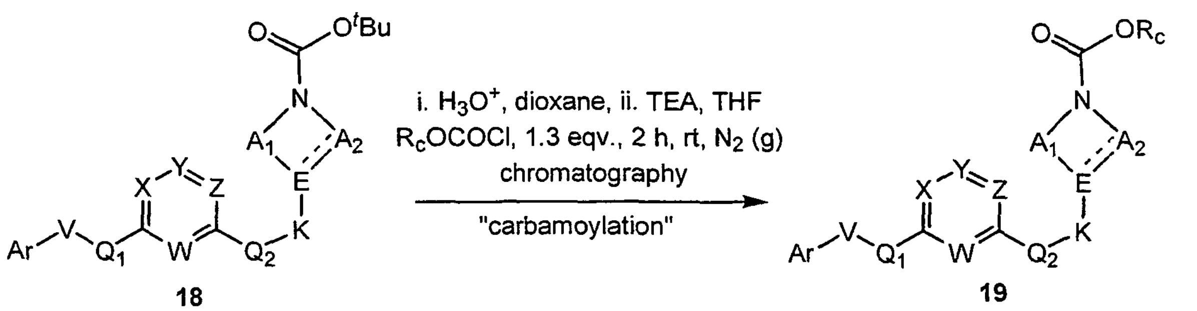


18 19

As shown in **Scheme 7a,** the amine intermediate obtained from acidic deprotection of **22** can be functionalized to amides represented by species **23.** Carbamate **22** is first reacted with 4N HCl in dioxane or alternatively TFA in dichloromethane and further reacted with a carboxylic acid ($R_dCO_2H$, wherein as used in **Scheme 7a**, $R_d$ is Ar, or a $C_{1-6}$-alkylene-Ar; Ar can be substituted or unsubstituted and has the same meaning as described herein) with a dehydrating condensing agent in an inert solvent with or without a base to provide the amide **23** of the present invention. The dehydrating condensing agent includes dicyclohexylcarbodiimide (DCC), 1,3-diisopropylcarbodiimide (DIC), 1-ethyl-3-(3-dimethylaminopropyl)carbodiimide hydrochloride (EDC•HCl), bromo-tris-pyrrolidino-phosphonium hexafluorophosphate (PyBroP), benzotriazoloyloxytris(dimethylamino)-phosphonium hexafluorophosphate (BOP), *O*-(7-azabenzo triazol-1-yl)-1,1,3,3-tetramethyluronium hexafluorophosphate (HATU), or 1-cyclohexyl-3-methylpolystyrene-carbodiimide. The base includes a tertiary amine (such as, *N,N*-diisopropylethylamine, triethylamine, and the like). The inert solvent includes lower halocarbon solvents (such as, dichloromethane, dichloroethane, chloroform, and the like), ethereal solvents (such as, tetrahydrofuran, dioxane, and the like), nitrile solvents (such as, acetonitrile, and the like), amide solvents (*N,N*-dimethylformamide, *N,N*-dimethylacetamide, and the like) and mixtures thereof. Optionally, 1-hydroxybenzotriazole (HOBT), HOBT-6-carboxaamidomethyl polystyrene, or 1-hydroxy-7-azabenzotriazole (HOAT) can be used as a reactant agent. Reaction temperature ranges from about -20 °C to 50 °C, preferably about 0 °C to 40 °C.

Scheme 7

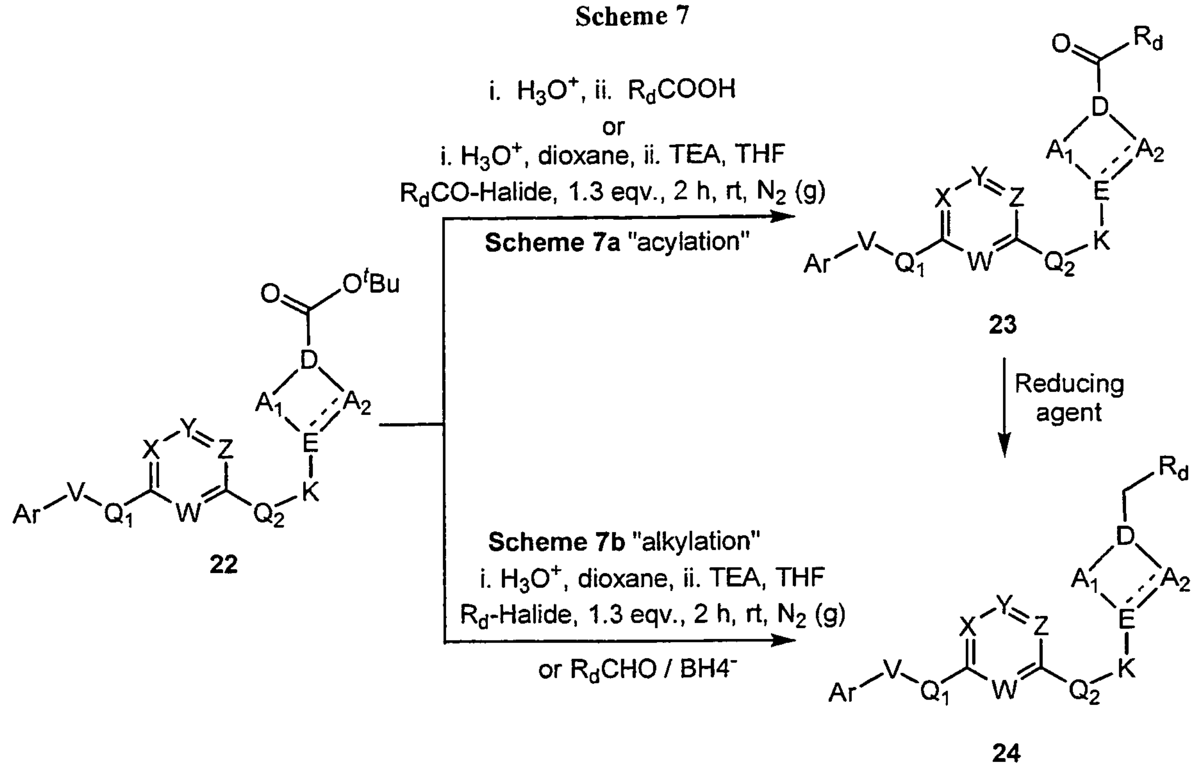


Alternatively, amides **23** of the present invention can be obtained by an amidation reaction using an acid halide (such as, $R_dCOCl$) and a base in an inert solvent (**Scheme 7a**). The base includes an alkali metal carbonate (such as, sodium carbonate, potassium carbonate, and the like), an alkali metal hydrogencarbonate (such as, sodium hydrogencarbonate, potassium hydrogencarbonate, and the like), an alkali hydroxide (such as, sodium hydroxide or potassium hydroxide, and like), a tertiary amine (such as, *N,N*-diisopropylethylamine, triethylamine, *N*-methylmorpholine, and the like), or an aromatic amine (such as, pyridine, imidazole, poly-(4-vinylpyridine), and the like). The inert solvent includes lower halocarbon solvents (such as, dichloromethane, dichloroethane, chloroform, and the like), ethereal solvents (such as, tetrahydrofuran, dioxane, and the like), amide solvents (such as, *N,N*-dimethylacetamide, *N,N*-dimethylformamide, and the like), aromatic solvents (benzene, toluene, pyridine, and the like) and mixtures thereof. Reaction temperature ranges from about -20 °C to 50 °C, preferably about 0 °C to 40 °C.

Also illustrated in **Scheme 7,** amide **23** can be reacted with a reducing agent in an inert solvent to provide the amine **24** of the present invention. The reducing agent includes alkali metal aluminum hydrides (such as, lithium aluminum hydride, and the like), alkali metal borohydrides (such as, lithium borohydride, and the like), alkali metal trialkoxyaluminum hydrides (such as, lithium tri-*tert*-butoxyaluminum hydride, and the like), dialkylaluminum hydrides (such as, di-isobutylaluminum hydride, and the like), borane, dialkylboranes (such as, di-isoamyl borane, and the like), alkali metal trialkylboron hydrides (such as, lithium triethylboron hydride, and the like). The inert solvent includes ethereal solvents (such as, tetrahydrofuran, dioxane, and the like), aromatic solvents (such as, toluene, and the like) and mixtures thereof. Reaction temperature ranges from about -78 ˚C to 200 ˚C, such as, about 50 ˚C to 120 ˚C.

Alternatively, the amine **24** of the present invention can be obtained by a reductive amination reaction using the acid deprotected secondary amine intermediate with an aldehyde ($R_6CHO$) and a reducing agent in an inert solvent with or without an acid. The reducing agent includes sodium triacetoxyborohydride, sodium cyanoborohydride, sodium borohydride, borane-pyridine complex, and the like. The inert solvent includes lower alkyl alcohol solvents (such as, methanol, ethanol, and the like), lower halocarbon solvents (such as, dichloromethane, dichloroethane, chloroform, and the like), ethereal solvents (such as, tetrahydrofuran, dioxane, and the like), aromatic solvents (such as, benzene, toluene, and the like) and mixtures thereof. The acid includes an inorganic acid (such as, hydrochloric acid, sulfuric acid, and the like) or an organic acid (such as, acetic acid, and the like). Reaction temperature ranges from about -20 ˚C to 120 ˚C, preferably about 0 ˚C to 100˚C. In addition, this reaction can optionally be carried out under microwave conditions.

In an alternative manner, the intermediate amine product of acid deprotection of **22** can be alkylated directly with an alkylating agent, such as $R_6$-halide (wherein $R_6$ is substituted or unsubstituted $C_{1-6}$ alkyl, or substituted or unsubstituted $C_{1-6}$ alkyl-Ar, and halide is chloro, bromo and iodo), in the presence of a base and in an inert solvent to provide amine **24.** The base includes an alkali metal carbonate (such as, sodium carbonate, potassium carbonate, and the like), an alkali metal hydride (such as, sodium hydride, potassium hydride, and the like), alkali metal alkoxide (such as, potassium *tert*-butoxide, sodium *tert*-butoxide, and the like); alkyl lithiums (such as, *tert*-butyl lithium, *n*-butyl lithium and the like). The inert solvents include, ethereal solvents (such as, tetrahydrofuran, dioxane), aromatic solvents (such as, benzene, toluene, and the like), amide solvents (such as, *N,N*-dimethylformamide, and the like) and mixtures thereof. Reaction temperature ranges from about -20˚C to 120 ˚C, preferably about 0˚C to 100˚C.

Also shown in Scheme 7 is the preparation of additional compounds of the invention via alkylating the nitrogen of ureas represented by **23** with an alkyl-halide (wherein halide is chloro, bromo and iodo) in the presence of a base in an inert solvent to provide di-substituted urea. The base includes an alkali metal hydride (such as, sodium hydride, potassium hydride, and the like), alkali metal alkoxide (such as, potassium *tert*-butoxide, sodium *tert*-butoxide, and the like); alkyl lithiums (such as, *tert*-butyl lithium, *n*-butyl lithium and the like). The inert solvents include, ethereal solvents (such as, tetrahydrofuran, dioxane), aromatic solvents (such as, benzene, toluene, and the like), amide solvents (such as, *N,N*-dimethylformamide, and the like) and mixtures thereof. Reaction temperature ranges from about -20˚C to 120˚C, preferably about 0 ˚C to 100˚C.

In addition, as illustrated in Scheme **8a,** urea **25a** can be obtained from deprotecting common intermediate **18** and allowing the amine (i.e., D = NH) to react with a variety isocyanates ($R_aNCO$, wherein $R_a$ has the same meaning as described herein) in an inert solvent with or without a base. Suitable bases include an alkali metal carbonate (such as, sodium carbonate, potassium carbonate, and the like), an alkali metal hydrogencarbonate (such as, sodium hydrogencarbonate, potassium hydrogencarbonate, and the like), an alkali hydroxide (such as, sodium hydroxide, potassium hydroxide, and the like), a tertiary amine (such as, *N,N*-diisopropylethylamine, triethylamine, *N*-methylmorpholine, and the like), or an aromatic amine (such as, pyridine, imidazole, and the like). The inert solvent includes lower halocarbon solvents (such as, dichloromethane, dichloroethane, chloroform, and the like), ethereal solvents (such as, tetrahydrofuran, dioxane, and the like), aromatic solvents (such as, benzene, toluene, and the like), or polar solvents (such as, *N,N*-dimethylformamide, dimethyl sulfoxide, and the like). Reaction temperature ranges from about -20 ˚C to 120 ˚C, preferably about 0˚C to 100˚C.

**Scheme 8**

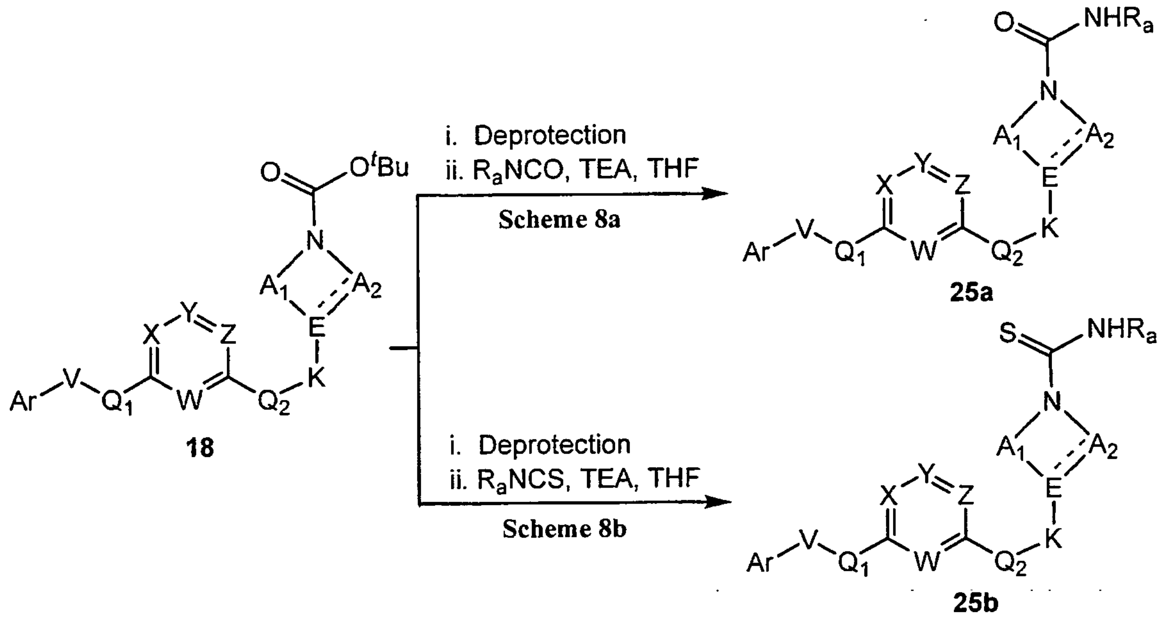


18 25a 25b

Further, as illustrated in Scheme **8b,** thiourea **25b** can be obtained from deprotecting common intermediate **18** and allowing the amine (i.e., D = NH) to react with a variety thioisocyanates ($R_aNCS$, wherein $R_a$ has the same meaning as described herein) in an inert solvent with or without a base. Suitable bases include an alkali metal carbonate (such as, sodium carbonate, potassium carbonate, and the like), an alkali metal hydrogencarbonate (such as, sodium hydrogencarbonate, potassium hydrogencarbonate, and the like), an alkali hydroxide (such as, sodium hydroxide, potassium hydroxide, and the like), a tertiary amine (such as, *N,N*-diisopropylethylamine, triethylamine, *N*-methylmorpholine, and the like), or an aromatic amine (such as, pyridine, imidazole, and the like). The inert solvent includes lower halocarbon solvents (such as, dichloromethane, dichloroethane, chloroform, and the like), ethereal solvents (such as, tetrahydrofuran, dioxane, and the like), aromatic solvents (such as, benzene, toluene, and the like), or polar solvents (such as, *N,N*-dimethylformamide, dimethyl sulfoxide, and the like). Reaction temperature ranges from about -20 °C to 120 °C, preferably about 0 °C to 100 °C.

Scheme **9** illustrates the synthesis of ara-alkyl sulfones (**27**) which are used as aryl building blocks in **Scheme 4** of the present invention, wherein $R_{10}$, $R_{11}$, $R_{12}$, and $R_{13}$ have the same meaning as described herein. The common methods for preparing these sulfones include the oxidation of sulfides or the sulfonylation of arenes using aryl sulfonyl halides or aryl sulfonic acids in th presence of a strong acid catalyst (see for general reference: the Organic Chemistry of Sulfur; Oae S., Ed.; Plenum Press: New York, 1977). Optimal conversion to the optionally 2,5-disubstituted arene 27 was achieved thermally wherein Hal is preferably iodo using 5 mol % $(CuOTf)_2$·PhH and 10 mol % N,N'-dimethylethylenediamine in DMSO by the method of Wang et al (see for reference Wang Z.; Baskin J. M., Org. Lett., 2002, 4, 25, 4423-4425). In some embodiments, $R_{10}$ and $R_{13}$ are each independently H, halogen, or $C_{1-6}$ alkyl; $R_{11}$ and $R_{12}$ are both H; Hal = Br, I; and Q1 = OH, or $NH_2$.

**Scheme 9**

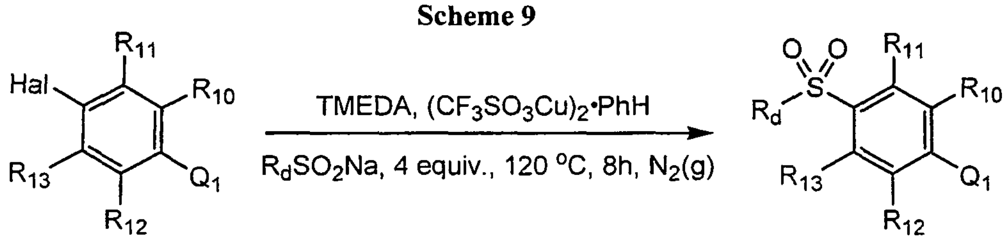


26 27

Alternative standard organic synthetic methods may be used to introduce alternate substituents in to the Ar component. In one example wherein the linker atom is $Q_1$ = N, the manipulation maybe carried out by protecting the

aniline amino functionality using standard FmocCl and CbzCl protection deprotection steps familiar to one skilled in the art (**Scheme 10,** wherein $R_{10}$, $R_{11}$, $R_{12}$, and $R_{13}$ have the same meaning as described herein) and subsequently using the deprotected aniline in subsequent steps such as those depicted in **Scheme 4.** Nitrile 29, maybe alternatively transformed in to amidine **A84, A90** or **A103** (see Table A) by using hydroxylamine HCl followed by reduction using zinc / acetic acid. In some embodiments of the invention $R_{10}$ is halogen, and $R_{13}$ is H or halogen.

**Scheme 10**

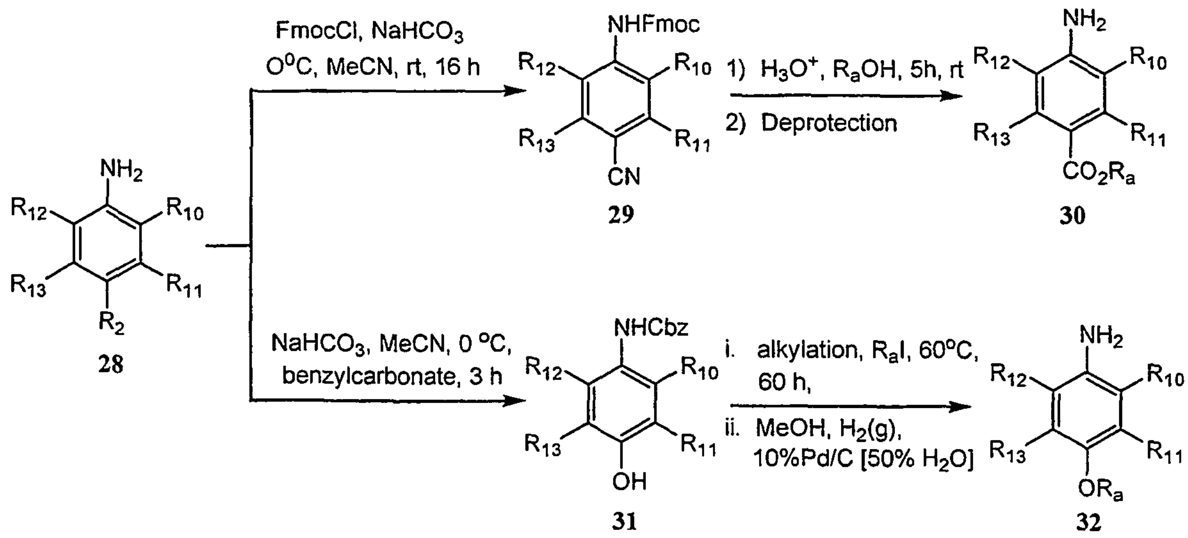


28, 29, 30, 31, 32

Synthesis of the 3,5-oxadiazolo variant is depicted in **Scheme 11**. Zinc(II)chloride catalysed coupling of amidoxime 34 with 4-hydroxypiperidine, CNBr derived 36 yielded building block 37 after acidic workup, which was subsequently utilized in reaction sequences depicted as illustrated in **Scheme 3**.

**Scheme 11**

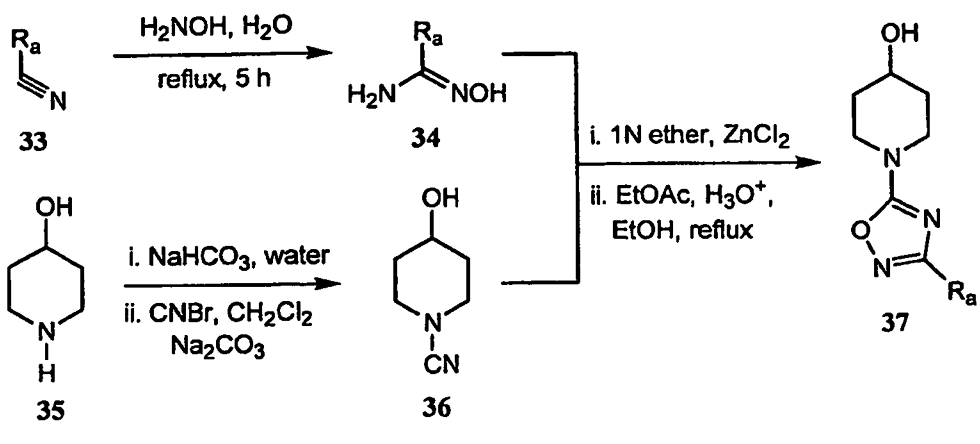


33, 34, 35, 36, 37

Protecting groups may be required for various functionality or functionalities during the synthesis of some of the compounds of the invention. Accordingly, representative protecting groups that are suitable for a wide variety of synthetic transformations are disclosed in Greene and Wuts, Protective Groups in Organic Synthesis, 3rd edition, John Wiley & Sons, New York, 1999 .

The present invention also encompasses diastereomers as well as optical isomers, e.g. mixtures of enantiomers including racemic mixtures, as well as individual enantiomers and diastereomers, which arise as a consequence of structural asymmetry in certain compounds of Formula (I). Separation of the individual isomers or selective synthesis of the individual isomers is accomplished by application of various methods which are well known to practitioners in the art.

## INDICATIONS AND METHODS OF TREATMENT

In addition to the foregoing beneficial uses for compounds of the present invention disclosed herein, compounds of the invention are useful in the treatment of additional diseases. Without limitation, these include the following.

The most significant pathologies in Type II diabetes are impaired insulin signaling at its target tissues ("insulin resistance") and failure of the insulin-producing cells of the pancreas to secrete an appropriate degree of insulin in response to a hyperglycemic signal. Current therapies to treat the latter include inhibitors of the β-cell ATP-sensitive potassium channel to trigger the release of endogenous insulin stores, or administration of exogenous insulin. Neither of these achieves accurate normalization of blood glucose levels and both carry the risk of inducing hypoglycemia. For these reasons, there has been intense interest in the development of pharmaceuticals that function in a glucose-dependent action, i.e. potentiators of glucose signaling. Physiological signaling systems which function in this manner are well-characterized and include the gut peptides GLP1, GIP and PACAP. These hormones act via their cognate G-protein coupled receptor to stimulate the production of cAMP in pancreatic β-cells. The increased cAMP does not appear to result in stimulation of insulin release during the fasting or preprandial state. However, a series of biochemical targets of cAMP signaling, including the ATP-sensitive potassium channel, voltage-sensitive potassium channels and the exocytotic machinery, are modified in such a way that the insulin secretory response to a postprandial glucose stimulus is markedly enhanced. Accordingly, agonists of novel, similarly functioning, β-cell GPCRs, including **RUP3,** would also stimulate the release of endogenous insulin and consequently promote normoglycemia in Type II diabetes.

It is also established that increased cAMP, for example as a result of GLP1 stimulation, promotes β-cell proliferation, inhibits β-cell death and thus improves islet mass. This positive effect on β-cell mass is expected to be beneficial in both Type II diabetes, where insufficient insulin is produced, and Type I diabetes, where β-cells are destroyed by an inappropriate autoimmune response.

Some β-cell GPCRs, including **RUP3,** are also present in the hypothalamus where they modulate hunger, satiety, decrease food intake, controlling or decreasing weight and energy expenditure. Hence, given their function within the hypothalamic circuitry, agonists or inverse agonists of these receptors mitigate hunger, promote satiety and therefore modulate weight.

It is also well-established that metabolic diseases exert a negative influence on other physiological systems. Thus, there is often the codevelopment of multiple disease states (e.g. type I diabetes, type II diabetes, inadequate glucose tolerance, insulin resistance, hyperglycemia, hyperlipidemia, hypertriglyceridemia, hypercholesterolemia, dyslipidemia, obesity or cardiovascular disease in "Syndrome X") or secondary diseases which clearly occur secondary to diabetes (e.g. kidney disease, peripheral neuropathy). Thus, it is expected that effective treatment of the diabetic condition will in turn be of benefit to such interconnected disease states.

In some embodiments of the present invention the metabolic-related disorder is hyperlipidemia, type 1 diabetes, type 2 diabetes mellitus, idiopathic type 1 diabetes (Type 1b), latent autoimmune diabetes in adults (LADA), early-onset type 2 diabetes (EOD), youth-onset atypical diabetes (YOAD), maturity onset diabetes of the young (MODY), malnutrition-related diabetes, gestational diabetes, coronary heart disease, ischemic stroke, restenosis after angioplasty, peripheral vascular disease, intermittent claudication, myocardial infarction (e.g. necrosis and apoptosis), dyslipidemia, post-prandial lipemia, conditions of impaired glucose tolerance (IGT), conditions of impaired fasting plasma glucose, metabolic acidosis, ketosis, arthritis, obesity, osteoporosis, hypertension, congestive heart failure, left ventricular hypertrophy, peripheral arterial disease, diabetic retinopathy, macular degeneration, cataract, diabetic nephropathy, glomerulosclerosis, chronic renal failure, diabetic neuropathy, metabolic syndrome, syndrome X, premenstrual syndrome, coronary heart disease, angina pectoris, thrombosis, atherosclerosis, myocardial infarction, transient ischemic attacks, stroke, vascular restenosis, hyperglycemia, hyperinsulinemia, hyperlipidemia, hypertrygliceridemia, insulin resistance, impaired glucose metabolism, conditions of impaired glucose tolerance, conditions of impaired fasting plasma glucose, obesity, erectile dysfunction, skin and connective tissue disorders, foot ulcerations and ulcerative colitis, endothelial dysfunction and impaired vascular compliance.

Described herein are methods for treatment of a metabolic-related disorder in an individual comprising administering to the individual in need of such treatment a therapeutically effective amount of a compound as described herein or a pharmaceutical composition thereof. In some embodiments the metabolic-related disorder is type I diabetes, type II diabetes, inadequate glucose tolerance, insulin resistance, hyperglycemia, hyperlipidemia, hypertriglyceridemia, hypercholesterolemia, dyslipidemia or syndrome X. In some embodiments the metabolic-related disorder is type II diabetes. In some embodiments the metabolic-related disorder is hyperglycemia. In some embodiments the metabolic-related disorder is hyperlipidemia. In some embodiments the metabolic-related disorder is hypertriglyceridemia. In some embodiments the metabolic-related disorder is type I diabetes. In some embodiments the metabolic-related disorder is dyslipidemia. In some embodiments the metabolic-related disorder is syndrome X. In some embodiments the individual is a mammal. In some embodiments the mammal is a human.

Described herein are methods of decreasing food intake of an individual comprising administering to the individual in need thereof a therapeutically effective amount of a compound of the present invention or pharmaceutical

composition thereof. In some embodiments the individual is a mammal. In some embodiments the mammal is a human.

Described herein are methods of inducing satiety in an individual comprising administering to the individual in need of such treatment a therapeutically effective amount of a compound of the present invention or pharmaceutical composition thereof. In some embodiments the individual is a mammal. In some embodiments the mammal is a human.

Described herein are methods of controlling or decreasing weight gain of an individual comprising administering to the individual in need of such treatment a therapeutically effective amount of a compound of the present invention or pharmaceutical composition thereof. In some embodiments the individual is a mammal. In some embodiments the mammal is a human.

Described herein are methods wherein the human has a body mass index of about 18.5 to about 45. In some embodiments, the human has a body mass index of about 25 to about 45. In some embodiments, the human has a body mass index of about 30 to about 45. In some embodiments, the human has a body mass index of about 35 to about 45.

Described herein are methods of modulating a **RUP3** receptor in an individual comprising contacting the receptor with a compound according to any one of claims 1 to 127. In some embodiments, the compound is an agonist. In some embodiments, the compound is an inverse agonist. In some embodiments, the compound is an antagonist. In some embodiments, the modulation of the **RUP3** receptor is treatment of a metabolic-related disorder and complications thereof. In some embodiments, the metabolic-related disorder is type I diabetes, type II diabetes, inadequate glucose tolerance, insulin resistance, hyperglycemia, hyperlipidemia, hypertriglyceridemia, hypercholesterolemia, dyslipidemia or syndrome X. In some embodiments, the metabolic-related disorder is type II diabetes. In some embodiments, the metabolic-related disorder is hyperglycemia. In some embodiments, the metabolic-related disorder is hyperlipidemia. In some embodiments, the metabolic-related disorder is hypertriglyceridemia. In some embodiments, the metabolic-related disorder is type I diabetes. In some embodiments, the metabolic-related disorder is dyslipidemia. In some embodiments, the metabolic-related disorder is syndrome X. In some embodiments, the individual is a mammal. In some embodiments, the mammal is a human.

Described herein is a method of modulating a **RUP3** receptor in an individual comprising contacting the receptor with a compound of the present invention wherein the modulation of the **RUP3** receptor reduces food intake of the individual. In some embodiments the individual is a mammal. In some embodiments the mammal is a human. In some embodiments the human has a body mass index of about 18.5 to about 45. In some embodiments the human has a body mass index of about 25 to about 45. In some embodiments the human has a body mass index of about 30 to about 45. In some embodiments the human has a body mass index of about 35 to about 45.

Described herein is a method of modulating a **RUP3** receptor in an individual comprising contacting the receptor with a compound of the present invention wherein the modulation of the **RUP3** receptor induces satiety in the individual. In some embodiments the individual is a mammal. In some embodiments the mammal is a human. In some embodiments the human has a body mass index of about 18.5 to about 45. In some embodiments the human has a body mass index of about 25 to about 45. In some embodiments the human has a body mass index of about 30 to about 45. In some embodiments the human has a body mass index of about 35 to about 45.

Described herein is a method of modulating a **RUP3** receptor in an individual comprising contacting the receptor with a compound of the present invention wherein the modulation of the **RUP3** receptor controls or reduces weight gain of the individual. In some embodiments the individual is a mammal. In some embodiments the mammal is a human. In some embodiments the human has a body mass index of about 18.5 to about 45. In some embodiments the human has a body mass index of about 25 to about 45. In some embodiments the human has a body mass index of about 30 to about 45. In some embodiments the human has a body mass index of about 35 to about 45.

One aspect of the present invention pertains to use of a compound as described herein, for production of a medicament for use in treatment of a metabolic-related disorder. In some embodiments, the metabolic-related disorder is type II diabetes, inadequate glucose tolerance, insulin resistance, hyperglycemia, hyperlipidemia, hypertriglyceridemia, hypercholesterolemia, dyslipidemia or syndrome X.

One aspect of the present invention pertains to use of a compound as described herein, for production of a medicament for use in decreasing food intake of an individual. In some embodiments, the individual is a mammal. In some embodiments, the mammal is a human. In some embodiments, the human has a body mass index of about 18.5 to about 45. In some embodiments, the human has a body mass index of about 25 to about 45. In some embodiments, the human has a body mass index of about 30 to about 45. In some embodiments, the human has a body mass index of about 35 to about 45.

One aspect of the present invention pertains to use of a compound as described herein, for production of a medicament for use of inducing satiety in an individual. In some embodiments, the individual is a mammal. In some embodiments, the mammal is a human. In some embodiments, the human has a body mass index of about 18.5 to about 45. In some embodiments, the human has a body mass index of about 25 to about 45. In some embodiments, the human has a body mass index of about 30 to about 45. In some embodiments, the human has a body mass index of about 35 to about 45.

One aspect of the present invention pertains to use of a compound as described herein, for production of a

medicament for use in controlling or decreasing weight gain in an individual. In some embodiments, the individual is a mammal. In some embodiments, the mammal is a human. In some embodiments, the human has a body mass index of about 18.5 to about 45. In some embodiments, the human has a body mass index of about 25 to about 45. In some embodiments, the human has a body mass index of about 30 to about 45. In some embodiments, the human has a body mass index of about 35 to about 45.

One aspect of the present invention pertains to a compound, as described herein, for use in a method of treatment of the human or animal body by therapy.

One aspect of the present invention pertains to a compound, as described herein, for use in a method of treatment of a metabolic-related disorder of the human or animal body by therapy.

## PHARMACEUTICAL COMPOSITIONS AND SALTS

A further aspect of the present invention pertains to pharmaceutical compositions comprising one or more compounds of Formula (**I**) or any formula disclosed herein, and one or more pharmaceutically acceptable carriers. Some embodiments of the present invention pertain to pharmaceutical compositions comprising a compound of Formula (**I**) and a pharmaceutically acceptable carrier.

Some embodiments of the present invention include a method of producing a pharmaceutical composition comprising admixing at least one compound according to any of the compound embodiments disclosed herein and a pharmaceutically acceptable carrier.

Formulations may be prepared by any suitable method, typically by uniformly mixing the active compound(s) with liquids or finely divided solid carriers, or both, in the required proportions, and then, if necessary, forming the resulting mixture into a desired shape.

Conventional excipients, such as binding agents, fillers, acceptable wetting agents, tabletting lubricants, and disintegrants may be used in tablets and capsules for oral administration. Liquid preparations for oral administration may be in the form of solutions, emulsions, aqueous or oily suspensions, and syrups. Alternatively, the oral preparations may be in the form of dry powder that can be reconstituted with water or another suitable liquid vehicle before use. Additional additives such as suspending or emulsifying agents, non-aqueous vehicles (including edible oils), preservatives, and flavorings and colorants may be added to the liquid preparations. Parenteral dosage forms may be prepared by dissolving the compound of the invention in a suitable liquid vehicle and filter sterilizing the solution before filling and sealing an appropriate vial or ampoule. These are just a few examples of the many appropriate methods well known in the art for preparing dosage forms.

A compound of the present invention can be formulated into pharmaceutical compositions using techniques well known to those in the art. Suitable pharmaceutically-acceptable carriers, outside those mentioned herein, are known in the art; for example, see Remington, The Science and Practice of Pharmacy, 20th Edition, 2000, Lippincott Williams & Wilkins, (Editors: Gennaro, A. R., et al.).

While it is possible that, for use in the treatment, a compound of the invention may, in an alternative use, be administered as a raw or pure chemical, it is preferable however to present the compound or active ingredient as a pharmaceutical formulation or composition further comprising a pharmaceutically acceptable carrier.

The invention thus further provides pharmaceutical formulations comprising a compound of the invention or a pharmaceutically acceptable salt or derivative thereof together with one or more pharmaceutically acceptable carriers thereof and/or prophylactic ingredients. The carrier(s) must be "acceptable" in the sense of being compatible with the other ingredients of the formulation and not overly deleterious to the recipient thereof.

Pharmaceutical formulations include those suitable for oral, rectal, nasal, topical (including buccal and sub-lingual), vaginal or parenteral (including intramuscular, sub-cutaneous and intravenous) administration or in a form suitable for administration by inhalation, insufflation or by a transdermal patch. Transdermal patches dispense a drug at a controlled rate by presenting the drug for absorption in an efficient manner with a minimum of degradation of the drug. Typically, transdermal patches comprise an impermeable backing layer, a single pressure sensitive adhesive and a removable protective layer with a release liner. One of ordinary skill in the art will understand and appreciate the techniques appropriate for manufacturing a desired efficacious transdermal patch based upon the needs of the artisan.

The compounds of the invention, together with a conventional adjuvant, carrier, or diluent, may thus be placed into the form of pharmaceutical formulations and unit dosages thereof, and in such form may be employed as solids, such as tablets or filled capsules, or liquids such as solutions, suspensions, emulsions, elixirs, gels or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

For oral administration, the pharmaceutical composition may be in the form of, for example, a tablet, capsule,

suspension or liquid. The pharmaceutical composition is preferably made in the form of a dosage unit containing a particular amount of the active ingredient. Examples of such dosage units are capsules, tablets, powders, granules or a suspension, with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethyl-cellulose; and with lubricants such as talc or magnesium stearate. The active ingredient may also be administered by injection as a composition wherein, for example, saline, dextrose or water may be used as a suitable pharmaceutically acceptable carrier.

Compounds of the present invention, including pharmaceutically acceptable salts and solvates thereof, can be used as active ingredients in pharmaceutical compositions, specifically as **RUP3** receptor modulators. By the term "active ingredient" is defined in the context of a "pharmaceutical composition" and shall mean a component of a pharmaceutical composition that provides the primary pharmacological effect, as opposed to an "inactive ingredient" which would generally be recognized as providing no pharmaceutical benefit.

The dose when using the compounds of the present invention can vary within wide limits, and as is customary and is known to the physician, it is to be tailored to the individual conditions in each individual case. It depends, for example, on the nature and severity of the illness to be treated, on the condition of the patient, on the compound employed or on whether an acute or chronic disease state is treated or prophylaxis is conducted or on whether further active compounds are administered in addition to the compounds of the present invention. Representative doses of the present invention include, but not limited to, about 0.001 mg to about 5000 mg, about 0.001 to about 2500 mg, about 0.001 to about 1000 mg, 0.001 to about 500 mg, 0.001 mg to about 250 mg, about 0.001 mg to 100 mg, about 0.001 mg to about 50 mg, and about 0.001 mg to about 25 mg. Multiple doses may be administered during the day, especially when relatively large amounts are deemed to be needed, for example 2, 3 or 4, doses. Depending on the individual and as deemed appropriate from the patient's physician or care-giver it may be necessary to deviate upward or downward from the doses described herein.

The amount of active ingredient, or an active salt or derivative thereof, required for use in treatment will vary not only with the particular salt selected but also with the route of administration, the nature of the condition being treated and the age and condition of the patient and will ultimately be at the discretion of the attendant physician or clinician. In general, one skilled in the art understands how to extrapolate in vivo data obtained in a model system, typically an animal model, to another, such as a human. Typically, animal models include, but are not limited to, the rodent diabetes model as described in Example 5, *infra* (as well as other animal models known in the art, such as those reported by Reed and Scribner in Diabetes, Obesity and Metabolism, 1, 1999, 75-86). In some circumstances, these extrapolations may merely be based on the weight of the animal in the respective model in comparison to another, such as a mammal, preferably a human, however, more often, these extrapolations are not simply based on weights, but rather incorporate a variety of factors. Representative factors include, but not limited to, the type, age, weight, sex, diet and medical condition of the patient, the severity of the disease, the route of administration, pharmacological considerations such as the activity, efficacy, pharmacokinetic and toxicology profiles of the particular compound employed, whether a drug delivery system is utilized, on whether an acute or chronic disease state is being treated or prophylaxis is conducted or on whether further active compounds are administered in addition to the compounds of the Formula (**I**) and as part of a drug combination. The dosage regimen for treating a disease condition with the compounds and/or compositions of this invention is selected in accordance with a variety factors as cited above. Thus, the actual dosage regimen employed may vary widely and therefore may deviate from a preferred dosage regimen and one skilled in the art will recognize that dosage and dosage regimen outside these typical ranges can be tested and, where appropriate, may be used in the methods of this invention.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations. The daily dose can be divided, especially when relatively large amounts are administered as deemed appropriate, into several, for example 2, 3 or 4, part administrations. If appropriate, depending on individual behavior, it may be necessary to deviate upward or downward from the daily dose indicated.

The compounds of the present invention can be administrated in a wide variety of oral and parenteral dosage forms. It will be obvious to those skilled in the art that the following dosage forms may comprise, as the active component, either a compound of the invention or a pharmaceutically acceptable salt of a compound of the invention.

For preparing pharmaceutical compositions from the compounds of the present invention, the selection of a suitable pharmaceutically acceptable carrier can be either solid, liquid or a mixture of both. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component.

In tablets, the active component is mixed with the carrier having the necessary binding capacity in suitable

proportions and compacted to the desire shape and size.

The powders and tablets may contain varying percentage amounts of the active compound. A representative amount in a powder or tablet may contain from 0.5 to about 90 percent of the active compound; however, an artisan would know when amounts outside of this range are necessary. Suitable carriers for powders and tablets are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as an admixture of fatty acid glycerides or cocoa butter, is first melted and the active component is dispersed homogeneously therein, as by stirring. The molten homogenous mixture is then poured into convenient sized molds, allowed to cool, and thereby to solidify.

Formulations suitable for vaginal administration may be presented as pessaries, tampons, creams, gels, pastes, foams or sprays containing in addition to the active ingredient such carriers as are known in the art to be appropriate.

Liquid form preparations include solutions, suspensions, and emulsions, for example, water or water-propylene glycol solutions. For example, parenteral injection liquid preparations can be formulated as solutions in aqueous polyethylene glycol solution. Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The pharmaceutical compositions may take such forms as suspensions, solutions, or emulsions in oily or aqueous vehicles, and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous formulations suitable for oral use can be prepared by dissolving or suspending the active component in water and adding suitable colorants, flavours, stabilizing and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

For topical administration to the epidermis the compounds according to the invention may be formulated as ointments, creams or lotions, or as a transdermal patch.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

Formulations suitable for topical administration in the mouth include lozenges comprising active agent in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations may be provided in single or multi-dose form. In the latter case of a dropper or pipette, this may be achieved by the patient administering an appropriate, predetermined volume of the solution or suspension. In the case of a spray, this may be achieved for example by means of a metering atomizing spray pump.

Administration to the respiratory tract may also be achieved by means of an aerosol formulation in which the active ingredient is provided in a pressurized pack with a suitable propellant. If the compounds of the Formula (I) or pharmaceutical compositions comprising them are administered as aerosols, for example as nasal aerosols or by inhalation, this can be carried out, for example, using a spray, a nebulizer, a pump nebulizer, an inhalation apparatus, a

metered inhaler or a dry powder inhaler. Pharmaceutical forms for administration of the compounds of the Formula (I) as an aerosol can be prepared by processes well-known to the person skilled in the art. For their preparation, for example, solutions or dispersions of the compounds of the Formula (I) in water, water/alcohol mixtures or suitable saline solutions can be employed using customary additives, for example benzyl alcohol or other suitable preservatives, absorption enhancers for increasing the bioavailability, solubilizers, dispersants and others, and, if appropriate, customary propellants, for example include carbon dioxide, CFC's, such as, dichlorodifluoromethane, trichlorofluoromethane, or dichlorotetrafluoroethane; and the like. The aerosol may conveniently also contain a surfactant such as lecithin. The dose of drug may be controlled by provision of a metered valve.

In formulations intended for administration to the respiratory tract, including intranasal formulations, the compound will generally have a small particle size for example of the order of 10 microns or less. Such a particle size may be obtained by means known in the art, for example by micronization. When desired, formulations adapted to give sustained release of the active ingredient may be employed.

Alternatively the active ingredients may be provided in the form of a dry powder, for example, a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration are preferred compositions.

The compounds according to the invention may optionally exist as pharmaceutically acceptable salts including pharmaceutically acceptable acid addition salts prepared from pharmaceutically acceptable non-toxic acids including inorganic and organic acids. Representative acids include, but are not limited to, acetic, benzenesulfonic, benzoic, camphorsulfonic, citric, ethenesulfonic, dichloroacetic, formic, fumaric, gluconic, glutamic, hippuric, hydrobromic, hydrochloric, isethionic, lactic, maleic, malic, mandelic, methanesulfonic, mucic, nitric, oxalic, pamoic, pantothenic, phosphoric, succinic, sulfiric, tartaric, oxalic, p-toluenesulfonic and the like, such as those pharmaceutically acceptable salts listed in Journal of Pharmaceutical Science, 66, 2 (1977).

The acid addition salts may be obtained as the direct product of compound synthesis. In the alternative, the free base may be dissolved in a suitable solvent containing the appropriate acid, and the salt isolated by evaporating the solvent or otherwise separating the salt and solvent. The compounds of this invention may form solvates with standard low molecular weight solvents using methods known to the skilled artisan.

In addition, compounds according to the invention may optionally exist as pharmaceutically acceptable basic addition salts. For example, these salts can be prepared in situ during the final isolation and purification of the compounds of the invention, or separately by reacting an acidic moiety, such as a carboxylic acid, with a suitable base such as the hydroxide, carbonate or bicarbonate of a pharmaceutically acceptable metal cation or with ammonia, or an organic primary, secondary or tertiary amine. Pharmaceutically acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine and the like.

Compounds of the present invention can be converted to "pro-drugs." The term "pro-drugs" refers to compounds that have been modified with specific chemical groups known in the art and when administered into an individual these groups undergo biotransformation to give the parent compound. Pro-drugs can thus be viewed as compounds of the invention containing one or more specialized non-toxic protective groups used in a transient manner to alter or to eliminate a property of the compound. In one general aspect, the "pro-drug" approach is utilized to facilitate oral absorption. A thorough discussion is provided in T. Higuchi and V. Stella, "Pro-drugs as Novel Delivery Systems," Vol. 14 of the A.C.S. Symposium Series; and in Bioreversible Carriers in Drug Design, ed. Edward B. Roche, American Pharmaceutical Association and Pergamon Press, 1987 .

Some embodiments of the present invention include a method of producing a pharmaceutical composition for "combination-therapy" comprising admixing at least one compound according to any of the compound embodiments disclosed herein, together with at least one known pharmaceutical agent as described herein and a pharmaceutically acceptable carrier.

In some embodiments the pharmaceutical agents is selected from the group consisting of: sulfonylureas, meglitinides, biguanides, $\alpha$-glucosidase inhibitors, peroxisome proliferators-activated receptor-$\gamma$ (i.e., PPAR-$\gamma$) agonists,

insulin, insulin analogues, HMG-CoA reductase inhibitors, cholesterol-lowering drugs (for example, fibrates that include: fenofibrate, bezafibrate, gemfibrozil, clofibrate and the like; bile acid sequestrants which include: cholestyramine, colestipol and the like; and niacin), antiplatelet agents (for example, aspirin and adenosine diphosphate receptor antagonists that include: clopidogrel, ticlopidine and the like), angiotensin-converting enzyme inhibitors, angiotensin II receptor antagonists and adiponectin.

It is noted that when the **RUP3** receptor modulators are utilized as active ingredients in a pharmaceutical composition, these are not intended for use only in humans, but in other non-human mammals as well. Indeed, recent advances in the area of animal health-care mandate that consideration be given for the use of active agents, such as **RUP3** receptor modulators, for the treatment of obesity in domestic animals (*e.g.*, cats and dogs), and **RUP3** receptor modulators in other domestic animals where no disease or disorder is evident (*e.g.*, food-oriented animals such as cows, chickens, fish, etc.). Those of ordinary skill in the art are readily credited with understanding the utility of such compounds in such settings.

## COMBINATION THERAPY

In the context of the present invention, a compound of Formula (**I**) or pharmaceutical composition thereof can be utilized for modulating the activity of **RUP3** receptor mediated diseases, conditions and/or disorders as described herein. Examples of modulating the activity of **RUP3** receptor mediated diseases include the treatment of metabolic related disorders. Metabolic related disorders includes, but not limited to, hyperlipidemia, type 1 diabetes, type 2 diabetes mellitus, and conditions associated therewith, such as, but not limited to coronary heart disease, ischemic stroke, restenosis after angioplasty, peripheral vascular disease, intermittent claudication, myocardial infarction (e.g. necrosis and apoptosis), dyslipidemia, post-prandial lipemia, conditions of impaired glucose tolerance (IGT), conditions of impaired fasting plasma glucose, metabolic acidosis, ketosis, arthritis, obesity, osteoporosis, hypertension, congestive heart failure, left ventricular hypertrophy, peripheral arterial disease, diabetic retinopathy, macular degeneration, cataract, diabetic nephropathy, glomerulosclerosis, chronic renal failure, diabetic neuropathy, metabolic syndrome, syndrome X, premenstrual syndrome, coronary heart disease, angina pectoris, thrombosis, atherosclerosis, myocardial infarction, transient ischemic attacks, stroke, vascular restenosis, hyperglycemia, hyperinsulinemia, hyperlipidemia, hypertriglyceridemia, insulin resistance, impaired glucose metabolism, conditions of impaired glucose tolerance, conditions of impaired fasting plasma glucose, obesity, erectile dysfunction, skin and connective tissue disorders, foot ulcerations and ulcerative colitis, endothelial dysfunction and impaired vascular compliance. In some embodiments, metabolic related disorders include type I diabetes, type II diabetes, inadequate glucose tolerance, insulin resistance, hyperglycemia, hyperlipidemia, hypertriglyceridemia, hypercholesterolemia, dyslipidemia and syndrome X. Other examples of modulating the activity of **RUP3** receptor mediated diseases include the treatment of obesity and/or overweight by decreasing food intake, inducing satiation (i.e., the feeling of fullness), controlling weight gain, decreasing body weight and/or affecting metabolism such that the recipient loses weight and/or maintains weight.

While the compounds of the invention can be administered as the sole active pharmaceutical agent (i.e., monotherapy), they can also be used in combination with other pharmaceutical agents (i.e., combination-therapy) for the treatment of the diseases/conditions/disorders described herein. Therefore, described herein are methods of prophylaxis and/or treatment of a metabolic related disorder or a weight related disorder, such as obesity, comprising administering to an individual in need of prophylaxis and/or treatment a therapeutically effective amount of a compound of the present invention, for example Formula (**I**), in combination with one or more additional pharmaceutical agent as described herein.

Suitable pharmaceutical agents that can be used in combination with the compounds of the present invention include anti-obesity agents such as apolipoprotein-B secretion/microsomal triglyceride transfer protein (apo-B/MTP) inhibitors, MCR-4 agonists, cholescystokinin-A (CCK-A) agonists, serotonin and norepinephrine reuptake inhibitors (for example, sibutramine), sympathomimetic agents, β3 adrenergic receptor agonists, dopamine agonists (for example, bromocriptine), melanocyte-stimulating hormone receptor analogs, cannabinoid 1 receptor antagonists [for example, SR141716: N-(piperidin-1-yl)-5-(4-chlorophenyl)-1-(2,4-dichlorophenyl)-4-methyl-1H-pyrazole-3-carboxamide], melanin concentrating hormone antagonists, leptons (the OB protein), leptin analogues, leptin receptor agonists, galanin antagonists, lipase inhibitors (such as tetrahydrolipstatin, i.e., Orlistat), anorectic agents (such as a bombesin agonist), Neuropeptide-Y antagonists, thyromimetic agents, dehydroepiandrosterone or an analogue thereof, glucocorticoid receptor agonists or antagonists, orexin receptor antagonists, urocortin binding protein antagonists, glucagon-like peptide-1 receptor agonists, ciliary neutrotrophic factors (such as Axokine™ available from Regeneron Pharmaceuticals, Inc., Tarrytown, NY and Procter & Gamble Company, Cincinnati, OH), human agouti-related proteins (AGRP), ghrelin receptor antagonists, histamine 3 receptor antagonists or reverse agonists, neuromedin U receptor agonists, noradrenergic anorectic agents (for example, phentermine, mazindol and the like) and appetite suppressants (for example, bupropion).

Other anti-obesity agents, including the agents set forth *infra*, are well known, or will be readily apparent in light of the instant disclosure, to one of ordinary skill in the art.

In some embodiments, the anti-obesity agents are selected from the group consisting of orlistat, sibutramine,

bromocriptine, ephedrine, leptin, and pseudoephedrine. In a further embodiment, compounds of the present invention and combination therapies are administered in conjunction with exercise and/or a sensible diet.

It will be understood that the scope of combination-therapy of the compounds of the present invention with other anti-obesity agents, anorectic agents, appetite suppressant and related agents is not limited to those listed above, but includes in principle any combination with any pharmaceutical agent or pharmaceutical composition useful for the treatment of overweight and obese individuals.

Other suitable pharmaceutical agents, in addition to anti-obesity agents, that can be used in combination with the compounds of the present invention include agents useful in the treatment of metabolic related disorders and/or concomitant diseases thereof. For example, but not limited to, congestive heart failure, type I diabetes, type II diabetes, inadequate glucose tolerance, insulin resistance, hyperglycemia, hyperlipidemia, hypertriglyceridemia, hypercholesterolemia, dyslipidemia, syndrome X, retinopathy, nephropathy and neuropathy. Treatment of one or more of the diseases cited herein include the use of one or more pharmaceutical agents known in the art belonging to the classes of drugs referred to, but not limited to, the following: sulfonylureas, meglitinides, biguanides, α-glucosidase inhibitors, peroxisome proliferators-activated receptor-γ (i.e., PPAR-γ) agonists, insulin, insulin analogues, HMG-CoA reductase inhibitors, cholesterol-lowering drugs (for example, fibrates that include: fenofibrate, bezafibrate, gemfibrozil, clofibrate and the like; bile acid sequestrants which include: cholestyramine, colestipol and the like; and niacin), antiplatelet agents (for example, aspirin and adenosine diphosphate receptor antagonists that include: clopidogrel, ticlopidine and the like), angiotensin-converting enzyme inhibitors, angiotensin II receptor antagonists, adiponectin and the like. In accordance to one aspect of the present invention, a compound of the present can be used in combination with a pharmaceutical agent or agents belonging to one or more of the classes of drugs cited herein.

It will be understood that the scope of combination-therapy of the compounds of the present invention with other pharmaceutical agents is not limited to those listed herein, *supra* or *infra,* but includes in principle any combination with any pharmaceutical agent or pharmaceutical composition useful for the treatment of diseases, conditions or disorders that are linked to metabolic related disorders.

Described herein are methods of treatment of a disease, disorder, condition or complication thereof as described herein, comprising administering to an individual in need of such treatment a therapeutically effective amount or dose of a compound of the present invention in combination with at least one pharmaceutical agent selected from the group consisting of: sulfonylureas, meglitinides, biguanides, α-glucosidase inhibitors, peroxisome proliferators-activated receptor-γ (i.e., PPAR-γ) agonists, insulin, insulin analogues, HMG-CoA reductase inhibitors, cholesterol-lowering drugs (for example, fibrates that include: fenofibrate, bezafibrate, gemfibrozil, clofibrate and the like; bile acid sequestrants which include: cholestyramine, colestipol and the like; and niacin), antiplatelet agents (for example, aspirin and adenosine diphosphate receptor antagonists that include: clopidogrel, ticlopidine and the like), angiotensin-converting enzyme inhibitors, angiotensin II receptor antagonists and adiponectin. In some embodiments, compounds of the present invention and the pharmaceutical agents are administered separately. In further embodiments, compounds of the present invention and the pharmaceutical agents are administered together.

Suitable pharmaceutical agents that can be used in conjunction with compounds of the present invention include sulfonylureas. The sulfonylureas (SU) are drugs which promote secretion of insulin from pancreatic P cells by transmitting signals of insulin secretion via SU receptors in the cell membranes. Examples of the sulfonylureas include glyburide , glipizide, glimepiride and other sulfonylureas known in the art.

Suitable pharmaceutical agents that can be used in conjunction with compounds of the present invention include the meglitinides. The meglitinides are benzoic acid derivatives represent a novel class of insulin secretagogues. These agents target postprandial hyperglycemia and show comparable efficacy to sulfonylureas in reducing HbA1c. Examples of meglitinides include repaglinide, nateglinide and other meglitinides known in the art.

Suitable pharmaceutical agents that can be used in conjunction with compounds of the present invention include the biguanides. The biguanides represent a class of drugs that stimulate anaerobic glycolysis, increase the sensitivity to insulin in the peripheral tissues, inhibit glucose absorption from the intestine, suppress of hepatic gluconeogenesis, and inhibit fatty acid oxidation. Examples of biguanides include phenformin, metformin, buformin, and biguanides known in the art.

Suitable pharmaceutical agents that can be used in conjunction with compounds of the present invention include the α-glucosidase inhibitors. The α-glucosidase inhibitors competitively inhibit digestive enzymes such as α-amylase, maltase, α-dextrinase, sucrase, etc. in the pancreas and or small intestine. The reversible inhibition by α-glucosidase inhibitors retard, diminish or otherwise reduce blood glucose levels by delaying the digestion of starch and sugars. Examples of α-glucosidase inhibitors include acarbose, N-(1,3-dihydroxy-2-propyl)valiolamine (generic name; voglibose), miglitol, and α-glucosidase inhibitors known in the art.

Suitable pharmaceutical agents that can be used in conjunction with compounds of the present invention include the peroxisome proliferators-activated receptor-γ (i.e., PPAR-γ) agonists. The peroxisome proliferators-activated receptor-γ agonists represent a class of compounds that activates the nuclear receptor PPAR-γ and therefore regulate the transcription of insulin-responsive genes involved in the control of glucose production, transport and utilization.

Agents in the class also facilitate the regulation of fatty acid metabolism. Examples of PPAR-$\gamma$ agonists include rosiglitazone, pioglitazone, tesaglitazar, netoglitazone, GW-409544, GW-501516 and PPAR-$\gamma$ agonists known in the art.

Suitable pharmaceutical agents that can be used in conjunction with compounds of the present invention include the HMG-CoA reductase inhibitors. The HMG-CoA reductase inhibitors are agents also referred to as Statin compounds that belong to a class of drugs that lower blood cholesterol levels by inhibiting hydroxymethylglutalyl CoA (HMG-CoA) reductase. HMG-CoA reductase is the rate-limiting enzyme in cholesterol biosynthesis. The statins lower serum LDL concentrations by upregulating the activity of LDL receptors and are responsible for clearing LDL from the blood. Some representative examples the statin compounds include rosuvastatin, pravastatin and its sodium salt, simvastatin, lovastatin, atorvastatin, fluvastatin, cerivastatin, rosuvastatin, pitavastatin, BMS's "superstatin", and HMG-CoA reductase inhibitors known in the art.

Suitable pharmaceutical agents that can be used in conjunction with compounds of the present invention include the Fibrates. Fibrate compounds belong to a class of drugs that lower blood cholesterol levels by inhibiting synthesis and secretion of triglycerides in the liver and activating a lipoprotein lipase. Fibrates have been known to activate peroxisome proliferators-activated receptors and induce lipoprotein lipase expression. Examples of fibrate compounds include bezafibrate, beclobrate, binifibrate, ciplofibrate, clinofibrate, clofibrate, clofibric acid, etofibrate, fenofibrate, gemfibrozil, nicofibrate, pirifibrate, ronifibrate, simfibrate, theofibrate, and fibrates known in the art.

Suitable pharmaceutical agents that can be used in conjunction with compounds of the present invention include the angiotensin converting enzyme (ACE) inhibitors. The angiotensin converting enzyme inhibitors belong to the class of drugs that partially lower blood glucose levels as well as lowering blood pressure by inhibiting angiotensin converting enzymes. Examples of the angiotensin converting enzyme inhibitors include captopril, enalapril, alacepril, delapril; ramipril, lisinopril, imidapril, benazepril, ceronapril, cilazapril, enalaprilat, fosinopril, moveltopril, perindopril, quinapril, spirapril, temocapril, trandolapril, and angiotensin converting enzyme inhibitors known in the art.

Suitable pharmaceutical agents that can be used in conjunction with compounds of the present invention include the angiotensin II receptor antagonists. Angiotensin II receptor antagonists target the angiotensin II receptor subtype 1 (i.e., AT1) and demonstrate a beneficial effect on hypertension. Examples of angiotensin II receptor antagonists include losartan (and the potassium salt form), and angiotensin II receptor antagonists known in the art.

Suitable pharmaceutical agents that can be used in conjunction with compounds of the present invention include the squalene synthesis inhibitors. Squalene synthesis inhibitors belong to a class of drugs that lower blood cholesterol levels by inhibiting synthesis of squalene. Examples of the squalene synthesis inhibitors include (S)-$\alpha$-[Bis [2,2-dimethyl-1-oxopropoxy)methoxy] phosphinyl]-3-phenoxybenzenebutanesulfonic acid, mono potassium salt (BMS-188494) and squalene synthesis inhibitors known in the art.

Suitable pharmaceutical agents that can be used in conjunction with compounds of the present invention include, but not limited to, amylin agonists (for example, pramlintide), insulin secretagogues (for example, GLP-1 agonists; exendin-4; insulinotropin (NN2211); dipeptyl peptidase inhibitors (for example, NVP-DPP-728), acyl CoA cholesterol acetyltransferase inhibitors (for example, Ezetimibe, eflucimibe, and like compounds), cholesterol absorption inhibitors (for example, ezetimibe, pamaqueside and like compounds), cholesterol ester transfer protein inhibitors (for example, CP-529414, JTT-705, CETi-1, and like compounds), microsomal triglyceride transfer protein inhibitors (for example, implitapide, and like compounds), cholesterol modulators (for example, NO-1886, and like compounds), bile acid modulators (for example, GT103-279 and like compounds), insulin signalling pathway modulators, like inhibitors of protein tyrosine phosphatases (PTPases), non-small mol. mimetic compds, and inhibitors of glutamine-fructose-6-phosphate amidotransferase (GFAT), compds. influencing a dysregulated hepatic glucose prodn., like inhibitors of glucose-6-phosphatase (G6Pase), inhibitors of fructose-1,6-bisphosphatase (F-1,6-BPase), inhibitors of glycogen phosphorylase (GP), glucagon receptor antagonists and inhibitors of phosphoenolpyruvate carboxykinase (PEPCK), pyruvate dehydrogenase kinase (PDHK) inhibitors, insulin sensitivity enhancers, insulin secretion enhancers, inhibitors of gastric emptying, $\alpha_2$-adrenergic antagonists, and retinoid X receptor (RXR) agonists. The

The combination can be used by mixing the respective active components either all together or independently with a physiologically acceptable carrier, excipient, binder, diluent, etc., as described herein above, and administering the mixture or mixtures either orally or non-orally as a pharmaceutical composition. When a compound or a mixture of compounds of Formula (**I**) are administered as a combination therapy with another active compound the therapeutic agents can be formulated as a separate pharmaceutical compositions given at the same time or at different times, or the therapeutic agents can be given as a single composition.

## OTHER UTILITIES

Another object of the present invention relates to radio-labeled compounds of Formula (**I**) that would be useful not only in radio-imaging but also in assays, both in vitro and in vivo, for localizing and quantitating the **RUP3** receptor in tissue samples, including human, and for identifying **RUP3** receptor ligands by inhibition binding of a radio-labeled compound. It is a further object of this invention to develop novel **RUP3** receptor assays of which comprise such radio-labeled compounds.

The present invention embraces isotopically-labeled compounds of Formula (**I**) and any subgenera herein, such as but not limited to, Formula (**Ia**) through Formula (**IIIo**). An "isotopically" or "radio-labeled" compounds are those which are identical to compounds disclosed herein, but for the fact that one or more atoms are replaced or substituted by an atom having an atomic mass or mass number different from the atomic mass or mass number typically found in nature (i.e., naturally occurring). Suitable radionuclides that may be incorporated in compounds of the present invention include but are not limited to $^{2}H$ (also written as D for deuterium), $^{3}H$ (also written as T for tritium), $^{11}C$, $^{13}C$, $^{14}C$, $^{13}N$, $^{15}N$, $^{15}O$, $^{17}O$, $^{18}O$, $^{18}F$, $^{35}S$, $^{36}Cl$, $^{82}Br$, $^{75}Br$, $^{76}Br$, $^{77}Br$, $^{123}I$, $^{124}I$, $^{125}I$ and $^{131}I$. The radionuclide that is incorporated in the instant radio-labeled compounds will depend on the specific application of that radio-labeled compound. For example, for in vitro **RUP3** receptor labeling and competition assays, compounds that incorporate $^{3}H$, $^{14}C$, $^{82}Br$, $^{125}I$, $^{131}I$, $^{35}S$ or will generally be most useful. For radio-imaging applications $^{11}C$, $^{18}F$, $^{125}I$, $^{123}I$, $^{124}I$, $^{131}I$, $^{75}Br$, $^{76}Br$ or $^{77}Br$ will generally be most useful.

It is understood that a "radio-labeled " or "labeled compound" is a compound of Formula (**I**) that has incorporated at least one radionuclide; in some embodiments the radionuclide is selected from the group consisting of $^{3}H$, $^{14}C$, $^{125}I$, $^{35}S$ and $^{82}Br$.

Certain isotopically-labeled compounds of the present invention are useful in compound and/or substrate tissue distribution assays. In some embodiments the radionuclide $^{3}H$ and/or $^{14}C$ isotopes are useful in these studies. Further, substitution with heavier isotopes such as deuterium (i.e., $^{2}H$) may afford certain therapeutic advantages resulting from greater metabolic stability (e.g., increased in vivo half-life or reduced dosage requirements) and hence may be preferred in some circumstances. Isotopically labeled compounds of the present invention can generally be prepared by following procedures analogous to those disclosed in the Schemes supra and Examples infra, by substituting an isotopically labeled reagent for a non-isotopically labeled reagent. Other synthetic methods that are useful are discussed *infra.* Moreover, it should be understood that all of the atoms represented in the compounds of the invention can be either the most commonly occurring isotope of such atoms or the more scarce radio-isotope or nonradio-active isotope.

Synthetic methods for incorporating radio-isotopes into organic compounds are applicable to compounds of the invention and are well known in the art. These synthetic methods, for example, incorporating activity levels of tritium into target molecules, are as follows:

A. Catalytic Reduction with Tritium Gas - This procedure normally yields high specific activity products and requires halogenated or unsaturated precursors.
B. Reduction with Sodium Borohydride [$^{3}H$] - This procedure is rather inexpensive and requires precursors containing reducible functional groups such as aldehydes, ketones, lactones, esters, and the like.
C. Reduction with Lithium Aluminum Hydride [$^{3}H$] - This procedure offers products at almost theoretical specific activities. It also requires precursors containing reducible functional groups such as aldehydes, ketones, lactones, esters, and the like.
D. Tritium Gas Exposure Labeling - This procedure involves exposing precursors containing exchangeable protons to tritium gas in the presence of a suitable catalyst.
E. N-Methylation using Methyl Iodide [$^{3}H$] - This procedure is usually employed to prepare O-methyl or N-methyl ($^{3}H$) products by treating appropriate precursors with high specific activity methyl iodide ($^{3}H$). This method in general allows for higher specific activity, such as for example, about 70-90 Ci/mmol.

Synthetic methods for incorporating activity levels of $^{125}I$ into target molecules include:

A. Sandmeyer and like reactions - This procedure transforms an aryl or heteroaryl amine into a diazonium salt, such as a tetrafluoroborate salt, and subsequently to $^{125}I$ labeled compound using $Na^{125}I$. A represented procedure was reported by Zhu, D.-G. and co-workers in J. Org. Chem. 2002, 67, 943-948.
B. Ortho $^{125}$Iodination of phenols - This procedure allows for the incorporation of $^{125}I$ at the ortho position of a phenol as reported by Collier, T. L. and co-workers in J. Labeled Compd Radiopharm. 1999, 42, S264-S266.
C. Aryl and heteroaryl bromide exchange with $^{125}I$- This method is generally a two step process. The first step is the conversion of the aryl or heteroaryl bromide to the corresponding trialkyltin intermediate using for example, a Pd catalyzed reaction [i.e. $Pd(Ph_3P)4$] or through an aryl or heteroaryl lithium, in the presence of a tri-alkyltinhalide or hexaalkylditin [e.g., $(CH_3)_3SnSn(CH_3)_3$]. A represented procedure was reported by Bas, M.-D. and co-workers in J. Labeled Compd Radiopharm. 2001, 44, S280-S282.

A radio-labeled **RUP3** receptor compound of Formula (**I**) can be used in a screening assay to identify/evaluate compounds. In general terms, a newly synthesized or identified compound (i.e., test compound) can be evaluated for its ability to reduce binding of the "radio-labeled compound of Formula (**I**)" to the **RUP3** receptor. Accordingly, the ability of a test compound to compete with the "radio-labeled compound of Formula (**I**)" for the binding to the **RUP3** receptor

directly correlates to its binding affinity.

The labeled compounds of the present invention bind to the **RUP3** receptor. In one embodiment the labeled compound has an $IC_{50}$ less than about 500 μM, in another embodiment the labeled compound has an $IC_{50}$ less than about 100 μM, in yet another embodiment the labeled compound has an $IC_{50}$ less than about 10 μM, in yet another embodiment the labeled compound has an $IC_{50}$ less than about 1 μM, and in still yet another embodiment the labeled inhibitor has an $IC_{50}$ less than about 0.1 μM.

Other uses of the disclosed receptors and methods will become apparent to those in the art based upon, inter alia, a review of this disclosure.

As will be recognized, the steps of the methods of the present invention need not be performed any particular number of times or in any particular sequence. Additional objects, advantages, and novel features of this invention will become apparent to those skilled in the art upon examination of the following examples thereof, which are intended to be illustrative and not intended to be limiting.

## EXAMPLES

The examples are provided to further define the invention without, however, limiting the invention to the specifics of these examples.

### Example 1

### 96- well Cyclic AMP membrane assay for RUP3

Materials:

1) Adenlyl cyclase Activation Flashplate Assay kit from Perkin Elmer -- 96 wells (SMP004B) and $^{125}$I tracer (NEX130) which comes with the kit. Keep in refrigerator, in a box, and do not expose the Flashplates to light.
2) Phosphocreatine - Sigma P-7936
3) Creatine Phosphokinase --- Sigma C-3755
4) GTP - Sigma G-8877
5) ATP- Sigma A-2383
6) IBMX - Sigma I-7018
7) Hepes -1M solution in distilled water - Gibco #15630080
8) MgCl2 - Sigma M-1028- 1M Solution
9) NaCl - Sigma - S6546 - 5M Solution
10) Bradford Protein Assay Kit - Biorad # 5000001
11) Proclin 300- Sigma #4-8126

Binding Buffer - filter through 45- micron Nalgene filter and keep in refrigerator. All buffers and membranes should be kept cold (in ice bucket) while performing assay.

20 mM Hepes, pH7.4
1 mM MgC12
100 mM NaCl

2X Regeneration Buffer (make in binding buffer):

20 mM Phosphocreatine (1.02 gm/200 ml binding buffer)
20 units Creatine phosphokinase (4 mg/200 ml)
20 uM GTP (make up 10.46 mg/ml in binding buffer and add 200 ul /200 ml)
0.2 mM ATP (22.04 mg/200 ml)
100 mM IBMX (44.4 mg IBMX dissolved in 1 ml 100% DMSO first and then add the entire amount to 200 ml of buffer).

Regeneration buffer can be aliquotted into 40-45 ml portions (in 50 ml sterile tubes) and kept frozen for up to 2 months. Simply put the tube in a beaker with room temperature water to thaw out the regeneration buffer on the day of the assay.

**A. Assay procedure**

1) Pipet 50 ul regeneration buffer in all 96 wells using Matrix 1250 8-channel pipettor.

2) Pipet 5 ul DMSO in columns 1 and columns 11 and 12.

3) Pipet 50 ul cAMP standards in columns 11 and 12 in this format: 50 pmole/well for row A, 25 pmole/well for row B, 12.5 pmol/well for row C, 5 picomol/well for row D, 2.5 pmole/well for row E, 1.25 pmole/well for row F, 0.5 pmole/well for row G, and 0 pmole/well (buffer only) for row H.

4) Pipet 5 ul compounds from each well of a compound dilution plate, for IC50s, using the following dilution scheme:

Well H: 400 uM compound (final concentration of compound in reaction mix = 5/100 x 400 uM = 20 uM
Well G: 1:10 dilution of Well H (i.e. 5ul compound from well H + 45 ul 100% DMSO) (final concentration = 2 uM)
Well F: 1:10 dilution of well G (final concentration = 0.2 uM)
Well E: 1:10 dilution of well F (final concentration = 0.02 uM)
Well D:1:10 dilution of well E (final concentration = 0.002 uM)
Well C:1:10 dilution of well D (final concentration = 0.0002 uM
Well B:1:10 dilution of well C (final concentration = 0.00002 uM)
Well A:1:10 dilution of well B (final concentration = 0.000002 uM)

$IC_{50}$s or $EC_{50}$s are done in triplicate. One Flashplate can therefore be set up to handle 3 compounds. (i.e., columns 2, 3, and 4 are for compound #1, columns 5, 6, and 7 are for compound #2, and columns 8, 9, and 10 are for compound #3.)

5) Add 50 ul of **RUP3** membranes to all wells in Columns 2 to 10. (Prior to the start of the assay, the frozen membrane pellets for both **RUP3** and CMV (cells transfected with an expression plasmid containing no **RUP3** sequences), are suspended in binding buffer, usually 1 ml binding buffer for 1 plate of membranes. The membranes are kept in ice all the time, and a polytron (Brinkmann polytron, model # PT-3100) is used (setting 6-7, for 15-20 seconds) to obtain a homogeneous membrane suspension.) Protein concentration is determined by Bradford protein assay kit using instructions given in the kit, using the standard supplied with the kit as a reference. The protein concentration of the membranes is adjusted with binding buffer, so that 50 ul membranes = 15 ug protein (i.e. 0.3 mg/ml protein).

6) In column 1, Wells A, B, C, and D, add 50 ul **RUP3** membranes. To wells E, F, G, and H, add 50 ul CMV membranes, (CMV membranes being of the same protein concentration as the **RUP3** membranes).

7) Incubate 1 hour at room temperature with agitation on a rotating platform shaker. Cover with foil while shaking.

8) After 1 hour, add (to all 96 wells), 100 ul of the $^{125}I$ tracer in detection buffer supplied with the Flashplate kit plus proclin, made up in the following manner:
Pipet per 10 ml per Flashplate: 100 ml of detection buffer + 1 ml $^{125}I$ + 0.2 ml ofProclin (the proclin helps to stop the production of cAMP). Make a smaller quantity of detection buffer mix if you have fewer plates.

9) Shake the plates on a rotating platform shaker for 2 hours, covering the plates with lead sheeting.

10) Seal the plates with the plastic film sealers provided with the Flashplate kit.

11) Count the plates using a TRILUX 1450 Microbeta Counter. See the door of the counter to determine which counting protocol to use.

12) Data is analyzed on the Arena Database according to the **RUP3** non-fusion, $IC_{50}$ $EC_{50}$ for 96-well cAMP membrane assay, and the compound numbers and the concentrations of compounds must be entered by the user.

### B. Membrane Cyclase Criteria

1) Signal to Noise:

An acceptable signal-to-noise ratio for **RUP3** can vary from 4 to 6. The raw cpms are approximately 1800 to 2500 for **RUP3** and 3500-4500 for CMV. The cpm (or ultimately pmoles of cAMP/well) cannot be outside the standard curve, and should not approach well A of the standard curve (50 pmole/well) and well H (no cAMP). Generally, the pmoles of cAMP produced by **RUP3** receptor are around 11 to 13 pmole/well (for 15 ug/well protein), and for CMV are between 2 to 3 pmole/well (for 15 ug protein /well).

2) Standard curve:

The slope should be linear and the error bars for duplicates should be very small. The receptor and CMV controls cannot be off scale of the standard curve, as described above. If the receptor controls are off the high end of the standard curve,i.e. 50 pmole/well or higher, one must repeat the experiment using less protein. However, such a case has not been observed with transiently transfected **RUP3** membranes (10 ug DNA/15 cm plate, using 60 ul Lipofectamine, and preparing membranes after 24 hour of transfection.)

3) The $IC_{50}$ or $EC_{50}$ curve should be at 100% (+ or - 20%) of control **RUP3** membranes at the top, and should go down to 0 (or up to 20%) at the bottom. The standard error of the triplicate determinations should be + or - 10%.

### C. Stimulation of cAMP in HIT-T15 cells

HIT-T15 (ATCC CRL#1777) is an immortalized hamster insulin-producing cell line. These cells express **RUP3** and therefore can be used to assess the ability of **RUP3** ligands to stimulate or inhibit cAMP accumulation via its endogenously expressed receptor. In this assay, cells are grown to 80% confluence and then distributed into a 96-well Flashplate (50,000 cells/ well) for detection of cAMP via a "cAMP Flashplate Assay" (NEN, Cat # SMP004). Briefly, cells are placed into anti-cAMP antibody-coated wells that contain either vehicle, the test ligand(s) at a concentration of interest, or 1 uM forskolin. The latter is a direct activator of adenylyl cyclase and serves as a positive control for stimulation of cAMP in HIT-T15 cells. All conditions are tested in triplicate. After a 1 hour incubation to allow for stimulation of cAMP, a Detection Mix containing $^{125}$I-cAMP is added to each well and the plate is allowed to incubate for another 1 hour. The wells are then aspirated to remove unbound $^{125}$I-cAMP. Bound $^{125}$I-cAMP is detected using a Wallac Microbeta Counter. The amount of cAMP in each sample is determined by comparison to a standard curve, obtained by placing known concentrations of cAMP in some wells on the plate.

### D. Stimulation of insulin secretion in HIT-T15 cells

It is known that stimulation of cAMP in HIT-T15 cells causes an increase in insulin secretion when the glucose concentration in the culture media is changed from 3mM to 15 mM. Thus, **RUP3** ligands can also be tested for their ability to stimulate glucose-dependent insulin secretion (GSIS) in HIT-T15 cells. In this assay, 30,000 cells/well in a 12-well plate are incubated in culture media containing 3 mM glucose and no serum for 2 hours. The media is then changed; wells receive media containing either 3 mM or 15 mM glucose, and in both cases the media contains either vehicle (DMSO) or **RUP3** ligand at a concentration of interest. Some wells receive media containing 1 uM forskolin as a positive control. All conditions are tested in triplicate. Cells are incubated for 30 minutes, and the amount of insulin secreted into the media is determined by ELISA, using a kit from either Peninsula Laboratories (Cat # ELIS-7536) or Crystal Chem Inc. (Cat # 90060).

### E. Stimulation of insulin secretion in isolated rat islets

As with HIT-T15 cells, it is known that stimulation of cAMP in isolated rat islets causes an increase in insulin secretion when the glucose concentration in the culture media is changed from 60 mg/dl to 300 mg/dl. **RUP3** is an endogenously expressed GPCR in the insulin-producing cells of rat islets. Thus, **RUP3** ligands can also be tested for their ability to stimulate GSIS in rat islet cultures. This assay is performed as follows:

**A.** Select 75-150 islet equivalents (IEQ) for each assay condition using a dissecting microscope. Incubate overnight in low-glucose culture medium. (Optional.)

**B.** Divide the islets evenly into triplicate samples of 25-40 islet equivalents per sample. Transfer to 40 μm mesh sterile cell strainers in wells of a 6-well plate with 5 ml of low (60 mg/dl) glucose Krebs-Ringers Buffer (KRB) assay

medium.

**C.** Incubate 30 minutes (1 hour if overnight step skipped) at 37˚C and 5% $CO_2$. Save the supernatants if a positive control for the RIA is desired.

**D.** Move strainers with islets to new wells with 5ml/well low glucose KRB. This is the second pre-incubation and serves to remove residual or carryover insulin from the culture medium. Incubate 30 minutes.

**E.** Move strainers to next wells (Low 1) with 4 or 5 ml low glucose KRB. Incubate @ 37˚ C for 30 minutes. Collect supernatants into low-binding polypropylene tubes pre-labelled for identification and keep cold.

**F.** Move strainers to high glucose wells (300mg/dl, which is equivalent to 16.7mM). Incubate and collect supernatants as before. Rinse islets in their strainers in low-glucose to remove residual insulin. If the rinse if to be collected for analysis, use one rinse well for each condition (i.e. set of triplicates.)

**G.** Move strainers to final wells with low-glucose assay medium (Low 2). Incubate and - collect supernatants as before.

**H.** Keeping cold, centrifuge supernatants at 1800 rpm for 5 minutes @ 4-8˚C to remove small islets/islet pieces that escape the 40mm mesh. Remove all but lower 0.5 - 1 ml and distribute in duplicate to pre-labelled low-binding tubes. Freeze and store at <-20˚ C until insulin concentrations can be determined.

**I.** Insulin determinations are done as above, or by Linco Labs as a custom service, using their rat insulin RIA (Cat. # RI-13K).

**Example 2**

**A. RT-PCR analysis of RUP3 expression in human tissues (Figure 1A).**

RT-PCR was applied to determine the tissue distribution of **RUP3**. Oligonucleotides used for PCR had the following sequences:

ZC47: 5'-CATTGCCGGGCTGTGGTTAGTGTC-3' (forward primer), (SEQ ID NO:3);
ZC48: 5'-GGCATAGATGAGTGGGTTGAGCAG-3' (reverse primer), (SEQ ID NO:4);
and the human multiple tissue cDNA panels (MTC, Clontech) were used as templates (1 ng cDNA per PCR amplification). Twenty-two (22) human tissues were analyzed. PCR was performed using Platinum PCR SuperMix (Life Technologies, Inc.; manufacture instructions were followed) in a 50 μl reaction by the following sequences: step 1, 95˚C for 4 min; step 2, 95˚C for 1 min; step 3, 60˚C for 30 sec; step 4, 72˚C for 1 min; and step 5, 72˚C for 7 min. Steps 2 through 4 were repeated 35 times.

The resulting PCR reactions (15 5 μl) were loaded on a 1.5% agarose gel to analyze the RT-PCR products, and a specific 466 base-pair DNA fragment representing **RUP3** was specifically amplified from cDNA of pancreas origin. Low expression was also evident in subregions of brain.

**B. cDNA Dot-Blot analysis of RUP3 expression in human tissues (Figure 1B).**

Results from RT-PCR analysis were further confirmed in cDNA dot-blot analysis. In this assay, a dot-blot membrane containing cDNA from 50 human tissues (Clontech) was hybridized with a $^{32}$P-radiolabelled DNA probe having sequences derived from human **RUP3**. Hybridyzation signals were seen in pancreas and fetal liver, suggesting these tissues express **RUP3**. No significant expression was detected in other tissues analyzed.

**C. Analysis of RUP3 by RT-PCR with isolated human pancreatic islets of Langerhans (Figure 1C).**

Further analysis of **RUP3** by RT-PCR with isolated human pancreatic islets of Langerhans showed robust expression of **RUP3** in islet cells but not in control samples.

**D. Analysis of RUP3 expression with cDNAs of rat origin by RT-PCR (Figure 1D).**

**RUP3** expression was further analyzed with cDNAs of rat origin by RT-PCR technique. Tissue cDNAs used

for this assay were obtained from Clontech except those for hypothalamus and islets, which were prepared in house. Concentrations of each cDNA sample were normalized via a control RT-PCR analysis of the house-keeping gene GAPDH before assaying for **RUP3** expression. Oligonucleotides used for PCR had the following sequences:

rat **RUP3** (**"rRUP3"**) forward: 5'-CATGGGCCCTGCACCTTCTTTG-3' (SEQ ID NO:5);
**rRUP3** reverse: 5'-GCTCCGGATGGCTGATGATAGTGA-3' (SEQ ID NO:6).

PCR was performed using Platinum PCR SuperMix (Life Technologies, Inc.; manufacture instructions were followed) in a 50 μl reaction by the following sequences: step 1, 95°C for 4 min; step 2, 95°C for 1 min; step 3, 60°C for 30 sec; step 4, 72°C for 1 min; and step 5, 72°C for 7 min. Steps 2 through 4 were repeated 35 times.

The resulting PCR reactions (15 μl) were loaded on a 1.5% agarose gel to analyze the RT-PCR products, and a specific 547 base-pair DNA fragment representing rat **RUP3** was specifically amplified from cDNA of pancreas origin, revealing a similar expression profile with human. Of particular note, robust expression was seen in isolated islets and hypothalamus.

**Example 3**

**RUP3 protein expression is restricted to β cell lineage of pancreatic islets (Figure 2).**

**A. A polyclonal anti-RUP3 antibody was prepared in rabbits (Figure 2A).**

Rabbits were immunized with an antigenic peptide with sequence derived from rat **RUP3** ("**rRUP3**"). The peptide sequence was RGPERTRESAYHIVTISHPELDG (SEQ ID NO: 7) and shared 100% identity with mouse **RUP3** in the corresponding region. A cysteine residue was incorporated at the N-terminal end of this antigenic peptide to facilitate KLH crosslinking before injecting into rabbits. The resulting antisera ("anti-**rRUP3"**) and the corresponding preimmune sera ("pre-**rRUP3**") were tested for immune reactivity to mouse **RUP3** in immunobloting assays (lanes 1 though 4). In this assay, the GST-**RUP3** fusion protein was readily recognized by the anti-**rRUP3** antisera (lane 4), but not by the preimmune sera (lane 2). The immunoreactive signal could be efficiently eliminated when the immunobloting assay was performed in the presence of excess antigenic peptide (lane 6).

**B. RUP3 expression in insulin-producing β cells of pancreatic islets (Figure 2B).**

Rat pancreas was perfused with 4% paraformaldehyde (PFA) in PBS and embedded in OCT embedding medium. Ten micron sections were prepared, fixed on glass slides, and immunostained with either pre-**rRUP3** (Figure **2B**, panel a) or with anti-**rRUP3** antisera (Figure **2B**, panels c and e) followed by secondary staining with donkey anti-rabbit IgG conjugated to the fluorochrome Cy-3. Each section was also co-immunostained with a monoclonal anti-insulin antibody (Santa Cruz, Figure **2B**, panels b and d) in primary staining followed by a secondary staining with donkey anti-mouse IgG conjugated with FITC, or with a goat anti-glucagon antibody (Santa Cruz, Figure **2B**, panel and donkey anti-goat IgG coupled to FITC. Immunofluorescent signals were examined under a fluorescent microscope. **RUP3** was found expressed in insulin producing cells (panels c and d), but not in glucagons producing cells (panels e and f). These data demonstrated that **RUP3** is expressed in P cells but not in β cells of the rat pancreatic islets. Analogous results were obtained when mouse pancreatic sections were investigated for **RUP3** expression.

**Example 4**

**Functional Activities of RUP3 *In Vitro* (Figure 3).**

It was established that **RUP3** stimulates the production of cAMP by cotransfection of 293 cells with: (1) a CRE-Luciferase reporter, wherein the ability to stimulate the production of firefly luciferase depends on increased cAMP in cells, and (2) an expression plasmid encoding the human form of **RUP3** (Figure **3A**). Note that cells co-transfected with an expression plasmid containing no **RUP3** sequences ("CMV" in Figure **3A**) produce very little luciferase activity, whereas cells transfected with an expression plasmid encoding **RUP3** ("**RUP3**" in Figure **3A**) have at least a 10-fold increase in luciferase activity. This indicates that **RUP3** stimulates the production of cAMP when introduced into 293 cells. This property of **RUP3** is conserved across species, because hamster **RUP3** stimulates luciferase activity when introduced into 293 cells in a manner analogous to that described for human **RUP3** (Figure **3B**).

It is established that, when cAMP is increased in insulin-producing cells of the pancreas, these cells exhibit an enhanced ability to secrete insulin when glucose concentrations rise. To test whether **RUP3** might impart enhanced glucose-dependent insulin release, retrovirus containing human **RUP3** was used to generate Tu6 cells that express high

levels of **RUP3**. Tu6 cells produce insulin, but do not express appreciable levels of **RUP3** and do not normally exhibit an increase in insulin release when increased glucose is present in the culture media. As shown in Figure **3C**, Tu6 cells transduced with a control virus that contains no receptor are still able to produce insulin, but do not show an increase in insulin secretion when the concentration of glucose in the culture media is shifted from 1 mM to 16 mM. By contrast, Tu6 cells transduced with **RUP3**-containing retrovirus display significant glucose-dependent insulin secretion (Figure 3C).

### Example 5

### *In vivo* effects of RUP3 agonists on glucose homeostasis in mice.

### A. Oral Glucose tolerance test (oGTT)

Male C57bl/6J mice at approximately 8 weeks of age were fasted for 18 hours and randomly grouped (n=5) to receive a **RUP3** agonist (either Compound B3 or B124) at 1, 3 or 10 mg/Kg. Compounds were delivered orally via a gavage needle (p.o., volume 10 mL/Kg). At time 0, levels of blood glucose were assessed using a glucometer (Elite XL, Bayer), and mice were administered either vehicle (20% hydroxypropyl-beta-cyclodextrin) or test compound. Thirty minutes after administration of test compound, levels of blood glucose were again assessed, and mice were administered dextrose orally at a dose of 3 g/Kg. Blood glucose measurements were then taken 20 min, 40 min, 60 min and 120 min after this time. Table 6 shows the mean percentage inhibition of glucose excursion for each dose of test compound, averaged across the five animals in each treatment group. These results demonstrated that the **RUP3** agonists, Compounds B3, and B124, lowered blood glucose in a dose-dependent manner in mice after challenged with glucose.

**TABLE 6**

| Mean % Inhibition of Glucose Excursion | | | |
|---|---|---|---|
| | Dose | | |
| Compound | 1 mg/Kg | 3 mg/Kg | 10 mg/Kg |
| B3 | 14.83 | 22.03 | 39.31 |
| B124 | 0 | 5.49 | 21.94 |

### Example 6

### Generation of Tu6/ RUP3 Stable Lines

To produce Tu6 cells that express **RUP3** at high levels, a retrovirus bearing an expression cassette for **RUP3** was generated. Briefly, **RUP3** coding sequence was cloned into the retroviral vector pLNCX2 (Clontech, Cat # 6102-1). The amphotropic packaging cell line PT-67 (Clontech, K1060-D) was then transfected with either the parental vector pLNCX2 or pLNCX2/**RUP3** using Lipofectamine and stable lines were established using guidelines provided by the PT-67 vendor. Retrovirus-containing supernatant was obtained by collecting media from the resultant stables according to the manufacturer's directions. Tu6 cells, in a 10 cm dish, were then infected with retrovirus by incubating in a solution of 1 ml viral supernatant/ 9 ml culture media containing 40 ug/ml polybrene for 24 hours. The medium was then changed to culture media containing 300 ug/ml G418. G418-resistant clones were ultimately created by virtue of the neomycin-resistance gene cassette present in the pLNCX2 vector, thus indicating the successful integration of retrovirus into the Tu6 genome. The expression of **RUP3** in the Tu6/**RUP3** G418-resistant colonies was confirmed by Northern blot.

### Example 7

### Insulin secretion, Tu6 Stables

To measure insulin secretion from rodent insulin-producing cell lines, cells were first cultured overnight in serum-free, glucose-deficient media. The following morning, the cells were then placed in the same media supplemented with either 1 mM or 16 mM glucose. After an incubation of 4 hours, the media was collected and analyzed for insulin content using a Rat Insulin Enzyme-Immunoassay (EIA) System (Amersham Pharmacia Biotech, Cat. # RPN 2567). Typically, the assay was performed using multiple dilutions of sample media in order to ensure that the sample measurements fell within the boundaries of the standard curve (generated using known amounts of insulin), as recommended by the manufacturer.

**Example 8**

**Receptor Binding Assay**

In addition to the methods described herein, another means for evaluating a test compound is by determining binding affinities to the **RUP3** receptor. This type of assay generally requires a radiolabelled ligand to the **RUP3** receptor. Absent the use of known ligands for the **RUP3** receptor and radiolabels thereof, compounds of Formula (**I**) can be labelled with a radioisotope and used in an assay for evaluating the affinity of a test compound to the **RUP3** receptor.

A radiolabelled **RUP3** compound of Formula (**I**) can be used in a screening assay to identify/evaluate compounds. In general terms, a newly synthesized or identified compound (i.e., test compound) can be evaluated for its ability to reduce binding of the "radiolabelled compound of Formula (**I**)" to the **RUP3** receptor. Accordingly, the ability to compete with the "radio-labelled compound of Formula (**I**)" or **Radiolabelled RUP3 Ligand** for the binding to the **RUP3** receptor directly correlates to its binding affinity of the test compound to the **RUP3** receptor.

**ASSAY PROTOCOL FOR DETERMINING RECEPTOR BINDING FOR RUP3:**

**A. RUP3 RECEPTOR PREPARATION**

293 cells (human kidney, ATCC), transiently transfected with 10 ug human **RUP3** receptor and 60 ul Lipofectamine (per 15-cm dish), were grown in the dish for 24 hours (75% confluency) with a media change and removed with 10 ml/dish of Hepes-EDTA buffer (20mM Hepes + 10 mM EDTA, pH 7.4). The cells were then centrifuged in a Beckman Coulter centrifuge for 20 minutes, 17,000 rpm (JA-25.50 rotor). Subsequently, the pellet was resuspended in 20 mM Hepes + 1 mM EDTA, pH 7.4 and homogenized with a 50- ml Dounce homogenizer and again centrifuged. After removing the supernatant, the pellets were stored at -80˚C, until used in binding assay. When used in the assay, membranes were thawed on ice for 20 minutes and then 10 mL of incubation buffer (20 mM Hepes, 1 mM $MgCl_2$, 100 mM NaCl, pH 7.4) added. The membranes were then vortexed to resuspend the crude membrane pellet and homogenized with a Brinkmann PT-3100 Polytron homogenizer for 15 seconds at setting 6. The concentration of membrane protein was determined using the BRL Bradford protein assay.

**B. BINDING ASSAY**

For total binding, a total volume of 50ul of appropriately diluted membranes (diluted in assay buffer containing 50mM Tris HCl (pH 7.4), 10mM $MgCl_2$, and 1 mM EDTA; 5-50ug protein) is added to 96-well polyproylene microtiter plates followed by addition of 100ul of assay buffer and 50ul of **Radiolabelled RUP3 Ligand**. For nonspecific binding, 50 ul of assay buffer is added instead of 100ul and an additional 50ul of 10uM cold **RUP3** is added before 50ul of **Radiolabelled RUP3 Ligand** is added. Plates are then incubated at room temperature for 60-120 minutes. The binding reaction is terminated by filtering assay plates through a Microplate Devices GF/C Unifilter filtration plate with a Brandell 96-well plate harvestor followed by washing with cold 50 mM Tris HCl, pH 7.4 containing 0.9% NaCl. Then, the bottom of the filtration plate are sealed, 50ul of Optiphase Supermix is added to each well, the top of the plates are sealed, and plates are counted in a Trilux MicroBeta scintillation counter. For compound competition studies, instead of adding 100ul of assay buffer, 100ul of appropriately diluted test compound is added to appropriate wells followed by addition of 50 ul of **Radiolabelled RUP3 Ligand**.

**C. CALCULATIONS**

The test compounds are initially assayed at 1 and 0.1 $\mu$M and then at a range of concentrations chosen such that the middle dose would cause about 50% inhibition of a **Radio-RUP3 Ligand** binding (i.e., $IC_{50}$). Specific binding in the absence of test compound ($B_O$) is the difference of total binding ($B_T$) minus non-specific binding (NSB) and similarly specific binding (in the presence of test compound) (B) is the difference of displacement binding ($B_D$) minus non-specific binding (NSB). $IC_{50}$ is determined from an inhibition response curve, logit-log plot of % $B/B_O$ vs concentration of test compound.

$K_i$ is calculated by the Cheng and Prustoff transformation:

$$K_i = IC_{50} / (1 + [L]/K_D)$$

where [L] is the concentration of a **Radio-RUP3 Ligand** used in the assay and $K_D$ is the dissociation constant of a

**Radio-RUP3 Ligand** determined independently under the same binding conditions.

## CHEMISTRY EXAMPLES

## SYNTHESES OF COMPOUNDS OF THE PRESENT INVENTION

The compounds described herein and their synthesis are further illustrated by the following examples. The following examples are provided to further describe the invention without, however, limiting the invention to the particulas of these examples. The compounds described herein, *supra* and *infra,* are named according to the CS Chem Draw Ultra Version 7.0.1. In certain instances common names are used and it is understood that these common names would be recognized by those skilled in the art.

**Chemistry:** Proton nuclear magnetic resonance ($^1$H NMR) spectra were recorded on a Varian Mercury Vx-400 equipped with a 4 nucleus auto switchable probe and z-gradient or a Bruker Avance-400 equipped with a QNP (Quad Nucleus Probe) or a BBI (Broad Band Inverse) and z-gradient. Chemical shifts are given in parts per million (ppm) with the residual solvent signal used as reference. NMR abbreviations are used as follows: s = singlet, d = doublet, t = triplet, q = quartet, m = multiplet, br = broad. Microwave irradiations were carried out using the Emyrs Synthesizer (Personal Chemistry). Thin-layer chromatography (TLC) was performed on silica gel 60 $F_{254}$ (Merck), preparatory thin-layer chromatography (prep TLC) was preformed on PK6F silica gel 60 A 1 mm plates (Whatman), and column chromatography was carried out on a silica gel column using Kieselgel 60, 0.063-0.200 mm (Merck). Evaporation was done in vacuo on a Buchi rotary evaporator. Celite 545 ® was used during palladium filtrations.

LCMS specs: 1) PC: HPLC-pumps: LC-10AD *VP*, Shimadzu Inc.; HPLC system controller: SCL-10A *VP,* Shimadzu Inc; UV-Detector: SPD-10A *VP,* Shimadzu Inc; Autosampler: CTC HTS, PAL, Leap Scientific; Mass spectrometer: API 150EX with Turbo Ion Spray source, AB/MDS Sciex; Software: Analyst 1.2. 2) Mac: HPLC-pumps: LC-8A *VP,* Shimadzu Inc; HPLC system controller: SCL-10A *VP,* Shimadzu Inc.
UV-Detector: SPD-10A *VP*, Shimadzu Inc; Autosampler: 215 Liquid Handler, Gilson Inc; Mass spectrometer: API 150EX with Turbo Ion Spray source, AB/MDS Sciex
Software: Masschrom 1.5.2.

### Example 9:

**Example 9.1: Preparation of 4-({[6-(2-Fluoro-4-methanesulfonyl-phenylamino)-pyrimidin-4-yl]-methyl-amino}-methyl)-piperidine-1-carboxylic acid tert-butyl ester, also referred to herein as Compound A4.**

**Step 1: Preparation of 4-{[(6-chloro-pyrimidin-4-yl)-methyl-amino]-methyl}-piperidine-1-carboxylic acid tert-butyl ester.**

A mixture of 4, 6-dichloropyrimidine (194 mg, 1.31 mmol), 4-methylaminomethyl-piperidine-1-carboxylic acid tert-butyl ester (300 mg, 1.31 mmol) and diisopropylethyl amine (0.45 mL, 2.62 mmol) in isopropyl alcohol (2 mL) was heated under microwave irradiation for 5 min at 100˚C. The crude mixture was concentrated in vacuo and purified by HPLC to provide 4-{[(6-chloro-pyrimidin-4-yl)-methyl-amino]-methyl}-piperidine-1-carboxylic acid tert-butyl ester as an oil (240 mg, 54%). $^1$HNMR ($CDCl_3$, 400 MHz) δ 0.90-0.92 (m, 2H), 1.13-1.25 (m, 2H), 1.45 (s, 9H), 1.58-1.61 (m, 2H), 1.86-2.04 (m, 1H), 2.64-2.70 (m, 2H), 3.05 (s, 3H), 4.11-4.12 (m, 2H), 6.38 (s, 1H), 8.35 (s, 1H). Exact mass calculated for $C_{16}H_{25}ClN_4O_2$ 340.2, found 341.0 ($MH^+$).

**Step 2: Preparation of 4-({[6-(2-Fluoro-4-methanesulfonyl-phenylamino)-pyrimidin-4-yl]-methyl-amino}-methyl)-piperidine-1-carboxylic acid tert-butyl ester (Compound A4).**

A mixture of 4-{[(6-chloro-pyrimidin-4-yl)-methyl-amino]-methyl}-piperidine-1-carboxylic acid tert-butyl ester (240 mg, 0.71 mmol), 2-fluoro-4-methanesulfonylaniline (113 mg, 0.60 mmol), palladium acetate (1.4 mg, 0.006 mmol), di-t-butyl-biphenylphosphine (2 mg, 0.0066 mmol) and sodium t-butoxide (144 mg, 1.5 mmol) in dioxane (2 ml) was heated under microwave irradiation at 120˚C for 2 hours. The crude mixture was purified by HPLC to provide compound A4 as a solid. $^1$HNMR ($CDCl_3$, 400 MHz) d 1.18-1.21 (m, 2H), 1.45 (s, 9H), 1.55-1.58 (m, 2H), 1.81-1.88 (m, 1H), 2.68 (t, 2H), 3.00 (s, 3H), 3.10 (s, 3H), 3.60-3.66 (m, 2H), 4.10-4.13 (m, 2H), 5.36 (d, 1H), 7.61-7.63 (m, 1H), 7.80 (d, 2H), 8.22 (s, 1H). Exact mass calculated for $C_{23}H_{32}FN_5O_4S$ 493.2, found 494.5 ($MH^+$).

Using essentially the same methodology and procedures as described in the Reaction Scheme(s) and Examples herein, the following compounds were prepared from the appropriate materials.

**Example 9.2: (2-fluoro-4-methanesulfonyl-phenyl)-{6-[4-(3-fluoro-phenoxy)-piperidin-1-yl]-pyrimidin-4-yl}-amine (Compound A8).**

Compound **A8** was obtained as tan solid (48mg, 52%). $^1H$ NMR 400MHz $CDCl_3$ δ (ppm): 8.25(s, 1H); 7.80(d, 2H); 7.60(t, 1H); 6.71-6.69(m,3H); 6.63(d,1H); 5.52(s,11H); 4.64(m,1H); 3.95(m, 2H); 3.83(m, 2H); 3.10(s, 3H); 2.04-1.98 (m, 4H). Exact mass calculated for $C_{22}H_{22}$ $F_2N_4O_3$ S 460.1, LCMS (ESI) m/z 461.1 ($M+H^+$, 100%).

**Example 9.3: 4-({[6-(4-Cyano-2-fluoro-phenylamino)-pyrimidin-4-yl]-methyl-amino}-methyl)-piperidine-1-carboaylic acid tert-butyl ester (Compound A14).**

Compound **A14** was obtained as white solid(13mg, 20%). $^1H$ NMR 400MHz $CDCl_3$ δ (ppm): 8.19(s, 1H); 7.53-7.50(m,3H); 5.35(s, 1H); 4.13 (m,2H); 3.66(m,2H); 2.99(s, 3H); 2.71-2.65 (m, 2H); 1.88(sb, 1H); 1-57-1.55(m, 2H); 1.46(s, 9H); 1.24-1.18(m,2H). Exact mass calculated for $C_{23}H_{29}$ $FN_6O_2$ 440.2, found LCMS (ESI) m/z 441.2($M+H^+$, 100%).

**Example 9.4: 4-[({6-[4-(2-Methanesulfonyl-ethyl)-phenylamino]-pyrimidin-4-yl}-methyl-amino)-methyl]-piperidine-1-carboxylic acid tert-butyl ester (Compound A15).**

Compound **A15** was obtained as yellow solid (5mg, 7%). $^1H$ NMR 400MHz $CDCl_3$ δ (ppm): 8.11(s, 1H); 7.30-7.21 (m,4H); 5.62(s, 1H); 4.12 (m,2H); 3.62(m,2H); 3.31-3.28 (m, 2H); 3.20-3.16 (m,2H); 2.95(s, 3H); 2.90 (s,3H); 2.70-2.64 (m, 2H); 1.87(sb, 1H); 1.56-1.54(m, 2H); 1.47(s, 9H); 1.20-1.17(m,2H). Exact mass calculated for $C_{25}H_{37}$ $N_5O_4$ S 503.3, LCMS (ESI) m/z 504.3($M+H^+$, 100%).

**Example 9.5: 4-({[6-(4-Ethylsulfanyl-phenylamino)-pyrimidin-4-yl]-methyl-amino}-methyl)-piperidine-1-carboxylic acid tert-butyl ester (Compound A16).**

Compound **A16** was obtained as yellow solid (9mg, 13%). Exact mass calculated for $C_{24}H_{35}$ $N_5O_2S$ 457.2, LCMS (ESI) m/z 458.3($M+H^+$, 100%).

**Example 9.6: 4-({[6-(4-Isopropylsulfanyl-phenylamino)-pyrimidin-4-yl]-methyl-amino}-methyl)-piperidine-1-carboxylic acid tert-butyl ester (Compound A17).**

Compound **A17** was obtained as yellow solid (18mg, 25%). Exact mass calculated for $C_{25}H_{37}$ $N_5O_2S$ 471.3, LCMS (ESI) m/z 472.4($M+H^+$, 100%).

**Example 9.7: 4-({[6-(4-Ethylsulfamoyl-phenylamino)-pyrimidin-4-yl]-methyl-amino}-methyl)-piperidine-1-carboxylic acid tert-butyl ester (Compound A18)**

Compound **A18** was obtained as white solid (4mg, 5%). $^1H$ NMR 400MHz $CDCl_3$ δ (ppm): 8.17(s, 1H); 7.91 (d,2H; 7.46 (d,2H); 5.80(s, 1H); 4.13 (sb,2H); 3.65(sb,2H); 3.07-3.01 (m, 2H); 2.99(s,3H); 2.69-2.64 (m,2H); 1.88(sb, 1H); 1.58-1.55(m, 2H); 1.45(s, 9H); 1.30-1.21(m,2H); 1.14(t,3H). Exact mass calculated for $C_{24}H_{36}N_6O_4$ S 504.2, LCMS (ESI) m/z 505.4($M+H^+$, 100%).

**Example 9.8: 4-({Methyl-[6-(4-methylsulfamoyl-phenylamino)-pyrimidin-4-yl]-amino}-methyl)-piperidine-1-carboxylic acid tert-butyl ester (Compound A19).**

Compound **A19** was obtained as white solid (3mg, 4%). $^1H$ NMR 400MHz $CDCl_3$ δ (ppm): 8.17(s, 1H); 7.90 (d,2H); 7.47 (d,2H); 5.82(s, 1H); 4.12 (sb,2H); 3.65(sb,2H); 3.16 (s, 3H); 2.99(s,3H); 2.68-2.62 (m,2H); 1.89(sb, 1H); 1.62-1.55(m, 2H); 1.45(s, 9H); 1.30-1.18(m,2H).Exact mass calculated for $C_{23}H_{34}N_6O_4$ S 490.2, LCMS (ESI) m/z 491.4 ($M+H^+$, 100%).

**Example 9.9: 4-({[6-(4-Dimethylsulfamoyl-phenylamino)-pyrimidin-4-yl]-methyl-amino}-methyl)-piperidine-1-carboxylic acid tert-butyl ester (Compound A20).**

Compound **A20** was obtained as white solid (25mg, 33%). $^1H$ NMR 400MHz $CDCl_3$ δ-(ppm): 8.19(s, 1H); 7.84 (d,2H); 7.49 (d,2H); 5.78(s, 1H); 4.14 (sb,2H); 3.67(sb,2H); 3.02 (s, 3H); 2.75(s,6H); 2.73-2.66 (m,2H); 1.89(sb, 1H); 1.59-1.56(m, 2H); 1.43(s, 9H); 1.22-1.18(m,2H). Exact mass calculated for $C_{24}H_{36}N_6O_4$ S 504.2, LCMS (ESI) m/z 505.4 ($M+H^+$, 100%).

**Example 9.10: 4-({Methyl-[6-(4-methylsulfamoylmethyl-phenylamino)-pyrimidin-4-yl]-amino}-methyl)-piperidine-1-carboxylic acid tert-butyl ester (Compound A21).**

Compound **A21** was obtained as yellow solid (7mg, 9%). Exact mass calculated for $C_{24}H_{36}N_6O_4$ S 504.2, LCMS (ESI) m/z 505.3(M+H$^+$, 100%).

**Example 9.11: 4-({Methyl-[6-(4-sulfamoyl-phenylamino)-pyrimidin-4-yl]-amino}-methyl)-piperidine-1-carboxylic acid tert-butyl ester (Compound A22).**

Compound **A22** was obtained as white solid (4mg, 6%). Exact mass calculated for $C_{22}H_{32}N_6O_4$ S 476.2, LCMS (ESI) m/z 477.2(M+H$^+$, 100%).

**Example 9.12: 4-({Methyl-[6-(4-[1,2,4]triazol-1-yl-phenylamino)-pyrimidin-4-yl]-amino}-methyl)-piperidine-1-carboxylic acid tert-butyl ester (Compound A23).**

Compound **A23** was obtained as white solid (12mg, 17%). Exact mass calculated for $C_{24}H_{32}N_8O_2$ 464.3, LCMS (ESI) m/z 465.4(M+H$^+$, 100%).

**Example 9.13: 4-({Methyl-[6-(4-[1,2,4]triazol-1-ylmethyl-phenylamino)-pyrimidin-4-yl]-amino}-methyl)-piperidine-1-carboaylic acid tert-butyl ester (Compound A24).**

Compound **A24** was obtained as yellow solid (3mg, 4 %). Exact mass calculated for $C_{25}H_{34}N_8O_2$ 475.3, LCMS (ESI) m/z 479.1(M+H$^+$, 100%).

**Example 9.14: 4-[(Methyl-{6-[4-(2-[1,2,4]triazol-1-yl-ethyl)-phenylamino]-pyrimidin-4-yl}-amino)-methyl]-piperidine-1-carboxylic acid tert-butyl ester (Compound A25).**

Compound **A25** was obtained as white solid (6mg, 8 %). Exact mass calculated for $C_{26}H_{36}N_8O_2$ 492.3, LCMS (ESI) m/z 493.4(M+H$^+$, 100%).

**Example 9.16: 4-({[6-(Benzo[1,3]dioxol-5-ylamino)-pyrimidin-4-yl]-methyl-amino}-methyl)-piperidine-1-carboxylic acid tert-butyl ester (Compound A26).**

Compound **A26** was obtained as tan solid (11mg, 17 %). Exact mass calculated for $C_{23}H_{31}N_5O_4$ 441.2, found LCMS (ESI) m/z 442.3(M+H$^+$, 100%).

**Example 9.17: 4-({[6-(6-Methanesulfonyl-pyridin-3-ylamino)-pyrimidin-4-yl]-methyl-amino}-methyl)-piperidine-1-carboxylic acid tert-butyl ester (Compound A27).**

Compound **A27** was obtained as off-white solid (3mg, 4 %). Exact mass calculated for $C_{22}H_{32}N_6O_4$ S 476.2, found LCMS (ESI) m/z 477.4(M+H$^+$, 100%).

**Example 9.18: 4-({[6-(3,5-Dimethoxy-phenylamino)-pyrimidin-4-yl]-methyl-amino}-methyl)-piperidine-1-carboxylic acid tert-butyl ester (Compound A28).**

Compound **A28** was obtained as yellow solid (13mg, 19 %). Exact mass calculated for $C_{24}H_{35}N_5O_4$ 457.3, found LCMS (ESI) m/z 458.3(M+H$^+$, 100%).

**Example 9.19: 4-[(Methyl-{6-[4-(2-oxo-oxazolidin-4-ylmethyl)-phenylamino]-pyrimidin-4-yl}-amino)-methyl]-piperidine-1-carboxylic acid tert-butyl ester (Compound A29).**

Compound **A29** was obtained as yellow solid (30mg, 40 %). Exact mass calculated for $C_{26}H_{36}N_6O_4$ 496.3, found LCMS (ESI) m/z 497.5(M+H$^+$, 100%).

**Example 9.20: 4-[({6-[4-(1,1-Dioxo-1λ6-thiomorpholin-4-ylmethyl)-phenylamino]-pyrimidin-4-yl}-methyl-amino)-methyl]-piperidine-1-carboxylic acid tert-butyl ester (Compound A30).**

Compound **A30** was obtained as white solid (12mg, 15 %). Exact mass calculated for $C_{27}H_{40}N_6O_4$ S 544.3, found LCMS (ESI) m/z 545.4(M+H$^+$, 100%).

**Example 9,21: 4-({Methyl-[6-(4-pyrazol-1-yl-phenylamino)-pyrimidin-4-yl]-amino}-methyl)-piperidine-1-carboxylic acid tert-butyl ester (Compound A31).**

Compound **A31** was obtained as white solid (6mg, 9 %). Exact mass calculated for $C_{25}H_{33}N_7O_2$ 463.3, found LCMS (ESI) m/z 464.3($M+H^+$, 100%).

**Example 9.22: 4-({[6-(2,2-Difluoro-benzo[1,3]dioxol-5-ylamino)-pyrimidin-4-yl]-methyl-amino}-methyl)-piperidine-1-carboxylic acid tert-butyl ester (Compound A32).**

Compound **A32** was obtained as white solid (3mg, 4 %). Exact mass calculated for $C_{23}H_{29}$ $F_2N_5O_4$ 477.2, found LCMS (ESI) m/z 478.3($M+H^+$, 100%).

**Example9.23:4-({Methyl-[6-(4-trifluoromethanesulfonyl-phenylamino)-pyrimidin-4-yl]-amino}-methyl)-piperidine-1-carboxylic acid tert-butyl ester (Compound A33).**

Compound **A33** was obtained as white solid (13mg, 16 %). Exact mass calculated for $C_{23}H_{30}$ $F_3N_5O_4S$ 529.2, found LCMS (ESI) m/z 530.2($M+H^+$, 100%).

**Example 9.24: 4-[(Methyl-{6-[4-(morpholine-4-sulfonyl)-phenylamino]-pyrimidin-4-yl}-amino)-methyl]-piperidine-1-carboxylic acid tert-butyl ester (Compound A34).**

Compound **A34** was obtained as white solid (13mg, 16 %). Exact mass calculated for $C_{26}H_{38}$ $N_6O_5S$ 546:3, found LCMS (ESI) m/z 547:3($M+H^+$, 100%).

**Example 9.25: 4-[(Methyl-{6-[2-(pyridine-2-carbonyl)-phenylamino]-pyrimidin-4-yl}-amino)-methyl]-piperidine-1-carboxylic acid tert-butyl ester (Compound A35).**

Compound **A35** was obtained as brown solid (0.4mg, 0.5 %). Exact mass calculated for $C_{28}H_{34}N_6O_3$ 502.3, found LCMS (ESI) m/z 503.5($M+H^+$, 100%).

**Example9.26:4-({[6-(2-Fluoro-5-methanesulfonyl-phenylamino)-pyrimidin-4-yl]-methyl-amino}-methyl)-piperidine-1-carboxylic acid tert-butyl ester (Compound A36).**

Compound **A36** was obtained as a solid (11 mg, 15 %). Exact mass calculated for $C_{25}H_{32}FN_5O_4S$ 493.2, found 494.3 ($M+H^+$),

**Example 9.27: 4-({[6-(3,4-Difluoro-phenylamino)-pyrimidin-4-yl]-methyl-amino}-methyl)-piperidine-1-carboxylic acid tert-butyl ester (Compound A39).**

Compound **A39** was obtained as a solid (6 mg, 9%). Exact mass calculated for $C_{22}H_{29}F_2N_5O_2$ 433.2, found 434.3 ($M+H^+$).

**Example 9.28: 4-({[6-(2,6-Difluoro-phenylamino)-pyrimidin-4-yl]-methyl-amino}-methyl)-piperidine-1-carboxylic acid tert-butyl ester (Compound A40).**

Compound **A40** was obtained as a solid (28 mg, 43%). Exact mass calculated for $C_{22}H_{29}F_2N_5O_2$ 433.2, found 434.3 ($M+H^+$).

**Example 9.29: 4-({[6-(2,5-Difluoro-phenylamino)-pyrimidin-4-yl]-methyl-amino}-methyl)-piperidine-1-carboxylic acid tert-butyl ester (Compound A41).**

Compound **A41** was obtained as a solid (22 mg, 34%). Exact mass calculated for $C_{22}H_{29}F_2N_5O_2$ 433.2, found 434.0 ($M+H^+$).

**Example 9.30: 4-({[6-(2,3-Difluoro-phenylamino)-pyrimidin-4-yl]-methyl-amino}-methyl)-piperidine-1-carboxylic acid tert-butyl ester (Compound A42).**

Compound **A42** was obtained as a solid (10 mg, 15%). Exact mass calculated for $C_{22}H_{29}F_2N_5O_2$ 433.2, found

434.2(M+H+).

**Example 9.31: 4-({Methyl-[6-(2,3,5-trifluoro-phenylamino)-pyrimidin-4-yl]-amino}-methyl)-piperidine-1-carboxylic acid tert-butyl ester (Compound A43).**

Compound **A43** was obtained as a solid (4 mg, 6%). Exact mass calculated for $C_{22}H_{28}F3N_5O_2$ 451.2, found 452.2 (M+H+).

**Example 9.32: 4-({[6-(-2-Fluoro-phenylamino)-pyrimidin-4-yl]-methyl-amino}-methyl)-piperidine-1-carboxylic acid tert-butyl ester (Compound A44).**

Compound **A44** was obtained as a solid (11 mg, 18%). Exact mass calculated for $C_{22}H_{30}FN_5O_2$ 415.2, found 416.3 (M+H+).

**Example 9.33: 4-({[6-(2-Fluoro-4-methyl-phenylamino)-pyrimidin-4-yl]-methyl-amino}-methyl)-piperidine-1-carboxylic acid tert-butyl ester (Compound A45).**

Compound **A45** was obtained as a solid (7 mg, 11%). Exact mass calculated for $C_{23}H_{32}FN_5O_2$ 429.2, found 430.1 (M+H+).

**Example 9.34: 4-({[6-(3-Chloro-2-fluoro-phenylamino)-pyrimidin-4-yl]-methyl-amino}-methyl)-piperidine-1-carboxylic acid tert-butyl ester (Compound A46).**

Compound **A46** was obtained as a solid (19 mg, 28%). Exact mass calculated for $C_{22}H_{29}FClN_5O_2$ 449.2, found 450.4 (M+H+).

**Example 9.35: 4-({[6-(2,4-Difluoro-phenylamino)-pyrimidin-4-yl]-methyl-amino}-methyl)-piperidine-1-carboxylic acid tert-butyl ester (Compound A47).**

Compound **A47** was obtained as a solid (26 mg, 40%). Exact mass calculated for $C_{22}H_{29}F_2N_5O_2$ 433.2, found 434.4 (M+H+).

**Example 9.36: 4-[(Methyl-{6-[2-(1-oxy-pyridin-3-yl)-ethylamino]-pyrimidin-4-yl}-amino)-methyl]-piperidine-1-carboxylic acid tert-butyl ester (Compound A48).**

Compound **A48** was obtained as a solid (6 mg, 9%). Exact mass calculated for $C_{23}H_{34}N_6O_3$ 442.2, found 443.3 (M+H+).

**Example 9.37: 4-({[6-(4-Cyano-2-fluoro-phenylamino)-pyrimidin-4-yl]-methyl-amino}-methyl)-piperidine-1-carboxylic acid isobutyl ester (Compound A51).**

Compound **A51** was obtained as a trifluoroacetic acid salt (37 mg, 9%). $^1$HNMR ($CDCl_3$, 400 MHz) δ 0.94 (d, 6H), 1.21-1.25 (m, 2H), 1.57-1.60 (m, 2H), 1.89-1.96 (m, 2H), 2.72-2.74 (m, 2H), 3.00 (s, 3H), 3.55-3.60 (m, 2H), 3.86 (d, 2H), 4.10-4.18 (m, 2H), 5.37 (d, 1H), 7.51-7.53 (m, 3H), 8.20 (s, 1H), 11.8 (s, 1H). Exact mass calculated for $C_{23}H_{29}FN_6O_2$ 440.2, found 441.3 (M+H+).

**Example 9.38: 4-({[6-(4-Ethylsulfamoyl-2-fluoro-phenylamino)-pyrimidin-4-yl]-methyl-amino}-methyl)-piperidine-1-carboxylic acid tert-butyl ester (Compound A58).**

Compound **A58** was obtained as white solid (6mg, 8 %). Exact mass calculated for $C_{24}H_{35}$ F $N_6O_4S$ 522.2, found LCMS (ESI) m/z 523.4(M+H+, 100%).

**Example 9.39: 4-({[6-(2-Fluoro-4-isopropylsulfamoyl-phenylamino)-pyrimidin-4-yl]-methyl-amino}-methyl)-piperidine-1-carboxylic acid tert-butyl ester (Compound A59).**

Compound **A59** was obtained as white solid (7mg, 9 %). Exact mass calculated for $C_{25}H_{37}$ F $N_6O_4S$ 536.3, found LCMS (ESI) m/z 537.4(M+H+, 100%).

**Example 9.40: 4-({[6-(4-Cyano-2,5-difluoro-phenylamino)-pyrimidin-4-yl]-methyl-amino}-methyl)-piperidine-1-carboxylic acid tert-butyl ester (Compound A60).**

Compound **A60** was obtained as white solid (4mg, 6 %). Exact mass calculated for $C_{23}H_{28}\ F_2\ N_6O_2$ 458.2, found LCMS (ESI) m/z 459.3(M+H$^+$, 100%).

**Example 9.41: 4-({[6-(4-Bromo-2,5-difluoro-phenylamino)-pyrimidin-4-yl]-methyl-amino}-methyl)-piperidine-1-carboxylic acid tert-butyl ester (Compound A61).**

Compound **A61** was obtained as yellow solid (25mg, 32 %). Exact mass calculated for $C_{22}H_{28}BrF_2N_5O_2$ 511.1, found LCMS (ESI) m/z 512.2(M+H$^+$, 100%).

**Example 9.42: 4-({[6-(5-Carboacy-2-fluoro-phenylamino)-pyrimidin-4-yl]-methyl-amino}-methyl)-piperidine-1-carboxylic acid tert-butyl ester (Compound A62).**

Compound **A62** was obtained as brown solid (2mg, 3 %). Exact mass calculated for $C_{23}H_{30}FN_5O_4$ 459.2, found LCMS (ESI) m/z 460.3(M+H$^+$, 100%).

**Example 9.43: 4-({[6-(6-Methoxy-pyridin-3-ylamino)-pyrimidin-4-yl]-methyl-amino}-methyl)-piperidine-1-carboxylic acid tert-butyl ester (Compound A63).**

Compound **A63** was obtained as brown solid (11mg, 17 %). Exact mass calculated for $C_{22}H_{32}N_6O_3$ 428.3, found LCMS (ESI) m/z 429.2(M+H$^+$, 100%).

**Example 9.44: 4-({[6-(2,6-Dimethoxy-pyridin-3-ylamino)-pyrimidin-4-yl]-methyl-amino}-methyl)-piperidine-1-carboxylic acid tert-butyl ester (Compound A64).**

Compound **A64** was obtained as brown solid (8mg, 12 %). Exact mass calculated for $C_{23}H_{34}N_6O_4$ 458.3, found LCMS (ESI) m/z 459.3(M+H$^+$, 100%).

**Example 9.45: 6-{6-[(1-tert-Butoxycarbonyl-piperidin-4-ylmethyl)-methyl-amino]-pyrimidin-4-ylamino}-nicotinic acid (Compound A65).**

Compound **A65** was obtained as tan solid (2mg, 3 %). Exact mass calculated for $C_{22}H_{30}N_6O_4$ 442.2, found LCMS (ESI) m/z 443.4(M+H$^+$, 100%).

**Example 9.46: 4-({[6-(6-Acetylamino-pyridin-3-ylamino)-pyrimidin-4-yl]-methyl-amino}-methyl)-piperidine-1-carboxylic acid tert-butyl ester (Compound A66).**

Compound **A66** was obtained as yellow solid (3mg, 4 %). Exact mass calculated for $C_{23}H_{33}N_7O_3$ 455.3, found LCMS (ESI) m/z 456.2(M+H$^+$, 100%).

**Example 9.47: 4-({[6-(5-Fluoro-pyridin-2-ylamino)-pyrimidin-4-yl]-methyl-amino}-methyl)-piperidine-1-carboaylic acid tert-butyl ester (Compound A67).**

Compound **A67** was obtained as yellow solid (19mg, 31%). Exact mass calculated for $C_{21}H_{29}\ FN_6O_2$ 416.2, found LCMS (ESI) m/z 417.3(M+H$^+$, 100%).

**Example 9.48: 4-({[6-(4-Cyano-2-ethyl-phenylamino)-pyrimidin-4-yl]-methyl-amino}-methyl)-piperidine-1-carboxylic acid tert-butyl ester (Compound A68).**

Compound **A68** was obtained as white solid (8mg, 12%). Exact mass calculated for $C_{25}H_{34}\ N_6O_2$ 450.3, found LCMS (ESI) m/z 451.3(M+H$^+$, 100%).

**Example 9.49: 4-({[6-(4-Butyryl-phenylamino)-pyrimidin-4-yl]-methyl-amino}-methyl)-piperidine-1-carboxylic acid tert-butyl ester (Compound A69).**

Compound **A69** was obtained as yellow solid (9mg, 13%). Exact mass calculated for $C_{26}H_{37}\ N_5O_3$ 467.3, found

LCMS (ESI) m/z 468.5(M+H$^+$, 100%).

**Example 9.50: 4-({[6-(5-Bromo-3-methyl-pyridin-2-ylamino)-pyrimidin-4-yl]-methyl-amino}-methyl)-piperidine-1-carboxylic acid tert-butyl ester (Compound A70).**

Compound **A70** was obtained as yellow solid (4mg, 5%). Exact mass calculated for $C_{22}H_{31}$ $BrN_6O_2$ 490.2, found LCMS (ESI) m/z 491.3(M+H$^+$, 100%).

**Example 9.51: 4-({[6-(3-Bromo-5-methyl-pyridin-2-ylamino)-pyrimidin-4-yl]-methyl-amino}-methyl)-piperidine-1-carboxylic acid tert-butyl ester (Compound A71).**

Compound **A71** was obtained as yellow solid (17mg, 23%). Exact mass calculated for $C_{22}H_{31}$ $BrN_6O_2$ 490.2, found LCMS (ESI) m/z 491.3(M+H$^+$, 100%).

**Example 9.52: 4-({Methyl-[6-(5-trifluoromethyl-pyridin-2-ylamino)-pyrimidin-4-yl]-amino}-methyl)-piperidine-1-carboxylic acid tert-butyl ester (Compound A72).**

Compound **A72** was obtained as yellow solid (2mg, 3%). Exact mass calculated for $C_{22}H_{29}F_3N_6O_2$ 466.2, found LCMS (ESI) m/z 467.3(M+H$^+$, 100%).

**Example 9.53: 4-({[6-(4-Bromo-2-fluoro-phenylamino)-pyrimidin-4-yl]-methyl-amino}-methyl)-piperidine-1-carboxylic acid tert-butyl ester (Compound A73).**

Compound **A73** was obtained as cream solid (9mg, 12%). Exact mass calculated for $C_{22}H_{29}$ $BrFN_5O_2$ 493.2, found LCMS (ESI)-m/z-496.4(M+H$^+$, 100%).

**Example 9.54: 4-({[6-(3-Carboxy-4-fluoro-phenylamino)-pyrimidin-4-yl]-methyl-amino}-methyl)-piperidine-1-carboxylic acid tert-butyl ester (Compound A74).**

Compound **A74** was obtained as cream solid (1mg, 1%). Exact mass calculated for $C_{23}H_{30}$ $FN_5O_4$459.2, found LCMS (ESI) m/z 460.4(M+H$^+$, 100%).

**Example 9.55: 4-({[6-(4-Ethoxycarbonyl-2-fluoro-phenylamino)-pyrimidin-4-yl]-methyl-amino}-methyl)-piperidine-1-carboxylic acid isobutyl ester (Compound A75).**

**and**

**Example 9.56: 4-({[6-(4-Carboxy-2-fluoro-phenylamino)-pyrimidin-4-yl]-methyl-amino}-methyl)-piperidine-1-carboxylic acid isobutyl ester (Compound A76).**

**Step 1: Preparation of (4-cyano-2-fluoro-phenyl)-carbamic acid 9H-fluoren-9-ylmethyl ester.**

A mixture of 2.05 g (15.06 mmol) 4-amino-3-fluorobenzonitrile and 2 g (23.8 mmol) sodium bicarbonate in 30 mL acetonitrile was cooled in an ice-bath and 4.4 g (17.0 mmol) FMOC-Cl were added. Mixture was allowed to warm to room temperature. After 16 h, mixture was concentrated and extracted with 1M HCl and ethyl acetate. Combined organic phases were dried over magnesium sulfate, filtered, and concentrated. Crystallization out of ethyl acetate/hexane gave (4-cyano-2-fluorophenyl)-carbamic acid 9H-fluoren-9-ylmethyl ester as a white solid (2.68 g, 50%). $^1$HNMR ($CDCl_3$, 400 MHz) d 4.21-4.24 (t, 1H), 4.50-4.54 (d, 2H), 6.99 (s br, 1H), 7.19-7.32 (m, 6H), 7.48-7.58 (m, 2H), 7.67-7.74 (m, 2H), 8.16 (s br, 1H). Exact mass calculated for $C_{22}H_{15}FN_2O_2$ 358.11, found 358.9 (MH$^+$).

**Step 2: Preparation of 4-amino-3-fluoro-benzoic acid ethyl ester.**

HCl was bubbled slowly through a solution of 1.6 g (4.46 mmol) (4-cyano-2-fluoro-phenyl)-carbamic acid 9*H*-fluoren-9-ylmethyl ester in 300 mL EtOH. After 5 h solution was concentrated. Residue was dissolved in 100 mL THF and 100 mL 6 M HCl in water. After stirring for 4 h at 50°C, mixture was concentrated. Residue was treated with 2M $NHEt_2$ in MeOH. After 3h, mixture was concentrated, and residue was purified by HPLC to give 4-amino-3-fluoro-benzoic acid ethyl ester as a white solid (TFA salt, 0.696 g, 42%). $^1$HNMR (MeOD, 400 MHz) δ 1.20-1.25 (t, 3H), 4.13-4.22 (q, 2H), 6.66-6.71 (m, 1H), 7.42-7.50 (m, 2H). Exact mass calculated for $C_9H_{10}FNO_2$ 183.07, found 184.0 (MH$^+$).

**Step 3: Preparation of Compound A75 and Compound A76.**

Compound **A75** was prepared in a similar manner as described above as a TFA salt, 13.2 mg, 11% (tan solid), and Compound **A76** was also obtained as a TFA salt, 24.4 mg, 21% (tan solid).

Compound **A75**: $^1$HNMR (MeOD, 400 MHz) δ 0.79-0.84 (d, 6H), 1.10-1.19 (m, 2H), 1.28-1.32 (t, 3H), 1.56-1.59 (m, 2H), 1.79-1.94 (m, 2H), 2.62-2.81 (m, 2H), 3.03 (s, 3H), 3.38-3.51 (m, 2H), 3.74-3.76 (d, 2H), 4.04-4.07(d br; 2H), 4.26-4.32 (m, 2H), 5.91 (s, 1H), 7.67-7.83 (m, 3H), 8.18 (s, 1H); Exact mass calculated for $C_{25}H_{34}FN_5O_4$ 487.26, found 488.2 ($MH^+$).

Compound A76: $^1$HNMR (MeOD, 400 MHz) δ 0.89-0.91 (d, 6H), 1.05-1.20 (m, 2H), 1.55-1.58 (m, 2H), 1.78-1.95 (m, 2H), 2.60-2.80 (m, 2H), 3.14 (s, 3H), 3.41-3.51 (m, 2H), 3.72-3.74 (d, 2H), 4.01-4.06 (d br, 2H), 5.89 (s, 1H), 7.62-7.66 (m, 1H), 7.76-7.84 (m, 2H), 8.18 (s, 1H); Exact mass calculated for $C_{23}H_{30}FN_5O_4$ 459.23, found 460.3 ($MH^+$).

**Example 9.57: 4-({[6-(4-Cyano-2-fluoro-phenylamino)-pyrimidin-4-yl]-methyl-amino}-methyl)-piperidine-1-carboxylic acid isopropyl ester (Compound A77).**

Compound **A77** was obtained as a solid (20 mg, 48%). $^1$HNMR ($CDCl_3$, 400 MHz) δ 1.19-1.24 (m, 8H), 1.61-1.64 (m, 2H), 1.90-1.96 (m, 1H), 2.68-2.74 (m, 2H), 3.01-3.04 (m, 3H), 3.45-3.55 (m, 2H), 4.10-4.30 (m, 2H), 4.89-4.92 (m, 1H), 5.73 (s, 1H), 7.36-7.45 (m, 2H), 8.34 (s, 1H), 8.50 (t, 1H). Exact mass calculated for $C_{22}H_{27}FN_6O_2$ 426.2, found 427.2 ($MH^+$).

**Example 9.58: 4-({[6-(4-Cyano-2-fluoro-phenylamino)-pyrimidin-4-yl]-methyl-amino }-methyl)-piperidine-1-carboxylic acid butyl ester (Compound A78).**

Compound **A78** was obtained as a trifluoroacetic acid salt (30 mg, 68%). $^1$HNMR ($CDCl_3$, 400 MHz) δ 0.94 (t, 3H), 1.20-1.26 (m, 2H), 1.33-1.43 (m, 2H), 1.58-1.63 (m, 4H), 1.90-1.96 (m, 1H), 2.62-2.78 (m, 2H), 3.00 (s, 3H), 3.66-3.68 (m, 2H), 4.06-4.30 (m, 4H), 5.37 (s, 1H), 7.48-7.58 (m, 3H), 8.20 (s, 1H), 11.9 (s, 1H). Exact mass calculated for $C_{23}H_{29}FN_6O_2$ 440.2, found 441.4 ($M+H^+$).

**Example 9.59: 4-({[6-(4-Cyano-2-fluoro-phenylamino)-pyrimidin-4-yl]-methyl-amino}-methyl)-piperidine-1-carboxylic acid cyclopropylmethyl ester (Compound A79).**

Compound **A79** was obtained as a solid (26 mg, 45%). Exact mass calculated for $C_{23}H_{27}FN_6O_2$ 438.2, found 439.3 ($M+H^+$).

**Example 9.60: {4-[6-(2-Fluoro-4-methanesulfonyl-phenylamino)-pyrimidin-4-yl]-piperazin-1-yl}-acetic acid ethyl ester (Compound A80).**

**Step 1: Preparation of [4-(6-chloro-pyrimidin-4-yl)-piperazin-1-yl]-acetic acid ethyl ester.**

A mixture of 4,6-dichloropyrimidine (1 g, 6.75 mmol), 1-(ethoxycarbonylmethyl) piperazine (1.16 g, 6.75 mmol) and diisopropyl ethyl amine in isopropyl alcohol (8 mL) was heated under microwave irradiation at 100˚C for 2 minutes. The crude was purified by flash chromatography (hexane : ethyl acetate = 1:2) to give [4-(6-chloro-pyrimidin-4-yl)-piperazin-1-yl]-acetic acid ethyl ester as an oil (1.80 g, 93%). $^1$HNMR ($CDCl_3$, 400 MHz) δ 1.29 (t, 3H), 2.69 (s, 4H), 3.29 (s, 2H), 3.72 (s, 4H), 4.20 (q, 2H), 6.50 (s, 1H), 8.37 (s, 1H). Exact mass calculated for $C_{12}H_{17}ClN_4O_2$ 284.2, found 258.0 ($M+H^+$).

**Step 2: Preparation of {4-[6-(2-Fluoro-4-methanesulfonyl-phenylamino)-pyrimidin-4-yl]-piperazin-1-yl}-acetic acid ethyl ester (Compound A80).**

Compound **A80** was prepared in a similar manner as described above as a solid (290 mg, 62%). $^1$HNMR ($CDCl_3$, 400 MHz) δ 1.42 (t, 3H), 2.90-2.93 (m, 4H), 3.06-3.07 (m, 3H), 3.46 (s, 2H), 3.80-3.90 (m, 4H), 4.40 (q, 2H), 5.88 (s, 1H), 7.70-7.78 (m, 2H), 8.46 (s, 1H), 8.61-8.65 (t, 1H), 9.83 (s, 1H). Exact mass calculated for $C_{19}H_{24}FN_5O_4S$ 437.2, found 438.2 ($M+H^+$).

**Example 9.61: (2-Fluoro-4-methanesulfonyl-phenyl)-{6-[4-(3-isopropyl-[1,2,4]oxadiazol-5-ylmethyl)-piperazin-1-yl]-pyrimidin-4-yl}-amine (Compound A81).**

Compound **A81** was obtained as a solid (30 mg, 14%). Exact mass calculated for $C_{21}H_{26}FN_7O_3S$ 475.2, found

476.2 ($M+H^+$).

**Example 9.62: 4-({[6-(2,5-Difluoro-4-hydroxy-phenylamino)-pyrimidin-4-yl]-methyl-amino}-methyl)-piperidine-1-carboxylic acid isobutyl ester (Compound A82).**

Compound **A82** (TFA salt, 77.9 mg, 44%) was obtained as a tanned solid. $^1$HNMR (DMSO, 400 MHz) δ 0.84-0.86 (d, 6H), 1.01-1.15 (m, 2H), 1.54-.1.57 (m, 2H), 1.80-1.99 (m, 2H), 2.65-2.85 (m, 2H), 3.06 (s, 3H), 3.40-3.52 (m, 2H), 3.76-3.81 (d, 2H), 3.92-4.01 (m, 3H), 5.72 (s br, 1H), 6.90-6.98 (m, 1H), 7.41-7.45 (s, 1H), 8.31 (s, 1H), 9.73 (s br 1H), 10.50 (s br, 1H). Exact mass calculated for $C_{22}H_{29}F_2N_5O_3$ 449.2, found 450.3 ($MH^+$).

**Example 9.63: 4-({[6-(4-Ethylcarbamoyl-2-fluoro-phenylamino)-pyrimidin-4-yl]-methyl-amino}-methyl)-piperidine-1-carboxylic acid isobutyl ester (Compound A83).**

A mixture of compound **A76** (TFA salt, 35.2 mg, ≤ 0.061 mmol) and HBTU (49 mg, 0.13 mmol) in 1 mL 2M ethyl amine was stirred at room temperature. After 10 min, the solution was continued to be stirred in microwave at 180˚C. After 1 hour, mixture was purified by HPLC to give compound **A83** as a white solid (TFA salt, 9.5 mg, 26%). $^1$HNMR (MeOD, 400 MHz) δ 0.81-0.83 (d, 6H), 1.05-1.16 (m, 5H), 1.53-1.61 (m, 2H), 1.79-1.99 (m, 2H), 2.62-2.80 (m, 2H), 3.07 (s, 3H), 3.30-3.35 (q, 2H), 3.42-3.55 (m, 2H), 3.74-3.76 (d, 2H), 4.03-4.07 (d br, 2H), 5.82 (s br, 1H), 7.54-7.68 (m, 3H), 8.17 (s,1H). Exact mass calculated for $C_{25}H_{35}FN_6O_3$ 486.28, found 487.3 ($MH^+$).

**Example 9.64: 4-[({6-[2-Fluoro-4-(N-hydroxycarbamimidoyl)-phenylamino]-pyrimidin-4-yl}-methyl-amino)-methyl]-piperidine-1-carboxylic acid isobutyl ester (Compound A84).**

A mixture of 4-({[6-(4-cyano-2-fluoro-phenylamino)-pyrimidin-4-yl]-methyl-amino}-methyl)-piperidine-1-carboxylic acid isobutyl ester (Compound **A51**, 33.5 mg, 0.060 mmol), hydroxylamine hydrochloride (430 mg, 6.19 mmol), and potassium carbonate (870 mg, 6.29 mmol) in 1 mL EtOH and 0.5 mL $H_2O$ was stirred for 20 min at 80˚C. Mixture was purified by HPLC to give Compound **A84** as a white solid (TFA salt, 32.2 mg, 91%). $^1$HNMR (MeOD, 400 MHz) δ 0.83-0.86 (d, 6H), 1.10-1.17 (m, 2H), 1.57-1.60 (m, 2H), 1.88-1.99 (m, 2H), 2.63-.2.80 (m, 2H), 3.03 (s, 3H), 3.40-3.45 (m, 2H), 3.74-3.76 (d, 2H), 4.04-4.07 (d br, 2H), 6.03 (s, 1H), 7.45-7.54 (m, 2H), 8.14-8.19 (m, 2H). Exact mass calculated for $C_{23}H_{32}FN_7O_3$ 473.26, found 474.5 ($MH^+$).

**Example 9.65: 4-({[6-(4-Carbamimidoyl-2-fluoro-phenylamino)-pyrimidin-4-yl]-methyl-amino}-methyl)-piperidine-1-carboxylic acid isobutyl ester (Compound A90).**

To a solution of Compound **A84** (TFA salt, 22.6 mg, 0.0385 mmol) in 1 mL acetic acid, zinc powder (50 mg, 0.76 mmol) was added. After stirring the mixture for 10 min, Zn was filtered off; filtrate was concentrated, and purified by HPLC to give Compound A90 as a white solid (8.4 mg, 38%). $^1$HNMR (MeOD, 400 MHz) δ 0.80-8.83 (d, 6H), 1.05-1.17 (m, 2H), 1.55-1.61 (m, 2H), 1.80-2.01 (m, 2H), 2.61-2.81 (m, 2H), 3.07 (s, 3H), 3.40-3.47 (m, 2H), 3.69-3.71 (d, 2H), 4.04-4.07 (d br, 2H), 6.05 (s, 1H), 7.54-7.67 (m, 2H), 8.07-8.12 (m, 1H), 8.22 (s, 1H). Exact mass calculated for $C_{23}H_{32}FN_7O_2$ 457.26, found 458.3 ($MH^+$).

**Example9.66:4-({[6-(2,5-Difluoro-4-methanesulfonyl-phenylamino)-pyrimidin-4-yl]-methyl-amino}-methyl)-piperidine-1-carboxylic acid tert-butyl ester (Compound A86).**

Compound **A86** was obtained as a solid (57 mg, 37%). Exact mass calculated for $C_{23}H_{31}F_2N_5O_4S$ 511.2, found 512.3 ($M+H^+$).

**Example 9.67: N-(2-Fluoro-4-methanesulfonyl-phenyl)-N'-(5'-fluoro-3,4,5,6-tetrahydro-2H-[1,2'] bipyridinyl-4-ylmethyl)-N'-methyl-pyrimidine-4,6-diamine (Compound A89).**

Compound **A89** was obtained as a solid (3 mg, 6%). Exact mass calculated for $C_{23}H_{26}F_2N_6O_2S$ 488.2, found 489.2 ($M+H^+$).

**Example 9.68: 4-[6-(2-Fluoro-4-methanesulfonyl-phenylamino)-pyrimidin-4-ylamino]-piperidine-1-carboxylic acid tert-butyl ester (Compound A92).**

Compound **A92** was obtained as a solid (18 mg, 13%). Exact mass calculated for $C_{21}H_{28}FN_5O_4S$ 465.2, found 466.3 ($M+H^+$).

**(Reference) Example 9.69: *N*-(2-Fluoro-4-methanesulfonyl-phenyl)-N'-[1-(3-isopropyl-[1,2,4]oxadiazol-5-ylmethyl)-piperidin-4-ylmethyl]-N'-methyl-pyrimidine-4,6-diamine (Compound A93).**

Compound A93 was obtained as a solid (45 mg, 38%). Exact mass calculated for $C_{24}H_{32}FN_7O_3S$ 517.2, found 518.4 (M+H$^+$).

**Example 9.70: 4-({[6-(4-Cyano-2,5-difluoro-phenylamino)-pyrimidin-4-yl]-ethyl-amino}-methyl)-piperidine-1-carboxylic acid isopropyl ester (Compound A97).**

A mixture of 4-[6-(ethyl-piperidin-4-ylmethyl-amino)-pyrimidin-4-ylamino]-2,5-difluoro-benzonittile (HCl-salt, 1.8712 g, 4.57 mmol), triethylamine (1.91 mL, 13.71 mmol), and isopropyl chloroformate (1M in toluene, 9.14 mL, 9.14 mmol) in 100 mL $CH_3CN$ was stirred at room temperature for 30 min. Mixture was purified by HPLC to give Compound **A97** as a white solid (TFA salt, 600 mg, 23%). $^1$HNMR (MeOD-d$_4$,400 MHz) δ 1.13-1.21 (d br, 11H), 1.59-1.67 (d, 2H), 1.96 (s br, 1H), 2.7 (s br, 2H), 3.4 (s br, 2H), 3.51-3.61 (d br, 2H), 4.05-4.15 (d, 2H), 4.75-4.83 (m, 1H), 6.26 (s, 1H), 7.61-7.67 (m, 1H), 8.04-8.11 (m, 1H), 8.33 (s, 1H). Exact mass calculated for $C_{23}H_{28}F_2N_6O_2$ 458.22, found 459.5 (MH$^+$).

**Example 9.71: 4-({[6-(2,5-Difluoro-4-methoxy-phenylamino)-pyrimidin-4-yl]-ethyl-amino}-methyl)-piperidine-1-carboxylic acid tert-butyl ester (Compound A99).**

A mixture of 4-({[6-(2,5-difluoro-4-hydroxy-phenylamino)-pyrimidin-4-yl]-ethyl-amino}-methyl)-piperidine-1-carboxylic acid tert-butyl ester (61.9 mg, 0.134 mmol), diisopropyl ethylamine (33 μl, 0.189 mmol), and (trimethylsilyl) diazomethane (2M in $Et_2O$, 94 μl, 0.188 mmol) in 0.5 mL $CH_3CN$/MeOH 9:1 was stirred at room temperature for 15 h. Mixture was purified by HPLC to give Compound **A99** as a white solid (TFA salt, 24.3 mg, 31%). $^1$HNMR (MeOD, 400 MHz) δ 1.02-1.12 (m, 5H), 1.36 (s, 9H), 1.50-1.60 (m, 2H), 1.81-1.90 (m, 1H), 2.59-2.70 (m, 2H), 3.33-3.52 (m, 4H), 3.82 (s, 3H), 3.98-4.05 (d br, 2H), 5.64 (s, 1H), 6.78-6.80 (m, 1H), 7.05-7.20 (m, 1H), 8.09 (s, 1H). Exact mass calculated for $C_{24}H_{33}F_2N_5O_3$ 477.26, found 478.1 (MH$^+$).

**Example 9.72: 4-({[6-(2,5-Difluoro-4-methanesulfonyl-phenylamino)-pyrimidin-4-yl]-ethyl-amino}-methyl)-piperidine-1-carboxylic acid tert-butyl ester (Compound A100).**

Compound **A100** was obtained as an off-white powder (32.5 mg, 22 %). Exact mass calculated for $C_{24}H_{33}F_2N_5O_4S$ 525.2, found LCMS (ESI) m/z 526.5 (M+H$^+$, 89%).

**Example 9.73: 4-({Ethyl-[6-(2,4,5-trifluoro-phenylamino)-pyrimidin-4-yl]-amino}-methyl)-piperidine-1-carboxylic acid tert-butyl ester (Compound A101).**

Compound **A101** was obtained as an off-white powder (16.3 mg, 13%). Exact mass calculated for $C_{23}H_{30}F_3N_5O_2$ 465.2, found LCMS (ESI) m/z 466.4 (M+H$^+$-100%).

**Example 9.74: 4-({[6-(4-Bromo-2,5-difluoro-phenylamino)-pyrimidin-4-yl]-ethyl-amino}-methyl)-piperidine-1-carboxylic acid tert-butyl ester (Compound A104).**

Compound **A104** was obtained as a white powder (20.1 mg, 14%). Exact mass calculated for $C_{23}H_{30}BrF_2N_5O_2$ 525.2, found LCMS (ESI) m/z 528.5 (M+H$^+$, 74%).

**Example 9.75: {1-[6-(2-Fluoro-4-methanesulfonyl-phenylamino)-pyrimidin-4-yl]-piperidin-4-yl}-acetic acid methyl ester (Compound A106).**

Compound **A106** was obtained as a solid (62 mg, 30%). Exact mass calculated for -$C_{19}H_{23}FN_4O_4S$ 422.1, found 423.1 (M+H$^+$).

**Example 9.76: 3-{4-[6-(2-Fluoro-4-methanesulfonyl-phenylamino)-pyrimidin-4-yl]-piperazin-1-yl}-propionic acid ethyl ester (Compound A107).**

Compound **A107** was obtained as a solid (50 mg, 23%). Exact mass calculated for $C_{20}H_{26}FN_5O_4S$ 451.2, found 452.1 (M+H$^+$).

**Example 9.77: (2-Fluoro-4-methanesulfonyl-phenyl)-{6-[4-(4-isobutyl-phenyl)-piperidin-1-yl]-pyrimidin-4-yl}-amine (Compound A108).**

Compound **A108** was obtained as a solid (35 mg, 15%). Exact mass calculated for $C_{26}H_{31}FN_4O_2S$ 482.2, found 483.4 ($M+H^+$).

**Example 9.78: (2-Fluoro-4-methanesulfonyl-phenyl)-{6-[4-(4-isopropyl-phenyl)-piperidin-1-yl]-pyrimidin-4-yl}-amine (Compound A109).**

Compound **A109** was obtained as a solid (44 mg, 19%). Exact mass calculated for $C_{25}H_{29}FN_4O_2S$ 468.2, found 469.4 ($M+H^+$).

**Example 9.79: (2-Fuoro-4-methanesulfonyl-phenyl)-(6-{4-[2-(3-isopropyl-[1,2,4]oxadiazol-5-yl)-ethyl]-piperazin-1-yl}-pyrimidin-4-yl)-amine (Compound A116).**

Compound **A116** was obtained as a solid (160 mg, 100%). Exact mass calculated for $C_{22}H_{28}FN_7O_3S$ 489.2, found 490.2 ($M+H^+$).

**Example 9.80: (2-Fluoro-4-methanesulfonyl-phenyl)-{6-[4-(5-isopropoxy-pyridin-2-yloxy)-piperidin-1-yl]-pyrimidin-4-yl}-amine (Compound A117).**

Compound **A117** was obtained as a solid (180 mg, 70%). $^1$HNMR ($CDCl_3$, 400 MHz) δ 1.36 (d, 6H), 1.95-2.08 (m, 4H), 3.07 (s, 3H), 4.50 (sept, 1H), 5.22 (s, 1H), 5.53 (s, 1H), 6.86-6.88 (m, 1H), 7.46-7.48 (m, 1H), 7.59-7.63 (m, 1H), 7.80-7.82 (m, 2H), 7.88 (s, 1H), 8.27 (s, 1H), 11.6 (s, 1H). Exact mass calculated for $C_{24}H_{28}FN_5O_4S$ 501.2, found 502.2 ($M+H^+$).

**Example9.81:4-({[6-(2-Fluoro-4-methanesulfonyl-phenylamino)-pyrimidin-4-yl]-isopropyl-amino}-methyl)-piperidine-1-carboxylic acid tert-butyl ester (Compound A122).**

Compound **A122** was obtained as a solid (33 mg, 32%). Exact mass calculated for $C_{25}H_{36}FN_5O_4S$ 521.2, found 522.5 ($MH^+$).

**Example9.82:4-({[6-(4-Methanesulfonyl-phenylamino)-pyrimidin-4-yl]-methyl-amino}-methyl)-piperidine-1-carboxylic acid tert-butyl ester (Compound A5).**

(6-Chloro-pyrimidin-4-yl)-(4-methanesulfonyl-phenyl)-amine (57mg, 0.2mmol), 4-methylaminomethyl-piperidine-1-carboxylic acid tert-butyl ester (0.2mmole, 1.0eq) and $K_2CO_3$ - (0.4mmol, 2eq) were dissolved in DMF (3mL) and then stirred at 120˚C for 24 hours. The crude was purified through HPLC provided Compound **A5** as white solid (48mg, 51 %). $^1$H NMR 400MHz $CDCL_3$ δ (ppm): 9.79(sb, NH); 8.30(s,1H); 7.84(d, 2H); 7.80 (d,2H); 5.95(s,1H); 3.94(m, 2H); 3.42(m, 2H); 3.15 (s, 3H); 3.03(s, 3H); 2.61(m, 2H); 1.95-1.87(m,1H); 1.57-1.54(m,2H); 1.39(s, 9H); 1.15-1.03(m, 2H). Exact mass calculated for $C_{23}H_{33}$ $N_5O_4$ S 475.2, LCMS (ESI) m/z 476.2($M+H^+$, 100%).

Using essentially the same methodology and procedures as described in the Reaction Scheme(s) and Examples herein, the following compounds are prepared from the appropriate materials:

**Example 9.83: 4-{[6-(2-Fluoro-4-methanesulfonyl-phenylamino)-pyrimidin-4-yl]-methyl-amino}-piperidine-1-carboxylic acid tert-butyl ester (Compound A3).**

Compound **A3** was obtained as a solid (6 mg, 20%). Exact mass calculated for $C_{22}H_{30}FN_5O_4S$ 479.2, found 480.4 ($M+H^+$),

**Example 9.84: 4-({Methyl-[6-(2-pyridin-4-yl-ethylamino)-pyrimidin-4-yl]-amino}-methyl)-piperidine-1-carboxylic acid tert-butyl ester (Compound A9).**

Compound **A9** was obtained as yellow solid (7mg, 8%). Exact mass calculated for $C_{23}H_{34}$ $N_6O_2$ 426.3, LCMS (ESI) m/z 427.2($M+H^+$, 100%).

**Example 9.85: 4-({Methyl-[6-(2-pyridin-3-yl-ethylamino)-pyrimidin-4-yl]-amino}-methyl)-piperidine-1-carboxylic acid tert-butyl ester (Compound A10).**

Compound **A10** was obtained as yellow solid (13mg, 15%). Exact mass calculated for $C_{23}H_{34}$ $N_6O_2$ 426.3,

LCMS (ESI) m/z 427.3(M+H+, 100%).

**Example 9.86: 4-[(Methyl-{6-[(pyridin-3-ylmethyl)-amino]-pyrimidin-4-yl}-amino)-methyl]-piperidine-1-carboxylic acid tert-butyl ester (Compound A11).**

Compound **A11** was obtained as yellow solid (5mg, 6%). Exact mass calculated for $C_{22}H_{32}N_6O_2$ 412.3, LCMS (ESI) m/z 413.4(M+H+, 100%).

**Example 9.87: 4-[(Methyl-{6-[2-(1-oxy-pyridin-3-yl)-ethylamino]-pyrimidin-4-yl}-amino)-methyl]-piperidine-1-carboxylic acid isobutyl ester (Compound A49).**

Compound **A49** was obtained as a solid (24 mg, 55%). Exact mass calculated for $C_{27}H_{39}FN_6O_4S$ 562.2, found 563.5 (M+H+).

**Example 9.88: 4-[({6-[2-(2-Fluoro-phenoxy)-ethylamino]-pyrimidin-4-yl}-methyl-amino)-methyl]-piperidine-1-carboxylic acid-tert-butyl ester (Compound A52).**

Compound **A52** was obtained as a solid (1 mg, 2%). Exact mass calculated for $C_{24}H_{34}FN_5O_3$ 459.2, found 460.3 (M+H+).

**Example 9.89: 4-({[6-(2-Fluoro-phenoxy)-pyrimidin-4-yl]-methyl-amino}-methyl)-piperidine-1-carboxylic acid tert-butyl ester (Compound A53).**

Compound **A53** was obtained as a solid (12 mg, 20%). Exact mass calculated for $C_{22}H_{29}FN_4O_3$ 416.2, found 417.4 (M+H+).

**Example 9.90: 4-({[6-(2,5-Difluoro-phenoxy)-pyrimidin-4-yl]-methyl-amino}-methyl)-piperidine-1-carboxylic acid tert-butyl ester (Compound A54).**

Compound **A54** was obtained as a solid (6 mg, 9%). Exact mass calculated for $C_{22}H_{28}F_2N_4O_3$ 434.2, found 435.2 (M+H+).

**Example 9.91: 4-[({6-[2-(2-Chloro-phenoxy)-ethylamino]-pyrimidin-4-yl}-methyl-amino)-methyl]-piperidine-1-carboxylic acid tert-butyl ester (Compound A55).**

Compound **A55** was obtained as a solid (5 mg, 9%). Exact mass calculated for $C_{24}H_{34}ClN_5O_3$ 475.2, found 476.3 (M+H+).

**Example 9.92: 4-({[6-(2-Chloro-phenoxy)-pyrimidin-4-yl]-methyl-amino}-methyl)-piperidine-1-carboxylic acid tert-butyl ester (Compound A56).**

Compound **A56** was obtained as a solid (16 mg, 25%). Exact mass calculated for $C_{22}H_{29}ClN_4O_3$ 432.2, found 433.2 (M+H+).

**Example 9.93: 4-[({6-[2-(4-Fluoro-phenoxy)-propylamino]-pyrimidin-4-yl}-methyl-amino)-methyl]-piperidine-1-carboxylic acid tert-butyl ester (Compound A57).**

Compound **A57** was obtained as a solid (12 mg, 17%). Exact mass calculated for $C_{25}H_{36}FN_5O_3$ 473.2, found 474.4 (M+H+).

**Example 10:**

**Example 10.1: Preparation of 4-[6-(2-Fluoro-4-methanesulfonyl-phenylamino)-pyrimidin-4-yloxy]-piperidine-1-carboxylic acid tert-butyl ester, also referred to herein as Compound B1.**

**Step 1: Preparation of 4-(6-chloro-pyrimidin-4-yloxy)-piperidine-1-carboxylic acid tert-butyl ester.**

4-Hydroxy-piperidine-1-carboxylic acid tert-butyl ester (46mmol, 1.3eq) and NaH (92mmole, 2eq, 60% in mineral oil) were dissolved in THF (30ml) under $N_2$ and stirred at 60˚C for 40 minutes then 4,6-dichloropyrimidine (5.237g, 35.4mmole) was added into the solution dropwise. The reaction mixture was stirred at room temp for another 20minutes. The reaction was quenched with water, extracted with ethyl acetate, and concentrated in vacuo, and purified by flash column (Hexane : Ethyl Acetate= 2:1, Rf=0.48) to provide 4-(6-chloro-pyrimidin-4-yloxy)-piperidine-1-carboxylic acid tert-butyl ester as a yellow oil (3.236 g, 29 %). Exact mass calculated for $C_{14}H_{20}ClN_3O_3$ 313.1, found 314.2 ($MH^+$).

**Step 2: Preparation of 2-fluoro-4-(methanesulfonyl)aniline.**

To a solution of 2-fluoro-4-iodoaniline (206.8 g, 872.3 mmol) in DMSO (1.1 L), was added by the sequential addition of copper (II) trifluoromethanesulfonate-benzene complex (30.74 g, 61.1 mmol), sodium methanesulfinate (106.9 g, 1.047 mol), and *N,N'*-dimethylethylenediamine (13.2 mL, 122 mmol). The reaction vessel was then placed in a preheated oil bath at 120 ˚C and stirred overnight. After cooling to room temperature, the reaction was diluted with water and extracted repeatedly with EtOAc. The combined organic extract was rinsed with brine (5X), dried over $MgSO_4$, and the solvent was removed. The resulting purple solid was rinsed with diisopropyl ether, then dried to constant weight in a vacuum oven at room temperature overnight to furnish a dull purple solid 151.5 g, 92% yield: $^1H$ NMR (DMSO-$d_6$) δ 7.48 (d, 1 H, J = 11.2 Hz), 7.41 (d, 1 H, J = 8.5 Hz), 6.87 (t, 1H, J = 8.6 Hz), 6.19 (s, 2 H), 3.10 (s, 3 H); MS m/z 190.3 ($M^+$).

**Step 3: Preparation of 4-[6-(2-Fluoro-4-methanesulfonyl-phenylamino)-pyrimidin-4-yloxyl-piperidine-1-carboxylic acid tert-butyl ester (Compound B1).**

A mixture of 4-(6-chloro-pyrimidin-4-yloxy)-piperidine-1-carboxylic acid tert-butyl ester (3.23 g, 10.3 mmol), 2-fluoro-4-methanesulfonylaniline (1.95 g, 10.3 mmol), palladium acetate (115 mg, 0.515 mmol), ditbutyl-biphenylphosphine (184 mg, 0.618 mmol) and sodium t-butoxide (2.47 g, 25.75 mmol) in dioxane (20 ml) was heated to reflux for 2 hours under nitrogen gas. The crude mixture was quenched with water, extracted with ethyl acetate and concentrated in vacuo. The crude was purified by flash column (hexanes: ethyl acetate =1:1) to provide Compound **B1** as a solid (1.69 g, 35%). $^1$HNMR ($CDCl_3$, 400 MHz) δ 1.44 (s, 9H), 1.63-1.80 (m, 2H), 1.97-2.06 (m, 2H), 3.07 (s, 3H), 3.25-3.31 (m, 2H), 3.70-3.78 (m, 2H), 5.27-5.33 (m, 1H), 6.18 (s, 1H), 7.52-7.57 (m, 1H), 7.70-7.77 (m, 2H), 8.51 (s, 1H). Exact mass calculated for $C_21H_{27}FN_4O_5S$ 466.2, found 467.2 ($MH^+$).

Using essentially the same methodology and procedures as described in the Reaction Scheme(s) and Examples herein, the following compounds were prepared from the appropriate materials.

(Reference) **Example 10.2: (2-Fluoro-4-methanesulfonyl-phenyl)-{6-[1-(3-isopropyl-[1,2,4]oxadiazol-5-ylmethyl)-piperidin-4-yloxy]-pyrimidin-4-yl}-amine (Compound B2).**

Compound **B2** was obtained as a solid (253 mg, 93%). Exact mass calculated for $C_{22}H_{27}FN_6O_4S$ 490.2, found 491.2 ($M+H^+$).

**Example 10.3: 4-[6-(2,5-Difluoro-4-methanesulfonyl-phenylamino)-pyrimidin-4-yloxy]-piperidine-1-carboxylic acid tert-butyl ester (Compound B13).**

Compound **B13** was obtained as a yellow powder (2.3 mg, 2 %). Exact mass calculated for $C_{21}H_{26}F_2N_4O_5S$ 484.2, found LCMS (ESI) m/z 485.2 ($M+H^{+,}$ 86%).

**Example 10.4: 4-[6-(2,4,5-Trifluoro-phenylamino)-pyrimidin-4-yloxy]-piperidine-1-carboxylic acid tert-butyl ester (Compound B14).**

Compound **B14** was obtained as a yellow powder (6 mg, 7%). Exact mass calculated for $C_{20}H_{23}F_3N_4O_3$ 424.2, found LCMS (ESI) m/z 425.3 ($M+H^{+,}$99%).

**Example 10.5: 4-[6-(4-Bromo-2,5-difluoro-phenylamino)-pyrimidin-4-yloxy]-piperidine-1-carboxylic acid tert-butyl ester (Compound B15).**

Compound **B15** was obtained as a yellow powder (1 mg, 1%). Exact mass calculated for $C_{20}H_{23}BrF_2N_4O_3$ 484.1, found LCMS (ESI) m/z 501.5 ($M+H^{+,}$78%).

**Example 10.6: 4-[6-(6-Chloro-pyridin-3-ylamino)-pyrimidin-4-yloxy]-piperidine-1-carboxylic acid tert-butyl ester (Compound B20).**

Compound **B20** was obtained as a tan powder (10.3 mg, 10%). Exact mass calculated for $C_{19}H_{24}ClN_5O_3$ 405.2, found LCMS (ESI) m/z 406.2 (M+H$^+$,100%).

**Example 10.7: 4-[6-(4-Ethylsulfanyl-phenylamino)-pyrimidin-4-yloxy]-piperidine-1-carboxylic acid tert-butyl ester (Compound B27).**

Compound **B27** was obtained as a white powder (1 mg, 1%). Exact mass calculated for $C_{22}H_{29}FN_4O_3S$ 430.2, found LCMS (ESI) m/z 431.2 (M+H$^+$,76%).

**Example 10.8: 4-[6-(4-Isopropylsulfanyl-phenylamino)-pyrimidin-4-yloxy]-piperidine-1-carboxylic acid tert-butyl ester (Compound B28).**

Compound **B28** was obtained as an oil (1.4 mg, 2%). Exact mass calculated for $C_{23}H_{31}FN_4O_3S$ 484.1, found LCMS (ESI) m/z 445.6 (M+H$^+$,80%).

**Example 10.9: 4-[6-(5-Chloro-3-methyl-pyridin-2-ylamino)-pyrimidin-4-yloxy]-piperidine-1-carboxylic acid tert-butyl ester (Compound B30).**

Compound **B30** was obtained as an off-white powder (9 mg, 10%). Exact mass calculated for $C_{20}H_{26}ClN_5O_3$ 419.2, found LCMS (ESI) m/z 420.6 (M+H$^+$,80%).

**Example 10.10: 4-[6-(6-Acetylamino-4-methyl-pyridin-3-ylamino)-pyrimidin-4-yloxy]-piperidine-1-carboxylic acid tert-butyl ester (Compound B31).**

Compound **B31** was obtained as a brown powder (2.3 mg, 2%). Exact mass calculated for $C_{22}H_{30}N_6O_4$ 442.2, found LCMS (ESI) m/z 443 (M+H$^+$,41%).

**Example 10.11: 4-[6-(5-Fluoro-4-methyl-pyridin-2-ylamino)-pyrimidin-4-yloxy]-piperidine-1-carboxylic acid tert-butyl ester (Compound B32).**

Compound **B32** was obtained as a peach powder (15.4 mg, 20%). Exact mass calculated for $C_{20}H_{26}FN_5O_3$ 403.2, found LCMS (ESI) m/z 404.3 (M+H$^+$,99%).

**Example 10.12: 4-[6-(6-Methoxy-5-methyl-pyridin-3-ylamino)-pyrimidin-4-yloxy]-piperidine-1-carboxylic acid tert-butyl ester (Compound B33).**

Compound **B33** was obtained as an off-white powder (10.7 mg, 10%). Exact mass calculated for $C_{21}H_{29}N_5O_4$ 415.2, found LCMS (ESI) m/z 416.3 (M+H$^+$,92%).

**Example 10.13: 4-[6-(6-Methoxy-2-methyl-pyridin-3-ylamino)-pyrimidin-4-yloxy]-piperidine-1-carboxylic acid tert-butyl ester (Compound B34).**

Compound **B34** was obtained as an off-white powder (2 mg, 2%). Exact mass calculated for $C_{21}H_{29}N_5O_4$ 415.2, found LCMS (ESI) m/z 416.3 (M+H$^+$,94%).

**Example 10.14: 4-[6-(6-Fluoro-5-methyl-pyridin-3-ylamino)-pyrimidin-4-yloxy]-piperidine-1-carboxylic acid tert-butyl ester (Compound B35).**

Compound **B35** was obtained as a tan powder (12.7 mg, 20%). Exact mass calculated for $C_{20}H_{26}FN_5O_3$ 403.2, found LCMS (ESI) m/z 404.3 (M+H$^+$,92%).

**Example 10.15: 4-[6-(2-Chloro-6-methyl-pyridin-3-ylamino)-pyrimidin-4-yloxy]-piperidine-1-carboxylic acid tert-butyl ester (Compound B36).**

Compound **B36** was obtained as a yellow powder (11.5 mg, 10%). Exact mass calculated for $C_{20}H_{26}ClN_5O_3$

419.2, found LCMS (ESI) m/z 420.5 ($M+H^{+}$,99%).

**Example 10.16: 4-[6-(4-Methyl-pyridin-3-ylamino)-pyrimidin-4-yloxy]-piperidine-1-carboxylic acid tert-butyl ester (Compound B37).**

Compound **B37** was obtained as an off-white powder (8.2 mg, 10%). Exact mass calculated for $C_{20}H_{27}N_5O_3$ 385.2, found LCMS (ESI) m/z 386.2 ($M+H^{+}$,97%).

**Example 10.17: 4-[6-(2-Methyl-pyridin-3-ylamino)-pyrimidin-4-yloxy]-piperidine-1-carboxylic acid tert-butyl ester (Compound B38).**

Compound **B38** was obtained as an off-white powder (7 mg, 10%). Exact mass calculated for $C_{20}H_{27}N_5O_3$ 385.2, found LCMS (ESI) m/z 386.2 ($M+H^{+}$,99%).

**Example 10.18: 4-[6-(6-Chloro-2-methyl-pyridin-3-ylamino)-pyrimidin-4-yloxy]-piperidine-1-carboxylic acid tert-butyl ester (Compound B39).**

Compound **B39** was obtained as a yellow powder (3.7 mg, 4%). Exact mass calculated for $C_{20}H_{26}ClN_5O_3$ 419.2, found LCMS (ESI) m/z 420.5 ($M+H^{+}$,95%).

**Example 10.19: 4-[6-(6-Fluoro-pyridin-3-ylamino)-pyrimidin-4-yloxy]-piperidine-1-carboxylic acid tert-butyl ester (Compound B40).**

Compound **B40** was obtained as a tan powder (13.8 mg, 20%). Exact mass calculated for $C_{19}H_{24}FN_5O_3$ 389.2, found LCMS (ESI) m/z 390.2 ($M+H^{+}$,91%).

**Example 10.20: 4-[6-(2-Chloro-4-methyl-pyridin-3-ylamino)-pyrimidin-4-yloxy]-piperidine-1-carboxylic acid tert-butyl ester (Compound B41).**

Compound **B41** was obtained as an off-white powder (7.8 mg, 10%). Exact mass calculated for $C_{20}H_{26}ClN_5O_3$ 419.2, found LCMS (ESI) m/z 420.5 ($M+H^{+}$,99%).

**Example 10.21: 4-[6-(6-Methoxy-pyridin-3-ylamino)-pyrimidin-4-yloxy]-piperidine-1-carboxylic acid tert-butyl ester (Compound B42).**

Compound **B42** was obtained as a gold powder (17.4 mg, 20%). Exact mass calculated for $C_{20}H_{27}N_5O_4$ 401.2, found LCMS (ESI) m/z 402.2 ($M+H^{+}$,99%).

**Example 10.22: 4-[6-(5-Fluoro-pyridin-2-ylamino)-pyrimidin-4-yloxy]-piperidine-1-carboxylic acid tert-butyl ester (Compound B43).**

Compound **B43** was obtained as a peach powder (16.2 mg, 20%). Exact mass calculated for $C_{19}H_{24}FN_5O_3$ 389.2, found LCMS (ESI) m/z 390.4 ($M+H^{+}$,96%).

**Example 10.23: 4-[6-(2-Fluoro-pyridin-3-ylamino)-pyrimidin-4-yloxy]-piperidine-1-carboxylic acid tert-butyl ester (Compound B44).**

Compound **B44** was obtained as a brown powder (9.6 mg, 10%). Exact mass calculated for $C_{19}H_{24}FN_5O_3$ 389.2, found LCMS (ESI) m/z 390.1 ($M+H^{+}$,99%).

**Example 10.24: 4-[6-(6-Chloro-5-methyl-pyridin-3-ylamino)-pyrimidin-4-yloxy]-piperidine-1-carboxylic acid tert-butyl ester (Compound B45).**

Compound **B45** was obtained as a gold powder (13.3 mg, 20%). Exact mass calculated for $C_{20}H_{26}ClN_5O_3$ 419.2, found LCMS (ESI) m/z 420.6 ($M+H^{+}$,100%).

**Example 10.25: 4-[6-(2-Methyl-pyridin-4-ylamino)-pyrimidin-4-yloxy]-piperidine-1-carboxylic acid tert-butyl ester (Compound B46).**

Compound **B46** was obtained as an off-white powder (13.1 mg, 20%). Exact mass calculated for $C_{20}H_{27}N_5O_3$ 385.2, found LCMS (ESI) m/z 386.1 ($M+H^{+}$,88%).

**Example 10.26: 4-[6-(2-Methoxy-pyridin-3-ylamino)-pyrimidin-4-yloxy]-piperidine-1-carboxylic acid tert-butyl ester (Compound B47).**

Compound **B47** was obtained as a beige powder (14.1 mg, 20%). Exact mass calculated for $C_{20}H_{27}N_5O_4$ 401.2, found LCMS (ESI) m/z 402.1 ($M+H^{+}$,99%).

**Example 10.27: 4-[6-(2,5-Difluoro-phenylamino)-pyrimidin-4-yloxy]-piperidine-1-carboxylic acid tert-butyl ester (Compound B48).**

Compound **B48** was obtained as a light brown powder (16 mg, 20%). Exact mass calculated for $C_{20}H_{24}F_2N_4O_3$ 406.2, found LCMS (ESI) m/z 407.3 ($M+H^{+}$,99%).

**Example 10.28: 4-[6-(4-Chloro-2-fluoro-phenylamino)-pyrimidin-4-yloxy]-piperidine-1-carboxylic acid tert-butyl ester (Compound B49).**

Compound **B49** was obtained as an ivory powder (18.3 mg, 20%). Exact mass calculated for $C_{20}H_{24}ClFN_4O_3$ 422.2, found LCMS (ESI) m/z 423.1 ($M+H^{+}$,99%).

**Example 10.29: 4-[6-(2,5-Difluoro-phenylamino)-pyrimidin-4-yloxy]-piperidine-1-carboxylic acid isopropyl ester (Compound B50).**

Compound **B50** was obtained as a yellow solid (282.4 mg, 18 %). Exact mass calculated for $C_{19}H_{22}F_2N_4O_3$ 392.2, found LCMS (ESI) m/z 393.5 ($M+H^{+}$,98%).

**Example 10.30: 4-[6-(6-Methoxy-pyridin-3-ylamino)-pyrimidin-4-yloxy]-piperidine-1-carboxylic acid isopropyl ester (Compound B51).**

Compound **B51** was obtained as a yellow powder (7.1 mg, 10 %). Exact mass calculated for $C_{19}H_{25}N_5O_4$ 387.2, found LCMS (ESI) m/z 388.2 ($M+H^{+}$, 93%).

**Example 10.31: 4-[6-(4-Cyano-3-methoxy-phenylamino)-pyrimidin-4-yloxy]-piperidine-1-carboxylic acid isopropyl ester (Compound B52).**

Compound **B52** was obtained as a yellow powder (6.3 mg, 7%). Exact mass calculated for $C_{21}H_{25}N_5O_4$ 411.2, found LCMS (ESI) m/z 412.1 ($M+H^{+}$,80%).

**Example 10.32: 4-[6-(3-Fluoro-4-hydroxy-phenylamino)-pyrimidin-4-yloxy]-piperidine-1-carboxylic acid isopropyl ester (Compound B53).**

Compound **B53** was obtained as an off-white powder (1.5 mg, 2%). Exact mass calculated for $C_{19}H_{23}FN_4O_4$ 390.2, found LCMS (ESI) m/z 391.2 ($M+H^{+}$,98%).

**Example 10.33: 4-[6-(6-Ethoxy-pyridin-3-ylamino)-pyrimidin-4-yloxy]-piperidine-1-carboxylic acid isopropyl ester (Compound B54).**

Compound **B54** was obtained as an off-white powder (3.1 mg, 4%). Exact mass calculated for $C_{20}H_{27}N_5O_4$ 401.2, found LCMS (ESI) m/z 402.3 ($M+H^{+}$,96%).

**Example 10.34: 4-[6-(2,5-Difluoro-4-isopropoxy-phenylamino)-pyrimidin-4-yloxy]-piperidine-1-carboxylic acid isopropyl ester (Compound B55).**

Compound **B54** was obtained as a brown powder (18.9 mg, 20%). Exact mass calculated for $C_{22}H_{28}F_2N_4O_4$

450.2, found LCMS (ESI) m/z 451.3 (M+H+,87%).

**Example 10.35: (2-Fluoro-4-methanesulfonyl-phenyl)-[6-(5'-isopropoxy-3,4,5,6-tetrahydro-2H-[1,2']bipyridinyl-4-yloxy)-pyrimidin-4-yl]-amine (Compound B56).**

Compound **B56** was obtained as a solid (18 mg, 25%). Exact mass calculated for $C_{24}H_{28}FN_5O_4S$ 501.2, found 502.3 ($M+H^+$).

**Example 10.36: 4-[6-(2,5-Difluoro-4-propoxy-phenylamino)-pyrimidin-4-yloxy]-piperidine-1-carboxylic acid isopropyl ester (Compound B61).**

**Step 1: Preparation of (2,5-difluoro-4-hydroxy-phenyl)-carbamic acid benzyl ester.**

A mixture of 4-amino-2,5-difluorophenol (2.1 g, 14.5 mmol) and sodium bicarbonate (1.33 g, 15.9 mmol) in 20 mL acetonitrile were cooled in an ice-bath. Benzylcarbonate was added (2.3 ml, 15.9 mmol) and mixture was allowed to warm to room temperature. After 3h, mixture was concentrated and residue was extracted with $CH_2Cl_2$ and 1M HCl solution. The resulting organic phase was extracted with 2M NaOH. The $H_2O$ layer was acidified with concentrated HCl and extracted with $CH_2Cl_2$. The organic phase was dried over $MgSO_4$, filtered, and concentrated to give (2,5-difluoro-4-hydroxy-phenyl)-carbamic acid benzyl ester as a reddish solid (3.84 g, 95%). $^1$HNMR ($CDCl_3$, 400 MHz) δ 5.16 (s, 1H), 5.30 (s, 2H), 6.73-6.79 (m, 2H), 7.33-7.42 (m, 5H), 7.92 (s br, 1H). Exact mass calculated for $C_{14}H_{11}F_2NO_3$ 279.07, found 280.0 ($MH^+$).

**Step 2: Preparation of (2,5-difluoro-4-propoxy-phenyl)-carbamic acid benzyl ester.**

A mixture of (2,5-difluoro-4-hydroxy-phenyl)-carbamic acid benzyl ester (743 mg, 2.66 mmol), potassium carbonate (703 mg, 5.72 mmol), and 1-iodopropane (556 μl, 5.72 mmol) were stirred at 60°C for 16 hours. Mixture was concentrated and residue was extracted with $CH_2Cl_2$ and 1M NaOH solution. Organic phases were dried over $MgSO_4$, filtred and concentrated to give (2,5-difluoro-4-propoxy-phenyl)-carbamic acid benzyl ester as a reddish compound (846 mg, 99%). $^1$HNMR ($CDCl_3$, 400 MHz) δ 1.19-1.23 (t, 3H), 1.95-2.04 (tq, 2H), 4.08-4.11 (t, 2H), 5.38 (s, 2H), 6.87-6.92 (m, 2H), 7.51-7.59 (m, 5H), 8.05 (s br, 1H). Exact mass calculated for $C_{17}H_{17}F_2NO_3$ 321.12, found 322.1 ($MH^+$), 643.7 ($2MH^+$).

**Step 3: Preparation of 2,5-difluoro-4-propoxy-phenylamine.**

To a solution of (2,5-difluoro-4-propoxy-phenyl)-carbamic acid benzyl ester (2.50 g, 7.81 mmol) in 100 mL methanol 10% palladium on carbon (50% water) was added and hydrogen was bubbled through the mixture for 1 min. Mixture was stirred under a hydrogen atmosphere (balloon) at room temperature. After 4 hours, Pd/C was filter off and washed with MeOH. 5 mL 4M HCl in dioxane were added to the filtrate, and it was concentrated to give 2,5-difluoro-4-propoxy-phenylamine as a reddish solid (1.65g, 94%). Exact mass calculated for $C_9H_{11}F_2NO$ 187.08, found 188.2 ($MH^+$).

**Step 4: Preparation of 4-[6-(2,5-Difluoro-4-propoxy-phenylamino)-pyrimidin-4-yloxy]-piperidine-1-carboxylic acid isopropyl ester (Compound B61).**

Compound **B61** was prepared in a similar manner as described herein using 4-(6-chloro-pyrimidin-4-yloxy)-piperidine-1-carboxylic acid isopropyl ester and 2,5-difluoro-4-propoxy-phenylamine to give a tan solid (TFA salt, 280 mg, 30%). $^1$HNMR (MeOD, 400 MHz) δ 0.83-.0.88 (t, 3H), 1.00-.1.04 (d, 6H), 1.45-1.66 (m, 4H), 1.75-1.80 (m, 2H), 8.09-3.20 (m, 2H), 3.51-3.57 (m, 2H), 3.78-3.81 (t, 2H), 4.62-4.70 (m, 1H), 4.95-5.00 (m, 1H), 5.78 (s, 1H), 6.78-6.83 (m, 1H), 7.26-7.31 (m, 1H), 8.06 (s, 1H). Exact mass calculated for $C_{22}H_{28}F_2N_4O_4$ 450.21, found 451.3 ($MH^+$).

**Example 10.37: 4-[6-(2-Methyl-6-propylamino-pyridin-3-ylamino)-pyrimidin-4-yloxy]-piperidine-1-carboxylic acid isopropyl ester (Compound B66).**

Compound **B66** was obtained as a solid (15 mg, 16%). Exact mass calculated for $C_{22}H_{32}N_6O_3$ 428.2, found 429.3 ($M+H^+$).

**Example 10.38: 4-[6-(2-Methyl-pyridin-3-ylamino)-pyrimidin-4-yloxy]-piperidine-1-carboxylic acid isopropyl ester (Compound B67).**

Compound **B67** was obtained as a solid. Exact mass calculated for $C_{19}H_{25}N_5O_3$ 371.2, found 372.2 (M+H+).

**Example 10.39: 4-[6-(6-Isopropylamino-2-methyl-pyridin-3-ylamino)-pyrimidin-4-yloxy]-piperidine-1-carboxylic acid isopropyl ester (Compound B68).**

Compound **B68** was obtained as a solid (7 mg, 10%). Exact mass calculated for $C_{22}H_{32}N_6O_3$ 428.2, found 429.3 (M+H+).

**Example 10.40: 4-[6-(2-Methyl-6-propoxy-pyridin-3-ylamino)-pyrimidin-4-yloxy]-piperidine-1-carboxylic acid isopropyl ester (Compound B69).**

Compound **B69** was obtained as a solid (40 mg, 55%). Exact mass calculated for $C_{21}H_{31}N_5O_4$ 429.2, found 430.2 (M+H+).

**Example 10.41: 4-[6-(2-Fluoro-4-iodo-phenylamino)-pyrimidin-4-yloxy]-piperidine-1-carboxylic acid isopropyl ester (Compound B71).**

Compound **B71** was obtained as a brown powder (216.2 mg, 43%). Exact mass calculated for $C_{19}H_{22}FIN_4O_3$ 500.1, found LCMS (ESI) m/z 501.1 (M+H+,90%).

(Reference) **Example 10.42: 4-{6-Methyl-(2-methyl-4,5,6,7-tetrahydro-2H-indazol-3-yl)-amino]-pyrimidin-4-yloxy}-piperidine-1-carboxylic acid isopropyl ester (Compound B72).**

Compound **B72** was obtained as a white powder (0.5 mg, 1%). Exact mass calculated for $C_{22}H_{32}N_6O_3$ 428.3, found LCMS (ESI) m/z 429.3 (M+H+,58%).

(Reference) **Example 10.43: 4-[6-(2-Methyl-2H-pyrazol-3-ylamino)-pyrimidin-4-yloxy]-piperidine-1-carboxylic acid isopropyl ester (Compound B73).**

Compound **B73** was obtained as an off-white powder (0.4 mg, 1%). Exact mass calculated for $C_{17}H_{24}N_6O_3$ 360.2, found LCMS (ESI) m/z 360.9 (M+H+,100%).

(Reference) **Example 10.44: 4-[6-(2-Phenyl-2H-pyrazol-3-ylamino)-pyrimidin-4-yloxy]-piperidine-1-carboxylic acid isopropyl ester (Compound B74).**

Compound **B74** was obtained as an off-white powder (6.5 mg, 20%). Exact mass calculated for $C_{22}H_{26}N_6O_3$ 422.2, found LCMS (ESI) m/z 423.1 (M+H+,100%).

(Reference) **Example 10.45: 4-[6-(5-tert-Butyl-1H-pyrazol-3-ylamino)-pyrimidin-4-yloxy]-piperidine-1-carboxylic acid isopropyl ester (Compound B75).**

Compound **B75** was obtained as a yellow powder (1.9 mg, 5%). Exact mass calculated for $C_{20}H_{30}N_6O_3$ 402.2, found LCMS (ESI) m/z 403.1 (M+H+,92%).

(Reference) **Example 10.46: 4-[6-(5-p-Tolyl-1H-pyrazol-3-ylamino)-pyrimidin-4-yloxy]-piperidine-1-carboxylic acid isopropyl ester (Compound B76).**

Compound **B76** was obtained as an off-white powder (2.8 mg, 6%). Exact mass calculated for $C_{23}H_{28}N_6O_3$ 436.2, found LCMS (ESI) m/z 437.3 (M+H+,97%).

**Example 10.47: 4-[6-(6-Methoxy-5-methyl-pyridin-3-ylamino)-pyrimidin-4-yloxy]-piperidine-1-carboxylic acid isopropyl ester (Compound B77).**

Compound **B77** was obtained as a white powder (4 mg, 10%). Exact mass calculated for $C_{20}H_{27}N_5O_4$ 401.2, found LCMS (ESI) m/z 402.1 (M+H+,100%).

**Example 10.48: 4-[6-(4-Methyl-pyridin-3-ylamino)-pyrimidin-4-yloxy]-piperidine-1-carboxylic acid isopropyl ester (Compound B78).**

Compound **B78** was obtained as an off-white powder (1.2 mg, 3%). Exact mass calculated for $C_{19}H_{25}N_5O_3$ 371.2, found LCMS (ESI) m/z 372.3 (M+H+,79%).

**Example 10.49: 4-[6-(4-Acetylamino-3-methyl-phenylamino)-pyrimidin-4-yloxy]-piperidine-1-carboxylic acid isopropyl ester (Compound B79).**

Compound **B79** was obtained as an off-white powder (1.2 mg, 3%). Exact mass calculated for $C_{22}H_{29}N_5O_4$ 427.2, found LCMS (ESI) m/z 428.1 (M+H+,98%).

**Example 10.50: 4-[6-(3-Chloro-4-fluoro-phenylamino)-pyrimidin-4-yloxy]-piperidine-1-carboxylic acid isopropyl ester (Compound B80).**

Compound **B80** was obtained as an off-white powder (7.2 mg, 20%). Exact mass calculated for $C_{19}H_{22}ClFN_4O_3$ 408.1, found LCMS (ESI) m/z 409.3 (M+H+,96%).

**Example 10.51: 4-[6-(3,5-Dimethoxy-phenylamino)-pyrimidin-4-yloxy]-piperidine-1-carboxylic acid isopropyl ester (Compound B81).**

Compound **B81** was obtained as a white powder (1.5 mg, 4%). Exact mass calculated for $C_{21}H_{28}N_4O_5$ 416.2, found LCMS (ESI) m/z 417.4 (M+H+,100%).

**Example 10.52: 4-[6-(6-Ethyl-pyridin-2-ylamino)-pyrimidin-4-yloxy]-piperidine-1-carboxylic acid isopropyl ester (Compound B82).**

Compound **B82** was obtained as an off-white powder (3.7 mg, 10%). Exact mass calculated for $C_{20}H_{27}N_5O_3$ 385.2, found LCMS (ESI) m/z 386.1 (M+H+,78%).

**Example 10.53: 4-[6-(5-Methyl-pyridin-2-ylamino)-pyrimidin-4-yloxy]-piperidine-1-carboxylic acid isopropyl ester (Compound B83).**

Compound **B83** was obtained as an off-white powder (4.9 mg, 10%). Exact mass calculated for $C_{19}H_{25}N_5O_3$ 371.2, found LCMS (ESI) m/z 372.3 (M+H+,88%).

**Example 10.54: 4-[6-(2-Methyl-quinolin-6-ylamino)-pyrimidin-4-yloxy]-piperidine-1-carboxylic acid isopropyl ester (Compound B84).**

Compound **B84** was obtained as a yellow powder (2.6 mg, 10%). Exact mass calculated for $C_{23}H_{27}N_5O_3$ 421.2, found LCMS (ESI) m/z 422.1 (M+H+,100%).

**Example 10.55: 4-[6-(2-Methylsulfanyl-benzothiazol-6-ylamino)-pyrimidin-4-yloxy]-piperidine-1-carboxylic acid isopropyl ester (Compound B85).**

Compound **B85** was obtained as a white powder (0.4 mg, 1%). Exact mass calculated for $C_{21}H_{25}N_5O_3S_2$ 459.1, found LCMS (ESI) m/z 460.3 (M+H+,85%).

**Example 10.56: 4-[6-(6-Morpholin-4-yl-pyridin-3-ylamino)-pyrimidin-4-yloxy)-piperidine-1-carboxylic acid isopropyl ester (Compound B86).**

Compound **B86** was obtained as a purple powder (3 mg, 7%). Exact mass calculated for $C_{22}H_{30}N_6O_4$ 442.2, found LCMS (ESI) m/z 443.4 (M+H+,99%).

(Reference) **Example 10.57: 4-[6-(4-Benzenesulfonyl-thiophen-3-ylamino)-pyrimidin-4-yloxy]-piperidine-1-carboxylic acid isopropyl ester (Compound B87).**

Compound **B87** was obtained as an off-white powder (3.9 mg, 8%). Exact mass calculated for $C_{23}H_{26}N_4O_5S_2$

502.1, found LCMS (ESI) m/z 503.3 (M+H+,98%).

**Example 10.58: 4-[6-(4-Piperidin-1-yl-phenylamino)-pyrimidin-4-yloxy]-piperidine-1-carboxylic acid isopropyl ester (Compound B88).**

Compound **B88** was obtained as a purple powder (1 mg, 2%). Exact mass calculated for $C_{24}H_{33}N_5O_3$ 439.3, found LCMS (ESI) m/z 440.4 (M+H+,100%).

**Example 10.59: 4-[6-(3-Trifluoromethoxy-phenylamino)-pyrimidin-4-yloxy]-piperidine-1-carboxylic acid isopropyl ester (Compound B89).**

Compound **B89** was obtained as a beige powder (6.3 mg, 10%). Exact mass calculated for $C_{20}H_{23}F_3N_4O_4$ 440.2, found LCMS (ESI) m/z 441.2 (M+H+,80%).

**Example 10.60: 4-[6-(5-Oxo-5,6,7,8-tetrahydro-naphthalen-2-ylamino)-pyrimidin-4-yloxy]-piperidine-1-carboxylic acid isopropyl ester (Compound B90).**

Compound **B90** was obtained as a yellow powder (0.5 mg, 1 %). Exact mass calculated for $C_{23}H_{28}N_4O_4$ 424.2, found LCMS (ESI) m/z 425.1 (M+H+,89%).

(Reference) **Example 10.61: 4-[6-(6-Methyl-1H-pyrazolo[3,4-b]pyridin-3-ylamino)-pyrimidin-4-yloxy]-piperidine-1-carboxylic acid isopropyl ester (Compound B91).**

Compound **B91** was obtained as a yellow powder (0.5 mg, 1%). Exact mass calculated for $C_{20}H_{25}N_7O_3$ 411.2, found LCMS (ESI) m/z 412.3 (M+H+,78%).

**Example 10.62: 4-[6-(5-Cyano-pyridin-2-ylamino)-pyrimidin-4-yloxy]-piperidine-1-carboxylic acid isopropyl ester (Compound B92).**

Compound **B92** was obtained as a white powder (1.3 mg, 3%). Exact mass calculated for $C_{19}H_{22}N_6O_3$ 382.2, found LCMS (ESI) m/z 383.3 (M+H+,88%).

**Example 10.63: 4-[6-(4-Bromo-2,5-difluoro-phenylamino)-pyrimidin-4-yloxy]-piperidine-1-carboxylic acid isopropyl ester (Compound B93).**

Compound **B93** was obtained as a tan solid (TFA salt, 286.5 mg, 29%). $^1$HNMR ($CDCl_3$, 400 MHz) δ 1.22-125 (d, 7H), 1.66-1.76 (m, 2H), 1.92-2.01 (m, 2H), 3.27-3.35 (m, 2H), 3.74-3.83 (m, 2H), 4.88-4.95 (m, 1H), 5.25-5.32 (m, 1H), 6.0 (s, 1H), 7.3-7.35 (m, 1H), 7.955-8.01 (m, 1H), 8.4 (s, 1H). Exact mass calculated for $C_{19}H_{21}BrF_2N_4O_3$ 470.08, found 471.0 (MH+).

**Example 10.64: 4-[6-(4-Trifluoromethyl-pyridin-2-ylamino)-pyrimidin-4-yloxy]-piperidine-1-carboxylic acid isopropyl ester (Compound B94).**

Compound **B94** was obtained as a white powder (2.3 mg, 5%). Exact mass calculated for $C_{19}H_{22}F_3N_5O_3$ 425.2, found LCMS (ESI) m/z 426 (M+H+,75%).

(Reference) **Example 10.65: 4-[6-(5-Methyl-1H-pyrazol-3-ylamino)-pyrimidin-4-yloxy]-piperidine-1-carboxylic acid isopropyl ester (Compound B95).**

Compound **B95** was obtained as a white powder (0.4mg, 1%). Exact mass calculated for $C_{17}H_{24}N_6O_3$ 360.2, found LCMS (ESI) m/z 360.9 (M+H+,84%).

(Reference) **Example 10.66: 4-[6-(5-Cyclopropyl-1H-pyrazol-3-ylamino)-pyrimidin-4-yloxy]-piperidine-1-carboxylic acid isopropyl ester (Compound B96).**

Compound **B96** was obtained as a brown powder (1.7 mg, 4%). Exact mass calculated for $C_{19}H_{26}N_6O_3$ 386.2, found LCMS (ESI) m/z 387.3 (M+H+,66%).

**Example 10.67: 4-[6-(2,6-Dimethyl-pyridin-3-ylamino)-pyrimidin-4-yloxy]-piperidine-1-carboxylic acid isopropyl ester (Compound B97).**

Compound **B97** was obtained as a solid (28 mg, 36%). Exact mass calculated for $C_{20}H_{27}N_5O_3$ 385.2, found 386.3 ($M+H^+$).

**Example 10.68: 4-[6-(4-Cyano-2-methyl-phenylamino)-pyrimidin-4-yloxy]-piperidine-1-carboxylic acid isopropyl ester (Compound B98).**

Compound **B98** was obtained as a solid (20 mg, 25%). Exact mass calculated for $C_{21}H_{25}N_5O_3$ 395.2, found 396.1 ($M+H^+$).

**Example 10.69: 4-[6-(4-Methoxy-2-methyl-phenylamino)-pyrimidin-4-yloxy]-piperidine-1-carboxylic acid isopropyl ester (Compound B99).**

Compound **B99** was obtained as a solid (24 mg, 30%). Exact mass calculated for $C_{21}H_{28}N_4O_4$ 400.2, found 401.4 ($M+H^+$).

**Example 10.70: 4-[6-(2,4-Dimethoxy-phenylamino)-pyrimidin-4-yloxy]-piperidine-1-carboxylic acid isopropyl ester (Compound B100).**

Compound **B100** was obtained as a solid (20 mg, 24%). Exact mass calculated for $C_{27}H_{28}N_4O_5$ 416.2, found 417.3 ($M+H^+$).

**Example 10.71: 4-[6-(5-Carbamoyl-pyridin-2-ylamino)-pyrimidin-4-yloxy]-piperidine-1-carboxylic acid isopropyl ester (Compound B102).**

Compound **B102** was obtained as an off-white powder (2.9 mg, 5%). Exact mass calculated for $C_{19}H_{24}N_6O_4$ 400.2, found LCMS (ESI) m/z 401.3 ($M+H^+$, 68%).

(Reference) **Example 10.72: 4-{6-[4-(3,4-Difluoro-phenyl)-thiazol-2-ylamino]-pyrimidin-4-yloxy}-piperidine-1-carboxylic acid isopropyl ester (Compound B103).**

Compound **B103** was obtained as a pale yellow powder (4.9 mg, 7%). Exact mass calculated for $C_{22}H_{23}F_5N_5O_3S$ 475.2, found LCMS (ESI) m/z 476.2 ($M+H^+$, 66%).

(Reference) **Example 10.73: 4-[6-(5-Oxo-1-phenyl-4,5-dihydro-1H-pyrazol-3-ylamino)-pyrimidin-4-yloxy]-piperidine-1-carboxylic acid isopropyl ester (Compound B104).**

Compound **B104** was obtained as a pale yellow powder (0.5 mg, 0.8%). Exact mass calculated for $C_{22}H_{26}N_6O_4$ 438.2, found LCMS (ESI) m/z 439.4 ($M+H^+$, 98%).

**Example 10.74: 4-[6-(3-Oxazol-5-yl-phenylamino)-pyrimidin-4-yloxy]-piperidine-1-carboxylic acid isopropyl ester (Compound B105).**

Compound **B105** was obtained as a pale yellow powder (5.4 mg, 9%). Exact mass calculated for $C_{22}H_{25}N_5O_4$ 423.2, found LCMS (ESI) m/z 424.3 ($M+H^+$, 100%).

**Example 10.75: 4-[6-(5-Trifluoromethyl-pyridin-2-ylamino)-pyrimidin-4-yloxy]-piperidine-1-carboxylic acid isopropyl ester (Compound B106).**

Compound **B106** was obtained as an off-white powder (4.8 mg, 7%). Exact mass calculated for $C_{19}H_{22}F_3N_5O_3$ 425.2, found LCMS (ESI) m/z 426.4 ($M+H^+$, 44%).

**Example 10.76: 4-[6-(4-Chloro-2-trifluoromethoxy-phenylamino)-pyrimidin-4-yloxy]-piperidine-1-carboxylic acid isopropyl ester (Compound B107).**

Compound **B107** was obtained as a brown powder (32.6 mg, 46%). Exact mass calculated for $C_{20}H_{22}ClF_3N_4O_4$

474.1, found LCMS (ESI) m/z 475.3 (M+H+,93%).

(Reference) **Example 10.77: 4-{6-[(5-Pyridin-2-yl-thiophen-2-ylmethyl)-amino]-pyrimidin-4-yloxy}-piperidine-1-carboxylic acid isopropyl ester (Compound B108).**

Compound **B108** was obtained as an off-white powder (1.6 mg, 2%). Exact mass calculated for $C_{23}H_{27}N_5O_3S$ 453.2, found LCMS (ESI) m/z 451.4 (M+H+,71%).

(Reference) **Example 10.78: 4-{6-[5-(4-Chloro-phenyl)-2H-pyrazol-3-ylamino]-pyrimidin-4-yloxy}-piperidine-1-carboxylic acid isopropyl ester (Compound B109).**

Compound **B109** was obtained as a brown powder (1.1 mg, 2%). Exact mass calculated for $C_{22}H_{25}ClN_6O_3$ 456.2, found LCMS (ESI) m/z 457.1 (M+H+,77%).

**Example 10.79: 4-[6-(1-Oxo-indan-5-ylamino)-pyrimidin-4-yloxy]-piperidine-1-carboxylic acid isopropyl ester (Compound B110).**

Compound **B110** was obtained as a brown powder (3.6 mg, 6%). Exact mass calculated for $C_{22}H_{26}N_4O_4$ 410.2, found LCMS (ESI) m/z 411.0 (M+H+, 42%).

**Example 10.80: 4-{6-[5-(1-Methyl-pyrrolidin-2-yl)-pyridin-2-ylamino]-pyrimidin-4-yloxy}-piperidine-1-carboxylic acid isopropyl ester (Compound B111).**

Compound **B111** was obtained as an off-white powder (0.6 mg, 0.9%). Exact mass calculated for $C_{23}H_{32}N_6O_3$.440.3, found LCMS (ESI) m/z 440.4 (M+H+, 100%).

**Example 10.81: 4-[6-(6-Methoxy-2-methyl-pyridin-3-ylamino)-pyrimidin-4-yloxy]-piperidine-1-carboxylic acid isopropyl ester (Compound B112).**

Compound **B112** was obtained as a yellow powder (3.4 mg, 6%). Exact mass calculated for $C_{20}H_{27}N_5O_4$ 401.2, found LCMS (ESI) m/z 402.1 (M+H+,99%).

**Example 10.82: 4-[6-(5-Bromo-3-methyl-pyridin-2-ylamino)-pyrimidin-4-yloxy]-piperidine-1-carboxylic acid isopropyl ester (Compound B113).**

Compound **B113** was obtained as a white powder (6.6 mg, 10%). Exact mass calculated for $C_{19}H_{24}BrN_5O_3$ 449.1, found LCMS (ESI) m/z 452.2 (M+H+,94%).

**Example 10.83: 4-[6-(2-Chloro-6-methyl-pyridin-3-ylamino)-pyrimidin-4-yloxy]-piperidine-1-carboxylic acid isopropyl ester (Compound B114).**

Compound **B114** was obtained as a beige powder (7.9 mg, 13%). Exact mass calculated for $C_{19}H_{24}ClN_5O_3$ 405.2, found LCMS (ESI) m/z 406.2 (M+H+,98%).

**Example 10.84: 4-[6-(2-Ethynyl-phenylamino)-pyrimidin-4-yloxy]-piperidine-1-carboxylic acid isopropyl ester (Compound B115).**

Compound **B115** was obtained as a brown powder (2.3 mg, 4%). Exact mass calculated for $C_{21}H_{24}N_4O_3$ 380.2, found LCMS (ESI) m/z 381.2 (M+H+,56%).

**Example 10.85: 4-[6-(4-Bromo-2-trifluoromethoxy-phenylamino)-pyrimidin-4-yloxy]-piperidine-1-carboxylic acid isopropyl ester (Compound B116).**

Compound **B116** was obtained as a beige powder (16.6 mg, 21%). Exact mass calculated for $C_{20}H_{22}BrF_3N_4O_4$. 518.1, found LCMS (ESI) m/z 519.2 (M+H+,91%).

**Example 10.86: 4-[6-(3-Iodo-4-methyl-phenylamino)-pyrimidin-4-yloxy]-piperidine-1-carboxylic acid isopropyl ester (Compound B117).**

Compound **B117** was obtained as an off-white powder (0.7 mg, 0.8%). Exact mass calculated for $C_{20}H_{25}IN_4O_3$ 496.1, found LCMS (ESI) m/z 497.3 ($M+H^+$,98%).

**Example 10.87: 4-[6-(2-Fluoro-5-methyl-phenylamino)-pyrimidin-4-yloxy]-piperidine-1-carboxylic acid isopropyl ester (Compound B118).**

Compound **B118** was obtained as an off-white powder (10.6 mg, 18%). Exact mass calculated for $C_{20}H_{25}FN_4O_3$ 388.2, found LCMS (ESI) m/z 389.4 ($M+H^{+,}$ 95%).

(Reference) **Example 10.88: 4-{6-[5-(4-Methoxy-phenyl)-[1,3,4]thiadiazol-2-ylamino]-pyrimidin-4-yloxy}-piperidine-1-carboxylic acid isopropyl ester (Compound B119).**

Compound **B119** was obtained as an off-white powder (2.5 mg, 4%). Exact mass calculated for $C_{22}H_{26}N_6O_4S$. 470.2, found LCMS (ESI) m/z 473.3 ($M+H^{+,}$ 52%).

(Reference) **Example 10.89: 4-[6-(3,5-Dimethyl-isoxazol-4-ylamino)-pyrimidin-4-yloxy]-piperidine-1-carboxylic acid isopropyl ester (Compound B120).**

Compound **B120** was obtained as a white powder (3.1 mg, 6%). Exact mass calculated for $C_{18}H_{25}N_5O_4$.375.2, found LCMS (ESI) m/z 376.1 ($M+H^{+,}$ 100%).

(Reference) **Example 10.90: 4-[2-(2,5-Difluoro-4-propoxy-phenylamino)-pyridin-4-yloxy]-piperidine-1-carboxylic acid isopropyl ester (Compound B121).**

Compound **B121** was obtained as a solid. $^1H$ NMR ($CDCl_3$, 400 MHz) δ 8.02 (d, 1H), 7.72 (m, 1H), 6.76 (m, 1H), 6.34 (m, 1H), 6.28 (bs, 1H), 4.90 (m, 1H), 4.50 (m, 1H), 3.93 (t, 2H), 3.66 (m, 2H), 3.39 (m, 2H), 1.87 (m, 2H), 1.80 (m, 2H), 1.75 (m, 2H), 1.43 (d, 6H), 1.02(t, 3H). LRMS calculated for $C_{23}H_{29}F_2N_3O_4$: 449.21. Found: 450.5 $(M+H)^+$.

**Example 10.91: (2,5-Difluoro-4-propoxy-phenyl)-{6-[1-(5-isopropyl-[1,2,4]oxadiazol-3-yl)-piperidin-4-yloxy]-pyrimidin-4-yl}-amine (Compound B133).**

Compound **B133** was obtained as a tan solid (HCl salt, 65.9 mg, 18%). $^1$HNMR (MeOD, 400 MHz) δ 0.84-0.88 (t, 3H), 1.05-1.07 (d, 6H), 1.60-1.76 (m, 4H), 1.92-2.01 (m, 2H), 2.63-2.68 (m, 1H), 3.42-3.47 (m, 2H), 3.61-3.65 (m, 2H), 3.83-3.86 (t, 2H), 5.03-5.08 (m, 1H), 6.08 (s, 1H), 6.92-6.97 (m, 1H), 7.16-7.21 (m, 1H), 8.32 (s, 1H). Exact mass calculated for $C_{23}H_{28}F_2N_6O_3$ 474.22, found 475.4 ($MH^+$).

**Example 10.92: 4-[6-(2,5-Difluoro-4-propylamino-phenylamino)-pyrimidin-4-yloxy]-piperidine-1-carboxylic acid isopropyl ester (Compound B122).**

A mixture of 4-[6-(4-bromo-2,5-difluoro-phenylamino)-pyrimidin-4-yloxy]-piperidine-1-carboxylic acid isopropyl ester (TFA salt, 51.2 mg, 0.087 mmol), copper iodide (17.6 mg, 0.092 mmol), potassium carbonate (37.3 mg, 0.269 mmol), propyl amine (57.4 μl, 0.698 mmol), and L-proline (21.2 mg, 0.184 mmol) in 1.5 mL DMSO was heated in microwave for 9 hours at 80 °C. The mixture was purified by HPLC to give compound **B122** as tan solid (TFA salt, 13 mg, 26%). $^1$HNMR (MeOD-$d_4$, 400 MHz) δ 0.85-0.91 (m, 3H), 1.09-1.14 (d, 6H), 1.51-1.60 (m, 2H), 1.61-1.71 (m, 2H), 1.85-1.95 (m, 2H), 3.02-3.08 (m, 2H), 3.25-3.35 (m, 2H), 3.55-3.65 (m, 2H), 4.70-4.80 (m, 1H), 5.04-5.12 (m, 1H), 6.14 (s, 1H), 6.63-6.71 (m, 1H), 7.16-7.24 (m, 1H), 8.36 (s, 1H). Exact mass calculated for $C_{22}H_{29}F_2N_5O_3$ 449.22, found 450.3 ($MH^+$).

**Example 10.93: 4-[6-(2,5-Difluoro-4-morpholin-4-yl-phenylamino)-pyrimidin-4-yloxy]-piperidine-1-carboxylic acid isopropyl ester (Compound B123).**

Compound **B123** was obtained as tan solid (TFA salt, 9.8 mg, 12%). $^1$HNMR (MeOD-$d_4$, 400 MHz) δ 1.00-1.05 (d, 6H), 1.53-1.63 (m, 2H), 1.76-1.87 (m, 2H), 2.88-2.94 (m, 4H), 3.17-3.27 (m, 2H), 3.46-3.55 (m, 2H), 3.59-3.65 (m, 4H), 4.6-4.7 (m, 1H), 4.93-5.00 (m, 1H), 6.1 (s, 1H), 6.75-6.83 (m, 1H), 7.15-7.23 (m, 1H), 8.30 (s, 1H). Exact mass calculated for $C_{23}H_{29}F_2N_3O_4$ 477.22, found 478.4 ($MH^+$).

**Example 10.94: 4-(6-{2,5-Difluoro-4-[(tetrahydro-furan-2-ylmethyl)-amino]-phenylamino}-pyrimidin-4-yloxy)-piperidine-1-carboxylic acid isopropyl ester (Compound B136).**

Compound **B136** was obtained as a white solid (HCl salt, 48.8 mg, 22%). $^1$HNMR (MeOD, 400 MHz) δ 1.05-1.10 (d, 6H), 1.49-1.70 (m, 3H), 1.78-1.98 (m, 5H), 3.02-.3.11 (m, 1H), 3.22-3.31 (m, 2H), 3.54-3.69 (m, 4H), 3.71-3.78 (m, 1H), 3.92-3.98 (m, 1H), 4.64-4.70 (s, 1H), 5.03-5.11 (m, 1H), 6.07 (s, 1H), 6.64-6.69 (m, 1H), 7.04-7.09 (m, 1H), 8.32 (s, 1H). Exact mass calculated for $C_{24}H_{31}F_2N_5O_4$ 491.23, found 492.4 ($MH^+$).

**Example 10.95: 4-{6-[2,5-Difluoro-4-(2-methoxy-ethylamino)-phenylamino]-pyrimidin-4-yloxy}-piperidine-1-carboxylic acid isopropyl ester (Compound B135).**

Compound **B135** was obtained as a white solid (HCl salt, 44.5 mg, 21%). $^1$HNMR (MeOD, 400 MHz) δ 1.14-1.16 (d, 6H), 1.63-1.72 (m, 2H), 1.88-1.98 (m, 2H), 3.26-3.38 (m, 7H), 3.47-3.51 (m, 2H), 3.60-3.68 (m, 2H), 4.64-4.70 (s, 1H), 5.10-5.06 (m, 1H), 6.13 (s, 1H), 6.68-6.73 (m, 1H), 7.13-7.17 (m, 1H), 8.44 (s, 1H). Exact mass calculated for $C_{22}H_{29}F_2N_5O_4$ 465.22, found 466.4 ($MH^+$).

**Example 10.96: 4-[6-(4-Butylamino-2,5-difluoro-phenylamino)-pyrimidin-4-yloxy]-piperidine-1-carboxylic acid isopropyl ester (Compound B137).**

Compound **B137** was obtained as a white solid (HCl salt, 70.1 mg, 33%). $^1$HNMR (MeOD, 400 MHz) δ 1.10-1.14 (t, 3H), 1.38-1.40 (d, 6H), 1.54-1.62 (m, 2H), 1.75-1.83 (m, 2H), 1.91-1.95 (m, 2H), 2.14-2.18 (m, 2H), 3.34-3.37 (m, 2H), 3.5-3.61 (m, 2H), 3.85-3.91 (m, 2H), 4.64-4.70 (s, 1H), 5.32-5.38 (m, 1H), 6.39 (s, 1H), 6.90-6.95 (m, 1H), 7.43-7.48 (s, 1H), 8.62 (s, 1H). Exact mass calculated for $C_{23}H_{31}F_2N_5O_4$ 463.24, found 464.5 ($MH^+$).

**Example 10.97: 4-{6-[2,5-Difluoro-4-(3-methyl-butylamino)-phenylamino]-pyrimidin-4-yloxy}-piperidine-1-carboxylic acid isopropyl ester (Compound B138).**

Compound **B138** was obtained as a white solid (HCl salt, 100 mg, 45%). $^1$HNMR (MeOD, 400 MHz) δ 0.77-0.78 (d, 6H), 1.04-.106 (d, 6H), 1.34-1.40 (m, 2H), 1.48-1.81 (m, 3H), 1.79-1.88 (m, 2H), 3.02-3.10 (m, 2H), 3.18-3.25 (m, 2H), 3.50-3.57 (m, 2H), 4.64-4.70 (m, 1H), 4.95-5.02 (m, 1H), 6.08 (s, 1H), 6.61-6.66 (m, 1H), 7.14-7.18 (m, 1H), 8.29 (s, 1H). Exact mass calculated for $C_{24}H_{33}F_2N_5O_3$ 477.26, found 478.5 ($MH^+$).

**Example 10.98: 4-{6-[2,5-Difluoro-4-(tetrahydro-furan-2-ylmethoxy)-phenylamino]-pyrimidin-4-yloxy}-piperidine-1-carboxylic acid isopropyl ester (Compound B145).**

Compound **B145** was obtained as a solid (4 mg, 4%). Exact mass calculated for $C_{24}H_{30}F_2N_4O_5$ 492.2, found 493.6 ($MH^+$).

**The following compounds were prepared using the general method as described in Example 10.1.**

**Example 10.99: 4-[6-(3-Fluoro-4-methyl-phenylamino)-pyrimidin-4-yloxy]-piperidine-1-carboxylic acid tert-butyl ester (Compound B16).**

Compound **B16** was obtained as yellow solid (16mg, 27 %). Exact mass calculated for $C_{21}H_{27}FN_4O_3$ 402.2, found LCMS (ESI) m/z 403.2($M+H^+$, 100%).

**Example 10.100: 4-[6-(3-Hydroxy-4-methoxy-phenylamino)-pyrimidin-4-yloxy]-piperidine-1-carboxylic acid tert-butyl ester (Compound B17).**

Compound **B17** was obtained as yellow solid (11mg, 18 %). Exact mass calculated for $C_{21}H_{28}N_4O_5$ 416.2, found LCMS (ESI) m/z 417.1($M+H^+$, 100%).

**Example 10.101: 4-[6-(6-Cyano-pyridin-3-ylamino)-pyrimidin-4-yloxy]-piperidine-1-carboxylic acid tert-butyl ester (Compound B18).**

Compound **B18** was obtained as yellow solid (7mg, 12 %). Exact mass calculated for $C_{20}H_{24}N_6O_3$ 396.2, found LCMS (ESI) m/z 397.1($M+H^+$, 100%).

**Example 10.102: 4-[6-(3-Chloro-4-cyano-phenylamino)-pyrimidin-4-yloxy]-piperidine-1-carboxylic acid tert-butyl ester (Compound B19).**

Compound **B19** was obtained as yellow solid (19mg, 30 %). Exact mass calculated for $C_{21}H_{24}$ $ClN_5O_3$ 429.2, found LCMS (ESI) m/z 430.2($M+H^+$, 100%).

**Example 10.103: 4-[6-(3-Fluoro-4-methoxy-phenylamino)-pyrimidin-4-yloxy]-piperidine-1-carboxylic acid tert-butyl ester (Compound B21).**

Compound **B21** was obtained as brown solid (12mg, 19 %). Exact mass calculated for $C_{21}$ $H_{27}$ $FN_4O_4$ 418.2, found LCMS (ESI) m/z 419.4($M+H^+$, 100%).

**Example 10.104: 4-[6-(3,4-Dimethoxy-phenylamino)-pyrimidin-4-yloxy]-piperidine-1-carboxylic acid tert-butyl ester (Compound B22).**

Compound **B22** was obtained as brown solid (9mg, 14 %). Exact mass calculated for $C_{22}H_{30}$ $N_4O_5$ 430.2, found LCMS (ESI) m/z 431.3($M+H^+$, 100%).

**Example 10.105: 4-[6-(2,3-Dihydro-benzo[1,4]dioxin-6-ylamino)-pyrimidin-4-yloxy]-piperidine-1-carboxylic acid tert-butyl ester (Compound B23).**

Compound **B23** was obtained as brown solid (7mg, 11 %). Exact mass calculated for $C_{22}H_{28}$ $N_4O_5$ 428.2, found LCMS (ESI) m/z 429.3($M+H^+$, 100%).

**Example 10.106: 4-[6-(4-Cyano-2,5-difluoro-phenylamino)-pyrimidin-4-yloxy]-piperidine-1-carboxylic acid isopropyl ester (Compound B24).**

2,5-Difluoro-4-[6-(piperidin-4-yloxy)-pyrimidin-4-ylamino]-benzonitrile (1.793g, 4.6mmol) and TEA (18.4 mmole, 4eq) were dissolved in THF (10mL) and then isopropyl chloroformate (5.98mmole, 1.3eq) was added into the solution. The reaction mixture was stirred at room temp for 2 hours. The crude was purified through flash column (Hexane: Ethyl Acetate=1:1) provided Compound **B24** as a white solid( 400 mg, 21 %). $^1H$ NMR 400MHz $CDCl_3$ δ(ppm): 9.86 (s, NH); 8.65 (dd, 1H); 8.55 (s,1H); 7.97 (dd,1H); 6.55 (s,1H); 5.21 (m, 1H); 4.78 (sep,1H); 3.75-3.70 (m,2H); 3.22-3.20 (m, 2H); 1.99-1.94 (m,2H); 1.59-1.55 (m,2H); 1.19 (d, 6H). Exact mass calculated for $C_{20}H_{21}$ $F_2N_5O_3$ 417.2, found LCMS (ESI) m/z 418.2($M+H^+$, 100%).

**Example 10.107: 4-[6-(4-Ethoxy-2,5-difluoro-phenylamino)-pyrimidin-4-yloxy]-piperidine-1-carboxylic acid isopropyl ester (Compound B26).**

Compound **B26** was obtained as tan solid (17mg, 20 %). Exact mass calculated for $C_{21}H_{26}F_2N_4O_4$ 436.2, found LCMS (ESI) m/z 437.3($M+H^+$, 100%).

**Example 10.108: (2-Fluoro-4-methanesulfonyl-phenyl)-{6-[1-(3-isopropyl-[1,2,4]oxadiazol-5-yl)-piperidin-4-yloxy]-pyrimidin-4-yl}-amine (Compound B57).**

**Step 1: Preparation of isopropyl amidoxime.**

A solution of isobutyronitrile (276 g, 4.0 mol) in EtOH (2.0 L) was combined with hydroxylamine (50% aqueous solution, 1.1 L, 16 mol), and refluxed for 5 h. The solvent was then removed in vacuo, and the residual water was azeotropically removed with toluene. The residue was then taken up in $CH_2Cl_2$, dried over $MgSO_4$, and the solvent was removed to afford a white solid (402 g, 98% yield). $^1H$ NMR ($CDCl_3$) δ 7.94 (br s, 1 H), 4.55 (br s, 2 H), 2.47 (m, 1 H), 1.20 (d, 6 H, *J*= 7.1 Hz).

**Step 2: Preparation of 1-cyano-4-hydroxypiperidine.**

A 5-liter, 3-neck flask was equipped with mechanical stirring, a reflux condenser, and a powder addition funnel. Sodium bicarbonate (840 g, 10 mmol) was added via the powder funnel while stirring, then water (ca. 300-400 mL) was gradually added while vigorously stirring to form a thick, uniform slurry. The flask was then placed in an ice bath, and a solution of 4-hydroxypiperidine (506 g, 5.00 mol) in $CH_2Cl_2$ (1.0 L) was added, and the contents were vigorously mixed while cooling. A solution of cyanogen bromide (640 g, 6.0 mol) in $CH_2Cl_2$ (600 mL) was added in a dropwise fashion over 2 h, and stirring was continued for an additional 30 min. The ice bath was removed, and the mechanical stirrer was

replaced by a magnetic stirrer, and the reaction mixture was stirred for 16 h. The flask was once again placed under mechanical stirring, and sodium carbonate (100 g) was added in order to ensure complete neutralization. $MgSO_4$ (500 g) was added, and vigorous stirring was continued for 15 min. The resulting suspension was filtered, rinsing with $CH_2Cl_2$ (2.0 L). A light amber, viscous oil was obtained upon solvent removal (574 g, 91 % yield). $^1H$ NMR ($CDCl_3$) δ 3.80 (m, 1 H), 3.39 (m, 2 H), 3.05 (m, 2 H), 1.87 (m, 2 H), 1.70 (br s, 1 H), 1.62 (m, 2 H); MS *mlz* 212.1 ($M^+$).

**Step 3: 4-Hydroxy-1-(3-isopropyl-1,2,4-oxadiazol-5-yl)piperidine**

In a variation of the method described by Yarovenko et al. in *Bull. Acad. Sci. USSR, Div. Chem. Sci.* **1991**, *40*, 1924; $ZnCl_2$ (1 N in ether, 120 mL, 120 mmol) was added in a dropwise fashion over 15 min to a magnetically stirred solution of **step 1** (12.2 g, 120 mmol) and **step 2** (12.6 g, 100 mmol) in ethyl acetate (500 mL). Precipitate formed immediately upon addition, and at a point the stirring bar became immobilized in the matrix, requiring the reaction to be manually shaken for the remainder of addition. After standing for 15 min, the supernatant was decanted and filtered, and the residue was rinsed twice with ether, furnishing a hard white precipitate which was collected by filtration. The precipitate was identified via LC/MS as the intermediate O-amidinoamidoxime (m/z 229). This material was taken up in conc. HCl (50 mL), diluted to 4 N with EtOH (100 mL), and refluxed for 1 h. Upon cooling, a white precipitate was removed by filtration, then the filtrate was reduced to 50 mL and diluted with 100 mL water. Solid $Na_2CO_3$ was added until the mixture was basic, $CH_2Cl_2$ was added, and the resulting mixture was filtered, rinsing with $CH_2Cl_2$. The organic extract was separated, dried over $MgSO_4$, and the solvent was removed to afford a viscous, amber oil (15.0 g, 71% yield): $^1H$NMR ($CDCl_3$) δ 3.95 (m, 3 H), 3.37 (m, 2 H), 2.88 (m, 1 H), 2.34 (br s, 1 H), 1.93 (m, 2 H), 1.63 (m, 2 H), 1.28 (d, 6 H, *J*= 7.1 Hz); MS *m*/*z* 212.3 ($M^+$).

**Step 4: Preparation of (2-Fluoro-4-methanesulfonyl-phenyl)-{6-[1-(3-isopropyl-[1,2,4]oxadiazol-5-yl)-piperidin-4-yloxy]-pyrimidin-4-yl}-amine (Compound B57).**

Compound **B57** was prepared in a similar manner as described above as a brown oil (230mg, 20 %). $^1H$ NMR 400MHz $CDCl_3$ δ (ppm): 9.64(s, NH); 8.49-8.45(m, 2H); 7.80(d,1 H); 7.71 (d,1H); 6.41(s,1H); 5.25(m,1H); 3.82-3.78(m, 2H); 3.50-3.44(m,2H); 3.23 (s, 3H); 2.07-2.04(m, 2H); 1.75-1.73(m,2H); 1.18(d, 6H). Exact mass calculated for $C_{21}H_{25}FN_6O_4S$ 476.2, found LCMS (ESI) m/z 477.2($M+H^+$, 100%).

**Example 10.109: 4-[6-(4-Cyano-2-fluoro-phenylamino)-pyrimidin-4-yloxy]-piperidine-1-carboxylic acid isopropyl ester (Compound B58).**

Compound **B58** was obtained as yellow solid (34mg, 57 %). $^1H$ NMR 400MHz $CDCl_3$ δ(ppm): 8.46(s, 1H); 7.62-7.53(m, 3H); 5.96(s,1H); 5.38(m,1H); 4.92(sep,1H); 3.81-3.78(db,2H); 3.35-3.29(m,2H); 1.97(sb,2H); 1.73 (mb, 2H); 1.26(d, 6H). Exact mass calculated for $C_{20}H_{22}FN_5O_3$ 399.2, found LCMS (ESI) m/z 400.2($M+H^+$, 100%).

**Example 10.110: 4-[6-(Pyridin-3-ylamino)-pyrimidin-4-yloxy]-piperidine-1-carboxylic acid isopropyl ester (Compound B59).**

Compound **B59** was obtained as yellow solid (34mg, 46 %). $^1H$NMR 400MHz $CDCl_3$ δ (ppm): 9.22(s,1H); 8.79 (d,1H); 8.54(s, 1H); 8.49(d, 1H); 7.86-7.83(m,1H); 6.41(s,1H); 5.30(m,1H); 4.93(sep,1H 3.81-3.76(m,2H); 3.41-3.34(m, 2H); 2.02-1.98(sb,2H); 1.80-1.76 (sb, 2H); 1.26(d, 6H). Exact mass calculated for $C_{18}H_{23}N_5O_3$ 357.2, found LCMS (ESI) m/z 358.2($M+H^+$, 100%).

**Example 10.111: 4-[6-(Pyridin-4-ylamino)-pyrimidin-4-yloxy]-piperidine-1-carboxylic acid isopropyl ester (Compound B60).**

Compound **B60** was obtained as yellow solid (21mg, 30 %). $^1H$NMR 400MHz $CDCl_3$ (ppm): 8.56(s, 1H); 8.36 (d, 2H); 8.27(d,2H); 6.57(s,1H); 5.30(m,1H); 4.93(sep,1H); 3.81-3.77(m,2H); 3.38-3.32(m,2H); 1.97(sb,2H); 1.77-1.73 (sb, 2H); 1.25(d, 6H). Exact mass calculated for $C_{18}H_{23}N_5O_3$ 357.2, found LCMS (ESI) m/z 358.2($M+H^+$, 100%).

**Example 10.112: 4-[6-(2,5-Difluoro-phenoxy)-pyrimidin-4-yloxy]-piperidine-1-carboxylic acid isopropyl ester (Compound B142).**

Compound **B142** was obtained as a solid. $^1H$ NMR 400MHz $CDCl_3$ δ(ppm): 8.49(s,1H); 7.20-7.15 (m, 1H); 7.01-6.96(m, 2H); 6.22(s,1H); 5.33-5.31 (m,1H); 4.93(sep,1H); 3.80-3.74(m, 2H); 3.44-3.38(m, 2H); 2.03-1.98(m,2H); 1.82-1.77(m,2H); 1.27(d,6H). Exact mass calculated: $C_{19}H_{21}F_2N_3O_4$ 393.2, found LCMS (ESI) m/z 394.2($M+H^+$, 100%)

**(Reference) Example 10.113: Preparation of 4-[6-(2-fluoro-4-methanesulfonyl-phenylamino)-pyridin-2-yloxy]-piperidine-1-carboxylic acid tert-butyl ester (Compound B146).**

A suspension of 4-(6-bromo-pyridin-2-yloxy)-piperidine-1-carboxylic acid tert-butyl ester (2.93 g, 8.23 mmol) and 2-fluoro-4-methanesulfonyl-phenylamine (1.87 g, 9.87 mmol) in anhydrous toluene (82 mL) was degassed by bubbling nitrogen gas through the suspension for 15 min. Tris(dibenzylideneacetone)dipalladium(0) ($Pd_2dba_3$) (754 mg, 0.82 mmol), 1,3-bis(diphenylphosphino)propane (dppp) (678 mg, 1.65 mmol), and NaOtBu (1.11 g, 11.5 mmol) were added, the reaction vessel was purged with nitrogen gas, and the reaction mixture was heated at 70C under a nitrogen atmosphere for 9h. The reaction mixture was diluted with ether (83 mL), washed with brine 3 times (3 x 83 mL), then the solids in the organic and aqueous layers were filtered and washed with ether 3 times. The organic extracts and washes were combined, dried with $MgSO_4$, and the solvent was evaporated in vacuo to give a solid which was purified by flash chromatography using hexanes-EtOAc (50:50, v/v) to afford 4-[6-(2-fluoro-4-methanesulfonyl-phenylamino)-pyridin-2-yloxy]-piperidine-1-carboxylic acid tert-butyl ester (Compound **B146**) as a pale yellow solid (3.47 g, 91%). $^1H$ NMR ($CDCl_3$, 400 MHz) δ 8.53 (m, 1H), 7.66 (m, 2H). 7.53 (m, 1H), 6.83 (bs, 1H), 6.42 (d, 1H), 6.36 (d, 1H), 5.13 (m, 1H), 3.80 (m, 2H), 3.31 (m, 2H), 3.07 (s, 3H), 2.01 (m, 2H), 1.78 (m, 2H), 1.48 (s, 9H). LRMS calculated for $C_{22}H_{28}FN_3O_5S$: 465.17. Found: 466.2 $(M+H)^+$.

(Reference) **Example 10.114: Preparation of 4-[2-(2-Fluoro-4-methanesulfonyl-phenylamino)-pyridin-4-yloxy]-piperidine-1-carboxylic acid isopropyl ester (Compound B151).**

Compound **B151** was prepared from 4-(2-chloro-pyridin-4-yloxy)-piperidine-1-carboxylic acid isopropyl ester and 2-fluoro-4-methanesulfonyl-phenylamine using the general method as described in Example 10.1, step 3. $^1H$ NMR ($CDCl_3$, 400 MHz) δ 8.59 (m, 1H), 8.14 (d, 1H), 7.66 (m, 2H), 6.84 (bs, 1H), 6.5 (m, 1H), 6.33 (s, 1H), 4.91 (m, 1H), 4.56 (m, 1H), 3.69 (m, 2H), 3.42 (m, 2H), 1.93 (m, 2H), 1.78 (m, 2H), 1.24 (d, 6H). LRMS calculated for $C_{21}H_{26}FN_3O_5S$: 451.16. Found: 452.3 $(M+H)^+$.

**Example 10.115: Preparation of 4-[4-(2-Fluoro-4-methanesulfonyl-phenylamino)-pyridin-2-yloxy]-piperidine-1-carboxylic acid isopropyl ester (Compound B149)**

A mixture of 4-(4-chloro-pyridin-2-yloxy)-piperidine-1-carboxylic acid isopropyl ester (82 mg, 0.274 mmol), palladium acetate (8.3 mg, 0.037 mmol), 2,8,9-triisobutyl-2,5,8,9-tetraaza-1-phospha-bicyclo[3,3,3]undecane (26.3 μl, 0.077 mmol), 2-fluoro-4-methanesulfonyl-phenylamine (77.8 mg, 0.41 mmol), sodium tert-butoxide (53 mg, 0.55 mmol), and 4 ml dioxane was heated in microwave for 2 hours at 120°C. Mixture was purified by HPLC to give 4-[4-(2-fluoro-4-methanesulfonyl-phenylamino)-pyridin-2-yloxy]-piperidine-1-carboxylic acid isopropyl ester as a tanned solid (TFA salt, 88 mg, 57%). $^1H$NMR (MeOD, 400 MHz) δ 1.27-1.28 (d, 6H), 1.82-1.86 (m, 2H), 2.03-2.08 (m, 2H), 3.22 (s, 3H), 3.42-3.50 (m, 2H), 3.71-3.78 (m, 2H), 4.98-5.01 (m, 1H), 6.65 (s, 1H), 6.87-6.89 (m, 1H), 7.75 (m, 1H), 7.89-7.99 (m, 3H). Exact mass calculated for $C_{21}H_{26}FN_3O_5S$ 451.16, found 452.3 $(MH^+)$.

(Reference) **Example 10.116: Preparation of 4-[5-(2-Fluoro-4-methanesulfonyl-phenylamino)-pyridin-3-yloxy]-piperidine-1-carboxylic acid tert-butyl ester (Compound B147)**

Sodium *t*-butoxide (17 mg, 0.180 mmol), BINAP (96 mg, 0.154 mmol), $Pd_2(dba)_3$ (75 mg, 0.0824 mmol), 4-(5-bromo-pyridin-3-yloxy)-piperidine-1-carboxylic acid tert-butyl ester (46 mg, 0.0129 mmol), and 2-fluoro-4-methanesulfonyl-phenylamine (29 mg, 0.155 mmol) were suspended in toluene (5 mL), and this mixture was heated at 70 C under a nitrogen atmosphere for 18 h. The solvent was evaporated in vacuo to give an oil which was dissolved in DMSO (1 mL), and this crude product was purified by mass-triggered preparative LCMS to give 4-[5-(2-fluoro-4-methanesulfonyl-phenylamino)-pyridin-3-yloxy]-piperidine-1-carboxylic acid tert-butyl ester as an oil (4 mg). Mass calcd. for $C_{22}H_{28}FN_3O_5S$: 465.17. Found 466.4 $(M+H)^+$, 410.3 $(M-56+H)^+$.

**Example 10.117: Preparation of 4-[6-(2-Methyl-4-propylamino-phenylamino)-pyrimidin-4-yloxy]-piperidine-1-carboxylic acid isopropyl ester (Compound B124).**

**Step 1: Preparation of 4-[6-(4-iodo-2-methyl-phenylamino)-pyrimidin-4-yloxy]-piperidine-1-carboxylic acid isopropyl ester.**

A mixture of 4-(6-chloro-pyrimidin-4-yloxy)-piperidine-1-carboxylic acid isopropyl ester (687 mg, 2.29 mmol), 2-methyl-4-iodoaniline ( 533 mg, 2.29 mmol), and sodium tert-butoxide (220 mg, 2.29 mmol)-in 20 mL dioxane were heated under microwave irradiation for 90 minutes at 80°C. Mixture was purified by HPLC to give 4-[6-(4-iodo-2-methyl-phenylamino)-pyrimidin-4-yloxy]-piperidine-1-carboxylic acid isopropyl ester as a white solid (TFA salt, 188 mg, 13%). $^1$HNMR (MeOD, 400 MHz) δ 1.01-1.03 (d, 6H), 1.45-1.52 (m, 2H), 1.73-1.80 (m, 2H), 1.99 (s, 3H), 3.10-3.18 (m, 2H), 3.47-3.53 (m, 1H), 4.94-4.97 (m, 1H), 5.70 (s, 1H), 6.88-6.90 (d, 1H), 7.37-7.39 (d, 1H), 7.49 (s, 1H), 8.06 (s, 1H). Exact mass calculated for $C_{20}H_{25}IN_4O_3$ 496.10, found 496.34 ($MH^+$).

**Step 2: Preparation of 4-[6-(2-Methyl-4-propylamino-phenylamino)-pyrimidin-4-yloxy]-piperidine-1-carboxylic acid isopropyl ester (Compound B124).**

Compound **B124** was prepared using a similar procedure as described herein as a white solid (TFA salt, 120 mg, 74%). $^1$HNMR (MeOD, 400 MHz) δ 0.86-0.90 (t, 3H), 1.07-1.09 (d, 6H), 1.50-1.60 (m, 4H), 1.80-1.87 (m, 2H), 2.11 (s, 3H), 3.09-3.20 (m, 4H), 3.55-3.62 (m, 2H), 4.67-4.74 (m, 1H), 5.03-5.06 (m, 1H), 5.79 (s, 1H), 6.92-6.94 (d, 1 H), 6.99 (s, 1H), 7.27-7.29 (d, 1H), 8.09 (s, 1 H). Exact mass calculated for $C_{23}H_{33}N_5O_3$ 427.26, found 428.2 ($MH^+$).

**Example 10.117: Preparation of 4-[6-(2-Fluoro-4-methanesulfonyl-phenylamino)-pyrimidin-4-yloxy]-piperidine-1-carboxylic acid isopropyl ester (Compound B3).**

(2-Fluoro-4-methanesulfonyl-phenyl)-[6-(piperidin-4-yloxy)-pyrimidin-4-yl]-amine (697mg, 1.3mmol) and TEA (5.2 mmole, 4eq) were dissolved in DMF (10 mL) and then isopropyl chloroformate (1.69mmole, 1.3eq) was added into the solution. The reaction mixture was stirred at Room Temp for 1 hours. The crude was purified through HPLC provided Compound B3 as a yellow solid (476 mg, 81 %). $^1$H NMR 400MHz $CDCl_3$ δ (ppm): 8.45 (s, 1H); 8.02 (t,1H); 7.80-7.76 (m,2H); 6.06 (s,1H); 5.35 (m, 1H); 4.92 (sep,1H); 3.79 (sb,2H); 3.35-3.28 (m, 2H); 3.09 (s,3H); 1.97-2.01 (m, 2H); 1.73-1.74 (m, 2H); 1.25 (d, 6H). Exact mass calculated for $C_{20}H_{25}FN_4O_5S$ 452.2, found LCMS (ESI) m/z 453.2($MH^+$, 100%).

**Example 10.118: Preparation of 4-[6-(2-Methyl-pyridin-3-yloxy)-pyrimidin-4-yloxy]-piperidine-1-carboxylic acid tert-butyl ester (Compound B157).**

A solution of 4-(6-chloro-pyrimidin-4-yloxy)-piperidine-1-carboxylic acid tert-butyl ester (1.57 g, 5.0 mmol) and 2-methyl-pyridin-3-ol (654 mg, 6.0 mmol) in anhydrous DMF (10 mL) containing $K_2CO_3$ (1.38 g, 10 mmol) was heated at 150˚C for 1.5 h. The reaction mixture was cooled to ambient temperature, filtered over Celite, and the solvent was removed from the filtrate under high vacuum. The residue was dissolved in ethyl acetate, rinsed with water and brine, and the organic extract was dried over $MgSO_4$. The solution was concentrated to dryness and the residue was dissolved in diethyl ether, filtered over Celite, and the filtrate was treated with IN HCl/ether (5 mL). The resulting precipitate was collected by filtration to afford the title compound as a white solid (1.83 g, 87% yield): MS m/z 387.3, 331.4; $^1$H NMR (DMSO-$d_6$) δ 8.59 (d, 1H, J = 5.3-Hz), 8.46 (s, 1H), 8.08 (d, 1 H, J = 8.0 Hz), 7.69 (t, 1 H, J = 8.1 Hz), 6.63 (s, 1 H), 5.26 (m, 1 H), 3.71 (m, 2 H), 3.18 (m, 2 H), 2.45 (s, 3 H), 1.97 (m, 2 H), 1.59 (m, 2 H), 1.41 (s, 9 H).

**Example 10.119: Preparation of 4-[6-(6-Bromo-2-methyl-pyridin-3-yloxy)-pyrimidin-4-yloxy]-piperidine-1-carboxylic acid isopropyl ester (Compound B156).**

A solution of 4-(6-chloro-pyrimidin-4-yloxy)-piperidine-1-carboxylic acid isopropyl ester (3.0 g, 10 mmol) and 6-bromo-2-methyl-pyridin-3-ol (2.25 g, 12 mmol) in anhydrous DMF (20 mL) containing $K_2CO_3$ (2.76 g, 20 mmol) was heated at 125˚C for 4h. Upon cooling to ambient temperature, the reaction mixture was filtered over Celite, and the solvent was removed from the filtrate under high vacuum. The residue was dissolved in ethyl acetate, and the resulting solution was rinsed twice with 1N NaOH, and subsequently rinsed with water and brine. The organic extract was dried over $MgSO_4$, the solution was concentrated to dryness, and the residue was dissolved in diethyl ether. The addition of 1N HCl/ether (10 mL) resulted in the formation of a small amount of dark precipitate, which was removed by filtration. The solvent was removed from the filtrate to give a colorless oil (loss of HCl upon solvent removal rendered the free base), which gradually formed the title compound as a white solid upon standing (3.91 g, 87% yield): MS m/z 451.4, 453.4; $^1$H NMR (DMSO-$d_6$) δ 8.44 (s, 1 H), 7.61 (d, 1 H, J = 8.5 Hz), 7.56 (d, 1 H, J = 8.5 Hz), 6.56 (s, 1H), 5.27 (m, 1H), 4.78 (m, 1H), 4.03 (m, 2H), 3.23 (m, 2H), 2.27 (s, 3H), 1.95 (m, 2 H), 1.59 (m, 2H), 1.18 (d, 6 H, J = 6.3 Hz).

(Reference) **Example 10.120: Preparation of 4-(6-{6-[(2,2-Dimethyl-[1,3]dioxolan-4-ylmethyl)-amino]-2-methyl-pyridin-3-yloxy}-pyrimidin-4-yloxy)-piperidine-1-carbocylic acid isopropyl ester (Compound B154).**

A mixture of 4-[6-(6-bromo-2-methyl-pyridin-3-yloxy)-pyrimidin-4-yloxy]-piperidine-1-carboxylic acid isopropyl ester (1.13 g, 2.5 mmol), 4-(aminomethyl)-2,2-dimethyl-1,3-dioxolane (393 mg, 3.0 mmol), 2-(di-t-butylphosphino)biphenyl (75 mg, 0.25 mmol), tris(dibenzylideneacetone)dipalladium (115 mg, 0.125 mmol), and sodium t-butoxide (480

mg, 5.0 mmol) in anhydrous toluene (10 mL) in a sealed vial was heated at 150°C for 2h. After cooling, the reaction mixture was rinsed twice with water, and then the organic extract was filtered over Celite, rinsing with a small amount of ethyl acetate. The filtrate was then directly subjected to flash chromatography (25-30% ethyl acetate/hexane). The title compound was obtained as an amber gum obtained upon solvent removal (505 mg, 40% yield): MS m/z 462.3 (due to loss of protective group upon ionization); $^1$H NMR (DMSO-$d_6$) δ 8.42 (s,1 H), 7.16 (d, 1 H, J = 8.8 Hz), 6.62 (t, 1H, J = 5.9 Hz), 6.40 (d, 1 H, J = 8.8 Hz), 6.25 (s, 1 H), 5.24 (m, 1 H), 4.77 (m, 1 H), 4.23 (m, 1 H), 4.01 (m, 1 H), 3.69 (m, 3 H), 3.37 (m, 2 H), 3.22 (m, 1 H), 2.07 (s, 3 H), 1.96 (m, 2 H), 1.57 (m, 2 H), 1.36 (s, 3 H), 1.27 (s, 3 H), 1.17 (d, 6 H, J = 6.2 Hz).

**Example 10.121: Preparation of 4-{6-[6-(2,3-Dihydroxy-propylamino)-2-methyl-pyridin-3-yloxy}-pyrimidin-4-yloxy}-piperidine-1-carboxylic acid isopropyl ester (Compound B153).**

A solution of 4-(6-{6-[(2,2-dimethyl-[1,3]dioxolan-4-ylmethyl)-amino]-2-methyl-pyridin-3-yloxy}-pyrimidin-4-yloxy)-piperidine-1-carboxylic acid isopropyl ester (Compound **154**,480 mg, 0.96 mmol) in THF was treated with 6 mL cone. HCl and stirred for 2h. The solvent was removed, and the residue was dissolved in $CH_2Cl_2$, to which was added diethyl ether, at which point a precipitate formed. This material was collected by filtration, yielding a hygroscopic solid which rapidly formed a sticky gum. The filter cake was then dissolved in $CH_2Cl_2$, and the resulting solution was washed with 2M $Na_2CO_3$ and dried over $MgSO_4$. The solvent was removed to furnish an amber gum (238 mg, 54% yield): MS m/z 462.6; $^1$H NMR (DMSO-$d_6$) δ 8.42 (s, 1 H), 7.17 (d, 1 H, J = 8.8 Hz), 6.47 (t, 1 H, J = 5.7 Hz), 6.42 (d, 1 H, J = 8.8 Hz), 6.26 (s, 1 H), 5.24 (m, 1 H), 4.96 (d, 1 H, J = 4.8 Hz), 4.77 (m, 1 H), 4.68 (t, 1 H, J = 5.9 Hz), 3.68 (m, 2 H), 3.61 (m, 1 H), 3.35 (m, 3 H), 3.19 (m, 3 H), 2.06 (s, 3 H), 1.95 (m, 2 H), 1.57 (m, 2 H), 1.17 (d, 6 H, J = 6.3 Hz).

**Example 11: Protocol for RUP3 Dose Responses in Melanophores**

Melanophores are maintained in culture as reported by Potenza, M. N. and Lerner, M. R., in Pigment Cell Research, Vol. 5, 372-378, 1992 and transfected with the **RUP3** expression vector (pCMV) using electroporation. Following electroporation, the transfected cells are plated into 96 well plates for the assay. The cells are then allowed to grow for 48 hours in order to both recover from the electroporation procedure and attain maximal receptor expression levels.

On the assay day, the growth medium on the cells is replaced with serum-free buffer containing 10nM melatonin. The melatonin acts via an endogenous Gi-coupled GPCR in the melanophores to lower intracellular cAMP levels. In response to lowered cAMP levels, the melanophores translocate their pigment to the center of the cell. The net effect of this is a significant decrease in the absorbance reading of the cell monolayer in the well, measured at 600-650nM.

After a 1-hour incubation in melatonin, the cells become completely pigment-aggregated. At this point a baseline absorbance reading is collected. Serial dilutions of test compounds are then added to the plate and compounds that stimulate **RUP3** produce increases in intracellular cAMP levels. In response to these increased cAMP levels, the melanophores translocate their pigment back into the cell periphery. After one hour, stimulated cells are fully pigment-dispersed. The cell monolayer in the dispersed state absorbs much more light in the 600-650nm range. The measured increase in absorbance compared to the baseline reading allows one to quantitate the degree of receptor stimulation and plot a dose-response curve.

The compounds in the above examples were screened using the melanophore dispersion assay, as described above. Representative compounds of the present invention and their corresponding $EC_{50}$-values are shown in Table 6 below. Certain other compounds illustrated in the Examples showed $EC_{50}$ activities in the melanophore dispersion assay of less than about 10 μM.

**TABLE 6**

| Compound | RUP3 ($EC_{50}$) (nM) |
|---|---|
| B16 | 62 |
| B21 | 128 |
| B143 | 48 |

Each of the embodiments of the present invention may in the alternative be limited to relate to those compounds that demonstrate about 100 fold or greater binding to **RUP3** compared to the corticotrophin-releasing factor-1 (CRF-1) receptor; a recent review of CRF-1 compounds can be found in Expert Opin. Ther. Patents 2002,12(11), 1619-1630 .

## Claims

1. A compound selected from compounds of Formula (I) and pharmaceutically acceptable salts, solvates, hydrates and N-oxides thereof:

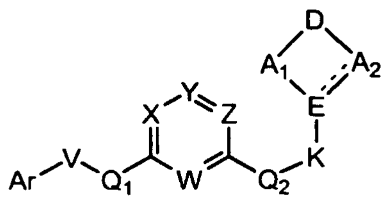

(I)

wherein:

$A_1$ and $A_2$ are both $-CH_2CH_2-$, and each $A_1$ and $A_2$ is optionally substituted with 1, 2, 3 or 4 methyl groups;
D is $CR_1R_2$ or $NR_2$, wherein $R_1$ is selected from H, $C_{1-6}$ alkyl, $C_{1-6}$ alkoxy, halogen and hydroxyl;
E is N or $CR_3$, wherein $R_3$ is H or $C_{1-6}$ alkyl;
- - - is a single bond;
K is absent, $C_{3-6}$ cycloalkylene, or $C_{1-3}$ alkylene group each optionally substituted with one or more substituents selected independently from $C_{1-6}$ alkyl, $C_{1-6}$ alkoxy, carboxy, cyano, and halogen;
$Q_1$ is $NR_4$, O, S, S(O), or $S(O)_2$, wherein $R_4$ is H, $C_{1-6}$ acyl, $C_{1-6}$ alkyl, $C_{2-6}$ alkenyl, $C_{2-6}$ alkynyl, $C_{3-7}$ cycloalkyl, or $C_{3-7}$-cycloalkyl-$C_{1-3}$-alkylene, wherein said $C_{1-6}$ alkyl is optionally substituted with one or more substituents selected independently from $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino and nitro;
$Q_2$ is $NR_5$ or O, wherein $R_5$ is H, $C_{1-6}$ acyl, $C_{1-6}$ alkyl, $C_{2-6}$ alkenyl, $C_{2-6}$ alkynyl, $C_{3-7}$ cycloalkyl, or $C_{3-7}$-cyoloalkyl-$C_{1-3}$-alkylene, wherein said $C_{1-6}$ alkyl is optionally substituted with one or more substituents selected independently from $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino and nitro;
W is CH;
X is N or $CR_6$;
Y is N or $CR_7$;
Z is N;
V is absent, $C_{1-3}$ heteroalkylene, or $C_{1-3}$ alkylene wherein each are optionally substituted with one or more substituents selected independently from $C_{1-3}$ alkyl, $C_{1-6}$ alkoxy, carboxy, cyano, $C_{1-3}$ haloalkyl, and halogen;
$R_6$, $R_7$, and $R_8$ are each independently selected from H, $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino and nitro, wherein said $C_{2-6}$ alkenyl, $C_{1-6}$ alkyl, $C_{2-6}$ alkynyl and $C_{3-6}$ cycloalkyl are each optionally substituted with one or more substituents independently selected from $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino and nitro;
Ar is phenyl, naphthyl, pyridyl, benzofuranyl, pyrazinyl, pyridazinyl, pyrimidinyl, triazinyl, quinoline, benzoxazole, benzothiazole, 1 H-benzimidazole, isoquinoline, quinazoline, quinoxaline, pyrrole, or indole, optionally substituted with $R_9$, $R_{10}$, $R_{11}$, $R_{12}$, and $R_{13}$;
$R_9$ is selected from $C_{1-6}$ acyl, $C_{1-6}$ acylsulfonamide, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$

alkylthio, $C_{1-6}$ alkylthiocarboxamide, $C_{1-6}$ alkylthioureyl, $C_{1-6}$ alkylureyl, amino, aryl, arylcarbonyl, arylsulfonyl, di-$C_{1-6}$-alkylamino, carbamimidoyl, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, guanidine, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, heterocyclic, heterocyclicsulfonyl, heteroaryl, hydroxyl, hydroxylamino, nitro, $C_{3-6}$ oxo-cycloalkyl, phenoxy, sulfonamide, sulfonic acid and thiol; and wherein each $R_9$ is optionally substituted with one or more substituents selected independently from $C_{1-6}$ acyl, $C_{1-6}$ acylsulfonamide, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyle, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, aryl, arylcarbonyl, arylsulfonyl, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, heteroaryl, heteroarylcarbonyl, heteroarylsulfonyl, heterocyclic, hydroxyl, hydroxylamino, and nitro;

$R_{10}$, $R_{11}$, $R_{12}$, and $R_{13}$ are independently selected from $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino, nitro, and thiol; or two adjacent $R_{10}$, $R_{11}$, $R_{12}$, and $R_{13}$ groups together with the atoms to which they are bonded form a 5, 6 or 7 member cycloalkyl, 5, 6 or 7 member cycloalkenyl, or 5, 6 or 7 member heterocyclic group wherein the 5, 6 or 7 member group is optionally substituted with halogen or oxo; and

either:

$R_2$ is aryl, arylcarbonyl, $C_{1-6}$ alkoxycarbonyl, $C_{3-7}$-cycloalkoxycarbonyl, heteroaryl, or heteroarylcarbonyl; each optionally substituted with one or more substituents selected independently from $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, aryl, di-$C_{1-6}$-alkylamino, carbo-$C_{1-6}$-alkoxy, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, heterocyclic, heteroaryl, hydroxyl, hydroxylamino, nitro, and thiol;

or:

$R_2$ is $C_{1-6}$ alkoxycarbonyl, $C_{1-6}$ alkoxycarbonyl substituted by $C_{3-6}$ cycloalkyl, $C_{3-7}$-cycloalkoxycarbonyl, or heteroaryl-$C_{1-3}$-alkylene optionally substituted with 1, 2, or 3 $C_{1-6}$ alkyl;

provided that the compound is not 4-[6-(2-fluoro-4-methanesulfonyl-phenoxy)-pyrimidin-4-yloxy]-piperidine-1-carboxylic acid isopropyl ester.

**2.** A compound according to claim 1, wherein K is absent.

**3.** A compound according to claim 1, wherein K is $-CH_2-$.

**4.** A compound according to any one of claims 1 to 3, wherein V is absent.

**5.** A compound according to any one of claims 1 to 4, wherein $Q_1$ is O.

**6.** A compound according to any one of claims 1 to 4, wherein $Q_1$ is NH.

**7.** A compound according to any one of claims 1 to 6, wherein $Q_2$ is O.

**8.** A compound according to any one of claims 1 to 6, wherein $Q_2$ is $NR_5$.

**9.** A compound according to claim 8, wherein $R_5$ is H or $C_{1-6}$ alkyl.

**10.** A compound according to any one of claims 1 to 9, wherein:

W is CH;

X is N;
Y is $CR_7$, wherein $R_7$ is H, $C_{1-6}$ alkyl, or halogen; and
Z is N.

**11.** A compound according to any one of claims 1 to 10, wherein E is CH and D is $NR_2$.

**12.** A compound according to any one of claims 1 to 11, wherein $R_2$ is aryl, arylcarbonyl, $C_{1-6}$ alkoxycarbonyl, $C_{3-7}$-cycloalkoxycarbonyl, heteroaryl, or heteroarylcarbonyl; each optionally substituted with one or more substituents selected independently from $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, aryl, di-$C_{1-6}$-alkylamino, carbo-$C_{1-6}$-alkoxy, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, heterocyclic, heteroaryl, hydroxyl, hydroxylamino, nitro, and thiol.

**13.** A compound according to any one of claims 1 to 11, wherein $R_2$ is $C_{1-6}$ alkoxycarbonyl, $C_{1-6}$ alkoxycarbonyl substituted by $C_{3-6}$ cycloalkyl, $C_{3-7}$-cycloalkoxycarbonyl, or heteroaryl-$C_{1-3}$-alkylene optionally substituted with 1, 2, or 3 $C_{1-6}$ alkyl.

**14.** A compound according to any one of claims 1 to 13, wherein Ar is phenyl optionally substituted with $R_9$, $R_{10}$, $R_{11}$, $R_{12}$, and $R_{13}$;
$R_9$ is selected from methanesulfonyl, 2-methanesulfonyl-ethyl, acetylsulfamoyl, propionylsulfamoyl, ethylsulfanyl, isopropylsulfanyl, ethylsulfamoyl, methylsulfamoyl, dimethylsulfamoyl, methylsulfamoylmethyl, sulfamoyl, [1,2,4] triazol-1-yl, [1,2,4]triazol-1-ylmethyl, 2-[1,2,4]triazol-1-yl-ethyl, methoxy, 2-oxo-oxazolidin-4-ylmethyl, 1,1-dioxo-1$\lambda^6$-thiomorpholin-4-ylmethyl, pyrazol-1-yl, trifluoromethanesulfonyl, morpholine-4-sulfonyl, pyridine-2-carbonyl, F, Cl, cyano, Br, carboxy, butyryl, propoxycarbonyl, hydroxy, propylcarbamoyl, *N*-hydroxycarbamimidoyl, carbamimidoyl, *N*-ethylcarbamimidoyl, and 2-amino-ethylamino; and
$R_{10}$, $R_{11}$, $R_{12}$, and $R_{13}$ are independently selected from F, methoxy, methyl, ethyl, and carboxy.

**15.** A compound according to any one of claims 1 to 13 wherein Ar is phenyl optionally substituted with $R_9$, $R_{10}$, $R_{11}$, $R_{12}$, and $R_{13}$;
$R_9$ is selected from methanesulfonyl, cyano, F, Cl, Br, I, methyl, methoxy, ethylamino, ethylsulfanyl, isopropylsulfanyl, hydroxy, isopropoxy, propoxy, dimethylamino, propylamino, isopropylamino, acetylamino, piperidin-1-yl, trifluoromethoxy, oxazol-5-yl, ethynyl, 3-methyl-butylamino, 2-morpholin-4-yl-ethylamino, acetylsulfamoyl, propionylsulfamoyl, tetrahydro-furan-2-ylmethoxy, morpholin-4-yl, 4-methyl-piperazin-1-yl, butylamino, 2-pyrrolidin-1-yl-ethoxy, 2-dimethylamino-ethoxy, 2-morpholin-4-yl-ethoxy, morpholin-4-ylamino, 2-methoxy-ethylamino, and tetrahydro-furan-2-ylmethyl-amino; and
$R_{10}$, $R_{11}$, $R_{12}$, and $R_{13}$ are independently selected from F, Cl, Br, I, hydroxyl, methoxy, cyano, methyl, and trifluoromethoxy.

**16.** A compound according to any one of claims 1 to 13, wherein Ar is selected from pyridin-2-yl, pyridin-3-yl, pyridin-4-yl, quinolin-6-yl, and benzothiazol-6-yl, each optionally substituted with $R_9$, $R_{10}$, $R_{11}$, $R_{12}$, and $R_{13}$;
$R_9$ is selected from methanesulfonyl, methoxy, carboxy, acetylsulfamoyl, propionylsulfamoyl, acetylamino, F, Cl, Br, methyl, and trifluoromethyl; and
$R_{10}$, $R_{11}$, $R_{12}$, and $R_{13}$ are independently selected from methoxy, methyl, F, Cl, and Br.

**17.** A compound according to any one of claims 1 to 13, wherein Ar is selected from pyridine-2-yl, pyridine-3-yl, pyridine-4-yl, quinolin-6-yl, and benzothiazol-6-yl, optionally substituted with $R_9$, $R_{10}$, $R_{11}$, $R_{12}$, and $R_{13}$;
$R_9$ is selected from cyano, F, Cl, Br, acetylamino, methoxy, methyl, propoxy, propylamino, isopropylamino, phenyl, *t*-butyl, 4-methylphenyl, ethyl, methylsulfanyl, morpholin-4-yl, benzenesulfonyl, trifluoromethyl, cyclopropyl, carbamoyl, 3,4-difluorophenyl, 4-chlorophenyl, 1-methyl-pyrrolidin-2-yl, acetylsulfamoyl, propionylsulfamoyl, and pyridine-2-yl; and
$R_{10}$, $R_{11}$, $R_{12}$, and $R_{13}$ are independently methyl, F or Cl.

**18.** A compound according to claim 1, selected from compounds having Formula (IIIi) and pharmaceutically acceptable salts, solvates, hydrates and N-oxides thereof:

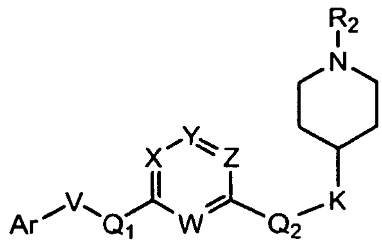

**(IIIi)**

wherein:

K is absent or a $C_{1-3}$ alkylene group;
$Q_1$ is NH or O;
$Q_2$ is $NR_5$ or O, wherein $R_5$ is H or $C_{1-6}$ alkyl;
W is CH, X is N, Y is CH, and Z is N;
V is absent;
Ar is selected from pyridine-2-yl, pyridine-3-yl, pyridine-4-yl, quinolin-6-yl, and benzothiazol-6-yl, each optionally substituted with $R_9$, $R_{10}$, $R_{11}$, $R_{12}$, and $R_{13}$;
$R_9$ is $C_{1-6}$ acyl, $C_{1-6}$ acylsulfonamide, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, amino, aryl, arylsulfonyl, di-$C_{1-6}$-alkylamino, carbamimidoyl, $C_{1-6}$ alkoxycarbonyl, carboxamide, carboxy, cyano, $C_{3-6}$ cycloalkoxy, $C_{3-6}$ cycloalkyl, di-$C_{1-6}$-alkylsulfonamide, guanidine, $C_{1-6}$ haloalkoxy, $C_{1-6}$ haloalkyl, halogen, $C_{1-6}$ haloalkylsulfonyl, heteroaryl, heteroarylcarbonyl, heteroarylsulfonyl, heterocyclic, heterocyclicsulfonyl, hydroxyl, sulfonamide, or thiol; wherein $C_{1-6}$ alkoxy, $C_{1-6}$ alkyl, $C_{1-6}$ alkylamino, amino, aryl, carbamimidoyl, heterocyclic, are optionally substituted with 1, 2, 3 or 4 substituents selected independently from $C_{1-6}$ alkoxy, $C_{1-6}$ alkyl, $C_{2-6}$ alkynyl, $C_{1-6}$ alkylsulfonamide, $C_{1-6}$ alkylsulfonyl, amino, aryl, $C_{3-6}$ cycloalkyl, di-$C_{1-6}$-alkylamino, halogen, heteroaryl, heterocyclic, and hydroxyl;
$R_{10}$, $R_{11}$, $R_{12}$, and $R_{13}$ are independently selected from $C_{1-6}$ alkoxy, $C_{1-6}$ alkyl, $C_{1-6}$ alkylamino, carboxy, cyano, halogen, $C_{1-6}$ haloalkoxy, $C_{1-6}$ haloalkylsulfonyl, and hydroxyl; or two adjacent $R_{10}$, $R_{11}$, $R_{12}$, and $R_{13}$ groups together with the atoms to which they are bonded form a 5 or 6 member cycloalkyl or 5 or 6 member heterocyclic group, wherein said 5 or 6 member group is optionally substituted with halogen or oxo; and
$R_2$ is *tert*-butoxycarbonyl, isobutoxycarbonyl, isopropoxycarbonyl, butoxycarbonyl, cyclopropylmethoxycarbonyl, 3-methyl-butoxycarbonyl, or 3-isopropyl-[1,2,4]oxadiazol-5-yl.

**19.** A compound according to claim 1, wherein said compound is selected from the following compounds and pharmaceutically acceptable salts, solvates, hydrates and N-oxides thereof:

(A3) 4-{[6-(2-Fluoro-4-methanesulfonyl-phenylamino)-pyrimidin-4-yl]-methyl-amino}-piperidine-1-carboxylic acid tert-butyl ester;
(A4) 4-({[6-(2-Fluoro-4-methanesulfonyl-phenylamino)-pyrimidin-4-yl]-methyl-amino}-methyl)-piperidine-1-carboxylic acid tert-butyl ester;
(A5) 4-({[6-(4-Methanesulfonyl-phenylamino)-pyrimidin-4-yl]-methyl-amino}-methyl)-piperidine-1-carboxylic acid tert-butyl ester;
(A6) 4-({[6-(2,5-Difluoro-benzylamino)-pyrimidin-4-yl]-methyl-amino}-methyl)-piperidine-1-carboxylic acid tert-butyl ester;
(A7) 4-[({6-[(Benzo[1,3]dioxol-5-ylmethyl)-amino]-pyrimidin-4-yl}-methyl-amino)-methyl]-piperidine-1-carboxylic acid tert-butyl ester;
(A9) 4-({Methyl-[6-(2-pyridin-4-yl-ethylamino)-pyrimidin-4-yl]-amino}-methyl)-piperidine-1-carboxylic acid tert-butyl ester;
(A10) 4-({Methyl-[6-(2-pyridin-3-yl-ethylamino)-pyrimidin-4-yl]-amino}-methyl)-piperidine-1-carboxylic acid tert-butyl ester;
(A11) 4-[(Methyl-{6-[(pyridin-3-ylmethyl)-amino]-pyrimidin-4-yl}-amino)-methyl]-piperidine-1-carboxylic acid tert-butyl ester;
(A12)4-[({6-[(2-Fluoro-4-methanesulfonyl-phenyl)-methyl-amino]-pyrimidin-4-yl}-methyl-amino)-methyl]-piperidine-1-carboxylic acid tert-butyl ester;
(A13) 4-({[6-(2-Fluoro-4-methanesulfonyl-phenylamino)-pyrimidin-4-yl]-methyl-amino}-methyl)-piperidine-1-carboxylic acid isobutyl ester;
(A14) 4-({[6-(4-Cyano-2-fluoro-phenylamino)-pyrimidin-4-yl]-methyl-amino}-methyl)-piperidine-1-carboxylic acid tert-butyl ester;
(A15) 4-[({6-[4-(2-Methanesulfonyl-ethyl)-phenylamino]-pyrimidin-4-yl}-methyl-amino)-methyl]-piperidine-1-carboxylic acid tert-butyl ester;
(A16) 4-({[6-(4-Ethylsulfanyl-phenylamino)-pyrimidin-4-yl]-methyl-amino}-methyl)-piperidine-1-carboxylic acid tert-butyl ester;
(A17) 4-({[6-(4-Isopropylsulfanyl-phenylamino)-pyrimidin-4-yl]-methyl-amino}-methyl)-piperidine-1-carboxylic acid tert-butyl ester;
(A18) 4-({[6-(4-Ethylsulfamoyl-phenylamino)-pyrimidin-4-yl]-methyl-amino}-methyl)-piperidine-1-carboxylic acid tert-butyl ester;
(A19) 4-({Methyl-[6-(4-methylsulfamoyl-phenylamino)-pyrimidin-4-yl]-amino}-methyl)-piperidine-1-carboxylic acid tert-butyl ester;
(A20) 4-({[6-(4-Dimethylsulfamoyl-phenylamino)-pyrimidin-4-yl]-methyl-amino}-methyl)-piperidine-1-carboxylic acid tert-butyl ester;
(A21) 4-({Methyl-[6-(4-methylsulfamoylmethyl-phenylamino)-pyrimidin-4-yl]-amino}-methyl)-piperidine-1-carboxylic acid tert-butyl ester;
(A22) 4-({Methyl-[6-(4-sulfamoyl-phenylamino)-pyrimidin-4-yl]-amino}-methyl)-piperidine-1-carboxylic acid tert-butyl ester;
(A23) 4-({Methyl-[6-(4-[1,2,4]triazol-1-yl-phenylamino)-pyrimidin-4-yl]-amino}-methyl)-piperidine-1-carboxylic acid tert-butyl ester;
(A24) 4-({Methyl-[6-(4-[1,2,4]triazol-1-ylmethyl-phenylamino)-pyrimidin-4-yl]-amino}-methyl)-piperidine-1-carboxylic acid tert-butyl ester;
(A25) 4-[(Methyl-{6-[4-(2-[1,2,4]triazol-1-yl-ethyl)-phenylamino]-pyrimidin-4-yl}-amino)-methyl]-piperidine-1-carboxylic acid tert-butyl ester;
(A26) 4-({[6-(Benzo[1,3]dioxol-5-ylamino)-pyrimidin-4-yl]-methyl-amino}-methyl)-piperidine-1-carboxylic acid tert-butyl ester;
(A27) 4-({[6-(6-Methanesulfonyl-pyridin-3-ylamino)-pyrimidin-4-yl]-methyl-amino}-methyl)-piperidine-1-carboxylic acid tert-butyl ester;
(A28) 4-({[6-(3,5-Dimethoxy-phenylamino)-pyrimidin-4-yl]-methyl-amino}-methyl)-piperidine-1-carboxylic acid tert-butyl ester;
(A29) 4-[(Methyl-{6-[4-(2-oxo-oxazolidin-4-ylmethyl)-phenylamino]-pyrimidin-4-yl}-amino)-methyl]-piperidine-1-carboxylic acid tert-butyl ester;
(A30) 4-[({6-[4-(1,1-Dioxo-1λ6-thiomorpholin-4-ylmethyl)-phenylamino]-pyrimidin-4-yl}-methyl-amino)-methyl]-piperidine-1-carboxylic acid tert-butyl ester;
(A31) 4-({Methyl-[6-(4-pyrazol-1-yl-phenylamino)-pyrimidin-4-yl]-amino}-methyl)-piperidine-1-carboxylic acid tert-butyl ester;
(A32) 4-({[6-(2,2-Difluoro-benzo[1,3]dioxol-5-ylamino)-pyrimidin-4-yl]-methyl-amino}-methyl)-piperidine-1-carboxylic acid tert-butyl ester;
(A33) 4-({Methyl-[6-(4-trifluoromethanesulfonyl-phenylamino)-pyrimidin-4-yl]-amino}-methyl)-piperidine-1-carboxylic acid tert-butyl ester;
(A34) 4-[(Methyl-{6-[4-(morpholine-4-sulfonyl)-phenylamino]-pyrimidin-4-yl}-amino)-methyl]-piperidine-1-carboxylic acid tert-butyl ester;
(A35) 4-[(Methyl-{6-[2-(pyridine-2-carbonyl)-phenylamino]-pyrimidin-4-yl}-amino)-methyl]-piperidine-1-carboxylic acid tert-butyl ester;
(A36) 4-({[6-(2-Fluoro-5-methanesulfonyl-phenylamino)-pyrimidin-4-yl]-methyl-amino}-methyl)-piperidine-1-carboxylic acid tert-butyl ester;
(A39) 4-({[6-(3,4-Difluoro-phenylamino)-pyrimidin-4-yl]-methyl-amino}-methyl)-piperidine-1-carboxylic acid tert-butyl ester;
(A40) 4-({[6-(2,6-Difluoro-phenylamino)-pyrimidin-4-yl]-methyl-amino}-methyl)-piperidine-1-carboxylic acid tert-butyl ester;
(A41) 4-({[6-(2,5-Difluoro-phenylamino)-pyrimidin-4-yl]-methyl-amino}-methyl)-piperidine-1-carboxylic acid tert-butyl ester;
(A42) 4-({[6-(2,3-Difluoro-phenylamino)-pyrimidin-4-yl]-methyl-amino}-methyl)-piperidine-1-carboxylic acid tert-butyl ester;
(A43) 4-({Methyl-[6-(2,3,5-trifluoro-phenylamino)-pyrimidin-4-yl]-amino}-methyl)-piperidine-1-carboxylic acid tert-butyl ester;
(A44) 4-({[6-(2-Fluoro-phenylamino)-pyrimidin-4-yl]-methyl-amino}-methyl)-piperidine-1-carboxylic acid tert-butyl ester;

(A45) 4-({[6-(2-Fluoro-4-methyl-phenylamino)-pyrimidin-4-yl]-methyl-amino}-methyl)-piperidine-1-carboxylic acid tert-butyl ester;

(A46) 4-({[6-(3-Chloro-2-fluoro-phenylamino)-pyrimidin-4-yl]-methyl-amino}-methyl)-piperidine-1-carboxylic acid tert-butyl ester;

(A47) 4-({[6-(2,4-Difluoro-phenylamino)-pyrimidin-4-yl]-methyl-amino}-methyl)-piperidine-1-carboxylic acid tert-butyl ester;

(A48) 4-[(Methyl-{6-[2-(1-oxy-pyridin-3-yl)-ethylamino]-pyrimidin-4-yl}-amino)-methyl]-piperidine-1-carboxylic acid tert-butyl ester;

(A49) 4-[(Methyl-{6-[2-(1-oxy-pyridin-3-yl)-ethylamino]-pyrimidin-4-yl}-amino)-methyl]-piperidine-1-carboxylic acid isobutyl ester;

(A50) 4-({[6-(2,5-Difluoro-phenylamino)-pyrimidin-4-yl]-methyl-amino}-methyl)-piperidine-1-carboxylic acid isobutyl ester;

(A51) 4-({[6-(4-Cyano-2-fluoro-phenylamino)-pyrimidin-4-yl]-methyl-amino}-methyl)-piperidine-1-carboxylic acid isobutyl ester;

(A52) 4-[({6-[2-(2-Fluoro-phenoxy)-ethylamino]-pyrimidin-4-yl}-methyl-amino)-methyl]-piperidine-1-carboxylic acid tert-butyl ester;

(A53) 4-({[6-(2-Fluoro-phenoxy)-pyrimidin-4-yl]-methyl-amino}-methyl)-piperidine-1-carboxylic acid tert-butyl ester;

(A54) 4-({[6-(2,5-Difluoro-phenoxy)-pyrimidin-4-yl]-methyl-amino}-methyl)-piperidine-1-carboxylic acid tert-butyl ester;

(A55) 4-[({6-[2-(2-Chloro-phenoxy)-ethylamino]-pyrimidin-4-yl}-methyl-amino)-methyl]-piperidine-1-carboxylic acid tert-butyl ester;

(A56) 4-({[6-(2-Chloro-phenoxy)-pyrimidin-4-yl]-methyl-amino}-methyl)-piperidine-1-carboxylic acid tert-butyl ester;

(A57) 4-[({6-[2-(4-Ftuoro-phenoxy)-propylamino]-pyrimidin-4-yl}-methyl-amino)-methyl]-piperidine-1-carboxylic acid tert-butyl ester;

(A58) 4-({[6-(4-Ethylsulfamoyl-2-fluoro-phenylamino)-pyrimidin-4-yl]-methyl-amino}-methyl)-piperidine-1-carboxylic acid tert-butyl ester;

(A59) 4-({[6-(2-Fluoro-4-isopropylsulfamoyl-phenylamino)-pyrimidin-4-yl]-methyl-amino}-methyl)-piperidine-1-carboxylic acid tert-butyl ester;

(A60) 4-({[6-(4-Cyano-2,5-difluoro-phenylamino)-pyrimidin-4-yl]-methyl-amino}-methyl)-piperidine-1-carboxylic acid tert-butyl ester;

(A61) 4-({[6-(4-Bromo-2,5-difluoro-phenylamino)-pyrimidin-4-yl]-methyl-amino}-methyl)-piperidine-1-carboxylic acid tert-butyl ester;

(A62) 4-({[6-(5-Carboxy-2-fluoro-phenylamino)-pyrimidin-4-yl]-methyl-amino}-methyl)-piperidine-1-carboxylic acid tert-butyl ester;

(A63) 4-({[6-(6-Methoxy-pyridin-3-ylamino)-pyrimidin-4-yl]-methyl-amino}-methyl)-piperidine-1-carboxylic acid tert-butyl ester;

(A64) 4-({[6-(2,6-Dimethoxy-pyridin-3-ylamino)-pyrimidin-4-yl]-methyl-amino}-methyl)-piperidine-1-carboxylic acid tert-butyl ester;

(A65) 6-{6-[(1-tert-Butoxycarbonyl-piperidin-4-ylmethyl)-methyl-amino]-pyrimidin-4-ylamino}-nicotinic acid;

(A66) 4-({[6-(6-Acetylamino-pyridin-3-ylamino)-pyrimidin-4-yl]-methyl-amino}-methyl)-piperidine-1-carboxylic acid tert-butyl ester;

(A67) 4-({[6-(5-Fluoro-pyridin-2-ylamino)-pyrimidin-4-yl]-methyl-amino}-methyl)-piperidine-1-carboxylic acid tert-butyl ester;

(A68) 4-({[6-(4-Cyano-2-ethyl-phenylamino)-pyrimidin-4-yl]-methyl-amino}-methyl)-piperidine-1-carboxylic acid tert-butyl ester;

(A69) 4-({[6-(4-Butyryl-phenylamino)-pyrimidin-4-yl]-methyl-amino}-methyl)-piperidine-1-carboxylic acid tert-butyl ester;

(A70) 4-({[6-(5-Bromo-3-methyl-pyridin-2-ylamino)-pyrimidin-4-yl]-methyl-amino}-methyl)-piperidine-1-carboxylic acid tert-butyl ester;

(A71) 4-({[6-(3-Bromo-5-methyl-pyridin-2-ylamino)-pyrimidin-4-yl]-methyl-amino}-methyl)-piperidine-1-carboxylic acid tert-butyl ester;

(A72) 4-({Methyl-[6-(5-trifluoromethyl-pyridin-2-ylamino)-pyrimidin-4-yl]-amino}-methyl)-piperidine-1-carboxylic acid tert-butyl ester;

(A73) 4-({[6-(4-Bromo-2-fluoro-phenylamino)-pyrimidin-4-yl]-methyl-amino}-methyl)-piperidine-1-carboxylic acid tert-butyl ester;

(A74) 4-({[6-(3-Carboxy-4-fluoro-phenylamino)-pyrimidin-4-yl]-methyl-amino}-methyl)-piperidine-1-carboxylic acid tert-butyl ester;
(A75) 4-({[6-(4-Ethoxycarbonyl-2-fluoro-phenylamino)-pyrimidin-4-yl]-methyl-amino}-methyl)-piperidine-1-carboxylic acid isobutyl ester;
(A76) 4-({[6-(4-Carboxy-2-fluoro-phenylamino)-pyrimidin-4-yl]-methyl-amino}-methyl)-piperidine-1-carboxylic acid isobutyl ester;
(A77) 4-({[6-(4-Cyano-2-fluoro-phenylamino)-pyrimidin-4-yl]-methyl-amino}-methyl)-piperidine-1-carboxylic acid isopropyl ester;
(A78) 4-({[6-(4-Cyano-2-fluoro-phenylamino)-pyrimidin-4-yl]-methyl-amino}-methyl)-piperidine-1-carboxylic acid butyl ester;
(A79) 4-({[6-(4-Cyano-2-fluoro-phenylamino)-pyrimidin-4-yl]-methyl-amino}-methyl)-piperidine-1-carboxylic acid cyclopropylmethyl ester;
(A82) 4-({[6-(2,5-Difluoro-4-hydroxy-phenylamino)-pyrimidin-4-yl]-methyl-amino}-methyl)-piperidine-1-carboxylic acid isobutyl ester;
(A83) 4-({[6-(4-Ethylcarbamoyl-2-fluoro-phenylamino)-pyrimidin-4-yl]-methyl-amino}-methyl)-piperidine-1-carboxylic acid isobutyl ester;
(A84) 4-[({6-[2-Fluoro-4-(N-hydroxycarbamimidoyl)-phenylamino]-pyrimidin-4-yl}-methyl-amino)-methyl]-piperidine-1-carboxylic acid isobutyl ester;
(A85) 4-({[6-(2-Fluoro-4-methanesulfonyl-phenylamino)-pyrimidin-4-yl]-methyl-amino}-methyl)-piperidine-1-carboxylic acid 3-methyl-butyl ester;
(A86) 4-({[6-(2,5-Difluoro-4-methanesulfonyl-phenylamino)-pyrimidin-4-yl]-methyl-amino}-methyl)-piperidine-1-carboxylic acid tert-butyl ester;
(A87) 4-({[6-(2-Fluoro-4-methanesulfonyl-phenylamino)-pyrimidin-4-yl]-methyl-amino}-methyl)-piperidine-1-carboxylic acid isopropyl ester;
(A88) (5-Butyl-pyridin-2-yl)-[4-({[6-(2-fluoro-4-methanesulfonyl-phenylamino)-pyrimidin-4-yl]-methyl-amino}-methyl)-piperidin-1-yl]-methanone;
(A89) N-(2-Fluoro-4-methanesulfonyl-phenyl)-N'-(5'-fluoro-3,4,5,6-tetrahydro-2H-[1,2']bipyridinyl-4-ylmethyl)-N'-methyl-pyrimidine-4,6-diamine;
(A90) 4-({[6-(4-Carbamimidoyl-2-fluoro-phenylamino)-pyrimidin-4-yl]-methyl-amino}-methyl)-piperidine-1-carboxylic acid isobutyl ester;
(A91) 4-({[6-(2-Fluoro-4-methanesulfonyl-phenylamino)-pyrimidin-4-yl]-methyl-amino}-methyl)-piperidine-1-carboxylic acid cyclobutyl ester;
(A92) 4-[6-(2-Fluoro-4-methanesulfonyl-phenylamino)-pyrimidin-4-ylamino]-piperidine-1-carboxylic acid tert-butyl ester;
(A94) 4-({[6-(2-Fluoro-4-methanesulfonyl-phenylamino)-pyrimidin-4-yl]-methyl-amino}-methyl)-piperidine-1-carboxylic acid 1-ethyl-propyl ester;
(A95) 4-({Ethyl-[6-(2-fluoro-4-methanesulfonyl-phenylamino)-pyrimidin-4-yl]-amino}-methyl)-piperidine-1-carboxylic acid tert-butyl ester;
(A96) 4-({Ethyl-[6-(2-fluoro-4-methanesulfonyl-phenylamino)-pyrimidin-4-yl]-amino}-methyl)-piperidine-1-carboxylic acid isopropyl ester;
(A97) 4-({[6-(4-Cyano-2,5-difluoro-phenylamino)-pyrimidin-4-yl]-ethyl-amino}-methyl)-piperidine-1-carboxylic acid isopropyl ester;
(A98) 4-({[6-(4-Amino-2,5-difluoro-phenoxy)-pyrimidin-4-yl]-ethyl-amino}-methyl)-piperidine-1-carboxylic acid tert-butyl ester;
(A99) 4-({[6-(2,5-Difluoro-4-methoxy-phenylamino)-pyrimidin-4-yl]-ethyl-amino}-methyl)-piperidine-1-carboxylic acid tert-butyl ester;
(A100) 4-({[6-(2,5-Difluoro-4-methanesulfonyl-phenylamino)-pyrimidin-4-yl]-ethyl-amino}-methyl)-piperidine-1-carboxylic acid tert-butyl ester;
(A101) 4-({Ethyl-[6-(2,4,5-trifluoro-phenylamino)-pyrimidin-4-yl]-amino}-methyl)-piperidine-1-carboxylic acid tert-butyl ester;
(A103) 4-[(Ethyl-{6-[4-(N-ethylcarbamimidoyl)-2,5-difluoro-phenylamino]-pyrimidin-4-yl}-amino)-methyl]-piperidine-1-carboxylic acid isopropyl ester;
(A104) 4-({[6-(4-Bromo-2,5-difluoro-phenylamino)-pyrimidin-4-yl]-ethyl-amino}-methyl)-piperidine-1-carboxylic acid tert-butyl ester;
(A105) 4-[({6-[5-(2-Amino-ethylamino)-4-cyano-2-fluoro-phenylamino]-pyrimidin-4-yl}-ethyl-amino)-methyl]-piperidine-1-carboxylic acid isopropyl ester;
(A120) 4-{[6-(2-Fluoro-4-methanesulfonyl-phenylamino)-pyrimidin-4-ylamino]-methyl}-piperidine-1-carboxylic acid tert-butyl ester;

(A121) 4-{[6-(2-Fluoro-4-methanesulfonyl-phenylamino)-pyrimidin-4-ylamino]-methyl}-piperidine-1-carboxylic acid isopropyl ester;
(A122) 4-({[6-(2-Fluoro-4-methanesulfonyl-phenylamino)-pyrimidin-4-yl]-isopropyl-amino}-methyl)-piperidine-1-carboxylic acid tert-butyl ester; and
(A123) 4-({[4-(2-Fluoro-4-methanesulfonyl-phenylamino)-pyridin-2-yl]-methyl-amino}-methyl)-piperidine-1-carboxylic acid isobutyl ester.

**20.** A compound according to claim 1, wherein said compound is selected from the following compounds and pharmaceutically acceptable salts, solvates, hydrates and N-oxides thereof:

(B1) 4-[6-(2-Fluoro-4-methanesulfonyl-phenylamino)-pyrimidin-4-yloxy]-piperidine-1-carboxylic acid tert-butyl ester;
(B3) 4-[6-(2-Fluoro-4-methanesulfonyl-phenylamino)-pyrimidin-4-yloxy]-piperidine-1-carboxylic acid isopropyl ester;
(B4) (6-Chloro-pyridin-2-yl)-{4-[6-(2-fluoro-4-methanesulfonyl-phenylamino)-pyrimidin-4-yloxy]-piperidin-1-yl}-methanone;
(B5) (6-Bromo-pyridin-2-yl)-{4-[6-(2-fluoro-4-methanesulfonyl-phenylamino)-pyrimidin-4-yloxy]-piperidin-1-yl}-methanone;
(B6) {4-[6-(2-Fluoro-4-methanesulfonyl-phenylamino)-pyrimidin-4-yloxy]-piperidin-1-yl}-(6-methyl-pyridin-2-yl)-methanone;
(B7) {4-[6-(2-Fluoro-4-methanesulfonyl-phenylamino)-pyrimidin-4-yloxy]-piperidin-1-yl}-(6-fluoro-pyridin-2-yl)-methanone;
(B8) {4-[6-(2-Fluoro-4-methanesulfonyl-phenylamino)-pyrimidin-4-yloxy]-piperidin-1-yl}-pyridin-2-yl-methanone;
(B9) (5-Bromo-pyridin-3-yl)-{4-[6-(2-fluoro-4-methanesulfonyl-phenylamino)-pyrimidin-4-yloxy]-piperidin-1-yl}-methanone;
(B10) {4-[6-(2-Fluoro-4-methanesulfonyl-phenylamino)-pyrimidin-4-yloxy]-piperidin-1-yl}-(5-methyl-pyridin-3-yl)-methanone;
(B11) (5,6-Dichloro-pyridin-3-yl)-{4-[6-(2-fluoro-4-methanesulfonyl-phenylamino)-pyrimidin-4-yloxy]-piperidin-1-yl}-methanone;
(B12) 4-[6-(4-Cyano-2,5-difluoro-phenylamino)-pyrimidin-4-yloxy]-piperidine-1-carboxylic acid tert-butyl ester;
(B13) 4-[6-(2,5-Difluoro-4-methanesulfonyl-phenylamino)-pyrimidin-4-yloxy]-piperidine-1-carboxylic acid tert-butyl ester;
(B14) 4-[6-(2,4,5-Trifluoro-phenylamino)-pyrimidin-4-yloxy]-piperidine-1-carboxylic acid tert-butyl ester;
(B15) 4-[6-(4-Bromo-2,5-difluoro-phenylamino)-pyrimidin-4-yloxy]-piperidine-1-carboxylic acid tert-butyl ester;
(B16) 4-[6-(3-Fluoro-4-methyl-phenylamino)-pyrimidin-4-yloxy]-piperidine-1-carboxylic acid tert-butyl ester;
(B17) 4-[6-(3-Hydroxy-4-methoxy-phenylamino)-pyrimidin-4-yloxy]-piperidine-1-carboxylic acid tert-butyl ester;
(B18) 4-[6-(6-Cyano-pyridin-3-ylamino)-pyrimidin-4-yloxy]-piperidine-1-carboxylic acid tert-butyl ester;
(B19) 4-[6-(3-Chloro-4-cyano-phenylamino)-pyrimidin-4-yloxy]-piperidine-1-carboxylic acid tert-butyl ester;
(B20)4-[6-(6-Chloro-pyridin-3-ylamino)-pyrimidin-4-yloxy]-piperidine-1-carboxylic acid tert-butyl ester;
(B21) 4-[6-(3-Fluoro-4-methoxy-phenylamino)-pyrimidin-4-yloxy]-piperidine-1-carboxylic acid tert-butyl ester;
(B22) 4-[6-(3,4-Dimethoxy-phenylamino)-pyrimidin-4-yloxy]-piperidine-1-carboxylic acid tert-butyl ester;
(B23) 4-[6-(2,3-Dihydro-benzo[1,4]dioxin-6-ylamino)-pyrimidin-4-yloxy]-piperidine-1-carboxylic acid tert-butyl ester;
(B24) 4-[6-(4-Cyano-2,5-difluoro-phenylamino)-pyrimidin-4-yloxy]-piperidine-1-carboxylic acid isopropyl ester;
(B25) 4-[6-(4-Cyano-5-ethylamino-2-fluoro-phenylamino)-pyrimidin-4-yloxy]-piperidine-1-carboxylic acid tert-butyl ester;
(B26) 4-[6-(4-Ethoxy-2,5-difluoro-phenylamino)-pyrimidin-4-yloxy]-piperidine-1-carboxylic acid isopropyl ester;
(B27) 4-[6-(4-Ethylsulfanyl-phenylamino)-pyrimidin-4-yloxy]-piperidine-1-carboxylic acid tert-butyl ester;
(B28) 4-[6-(4-Isopropylsulfanyl-phenylamino)-pyrimidin-4-yloxy]-piperidine-1-carboxylic acid tert-butyl ester;
(B29) (5-Butyl-pyridin-2-yl)-{4-[6-(2-fluoro-4-methanesulfonyl-phenylamino)-pyrimidin-4-yloxy]-piperidin-1-yl}-methanone;
(B30) 4-[6-(5-Chloro-3-methyl-pyridin-2-ylamino)-pyrimidin-4-yloxy]-piperidine-1-carboxylic acid tert-butyl ester;
(B31) 4-[6-(6-Acetylamino-4-methyl-pyridin-3-ylamino)-pyrimidin-4-yloxy]-piperidine-1-carboxylic acid tert-butyl ester;
(B32) 4-[6-(5-Fluoro-4-methyl-pyridin-2-ylamino)-pyrimidin-4-yloxy]-piperidine-1-carboxylic acid tert-butyl ester;

(B33) 4-[6-(6-Methoxy-5-methyl-pyridin-3-ylamino)-pyrimidin-4-yloxy]-piperidine-1-carboxylic acid tert-butyl ester;
(B34) 4-[6-(6-Methoxy-2-methyl-pyridin-3-ylamino)-pyrimidin-4-yloxy]-piperidine-1-carboxylic acid tert-butyl ester;
(B35) 4-[6-(6-Fluoro-5-methyl-pyridin-3-ylamino)-pyrimidin-4-yloxy]-piperidine-1-carboxylic acid tert-butyl ester;
(B36) 4-[6-(2-Chloro-6-methyl-pyridin-3-ylamino)-pyrimidin-4-yloxy]-piperidine-1-carboxylic acid tert-butyl ester;
(B37) 4-[6-(4-Methyl-pyridin-3-ylamino)-pyrimidin-4-yloxy]-piperidine-1-carboxylic acid tert-butyl ester;
(B38) 4-[6-(2-Methyl-pyridin-3-ylamino)-pyrimidin-4-yloxy]-piperidine-1-carboxylic acid tert-butyl ester;
(B39) 4-[6-(6-Chloro-2-methyl-pyridin-3-ylamino)-pyrimidin-4-yloxy]-piperidine-1-carboxylic acid tert-butyl ester;
(B40) 4-[6-(6-Fluoro-pyridin-3-ylamino)-pyrimidin-4-yloxy]-piperidine-1-carboxylic acid tert-butyl ester;
(B41) 4-[6-(2-Chloro-4-methyl-pyridin-3-ylamino)-pyrimidin-4-yloxy]-piperidine-1-carboxylic acid tert-butyl ester;
(B42) 4-[6-(6-Methoxy-pyridin-3-ylamino)-pyrimidin-4-yloxy]-piperidine-1-carboxylic acid tert-butyl ester;
(B43) 4-[6-(5-Fluoro-pyridin-2-ylamino)-pyrimidin-4-yloxy]-piperidine-1-carboxylic acid tert-butyl ester;
(B44) 4-[6-(2-Fluoro-pyridin-3-ylamino)-pyrimidin-4-yloxy]-piperidine-1-carboxylic acid tert-butyl ester;
(B45) 4-[6-(6-Chloro-5-methyl-pyridin-3-ylamino)-pyrimidin-4-yloxy]-piperidine-1-carboxylic acid tert-butyl ester;
(B46) 4-[6-(2-Methyl-pyridin-4-ylamino)-pyrimidin-4-yloxy]-piperidine-1-carboxylic acid tert-butyl ester;
(B47) 4-[6-(2-Methoxy-pyridin-3-ylamino)-pyrimidin-4-yloxy]-piperidine-1-carboxylic acid tert-butyl ester;
(B48) 4-[6-(2,5-Difluoro-phenylamino)-pyrimidin-4-yloxy]-piperidine-1-carboxylic acid tert-butyl ester;
(B49) 4-[6-(4-Chloro-2-fluoro-phenylamino)-pyrimidin-4-yloxy]-piperidine-1-carboxylic acid tert-butyl ester;
(B50) 4-[6-(2,5-Difluoro-phenylamino)-pyrimidin-4-yloxy]-piperidine-1-carboxylic acid isopropyl ester;
(B51) 4-[6-(6-Methoxy-pyridin-3-ylamino)-pyrimidin-4-yloxy]-piperidine-1-carboxylic acid isopropyl ester;
(B52) 4-[6-(4-Cyano-3-methoxy-phenylamino)-pyrimidin-4-yloxy]-piperidine-1-carboxylic acid isopropyl ester;
(B53) 4-[6-(3-Fluoro-4-hydroxy-phenylamino)-pyrimidin-4-yloxy]-piperidine-1-carboxylic acid isopropyl ester;
(B54) 4-[6-(6-Ethoxy-pyridin-3-ylamino)-pyrimidin-4-yloxy]-piperidine-1-carboxylic acid isopropyl ester;
(B55) 4-[6-(2,5-Difluoro-4-isopropoxy-phenylamino)-pyrimidin-4-yloxy]-piperidine-1-carboxylic acid isopropyl ester;
(B56) (2-Fluoro-4-methanesulfonyl-phenyl)-[6-(5'-isopropoxy-3,4,5,6-tetrahydro-2H-[1,2']bipyridinyl-4-yloxy)-pyrimidin-4-yl]-amine;
(B57) (2-Fluoro-4-methanesulfonyl-phenyl)-{6-[1-(3-isopropyl-[1,2,4]oxadiazol-5-yl)-piperidin-4-yloxy]-pyrimidin-4-yl}-amine;
(B58) 4-[6-(4-Cyano-2-fluoro-phenylamino)-pyrimidin-4-yloxy]-piperidine-1-carboxylic acid isopropyl ester;
(B59) 4-[6-(Pyridin-3-ylamino)-pyrimidin-4-yloxy]-piperidine-1-carboxylic acid isopropyl ester;
(B60) 4-[6-(Pyridin-4-ylamino)-pyrimidin-4-yloxy]-piperidine-1-carboxylic acid isopropyl ester;
(B61) 4-[6-(2,5-Difluoro-4-propoxy-phenylamino)-pyrimidin-4-yloxy]-piperidine-1-carboxylic acid isopropyl ester;
(B62) 4-[6-(4-Ethylamino-2-fluoro-phenylamino)-pyrimidin-4-yloxy]-piperidine-1-carboxylic acid isopropyl ester;
(B63) 4-[6-(4-Dimethylamino-2-fluoro-phenylamino)-pyrimidin-4-yloxy]-piperidine-1-carboxylic acid isopropyl ester;
(B64) 4-[6-(2-Fluoro-4-propylamino-phenylamino)-pyrimidin-4-yloxy]-piperidine-1-carboxylic acid isopropyl ester;
(B65) 4-[6-(2-Fluoro-4-isopropylamino-phenylamino)-pyrimidin-4-yloxy]-piperidine-1-carboxylic acid isopropyl ester;
(B66) 4-[6-(2-Methyl-6-propylamino-pyridin-3-ylamino)-pyrimidin-4-yloxy]-piperidine-1-carboxylic acid isopropyl ester;
(B67) 4-[6-(2-Methyl-pyridin-3-ylamino)-pyrimidin-4-yloxy]-piperidine-1-carboxylic acid isopropyl ester;
(B68) 4-[6-(6-Isopropylamino-2-methyl-pyridin-3-ylamino)-pyrimidin-4-yloxy]-piperidine-1-carboxylic acid isopropyl ester;
(B69) 4-[6-(2-Methyl-6-propoxy-pyridin-3-ylamino)-pyrimidin-4-yloxy]-piperidine-1-carboxylic acid isopropyl ester;
(B70) 4-[6-(4-Iodo-2-methyl-phenylamino)-pyrimidin-4-yloxy]-piperidine-1-carboxylic acid isopropyl ester;
(B71) 4-[6-(2-Fluoro-4-iodo-phenylamino)-pyrimidin-4-yloxy]-piperidine-1-carboxylic acid isopropyl ester;
(B77) 4-[6-(6-Methoxy-5-methyl-pyridin-3-ylamino)-pyrimidin-4-yloxy]-piperidine-1-carboxylic acid isopropyl ester;

(B78) 4-[6-(4-Methyl-pyridin-3-ylamino)-pyrimidin-4-yloxy]-piperidine-1-carboxylic acid isopropyl ester;
(B79) 4-[6-(4-Acetylamino-3-methyl-phenylamino)-pyrimidin-4-yloxy]-piperidine-1-carboxylic acid isopropyl ester;
(B80) 4-[6-(3-Chloro-4-fluoro-phenylamino)-pyrimidin-4-yloxy]-piperidine-1-carboxylic acid isopropyl ester;
(B81) 4-[6-(3,5-Dimethoxy-phenylamino)-pyrimidin-4-yloxy]-piperidine-1-carboxylic acid isopropyl ester;
(B82) 4-[6-(6-Ethyl-pyridin-2-ylamino)-pyrimidin-4-yloxy]-piperidine-1-carboxylic acid isopropyl ester;
(B83) 4-[6-(5-Methyl-pyridin-2-ylamino)-pyrimidin-4-yloxy]-piperidine-1-carboxylic acid isopropyl ester;
(B84) 4-[6-(2-Methyl-quinolin-6-ylamino)-pyrimidin-4-yloxy]-piperidine-1-carboxylic acid isopropyl ester;
(B85) 4-[6-(2-Methylsulfanyl-benzothiazol-6-ylamino)-pyrimidin-4-yloxy]-piperidine-1-carboxylic acid isopropyl ester;
(B86) 4-[6-(6-Morpholin-4-yl-pyridin-3-ylamino)-pyrimidin-4-yloxy]-piperidine-1-carboxylic acid isopropyl ester;
(B88) 4-[6-(4-Piperidin-1-yl-phenylamino)-pyrimidin-4-yloxy]-piperidine-1-carboxylic acid isopropyl ester;
(B89) 4-[6-(3-Trifluoromethoxy-phenylamino)-pyrimidin-4-yloxy]-piperidine-1-carboxylic acid isopropyl ester;
(B90) 4-[6-(5-Oxo-5,6,7,8-tetrahydro-naphthalen-2-ylamino)-pyrimidin-4-yloxy]-piperidine-1-carboxylic acid isopropyl ester;
(B92) 4-[6-(5-Cyano-pyridin-2-ylamino)-pyrimidin-4-yloxy]-piperidine-1-carboxylic acid isopropyl ester;
(B93) 4-[6-(4-Bromo-2,5-difluoro-phenylamino)-pyrimidin-4-yloxy]-piperidine-1-carboxylic acid isopropyl ester;
(B94) 4-[6-(4-Trifluoromethyl-pyridin-2-ylamino)-pyrimidin-4-yloxy]-piperidine-1-carboxylic acid isopropyl ester;
(B97) 4-[6-(2,6-Dimethyl-pyridin-3-ylamino)-pyrimidin-4-yloxy]-piperidine-1-carboxylic acid isopropyl ester;
(B98) 4-[6-(4-Cyano-2-methyl-phenylamino)-pyrimidin-4-yloxy]-piperidine-1-carboxylic acid isopropyl ester;
(B99) 4-[6-(4-Methoxy-2-methyl-phenylamino)-pyrimidin-4-yloxy]-piperidine-1-carboxylic acid isopropyl ester;
(B100) 4-[6-(2,4-Dimethoxy-phenylamino)-pyrimidin-4-yloxy]-piperidine-1-carboxylic acid isopropyl ester;
(B101) 4-{6-[Acetyl-(2-fluoro-4-methanesulfonyl-phenyl)-amino]-pyrimidin-4-yloxy}-piperidine-1-carboxylic acid isopropyl ester;
(B102) 4-[6-(5-Carbamoyl-pyridin-2-ylamino)-pyrimidin-4-yloxy]-piperidine-1-carboxylic acid isopropyl ester;
(B105) 4-[6-(3-Oxazol-5-yl-phenylamino)-pyrimidin-4-yloxy]-piperidine-1-carboxylic acid isopropyl ester;
(B106) 4-[6-(5-Trifluoromethyl-pyridin-2-ylamino)-pyrimidin-4-yloxy]-piperidine-1-carboxylic acid isopropyl ester;
(B107) 4-[6-(4-Chloro-2-trifluoromethoxy-phenylamino)-pyrimidin-4-yloxy]-piperidine-1-carboxylic acid isopropyl ester;
(B110) 4-[6-(1-Oxo-indan-5-ylamino)-pyrimidin-4-yloxy]-piperidine-1-carboxylic acid isopropyl ester;
(B111) 4-{6-[5-(1-Methyl-pyrrolidin-2-yl)-pyridin-2-ylamino]-pyrimidin-4-yloxy}-piperidine-1-carboxylic acid isopropyl ester;
(B112) 4-[6-(6-Methoxy-2-methyl-pyridin-3-ylamino)-pyrimidin-4-yloxy]-piperidine-1-carboxylic acid isopropyl ester;
(B113) 4-[6-(5-Bromo-3-methyl-pyridin-2-ylamino)-pyrimidin-4-yloxy]-piperidine-1-carboxylic acid isopropyl ester;
(B114) 4-[6-(2-Chloro-6-methyl-pyridin-3-ylamino)-pyrimidin-4-yloxy]-piperidine-1-carboxylic acid isopropyl ester;
(B115)4-[6-(2-Ethynyl-phenylamino)-pyrimidin-4-yloxy]-piperidine-1-carboxylic acid isopropyl ester;
(B116) 4-[6-(4-Bromo-2-trifluoromethoxy-phenylamino)-pyrimidin-4-yloxy]-piperidine-1-carboxylic acid isopropyl ester;
(B117) 4-[6-(3-Iodo-4-methyl-phenylamino)-pyrimidin-4-yloxy]-piperidine-1-carboxylic acid isopropyl ester;
(B118) 4-[6-(2-Fluoro-5-methyl-phenylamino)-pyrimidin-4-yloxy]-piperidine-1-carboxylic acid isopropyl ester;
(B122) 4-[6-(2,5-Difluoro-4-propylamino-phenylamino)-pyrimidin-4-yloxy]-piperidine-1-carboxylic acid isopropyl ester;
(B123) 4-[6-(2,5-Difluoro-4-morpholin-4-yl-phenylamino)-pyrimidin-4-yloxy]-piperidine-1-carboxylic acid isopropyl ester;
(B124) 4-[6-(2-Methyl-4-propylamino-phenylamino)-pyrimidin-4-yloxy]-piperidine-1-carboxylic acid isopropyl ester;
(B125) 4-{6-[2,5-Difluoro-4-(4-methyl-piperazin-1-yl)-phenylamino]-pyrimidin-4-yloxy}-piperidine-1-carboxylic acid isopropyl ester;
(B126) 4-{6-[2,5-Difluoro-4-(2-pyrrolidin-1-yl-ethoxy)-phenylamino]-pyrimidin-4-yloxy}-piperidine-1-carboxylic acid isopropyl ester;
(B127) 4-{6-[4-(2-Dimethylamino-ethoxy)-2,5-difluoro-phenylamino]-pyrimidin-4-yloxy}-piperidine-1-carboxylic acid isopropyl ester;
(B128) 4-{6-[2,5-Difluoro-4-(2-morpholin-4-yl-ethoxy)-phenylamino]-pyrimidin-4-yloxy}-piperidine-1-carboxylic acid isopropyl ester;

(B129) 4-[6-(2,4-Difluoro-phenylamino)-pyrimidin-4-yloxy]-piperidine-1-carboxylic acid isopropyl ester;
(B130) 4-[6-(2,4,5- Trifluoro-phenylamino)-pyrimidin-4-yloxy]-piperidine-1-carboxylic acid isopropyl ester;
(B131) 4-[6-(4-Methanesulfonyl-phenylamino)-pyrimidin-4-yloxy]-piperidine-1-carboxylic acid isopropyl ester;
(B132)4-{6-[Acetyl-(4-methanesulfonyl-phenyl)-amino]-pyrimidin-4-yloxy}-piperidine-1-carboxylic acid isopropyl ester;
(B133) (2,5-Difluoro-4-propoxy-phenyl)-{6-[1-(5-isopropyl-[1,2,4]oxadiazol-3-yl)-piperidin-4-yloxy]-pyrimidin-4-yl}-amine;
(B134) 4-{6-[2,5-Difluoro-4-(morpholin-4-ylamino)-phenylamino]-pyrimidin-4-yloxy}-piperidine-1-carboxylic acid isopropyl ester;
(B135) 4-{6-[2,5-Difluoro-4-(2-methoxy-ethylamino)-phenylamino]-pyrimidin-4-yloxy}-piperidine-1-carboxylic acid isopropyl ester;
(B136) 4-(6-{2,5-Difluoro-4-[(tetrahydro-furan-2-ylmethyl)-amino]-phenylamino}-pyrimidin-4-yloxy)-piperidine-1-carboxylic acid isopropyl ester;
(B137) 4-[6-(4-Butylamino-2,5-difluoro-phenylamino)-pyrimidin-4-yloxy]-piperidine-1-carboxylic acid isopropyl ester;
(B138) 4-{6-[2,5-Difluoro-4-(3-methyl-butylamino)-phenylamino]-pyrimidin-4-yloxy}-piperidine-1-carboxylic acid isopropyl ester;
(B139) 4-[6-(2-Fluoro-4-methanesulfonyl-phenylamino)-2-methyl-pyrimidin-4-yloxy]-piperidine-1-carboxylic acid isopropyl ester;
(B140) 4-{6-[2,5-Difluoro-4-(2-morpholin-4-yl-ethylamino)-phenylamino]-pyrimidin-4-yloxy}-piperidine-1-carboxylic acid isopropyl ester;
(B141) 4-{6-[2-(2,5-Difluoro-phenoxy)-ethylamino]-pyrimidin-4-yloxy}-piperidine-1-carboxylic acid isopropyl ester;
(B142) 4-[6-(2,5-Difluoro-phenoxy)-pyrimidin-4-yloxy]-piperidine-1-carboxylic acid isopropyl ester;
(B143) 4-[6-(4-Bromo-2-fluoro-phenoxy)-pyrimidin-4-yloxy]-piperidine-1-carboxylic acid isopropyl ester;
(B144)4-[6-(2-Fluoro-4-morpholin-4-yl-phenoxy)-pyrimidin-4-yloxy]-piperidine-1-carboxylic acid isopropyl ester;
(B145) 4-{6-[2,5-Difluoro-4-(tetrahydro-furan-2-ylmethoxy)-phenylamino]-pyrimidin-4-yloxy}-piperidine-1-carboxylic acid isopropyl ester;
(B149) 4-[4-(2-Fluoro-4-methanesulfonyl-phenylamino)-pyridin-2-yloxy]-piperidine-1-carboxylic acid isopropyl ester; and
(B150) 4-[4-(2,5-Difluoro-4-propoxy-phenylamino)-pyridin-2-yloxy]-piperidine-1-carboxylic acid isopropyl ester.

**21.** A compound according to claim 1, wherein said compound is selected from the following compounds and pharmaceutically acceptable salts, solvates, hydrates and N-oxides thereof:

(B153) 4-{6-[6-(2,3-Dihydroxy-propylamino)-2-methyl-pyridin-3-yloxy]-pyrimidin-4-yloxy}-piperidine-1-carboxylic acid isopropyl ester;
(B155) 4-[6-(2-Methyl-pyridin-3-yloxy)-pyrimidin-4-yloxy]-piperidine-1-carboxylic acid isopropyl ester;
(B156) 4-[6-(6-Bromo-2-methyl-pyridin-3-yloxy)-pyrimidin-4-yloxy]-piperidine-1-carboxylic acid isopropyl ester; and
(B157) 4-[6-(2-Methyl-pyridin-3-yloxy)-pyrimidin-4-yloxy]-piperidine-1-carboxylic acid tert-butyl ester.

**22.** A compound selected from compounds of Formula (I) and pharmaceutically acceptable salts, solvates, hydrates and N-oxides thereof:

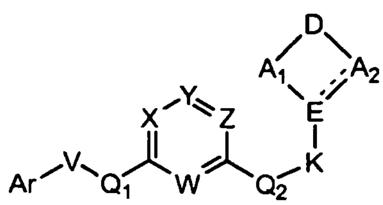

**(I)**

wherein:

$A_1$ and $A_2$ are independently $C_{1-3}$ alkylene optionally substituted with one or more substituents selected inde-

pendently from $C_{1-6}$ alkyl, $C_{1-6}$ alkoxy, and carboxy;
D is $CR_1R_2$, wherein $R_1$ is selected from H, $C_{1-6}$ alkyl, $C_{1-6}$ alkoxy, halogen and hydroxyl;
E is N, C or $CR_3$, wherein $R_3$ is H or $C_{1-6}$ alkyl;
- - - is a single bond when E is N or $CR_3$, or a double bond when E is C;
K is absent, $C_{3-6}$ cycloalkylene, or $C_{1-3}$ alkylene group each optionally substituted with one or more substituents selected independently from $C_{1-6}$ alkyl, $C_{1-6}$ alkoxy, carboxy, cyano, and halogen;
$Q_1$ is NH;
$Q_2$ is absent;
W is CH;
X is N or $CR_6$;
Y is N or $CR_7$;
Z is N;
V is absent, $C_{1-3}$ heteroalkylene, or $C_{1-3}$ alkylene wherein each are optionally substituted with one or more substituents selected independently from $C_{1-3}$ alkyl, $C_{1-6}$ alkoxy, carboxy, cyano, $C_{1-3}$ haloalkyl, and halogen;
$R_6$, $R_7$, and $R_8$ are each independently selected from H, $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino and nitro, wherein said $C_{2-6}$ alkenyl, $C_{1-6}$ alkyl, $C_{2-6}$ alkynyl and $C_{3-6}$ cycloalkyl are each optionally substituted with one or more substituents independently selected from $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino and nitro;
Ar is phenyl, naphthyl, pyridyl, benzofuranyl, pyrazinyl, pyridazinyl, pyrimidinyl, triazinyl, quinoline, benzoxazole, benzothiazole, 1*H*-benzimidazole, isoquinoline, quinazoline, quinoxaline, pyrrole, or indole, optionally substituted with $R_9$, $R_{10}$, $R_{11}$, $R_{12}$, and $R_{13}$;
$R_9$ is selected from $C_{1-6}$ acyl, $C_{1-6}$ acylsulfonamide, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, aryl, arylcarbonyl, arylsulfonyl, di-$C_{1-6}$-alkylamino, carbamimidoyl, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, guanidine, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, heterocyclic, heterocyclicsulfonyl, heteroaryl, hydroxyl, hydroxylamino, nitro, $C_{3-6}$ oxo-cycloalkyl, phenoxy, sulfonamide, sulfonic acid and thiol; and wherein each $R_9$ is optionally substituted with one or more substituents selected independently from $C_{1-6}$ acyl, $C_{1-6}$ acylsulfonamide, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, aryl, arylcarbonyl, arylsulfonyl, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, heteroaryl, heteroarylcarbonyl, heteroarylsulfonyl, heterocyclic, hydroxyl, hydroxylamino, and nitro;
$R_{10}$, $R_{11}$, $R_{12}$, and $R_{13}$ are independently selected from $C_{1-6}$ acyl, $C_{1-6}$ acyloxy, $C_{2-6}$ alkenyl, $C_{1-6}$ alkoxy, $C_{1-6}$ alkyl, $C_{1-6}$ alkylamino, $C_{1-6}$ alkylcarboxamide, $C_{2-6}$ alkynyl, $C_{1-6}$ alkylsulfonamide, $C_{1-6}$ alkylsulfinyl, $C_{1-6}$ alkylsulfonyl, $C_{1-6}$ alkylthio, $C_{1-6}$ alkylthiocarboxamide, $C_{1-6}$ alkylthioureyl, $C_{1-6}$ alkylureyl, amino, di-$C_{1-6}$-alkylamino, $C_{1-6}$ alkoxycarbonyl, carboxamide, carboxy, cyano, $C_{3-6}$ cycloalkyl, di-$C_{1-6}$-alkylcarboxamide, di-$C_{1-6}$-alkylsulfonamide, di-$C_{1-6}$-alkylthiocarboxamido, $C_{1-6}$ haloalkoxy, $C_{1-6}$ haloalkyl, halogen, $C_{1-6}$ haloalkylsulfinyl, $C_{1-6}$ haloalkylsulfonyl, $C_{1-6}$ haloalkylthio, hydroxyl, hydroxylamino, nitro, and thiol; or two adjacent $R_{10}$, $R_{11}$, $R_{12}$, and $R_{13}$ groups together with the atoms to which they are bonded form a 5, 6 or 7 member cycloalkyl, 5, 6 or 7 member cycloalkenyl, or 5, 6 or 7 member heterocyclic group wherein the 5, 6 or 7 member group is optionally substituted with halogen or oxo; and
$R_2$ is selected from $C_{1-6}$ alkyl, aryl, aryloxy, heteroaryl, and heteroaryloxy;

wherein $R_2$ is optionally substituted with 1, 2, 3, 4, or 5 substituents selected independently from $C_{1-6}$ alkoxy, $C_{1-6}$ alkyl, $C_{1-6}$ alkylamino, amino, di-$C_{1-6}$-alkylamino, $C_{1-6}$ alkoxycarbonyl, carboxy, halogen, and heteroaryl, and wherein

$C_{1-6}$ alkyl is further optionally substituted with 1, 2, or 3 substituents selected independently from $C_{1-6}$ alkylamino, di-$C_{1-6}$-alkylamino, $C_{3-6}$ cycloalkyl, and halogen.

**23.** A compound according to claim 22, wherein K is absent.

**24.** A compound according to claim 22 or 23, wherein V is absent.

**25.** A compound according to any one of claims 22 to 24, wherein E is N and D is $CHR_2$.

**26.** A compound according to any one of claims 22 to 25, wherein $A_1$ and $A_2$ are both $-CH_2CH_2-$, and each $A_1$ and $A_2$ is optionally substituted with 1, 2, 3 or 4 methyl groups.

**27.** A compound according to any one of claims 22 to 26, wherein:

W is CH;
X is N;
Y is $CR_7$, wherein $R_7$ is H, $C_{1-6}$, alkyl, or halogen; and
Z is N.

**28.** A compound according to claim 22, wherein said compound is selected from the following compounds and pharmaceutically acceptable salts, solvates, hydrates and N-oxides thereof:

(A2) {6-[4-(3-Isopropyl-[1,2,4]oxadiazol-5-yl)-piperidin-1-yl]-pyrimidin-4-yl}-(4-methanesulfonyl-phenyl)-amine;
(A8) (2-Fluoro-4-methanesulfonyl-phenyl)-{6-[4-(3-fluoro-phenoxy)-piperidin-1-yl]-pyrimidin-4-yl}-amine;
(A102) (2-Fluoro-4-methanesulfonyl-phenyl)-{6-[4-(3-isopropyl-[1,2,4]oxadiazol-5-yl)-piperidin-1-yl]-pyrimidin-4-yl}-amine;
(A108) (2-Fluoro-4-methanesulfonyl-phenyl)-{6-[4-(4-isobutyl-phenyl)-piperidin-1-yl]-pyrimidin-4-yl}-amine;
(A109) (2-Fluoro-4-methanesulfonyl-phenyl)-{6-[4-(4-isopropyl-phenyl)-piperidin-1-yl]-pyrimidin-4-yl}-amine;
(A110) {6-[4-(3-Cyclopropylmethyl-[1,2,4]oxadiazol-5-yl)-piperidin-1-yl]-pyrimidin-4-yl}-(2-fluoro-4-methanesulfonyl-phenyl)-amine;
(A111) (2-Fluoro-4-methanesulfonyl-phenyl)-{6-[4-(3-isobutyl-[1,2,4]oxadiazol-5-yl)-piperidin-1-yl]-pyrimidin-4-yl}-amine;
(A113) (2-Fluoro-4-methanesulfonyl-phenyl)-{6-[4-(4-isopropoxy-phenyl)-piperidin-1-yl]-pyrimidin-4-yl}-amine;
(A115) {6-[4-(3-Dimethylaminomethyl-[1,2,4]oxadiazol-5-yl)-piperidin-1-yl]-pyrimidin-4-yl}-(2-fluoro-4-methanesulfonyl-phenyl)-amine;
(A117) (2-Fluoro-4-methanesulfonyl-phenyl)-{6-[4-(5-isopropoxy-pyridin-2-yloxy)-piperidin-1-yl]-pyrimidin-4-yl}-amine; and
(A118) (2-Fluoro-4-methanesulfonyl-phenyl)-{6-[4-(3-pyridin-3-yl-[1,2,4]oxadiazol-5-yl)-piperidin-1-yl]-pyrimidin-4-yl}-amine.

**29.** A pharmaceutical composition comprising at least one compound according to any one of claims 1 to 28 or 4-[6-(2-fluoro-4-methanesulfonyl-phenoxy)-pyrimidin-4-yloxy]-piperidine-1-carboxylic acid isopropyl ester, and a pharmaceutically acceptable carrier.

**30.** A method of producing a pharmaceutical composition comprising admixing at least one compound according to any one of claims 1 to 28 or 4-[6-(2-fluoro-4-methanesulfonyl-phenoxy)-pyrimidin-4-yloxy]-piperidine-1-carboxylic acid isopropyl ester, and a pharmaceutically acceptable carrier.

**31.** A compound according to any one of claims 1 to 28 or 4-[6-(2-fluoro-4-methanesulfonyl-phenoxy)-pyrimidin-4-yloxy]-piperidine-1-carboxylic acid isopropyl ester, for use in a method of treatment of the human or animal body by therapy.

**32.** A compound according to any one of claims 1 to 28 or 4-[6-(2-fluoro-4-methanesulfonyl-phenoxy)-pyrimidin-4-yloxy]-piperidine-1-carboxylic acid isopropyl ester, for use in a method of prophylaxis or treatment of a metabolic-related disorder.

**33.** Use of a compound according to any one of claims 1 to 28 or 4-[6-(2-fluoro-4-methanesulfonyl-phenoxy)-pyrimidin-4-yloxy]-piperidine-1-carboxylic acid isopropyl ester, in the production of a medicament for use in treatment of a

metabolic-related disorder.

**34.** A compound according to any one of claims 1 to 28 or 4-[6-(2-fluoro-4-methanesulfonyl-phenoxy)-pyrimidin-4-yloxy]-piperidine-1-carboxylic acid isopropyl ester, for use in a method of treatment of type I diabetes, type II diabetes, inadequate glucose tolerance, insulin resistance, hyperglycemia, hyperlipidemia, hypertriglyceridemia, hypercholesterolemia, dyslipidemia, or syndrome X.

**35.** Use of a compound according to any one of claims 1 to 28 or 4-[6-(2-fluoro-4-methanesulfonyl-phenoxy)-pyrimidin-4-yloxy]-piperidine-1-carboxylic acid isopropyl ester, in the production of a medicament for use in treatment of type I diabetes, type II diabetes, inadequate glucose tolerance, insulin resistance, hyperglycemia, hyperlipidemia, hypertriglyceridemia, hypercholesterolemia, dyslipidemia, or syndrome X.

**Patentansprüche**

**1.** Verbindung, ausgewählt aus Verbindungen der Formel (I) und pharmazeutisch annehmbaren Salzen, Solvaten, Hydraten und N-Oxiden davon:

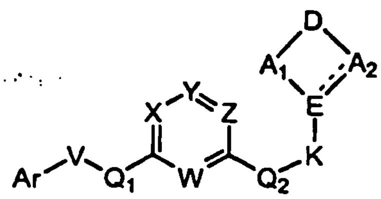

**(I)**

worin:

$A_1$ und $A_2$ beide $-CH_2CH_2-$ sind und $A_1$ und $A_2$ jeweils gegebenenfalls mit 1, 2, 3 oder 4 Methylgruppen substituiert sind;
D $CR_1R_2$ oder $NR_2$ ist, worin $R_1$ aus H, $C_{1-6}$-Alkyl, $C_{1-6}$-Alkoxy, Halogen und Hydroxyl ausgewählt ist;
E N oder $CR_3$ ist, worin $R_3$ H oder $C_{1-6}$-Alkyl ist;
- - - eine Einfachbindung ist;
K fehlt, eine $C_{3-6}$-Cycloalkylen- oder $C_{1-3}$-Alkylengruppe ist, die jeweils gegebenenfalls mit einem oder mehreren Substituenten substituiert sind, die unabhängig voneinander ausgewählt sind aus: $C_{1-6}$-Alkyl, $C_{1-6}$-Alkoxy, Carboxy, Cyano und Halogen;
$Q_1$ $NR_4$, O, S, S(O) oder $S(O)_2$ ist, worin $R_4$ H, $C_{1-6}$-Acyl, $C_{1-6}$-Alkyl, $C_{2-6}$-Alkenyl, $C_{2-6}$-Alkinyl, $C_{3-7}$-Cycloalkyl oder $C_{3-7}$-Cycloalkyl-$C_{1-3}$-alkylen ist, worin das $C_{1-6}$-Alkyl gegebenenfalls mit einem oder mehreren Substituenten substituiert ist, die unabhängig voneinander ausgewählt sind aus: $C_{1-6}$-Acyl, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkylcarboxamid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthiocarbox-amid, $C_{1-6}$-Alkylthioureyl, $C_{1-6}$-Alkylureyl, Amino, Di-$C_{1-6}$-alkylamino, $C_{1-6}$-Alkoxycarbonyl, Carboxamid, Carboxy, Cyano, $C_{3-6}$-Cycloalkyl, Di-$C_{1-6}$-alkylcarboxamid, Di-$C_{1-6}$-alkylsulfonamid, Di-$C_{1-6}$-alkylthiocarboxamido, $C_{1-6}$-Halogenalkoxy, $C_{1-6}$-Halogenalkyl, Halogen, $C_{1-6}$-Halogenalkylsulfinyl, $C_{1-6}$-Halogenalkylsulfonyl, $C_{1-6}$-Halogen-alkylthio, Hydroxyl, Hydroxylamino und Nitro;
$Q_2$ $NR_5$ oder O ist, worin $R_5$ H, $C_{1-6}$-Acyl, $C_{1-6}$-Alkyl, $C_{2-6}$-Alkenyl, $C_{2-6}$-Alkinyl, $C_{3-7}$-Cycloalkyl oder $C_{3-7}$-Cycloalkyl-$C_{1-3}$-alkylen ist, worin das $C_{1-6}$-Alkyl gegebenenfalls mit einem oder mehreren Substituenten substituiert ist, die unabhängig voneinander ausgewählt sind aus: $C_{1-6}$-Acyl, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkylcarboxamid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthiocarboxamid, $C_{1-6}$-Alkylthioureyl, $C_{1-6}$-Alkylureyl, Amino, Di-$C_{1-6}$-alkylamino, $C_{1-6}$-Alkoxycarbonyl, Carboxamid, Carboxy, Cyano, $C_{3-6}$-Cycloalkyl, Di-$C_{1-6}$-alkylcarboxamid, Di-$C_{1-6}$-alkylsulfonamid, Di-$C_{1-6}$-alkylthiocarboxamido, $C_{1-6}$-Halogenalkoxy, $C_{1-6}$-Halogenalkyl, Halogen, $C_{1-6}$-Halogenalkylsulfinyl, $C_{1-6}$-Halogenalkylsulfonyl, $C_{1-6}$-Halogenalkylthio, Hydroxyl, Hydroxylamino und Nitro;
W CH ist;
X N oder $CR_6$ ist;
Y N oder $CR_7$ ist;

Z N ist;

V fehlt, $C_{1-3}$-Heteroalkylen oder $C_{1-3}$-Alkylen ist, die jeweils gegebenenfalls mit einem oder mehreren Substituenten substituiert sind, die unabhängig voneinander ausgewählt sind aus: $C_{1-3}$-Alkyl, $C_{1-6}$-Alkoxy, Carboxy, Cyano, $C_{1-3}$-Halogenalkyl und Halogen;

$R_6$, $R_7$ und $R_8$ jeweils unabhängig voneinander ausgewählt sind aus: H, $C_{1-6}$-Acyl, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkyl-carboxamid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthiocarboxamid, $C_{1-6}$-Alkylthioureyl, $C_{1-6}$-Alkylureyl, Amino, Di-$C_{1-6}$-alkylamino, $C_{1-6}$-Alkoxycarbonyl, Carboxamid, Carboxy, Cyano, $C_{3-6}$-Cycloalkyl, Di-$C_{1-6}$-alkylcarboxamid, Di-$C_{1-6}$-alkylsulfonamid, Di-$C_{1-6}$-alkylthiocarboxamido, $C_{1-6}$-Halogenalkoxy, $C_{1-6}$-Halogenalkyl, Halogen, $C_{1-6}$-Halogenalkylsulfinyl, $C_{1-6}$-Halogenalkylsulfonyl, $C_{1-6}$-Halogenalkylthio, Hydroxyl, Hydroxylamino und Nitro, worin das $C_{2-6}$-Alkenyl, $C_{1-6}$-Alkyl, $C_{2-6}$-Alkinyl und $C_{3-6}$-Cycloalkyl jeweils gegebenenfalls mit einem oder mehreren Substituenten substituiert sind, die unabhängig voneinander ausgewählt sind aus: $C_{1-6}$-Acyl, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkylcarboxamid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthiocarboxamid, $C_{1-6}$-Alkylthioureyl, $C_{1-6}$-Alkylureyl, Amino, Di-$C_{1-6}$-alkylamino, $C_{1-6}$-Alkoxycarbonyl, Carboxamid, Carboxy, Cyano, $C_{3-6}$-Cycloalkyl, Di-$C_{1-6}$-alkylcarboxamid, Di-$C_{1-6}$-alkylsulfonamid, Di-$C_{1-6}$-alkylthiocarboxamido, $C_{1-6}$-Halogenalkoxy, $C_{1-6}$-Halogenalkyl, Halogen, $C_{1-6}$-Halogenalkylsulfinyl, $C_{1-6}$-Halogenalkylsulfonyl, $C_{1-6}$-Halogenalkylthio, Hydroxyl, Hydroxylamino und Nitro;

Ar Phenyl, Naphthyl, Pyridyl, Benzofuranyl, Pyrazinyl, Pyridazinyl, Pyrimidinyl, Triazinyl, Chinolin, Benzoxazol, Benzothiazol, 1H-Benzimidazol, Isochinolin, Chinazolin, Chinoxalin, Pyrrol oder Indol ist und gegebenenfalls mit $R_9$, $R_{10}$, $R_{11}$, $R_{12}$ und $R_{13}$ substituiert ist;

$R^9$ ausgewählt ist aus: $C_{1-6}$-Acyl, $C_{1-6}$-Acylsulfonamid, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkylcarboxamid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthio-carboxamid, $C_{1-6}$-Alkylthioureyl, $C_{1-6}$-Alkylureyl, Amino, Aryl, Arylcarbonyl, Arylsulfonyl, Di-$C_{1-6}$-alkylamino, Carbamimidoyl, $C_{1-6}$-Alkoxycarbonyl, Carboxamid, Carboxy, Cyano, $C_{3-6}$-Cycloalkyl, Di-$C_{1-6}$-Alkylcarboxamid, Di-$C_{1-6}$-alkylsulfonamid, Di-$C_{1-6}$-alkylthiocarboxamido, Guanidin, $C_{1-6}$-Halogenalkoxy, $C_{1-6}$-Halogenalkyl, Halogen, $C_{1-6}$-Halogenalkylsulfinyl, $C_{1-6}$-Halogenalkylsulfonyl, $C_{1-6}$-Halogenalkylthio, Heterocyclyl, Heterocyclylsulfonyl, Heteroaryl, Hydroxyl, Hydroxylamino, Nitro, $C_{3-6}$-Oxo-cycloalkyl, Phenoxy, Sulfonamid, Sulfonsäure und Thiol; und worin die $R_9$ jeweils gegebenenfalls mit einem oder mehreren Substituenten substituiert sind, die jeweils unabhängig voneinander ausgewählt sind aus: $C_{1-6}$-Acyl, $C_{1-6}$-Acylsulfonamid, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkylcarboxamid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthiocarboxamid, $C_{1-6}$-Alkylthioureyl, $C_{1-6}$-Alkylureyl, Amino, Aryl, Arylcarbonyl, Arylsulfonyl, Di-$C_{1-6}$-alkylamino, $C_{1-6}$-Alkoxycarbonyl, Carboxamid, Carboxy, Cyano, $C_{3-6}$-Cycloalkyl, Di-$C_{1-6}$-alkylcarboxamid, Di-$C_{1-6}$-alkylsulfonamid, Di-$C_{1-6}$-alkylthiocarboxamido, $C_{1-6}$-Halogenalkoxy, $C_{1-6}$-Halogenalkyl, Halogen, $C_{1-6}$-Halogenalkylsulfinyl, $C_{1-6}$-Halogenalkylsulfonyl, $C_{1-6}$-Halogenalkylthio, Heteroaryl, Heteroarylcarbonyl, Heteroarylsulfonyl, Heterocyclyl, Hydroxyl, Hydroxylamino und Nitro;

$R_{10}$, $R_{11}$, $R_{12}$ und $R_{13}$ unabhängig voneinander ausgewählt sind aus: $C_{1-6}$-Acyl, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkylcarbox-amid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthiocarboxamid, $C_{1-6}$-Alkylthioureyl, $C_{1-6}$-Alkylureyl, Amino, Di-$C_{1-6}$-alkylamino, $C_{1-6}$-Alkoxycarbonyl, Carboxamid, Carboxy, Cyano, $C_{3-6}$-Cycloalkyl, Di-$C_{1-6}$-alkylcarboxamid, Di-$C_{1-6}$-alkylsulfonamid, Di-$C_{1-6}$-alkylthiocarboxamido, $C_{1-6}$-Halo-genalkoxy, $C_{1-6}$-Halogenalkyl, Halogen, $C_{1-6}$-Halogenalkylsulfinyl, $C_{1-6}$-Halogenalkylsulfonyl, $C_{1-6}$-Halogenalkylthio, Hydroxyl, Hydroxylamino, Nitro und Thiol; oder zwei benachbarte Gruppen von $R_{10}$, $R_{11}$, $R_{12}$ und $R_{13}$ gemeinsam mit den Atomen, an die sie gebunden sind, eine 5-, 6- oder 7-gliedrige Cycloalkylgruppe, 5-, 6- oder 7-gliedrige Cycloalkenylgruppe oder 5-, 6- oder 7-gliedrige Heterocyclylgruppe bilden, worin die 5-, 6- oder 7-gliedrige Gruppe gegebenenfalls mit Halogen oder Oxo substituiert ist; und

entweder:

$R_2$ Aryl, Arylcarbonyl, $C_{1-6}$-Alkoxycarbonyl, $C_{3-7}$-Cycloalkoxycarbonyl, Heteroaryl oder Heteroarylcarbonyl ist; die jeweils gegebenenfalls mit einem oder mehreren Substituenten substituiert sind, die unabhängig voneinander ausgewählt sind aus: $C_{1-6}$-Acyl, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkylcarboxamid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthiocarboxamid, $C_{1-6}$-Alkylthioureyl, $C_{1-6}$-Alkylureyl, Amino, Aryl, Di-$C_{1-6}$-alkylamino, Carbo-$C_{1-6}$-alkoxy, Carboxamid, Carboxy, Cyano, $C_{3-6}$-Cycloalkyl, Di-$C_{1-6}$-alkylcarboxamid, Di-$C_{1-6}$-alkylsulfonamid, Di-$C_{1-6}$-alkylthiocarboxamido, $C_{1-6}$-Halogenalkoxy, $C_{1-6}$-Halogenalkyl, Halogen, $C_{1-6}$-Halogenalkylsulfinyl, $C_{1-6}$-Halogenalkylsulfonyl, $C_{1-6}$-Halogenalkylthio, Heterocyclyl, Heteroaryl, Hydroxyl, Hydroxylamino, Nitro und

Thiol;
oder:
$R_2$ $C_{1-6}$-Alkoxycarbonyl, $C_{1-6}$-Alkoxycarbonyl, das mit $C_{3-6}$-Cycloalkyl substituiert ist, $C_{3-7}$-Cycloalkoxycarbonyl oder Heteroaryl-$C_{1-3}$-alkylen ist, die gegebenenfalls 1-, 2- oder 3-fach mit $C_{1-6}$-Alkyl substituiert sind;
mit der Maßgabe, dass die Verbindung nicht 4-[6-(2-Fluor-4-methansulfonyl-phenoxy)pyrimidin-4-yloxy]piperidin-1-carbonsäureisopropylester ist.

**2.** Verbindung nach Anspruch 1, worin K fehlt.

**3.** Verbindung nach Anspruch 1, worin K $-CH_2-$ ist.

**4.** Verbindung nach einem der Ansprüche 1 bis 3, worin V fehlt.

**5.** Verbindung nach einem der Ansprüche 1 bis 4, worin $Q_1$ O ist.

**6.** Verbindung nach einem der Ansprüche 1 bis 4, worin $Q_1$ NH ist.

**7.** Verbindung nach einem der Ansprüche 1 bis 6, worin $Q_2$ O ist.

**8.** Verbindung nach einem der Ansprüche 1 bis 6, worin $Q_2$ $NR_5$ ist.

**9.** Verbindung nach Anspruch 8, worin $R_5$ H oder $C_{1-6}$-Alkyl ist.

**10.** Verbindung nach einem der Ansprüche 1 bis 9, worin:

W CH ist;
X N ist;
Y $CR_7$ ist, worin $R_7$ H, $C_{1-6}$-Alkyl oder Halogen ist; und
Z N ist.

**11.** Verbindung nach einem der Ansprüche 1 bis 10, worin E CH und D $NR_2$ ist.

**12.** Verbindung nach einem der Ansprüche 1 bis 11, worin $R_2$ Aryl, Arylcarbonyl, $C_{1-6}$-Alkoxycarbonyl, $C_{3-7}$-Cycloalkoxycarbonyl, Heteroaryl oder Heteroarylcarbonyl ist; die jeweils gegebenenfalls mit einem oder mehreren Substituenten substituiert sind, die unabhängig voneinander ausgewählt sind aus: $C_{1-6}$-Acyl, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkylcarboxamid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthio-carboxamid, $C_{1-6}$-Alkylthioureyl, $C_{1-6}$-Alkylureyl, Amino, Aryl, Di-$C_{1-6}$-alkylamino, Carbo-$C_{1-6}$-alkoxy, Carboxamid, Carboxy, Cyano, $C_{3-6}$-Cycloalkyl, Di-$C_{1-6}$-alkyl-carboxamid, Di-$C_{1-6}$-alkylsulfonamid, Di-$C_{1-6}$-alkylthiocarboxamido, $C_{1-6}$-Halogenalk-oxy, $C_{1-6}$-Halogenalkyl, Halogen, $C_{1-6}$-Halogenalkylsulfinyl, $C_{1-6}$-Halogenalkylsulfonyl, $C_{1-6}$-Halogenalkylthio, Heterocyclyl, Heteroaryl, Hydroxyl, Hydroxylamino, Nitro und Thiol.

**13.** Verbindung nach einem der Ansprüche 1 bis 11, worin $R_2$ $C_{1-6}$-Alkoxycarbonyl, $C_{1-6}$-Alkoxycarbonyl, das mit $C_{3-6}$-Cycloalkyl substituiert ist, $C_{3-7}$-Cycloalkoxycarbonyl oder Heteroaryl-$C_{1-3}$-alkylen ist, die gegebenenfalls 1-, 2- oder 3-fach mit $C_{1-6}$-Alkyl substituiert sind.

**14.** Verbindung nach einem der Ansprüche 1 bis 13, worin Ar Phenyl ist, das gegebenenfalls mit $R_9$, $R_{10}$, $R_{11}$, $R_{12}$ und $R_{13}$ substituiert ist;
$R_9$ ausgewählt ist aus: Methansulfonyl, 2-Methansulfonylethyl, Acetylsulfamoyl, Propionylsulfamoyl, Ethylsulfanyl, Isopropylsulfanyl, Ethylsulfamoyl, Methylsulfamoyl, Dimethylsulfamoyl, Methylsulfamoylmethyl, Sulfamoyl, [1,2,4]-Triazol-1-yl, [1,2,4]-Triazol-1-ylmethyl, 2-[1,2,4]-Triazol-1-ylethyl, Methoxy, 2-Oxooxazolidin-4-yl-methyl, 1,1-Dioxo-1$\lambda^6$-thiomorpholin-4-ylmethyl, Pyrazol-1-yl, Trifluormethansulfonyl, Morpholin-4-sulfonyl, Pyridin-2-carbonyl, F, Cl, Cyano, Br, Carboxy, Butyryl, Propoxycarbonyl, Hydroxy, Propylcarbamoyl, N-Hydroxycarbamimidoyl, Carbamimidoyl, N-Ethylcarbamimidoyl und 2-Aminoethylamino; und
$R_{10}$, $R_{11}$, $R_{12}$ und $R_{13}$ unabhängig voneinander aus F, Methoxy, Methyl, Ethyl und Carboxy ausgewählt sind.

**15.** Verbindung nach einem der Ansprüche 1 bis 13, worin Ar Phenyl ist, das gegebenenfalls mit $R_9$, $R_{10}$, $R_{11}$, $R_{12}$ und $R_{13}$ substituiert ist;

$R_9$ ausgewählt ist aus: Methansulfonyl, Cyano, F, Cl, Br, I, Methyl, Methoxy, Ethylamino, Ethylsulfanyl, Isopropylsulfanyl, Hydroxy, Isopropoxy, Propoxy, Dimethylamino, Propylamino, Isopropylamino, Acetylamino, Piperidin-1-yl, Trifluormethoxy, Oxazol-5-yl, Ethinyl, 3-Methylbutylamino, 2-Morpholin-4-ylethylamino, Acetylsulfamoyl, Propionylsulfamoyl, Tetrahydrofuran-2-ylmethoxy, Morpholin-4-yl, 4-Methylpiperazin-1-yl, Butylamino, 2-Pyrrolidin-1-ylethoxy, 2-Dimethylaminoethoxy, 2-Morpholin-4-ylethoxy, Morpholin-4-ylamino, 2-Methoxyethylamino und Tetrahydrofuran-2-ylmethyl-amino; und

$R_{10}$, $R_{11}$, $R_{12}$ und $R_{13}$ unabhängig voneinander aus F, Cl, Br, I, Hydroxyl, Methoxy, Cyano, Methyl und Trifluormethoxy ausgewählt sind.

**16.** Verbindung nach einem der Ansprüche 1 bis 13, worin Ar ausgewählt ist aus: Pyridin-2-yl, Pyridin-3-yl, Pyridin-4-yl, Chinolin-6-yl und Benzothiazol-6-yl, die jeweils gegebenenfalls mit $R_9$, $R_{10}$, $R_{11}$, $R_{12}$ und $R_{13}$ substituiert sind;
$R_9$ ausgewählt ist aus: Methansulfonyl, Methoxy, Carboxy, Acetylsulfamoyl, Propionylsulfamoyl, Acetylamino, F, Cl, Br, Methyl und Trifluormethyl; und
$R_{10}$, $R_{11}$, $R_{12}$ und $R_{13}$ unabhängig voneinander aus Methoxy, Methyl, F, Cl und Br ausgewählt sind.

**17.** Verbindung nach einem der Ansprüche 1 bis 13, worin Ar ausgewählt ist aus: Pyridin-2-yl, Pyridin-3-yl, Pyridin-4-yl, Chinolin-6-yl und Benzothiazol-6-yl, die gegebenenfalls mit $R_9$, $R_{10}$, $R_{11}$, $R_{12}$ und $R_{13}$ substituiert sind;
$R_9$ ausgewählt ist aus: Cyano, F, Cl, Br, Acetylamino, Methoxy, Methyl, Propoxy, Propylamino, Isopropylamino, Phenyl, t-Butyl, 4-Methylphenyl, Ethyl, Methylsulfanyl, Morpholin-4-yl, Benzolsulfonyl, Trifluormethyl, Cyclopropyl, Carbamoyl, 3,4-Difluorphenyl, 4-Chlorphenyl, 1-Methylpyrrolidin-2-yl, Acetylsulfamoyl, Propionylsulfamoyl und Pyridin-2-yl; und
$R_{10}$, $R_{11}$, $R_{12}$ und $R_{13}$ unabhängig voneinander Methyl, F oder Cl sind.

**18.** Verbindung nach Anspruch 1, ausgewählt aus Verbindungen der Formel (IIIi) und pharmazeutisch annehmbaren Salzen, Solvaten, Hydraten und N-Oxiden davon;

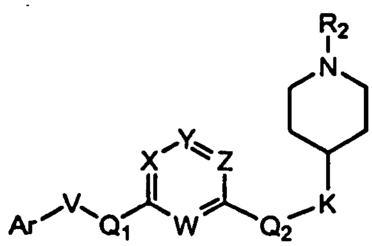

**(IIIi)**

worin

K fehlt oder eine $C_{1-3}$-Alkylengruppe ist;
$Q_1$ NH oder O ist;
$Q_2$ $NR_5$ oder O ist, worin $R_5$ H oder $C_{1-6}$-Alkyl ist;
W CH ist, X N ist, Y CH ist und Z N ist;
V fehlt;
Ar ausgewählt ist aus: Pyridin-2-yl, Pyridin-3-yl, Pyridin-4-yl, Chinolin-6-yl und Benzothiazol-6-yl, die jeweils gegebenenfalls mit $R_9$, $R_{10}$, $R_{11}$, $R_{12}$ und $R_{13}$ substituiert sind;
$R_9$ $C_{1-6}$-Acyl, $C_{1-6}$-Acylsulfonamid, $C_{1-6}$-Alkoxy, $C_{1-6}$-Alkyl, $C_{1-6}$-Alkylamino, $C_{1-6}$-Alkylcarboxamid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, Amino, Aryl, Arylsulfonyl, Di-$C_{1-6}$-alkylamino, Carbamimidoyl, $C_{1-6}$-Alkoxycarbonyl, Carboxamid, Carboxy, Cyano, $C_{3-6}$-Cycloalkoxy, $C_{3-6}$-Cycloalkyl, Di-$C_{1-6}$-alkylsulfonamid, Guanidin, $C_{1-6}$-Halogenalkoxy, $C_{1-6}$-Halogenalkyl, Halogen, $C_{1-6}$-Halogenalkylsulfonyl, Heteroaryl, Heteroarylcarbonyl, Heteroarylsulfonyl, Heterocyclyl, Heterocyclylsulfonyl, Hydroxyl, Sulfonamid oder Thiol ist; worin $C_{1-6}$-Alkoxy, $C_{1-6}$-Alkyl, $C_{1-6}$-Alkylamino, Amino, Aryl, Carbamimidoyl und Heterocyclyl gegebenenfalls mit 1, 2, 3 oder 4 Substituenten substituiert sind, die unabhängig voneinander ausgewählt sind aus: $C_{1-6}$-Alkoxy, $C_{1-6}$-Alkyl, $C_{2-6}$-Alkinyl, $C_{1-6}$-Alkylsulfonamid, $C_{1-6}$-Alkylsulfonyl, Amino, Aryl, $C_{3-6}$-Cycloalkyl, Di-$C_{1-6}$-alkylamino, Halogen, Heteroaryl, Heterocyclyl und Hydroxyl;
$R_{10}$, $R_{11}$, $R_{12}$ und $R_{13}$ unabhängig voneinander ausgewählt sind aus: $C_{1-6}$-Alkoxy, $C_{1-6}$-Alkyl, $C_{1-6}$-Alkylamino, Carboxy, Cyano, Halogen, $C_{1-6}$-Halogenalkoxy, $C_{1-6}$-Halogenalkylsulfonyl und Hydroxyl; oder zwei benachbarte

Gruppen von $R_{10}$, $R_{11}$, $R_{12}$ und $R_{13}$ gemeinsam mit den Atomen, an die sie gebunden sind, eine 5- oder 6-gliedrige Cycloalkyl- oder 5- oder 6-gliedrige Heterocyclylgruppe bilden, worin die 5- oder 6-gliedrige Gruppe gegebenenfalls mit Halogen oder Oxo substituiert ist; und

$R_2$ tert-Butoxycarbonyl, Isobutoxycarbonyl, Isopropoxycarbonyl, Butoxycarbonyl, Cyclopropylmethoxycarbonyl, 3-Methylbutoxycarbonyl oder 3-Isopropyl[1,2,4]-oxadiazol-5-yl ist.

**19.** Verbindung nach Anspruch 1, worin die Verbindung aus den nachstehenden Verbindungen und pharmazeutisch annehmbaren Salzen, Solvaten, Hydraten und N-Oxiden davon ausgewählt ist:

(A3) 4-{[6-(2-Fluor-4-methansulfonylphenylamino)pyrimidin-4-yl]methylamino}-piperidin-1-carbonsäure-tert-butylester;

(A4) 4-({[6-(2-Fluor-4-methansulfonylphenylamino)pyrimidin-4-yl]methylamino}-methyl)piperidin-1-carbonsäure-tert-butylester;

(A5) 4-({[6-(4-Methansulfonylphenylamino)pyrimidin-4-yl]methylamino}methyl)-piperidin-1-carbonsäure-tert-butylester;

(A6) 4-({[6-(2,5-Difluorbenzylamino)pyrimidin-4-yl]methylamino}methyl)-piperidin-1-carbonsäure-tert-butylester;

(A7) 4-[({6-[(Benzo[1,3]dioxol-5-ylmethyl)amino]pyrimidin-4-yl}methylamino)-methyl]piperidin-1-carbonsäure-tert-butylester;

(A9) 4-({Methyl-[6-(2-pyridin-4-ylethylamino)pyrimidin-4-yl]amino}methyl)-piperidin-1-carbonsäure-tert-butylester;

(A10) 4-({Methyl-[6-(2-pyridin-3-ylethylamino)pyrimidin-4-yl]amino}methyl)-piperidin-1-carbonsäure-tert-butylester;

(A11) 4-[(Methyl-{6-[(pyridin-3-ylmethyl)amino]pyrimidin-4-yl}amino)methyl]-piperidin-1-carbonsäure-tert-butylester;

(A12) 4-[({6-[(2-Fluor-4-methansulfonylphenyl)methylamino]pyrimidin-4-yl}-methylamino)methyl]piperidin-1-carbonsäure-tert-butylester;

(A13) 4-({[6-(2-Fluor-4-methansulfonylphenylamino)pyrimidin-4-yl]methyl-amino}methyl)piperidin-1-carbonsäureisobutylester;

(A14) 4-({[6-(4-Cyano-2-fluorphenylamino)pyrimidin-4-yl]methylamino}methyl)-piperidin-1-carbonsäure-tert-butylester;

(A15) 4-[({6-[4-(2-Methansulfonylethyl)phenylamino]pyrimidin-4-yl}methyl-amino)methyl]piperidin-1-carbonsäure-tert-butylester;

(A16) 4-({[6-(4-Ethylsulfanylphenylamino)pyrimidin-4-yl]methylamino}methyl)-piperidin-1-carbonsäure-tert-butylester;

(A17) 4-({[6-(4-Isopropylsulfanylphenylamino)pyrimidin-4-yl]methylamino}-methyl)piperidin-1-carbonsäure-tert-butylester;

(A18) 4-({[6-(4-Ethylsulfamoylphenylamino)pyrimidin-4-yl]methylamino}-methyl)piperidin-1-carbonsäure-tert-butylester;

(A19) 4-({Methyl-[6-(4-methylsulfamoylphenylamino)pyrimidin-4-yl]amino}-methyl)piperidin-1-carbonsäure-tert-butylester;

(A20) 4-({[6-(4-Dimethylsulfamoylphenylamino)pyrimidin-4-yl]methylamino}-methyl)piperidin-1-carbonsäure-tert-butylester;

(A21) 4-({Methyl-[6-(4-methylsulfamoylmethylphenylamino)pyrimidin-4-yl]-amino}methyl)piperidin-1-carbonsäure-tert-butylester;

(A22) 4-({Methyl-[6-(4-sulfamoylphenylamino)pyrimidin-4-yl]amino}methyl)-piperidin-1-carbonsäure-tert-butylester;

(A23) 4-({Methyl-[6-(4-[1,2,4]-triazol-1-ylphenylamino)pyrimidin-4-yl]amino}-methyl)piperidin-1-carbonsäure-tert-butylester;

(A24) 4-({Methyl-[6-(4-[1,2,4]-triazol-1-ylmethylphenylamino)pyrimidin-4-yl]-amino}methyl)piperidin-1-carbonsäure-tert-butylester;

(A25) 4-[(Methyl-{6-[4-(2-[1,2,4]-triazol-1-ylethyl)phenylamino]pyrimidin-4-yl}-amino)methyl]piperidin-1-carbonsäure-tert-butylester;

(A26) 4-({[6-Benzo[1,3]dioxol-5-ylamino)pyrimidin-4-yl]methylamino}methyl)-piperidin-1-carbonsäure-tert-butylester;

(A27) 4-({[6-(6-Methansulfonylpyridin-3-ylamino)pyrimidin-4-yl]methylamino}-methyl)piperidin-1-carbonsäure-tert-butylester;

(A28) 4-({[6-(3,5-Dimethoxyphenylamino)pyrimidin-4-yl]methylamino}methyl)-piperidin-1-carbonsäure-tert-butylester;
(A29) 4-[(Methyl-{6-[4-(2-oxooxazolidin-4-ylmethyl)phenylamino]pyrimidin-4-yl}amino)methyl]piperidin-1-carbonsäure-tert-butylester;
(A30) 4-[({6-[4-(1,1-Dioxo-1λ6-thiomorpholin-4-ylmethyl)phenylamino]-pyrimidin-4-yl}methylamino)methyl] piperidin-1-carbonsäure-tert-butylester;
(A31) 4-({Methyl-[6-(4-pyrazol-1-ylphenylamino)pyrimidin-4-yl]amino}methyl)-piperidin-1-carbonsäure-tert-butylester,
(A32) 4-({[6-(2,2-Difluorbenzo[1,3]dioxol-5-ylamino)pyrimidin-4-yl]methyl-amino}methyl)piperidin-1-carbonsäure-tert-butylester;
(A33) 4-({Methyl-[6-(4-trifluormethansulfonylphenylamino)pyrimidin-4-yl]-amino}methyl)piperidin-1-carbonsäure-tert-butylester;
(A34) 4-[(Methyl-{6-[4-(morpholin-4-sulfonyl)phenylamino]pyrimidin-4-yl}-amino)methyl]piperidin-1-carbonsäure-tert-butylester;
(A35) 4-[(Methyl-{6-[2-(pyridin-2-carbonyl)phenylamino]pyrimidin-4-yl}amino)-methyl]piperidin-1-carbonsäure-tert-butylester;
(A36) 4-({[6-(2-Fluor-5-methansulfonylphenylamino)pyrimidin-4-yl]methyl-amino}methyl)piperidin-1-carbonsäure-tert-butylester;
(A39) 4-({[6-(3,4-Difluorphenylamino)pyrimidin-4-yl]methylamino}methyl)-piperidin-1-carbonsäure-tert-butylester;
(A40) 4-({[6-(2,6-Difluorphenylamino)pyrimidin-4-yl]methylamino}methyl)-piperidin-1-carbonsäure-tert-butylester;
(A41)4-({[6-(2,5-Difluorphenylamino)pyrimidin-4-yl]methylamino}methyl)-piperidin-1-carbonsäure-tert-butylester;
(A42) 4-({[6-(2,3-Difluorphenylamino)pyrimidin-4-yl]methylamino}methyl)-piperidin-1-carbonsäure-tert-butylester;
(A43) 4-({Methyl-[6-(2,3,5-trifluorphenylamino)pyrimidin-4-yl]amino}methyl)-piperidin-1-carbonsäure-tert-butylester;
(A44) 4-({[6-(2-Fluorphenylamino)pyrimidin-4-yl]methylamino}methyl)-piperidin-1-carbonsäure-tert-butylester;
(A45) 4-({[6-(2-Fluor-4-methylphenylamino)pyrimidin-4-yl]methylamino}-methyl)piperidin-1-carbonsäure-tert-butylester;
(A46) 4-({[6-(3-Chlor-2-fluorphenylamino)pyrimidin-4-yl]methylamino}methyl)-piperidin-1-carbonsäure-tert-butylester;
(A47) 4-({[6-(2,4-Difluorphenylamino)pyrimidin-4-yl]methylamino}methyl)-piperidin-1-carbonsäure-tert-butylester;
(A48) 4-[(Methyl-{6-[2-(1-oxypyridin-3-yl)ethylamino]pyrimidin-4-yl}amino)-methyl]piperidin-1-carbonsäure-tert-butylester;
(A49) 4-[(Methyl-{6-[2-(1-oxypyridin-3-yl)ethylamino]pyrimidin-4-yl}amino)-methyl]piperidin-1-carbonsäureisobutylester;
(A50) 4-({[6-(2,5-Difluorphenylamino)pyrimidin-4-yl]methylamino}methyl)-piperidin-1-carbonsäureisobutylester;
(A51) 4-({[6-(4-Cyano-2-fluorphenylamino)pyrimidin-4-yl]methylamino}methyl)-piperidin-1-carbonsäureisobutylester;
(A52) 4-[({6-[2-(2-Fluorphenoxy)ethylamino]pyrimidin-4-yl}methylamino)-methyl]piperidin-1-carbonsäure-tert-butylester;
(A53) 4-({[6-(2-Fluorphenoxy)pyrimidin-4-yl]methylamino}methyl)-piperidin-1-carbonsäure-tert-butylester;
(A54) 4-({[6-(2,5-Difluorphenoxy)pyrimidin-4-yl]methylamino}methyl)-piperidin-1-carbonsäure-tert-butylester;
(A55) 4-[({6-[2-(2-Chlorphenoxy)ethylamino]pyrimidin-4-yl}methylamino)-methyl]piperidin-1-carbonsäure-tert-butylester;
(A56) 4-({[6-(2-Chlorphenoxy)pyrimidin-4-yl]methylamino}methyl)-piperidin-1-carbonsäure-tert-butylester;
(A57) 4-[({6-[2-(4-Fluorphenoxy)propylamino]pyrimidin-4-yl}methylamino)-methyl]piperidin-1-carbonsäure-tert-butylester;
(A58) 4-({[6-(4-Ethylsulfamoyl-2-fluorphenylamino)pyrimidin-4-yl]methyl-amino}methyl)piperidin-1-carbonsäure-tert-butylester;
(A59) 4-({[6-(2-Fluor-4-isopropylsulfamoylphenylamino)pyrimidin-4-yl]methyl-amino}methyl)piperidin-1-carbonsäure-tert-butylester;
(A60) 4-({[6-(4-Cyano-2,5-difluorphenylamino)pyrimidin-4-yl]methylamino}-methyl)piperidin-1-carbonsäure-tert-butylester;
(A61) 4-({[6-(4-Brom-2,5-difluorphenylamino)pyrimidin-4-yl]methylamino}-methyl)piperidin-1-carbonsäure-tert-butylester;
(A62) 4-({[6-(5-Carboxy-2-fluorphenylamino)pyrimidin-4-yl]methylamino}-methyl)piperidin-1-carbonsäure-tert-butylester;
(A63) 4-({[6-(6-Methoxypyridin-3-ylamino)pyrimidin-4-yl]methylamino}methyl)-piperidin-1-carbonsäure-tert-butylester;
(A64) 4-({[6-(2,6-Dimethoxypyridin-3-ylamino)pyrimidin-4-yl]methylamino}-methyl)piperidin-1-carbonsäure-tert-butylester;
(A65) 6-{6-[(1-tert-Butoxycarbonylpiperidin-4-ylmethyl)methylamino]pyrimidin-4-ylamino}nicotinsäure;
(A66) 4-({[6-(6-Acetylaminopyridin-3-ylamino)pyrimidin-4-yl]methylamino}-methyl)piperidin-1-carbonsäure-tert-butylester;
(A67) 4-({[6-(5-Fluorpyridin-2-ylamino)pyrimidin-4-yl]methylamino}methyl)-piperidin-1-carbonsäure-tert-butylester;
(A68) 4-({[6-(4-Cyano-2-ethylphenylamino)pyrimidin-4-yl]methylamino}methyl)-piperidin-1-carbonsäure-tert-butylester;
(A69) 4-({[6-(4-Butyrylphenylamino)pyrimidin-4-yl]methylamino}methyl)-piperidin-1-carbonsäure-tert-butylester;
(A70) 4-({[6-(5-Brom-3-methylpyridin-2-ylamino)pyrimidin-4-yl]methylamino}-methyl)piperidin-1-carbonsäure-tert-butylester;
(A71) 4-({[6-(3-Brom-5-methylpyridin-2-ylamino)pyrimidin-4-yl]methylamino}-methyl)piperidin-1-carbonsäure-tert-butylester;
(A72) 4-({Methyl-[6-(5-trifluormethylpyridin-2-ylamino)pyrimidin-4-yl]amino}-methyl)piperidin-1-carbonsäure-tert-butylester;
(A73) 4-({[6-(4-Brom-2-fluorphenylamino)pyrimidin-4-yl]methylamino}methyl)-piperidin-1-carbonsäure-tert-butylester;
(A74) 4-({[6-(3-Carboxy-4-fluorphenylamino)pyrimidin-4-yl]methylamino}-methyl)piperidin-1-carbonsäure-tert-butyester;
(A75) 4-({[6-(4-Ethoxycarbonyl-2-fluorphenylamino)pyrimidin-4-yl]methyl-amino}methyl)piperidin-1-carbonsäureisobutylester;
(A76) 4-({[6-(4-Carboxy-2-fluorphenylamino)pyrimidin-4-yl]methylamino}-methyl)piperidin-1-carbonsäureisobutylester;
(A77) 4-({[6-(4-Cyano-2-fluorphenylamino)pyrimidin-4-yl]methylamino}methyl)-piperidin-1-carbonsäureisopropylester;
(A78) 4-({[6-(4-Cyano-2-fluorphenylamino)pyrimidin-4-yl]methylamino}methyl)-piperidin-1-carbonsäurebutylester;
(A79) 4-({[6-(4-Cyano-2-fluorphenylamino)pyrimidin-4-yl]methylamino}methyl)-piperidin-1-carbonsäurecyclopropylmethylester;
(A82) 4-({[6-(2,5-Difluor-4-hydroxyphenylamino)pyrimidin-4-yl]methylamino}-methyl)piperidin-1-carbonsäureisobutylester;
(A83) 4-({[6-(4-Ethylcarbamoyl-2-fluorphenylamino)pyrimidin-4-yl]methyl-amino}methyl)piperidin-1-carbonsäureisobutylester;
(A84) 4-[({6-[2-Fluor-4-(N-hydroxycarbamimidoyl)phenylamino]pyrimidin-4-yl}-methylamino)methyl]piperidin-1-carbonsäureisobutylester;
(A85) 4-({[6-(2-Fluor-4-methansulfonylphenylamino)pyrimidin-4-yl]methyl-amino}methyl)piperidin-1-carbonsäure-3-methylbutylester;
(A86) 4-({[6-(2,5-Difluor-4-methansulfonylphenylamino)pyrimidin-4-yl]methyl-amino}methyl)piperidin-1-carbonsäure-tert-butylester;
(A87) 4-({[6-(2-Fluor-4-methansulfonylphenylamino)pyrimidin-4-yl]methyl-amino}methyl)piperidin-1-carbonsäureisopropylester;
(A88) (5-Butylpyridin-2-yl)-[4-({[6-(2-fluor-4-methansulfonylphenylamino)-pyrimidin-4-yl]methylamino}methyl) piperidin-1-yl]methanon;
(A89) N-(2-Fluor-4-methansulfonylphenyl)-N'-(5'-fluor-3,4,5,6-tetrahydro-2H-[1,2']bipyridinyl-4-ylmethyl)-N'-methylpyrimidin-4,6-diamin;
(A90) 4-({[6-(4-Carbamimidoyl-2-fluorphenylamino)pyrimidin-4-yl]methyl-amino}methyl)piperidin-1-carbonsäureisobutylester;
(A91) 4-({[6-(2-Fluor-4-methansulfonylphenylamino)pyrimidin-4-yl]methyl-amino}methyl)piperidin-1-carbonsäurecyclobutylester;
(A92) 4-[6-(2-Fluor-4-methansulfonylphenylamino)pyrimidin-4-ylamino]-piperidin-1-carbonsäure-tert-butylester;

(A94) 4-({[6-(2-Fluor-4-methansulfonylphenylamino)pyrimidin-4-yl]methyl-amino}methyl)piperidin-1-carbonsäure-1-ethylpropylester;
(A95) 4-({Ethyl-[6-(2-fluor-4-methansulfonylphenylamino)pyrimidin-4-yl]amino}-methyl)piperidin-1-carbonsäure-tert-butylester;
(A96) 4-({Ethyl-[6-(2-fluor-4-methansulfonylphenylamino)pyrimidin-4-yl]amino}-methyl)piperidin-1-carbonsäureisopropylester;
(A97) 4-({[6-(4-Cyano-2,5-difluorphenylamino)pyrimidin-4-yl]ethylamino}-methyl)piperidin-1-carbonsäureisopropylester;
(A98) 4-({[6-(4-Amino-2,5-difluorphenoxy)pyrimidin-4-yl]ethylamino}methyl)-piperidin-1-carbonsäure-tert-butylester;
(A99) 4-({[6-(2,5-Difluor-4-methoxyphenylamino)pyrimidin-4-yl]ethylamino}-methyl)piperidin-1-carbonsäure-tert-butylester;
(A100) 4-({[6-(2,5-Difluor-4-methansulfonylphenylamino)pyrimidin-4-yl]ethyl-amino}methyl)piperidin-1-carbonsäure-tert-butylester;
(A101) 4-({Ethyl-[6-(2,4,5-trifluorphenylamino)pyrimidin-4-yl]amino}methyl)-piperidin-1-carbonsäure-tert-butylester;
(A103) 4-[(Ethyl-{6-[4-(N-ethylcarbamimidoyl)-2,5-difluorphenylamino]-pyrimidin-4-yl}amino)methyl]piperidin-1-carbsonäureisopropylester;
(A104) 4-({[6-(4-Brom-2,5-difluorphenylamino)pyrimidin-4-yl]ethylamino}-methyl)piperidin-1-carbonsäure-tert-butylester;
(A105) 4-[({6-[5-(2-Aminoethylamino)-4-cyano-2-fluorphenylamino]pyrimidin-4-yl}ethylamino)methyl]piperidin-1-carbonsäureisopropylester;
(A120) 4-{[6-(2-Fluor-4-methansulfonylphenylamino)pyrimidin-4-ylamino]-methyl}piperidin-1-carbonsäure-tert-butylester;
(A121) 4-{[6-(2-Fluor-4-methansulfonylphenylamino)pyrimidin-4-ylamino]-methyl}piperidin-1-carbonsäureisopropylester;
(A122) 4-({[6-(2-Fluor-4-methansulfonylphenylamino)pyrimidin-4-yl]isopropylamino}methyl)piperidin-1-carbonsäure-tert-butylester; und
(A123) 4-({[4-(2-Fluor-4-methansulfonylphenylamino)pyridin-2-yl]methyl-amino}methyl)piperidin-1-carbonsäureisobutylester.

**20.** Verbindung nach Anspruch 1, worin die Verbindung aus den nachstehenden Verbindungen und pharmazeutisch annehmbaren Salzen, Solvaten, Hydraten und N-Oxiden davon ausgewählt ist:

(B1) 4-[6-(2-Fluor-4-methansulfonylphenylamino)pyrimidin-4-yloxy]-piperidin-1-carbonsäure-tert-butylester;
(B3) 4-[6-(2-Fluor-4-methansulfonylphenylamino)pyrimidin-4-yloxy]-piperidin-1-carbonsäureisopropylester;
(B4) (6-Chlorpyridin-2-yl)-{4-[6-(2-fluor-4-methansulfonylphenylamino)-pyrimidin-4-yloxy]piperidin-1-yl}methanon;
(B5) (6-Brompyridin-2-yl)-{4-[6-(2-fluor-4-methansulfonylphenylamino)-pyrimidin-4-yloxy]piperidin-1-yl}methanon;
(B6) {4-[6-(2-Fluor-4-methansulfonylphenylamino)pyrimidin-4-yloxy]piperidin-1-yl}-(6-methylpyridin-2-yl)methanon;
(B7) {4-[6-(2-Fluor-4-methansulfonylphenylamino)pyrimidin-4-yloxy]piperidin-1-yl}-(6-fluorpyridin-2-yl)methanon;
(B8) {4-[6-(2-Fluor-4-methansulfonylphenylamino)pyrimidin-4-yloxy]piperidin-1-yl}pyridin-2-ylmethanon;
(B9) (5-Brompyridin-3-yl)-{4-[6-(2-fluor-4-methansulfonylphenylamino)-pyrimidin-4-yloxy]piperidin-1-yl}methanon;
(B10) {4-[6-(2-Fluor-4-methansulfonylphenylamino)pyrimidin-4-yloxy]piperidin-1-yl}-(5-methylpyridin-3-yl)methanon;
(B11) (5,6-Dichlorpyridin-3-yl)-{4-[6-(2-fluor-4-methansulfonylphenylamino)-pyrimidin-4-yloxy]piperidin-1-yl}methanon;
(B12) 4-[6-(4-Cyano-2,5-difluorphenylamino)pyrimidin-4-yloxy]-piperidin-1-carbonsäure-tert-butylester;
(B13) 4-[6-(2,5-Difluor-4-methansulfonylphenylamino)pyrimidin-4-yloxy]-piperidin-1-carbonsäure-tert-butylester;
(B14) 4-[6-(2,4,5-Trifluorphenylamino)pyrimidin-4-yloxy]-piperidin-1-carbonsäure-tert-butylester;
(B15) 4-[6-(4-Brom-2,5-difluorphenylamino)pyrimidin-4-yloxy]-piperidin-1-carbonsäure-tert-butylester;
(B16) 4-[6-(3-Fluor-4-methylphenylamino)pyrimidin-4-yloxy]-piperidin-1-carbonsäure-tert-butylester;
(B17) 4-[6-(3-Hydroxy-4-methoxyphenylamino)pyrimidin-4-yloxy]-piperidin-1-carbonsäure-tert-butylester;

(B18) 4-[6-(6-Cyanopyridin-3-ylamino)pyrimidin-4-yloxy]-piperidin-1-carbonsäure-tert-butylester;
(B19) 4-[6-(3-Chlor-4-cyanophenylamino)pyrimidin-4-yloxy]-piperidin-1-carbonsäure-tert-butylester;
(B20) 4-[6-(6-Chlorpyridin-3-ylamino)pyrimidin-4-yloxy]-piperidin-1-carbonsäure-tert-butylester;
(B21) 4-[6-(3-Fluor-4-methoxyphenylamino)pyrimidin-4-yloxy]-piperidin-1-carbonsäure-tert-butylester;
(B22) 4-[6-(3,4-Dimethoxyphenylamino)pyrimidin-4-yloxy]-piperidin-1-carbonsäureester-tert-butylester;
(B23) 4-[6-(2,3-Dihydrobenzo[1,4]dioxin-6-ylamino)pyrimidin-4-yloxy]-piperidin-1-carbonsäure-tert-butylester;
(B24) 4-[6-(4-Cyano-2,5-difluorphenylamino)pyrimidin-4-yloxy]-piperidin-1-carbonsäureisopropylester;
(B25) 4-[6-(4-Cyano-5-ethylamino-2-fluorphenylamino)pyrimidin-4-yloxy]-piperidin-1-carbonsäure-tert-butylester;
(B26) 4-[6-(4-Ethoxy-2,5-difluorphenylamino)pyrimidin-4-yloxy]-piperidin-1-carbonsäureisopropylester;
(B27) 4-[6-(4-Ethylsulfanylphenylamino)pyrimidin-4-yloxy]-piperidin-1-carbonsäure-tert-butylester;
(B28) 4-[6-(4-Isopropylsulfanylphenylamino)pyrimidin-4-yloxy]-piperidin-1-carbonsäure-tert-butylester;
(B29) (5-Butylpyridin-2-yl)-{4-[6-(2-fluor-4-methansulfonylphenylamino)-pyrimidin-4-yloxy]piperidin-1-yl}methanon;
(B30) 4-[6-(5-Chlor-3-methylpyridin-2-ylamino)pyrimidin-4-yloxy]-piperidin-1-carbonsäure-tert-butylester;
(B31) 4-[6-(6-Acetylamino-4-methylpyridin-3-ylamino)pyrimidin-4-yloxy]-piperidin-1-carbonsäure-tert-butylester;
(B32) 4-[6-(5-Fluor-4-methylpyridin-2-ylamino)pyrimidin-4-yloxy]-piperidin-1-carbonsäure-tert-butylester;
(B33) 4-[6-(6-Methoxy-5-methylpyridin-3-ylamino)pyrimidin-4-yloxy]-piperidin-1-carbonsäure-tert-butylester;
(B34) 4-[6-(6-Methoxy-2-methylpyridin-3-ylamino)pyrimidin-4-yloxy]-piperidin-1-carbonsäure-tert-butylester;
(B35) 4-[6-(6-Fluor-5-methylpyridin-3-ylamino)pyrimidin-4-yloxy]-piperidin-1-carbonsäure-tert-butylester;
(B36) 4-[6-(2-Chlor-6-methylpyridin-3-ylamino)pyrimidin-4-yloxy]-piperidin-1-carbonsäure-tert-butylester;
(B37) 4-[6-(4-Methylpyridin-3-ylamino)pyrimidin-4-yloxy]-piperidin-1-carbonsäure-tert-butylester;
(B38) 4-[6-(2-Methylpyridin-3-ylamino)pyrimidin-4-yloxy]-piperidin-1-carbonsäure-tert-butylester;
(B39) 4-[6-(6-Chlor-2-methylpyridin-3-ylamino)pyrimidin-4-yloxy]-piperidin-1-carbonsäure-tert-butylester;
(B40) 4-[6-(6-Fluorpyridin-3-ylamino)pyrimidin-4-yloxy]-piperidin-1-carbonsäure-tert-butylester;
(B41) 4-[6-(2-Chlor-4-methylpyridin-3-ylamino)pyrimidin-4-yloxy]-piperidin-1-carbonsäure-tert-butylester;
(B42) 4-[6-(6-Methoxypyridin-3-ylamino)pyrimidin-4-yloxy]-piperidin-1-carbonsäure-tert-butylester;
(B43) 4-[6-(5-Fluorpyridin-2-ylamino)pyrimidin-4-yloxy]-piperidin-1-carbonsäure-tert-butylester;
(B44) 4-[6-(2-Fluorpyridin-3-ylamino)pyrimidin-4-yloxy]-piperidin-1-carbonsäure-tert-butylester;
(B45) 4-[6-(6-Chlor-5-methylpyridin-3-ylamino)pyrimidin-4-yloxy]-piperidin-1-carbonsäure-tert-butylester;
(B46) 4-[6-(2-Methylpyridin-4-ylamino)pyrimidin-4-yloxy]-piperidin-1-carbonsäure-tert-butylester;
(B47) 4-[6-(2-Methoxypyridin-3-ylamino)pyrimidin-4-yloxy]-piperidin-1-carbonsäure-tert-butylester;
(B48) 4-[6-(2,5-Difluorphenylamino)pyrimidin-4-yloxy]-piperidin-1-carbonsäure-tert-butylester;
(B49) 4-[6-(4-Chlor-2-fluorphenylamino)pyrimidin-4-yloxy]-piperidin-1-carbonsäure-tert-butylester;
(B50) 4-[6-(2,5-Difluorphenylamino)pyrimidin-4-yloxy]-piperidin-1-carbonsäureisopropylester;
(B51) 4-[6-(6-Methoxypyridin-3-ylamino)pyrimidin-4-yloxy]-piperidin-1-carbonsäureisopropylester;
(B52) 4-[6-(4-Cyano-3-methoxyphenylamino)pyrimidin-4-yloxy]-piperidin-1-carbonsäureisopropylester;
(B53) 4-[6-(3-Fluor-4-hydroxyphenylamino)pyrimidin-4-yloxy]-piperidin-1-carbonsäureisopropylester;
(B54) 4-[6-(6-Ethoxypyridin-3-ylamino)pyrimidin-4-yloxy]-piperidin-1-carbonsäureisopropylester;
(B55) 4-[6-(2,5-Difluor-4-isopropoxyphenylamino)pyrimidin-4-yloxy]-piperidin-1-carbonsäureisopropylester;
(B56) (2-Fluor-4-methansulfonylphenyl)-[6-(5'-isopropoxy-3,4,5,6-tetrahydro-2H[1,2']bipyridinyl-4-yloxy)pyrimidin-4-yl]amin;
(B57) (2-Fluor-4-methansulfonylphenyl)-{6-[1-(3-isopropyl[1,2,4]oxadiazol-5-yl)piperidin-4-yloxy]pyrimidin-4-yl}amin;
(B58) 4-[6-(4-Cyano-2-fluorphenylamino)pyrimidin-4-yloxy]-piperidin-1-carbonsäureisopropylester;
(B59) 4-[6-(Pyridin-3-ylamino)pyrimidin-4-yloxy]-piperidin-1-carbonsäureisopropylester;
(B60) 4-[6-(Pyridin-4-ylamino)pyrimidin-4-yloxy]-piperidin-1-carbonsäureisopropylester;
(B61) 4-[6-(2,5-Difluor-4-propoxyphenylamino)pyrimidin-4-yloxy]-piperidin-1-carbonsäureisopropylester;
(B62) 4-[6-(4-Ethylamino-2-fluorphenylamino)pyrimidin-4-yloxy]-piperidin-1-carbonsäureisopropylester;
(B63) 4-[6-(4-Dimethylamino-2-fluorphenylamino)pyrimidin-4-yloxy]-piperidin-1-carbonsäureisopropylester;
(B64) 4-[6-(2-Fluor-4-propylaminophenylamino)pyrimidin-4-yloxy]-piperidin-1-carbonsäureisopropylester;
(B65) 4-[6-(2-Fluor-4-isopropylaminophenylamino)pyrimidin-4-yloxy]-piperidin-1-carbonsäureisopropylester;
(B66) 4-[6-(2-Methyl-6-propylaminopyridin-3-ylamino)pyrimidin-4-yloxy]-piperidin-1-carbonsäureisopropylester;
(B67) 4-[6-(2-Methylpyridin-3-ylamino)pyrimidin-4-yloxy]-piperidin-1-carbonsäureisopropylester;
(B68) 4-[6-(6-Isopropylamino-2-methylpyridin-3-ylamino)pyrimidin-4-yloxy]-piperidin-1-carbonsäureisopropylester;

(B69) 4-[6-(2-Methyl-6-propoxypyridin-3-ylamino)pyrimidin-4-yloxy]-piperidin-1-carbonsäureisopropylester;
(B70) 4-[6-(4-Iod-2-methylphenylamino)pyrimidin-4-yloxy]-piperidin-1-carbonsäureisopropylester;
(B71) 4-[6-(2-Fluor-4-iodphenylamino)pyrimidin-4-yloxy]-piperidin-1-carbonsäureisopropylester;
(B77) 4-[6-(6-Methoxy-5-methylpyridin-3-ylamino)pyrimidin-4-yloxy]-piperidin-1-carbonsäureisopropylester;
(B78) 4-[6-(4-Methylpyridin-3-ylamino)pyrimidin-4-yloxy]-piperidin-1-carbonsäureisopropylester;
(B79) 4-[6-(4-Acetylamino-3-methylphenylamino)pyrimidin-4-yloxy]-piperidin-1-carbonsäureisopropylester;
(B80) 4-[6-(3-Chlor-4-fluorphenylamino)pyrimidin-4-yloxy]-piperidin-1-carbonsäureisopropylester;
(B81) 4-[6-(3,5-Dimethoxyphenylamino)pyrimidin-4-yloxy]-piperidin-1-carbonsäureisopropylester;
(B82) 4-[6-(6-Ethylpyridin-2-ylamino)pyrimidin-4-yloxy]-piperidin-1-carbonsäureisopropylester;
(B83) 4-[6-(5-Methylpyridin-2-ylamino)pyrimidin-4-yloxy]-piperidin-1-carbonsäurisopropylester;
(B84) 4-[6-(2-Methylchinolin-6-ylamino)pyrimidin-4-yloxy]-piperidin-1-carbonsäureisopropylester;
(B85) 4-[6-(2-Methylsulfanylbenzothiazol-6-ylamino)pyrimidin-4-yloxy]-piperidin-1-carbonsäureisopropylester;
(B86) 4-[6-(6-Morpholin-4-ylpyridin-3-ylamino)pyrimidin-4-yloxy]-piperidin-1-carbonsäureisopropylester;
(B88) 4-[6-(4-Piperidin-1-ylphenylamino)pyrimidin-4-yloxy]-piperidin-1-carbonsäureisopropylester;
(B89) 4-[6-(3-Trifluormethoxyphenylamino)pyrimidin-4-yloxy]-piperidin-1-carbonsäureisopropylester;
(B90) 4-[6-(5-Oxo-5,6,7,8-tetrahydronaphthalin-2-ylamino)pyrimidin-4-yloxy]-piperidin-1-carbonsäureisopropylester;
(B92) 4-[6-(5-Cyanopyridin-2-ylamino)pyrimidin-4-yloxy]-piperidin-1-carbonsäureisopropylester;
(B93) 4-[6-(4-Brom-2,5-difluorphenylamino)pyrimidin-4-yloxy]-piperidin-1-carbonsäureisopropylester;
(B94) 4-[6-(4-Trifluormethylpyridin-2-ylamino)pyrimidin-4-yloxy]-piperidin-1-carbonsäureisopropylester;
(B97) 4-[6-(2,6-Dimethylpyridin-3-ylamino)pyrimidin-4-yloxy]-piperidin-1-carbonsäureisopropylester;
(B98) 4-[6-(4-Cyano-2-methylphenylamino)pyrimidin-4-yloxy]-piperidin-1-carbonsäureisopropylester;
(B99) 4-[6-(4-Methoxy-2-methylphenylamino)pyrimidin-4-yloxy]-piperidin-1-carbonsäureisopropylester;
(B100) 4-[6-(2,4-Dimethoxyphenylamino)pyrimidin-4-yloxy]-piperidin-1-carbonsäureisopropylester;
(B101) 4-{6-[Acetyl-(2-fluor-4-methansulfonylphenyl)amino]pyrimidin-4-yloxy}-piperidin-1-carbonsäureisopropylester;
(B102) 4-[6-(5-Carbamoylpyridin-2-ylamino)pyrimidin-4-yloxy]-piperidin-1-carbonsäureisopropylester;
(B105) 4-[6-(3-Oxazol-5-ylphenylamino)pyrimidin-4-yloxy]-piperidin-1-carbonsäureisopropylester;
(B106) 4-[6-(5-Trifluormethylpyridin-2-ylamino)pyrimidin-4-yloxy]-piperidin-1-carbonsäureisopropylester;
(B107) 4-[6-(4-Chlor-2-trifluormethoxyphenylamino)pyrimidin-4-yloxy]-piperidin-1-carbonsäureisopropylester;
(B110) 4-[6-(1-Oxoindan-5-ylamino)pyrimidin-4-yloxy]-piperidin-1-carbonsäureisopropylester;
(B111) 4-{6-[5-(1-Methylpyrrolidin-2-yl)pyridin-2-ylamino]pyrimidin-4-yloxy}-piperidin-1-carbonsäureisopropylester;
(B112) 4-[6-(6-Methoxy-2-methylpyridin-3-ylamino)pyrimidin-4-yloxy]-piperidin-1-carbonsäureisopropylester;
(B113) 4-[6-(5-Brom-3-methylpyridin-2-ylamino)pyrimidin-4-yloxy]-piperidin-1-carbonsäureisopropylester;
(B114) 4-[6-(2-Chlor-6-methylpyridin-3-ylamino)pyrimidin-4-yloxy]-piperidin-1-carbonsäureisopropylester;
(B115) 4-[6-(2-Ethinylphenylamino)pyrimidin-4-yloxy]-piperidin-1-carbonsäureisopropylester;
(B116) 4-[6-(4-Brom-2-trifluormethoxyphenylamino)pyrimidin-4-yloxy]-piperidin-1-carbonsäureisopropylester;
(B117) 4-[6-(3-Iod-4-methylphenylamino)pyrimidin-4-yloxy]-piperidin-1-carbonsäureisopropylester;
(B118) 4-[6-(2-Fluor-5-methylphenylamino)pyrimidin-4-yloxy]-piperidin-1-carbonsäureisopropylester;
(B122) 4-[6-(2,5-Difluor-4-propylaminophenylamino)pyrimidin-4-yloxy]-piperidin-1-carbonsäureisopropylester;
(B123) 4-[6-(2,5-Difluor-4-morpholin-4-ylphenylamino)pyrimidin-4-yloxy]-piperidin-1-carbonsäureisopropylester;
(B124) 4-[6-(2-Methyl-4-propylaminophenylamino)pyrimidin-4-yloxy]-piperidin-1-carbonsäureisopropylester;
(B125) 4-{6-[2,5-Difluor-4-(4-methylpiperazin-1-yl)phenylamino]pyrimidin-4-yl-oxy}piperidin-1-carbonsäureisopropylester;
(B126) 4-{6-[2,5-Difluor-4-(2-pyrrolidin-1-ylethoxy)phenylamino]pyrimidin-4-yl-oxy}piperidin-1-carbonsäureisopropylester;
(B127) 4-{6-[4-(2-Dimethylaminoethoxy)-2,5-difluorphenylamino]pyrimidin-4-yl-oxy}piperidin-1-carbonsäureisopropylester;
(B128) 4-{6-[2,5-Difluor-4-(2-morpholin-4-ylethoxy)phenylamino]pyrimidin-4-yl-oxy}piperidin-1-carbonsäureisopropylester;
(B129) 4-[6-(2,4-Difluorphenylamino)pyrimidin-4-yloxy]-piperidin-1-carbonsäureisopropylester;
(B130) 4-[6-(2,4,5-Trifluorphenylamino)pyrimidin-4-yloxy]-piperidin-1-carbonsäureisopropylester;
(B131) 4-[6-(4-Methansulfonylphenylamino)pyrimidin-4-yloxy]-piperidin-1-carbonsäureisopropylester;
(B132) 4-{6-[Acetyl-(4-methansulfonylphenyl)amino]pyrimidin-4-yloxy}-piperidin-1-carbonsäureisopropylester;
(B133) (2,5-Difluor-4-propoxyphenyl)-{6-[1-(5-isopropyl[1,2,4]oxadiazol-3-yl)-piperidin-4-yloxy]pyrimidin-4-yl}amin;

(B134) 4-{6-[2,5-Difluor-4-(morpholin-4-ylamino)phenylamino]pyrimidin-4-yl-oxy}piperidin-1-carbonsäureisopropylester;
(B135) 4-{6-[2,5-Difluor-4-(2-methoxyethylamino)phenylamino]pyrimidin-4-yl-oxy}piperidin-1-carbonsäureisopropylester;
(B136) 4-(6-{2,5-Difluor-4-[(tetrahydrofuran-2-ylmethyl)amino]phenylamino}-pyrimidin-4-yloxy)piperidin-1-carbonsäureisopropylester;
(B137) 4-[6-(4-Butylamino-2,5-difluorphenylamino)pyrimidin-4-yloxy]-piperidin-1-carbonsäureisopropylester;
(B138) 4-{6-[2,5-Difluor-4-(3-methylbutylamino)phenylamino]pyrimidin-4-yl-oxy}piperidin-1-carbonsäureisopropylester;
(B139) 4-[6-(2-Fluor-4-methansulfonylphenylamino)-2-methylpyrimidin-4-yl-oxy]piperidin-1-carbonsäureisopropylester;
(B140) 4-{6-[2,5-Difluor-4-(2-morpholin-4-ylethylamino)phenylamino]pyrimidin-4-yloxy}piperidin-1-carbonsäureisopropylester;
(B141) 4-{6-[2-(2,5-Difluorphenoxy)ethylamino]pyrimidin-4-yloxy}-piperidin-1-carbonsäureisopropylester;
(B142) 4-[6-(2,5-Difluorphenoxy)pyrimidin-4-yloxy]-piperidin-1-carbonsäureisopropylester;
(B143) 4-[6-(4-Brom-2-fluorphenoxy)pyrimidin-4-yloxy]-piperidin-1-carbonsäureisopropylester;
(B144) 4-[6-(2-Fluor-4-morpholin-4-ylphenoxy)pyrimidin-4-yloxy]-piperidin-1-carbonsäureisopropylester;
(B145) 4-{6-[2,5-Difluor-4-(tetrahydrofuran-2-ylmethoxy)phenylamino]-pyrimidin-4-yloxy}piperidin-1-carbonsäureisopropylester;
(B149) 4-[4-(2-Fluor-4-methansulfonylphenylamino)pyridin-2-yloxy]-piperidin-1-carbonsäureisopropylester; und
(B150) 4-[4-(2,5-Difluor-4-propoxyphenylamino)pyridin-2-yloxy]-piperidin-1-carbonsäureisopropylester.

**21.** Verbindung nach Anspruch 1, worin die Verbindung aus den nachstehenden Verbindungen und pharmazeutisch annehmbaren Salzen, Solvaten, Hydraten und N-Oxiden davon ausgewählt ist:

(B153) 4-{6-[6-(2,3-Dihydroxypropylamino)-2-methylpyridin-3-yloxy]pyrimidin-4-yloxy}piperidin-1-carbonsäureisopropylester;
(B155) 4-[6-(2-Methylpyridin-3-yloxy)pyrimidin-4-yloxy]-piperidin-1-carbonsäureisopropylester;
(B156) 4-[6-(6-Brom-2-methylpyridin-3-yloxy)pyrimidin-4-yloxy]-piperidin-1-carbonsäureisopropylester; und
(B157) 4-[6-(2-Methylpyridin-3-yloxy)pyrimidin-4-yloxy]-piperidin-1-carbonsäure-tert-butylester.

**22.** Verbindung, ausgewählt aus Verbindungen der Formel (I) und pharmazeutisch annehmbaren Salzen, Solvaten, Hydraten und N-Oxide davon:

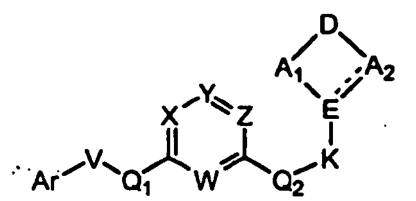

**(I)**

worin:

$A_1$ und $A_2$ unabhängig voneinander $C_{1-3}$-Alkylen sind, das gegebenenfalls mit einem oder mehreren Substituenten substituiert ist, die unabhängig voneinander aus $C_{1-6}$-Alkyl, $C_{1-6}$-Alkoxy und Carboxy ausgewählt sind;
D $CR_1R_2$ ist, worin $R_1$ aus H, $C_{1-6}$-Alkyl, $C_{1-6}$-Alkoxy, Halogen und Hydroxyl ausgewählt ist;
E N, C oder $CR_3$ ist, worin $R_3$ H oder $C_{1-6}$-Alkyl ist;
- - - eine Einfachbindung ist, wenn E N oder $CR_3$ ist, oder eine Doppelbindung ist, wenn E C ist;
K fehlt, eine $C_{3-6}$-Cycloalkylen- oder $C_{1-3}$-Alkylengruppe ist, die jeweils gegebenenfalls mit einem oder mehreren Substituenten substituiert sind, die unabhängig voneinander aus $C_{1-6}$-Alkyl, $C_{1-6}$-Alkoxy, Carboxy, Cyano und Halogen ausgewählt sind;
$Q_1$ NH ist;
$Q_2$ fehlt;
W CH ist;

X N oder $CR_6$ ist;
Y N oder $CR_7$ ist;
Z N ist;
V fehlt, $C_{1-3}$-Heteroalkylen oder $C_{1-3}$-Alkylen ist, die jeweils gegebenenfalls mit einem oder mehreren Substituenten substituiert sind, die unabhängig voneinander aus $C_{1-3}$-Alkyl, $C_{1-6}$-Alkoxy, Carboxy, Cyano, $C_{1-3}$-Halogenalkyl und Halogen ausgewählt sind;
$R_6$, $R_7$ und $R_8$ jeweils unabhängig voneinander ausgewählt sind aus: H, $C_{1-6}$-Acyl, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkyl-carboxamid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthiocarboxamid, $C_{1-6}$-Alkylthioureyl, $C_{1-6}$-Alkylureyl, Amino, Di-$C_{1-6}$-alkylamino, $C_{1-6}$-Alkoxycarbonyl, Carboxamid, Carboxy, Cyano, $C_{3-6}$-Cycloalkyl, Di-$C_{1-6}$-alkylcarboxamid, Di-$C_{1-6}$-alkylsulfonamid, Di-$C_{1-6}$-alkylthiocarboxamido, $C_{1-6}$-Halogenalkoxy, $C_{1-6}$-Halogenalkyl, Halogen, $C_{1-6}$-Halogenalkylsulfinyl, $C_{1-6}$-Halogenalkylsulfonyl, $C_{1-6}$-Halogenalkylthio, Hydroxyl, Hydroxylamino und Nitro, worin das $C_{2-6}$-Alkenyl, $C_{1-6}$-Alkyl, $C_{2-6}$-Alkinyl und $C_{3-6}$-Cycloalkyl jeweils gegebenenfalls mit einem oder mehreren Substituenten substituiert sind, die jeweils unabhängig voneinander ausgewählt sind aus: $C_{1-6}$-Acyl, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkylcarboxamid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthiocarboxamid, $C_{1-6}$-Alkylthioureyl, $C_{1-6}$-Alkylureyl, Amino, Di-$C_{1-6}$-alkylamino, $C_{1-6}$-Alkoxycarbonyl, Carboxamid, Carboxy, Cyano, $C_{3-6}$-Cycloalkyl, Di-$C_{1-6}$-alkylcarboxamid, Di-$C_{1-6}$-alkylsulfonamid, Di-$C_{1-6}$-alkylthiocarboxamido, $C_{1-6}$-Halogenalkoxy, $C_{1-6}$-Halogenalkyl, Halogen, $C_{1-6}$-Halogenalkylsulfinyl, $C_{1-6}$-Halogenalkylsulfonyl, $C_{1-6}$-Halogenalkylthio, Hydroxyl, Hydroxylamino und Nitro;
Ar Phenyl, Naphthyl, Pyridyl, Benzofuranyl, Pyrazinyl, Pyridazinyl, Pyrimidinyl, Triazinyl, Chinolin, Benzoxazol, Benzothiazol, 1H-Benzimidazol, Isochinolin, Chinazolin, Chinoxalin, Pyrrol oder Indol ist, die gegebenenfalls mit $R_9$, $R_{10}$, $R_{11}$, $R_{12}$ und $R_{13}$ substituiert sind;
$R^9$ ausgewählt ist aus: $C_{1-6}$-Acyl, $C_{1-6}$-Acylsulfonamid, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkylcarboxamid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthio-carboxamid, $C_{1-6}$-Alkylthioureyl, $C_{1-6}$-Alkylureyl, Amino, Aryl, Arylcarbonyl, Arylsulfonyl, Di-$C_{1-6}$-alkylamino, Carbamimidoyl, $C_{1-6}$-Alkoxycarbonyl, Carboxamid, Carboxy, Cyano, $C_{3-6}$-Cycloalkyl, Di-$C_{1-6}$-alkylcarboxamid, Di-$C_{1-6}$-alkylsulfonamid, Di-$C_{1-6}$-alkylthiocarboxamido, Guanidin, $C_{1-6}$-Halogenalkoxy, $C_{1-6}$-Halogenalkyl, Halogen, $C_{1-6}$-Halogenalkylsulfinyl, $C_{1-6}$-Halogenalkylsulfonyl, $C_{1-6}$-Halogenalkylthio, Heterocyclyl, Heterocyclylsulfonyl, Heteroaryl, Hydroxyl, Hydroxylamino, Nitro, $C_{3-6}$-Oxocycloalkyl, Phenoxy, Sulfonamid, Sulfonsäure und Thiol; und worin jedes $R_9$ gegebenenfalls mit einem oder mehreren Substituenten substituiert ist, die jeweils unabhängig voneinander ausgewählt sind aus: $C_{1-6}$-Acyl, $C_{1-6}$-Acylsulfonamid, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkylcarboxamid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthiocarbox-amid, $C_{1-6}$-Alkylthioureyl, $C_{1-6}$-Alkylureyl, Amino, Aryl, Arylcarbonyl, Arylsulfonyl, Di-$C_{1-6}$-alkylamino, $C_{1-6}$-Alkoxycarbonyl, Carboxamid, Carboxy, Cyano, $C_{3-6}$-Cycloalkyl, Di-$C_{1-6}$-alkylcarboxamid, Di-$C_{1-6}$-alkylsulfonamid, Di-$C_{1-6}$-alkylthiocarboxamido, $C_{1-6}$-Halogenalkoxy, $C_{1-6}$-Halogenalkyl, Halogen, $C_{1-6}$-Halogenalkylsulfinyl, $C_{1-6}$-Halogenalkylsulfonyl, $C_{1-6}$-Halogenalkylthio, Heteroaryl, Heteroarylcarbonyl, Heteroarylsulfonyl, Heterocyclyl, Hydroxyl, Hydroxylamino und Nitro;
$R_{10}$, $R_{11}$, $R_{12}$ und $R_{13}$ unabhängig voneinander ausgewählt sind aus: $C_{1-6}$-Acyl, $C_{1-6}$-Acyloxy, $C_{2-6}$-Alkenyl, $C_{1-6}$-Alkoxy, $C_{1-6}$-Alkyl, $C_{1-6}$-Alkylamino, $C_{1-6}$-Alkylcarbox-amid, $C_{2-6}$-Alkinyl, $C_{1-6}$-Alkylsulfonamid, $C_{1-6}$-Alkylsulfinyl, $C_{1-6}$-Alkylsulfonyl, $C_{1-6}$-Alkylthio, $C_{1-6}$-Alkylthiocarboxamid, $C_{1-6}$-Alkylthioureyl, $C_{1-6}$-Alkylureyl, Amino, Di-$C_{1-6}$-alkylamino, $C_{1-6}$-Alkoxycarbonyl, Carboxamid, Carboxy, Cyano, $C_{3-6}$-Cycloalkyl, Di-$C_{1-6}$-alkylcarboxamid, Di-$C_{1-6}$-alkylsulfonamid, Di-$C_{1-6}$-alkylthiocarboxamido, $C_{1-6}$-Halo-genalkoxy, $C_{1-6}$-Halogenalkyl, Halogen, $C_{1-6}$-Halogenalkylsulfinyl, $C_{1-6}$-Halogenalkylsulfonyl, $C_{1-6}$-Halogenalkylthio, Hydroxyl, Hydroxylamino, Nitro und Thiol; oder zwei benachbarte Gruppen von $R_{10}$, $R_{11}$, $R_{12}$ und $R_{13}$ gemeinsam mit den Atomen, an die sie gebunden sind, eine 5-, 6- oder 7-gliedrige Cycloalkylgruppe, 5-, 6-, oder 7-gliedrige Cycloalkenylgruppe oder 5-, 6- oder 7-gliedrige Heterocyclylgruppe bilden, worin die 5-, 6- oder 7-gliedrige Gruppe gegebenenfalls mit Halogen oder Oxo substituiert ist; und
$R_2$ ausgewählt ist aus: $C_{1-6}$-Alkyl, Aryl, Aryloxy, Heteroaryl und Heteroaryloxy; worin $R_2$ gegebenenfalls mit 1, 2, 3, 4 oder 5 Substituenten substituiert ist, die unabhängig voneinander ausgewählt sind aus: $C_{1-6}$-Alkoxy, $C_{1-6}$-Alkyl, $C_{1-6}$-Alkylamino, Amino, Di-$C_{1-6}$-alkylamino, $C_{1-6}$-Alkoxycarbonyl, Carboxy, Halogen und Heteroaryl, und worin $C_{1-6}$-Alkyl gegebenenfalls mit 1, 2 oder 3 Substituenten weiter substituiert ist, die unabhängig voneinander aus $C_{1-6}$-Alkylamino, Di-$C_{1-6}$-alkylamino, $C_{3-6}$-Cycloalkyl und Halogen ausgewählt sind.

**23.** Verbindung nach Anspruch 22, worin K fehlt.

**24.** Verbindung nach Anspruch 22 oder 23, worin V fehlt.

**25.** Verbindung nach einem der Ansprüche 22 bis 24, worin E N ist und D $CHR_2$ ist.

**26.** Verbindung nach einem der Ansprüche 22 bis 25, worin $A_1$ und $A_2$ beide $-CH_2CH_2-$ sind und $A_1$ und $A_2$ jeweils gegebenenfalls mit 1, 2, 3 oder 4 Methylgruppen substituiert sind.

**27.** Verbindung nach einem der Ansprüche 22 bis 26, worin:

W CH ist;
X N ist;
Y $CR_7$ ist, worin $R_7$ H, $C_{1-6}$-Alkyl oder Halogen ist; und
Z N ist.

**28.** Verbindung nach Anspruch 22, worin die Verbindung aus den nachstehenden Verbindungen und pharmazeutisch annehmbaren Salzen, Solvaten, Hydraten und N-Oxiden davon ausgewählt ist:

(A2) {6-[4-(3-Isopropyl[1,2,4]oxadiazol-5-yl)piperidin-1-yl]pyrimidin-4-yl}-(4-methansulfonylphenyl)amin;
(A8) (2-Fluor-4-methansulfonylphenyl)-{6-[4-(3-fluorphenoxy)piperidin-1-yl]-pyrimidin-4-yl}amin;
(A102) (2-Fluor-4-methansulfonylphenyl)-{6-[4-(3-isopropyl[1,2,4]oxadiazol-5-yl)piperidin-1-yl]pyrimidin-4-yl}amin;
(A108) (2-Fluor-4-methansulfonylphenyl)-{6-[4-(4-isobutylphenyl)piperidin-1-yl]pyrimidin-4-yl}amin;
(A109) (2-Fluor-4-methansulfonylphenyl)-{6-[4-(4-isopropylphenyl)piperidin-1-yl]pyrimidin-4-yl}amin;
(A110) {6-[4-(3-Cyclopropylmethyl[1,2,4]oxadiazol-5-yl)piperidin-1-yl]pyrimidin-4-yl}-(2-fluor-4-methansulfonylphenyl)amin;
(A111) (2-Fluor-4-methansulfonylphenyl)-{6-[4-(3-isobutyl[1,2,4]oxadiazol-5-yl)piperidin-1-yl]pyrimidin-4-yl}amin;
(A113) (2-Fluor-4-methansulfonylphenyl)-{6-[4-(4-isopropoxyphenyl)piperidin-1-yl]pyrimidin-4-yl}amin;
(A115) {6-[4-(3-Dimethylaminomethyl[1,2,4]oxadiazol-5-yl)piperidin-1-yl]-pyrimidin-4-yl}-(2-fluor-4-methansulfonylphenyl)amin;
(A117) (2-Fluor-4-methansulfonylphenyl)-{6-[4-(5-isopropoxypyridin-2-yloxy)-piperidin-1-yl]pyrimidin-4-yl}amin; und
(A118) (2-Fluor-4-methansulfonylphenyl)-{6-[4-(3-pyridin-3-yl[1,2,4]oxadiazol-5-yl)piperidin-1-yl]pyrimidin-4-yl}amin.

**29.** Pharmazeutische Zusammensetzung, die zumindest eine Verbindung nach einem der Ansprüche 1 bis 28 oder 4-[6-(2-Fluor-4-methansulfonylphenoxy)pyrimidin-4-yloxy]piperidin-1-carbonsäureisopropylester und einen pharmazeutisch annehmbaren Träger umfasst.

**30.** Verfahren zur Herstellung einer pharmazeutischen Zusammensetzung, umfassend das Vermischen zumindest einer Verbindung nach einem der Ansprüche 1 bis 28 oder von 4-[6-(2-Fluor-4-methansulfonylphenoxy)pyrimidin-4-yloxy]piperidin-1-carbonsäureisopropylester mit einem pharmazeutisch annehmbaren Träger.

**31.** Verbindung nach einem der Ansprüche 1 bis 28 oder 4-[6-(2-Fluor-4-methan-sulfonylphenoxy)pyrimidin-4-yloxy]piperidin-1 -carbonsäureisopropylester zur Verwendung in einem Behandlungsverfahren für den menschlichen oder tierischen Körper durch Therapie.

**32.** Verbindung nach einem der Ansprüche 1 bis 28 oder 4-[6-(2-Fluor-4-methan-sulfonylphenoxy)pyrimidin-4-yloxy]piperidin-1-carbonsäureisopropylester zur Verwendung in einem Verfahren zur Prophylaxe oder Behandlung einer mit dem Stoffwechsel in Verbindung stehenden Störung.

**33.** Verwendung einer Verbindung nach einem der Ansprüche 1 bis 28 oder von 4-[6-(2-Fluor-4-methansulfonylphenoxy)pyrimidin-4-yloxy]piperidin-1-carbonsäureiso-propylester zur Herstellung eines Medikaments zur Verwendung bei der Behandlung einer mit dem Stoffwechsel in Verbindung stehenden Störung.

**34.** Verbindung nach einem der Ansprüche 1 bis 28 oder 4-[6-(2-Fluor-4-methan-sulfonylphenoxy)pyrimidin-4-yloxy]piperidin-1-carbonsäureisopropylester zur Verwendung in einem Verfahren zur Behandlung von Typ-I-Diabetes, Typ-II-Diabetes, mangelhafter Glucosetoleranz, Insulinresistenz, Hyperglykämie, Hyperlipidämie, Hypertriglyceridämie, Hypercholesterinämie, Dyslipidämie oder Syndrom X.

**35.** Verwendung einer Verbindung nach einem der Ansprüche 1 bis 28 oder von 4-[6-(2-Fluor-4-methansulfonylphenoxy) pyrimidin-4-yloxy]piperidin-1-carbonsäureiso-propylester zur Herstellung eines Medikaments zur Verwendung bei der Behandlung von Typ-I-Diabetes, Typ-II-Diabetes, mangelhafter Glucosetoleranz, Insulinresistenz, Hyperglykämie, Hyperlipidämie, Hypertriglyceridämie, Hypercholesterinämie, Dyslipidämie oder Syndrom X.

## Revendications

**1.** Composé choisi parmi des composés de formule (I) et des sels, des solvates, des hydrates et des N-oxydes pharmaceutiquement acceptables de ceux-ci :

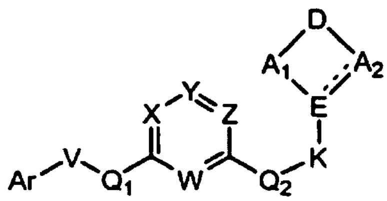

(I)

dans laquelle :

$A_1$ et $A_2$ sont tous les deux $-CH_2CH_2-$, et chacun de $A_1$ et $A_2$ est facultativement substitué par 1, 2, 3 ou 4 groupes méthyle ;
D est $CR_1R_2$ ou $NR_2$, où $R_1$ est choisi parmi H, alkyle en $C_{1\ à\ 6}$, alcoxy en $C_{1\ à\ 6}$, halogène et hydroxyle ;
E est N ou $CR_3$, où $R_3$ est H ou alkyle en $C_{1\ à\ 6}$;
- - - est une simple liaison ;
K est absent, un groupe cycloalkylène en $C_{3\ à\ 6}$, ou alkylène en $C_{1\ à\ 3}$ facultativement substitué chacun par un ou plusieurs substituants choisis indépendamment parmi un groupe alkyle en $C_{1\ à\ 6}$, un groupe alcoxy en $C_{1\ à\ 6}$, carboxy, cyano, et un atome d'halogène ;
$Q_1$ est $NR_4$, O, S, S(O), ou $S(O)_2$, où $R_4$ est H, un groupe acyle en $C_{1\ à\ 6}$, alkyle en $C_{1\ à\ 6}$, alcényle en $C_{2\ à\ 6}$, alcynyle en $C_{2\ à\ 6}$, cycloalkyle en $C_{3\ à\ 7}$, ou cycloalkyle en $C_{3\ à\ 7}$-alkylène en $C_{1\ à\ 3}$, où ledit groupe alkyle en $C_{1\ à\ 6}$ est facultativement substitué par un ou plusieurs substituants choisis indépendamment parmi un groupe acyle en $C_{1\ à\ 6}$, acyloxy en $C_{1\ à\ 6}$, alcényle en $C_{2\ à\ 6}$, alcoxy en $C_{1\ à\ 6}$, alkyle en $C_{1\ à\ 6}$, alkylamino en $C_{1\ à\ 6}$, alkylcarboxamide en $C_{1\ à\ 6}$, alcynyle en $C_{2\ à\ 6}$, alkylsulfonamide en $C_{1\ à\ 6}$, alkylsulfinyle en $C_{1\ à\ 6}$, alkylsulfonyle en $C_{1\ à\ 6}$, alkylthio en $C_{1\ à\ 6}$, alkylthiocarboxamide en $C_{1\ à\ 6}$, alkylthiouréyle en $C_{1\ à\ 6}$, alkyluréyle en $C_{1\ à\ 6}$, amino, di-alkylamino en $C_{1\ à\ 6}$, alcoxycarbonyle en $C_{1\ à\ 6}$, carboxamide, carboxy, cyano, cycloalkyle en $C_{3\ à\ 6}$, di-alkylcarboxamide en $C_{1\ à\ 6}$, di-alkylsulfonamide en $C_{1\ à\ 6}$, di-alkylthiocarboxamido en $C_{1\ à\ 6}$, halogénoalcoxy en $C_{1\ à\ 6}$, halogénoalkyle en $C_{1\ à\ 6}$, un atome d'halogène, un groupe halogénoalkylsulfinyle en $C_{1\ à\ 6}$, halogénoalkylsulfonyle en $C_{1\ à\ 6}$, halogénoalkylthio en $C_{1\ à\ 6}$, hydroxyle, hydroxylamino et nitro ;
$Q_2$ est $NR_5$ ou O, où $R_5$ est H, un groupe acyle en $C_{1\ à\ 6,}$ alkyle en $C_{1\ à\ 6}$, alcényle en $C_{2\ à\ 6}$, alcynyle en $C_{2\ à\ 6}$, cycloalkyle en $C_{3\ à\ 7}$, ou cycloalkyle en $C_{3\ à\ 7}$-alkylène en $C_{1\ à\ 3}$, où ledit groupe alkyle en $C_{1\ à\ 6}$ est facultativement substitué par un ou plusieurs substituants choisis indépendamment parmi un groupe acyle en $C_{1\ à\ 6}$, acyloxy en $C_{1\ à\ 6}$, alcényle en $C_{2\ à\ 6}$, alcoxy en $C_{1\ à\ 6}$, alkyle en $C_{1\ à\ 6}$, alkylamino en $C_{1\ à\ 6}$, alkylcarboxamide en $C_{1\ à\ 6}$, alcynyle en $C_{2\ à\ 6}$, alkylsulfonamide en $C_{1\ à\ 6}$, alkylsulfinyle en $C_{1\ à\ 6}$, alkylsulfonyle en $C_{1\ à\ 6}$, alkylthio en $C_{1\ à\ 6}$, alkylthiocarboxamide en $C_{1\ à\ 6}$, alkylthiouréyle en $C_{1\ à\ 6}$, alkyluréyle en $C_{1\ à\ 6}$, amino, di-alkylamino en $C_{1\ à\ 6}$, alcoxycarbonyle en $C_{1\ à\ 6}$, carboxamide, carboxy, cyano, cycloalkyle en $C_{3\ à\ 6}$, di-alkylcarboxamide en $C_{1\ à\ 6}$, di-alkylsulfonamide en $C_{1\ à\ 6}$, di-alkylthiocarboxamido en $C_{1\ à\ 6}$, halogénoalcoxy en $C_{1\ à\ 6}$, halogénoalkyle en $C_{1\ à\ 6}$, un atome d'halogène, un groupe halogénoalkylsulfinyle en $C_{1\ à\ 6}$, halogénoalkylsulfonyle en $C_{1\ à\ 6}$, halogénoalkylthio en $C_{1\ à\ 6}$, hydroxyle, hydroxylamino et nitro ;
W est CH ;
X est N ou $CR_6$ ;
Y est N ou Cru ;
Z est N ;
V est absent, un groupe hétéroalkylène en $C_{1\ à\ 3}$, ou alkylène en $C_{1\ à\ 3}$ où chacun est facultativement substitué par un ou plusieurs substituants choisis indépendamment parmi un groupe alkyle en $C_{1\ à\ 3}$, alcoxy en $C_{1\ à\ 6}$,

carboxy, cyano, halogénoalkyle en $C_{1\ à\ 3}$, et un atome d'halogène ;

$R_6$, $R_7$, et $R_8$ sont chacun indépendamment choisis parmi H, un groupe acyle en $C_{1\ à\ 6}$, acyloxy en $C_{1\ à\ 6}$, alcényle en $C_{2\ à\ 6}$, alcoxy en $C_{1\ à\ 6}$, alkyle en $C_{1\ à\ 6}$, alkylamino en $C_{1\ à\ 6}$, alkylcarboxamide en $C_{1\ à\ 6}$, alcynyle en $C_{2\ à\ 6}$, alkylsulfonamide en $C_{1\ à\ 6}$, alkylsulfinyle en $C_{1\ à\ 6}$, alkylsulfonyle en $C_{1\ à\ 6}$, alkylthio en $C_{1\ à\ 6}$, alkylthiocarboxamide en $C_{1\ à\ 6}$, alkylthiouréyle en $C_{1\ à\ 6}$, alkyluréyle en $C_{1\ à\ 6}$, amino, di-alkylamino en C1 $_{à\ 6}$, alcoxycarbonyle en $C_{1\ à\ 6}$, carboxamide, carboxy, cyano, cycloalkyle en $C_{3\ à\ 6}$, di-alkylcarboxamide en $C_{1\ à\ 6}$, di-alkylsulfonamide en $C_{1\ à\ 6}$, di-alkylthiocarboxamido en $C_{1\ à\ 6}$, halogénoalcoxy en $C_{1\ à\ 6}$, halogénoalkyle en $C_{1\ à\ 6}$, un atome d'halogène, un groupe halogénoalkylsulfinyle en $C_{1\ à\ 6}$, halogénoalkylsulfonyle en $C_{1\ à\ 6}$, halogénoalkylthio en $C_{1\ à\ 6}$, hydroxyle, hydroxylamino et nitro, où lesdits groupes alcényle en $C_{2\ à\ 6}$, alkyle en $C_{1\ à\ 6}$, alcynyle en $C_{2\ à\ 6}$ et cycloalkyle en $C_{3\ à\ 6}$ sont chacun facultativement substitués par un ou plusieurs substituants indépendamment choisis parmi un groupe acyle en $C_{1\ à\ 6}$, acyloxy en $C_{1\ à\ 6}$, alcényle en $C_{2\ à\ 6}$, alcoxy en $C_{1\ à\ 6}$ alkyle en $C_{1\ à\ 6}$, alkylamino en $C_{1\ à\ 6}$, alkylcarboxamide en $C_{1\ à\ 6}$, alcynyle en $C_{2\ à\ 6}$, alkylsulfonamide en $C_{1\ à\ 6}$, alkylsulfinyle en $C_{1\ à\ 6}$, alkylsulfonyle en $C_{1\ à\ 6}$, alkylthio en $C_{1\ à\ 6}$, alkylthiocarboxamide en $C_{1\ à\ 6}$, alkylthiouréyle en $C_{1\ à\ 6}$, alkyluréyle en $C_{1\ à\ 6}$, amino, di-alkylamino en $C_{1\ à\ 6}$ alcoxycarbonyle en $C_{1\ à\ 6}$, carboxamide, carboxy, cyano, cycloalkyle en $C_{3\ à\ 6}$, di-alkylcarboxamide en $C_{1\ à\ 6}$, di-alkylsulfonamide en $C_{1\ à\ 6}$, di-alkylthiocarboxamido en $C_{1\ à\ 6}$, halogénoalcoxy en $C_{1\ à\ 6}$, halogénoalkyle en $C_{1\ à\ 6}$, un atome d'halogène, un groupe halogénoalkylsulfinyle en $C_{1\ à\ 6}$, halogénoalkylsulfonyle en $C_{1\ à\ 6}$, halogénoalkylthio en $C_{1\ à\ 6}$, hydroxyle, hydroxylamino et nitro ;

Ar est un groupe phényle, naphtyle, pyridyle, benzofuranyle, pyrazinyle, pyridazinyle, pyrimidinyle, triazinyle, quinoléine, benzoxazole, benzothiazole, 1H-benzimidazole, isoquinoléine, quinazoline, quinoxaline, pyrrole, ou indole, facultativement substitué par $R_9$, $R_{10}$, $R_{11}$, $R_{12}$ , et $R_{13}$ ;

$R_9$ est choisi parmi un groupe acyle en $C_{1\ à\ 6}$ acylsulfonamide en $C_{1\ à\ 6}$, acyloxy en $C_{1\ à\ 6}$, alcényle en $C_{2\ à\ 6}$, alcoxy en $C_{1\ à\ 6}$, alkyle en $C_{1\ à\ 6}$, alkylamino en $C_{1\ à\ 6}$, alkylcarboxamide en $C_{1\ à\ 6}$, alcynyle en $C_{2\ à\ 6}$, alkylsulfonamide en $C_{1\ à\ 6}$, alkylsulfinyle en $C_{1\ à\ 6}$ alkylsulfonyle en $C_{1\ à\ 6}$, alkylthio en $C_{1\ à\ 6}$ alkylthiocarboxamide en $C_{1\ à\ 6}$, alkylthiouréyle en $C_{1\ à\ 6}$, alkyluréyle en $C_{1\ à}$ 6, amino, aryle, arylcarbonyle, arylsulfonyle, di-alkylamino en $C_{1\ à\ 6}$, carbamimidoyle, alcoxycarbonyle en $C_{1\ à\ 6}$, carboxamide, carboxy, cyano, cycloalkyle en $C_{3\ à\ 6}$, di-alkylcarboxamide en $C_{1\ à\ 6}$, di-alkylsulfonamide en $C_{1\ à\ 6}$, di-alkylthiocarboxamido en $C_{1\ à\ 6}$, guanidine, halogénoalcoxy en $C_{1\ à\ 6}$, halogénoalkyle en $C_{1\ à\ 6}$, un atome d'halogène, un groupe halogénoalkylsulfinyle en $C_{1\ à\ 6}$, halogénoalkylsulfonyle en $C_{1\ à\ 6}$, halogénoalkylthio en $C_{1\ à\ 6}$, hétérocyclique, sulfonyle hétérocyclique, hétéroaryle, hydroxyle, hydroxylamino, nitro, oxo en $C_{3\ à\ 6}$-cycloalkyle, phénoxy, sulfonamide, acide sulfonique et thiol ; et où chaque $R_9$ est facultativement substitué avec un ou plusieurs substituants choisis indépendamment parmi un groupe acyle en $C_{1\ à\ 6}$, acylsulfonamide en $C_{1\ à\ 6}$, acyloxy en $C_{1\ à\ 6}$, alcényle en $C_{2\ à\ 6}$, alcoxy en $C_{1\ à\ 6}$, alkyle en $C_{1\ à\ 6}$, alkylamino en $C_{1\ à\ 6}$, alkylcarboxamide en $C_{1\ à\ 6}$, alcynyle en $C_{2\ à\ 6}$, alkylsulfonamide en $C_{1\ à\ 6}$, alkylsulfinyle en $C_{1\ à\ 6}$, alkylsulfonyle en $C_{1\ à\ 6}$, alkylthio en $C_{1\ à\ 6}$, alkylthiocarboxamide en $C_{1\ à\ 6}$, alkylthiouréyle en $C_{1\ à\ 6}$, alkyluréyle en $C_{1\ à\ 6}$, amino, aryle, arylcarbonyle, arylsulfonyle, di-alkylamino en $C_{1\ à\ 6}$, alcoxycarbonyle en $C_{1\ à\ 6}$, carboxamide, carboxy, cyano, cycloalkyle en $C_{3\ à\ 6}$, di-alkylcarboxamide en $C_{1\ à\ 6}$, di-alkylsulfonamide en $C_{1\ à\ 6}$, di-alkylthiocarboxamido en $C_{1\ à\ 6}$, halogénoalcoxy en $C_{1\ à\ 6}$, halogénoalkyle en $C_{1\ à\ 6}$, un atome d'halogène, un groupe halogénoalkylsulfinyle en $C_{1\ à\ 6}$, halogénoalkylsulfonyle en $C_{1\ à\ 6}$, halogénoalkylthio en $C_{1\ à\ 6}$, hétéroaryle, hétéroarylcarbonyle, hétéroarylsulfonyle, hétérocyclique, hydroxyle, hydroxylamino, et nitro ;

$R_{10}$, $R_{11}$, $R_{12}$ et $R_{13}$ sont indépendamment choisis parmi un groupe acyle en $C_{1\ à\ 6}$, acyloxy en $C_{1\ à\ 6}$, alcényle en $C_{2\ à\ 6}$, alcoxy en $C_{1\ à\ 6}$, alkyle en $C_{1\ à\ 6}$, alkylamino en $C_{1\ à\ 6}$, alkylcarboxamide en $C_{1\ à\ 6}$, alcynyle en $C_2$ à $_6$, alkylsulfonamide en $C_{1\ à\ 6}$, alkylsulfinyle en $C_{1\ à\ 6}$ alkylsulfonyle en $C_{1\ à\ 6}$, alkylthio en $C_{1\ à\ 6}$, alkylthiocarboxamide en $C_{1\ à\ 6}$, alkylthiouréyle en $C_{1\ à\ 6}$, alkyluréyle en $C_{1\ à\ 6}$, amino, di-alkylamino en $C_1$ à $_6$, alcoxycarbonyle en $C_{1\ à\ 6}$, carboxamide, carboxy, cyano, cycloalkyle en $C_{3\ à\ 6}$, di-alkylcarboxamide en $C_{1\ à\ 6}$, di-alkylsulfonamide en $C_{1\ à\ 6}$, di-alkylthiocarboxamido en $C_{1\ à\ 6}$, halogénoalcoxy en $C_{1\ à\ 6}$, halogénoalkyle en $C_{1\ à\ 6}$, un atome d'halogène, un groupe halogénoalkylsulfinyle en $C_{1\ à\ 6}$, halogénoalkylsulfonyle en $C_{1\ à\ 6}$, halogénoalkylthio en $C_{1\ à\ 6}$, hydroxyle, hydroxylamino, nitro, et thiol ; ou deux groupes $R_{10}$, $R_{11}$, $R_{12}$ et $R_{13}$ adjacents conjointement avec les atomes auxquels ils sont liés forment un groupe cycloalkyle à 5, 6 ou 7 chaînons, un groupe cycloalcényle à 5, 6 ou 7 chaînons ou un groupe hétérocyclique à 5, 6 ou 7 chaînons où le groupe à 5, 6 ou 7 chaînons est facultativement substitué par un atome d'halogène ou un groupe oxo ; et

soit :

$R_2$ est un groupe aryle, arylcarbonyle, alcoxycarbonyle en $C_{1\ à\ 6}$, cycloalcoxycarbonyle en $C_{3\ à\ 7}$, hétéroaryle, ou hétéroarylcarbonyle ; chacun facultativement substitué par un ou plusieurs substituants choisis indépendamment parmi un groupe acyle en $C_{1\ à\ 6}$, acyloxy en $C_{1\ à\ 6}$, alcényle en $C_{2\ à\ 6}$, alcoxy en $C_{1\ à\ 6}$, alkyle en $C_{1\ à\ 6}$, alkylamino en $C_{1\ à\ 6}$, alkylcarboxamide en $C_{1\ à\ 6}$, alcynyle en $C_{2\ à\ 6}$, alkylsulfonamide en $C_{1\ à\ 6}$, alkylsulfinyle en $C_{1\,à\,6}$, alkylsulfonyle en $C_{1\,à\,6}$, alkylthio en $C_{1\,à\,6}$, alkylthiocarboxamide en $C_{1\,à\,6}$, alkylthiouréyle en $C_{1\,à\,6}$, alkyluréyle en $C_{1\,à\,6}$, amino, aryle, di-alkylamino en $C_{1\,à\,6}$, carbo-alcoxy en $C_{1\,à\,6}$, carboxamide, carboxy, cyano, cycloalkyle en $C_{3\,à\,6}$, di-alkylcarboxamide en $C_{1\,à\,6}$, di-alkylsulfonamide en $C_{1\,à\,6}$, di-alkylthiocarboxamido en $C_{1\,à\,6}$, halogénoalcoxy en $C_{1\,à\,6}$, halogénoalkyle en $C_{1\,à\,6}$, un atome d'halogène, un groupe halogénoalkylsulfinyle en $C_{1\,à\,6}$, halogénoalkylsulfonyle en $C_{1\,à\,6}$, halogénoalkylthio en $C_1$ à $_6$, hétérocyclique, hétéroaryle, hydroxyle, hydroxylamino, nitro, et thiol ;

ou :

$R_2$ est un groupe alcoxycarbonyle en $C_{1\,à\,6}$. alcoxycarbonyle en $C_{1\,à\,6}$ substitué par un groupe cycloalkyle en $C_{3\,à\,6}$, cycloalcoxycarbonyle en $C_{3\,à\,7}$, ou hétéroaryl-alkylène en $C_{1\,à\,3}$ facultativement substitué par 1, 2 ou 3 groupes alkyle en $C_{1\,à\,6}$;
à condition que le composé ne soit pas de l'ester d'isopropyle d'acide 4-[6-(2-fluoro-4-méthanesulfonyl-phénoxy)-pyrimidin-4-yloxy]-pipéridine-1-carboxylique.

**2.** Composé selon la revendication 1, dans lequel K est absent.

**3.** Composé selon la revendication 1, dans lequel K est $-CH_2-$.

**4.** Composé selon l'une quelconque des revendications 1 à 3, dans lequel V est absent.

**5.** Composé selon l'une quelconque des revendications 1 à 4, dans lequel $Q_1$ est O.

**6.** Composé selon l'une quelconque des revendications 1 à 4, dans lequel $Q_1$ est NH.

**7.** Composé selon l'une quelconque des revendications 1 à 6, dans lequel $Q_2$ est O.

**8.** Composé selon l'une quelconque des revendications 1 à 6, dans lequel $Q_2$ est $NR_5$.

**9.** Composé selon la revendication 8, dans lequel $R_5$ est H ou un groupe alkyle en $C_{1\,à\,6}$ .

**10.** Composé selon l'une quelconque des revendications 1 à 9, dans lequel :

W est CH ;
X est N ;
Y est $CR_7$, dans lequel $R_7$ est H, un groupe alkyle en $C_{1\,à\,6}$, ou un atome d'halogène ; et
Z est N.

**11.** Composé selon l'une quelconque des revendications 1 à 10, dans lequel E est CH et D est $NR_2$.

**12.** Composé selon l'une quelconque des revendications 1 à 11, dans lequel $R_2$ est aryle, arylcarbonyle, alcoxycarbonyle en $C_{1\,à\,6}$, cycloalcoxycarbonyle en $C_{3\,à\,7}$, hétéroaryle, ou hétéroarylcarbonyle ; chacun facultativement substitué par un ou plusieurs substituants choisis indépendamment parmi un groupe acyle en $C_{1\,à\,6}$, acyloxy en $C_{1\,à\,6}$, alcényle en $C_{2\,à\,6}$, alcoxy en $C_{1\,à\,6}$, alkyle en $C_{1\,à\,6}$, alkylamino en $C_{1\,à\,6}$, alkylcarboxamide en $C_{1\,à\,6}$, alcynyle en $C_{2\,à\,6}$, alkylsulfonamide en $C_{1\,à\,6}$, alkylsulfinyle en $C_{1\,à\,6}$, alkylsulfonyle en $C_{1\,à\,6}$, alkylthio en $C_{1\,à\,6}$, alkylthiocarboxamide en $C_{1\,à\,6}$, alkylthiouréyle en $C_{1\,à\,6}$, alkyluréyle en $C_{1\,à\,6}$, amino, aryle, di-alkylamino en $C_{1\,à\,6}$, carbo-alcoxy en $C_{1\,à\,6}$, carboxamide, carboxy, cyano, cycloalkyle en $C_{3\,à\,6}$, di-alkylcarboxamide en $C_{1\,à\,6}$, di-alkylsulfonamide en $C_{1\,à\,6}$, di-alkylthiocarboxamido en $C_{1\,à\,6}$, halogénoalcoxy en $C_{1\,à\,6}$, halogénoalkyle en $C_{1\,à\,6}$, un atome d'halogène, un groupe halogénoalkylsulfinyle en $C_1$ à $_6$, halogénoalkylsulfonyle en $C_{1\,à\,6}$, halogénoalkylthio en $C_{1\,à\,6}$, hétérocyclique, hétéroaryle, hydroxyle, hydroxylamino, nitro, et thiol.

**13.** Composé selon l'une quelconque des revendications 1 à 11, dans lequel $R_2$ est un groupe alcoxycarbonyle en $C_{1\,à\,6}$, alcoxycarbonyle en $C_{1\,à\,6}$ substitué par un groupe cycloalkyle en $C_{3\,à\,6}$, cycloalcoxycarbonyle en $C_{3\,à\,7}$, ou hétéroaryl-alkylène en $C_{1\,à\,3}$ facultativement substitué par 1, 2 ou 3 groupes alkyle en $C_{1\,à\,6}$.

**14.** Composé selon l'une quelconque des revendications 1 à 13, dans lequel Ar est un groupe phényle facultativement substitué par $R_9$, $R_{10}$, $R_{11}$, $R_{12}$ et $R_{13}$ ;
$R_9$ est choisi parmi les groupes méthanesulfonyle, 2-méthanesulfonyl-éthyle, acétylsulfamoyle, propionylsulfamoyle,

éthylsulfanyle, isopropylsulfanyle, éthylsulfamoyle, méthylsulfamoyle, diméthylsulfamoyle, méthylsulfamoylméthyle, sulfamoyle, [1,2,4]triazol-1-yle, [1,2,4]triazol-1-ylméthyle, 2-[1,2,4]triazol-1-yl-éthyle, méthoxy, 2-oxo-oxazolidin-4-ylméthyle, 1,1-dioxo-1$\lambda^6$-thiomorpholin-4-ylméthyle, pyrazol-1-yle, trifluorométhanesulfonyle, morpholine-4-sulfonyle, pyridine-2-carbonyle, F, Cl, cyano, Br, carboxy, butyryle, propoxycarbonyle, hydroxy, propylcarbamoyle, N-hydroxycarbamimidoyle, carbamimidoyle, N-éthylcarbamimidoyle, et 2-aminoéthylamino ; et
$R_{10}$, $R_{11}$, $R_{12}$ et $R_{13}$ sont indépendamment choisis parmi F, un groupe méthoxy, méthyle, éthyle et carboxy.

**15.** Composé selon l'une quelconque des revendications 1 à 13, dans lequel Ar est un groupe phényle facultativement substitué par $R_9$, $R_{10}$, $R_{11}$, $R_{12}$ et $R_{13}$ ;
$R_9$ est choisi parmi les groupes méthanesulfonyle, cyano, F, Cl, Br, I, méthyle, méthoxy, éthylamino, éthylsulfanyle, isopropylsulfanyle, hydroxy, isopropoxy, propoxy, diméthylamino, propylamino, isopropylamino, acétylamino, pipéridin-1-yle, trifluorométhoxy, oxazol-5-yle, éthynyle, 3-méthyl-butylamino, 2-morpholin-4-yl-éthylamino, acétylsulfamoyle, propionylsulfamoyle, tétrahydro-furan-2-ylméthoxy, morpholin-4-yle, 4-méthyl-pipérazin-1-yle, butylamino, 2-pyrrolidin-1-yl-éthoxy, 2-diméthylamino-éthoxy, 2-morpholin-4-yl-éthoxy, morpholin-4-ylamino, 2-méthoxyéthylamino, et tétrahydro-furan-2-ylméthyl-amino ; et
$R_{10}$, $R_{11}$, $R_{12}$ et $R_{13}$ sont indépendamment choisis parmi F, Cl, Br, I, hydroxyle, méthoxy, cyano, méthyle, et trifluorométhoxy.

**16.** Composé selon l'une quelconque des revendications 1 à 13, dans lequel Ar est choisi parmi les groupes pyridin-2-yle, pyridin-3-yle, pyridin-4-yle, quinoléin-6-yle, et benzothiazol-6-yle, chacun facultativement substitué par $R_9$, $R_{10}$, $R_{11}$, $R_{12}$ et $R_{13}$ ;
$R_9$ est choisi parmi les groupes méthanesulfonyle, méthoxy, carboxy, acétylsulfamoyle, propionylsulfamoyle, acétylamino, F, Cl, Br, méthyle, et trifluorométhyle ; et
$R_{10}$, $R_{11}$, $R_{12}$ et $R_{13}$ sont indépendamment choisis parmi les groupes méthoxy, méthyle, F, Cl, et Br.

**17.** Composé selon l'une quelconque des revendications 1 à 13, dans lequel Ar est choisi parmi les groupes pyridine-2-yle, pyridine-3-yle, pyridine-4-yle, quinoléin-6-yle, et benzothiazol-6-yle, facultativement substitué par $R_9$, $R_{10}$, $R_{11}$, $R_{12}$ et $R_{13}$ ;
$R_9$ est choisi parmi les groupes cyano, F, Cl, Br, acétylamino, méthoxy, méthyle, propoxy, propylamino, isopropylamino, phényle, t-butyle, 4-méthylphényle, éthyle, méthylsulfanyle, morpholin-4-yle, benzènesulfonyle, trifluorométhyle, cyclopropyle, carbamoyle, 3,4-difluorophényle, 4-chlorophényle, 1-méthyl-pyrrolidin-2-yle, acétylsulfamoyle, propionylsulfamoyle, et pyridine-2-yle ; et
$R_{10}$, $R_{11}$, $R_{12}$ et $R_{13}$ sont indépendamment méthyle, F ou C1.

**18.** Composé selon la revendication 1, choisi parmi des composés ayant la formule (IIIi) et des sels, solvates, hydrates et N-oxydes pharmaceutiquement acceptables de ceux-ci :

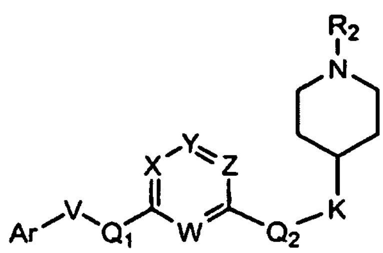

(IIIi)

dans laquelle :

K est absent ou un groupe alkylène en $C_{1\ à\ 3}$ ;
$Q_1$ est NH ou O ;
$Q_2$ est $NR_5$ ou O, dans lequel $R_5$ est H ou un groupe alkyle en $C_{1\ à\ 6}$;
W est CH, X est N, Y est CH, et Z est N ;
V est absent ;
Ar est choisi parmi les groupes pyridine-2-yle, pyridine-3-yle, pyridine-4-yle, quinoléin-6-yle, et benzothiazol-6-yle, chacun facultativement substitué par $R_9$, $R_{10}$, $R_{11}$, $R_{12}$ et $R_{13}$ ;
$R_9$ est un groupe acyle en $C_{1\ à\ 6}$, acylsulfonamide en $C_{1\ à\ 6}$, alcoxy en $C_{1\ à\ 6}$, alkyle en $C_{1\ à\ 6}$, alkylamino en $C_{1\ à\ 6}$, alkylcarboxamide en $C_1$ à $_6$, alcynyle en $C_{2\ à\ 6}$, alkylsulfonamide en $C_{1\ à\ 6}$, alkylsulfinyle en $C_{1\ à\ 6}$, alkylsulfonyle en $C_{1\ à\ 6}$, alkylthio en $C_{1\ à\ 6}$, amino, aryle, arylsulfonyle, di-alkylamino en $C_1$ à $_6$, carbamimidoyle, alcoxycarbonyle en $C_{1\ à\ 6}$, carboxamide, carboxy, cyano, cycloalcoxy en $C_{3\ à\ 6}$, cycloalkyle en $C_{3\ à\ 6}$, di-alkylsulfonamide en $C_{1\ à\ 6}$, guanidine, halogénoalcoxy en $C_{1\ à\ 6}$, halogénoalkyle en $C_{1\ à\ 6}$, un atome d'halogène, un groupe halogénoalkylsulfonyle en $C_{1\ à\ 6}$, hétéroaryle, hétéroarylcarbonyle, hétéroarylsulfonyle, hétérocyclique, sulfonyle hétérocyclique, hydroxyle, sulfonamide, ou thiol ; où les groupes alcoxy en $C_{1\ à\ 6}$, alkyle en $C_{1\ à\ 6}$, alkylamino en $C_{1\ à\ 6}$, amino, aryle, carbamimidoyle, hétérocyclique sont facultativement substitués par 1, 2, 3 ou 4 substituants choisis indépendamment parmi les groupes alcoxy en $C_{1\ à\ 6}$, alkyle en $C_{1\ à\ 6}$, alcynyle en $C_{2\ à\ 6}$, alkylsulfonamide en $C_{1\ à\ 6}$, alkylsulfonyle en $C_{1\ à\ 6}$, amino, aryle, cycloalkyle en $C_{3\ à\ 6}$, di-alkylamino en $C_{1\ à\ 6}$, un atome d'halogène, les groupes hétéroaryle, hétérocyclique, et hydroxyle ;
$R_{10}$, $R_{11}$, $R_{12}$ et $R_{13}$ sont indépendamment choisis parmi les groupes alcoxy en $C_{1\ à\ 6}$, alkyle en $C_{1\ à\ 6}$, alkylamino en $C_{1\ à\ 6}$, carboxy, cyano, un atome d'halogène, les groupes halogénoalcoxy en $C_{1\ à\ 6}$, halogénoalkylsulfonyle en $C_{1\ à\ 6}$, et hydroxyle ; ou deux groupes $R_{10}$, $R_{11}$, $R_{12}$ et $R_{13}$ adjacents conjointement avec les atomes auxquels ils sont liés forment un groupe cycloalkyle à 5 ou 6 chaînons ou un groupe hétérocyclique à 5 à 6 chaînons, où ledit groupe à 5 ou 6 chaînons est facultativement substitué par un atome halogène ou un groupe oxo ; et
$R_2$ est un groupe tert-butoxycarbonyle, isobutoxycarbonyle, isopropoxycarbonyle, butoxycarbonyle, cyclopropylméthoxycarbonyle, 3-méthyl-butoxycarbonyle, ou 3-isopropyl-[1,2,4]oxadiazol-5-yle.

**19.** Composé selon la revendication 1, dans lequel ledit composé est choisi parmi les composés et sels, solvates, hydrates et N-oxydes pharmaceutiquement acceptables de ceux-ci suivantes :

(A3) l'ester de tert-butyle d'acide 4-{[6-(2-fluoro-4-méthanesulfonyl-phénylamino)-pyrimidin-4-yl]-méthylamino}-pipéridine-1-carboxylique ;
(A4) l'ester de tert-butyle d'acide 4-({[6-(2-fluoro-4-méthanesulfonyl-phénylamino)-pyrimidin-4-yl]-méthylamino}-méthyl)-pipéridine-1-carboxylique ;
(A5) l'ester de tert-butyle d'acide 4-({[6-(4-méthanesulfonyl-phénylamino)-pyrimidin-4-yl]-méthyl-amino}-méthyl)-pipéridine-1-carboxylique ;
(A6) l'ester de tert-butyle d'acide 4-({[6-(2,5-difluoro-benzylamino)-pyrimidin-4-yl]-méthyl-amino}-méthyl)-pipéridine-1-carboxylique ;
(A7) l'ester de tert-butyle d'acide 4-[({6-[(benzo[1,3]dioxol-5-ylméthyl)-amino]-pyrimidin-4-yl}-méthylamino)-méthyl]-pipéridine-1-carboxylique ;
(A9) l'ester de tert-butyle d'acide 4-({méthyl-[6-(2-pyridin-4-yl-éthylamino)-pyrimidin-4-yl]-amino}-méthyl)-pipéridine-1-carboxylique ;
(A10) l'ester de tert-butyle d'acide 4-({méthyl-[6-(2-pyridin-3-yl-éthylamino)-pyrimidin-4-yl]-amino}-méthyl)-pipéridine-1-carboxylique ;
(A11) l'ester de tert-butyle d'acide 4-[(méthyl-{6-[(pyridin-3-ylméthyl)-amino]-pyrimidin-4-yl}-amino)-méthyll-pipéridine-1-carboxylique ;
(A12) l'ester de tert-butyle d'acide 4-[({6-[(2-fluoro-4-méthanesulfonyl-phényl)-méthyl-amino]-pyrimidin-4-yl}-méthyl-amino)-méthyll-pipéridine-1-carboxylique ;
(A13) l'ester d' isobutyle d'acide 4-({[6-(2-fluoro-4-méthanesulfonyl-phénylamino)-pyrimidin-4-yl]-méthyl-amino}-méthyl)-pipéridine-1-carboxylique ;
(A14) l'ester de tert-butyle d'acide 4-({[6-(4-cyano-2-fluoro-phénylamino)-pyrimidin-4-yl]-méthyl-amino}-méthyl)-pipéridine-1-carboxylique ;
(A15) l'ester de tert-butyle d'acide 4-[({6-[4-(2-méthanesulfonyl-éthyl)-phénylamino]-pyrimidin-4-yl}-méthylamino)-méthyll-pipéridine-1-carboxylique ;
(A16) l'ester de tert-butyle d'acide 4-({[6-(4-éthylsulfanyl-phénylamino)-pyrimidin-4-yl]-méthyl-amino}-méthyl)-pipéridine-1-carboxylique ;
(A17) l'ester de tert-butyle d'acide 4-({[6-(4-isopropylsulfanyl-phénylamino)-pyrimidin-4-yl]-méthyl-amino}-méthyl)-pipéridine-1-carboxylique ;
(A18) l'ester de tert-butyle d'acide 4-({[6-(4-éthylsulfamoyl-phénylamino)-pyrimidin-4-yl]-méthyl-amino}-méthyl)-pipéridine-1-carboxylique ;
(A19) l'ester de tert-butyle d'acide 4-({méthyl-[6-(4-méthylsulfamoyl-phénylamino)-pyrimidin-4-yl]-amino}-méthyl)-pipéridine-1-carboxylique ;
(A20) l'ester de tert-butyle d'acide 4-({[6-(4-diméthylsulfamoyl-phénylamino)-pyrimidin-4-yl]-méthylamino}-méthyl)-pipéridine-l-carboxylique ;
(A21) l'ester de tert-butyle d'acide 4-({méthyl-[6-(4-méthylsulfamoylméthyl-phénylamino)-pyrimidin-4-yl]-amino}-méthyl)-pipéridine-l-carboxylique ;

(A22) l'ester de tert-butyle d'acide 4-({méthyl-[6-(4-sulfamoyl-phénylamino)-pyrimidin-4-yl]-amino}-méthyl)-pipéridine-1-carboxylique ;

(A23) l'ester de tert-butyle d'acide 4-({méthyl-[6-(4-[1,2,4]triazol-1-yl-phénylamino)-pyrimidin-4-yl]-amino}-méthyl)-pipéridine-1-carboxylique ;

(A24) l'ester de tert-butyle d'acide 4-({méthyl-[6-(4-[1,2,4]triazol-1-ylméthyl-phénylamino)-pyrimidin-4-yl]-amino}-méthyl)-pipéridine-1-carboxylique ;

(A25) l'ester de tert-butyle d'acide 4-[(méthyl-{6-[4-(2-[1,2,4]triazol-1-yl-éthyl)-phénylamino]-pyrimidin-4-yl}-amino)-méthyl]-pipéridine-l-carboxylique ;

(A26) l'ester de tert-butyle d'acide 4-({[6-(benzo[1,3]dioxol-5-ylamino)-pyrimidin-4-yl]-méthyl-amino}-méthyl)-pipéridine-1-carboxylique ;

(A27) l'ester de tert-butyle d'acide 4-({[6-(6-méthanesulfonyl-pyridin-3-ylamino)-pyrimidin-4-yl]-méthylamino}-méthyl)-pipéridine-1-carboxylique ;

(A28) l'ester de tert-butyle d'acide 4-({[6-(3,5-diméthoxy-phénylamino)-pyrimidin-4-yl]-méthyl-amino}-méthyl)-pipéridine-1-carboxylique ;

(A29) l'ester de tert-butyle d'acide 4-[(méthyl-{6-[4-(2-oxo-oxazolidin-4-ylméthyl)-phénylamino]-pyrimidin-4-yl}-amino)-méthyl]-pipéridine-1-carboxylique ;

(A30) l'ester de tert-butyle d'acide 4-[({6-[4-(1,1-dioxo-1λ6-thiomorpholin-4-ylméthyl)-phénylamino]-pyrimidin-4-yl}-méthyl-amino)-méthyl]-pipéridine-1-carboxylique ;

(A31) l'ester de tert-butyle d'acide 4-({méthyl-[6-(4-pyrazol-l-yl-phénylamino)-pyrimidin-4-yl]-amino}-méthyl)-pipéridine-1-carboxylique ;

(A32) l'ester de tert-butyle d'acide 4-({[6-(2,2-difluoro-benzo[1,3]dioxol-5-ylamino)-pyrimidin-4-yl]-méthylamino}-méthyl)-pipéridine-1-carboxylique ;

(A33) l'ester de tert-butyle d'acide 4-({méthyl-[6-(4-trifluorométhanesulfonyl-phénylamino)-pyrimidin-4-yl]-amino}-méthyl)-pipéridine-1-carboxylique ;

(A34) l'ester de tert-butyle d'acide 4-[(méthyl-{6-[4-(morpholine-4-sulfonyl)-phénylamino]-pyrimidin-4-yl}-amino)-méthyl]-pipéridine-1-carboxylique ;

(A35) l'ester de tert-butyle d'acide 4-[(méthyl-{6-[2-(pyridine-2-carbonyl)-phénylamino]-pyrimidin-4-yl}-amino)-méthyl]-pipéridine-1-carboxylique ;

(A36) l'ester de tert-butyle d'acide 4-({[6-(2-fluoro-5-méthanesulfonyl-phénylamino)-pyrimidin-4-yl]-méthyl-amino}-méthyl)-pipéridine-1-carboxylique ;

(A39) l'ester de tert-butyle d'acide 4-({[6-(3,4-difluoro-phénylamino)-pyrimidin-4-yl]-méthyl-amino}-méthyl)-pipéridine-1-carboxylique ;

(A40) l'ester de tert-butyle d'acide 4-({[6-(2,6-difluoro-phénylamino)-pyrimidin-4-yl]-méthyl-amino}-méthyl)-pipéridine-1-carboxylique ;

(A41) l'ester de tert-butyle d'acide 4-({[6-(2,5-difluoro-phénylamino)-pyrimidin-4-yl]-méthyl-amino}-méthyl)-pipéridine-1-carboxylique ;

(A42) l'ester de tert-butyle d'acide 4-({[6-(2,3-difluoro-phénylamino)-pyrimidin-4-yl]-méthyl-amino}-méthyl)-pipéridine-1-carboxylique ;

(A43) l'ester de tert-butyle d'acide 4-({méthyl-[6-(2,3,5-trifluoro-phénylamino)-pyrimidin-4-yl]-amino}-méthyl)-pipéridine-1-carboxylique ;

(A44) l'ester de tert-butyle d'acide 4-({[6-(2-fluoro-phénylamino)-pyrimidin-4-yl]-méthyl-amino}-méthyl)-pipéridine-1-carboxylique ;

(A45) l'ester de tert-butyle d'acide 4-({[6-(2-fluoro-4-méthyl-phénylamino)-pyrimidin-4-yl]-méthyl-amino}-méthyl)-pipéridine-1-carboxylique ;

(A46) l'ester de tert-butyle d'acide 4-({[6-(3-chloro-2-fluoro-phénylamino)-pyrimidin-4-yl]-méthyl-amino)-méthyl)-pipéridine-1-carboxylique ;

(A47) l'ester de tert-butyle d'acide 4-({[6-(2,4-difluoro-phénylamino)-pyrimidin-4-yl]-méthyl-amino}-méthyl)-pipéridine-1-carboxylique ;

(A48) l'ester de tert-butyle d'acide 4-[(méthyl-{6-[2-(1-oxy-pyridin-3-yl)-éthylamino]-pyrimidin-4-yl}-amino)-méthyl]-pipéridine-l-carboxylique ;

(A49) l'ester d'isobutyle d'acide 4-[(méthyl-{6-[2-(1-oxy-pyridin-3-yl)-éthylamino]-pyrimidin-4-yl}-amino)-méthyl]-pipéridine-1-carboxylique ;

(A50) l'ester d'isobutyle d'acide 4-({[6-(2,5-difluoro-phénylamino)-pyrimidin-4-yl]-méthyl-amino}-méthyl)-pipéridine-1-carboxylique ;

(A51) l'ester d'isobutyle d'acide 4-({[6-(4-cyano-2-fluoro-phénylamino)-pyrimidin-4-yl]-méthyl-amino}-méthyl)-pipéridine-1-carboxylique ;

(A52) l'ester de tert-butyle d'acide 4-[({6-[2-(2-fluoro-phénoxy)-éthylamino]-pyrimidin-4-yl}-méthyl-amino)-méthyl]-pipéridine-1-carboxylique ;

(A53) l'ester de tert-butyle d'acide 4-({[6-(2-fluoro-phénoxy)-pyrimidin-4-yl]-méthyl-amino}-méthyl)-pipéridine-1-carboxylique ;

(A54) l'ester de tert-butyle d'acide 4-({[6-(2,5-difluoro-phénoxy)-pyrimidin-4-yl]-méthyl-amino}-méthyl)-pipéridine-1-carboxylique ;

(A55) l'ester de tert-butyle d'acide 4-[({6-[2-(2-chloro-phénoxy)-éthylamino]-pyrimidin-4-yl}-méthyl-amino)-méthyl]-pipéridine-1-carboxylique ;

(A56) l'ester de tert-butyle d'acide 4-({[6-(2-chloro-phénoxy)-pyrimidin-4-yl]-méthyl-amino}-méthyl)-pipéridine-1-carboxylique ;

(A57) l'ester de tert-butyle d'acide 4-[({6-[2-(4-fluoro-phénoxy)-propylamino]-pyrimidin-4-yl}-méthylamino)-méthyl]-pipéridine-1-carboxylique ;

(A58) l'ester de tert-butyle d'acide 4-({[6-(4-éthylsulfamoyl-2-fluoro-phénylamino)-pyrimidin-4-yl]-méthylamino}-méthyl)-pipéridine-1-carboxylique ;

(A59) l'ester de tert-butyle d'acide 4-({[6-(2-fluoro-4-isopropylsulfamoyl-phénylamino)-pyrimidin-4-yl]-méthylamino}-méthyl)-pipéridine-1-carboxylique ;

(A60) l'ester de tert-butyle d'acide 4-({[6-(4-cyano-2,5-difluoro-phénylamino)-pyrimidin-4-yl]-méthyl-amino}-méthyl)-pipéridine-1-carboxylique ;

(A61) l'ester de tert-butyle d'acide 4-({[6-(4-bromo-2,5-difluoro-phénylamino)-pyrimidin-4-yl]-méthyl-amino}-méthyl)-pipéridine-1-carboxylique ;

(A62) l'ester de tert-butyle d'acide 4-({[6-(5-carboxy-2-fluoro-phénylamino)-pyrimidin-4-yl]-méthyl-amino}-méthyl)-pipéridine-1-carboxylique ;

(A63) l'ester de tert-butyle d'acide 4-({[6-(6-méthoxy-pyridin-3-ylamino)-pyrimidin-4-yl]-méthyl-amino}-méthyl)-pipéridine-1-carboxylique ;

(A64) l'ester de tert-butyle d'acide 4-({[6-(2,6-diméthoxy-pyridin-3-ylamino)-pyrimidin-4-yl]-méthyl-amino}-méthyl)-pipéridine-1-carboxylique ;

(A65) l'acide 6-{6-[(1-tert-butoxycarbonyl-pipéridin-4-ylméthyl)-méthyl-amino]-pyrimidin-4-ylamino}-nicotinique ;

(A66) l'ester de tert-butyle d'acide 4-({[6-(6-acétylamino-pyridin-3-ylamino)-pyrimidin-4-yl]-méthyl-amino}-méthyl)-pipéridine-1-carboxylique ;

(A67) l'ester de tert-butyle d'acide 4-({[6-(5-fluoro-pyridin-2-ylamino)-pyrimidin-4-yl]-méthyl-amino}-méthyl)-pipéridine-1-carboxylique ;

(A68) l'ester de tert-butyle d'acide 4-({[6-(4-cyano-2-éthyl-phénylamino)-pyrimidin-4-yl]-méthyl-amino}-méthyl)-pipéridine-1-carboxylique ;

(A69) l'ester de tert-butyle d'acide 4-({[6-(4-butyryl-phénylamino)-pyrimidin-4-yl]-méthyl-amino}-méthyl)-pipéridine-1-carboxylique ;

(A70) l'ester de tert-butyle d'acide 4-({[6-(5-bromo-3-méthyl-pyridin-2-ylamino)-pyrimidin-4-yl]-méthyl-amino}-méthyl)-pipéridine-1-carboxylique ;

(A71) l'ester de tert-butyle d'acide 4-({[6-(3-bromo-5-méthyl-pyridin-2-ylamino)-pyrimidin-4-yl]-méthyl-amino}-méthyl)-pipéridine-1-carboxylique ;

(A72) l'ester de tert-butyle d'acide 4-({méthyl-[6-(5-trifluorométhyl-pyridin-2-ylamino)-pyrimidin-4-yl]-amino}-méthyl)-pipéridine-1-carboxylique ;

(A73) l'ester de tert-butyle d'acide 4-({[6-(4-Bromo-2-fluoro-phénylamino)-pyrimidin-4-yl]-méthyl-amino}-méthyl)-pipéridine-1-carboxylique ;

(A74) l'ester de tert-butyle d'acide 4-({[6-(3-carboxy-4-fluoro-phénylamino)-pyrimidin-4-yl]-méthyl-amino}-méthyl)-pipéridine-l-carboxylique ;

(A75) l'ester d'isobutyle d'acide 4-({[6-(4-éthoxycarbonyl-2-fluoro-phénylamino)-pyrimidin-4-yl]-méthyl-amino}-méthyl)-pipéridine-1-carboxylique ;

(A76) l'ester d'isobutyle d'acide 4-({[6-(4-carboxy-2-fluoro-phénylamino)-pyrimidin-4-yl]-méthyl-amino}-méthyl)-pipéridine-1-carboxylique ;

(A77) l'ester d'isopropyle d'acide 4-({[6-(4-Cyano-2-fluoro-phénylamino)-pyrimidin-4-yl]-méthyl-amino}-méthyl)-pipéridine-1-carboxylique ;

(A78) l'ester de butyle d'acide 4-({[6-(4-Cyano-2-fluoro-phénylamino)-pyrimidin-4-yl]-méthyl-amino}-méthyl)-pipéridine-1-carboxylique ;

(A79) l'ester de cyclopropylmétyle d'acide 4-({[6-(4-cyano-2-fluoro-phénylamino)-pyrimidin-4-yl]-méthyl-amino}-méthyl)-pipéridine-l-carboxylique ;

(A82) l'ester d'isobutyle d'acide 4-({[6-(2,5-difluoro-4-hydroxy-phénylamino)-pyrimidin-4-yl]-méthyl-amino}-méthyl)-pipéridine-1-carboxylique ;

(A83) l'ester d'isobutyle d'acide 4-({[6-(4-éthylcarbamoyl-2-fluoro-phénylamino)-pyrimidin-4-yl]-méthyl-amino}-méthyl)-pipéridine-1-carboxylique ;

(A84) l'ester d'isobutyle d'acide 4-[({6-[2-fluoro-4-(N-hydroxycarbamimidoyl)-phénylamino]-pyrimidin-4-yl}-méthyl-amino)-méthyl]-pipéridine-1-carboxylique ;

(A85) l'ester de 3-méthyl-butyle d'acide 4-({[6-(2-fluoro-4-méthanesulfonyl-phénylamino)-pyrimidin-4-yl]-méthyl-amino}-méthyl)-pipéridine-1-carboxylique ;

(A86) l'ester de tert-butyle d'acide 4-({[6-(2,5-difluoro-4-méthanesulfonyl-phénylamino)-pyrimidin-4-yl]-méthyl-amino}-méthyl)-pipéridine-1-carboxylique ;

(A87) l'ester d'isopropyle d'acide 4-({[6-(2-fluoro-4-méthanesulfonyl-phénylamino)-pyrimidin-4-yl]-méthyl-amino}-méthyl)-pipéridine-l-carboxylique ;

(A88) la (5-butyl-pyridin-2-yl)-[4-({[6-(2-fluoro-4-méthanesulfonyl-phénylamino)-pyrimidin-4-yl]-méthyl-amino}-méthyl)-pipéridin-1-yl]-méthanone ;

(A89) la N-(2-fluoro-4-méthanesulfonyl-phényl)-N'-(5'-fluoro-3,4,5,6-tétrahydro-2H-[1,2']bipyridinyl-4-ylméthyl)-N'-méthyl-pyrimidine-4,6-diamine ;

(A90) l'ester d'isobutyle d'acide 4-({[6-(4-carbamimidoyl-2-fluoro-phénylamino)-pyrimidin-4-yl]-méthylamino}-méthyl)-pipéridine-1-carboxylique ;

(A91) l'ester de cyclobutyle d'acide 4-({[6-(2-fluoro-4-méthanesulfonyl-phénylamino)-pyrimidin-4-yl]-méthyl-amino}-méthyl)-pipéridine-1-carboxylique ;

(A92) l'ester de tert-butyle d'acide 4-[6-(2-fluoro-4-méthanesulfonyl-phénylamino)-pyrimidin-4-ylamino]-pipéridine-1-carboxylique ;

(A94) l'ester de 1-éthyl-propyle d'acide 4-({[6-(2-fluoro-4-méthanesulfonyl-phénylamino)-pyrimidin-4-yl]-méthyl-amino}-méthyl)-pipéridine-1-carboxylique ;

(A95) l'ester de tert-butyle d'acide 4-({éthyl-[6-(2-fluoro-4-méthanesulfonyl-phénylamino)-pyrimidin-4-yl]-amino}-méthyl)-pipéridine-1-carboxylique ;

(A96) l'ester d'isopropyle d'acide 4-({éthyl-[6-(2-fluoro-4-méthanesulfonyl-phénylamino)-pyrimidin-4-yl]-amino}-méthyl)-pipéridine-1-carboxylique ;

(A97) l'ester d'isopropyle d'acide 4-({[6-(4-cyano-2,5-difluoro-phénylamino)-pyrimidin-4-yl]-éthyl-amino}-méthyl)-pipéridine-1-carboxylique ;

(A98) l'ester de tert-butyle d'acide 4-({[6-(4-amino-2,5-difluoro-phénoxy)-pyrimidin-4-yl]-éthyl-amino}-méthyl)-pipéridine-1-carboxylique ;

(A99) l'ester de tert-butyle d'acide 4-({[6-(2,5-difluoro-4-méthoxy-phénylamino)-pyrimidin-4-yl]-éthylamino}-méthyl)-pipéridine-1-carboxylique ;

(A100) l'ester de tert-butyle d'acide 4-({[6-(2,5-difluoro-4-méthanesulfonyl-phénylamino)-pyrimidin-4-yl]-éthyl-amino}-méthyl)-pipéridine-1-carboxylique ;

(A101) l'ester de tert-butyle d'acide 4-({éthyl-[6-(2,4,5-trifluoro-phénylamino)-pyrimidin-4-yl]-amino}-méthyl)-pipéridine-1-carboxylique ;

(A103) l'ester d'isopropyle d'acide 4-[(éthyl-{6-[4-(N-éthylcarbamimidoyl)-2,5-difluoro-phénylamino]-pyrimidin-4-yl}-amino)-méthy1]-pipéridine-1-carboxylique ;

(A104) l'ester de tert-butyle d'acide 4-({[6-(4-bromo-2,5-difluoro-phénylamino)-pyrimidin-4-yl]-éthylamino}-méthyl)-pipéridine-1-carboxylique ;

(A105) l'ester d'isopropyle d'acide 4-[({6-[5-(2-amino-éthylamino)-4-cyano-2-fluoro-phénylamino]-pyrimidin-4-yl}-éthyl-amino)-méthyl]-pipéridine-1-carboxylique ;

(A120) l'ester de tert-butyle d'acide 4-{[6-(2-fluoro-4-méthanesulfonyl-phénylamino)-pyrimidin-4-ylamino]-méthyl}-pipéridine-1-carboxylique ;

(A121) l'ester d'isopropyle d'acide 4-{[6-(2-fluoro-4-méthanesulfonyl-phénylamino)-pyrimidin-4-ylamino]-méthyl}-pipéridine-1-carboxylique ;

(A122) l'ester de tert-butyle d'acide 4-({[6-(2-fluoro-4-méthanesulfonyl-phénylamino)-pyrimidin-4-yl]-isopropyl-amino}-méthyl)-pipéridine-1-carboxylique ; et

(A123) l'ester d'isobutyle d'acide 4-({[4-(2-fluoro-4-méthanesulfonyl-phénylamino)-pyridin-2-yl]-méthylamino}-méthyl)-pipéridine-1-carboxylique.

**20.** Composé selon la revendication 1, dans lequel ledit composé est choisi parmi les composés et sels , solvates, hydrates et N-oxydes pharmaceutiquement acceptables de ceux-ci suivants :

(B1) l'ester de tert-butyle d'acide 4-[6-(2-fluoro-4-méthanesulfonyl-phénylamino)-pyrimidin-4-yloxy]-pipéridine-1-carboxylique ;

(B3) l'ester d'isopropyle d'acide 4-[6-(2-fluoro-4-méthanesulfonyl-phénylamino)-pyrimidin-4-yloxy]-pipéridine-1-carboxylique ;

(B4) la (6-chloro-pyridin-2-yl)-{4-[6-(2-fluoro-4-méthanesulfonyl-phénylamino)-pyrimidin-4-yloxy]-pipéridin-1-yl}-méthanone ;
(B5) la (6-bromo-pyridin-2-yl)-{4-[6-(2-fluoro-4-méthanesulfonyl-phénylamino)-pyrimidin-4-yloxy]-pipéridin-1-yl}-méthanone ;
(B6) la {4-[6-(2-fluoro-4-méthanesulfonyl-phénylamino)-pyrimidin-4-yloxy]-pipéridin-1-yl}-(6-méthyl-pyridin-2-yl)-méthanone ;
(B7) la {4-[6-(2-fluoro-4-méthanesulfonyl-phénylamino)-pyrimidin-4-yloxy]-pipéridin-1-yl}-(6-fluoro-pyridin-2-yl)-méthanone ;
(B8) la {4-[6-(2-fluoro-4-méthanesulfonyl-phénylamino)-pyrimidin-4-yloxy]-pipéridin-1-yl}-pyridin-2-yl-métha-no- ne ;
(B9) la (5-bromo-pyridin-3-yl)-{4-[6-(2-fluoro-4-méthanesulfonyl-phénylamino)-pyrimidin-4-yloxy]-pipéridin-1-yl}-méthanone ;
(B10) la {4-[6-(2-fluoro-4-méthanesulfonyl-phénylamino)-pyrimidin-4-yloxy]-pipéridin-l-yl}-(5-méthyl-pyridin-3-yl)-méthanone ;
(B11) la (5,6-dichloro-pyridin-3-yl)-{4-[6-(2-fluoro-4-méthanesulfonylphénylamino)-pyrimidin-4-yloxy]-pipéridin-1-yl}-méthanone ;
(B12) l'ester de tert-butyle d'acide 4-[6-(4-cyano-2,5-difluoro-phénylamino)-pyrimidin-4-yloxy]-pipéridine-1-carboxylique ;
(B13) l'ester de tert-butyle d'acide 4-[6-(2,5-difluoro-4-méthanesulfonyl-phénylamino)-pyrimidin-4-yloxyl-pipéridine-1-carboxylique ;
(B14) l'ester de tert-butyle d'acide 4-[6-(2,4,5-trifluoro-phénylamino)-pyrimidin-4-yloxy]-pipéridine-1-carboxylique ;
(B15) l'ester de tert-butyle d'acide 4-[6-(4-bromo-2,5-difluoro-phénylamino)-pyrimidin-4-yloxy]-pipéridine-1-carboxylique ;
(B16) l'ester de tert-butyle d'acide 4-[6-(3-fluoro-4-méthyl-phénylamino)-pyrimidin-4-yloxy]-pipéridine-1-carboxylique ;
(B17) l'ester de tert-butyle d'acide 4-[6-(3-hydroxy-4-méthoxy-phénylamino)-pyrimidin-4-yloxy]-pipéridine-1-carboxylique ;
(B18) l'ester de tert-butyle d'acide 4-[6-(6-cyano-pyridin-3-ylamino)-pyrimidin-4-yloxy]-pipéridine-1-carboxylique ;
(B19) l'ester de tert-butyle d'acide 4-[6-(3-chloro-4-cyano-phénylamino)-pyrimidin-4-yloxy]-pipéridine-1-carboxylique ;
(B20) l'ester de tert-butyle d'acide 4-[6-(6-chloro-pyridin-3-ylamino)-pyrimidin-4-yloxy]-pipéridine-1-carboxylique ;
(B21) l'ester de tert-butyle d'acide 4-[6-(3-fluoro-4-méthoxy-phénylamino)-pyrimidin-4-yloxy]-pipéridine-1-carboxylique ;
(B22) l'ester de tert-butyle d'acide 4-[6-(3,4-diméthoxy-phénylamino)-pyrimidin-4-yloxy]-pipéridine-1-carboxylique ;
(B23) l'ester de tert-butyle d'acide 4-[6-(2,3-dihydro-benzo[1,4]dioxin-6-ylamino)-pyrimidin-4-yloxy]-pipéridine-1-carboxylique ;
(B24) l'ester d'isopropyle d'acide 4-[6-(4-cyano-2,5-difluoro-phénylamino)-pyrimidin-4-yloxy]-pipéridine-1-carboxylique ;
(B25) l'ester de tert-butyle d'acide 4-[6-(4-cyano-5-éthylamino-2-fluoro-phénylamino)-pyrimidin-4-yloxy]-pipéridine-1-carboxylique ;
(B26) l'ester d'isopropyle d'acide 4-[6-(4-éthoxy-2,5-difluoro-phénylamino)-pyrimidin-4-yloxy]-pipéridine-1-carboxylique ;
(B27) l'ester de tert-butyle d'acide 4-[6-(4-éthylsulfanyl-phénylamino)-pyrimidin-4-yloxy]-pipéridine-1-carboxylique ;
(B28) l'ester de tert-butyle d'acide 4-[6-(4-isopropylsulfanyl-phénylamino)-pyrimidin-4-yloxy]-pipéridine-1-carboxylique ;
(B29) la (5-Butyl-pyridin-2-yl)-{4-[6-(2-fluoro-4-méthanesulfonyl-phénylamino)-pyrimidin-4-yloxy]-pipéridin-l-yl}-méthanone ;
(B30) l'ester de tert-butyle d'acide 4-[6-(5-chloro-3-méthyl-pyridin-2-ylamino)-pyrimidin-4-yloxy]-pipéridine-1-carboxylique ;
(B31) l'ester de tert-butyle d'acide 4-[6-(6-acétylamino-4-méthyl-pyridin-3-ylamino)-pyrimidin-4-yloxy]-pipéridine-l-carboxylique ;
(B32) l'ester de tert-butyle d'acide 4-[6-(5-fluoro-4-méthyl-pyridin-2-ylamino)-pyrimidin-4-yloxy]-pipéridine-1-carboxylique ;
(B33) l'ester de tert-butyle d'acide 4-[6-(6-méthoxy-5-méthyl-pyridin-3-ylamino)-pyrimidin-4-yloxy]-pipéridine-1-carboxylique ;
(B34) l'ester de tert-butyle d'acide 4-[6-(6-méthoxy-2-méthyl-pyridin-3-ylamino)-pyrimidin-4-yloxy]-pipéridine-1-carboxylique ;
(B35) l'ester de tert-butyle d'acide 4-[6-(6-fluoro-5-méthyl-pyridin-3-ylamino)-pyrimidin-4-yloxy]-pipéridine-1-carboxylique ;
(B36) l'ester de tert-butyle d'acide 4-[6-(2-chloro-6-méthyl-pyridin-3-ylamino)-pyrimidin-4-yloxy]-pipéridine-1-carboxylique ;
(B37) l'ester de tert-butyle d'acide 4-[6-(4-méthyl-pyridin-3-ylamino)-pyrimidin-4-yloxy]-pipéridine-1-carboxylique ;
(B38) l'ester de tert-butyle d'acide 4-[6-(2-méthyl-pyridin-3-ylamino)-pyrimidin-4-yloxy]-pipéridine-1-carboxylique ;
(B39) l'ester de tert-butyle d'acide 4-[6-(6-chloro-2-méthyl-pyridin-3-ylamino)-pyrimidin-4-yloxy]-pipéridine-1-carboxylique ;
(B40) l'ester de tert-butyle d'acide 4-[6-(6-fluoro-pyridin-3-ylamino)-pyrimidin-4-yloxy]-pipéridine-1-carboxylique ;
(B41) l'ester de tert-butyle d'acide 4-[6-(2-chloro-4-méthyl-pyridin-3-ylamino)-pyrimidin-4-yloxy]-pipéridine-1-carboxylique ;
(B42) l'ester de tert-butyle d'acide 4-[6-(6-méthoxy-pyridin-3-ylamino)-pyrimidin-4-yloxy]-pipéridine-1-carboxylique ;
(B43) l'ester de tert-butyle d'acide 4-[6-(5-fluoro-pyridin-2-ylamino)-pyrimidin-4-yloxy]-pipéridine-1-carboxylique ;
(B44) l'ester de tert-butyle d'acide 4-[6-(2-fluoro-pyridin-3-ylamino)-pyrimidin-4-yloxy]-pipéridine-1-carboxylique ;
(B45) l'ester de tert-butyle d'acide 4-[6-(6-chloro-5-méthyl-pyridin-3-ylamino)-pyrimidin-4-yloxy]-pipéridine-1-carboxylique ;
(B46) l'ester de tert-butyle d'acide 4-[6-(2-méthyl-pyridin-4-ylamino)-pyrimidin-4-yloxy]-pipéridine-1-carboxylique
(B47) l'ester de tert-butyle d'acide 4-[6-(2-méthoxy-pyridin-3-ylamino)-pyrimidin-4-yloxy]-pipéridine-1-carboxylique ;
(B48) l'ester de tert-butyle d'acide 4-[6-(2,5-difluoro-phénylamino)-pyrimidin-4-yloxy]-pipéridine-1-carboxylique ;
(B49) l'ester de tert-butyle d'acide 4-[6-(4-chloro-2-fluoro-phénylamino)-pyrimidin-4-yloxy]-pipéridine-1-carboxylique ;
(B50) l'ester d'isopropyle d'acide 4-[6-(2,5-difluoro-phénylamino)-pyrimidin-4-yloxy]-pipéridine-1-carboxylique ;
(B51) l'ester d'isopropyle d'acide 4-[6-(6-méthoxy-pyridin-3-ylamino)-pyrimidin-4-yloxy]-pipéridine-1-carboxylique ;
(B52) l'ester d'isopropyle d'acide 4-[6-(4-cyano-3-méthoxy-phénylamino)-pyrimidin-4-yloxy]-pipéridine-1-carboxylique ;
(B53) l'ester d'isopropyle d'acide 4-[6-(3-fluoro-4-hydroxy-phénylamino)-pyrimidin-4-yloxy]-pipéridine-1-carboxylique ;
(B54) l'ester d'isopropyle d'acide 4-[6-(6-éthoxy-pyridin-3-ylamino)-pyrimidin-4-yloxy]-pipéridine-1-carboxylique ;
(B55) l'ester d'isopropyle d'acide 4-[6-(2,5-difluoro-4-isopropoxy-phénylamino)-pyrimidin-4-yloxy]-pipéridine-1-carboxylique ;
(B56) la (2-fluoro-4-méthanesulfonyl-phényl)-[6-(5'-isopropoxy-3,4,5,6-tétrahydro-2H-[1,2]bipyridinyl-4-yloxy)-pyrimidin-4-yl]-amine ;
(B57) la (2-fluoro-4-méthanesulfonyl-phényl)-{6-[1-(3-isopropyl-[1,2,4]oxadiazol-5-yl)-pipéridin-4-yloxy]-pyrimidin-4-yl}-amine ;
(B58) l'ester d'isopropyle d'acide 4-[6-(4-cyano-2-fluoro-phénylamino)-pyrimidin-4-yloxy]-pipéridine-1-carboxylique ;
(B59) l'ester d'isopropyle d'acide 4-[6-(pyridin-3-ylamino)-pyrimidin-4-yloxyl]-pipéridine-1-carboxylique ;
(B60) l'ester d'isopropyle d'acide 4-[6-(pyridin-4-ylamino)-pyrimidin-4-yloxy]-pipéridine-1-carboxylique ;
(B61) l'ester d'isopropyle d'acide 4-[6-(2,5-difluoro-4-propoxy-phénylamino)-pyrimidin-4-yloxy]-pipéridine-1-carboxylique ;
(B62) l'ester d'isopropyle d'acide 4-[6-(4-éthylamino-2-fluoro-phénylamino)-pyrimidin-4-yloxy]-pipéridine-1-carboxylique ;
(B63) l'ester d'isopropyle d'acide 4-[6-(4-diméthylamino-2-fluoro-phénylamino)-pyrimidin-4-yloxy]-pipéridine-1-carboxylique ;

(B64) l'ester d'isopropyle d'acide 4-[6-(2-fluoro-4-propylamino-phénylamino)-pyrimidin-4-yloxy]-pipéridine-1-carboxylique ;
(B65) l'ester d'isopropyle d'acide 4-[6-(2-fluoro-4-isopropylamino-phénylamino)-pyrimidin-4-yloxy]-pipéridine-1-carboxylique ;
(B66) l'ester d'isopropyle d'acide 4-[6-(2-méthyl-6-propylamino-pyridin-3-ylamino)-pyrimidin-4-yloxy]-pipéridine-1-carboxylique ;
(B67) l'ester d'isopropyle d'acide 4-[6-(2-méthyl-pyridin-3-ylamino)-pyrimidin-4-yloxy]-pipéridine-1-carboxylique ;
(B68) l'ester d'isopropyle d'acide 4-[6-(6-isopropylamino-2-méthyl-pyridin-3-ylamino)-pyrimidin-4-yloxyl-pipéridine-1-carboxylique ;
(B69) l'ester d'isopropyle d'acide 4-[6-(2-méthyl-6-propoxy-pyridin-3-ylamino)-pyrimidin-4-yloxy]-pipéridine-1-carboxylique ;
(B70) l'ester d'isopropyle d'acide 4-[6-(4-iodo-2-méthyl-phénylamino)-pyrimidin-4-yloxy]-pipéridine-l-carboxylique ;
(B71) l'ester d'isopropyle d'acide 4-[6-(2-fluoro-4-iodo-phénylamino)-pyrimidin-4-yloxy]-pipéridine-1-carboxylique ;
(B77) l'ester d'isopropyle d'acide 4-[6-(6-méthoxy-5-méthyl-pyridin-3-ylamino)-pyrimidin-4-yloxy]-pipéridine-1-carboxylique ;
(B78) l'ester d'isopropyle d'acide 4-[6-(4-méthyl-pyridin-3-ylamino)-pyrimidin-4-yloxy]-pipéridine-1-carboxylique ;
(B79) l'ester d'isopropyle d'acide 4-[6-(4-acétylamino-3-méthyl-phénylamino)-pyrimidin-4-yloxy]-pipéridine-1-carboxylique ;
(B80) l'ester d'isopropyle d'acide 4-[6-(3-chloro-4-fluoro-phénylamino)-pyrimidin-4-yloxy]-pipéridine-1-carboxylique ;
(B81) l'ester d'isopropyle d'acide 4-[6-(3,5-diméthoxy-phénylamino)-pyrimidin-4-yloxy]-pipéridine-1-carboxylique ;
(B82) l'ester d'isopropyle d'acide 4-[6-(6-éthyl-pyridin-2-ylamino)-pyrimidin-4-yloxy]-pipéridine-1-carboxylique ;
(B83) l'ester d'isopropyle d'acide 4-[6-(5-méthyl-pyridin-2-ylamino)-pyrimidin-4-yloxy]-pipéridine-1-carboxylique ;
(B84) l'ester d'isopropyle d'acide 4-[6-(2-méthyl-quinolin-6-ylamino)-pyrimidin-4-yloxy]-pipéridine-1-carboxylique ;
(B85) l'ester d'isopropyle d'acide 4-[6-(2-méthylsulfanyl-benzothiazol-6-ylamino)-pyrimidin-4-yloxy]-pipéridine-1-carboxylique ;
(B86) l'ester d'isopropyle d'acide 4-[6-(6-morpholin-4-yl-pyridin-3-ylamino)-pyrimidin-4-yloxy]-pipéridine-1-carboxylique ;
(B88) l'ester d'isopropyle d'acide 4-[6-(4-pipéridin-1-yl-phénylamino)-pyrimidin-4-yloxy]-pipéridine-1-carboxylique ;
(B89) l'ester d'isopropyle d'acide 4-[6-(3-trifluorométhoxy-phénylamino)-pyrimidin-4-yloxy]-pipéridine-1-carboxylique ;
(B90) l'ester d'isopropyle d'acide 4-[6-(5-oxo-5,6,7,8-tétrahydro-naphthalén-2-ylamino)-pyrimidin-4-yloxy]-pipéridine-1-carboxylique ;
(B92) l'ester d'isopropyle d'acide 4-[6-(5-cyano-pyridin-2-ylamino)-pyrimidin-4-yloxy]-pipéridine-1-carboxylique ;
(B93) l'ester d'isopropyle d'acide 4-[6-(4-bromo-2,5-difluoro-phénylamino)-pyrimidin-4-yloxy]-pipéridine-1-carboxylique ;
(B94) l'ester d'isopropyle d'acide 4-[6-(4-trifluorométhyl-pyridin-2-ylamino)-pyrimidin-4-yloxy]-pipéridine-1-carboxylique ;
(B97) l'ester d'isopropyle d'acide 4-[6-(2,6-diméthyl-pyridin-3-ylamino)-pyrimidin-4-yloxy]-pipéridine-1-carboxylique ;
(B98) l'ester d'isopropyle d'acide 4-[6-(4-cyano-2-méthyl-phénylamino)-pyrimidin-4-yloxy]-pipéridine-1-carboxylique ;
(B99) l'ester d'isopropyle d'acide 4-[6-(4-méthoxy-2-méthyl-phénylamino)-pyrimidin-4-yloxy]-pipéridine-1-carboxylique ;
(B100) l'ester d'isopropyle d'acide 4-[6-(2,4-diméthoxy-phénylamino)-pyrimidin-4-yloxy]-pipéridine-1-carboxylique ;
(B101) l'ester d'isopropyle d'acide 4-{6-[acétyl-(2-fluoro-4-méthanesulfonyl-phényl)-amino]-pyrimidin-4-yloxy}-pipéridine-1-carboxylique ;
(B102) l'ester d'isopropyle d'acide 4-[6-(5-carbamoyl-pyridin-2-ylamino)-pyrimidin-4-yloxy]-pipéridine-1-carboxylique ;
(B105) l'ester d'isopropyle d'acide 4-[6-(3-oxazol-5-yl-phénylamino)-pyrimidin-4-yloxy]-pipéridine-1-carboxylique ;
(B106) l'ester d'isopropyle d'acide 4-[6-(5-trifluorométhyl-pyridin-2-ylamino)-pyrimidin-4-yloxy]-pipéridine-1-carboxylique ;
(B107) l'ester d'isopropyle d'acide 4-[6-(4-chloro-2-trifluorométhoxy-phénylamino)-pyrimidin-4-yloxy]-pipéridine-1-carboxylique ;
(B110) l'ester d'isopropyle d'acide 4-[6-(1-oxo-indan-5-ylamino)-pyrimidin-4-yloxy]-pipéridine-1-carboxylique ;
(B111) l'ester d'isopropyle d'acide 4-{6-[5-(1-méthyl-pyrrolidin-2-yl)-pyridin-2-ylamino]-pyrimidin-4-yloxy}-pipéridine-1-carboxylique ;
(B112) l'ester d'isopropyle d'acide 4-[6-(6-méthoxy-2-méthyl-pyridin-3-ylamino)-pyrimidin-4-yloxy]-pipéridine-1-carboxylique ;
(B113) l'ester d'isopropyle d'acide 4-[6-(5-bromo-3-méthyl-pyridin-2-ylamino)-pyrimidin-4-yloxy]-pipéridine-1-carboxylique ;
(B114) l'ester d'isopropyle d'acide 4-[6-(2-chloro-6-méthyl-pyridin-3-ylamino)-pyrimidin-4-yloxy]-pipéridine-1-carboxylique ;
(B115) l'ester d'isopropyle d'acide 4-[6-(2-éthynyl-phénylamino)-pyrimidin-4-yloxy]-pipéridine-1-carboxylique ;
(B116) l'ester d'isopropyle d'acide 4-[6-(4-bromo-2-trifluorométhoxy-phénylamino)-pyrimidin-4-yloxy]-pipéridine-1-carboxylique ;
(B117) l'ester d'isopropyle d'acide 4-[6-(3-iodo-4-méthyl-phénylamino)-pyrimidin-4-yloxy]-pipéridine-1-carboxylique ;
(B118) l'ester d'isopropyle d'acide 4-[6-(2-fluoro-5-méthyl-phénylamino)-pyrimidin-4-yloxy]-pipéridine-1-carboxylique ;
(B122) l'ester d'isopropyle d'acide 4-[6-(2,5-difluoro-4-propylamino-phénylamino)-pyrimidin-4-yloxy]-pipéridine-1-carboxylique ;
(B123) l'ester d'isopropyle d'acide 4-[6-(2,5-difluoro-4-morpholin-4-yl-phénylamino)-pyrimidin-4-yloxy]-pipéridine-1-carboxylique ;
(B124) l'ester d'isopropyle d'acide 4-[6-(2-méthyl-4-propylamino-phénylamino)-pyrimidin-4-yloxy]-pipéridine-1-carboxylique ;
(B125) l'ester d'isopropyle d'acide 4-{6-[2,5-difluoro-4-(4-méthyl-pipérazin-1-yl)-phénylamino]-pyrimidin-4-yloxy}-pipéridine-1-carboxylique ;
(B126) l'ester d'isopropyle d'acide 4-{6-[2,5-difluoro-4-(2-pyrrolidin-1-yl-éthoxy)-phénylamino]-pyrimidin-4-yloxy)-pipéridine-1-carboxylique ;
(B127) l'ester d'isopropyle d'acide 4-{6-[4-(2-diméthylamino-éthoxy)-2,5-difluoro-phénylamino]-pyrimidin-4-yloxy}-pipéridine-1-carboxylique ;
(B128) l'ester d'isopropyle d'acide 4-{6-[2,5-difluoro-4-(2-morpholin-4-yl-éthoxy)-phénylamino]-pyrimidin-4-yloxy}-pipéridine-1-carboxylique ;
(B129) l'ester d'isopropyle d'acide 4-[6-(2,4-difluoro-phénylamino)-pyrimidin-4-yloxy]-pipéridine-1-carboxylique ;
(B130) l'ester d'isopropyle d'acide 4-[6-(2,4,5-trifluoro-phénylamino)-pyrimidin-4-yloxy]-pipéridine-1-carboxylique ;
(B131) l'ester d'isopropyle d'acide 4-[6-(4-méthanesulfonyl-phénylamino)-pyrimidin-4-yloxy]-pipéridine-1-carboxylique ;
(B132) l'ester d'isopropyle d'acide 4-{6-[acétyl-(4-méthanesulfonyl-phényl)-amino]-pyrimidin-4-yloxy}-pipéridine-1-carboxylique ;
(B133) la (2,5-difluoro-4-propoxy-phényl)-{6-[1-(5-isopropyl-[1,2,4]oxadiazol-3-yl)-pipéridin-4-yloxy]-pyrimidin-4-yl}-amine ;
(B134) l'ester d'isopropyle d'acide 4-{6-[2,5-difluoro-4-(morpholin-4-ylamino)-phénylamino]-pyrimidin-4-yloxy}-pipéridine-1-carboxylique ;
(B135) l'ester d'isopropyle d'acide 4-{6-[2,5-difluoro-4-(2-méthoxy-éthylamino)-phénylamino]-pyrimidin-4-yloxy}-pipéridine-1-carboxylique ;
(B136) l'ester d'isopropyle d'acide 4-(6-{2,5-difluoro-4-[(tétrahydro-furan-2-ylméthyl)-amino]-phénylamino}-pyrimidin-4-yloxy)-pipéridine-1-carboxylique ;
(B137) l'ester d'isopropyle d'acide 4-[6-(4-butylamino-2,5-difluoro-phénylamino)-pyrimidin-4-yloxy]-pipéridine-1-carboxylique ;
(B138) l'ester d'isopropyle d'acide 4-{6-[2,5-difluoro-4-(3-méthyl-butylamino)-phénylamino]-pyrimidin-4-yloxy}-pipéridine-1-carboxylique ;
(B139) l'ester d'isopropyle d'acide 4-[6-(2-fluoro-4-méthanesulfonyl-phénylamino)-2-méthyl-pyrimidin-4-yloxy]-pipéridine-1-carboxylique ;
(B140) l'ester d'isopropyle d'acide 4-{6-[2,5-difluoro-4-(2-morpholin-4-yl-éthylamino)-phénylamino]-pyrimidin-4-yloxy}-pipéridine-1-carboxylique ;
(B141) l'ester d'isopropyle d'acide 4-{6-[2-(2,5-difluoro-phénoxy)-éthylamino]-pyrimidin-4-yloxy}-pipéridine-1-carboxylique ;
(B142) l'ester d'isopropyle d'acide 4-[6-(2,5-difluoro-phénoxy)-pyrimidin-4-yloxy]-pipéridine-1-carboxylique ;
(B143) l'ester d'isopropyle d'acide 4-[6-(4-bromo-2-fluoro-phénoxy)-pyrimidin-4-yloxy]-pipéridine-1-carboxylique ;
(B144) l'ester d'isopropyle d'acide 4-[6-(2-fluor-4-morpholin-4-yl-phénoxy)-pyrimidin-4-yloxy]-pipéridine-1-carboxylique ;
(B145) l'ester d'isopropyle d'acide 4-{6-[2,5-difluoro-4-(tétrahydro-furan-2-ylméthoxy)-phénylamino]-pyrimidin-4-yloxy}-pipéridine-1-carboxylique ;
(B149) l'ester d'isopropyle d'acide 4-[4-(2-fluoro-4-méthanesulfonyl-phénylamino)-pyridin-2-yloxy]-pipéridine-1-carboxylique ; et
(B150) l'ester d'isopropyle d'acide 4-[4-(2,5-difluoro-4-propoxy-phénylamino)-pyridin-2-yloxy]-pipéridine-1-carboxylique.

**21.** Composé selon la revendication 1, dans lequel ledit composé est choisi parmi les composés et sels, solvates, hydrates et N-oxydes pharmaceutiquement acceptables de ceux-ci suivants :

(B153) l'ester d'isopropyle d'acide 4-{6-[6-(2,3-dihydroxy-propylamino)-2-méthyl-pyridin-3-yloxy]-pyrimidin-4-yloxy}-pipéridine-1-carboxylique ;
(B155) l'ester d'isopropyle d'acide 4-[6-(2-méthyl-pyridin-3-yloxy)-pyrimidin-4-yloxy]-pipéridine-1-carboxylique ;
(B156) l'ester d'isopropyle d'acide 4-[6-(6-bromo-2-méthyl-pyridin-3-yloxy)-pyrimidin-4-yloxy]-pipéridine-1-carboxylique ; et
(B157) l'ester de tert-butyle d'acide 4-[6-(2-méthyl-pyridin-3-yloxy)-pyrimidin-4-yloxy]-pipéridine-1-carboxylique.

**22.** Composé choisi parmi les composés de formule (I) et les sels, solvates, hydrates et N-oxydes pharmaceutiquement acceptables de ceux-ci :

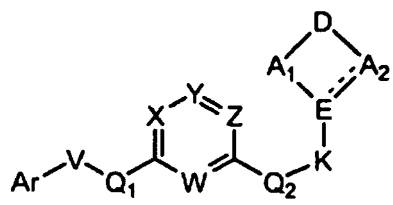

(I)

dans laquelle :

$A_1$ et $A_2$ sont indépendamment un groupe alkylène en $C_1$ à 3 facultativement substitué par un ou plusieurs substituants choisis indépendamment parmi les groupes alkyle en $C_{1\ à\ 6}$, alcoxy en $C_{1\ à\ 6}$, et carboxy ;
D est $CR_1R_2$, où $R_1$ est choisi parmi H, les groupes alkyle en $C_{1\ à\ 6}$, alcoxy en $C_{1\ à\ 6}$, un atome d'halogène et un groupe hydroxyle ;
E est N, C ou $CR_3$, où $R_3$ est H ou un groupe alkyle en $C_1$ à $_6$ ;
- - - est une simple liaison lorsque E est N ou $CR_3$, ou une double liaison lorsque E est C ;
K est absent, un groupe cycloalkylène en $C_{3\ à\ 6}$, ou alkylène en $C_{1\ à\ 3}$ facultativement substitués chacun par un ou plusieurs substituants choisis indépendamment parmi les groupes alkyle en $C_{1\ à\ 6}$, alcoxy en $C_{1\ à\ 6}$, carboxy, cyano, et un atome d'halogène ;
$Q_1$ est NH ;
$Q_2$ est absent ;
W est CH ;
X est N ou $CR_6$ ;

Y est N ou $CR_7$ ;

Z est N ;

V est absent, un groupe hétéroalkylène en $C_{1\ à\ 3}$, ou alkylène en $C_{1\ à\ 3}$ où chacun est facultativement substitué par un ou plusieurs substituants choisis indépendamment parmi les groupes alkyle en $C_{1\ à\ 3}$, alcoxy en $C_{1\ à\ 6}$, carboxy, cyano, halogénoalkyle en $C_{1\ à\ 3}$, et un atome d'halogène ;

$R_6$, $R_7$, et $R_8$ sont chacun indépendamment choisis parmi H, les groupes acyle en $C_{1\ à\ 6}$, acyloxy en $C_{1\ à\ 6}$, alcényle en $C_{2\ à\ 6}$, alcoxy en $C_{1\ à\ 6}$, alkyle en $C_{1\ à\ 6}$, alkylamino en $C_{1\ à\ 6}$, alkylcarboxamide en $C_{1\ à\ 6}$, alcynyle en $C_{2\ à\ 6}$, alkylsulfonamide en $C_{1\ à\ 6}$, alkylsulfinyle en $C_{1\ à\ 6}$, alkylsulfonyle en $C_{1\ à\ 6}$, alkylthio en $C_{1\ à\ 6}$, alkylthiocarboxamide en $C_{1\ à\ 6}$, alkylthiouréyle en $C_{1\ à\ 6}$, alkyluréyle en $C_{1\ à\ 6}$, amino, di-alkylamino en $C_{1\ à\ 6}$, alcoxycarbonyle en $C_{1\ à\ 6}$, carboxamide, carboxy, cyano, cycloalkyle en $C_{3\ à\ 6}$, di-alkylcarboxamide en $C_{1\ à\ 6}$, di-alkylsulfonamide en $C_{1\ à\ 6}$, di-alkylthiocarboxamido en $C_{1\ à\ 6}$, halogénoalcoxy en $C_{1\ à\ 6}$, halogénoalkyle en $C_{1\ à\ 6}$, un atome d'halogène, un groupe halogénoalkylsulfinyle en $C_{1\ à\ 6}$, halogénoalkylsulfonyle en $C_{1\ à\ 6}$, halogénoalkylthio en $C_{1\ à\ 6}$, hydroxyle, hydroxylamino et nitro, où lesdits groupes alcényle en $C_{2\ à\ 6}$, alkyle en $C_{1\ à\ 6}$, alcynyle en $C_{2\ à\ 6}$ et cycloalkyle en $C_{3\ à\ 6}$ sont chacun facultativement substitués par un ou plusieurs substituants indépendamment choisis parmi les groupes acyle en $C_{1\ à\ 6}$, acyloxy en $C_{1\ à\ 6}$, alcényle en $C_{2\ à\ 6}$, alcoxy en $C_{1\ à\ 6}$, alkyle en $C_{1\ à\ 6}$, alkylamino en $C_{1\ à\ 6}$, alkylcarboxamide en $C_{1\ à\ 6}$, alcynyle en $C_{2\ à\ 6}$, alkylsulfonamide en $C_{1\ à\ 6}$, alkylsulfinyle en $C_{1\ à\ 6}$, alkylsulfonyle en $C_{1\ à\ 6}$, alkylthio en $C_{1\ à\ 6}$, alkylthiocarboxamide en $C_{1\ à\ 6}$, alkylthiouréyle en $C_{1\ à\ 6}$, alkyluréyle en $C_{1\ à\ 6}$, amino, di-alkylamino en $C_{1\ à\ 6}$, alcoxycarbonyle en $C_{1\ à\ 6}$, carboxamide, carboxy, cyano, cycloalkyle en $C_{3\ à\ 6}$, di-alkylcarboxamide en $C_{1\ à\ 6}$, di-alkylsulfonamide en $C_{1\ à\ 6}$, di-alkylthiocarboxamido en $C_{1\ à\ 6}$, halogénoalcoxy en $C_{1\ à\ 6}$, halogénoalkyle en $C_{1\ à\ 6}$, un atome d'halogène, un groupe halogénoalkylsulfinyle en $C_{1\ à\ 6}$, halogénoalkylsulfonyle en $C_{1\ à\ 6}$, halogénoalkylthio en $C_{1\ à\ 6}$, hydroxyle, hydroxylamino et nitro ;

Ar est un groupe phényle, naphtyle, pyridyle, benzofuranyle, pyrazinyle, pyridazinyle, pyrimidinyle, triazinyle, quinoléine, benzoxazole, benzothiazole, 1*H*-benzimidazole, isoquinoléine, quinazoline, quinoxaline, pyrrole, ou indole, facultativement substitué par $R_9$, $R_{10}$, $R_{11}$, $R_{12}$ et $R_{13}$ ;

$R_9$ est choisi parmi les groupes acyle en $C_{1\ à\ 6}$, acylsulfonamide en $C_{1\ à\ 6}$, acyloxy en $C_{1\ à\ 6}$, alcényle en $C_{2\ à\ 6}$, alcoxy en $C_{1\ à\ 6}$, alkyle en $C_{1\ à\ 6}$, alkylamino en $C_{1\ à\ 6}$, alkylcarboxamide en $C_{1\ à\ 6}$, alcynyle en $C_{2\ à\ 6}$, alkylsulfonamide en $C_{1\ à\ 6}$, alkylsulfinyle en $C_{1\ à\ 6}$, alkylsulfonyle en $C_{1\ à\ 6}$, alkylthio en $C_{1\ à\ 6}$, alkylthiocarboxamide en $C_{1\ à\ 6}$, alkylthiouréyle en $C_{1\ à\ 6}$, alkyluréyle en $C_{1\ à\ 6}$, amino, aryle, arylcarbonyle, arylsulfonyle, di-alkylamino en $C_{1\ à\ 6}$, carbamimidoyle, alcoxycarbonyle en $C_{1\ à\ 6}$, carboxamide, carboxy, cyano, cycloalkyle en $C_{3\ à\ 6}$, di-alkylcarboxamide en $C_{1\ à\ 6}$, di-alkylsulfonamide en $C_{1\ à\ 6}$, di-alkylthiocarboxamido en $C_{1\ à\ 6}$, guanidine, halogénoalcoxy en $C_{1\ à\ 6}$, halogénoalkyle en $C_{1\ à\ 6}$, un atome d'halogène, un groupe halogénoalkylsulfinyle en $C_{1\ à\ 6}$, halogénoalkylsulfonyle en $C_{1\ à\ 6}$, halogénoalkylthio en $C_{1\ à\ 6}$, hétérocyclique, sulfonyle hétérocyclique, hétéroaryle, hydroxyle, hydroxylamino, nitro, oxo en $C_{3\ à\ 6}$ -cycloalkyle, phénoxy, sulfonamide, acide sulfonique et thiol ; et où chaque $R_9$ est facultativement substitué par un ou plusieurs substituants choisis indépendamment parmi les groupes acyle en $C_{1\ à\ 6}$, acylsulfonamide en $C_{1\ à\ 6}$, acyloxy en $C_{1\ à\ 6}$, alcényle en $C_{2\ à\ 6}$, alcoxy en $C_{1\ à\ 6}$, alkyle en $C_{1\ à\ 6}$, alkylamino en $C_{1\ à\ 6}$, alkylcarboxamide en $C_{1\ à\ 6}$, alcynyle en $C_{2\ à\ 6}$, alkylsulfonamide en $C_{1\ à\ 6}$, alkylsulfinyle en $C_{1\ à\ 6}$, alkylsulfonyle en $C_{1\ à\ 6}$, alkylthio en $C_{1\ à\ 6}$, alkylthiocarboxamide en $C_{1\ à\ 6}$, alkylthiouréyle en $C_{1\ à\ 6}$, alkyluréyle en $C_{1\ à\ 6}$, amino, aryle, arylcarbonyle, arylsulfonyle, di-alkylamino en $C_{1\ à\ 6}$, alcoxycarbonyle en $C_{1\ à\ 6}$, carboxamide, carboxy, cyano, cycloalkyle en $C_{3\ à\ 6}$, di-alkylcarboxamide en $C_{1\ à\ 6}$, di-alkylsulfonamide en $C_{1\ à\ 6}$, di- alkylthiocarboxamido en $C_{1\ à\ 6}$, halogénoalcoxy en $C_{1\ à\ 6}$, halogénoalkyle en $C_{1\ à\ 6}$, un atome d'halogène, un groupe halogénoalkylsulfinyle en $C_{1\ à\ 6}$, halogénoalkylsulfonyle en $C_{1\ à\ 6}$, halogénoalkylthio en $C_{1\ à\ 6}$, hétéroaryle, hétéroarylcarbonyle, hétéroarylsulfonyle, hétérocyclique, hydroxyle, hydroxylamino, et nitro ;

$R_{10}$, $R_{11}$, $R_{12}$ et $R_{13}$ sont indépendamment choisis parmi les groupes acyle en $C_{1\ à\ 6}$, acyloxy en $C_{1\ à\ 6}$, alcényle en $C_{2\ à\ 6}$, alcoxy en $C_{1\ à\ 6}$, alkyle en $C_{1\ à\ 6}$, alkylamino en $C_{1\ à\ 6}$, alkylcarboxamide en $C_{1\ à\ 6}$, alcynyle en $C_{2\ à\ 6}$, alkylsulfonamide en $C_{1\ à\ 6}$, alkylsulfinyle en $C_{1\ à\ 6}$, alkylsulfonyle en $C_{1\ à\ 6}$, alkylthio en $C_{1\ à\ 6}$, alkylthiocarboxamide en $C_{1\ à\ 6}$, alkylthiouréyle en $C_{1\ à\ 6}$, alkyluréyle en $C_{1\ à\ 6}$, amino, di-alkylamino en $C_{1\ à\ 6}$, alcoxycarbonyle en $C_{1\ à\ 6}$, carboxamide, carboxy, cyano, cycloalkyle en $C_{3\ à\ 6}$, di-alkylcarboxamide en $C_{1\ à\ 6}$, di-alkylsulfonamide en $C_{1\ à\ 6}$, di-alkylthiocarboxamido en $C_{1\ à\ 6}$, halogénoalcoxy en $C_{1\ à\ 6}$, halogénoalkyle en $C_{1\ à\ 6}$, un atome d'halogène, un groupe halogénoalkylsulfinyle en $C_{1\ à\ 6}$, halogénoalkylsulfonyle en $C_{1\ à\ 6}$, halogénoalkylthio en $C_{1\ à\ 6}$, hydroxyle, hydroxylamino, nitro, et thiol ; ou deux groupes $R_{10}$, $R_{11}$, $R_{12}$ et $R_{13}$ adjacents conjointement avec les atomes auxquels ils sont liés forment un groupe cycloalkyle à 5, 6 ou 7 chaînons, un groupe cycloalcényle à 5, 6 ou 7 chaînons où un groupe hétérocyclique à 5, 6 ou 7 chaînons ou le groupe à 5, 6 ou 7 chaînons est facultativement substitué par un atome d'halogène ou un groupe oxo ; et

$R_2$ est choisi parmi les groupes alkyle en $C_{1\ à\ 6}$, aryle, aryloxy, hétéroaryle, et hétéroaryloxy ; dans lequel $R_2$ est facultativement substitué par 1, 2, 3, 4 ou 5 substituants choisis indépendamment parmi les groupes alcoxy en $C_{1\ à\ 6}$, alkyle en $C_{1\ à\ 6}$, alkylamino en $C_{1\ à\ 6}$, amino, di-alkylamino en $C_{1\ à\ 6}$, alcoxycarbonyle en $C_{1\ à\ 6}$,

carboxy, un atome d'halogène, et un groupe hétéroaryle, et où le groupe alkyle en $C_{1\ à\ 6}$ est en outre facultativement substitué par 1, 2, ou 3 substituants choisis indépendamment parmi les groupes alkylamino en $C_{1\ à\ 6}$, di-alkylamino en $C_{1\ à\ 6}$, cycloalkyle en $C_{3\ à\ 6}$, et un atome d'halogène.

**23.** Composé selon la revendication 22, dans lequel K est absent.

**24.** Composé selon la revendication 22 ou 23, dans lequel V est absent.

**25.** Composé selon l'une quelconque des revendications 22 à 24, dans lequel E est N et D est $CHR_2$.

**26.** Composé selon l'une quelconque des revendications 22 à 25, dans lequel $A_1$ et $A_2$ sont tous les deux $-CH_2CH_2-$, et chacun de $A_1$ et $A_2$ est facultativement substitué par 1, 2, 3 ou 4 groupes méthyle.

**27.** Composé selon l'une quelconque des revendications 22 à 26, dans lequel :

W est CH ;
X est N ;
Y est $CR_7$, dans lequel $R_7$ est H, un groupe alkyle en $C_{1\ à\ 6}$, ou un atome d'halogène ; et
Z est N.

**28.** Composé selon la revendication 22, dans lequel ledit composé est choisi parmi les composés et sels, solvates, hydrates et N-oxydes pharmaceutiquement acceptables de ceux-ci suivants :

(A2) la {6-[4-(3-isopropyl-[1,2,4]oxadiazol-5-yl)-pipéridin-1-yl]-pyrimidin-4-yl}-(4-méthanesulfonyl-phényl)-amine ;
(A8) la (2-fluoro-4-méthanesulfonyl-phényl)-{6-[4-(3-fluoro-phénoxy)-pipéridin-1-yl]-pyrimidin-4-yl}-amine ;
(A102) la (2-fluoro-4-méthanesulfonyl-phényl)-{6-[4-(3-isopropyl-[1,2,4]oxadiazol-5-yl)-pipéridin-1-yl]-pyrimidin-4-yl}-amine ;
(A108) la (2-fluoro-4-méthanesulfonyl-phényl)-{6-[4-(4-isobutyl-phényl)-pipéridin-1-yl]-pyrimidin-4-yl}-amine ;
(A109) la (2-fluoro-4-méthanesulfonyl-phényl)-{6-[4-(4-isopropyl-phényl)-pipéridin-1-yl]-pyrimidin-4-yl}-amine ;
(A110) la {6-[4-(3-cyclopropylméthyl-[1,2,4]oxadiazol-5-yl)-pipéridin-1-yl]-pyrimidin-4-yl}-(2-fluoro-4-méthanesulfonyl-phényl)-amine ;
(A111) la (2-fluoro-4-méthanesulfonyl-phényl)-{6-[4-(3-isobutyl-[1,2,4]oxadiazol-5-yl)-pipéridin-1-yl]-pyrimidin-4-yl}-amine ;
(A113) la (2-fluoro-4-méthanesulfonyl-phényl)-{6-[4-(4-isopropoxy-phényl)-pipéridin-1-yl]-pyrimidin-4-yl}-amine ;
(A115) la {6-[4-(3-diméthylaminométhyl-[1,2,4]oxadiazol-5-yl)-pipéridin-1-yl]-pyrimidin-4-yl}-(2-fluoro-4-méthanesulfonyl-phényl)-amine ;
(A117) la (2-fluoro-4-méthanesulfonyl-phényl)-{6-[4-(5-isopropoxy-pyridin-2-yloxy)-pipéridin-1-yl]-pyrimidin-4-yl}-amine ; et
(A118) la (2-fluoro-4-méthanesulfonyl-phényl)-{6-[4-(3-pyridin-3-yl-[1,2,4]oxadiazol-5-yl)-pipéridin-1-yl]-pyrimidin-4-yl}-amine.

**29.** Composition pharmaceutique comprenant au moins un composé selon l'une quelconque des revendications 1 à 28 ou de l'ester d'isopropyle d'acide 4-[6-(2-fluoro-4-méthanesulfonyl-phénoxy) -pyrimidin-4-yloxy]-pipéridine-1-carboxylique, et un véhicule pharmaceutiquement acceptable.

**30.** Procédé de production d'une composition pharmaceutique comprenant le mélange d'au moins un composé selon l'une quelconque des revendications 1 à 28 ou de l'ester d'isopropyle d'acide 4-[6-(2-fluoro-4-méthanesulfonyl-phénoxy)-pyrimidin-4-yloxy]-pipéridine-1-carboxylique, et d'un véhicule pharmaceutiquement acceptable.

**31.** Composé selon l'une quelconque des revendications 1 à 28 ou ester d'isopropyle d'acide 4-[6-(2-fluoro-4-méthanesulfonyl-phénoxy)-pyrimidin-4-yloxy]-pipéridine-1-carboxylique, destiné à une utilisation dans un procédé de traitement du corps humain ou animal en thérapie.

**32.** Composé selon l'une quelconque des revendications 1 à 28 ou ester d'isopropyle d'acide 4-[6-(2-fluoro-4-méthanesulfonyl-phénoxy)-pyrimidin-4-yloxy]-pipéridine-1-carboxylique, destiné à une utilisation dans un procédé de prophylaxie ou de traitement d'un trouble lié au métabolisme.

**33.** Utilisation d'un composé selon l'une quelconque des revendications 1 à 28 ou ester d'isopropyle d'acide 4-[6-(2-fluoro-4-méthanesulfonyl-phénoxy)-pyrimidin-4-yloxy]-pipéridine-1-carboxylique, dans la production d'un médicament destiné à une utilisation en traitement d'un trouble lié au métabolisme.

**34.** Composé selon l'une quelconque des revendications 1 à 28 ou ester d'isopropyle d'acide 4-[6-(2-fluoro-4-méthanesulfonyl-phénoxy)-pyrimidin-4-yloxy]-pipéridine-1-carboxylique, destiné à une utilisation dans un procédé de traitement du diabète de type I, du diabète de type II, de la tolérance inadéquate au glucose, de la résistance à l'insuline, de l'hyperglycémie, de l'hyperlipidémie, de l'hypertriglycéridémie, de l'hypercholestérolémie, de la dyslipidémie ou du syndrome X.

**35.** Utilisation d'un composé selon l'une quelconque des revendications 1 à 28 ou d'ester d'isopropyle d'acide 4-[6-(2-fluoro-4-méthanesulfonyl-phénoxy)-pyrimidin-4-yloxy]-pipéridine-1-carboxylique, dans la production d'un médicament destiné à une utilisation dans le traitement du diabète de type I, du diabète de type II, de la tolérance inadéquate au glucose, de la résistance à l'insuline, de l'hyperglycémie, de l'hyperlipidémie, de l'hypertriglycéridémie, de l'hypercholestérolémie, de la dyslipidémie ou du syndrome X.

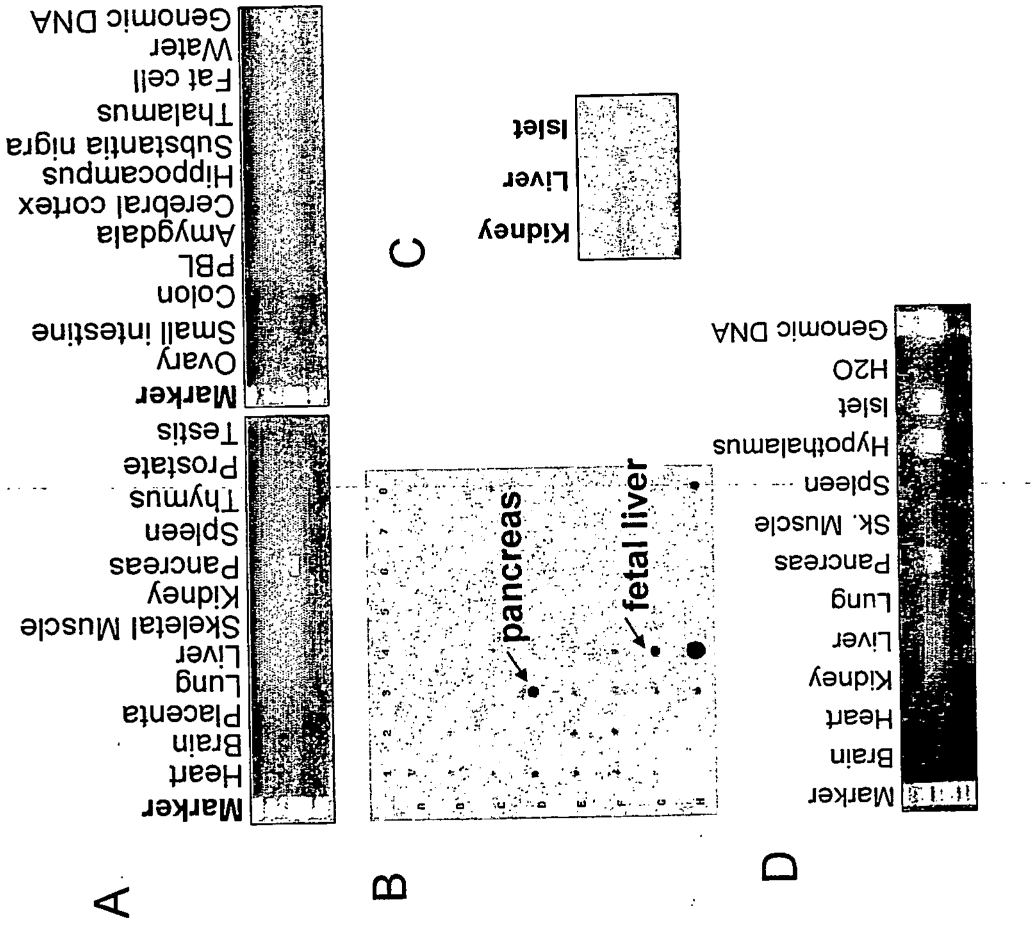
A
Marker
Heart
Brain
Placenta
Lung
Liver
Skeletal Muscle
Kidney
Pancreas
Spleen
Thymus
Prostate
Testis
Marker
Ovary
Small intestine
Colon
PBL
Amygdala
Cerebral cortex
Hippocampus
Substantia nigra
Thalamus
Fat cell
Water
Genomic DNA
B
pancreas
fetal liver
C
Kidney
Liver
Islet
D
Marker
Brain
Heart
Kidney
Liver
Lung
Pancreas
SK. Muscle
Spleen
Hypothalamus
Islet
H2O
Genomic DNA

FIGURE 1

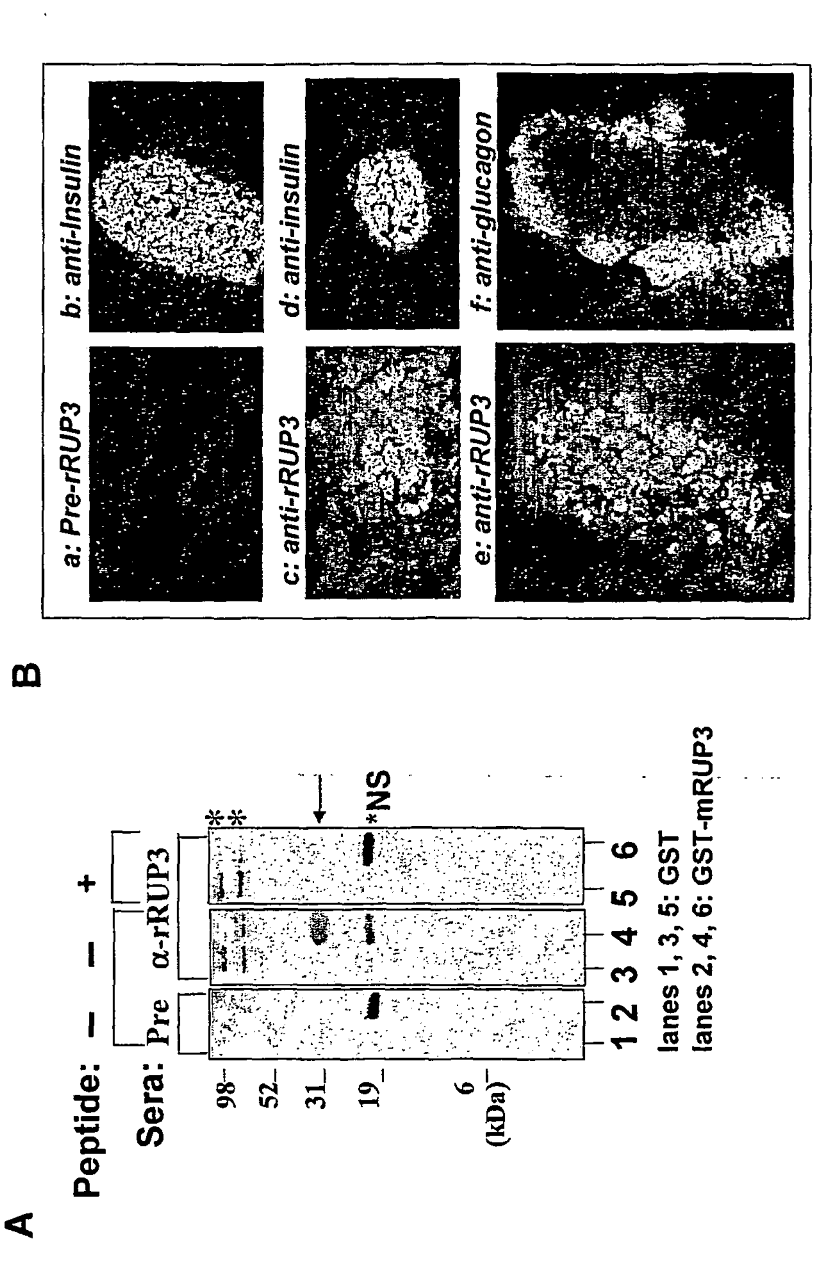
A
Peptide: – – +
Sera: Pre α-rRUP3
98
52
31
19
6
(kDa)
*NS
1 2 3 4 5 6
lanes 1, 3, 5: GST
lanes 2, 4, 6: GST-mRUP3
B
a: Pre-rRUP3
b: anti-Insulin
c: anti-rRUP3
d: anti-insulin
e: anti-rRUP3
f: anti-glucagon

FIGURE 2

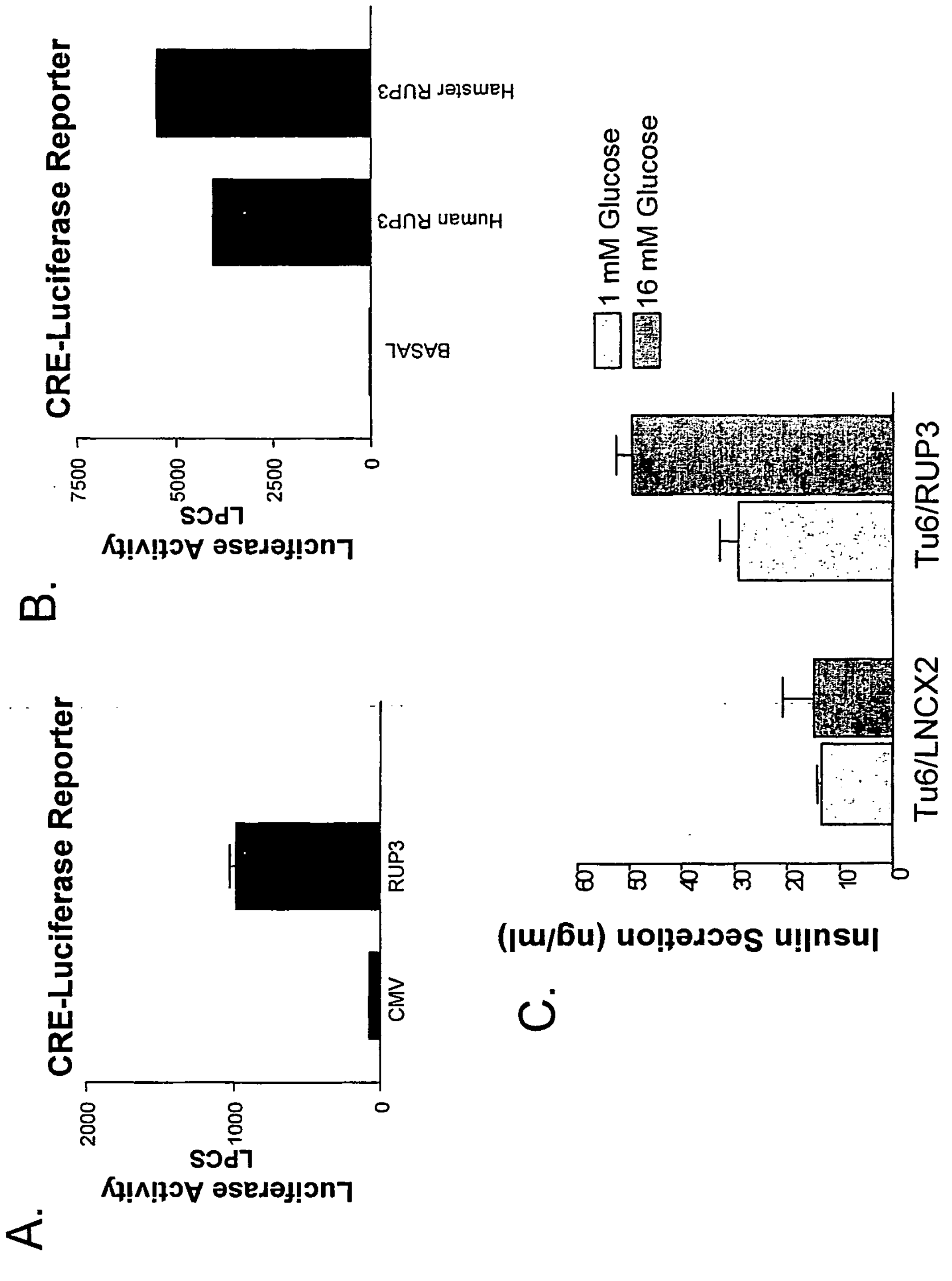
A.
CRE-Luciferase Reporter
Luciferase Activity
LPCS
2000
1000
0
CMV
RUP3
B.
CRE-Luciferase Reporter
Luciferase Activity
LPCS
7500
5000
2500
0
BASAL
Human RUP3
Hamster RUP3
C.
Insulin Secretion (ng/ml)
60
50
40
30
20
10
0
Tu6/LNCX2
Tu6/RUP3
1 mM Glucose
16 mM Glucose

FIGURE 3

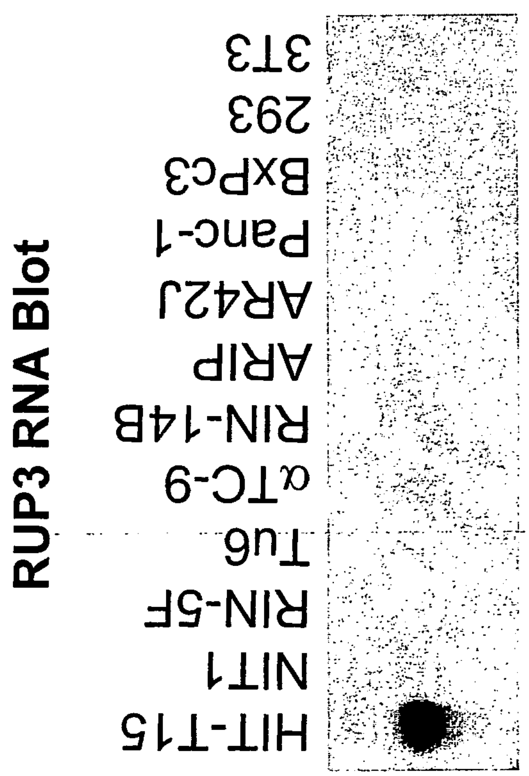
RUP3 RNA Blot
HIT-T15
NIT1
RIN-5F
Tu6
αTC-9
RIN-14B
ARIP
AR42J
Panc-1
BxPc3
293
3T3

FIGURE 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 97008152 A1

### Non-patent literature cited in the description


- **ROTWEIN, R. et al.** *N. Engl. J. Med.,* 1983, vol. 308, 65-71
- **RIMOIN, D. L.** Emery and Rimoin's Principles and Practice of Medical Genetics. 1996, vol. 1, 1401-1402
- **LE STUNFF et al.** *Diabetes,* 1989, vol. 43, 696-702
- **PEDERSON, P.** *Diab. Metab. Rev.,* 1989, vol. 5, 505-509
- **BRANCATI, F. L. et al.** *Arch. Intern. Med.,* 1999, vol. 159, 957-963
- **HILL, J. O. et al.** *Science,* 1998, vol. 280, 1371-1374
- **PERRY, I. J. et al.** *BMJ,* 1995, vol. 310, 560-564
- **GOMTSYAN et al.** *J. Med. Chem.,* 2002, vol. 45, 3639-3648
- **A. G. ARVANITIS et al.** *J. Medicinal Chemistry,* 1999, vol. 42, 805-818
- **S.L. BUCHWALD.** *Top. Curr. Chem.,* 2002, vol. 219, 131
- **D. MA.** *Organic Lett.,* 2003, vol. 5 (14), 2453-2455
- **S. L. BUCHWALD.** *Organic Lett.,* 2002, vol. 4 (6), 973-976
- Organic Chemistry of Sulfur. Plenum Press, 1977
- **WANG Z. ; BASKIN J. M.** *Org. Lett.,* 2002, vol. 4 (25), 4423-4425
- **GREENE ; WUTS.** Protective Groups in Organic Synthesis. John Wiley & Sons, 1999
- Remington, The Science and Practice of Pharmacy. Lippincott Williams & Wilkins, 2000
- **REED ; SCRIBNER.** *Diabetes, Obesity and Metabolism,* 1999, vol. 1, 75-86
- *Journal of Pharmaceutical Science,* 1977, vol. 66, 2
- **T. HIGUCHI ; V. STELLA.** Pro-drugs as Novel Delivery Systems. *A.C.S. Symposium Series,* vol. 14
- Bioreversible Carriers in Drug Design. American Pharmaceutical Association and Pergamon Press, 1987
- **ZHU, D.-G.** *J. Org. Chem.,* 2002, vol. 67, 943-948
- **COLLIER, T. L.** *J. Labeled Compd Radiopharm.,* 1999, vol. 42, S264-S266
- **BAS, M.-D.** *J. Labeled Compd Radiopharm.,* 2001, vol. 44, S280-S282
- **POTENZA, M. N. ; LERNER, M. R.** *Pigment Cell Research,* 1992, vol. 5, 372-378
- *Expert Opin. Ther. Patents,* 2002, vol. 12 (11), 1619-1630